# EUROPÄISCHE PATENTSCHRIFT

(11) **EP 0 937 464 B1**
(45) Veröffentlichungstag und Bekanntmachung des Hinweises auf die Patenterteilung: **11.07.2012**
(21) Anmeldenummer: 98115754.8
(22) Anmeldetag: 21.08.1998
(51) Int. Cl.: A61K 45/06

(54) **Pharmazeutisches Kombinationspräparat aus einem Inhibitor des Natrium-Wasserstoff-Austauschers und einem Arzneimittel zur Behandlung von Herz-Kreislauferkrankungen**
Combined pharmaceutical preparation containing inhibitors of the sodium hydrogen exchange and a medicament for the treatment of cardiovascular diseases
Préparation pharmaceutique sous forme d'association contenant un inhibiteur de l'échange sodium-hydrogène et un médicament pour le traitement de maladies cardiovasculaires

(30) Priorität: 27.08.1997 DE 19737224
(43) Veröffentlichungstag der Anmeldung: 25.08.1999
(73) Patentinhaber: Sanofi-Aventis Deutschland GmbH, 65929 Frankfurt am Main (DE)
(72) Erfinder: Scholz, Wolfgang, Dr., 65760 Eschborn (DE); Albus, Udo, Dr., 61197 Florstadt (DE)
(74) Vertreter: Michalski Hüttermann & Partner

(56) Entgegenhaltungen:
- FRÖHLICH ET AL : "The Na-H exchanger Revisited. " CARDIOVASCULAR RESEARCH, Bd. 36, 1997, Seiten 138-148, XP002246323
- KARWATOWSKA-PROKOCZUK ET AL : "Combined therapy with Dimethylthiourea, diltiazem, and amoloride/dimethylamiloride in the ischemic/reperfused heart" CARDIOVASCULAR RESEARCH, Bd. 30, 1995, Seiten 70-78, XP000985789

## Beschreibung

Die Erfindung betrifft die Kombination von Inhibitoren des Natrium-Wasserstoff-Austauschers mit anderen herz-kreislaufaktiven Substanzen zur Behandlung zur Behandlung von Herz-Kreislauf-Erkrankungen.

Inhibitoren des Natrium/Wasserstoff-Austauschers (NHE) sind in den letzten Jahren in zahlreichen präklinischen Studien als Substanzen charakterisiert worden, die bei Minderdurchblutung des Herzens in überlegener Weise geeignet sind, das gefährdete Herzgewebe vor dem Untergang zu schützen. Der Schutz des Herzgewebes durch NHE Inhibitoren umfaßt alle Ausprägungen der durch die Mangeldurchblutung hervorgerufenen Schädigungen, angefangen bei Herzrhythmusstörungen über Hyperkontraktur des Herzmuskels und vorübergehenden Funktionsverlust bis hin zum Absterben des Herzgewebes und damit verbundenen dauerhaften Schäden.

Der Wirkmechanismus der NHE-Inhibitoren besteht darin, daß Sie den verstärkten Natrium-Ioneneinstrom, der in mangeldurchblutetem Geweben infolge intrazellulärer Ansäuerung und nachfolgender Aktivierung des NHE entsteht, vermindern. Dadurch wird eine Natriumüberladung des Gewebes hinausgezögert. Da im Herzgewebe Natrium- und Calcium-Ionentransport miteinander gekoppelt sind, wird damit auch die lebensbedrohliche Calciumüberladung der Herzzellen verhindert. Dieser einzigartige Wirkmechanismus der NHE-Inhibitoren ermöglicht ihre vorteilhafte Kombination mit Wirkstoffen, die zur Behandlung unterschiedlicher Herz-Kreislauf-Erkrankungen eingesetzt werden und deren Herz-Kreislaufwirkung auf unterschiedlichen Wirkmechanismen beruht.

Dabei können diese Kombinationen eines NHE-Inhibitors aus einem oder mehreren therapeutischen kreislaufaktiven Wirkkomponenten bestehen. Die Kombination der herzschützenden Eigenschaften mit bekannten Therapien von Herzkreislauferkrankungen führt einerseits zu einer Verbesserung der Behandlungsqualität und zum anderen in zahlreichen Kombinationen zu einer additiven oder potenzierenden Verstärkung der Herzkreislauf-Effekte der einzelnen Wirkkomponenten allein. Dabei wirkt sich die mechanistisch bedingte Verhinderung der Natriumüberladung der Herzzellen durch die NHE-Inhibitoren besonders günstig auf den Behandlungserfolg mit dem herzkreislaufaktiven Kombinationspartner aus.

Bei den bekannten und als NHE-Inhibitoren identifizierten Wirkstoffen handelt es sich um Verbindungen der folgenden Formeln:
I. (HOE 89/F 288 - US 5 292 755)
a) Benzoylguanidine der Formel I 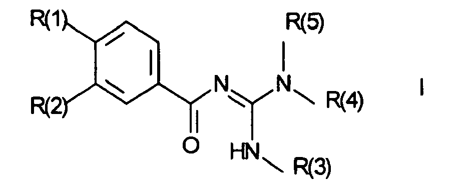 worin bedeuten:
R(1) oder R(2)
   R(6)-S(O)ₙ- oder R(7)R(8)N-O₂S-;
   und der jeweils andere Substituent R(1) oder R(2)
   H, F, Cl, Br, (C₁-C₄)-Alkyl, (C₁-C₄)-Alkoxy oder Phenoxy,
   das unsubstituiert ist oder substituiert mit 1 - 3 Substituenten ausgewählt aus der Gruppe bestehend aus Fluor, Chlor, Methyl und Methoxy;
   oder der jeweils andere Substituent R(1) oder R(2)
R(6)-S(O)ₙ oder R(7)R(8)N-;
n Null, 1 oder 2;
R(6) (C₁-C₆)-Alkyl, (C₅-C₇)-Cycloalkyl, Cyclopentylmethyl, Cyclohexylmethyl oder Phenyl,
   das unsubstituiert ist oder substituiert mit 1 - 3 Substituenten ausgewählt aus der Gruppe bestehend aus Fluor, Chlor, Methyl und Methoxy;
R(7) und R(8)
   gleich oder verschieden H oder (C₁-C₆)-Alkyl;
   oder
R(7) Phenyl-(CH₂)ₘ;
   m 1 - 4;
   oder
R(7) Phenyl,
   das unsubstituiert ist oder substituiert mit 1 - 2 Substituenten ausgewählt aus der Gruppe bestehend aus Fluor, Chlor, Methyl und Methoxy;
   oder
R(7) und R(8)
   gemeinsam eine geradkettige oder verzweigte (C₄-C₇)-Kette, wobei die Kette zusätzlich durch O, S oder NR(9) unterbrochen sein kann;
   R(9) H oder Methyl;
   oder
R(7) und R(8)
   gemeinsam mit dem Stickstoffatom, an das sie gebunden sind, ein Dihydroindol-, Tetrahydrochinolin- oder Tetrahydroisochinolin-System;
R(3), R(4) und R(5) unabhängig voneinander H oder (C₁-C₂)-Alkyl,
   oder
R(3) und R(4)
   gemeinsam eine (C₂-C₄)-Alkylenkette;
   oder
R(4) und R(5)
   gemeinsam eine (C₄-C₇)-Alkylenkette; sowie deren pharmazeutisch verträgliche Salze;
   (HOE 92/F 34 - US 5 373 924)

b) Benzoylguanidine der Formel I 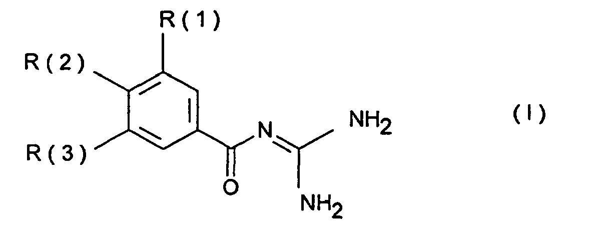 worin bedeuten:
R(1) R(4)-SOₘ oder R(5)R(6)N-SO₂-;
   m Null, 1 oder 2;
   R(4) und R(5)
   C₁-C₈-Alkyl, C₃-C₆-Alkenyl oder -CₙH₂ₙ-R(7);
   n Null, 1, 2, 3 oder 4;
   R(7) C₅-C₇-Cycloalkyl oder Phenyl,
   welches unsubstituiert ist oder substituiert mit 1 - 3 Substituenten ausgewählt aus der Gruppe bestehend aus F, Cl, CF₃, Methyl, Methoxy und NR(8)R(9);
   R(8) und R(9)
   H oder C₁-C₄-Alkyl;
   oder
   R(5) H;
   R(6) H oder C₁-C₄-Alkyl,
   oder
   R(5) und R(6)
   gemeinsam 4 oder 5 Methylengruppen, von denen eine CH₂-Gruppe
   durch ein O, S, NH, N-CH₃ oder N-Benzyl ersetzt sein kann; R(2) Wasserstoff, F, Cl, Br, (C₁-C₄)-Alkyl-, O-(CH₂)ₘCₚF₂ₚ₊₁ oder -X-R(10);
   m Null oder 1;
   p 1, 2 oder 3;
   X O, S oder NR(11);
   R(10) H, C₁-C₆-Alkyl, C₅-C₇-Cycloalkyl, Cyclohexylmethyl, Cyclopentylmethyl oder -CₙH₂ₙ-R(12);
   n Null, 1, 2, 3 oder 4;
   R(12) Phenyl,
   das unsubstituiert oder substituiert ist mit 1 - 3 Substituenten ausgewählt aus der Gruppe bestehend aus F, Cl, CF₃, Methyl, Methoxy und NR(8)R(9);
   R(8) und R(9)
   gleich H oder C₁-C₄-Alkyl;
   R(11) Wasserstoff oder C₁-C₃-Alkyl;
   oder
   R(10) und R(11)
   gemeinsam 4 oder 5 Methylengruppen, von denen eine CH₂-Gruppe durch O, S, NH, N-CH₃ oder N-Benzyl ersetzt sein
   kann;
R(3) wie R(1) definiert, oder C₁-C₆-Alkyl, Nitro, Cyano, Trifluormethyl, F, Cl, Br, J oder -X-R(10);
   X O, S oder NR(11);
      R(10) gleich H, C₁-C₆-Alkyl, C₅-C₇-Cycloalkyl, Cyclohexylmethyl, Cyclopentylmethyl oder -CₙH₂ₙ-R(12);
      n null bis 4;
      R(12) Phenyl, das unsubstituiert ist oder substituiert mit 1 - 3 Substituenten ausgewählt aus der Gruppe bestehend aus F, Cl, CF₃, Methyl, Methoxy und NR(8)R(9);
      R(8) und R(9)
      H oder C₁-C₄-Alkyl;
   R(11) C₁-C₃-Alkyl,
      oder
   R(10) und R(11)
      gemeinsam 4 oder 5 Methylengruppen, von denen eine CH₂-Gruppe durch O, S, NH, N-CH₃ oder N-Benzyl ersetzt sein kann;
   sowie deren pharmazeutisch verträgliche Salze;
   (HOE 92/F 035 EP-OS 556 673)

c) Ortho-substituierte Benzoylguanidine der Formel I 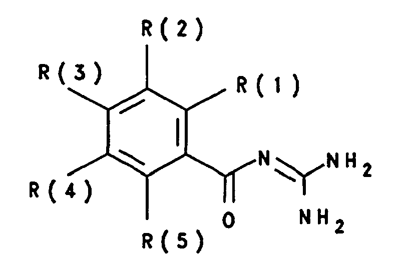 worin bedeuten:
R(1) F, Cl, Br, J, C₁-C₆-Alkyl oder -X-R(6);
   X O, S, NR(7) oder Y-ZO;
   Y O oder NR(7);
   Z C oder SO;
   R(6) H, C₁-C₆-Alkyl, C₅-C₇-Cycloalkyl, Cyclohexylmethyl, Cyclopentylmethyl,
   -(CH₂)ₘCₚF₂ₚ₊₁ oder -CₙH₂ₙ-R(8);
   m Null oder 1;
   p 1 - 3;
   n Null bis 4;
   R(8) Phenyl, das unsubstituiert ist oder substituiert mit 1 - 3 Substituenten ausgewählt aus der Gruppe bestehend aus den Gruppen F, Cl, CF₃, Methyl, Methoxy und NR(9)R(10);
   R(9) und R(10)
   H oder C₁-C₄-Alkyl;
   R(7) H oder C₁-C₃-Alkyl; oder
R(6) und R(7)
   gemeinsam 4 oder 5 Methylengruppen, von denen eine CH₂-Gruppe durch ein O, S, NH, N-CH₃ oder N-Benzyl ersetzt sein kann;
R(3) H oder -X-R(6);
   X O, S, NR(7) oder Y-ZO;
      R(7) H oder C₁-C₃-Alkyl;
      Y O oder NR(7); wobei Y am Phenylrest der Formel I gebunden ist,
      Z C oder SO;
   R(6) H, C₁-C₆-Alkyl, C₅-C₇-Cycloalkyl, Cyclohexylmethyl, Cyclopentylmethyl, -(CH₂)ₘCₚF₂ₚ₊₁ oder -CₙH₂ₙ-R(8);
      m null oder 1;
      p 1 - 3;
      n null bis 4;
      R(8) Phenyl,
      das unsubstituiert ist oder substituiert mit 1 - 3 Substituenten ausgewählt aus der Gruppe bestehend aus F, Cl, CF₃, Methyl, Methoxy und NR(9)R(10); R(9) und R(10)
      H oder C₁-C₄-Alkyl;
      oder
   R(6) und R(7)
      gemeinsam 4 oder 5 Methylengruppen, von denen eine CH₂-Gruppe
      durch ein O, S, NH, N-CH₃ oder N-Benzyl ersetzt sein kann;
   R(2) und R(4)
      gleich oder verschieden R(11)-SO_{q}- oder R(12)R(13)N-SO₂-;
      q null - 2;
      R(11) C₁-C₄-Alkyl, das unsubstituiert ist oder Phenyl als Substituenten trägt, wobei Phenyl unsubstituiert ist oder substituiert mit 1 - 3 Substituenten ausgewählt aus der Gruppe bestehend aus F, Cl, CF₃, Methyl, Methoxy und NR(9)R(10);
      R(9) und R(10)
      H oder C₁-C₄-Alkyl;
      R(12) und R(13)
      wie R(6) und R(7) definiert;
      oder einer der beiden Reste R(2) oder R(4)
      Wasserstoff oder wie R(1) definiert;
   R(5) H, Methyl, F, Cl oder Methoxy,
      sowie deren pharmazeutisch verträgliche Salze.
      (HOE 92/F 036 - US 5 364 868)

d) Benzoylguanidine der Formel I 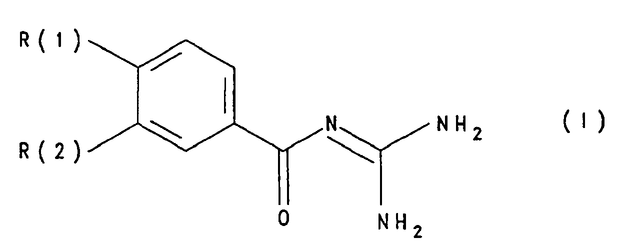 worin bedeuten:
R(1) oder R(2)
   eine Aminogruppe -NR(3)R(4);
   R(3) und R(4) gleich oder verschieden H, C₁-C₆-Alkyl oder C₃-C₇-Cycloalkyl;
      oder
   R(3) Phenyl-(CH₂)ₚ-;
      p 0, 1, 2, 3 oder 4;
      oder
   R(3) Phenyl,
      wobei das Phenyl jeweils unsubstituiert ist oder einen bis zwei Substituenten ausgewählt aus der Gruppe bestehend aus Fluor, Chlor, Methyl und Methoxy trägt;
      oder
   R(3) und R(4)
      gemeinsam eine geradkettige oder verzweigte C₄-C₇-Methylenkette sein können, wobei ein -CH₂-Glied der Methylenkette durch Sauerstoff, S oder NR(5) ersetzt sein kann;
      R(5) H oder niedriges Alkyl;
der jeweils andere Substituent R(1) oder R(2)
H, F, Cl, C₁-C₄-Alkyl, C₁-C₄-Alkoxy, CF₃, CₘF₂ₘ₊₁-CH₂-, Benzyl oder Phenoxy,
wobei der jeweilige Phenylrest unsubstituiert ist oder einen bis zwei Substituenten ausgewählt aus der Gruppe bestehend aus Methyl, Methoxy, Fluor und Chlor trägt;
m 1, 2 oder 3;
sowie deren pharmazeutisch verträgliche Salze;
(92/F 197 K - NZ 248 013)
e) Benzoylguanidine der Formel I 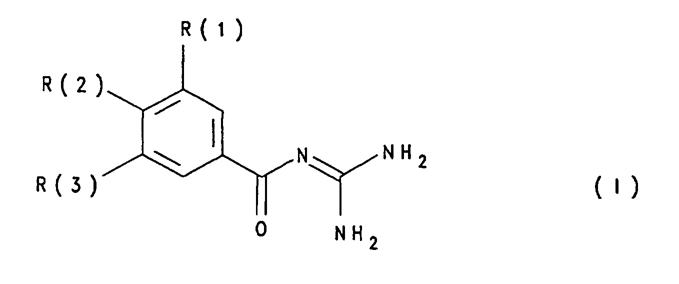 worin bedeuten:
R(1) R(4)-SOₘ oder R(5)R(6)N-SO₂-;
   m Null, 1 oder 2;
   R(4) und R(5)
   C₁-C₈-Alkyl, C₃-C₆-Alkenyl oder -CₙH₂ₙ-R(7);
   n Null, 1, 2, 3 oder 4;
   R(7) C₅-C₇-Cycloalkyl oder Phenyl,
   welches unsubstituiert ist oder substituiert mit 1 - 3 Substituenten ausgewählt aus der Gruppe bestehend aus F, Cl, CF₃, Methyl, Methoxy und NR(8)R(9);
   R(8) und R(9)
   H oder C₁-C₄-Alkyl;
   oder
   R(5) H;
   R(6) H oder C₁-C₄-Alkyl;
   oder
   R(5) und R(6)
   gemeinsam 4 oder 5 Methylengruppen, von denen eine CH₂-Gruppe
   durch ein O, S, NH, N-CH₃ oder N-Benzyl ersetzt sein kann;
R(2) Wasserstoff, geradkettiges oder verzweigtes (C₅-C₈)-Alkyl, -CR(13)=CHR(12) oder -C≡CR(12);
   R(12) Phenyl,
   das unsubstituiert oder substituiert ist mit 1 - 3 Substituenten ausgewählt aus der Gruppe bestehend aus F, Cl, CF₃, Methyl, Methoxy und NR(14)R(15);
   R(14) und R(15)
   H oder (C₁-C₄)-Alkyl;
   oder
   R(12) (C₁-C₉)-Heteroaryl,
   das unsubstituiert oder wie Phenyl substituiert ist, oder
   R(12) (C₁-C₆)-Alkyl,
   das unsubstituiert oder mit 1 - 3 OH substituiert ist,
   oder
   R(12) (C₃-C₈)-Cycloalkyl;
   R(13) Wasserstoff oder Methyl, oder
   R(12) (C₃-C₈)-Cycloalkyl, (C₃-C₈)-Cycloalkyl-(C₁-C₄)-alkyl, Phenyl, C₆H₅-(C₁-C₄)-Alkyl, Naphthyl, Biphenylyl, 1,1-Diphenyl-(C₁-C₄)-Alkyl, Cyclopentadienyl, Pyridyl, Pyrrolyl, Furanyl, Thienyl, Thiazolyl, Oxazolyl, Indenyl, Chinolyl, Indolyl, Benzofuranyl, Benzothienyl, Benzothiazolyl, Benzoxazolyl, Imidazolyl, Pyrazolyl, Triazolyl, Tetrazolyl, Isoxazolyl, Isothiazolyl, Pyrazinyl, Pyrimidinyl, Pyridazinyl, Indazolyl, Isochinolyl, Phthalazinyl, Chinoxalinyl, Chinazolinyl oder Cinnolinyl;
R(3) wie R(2) definiert;
   und wobei die aromatischen Substituenten R(2) bzw. R(3) unsubstituiert oder mit 1-3 Substituenten aus den Gruppen F, Cl, CF₃, (C₁-C₄)-Alkyl, -Alkoxy, oder NR(10)R(11) mit R(10) und R(11) in der Bedeutung von H oder (C₁-C₄)-Alkyl substituiert sind;
   sowie deren pharmazeutisch verträgliche Salze.
   (HOE 92/F 303 K - EP-OS589 336, NZ 248 703)

f) Benzoylguanidine der Formel I 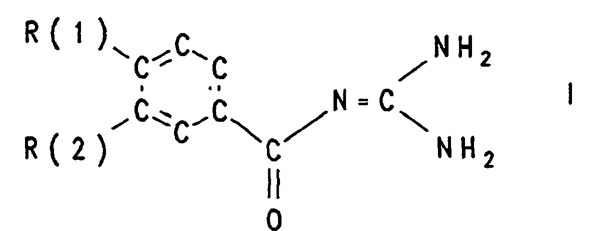 worin bedeuten:
R(1) oder R(2)
   R(3)-S(O)ₙ- oder 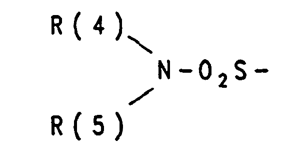 der jeweils andere Substituent R(1) oder R(2)
   H, OH, F, Cl, Br, J, C₁-C₄-Alkyl, C₁-C₄-Alkoxy, Benzyloxy oder Phenoxy, das unsubstituiert ist oder einen bis drei Substituenten ausgewählt aus der Gruppe bestehend aus Fluor, Chlor, Methyl, Methoxy, Hydroxy oder Benzyloxy trägt,
   R(3)-S(O)ₙ, -NR4)R(5) oder 3,4-Dehydropiperidin
   R(3) C₁-C₆-Alkyl, C₅-C₇-Cycloalkyl, Cyclopentylmethyl, Cyclohexylmethyl oder Phenyl,
   das unsubstituiert ist oder einen bis drei Substituenten ausgewählt aus der Gruppe bestehend aus Fluor, Chlor, Methyl und Methoxy trägt;
   R(4) und R(5)
   gleich oder verschieden, H oder C₁-C₆-Alkyl;
   oder
   R(4) Phenyl-(CH₂)ₘ-;
   m 1, 2, 3 oder 4;
   oder
   R(4) Phenyl,
   das unsubstituiert ist oder einen bis zwei Substituenten ausgewählt aus der Gruppe bestehend aus Fluor, Chlor, Methyl und Methoxy trägt;
   oder
   R(4) und R(5)
   gemeinsam eine geradkettige oder verzweigte C₄-C₇-Kette, wobei die Kette zusätzlich durch O, S oder NR(6) unterbrochen sein kann,
   R(6) H oder Methyl;
   oder
   R(4) und R(5)
   gemeinsam mit dem Stickstoffatom, an das sie gebunden sind, ein Dihydroindol, Tetrahydrochinolin oder Tetrahydroisochinolin-System;
   n Null, 1 oder 2;
   sowie deren pharmazeutisch verträgliche Salze.
   (92/F 304 - US 5 416 094)

g) Isochinoline der Formel I 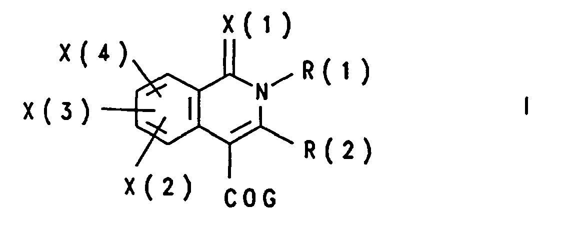 worin bedeuten:
R(1) Wasserstoff, Alkyl, Cycloalkyl, Arylalkyl, Alkenyl, substituiertes Aminoalkyl oder ein Aryl- oder Heteroarylring;
   wobei die ringe unsubstituiert oder substituiert sind mit 1 - 3 Gruppen ausgewählt aus der Gruppe bestehend aus Halogen, Nitro, Amino, Mono(niederalkyl)amino, Di(niederalkyl)amino, Nieder alkyl, Niederalkoxy, Benzyloxy, Phenoxy, Hydroxy, Trifluoromethyl,
R(2) Wasserstoff, Halogen, Alkyl, oder Aryl; welches unsubstituiert ist oder substituiert mit 1 - 3 Gruppen ausgewählt aus der Gruppe bestehend aus Halogen, Nitro, Amino, Mono(niederalkyl)amino, Di(niederalkyl)amino, Niederalkyl, Niederalkoxy, Benzyloxy, Phenoxy, Hydroxy,
G 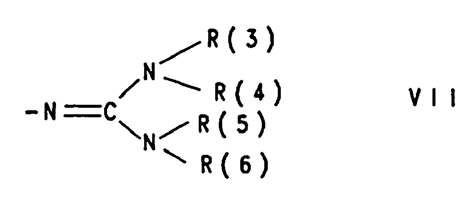
X(2), X(3) and X(4)
   unabhängig voneinander Wasserstoff, Halogen, Nitro, Amino, Alkyl, Sulfonamid, Mono(niederalkyl)amino, Di(niederalkyl)amino, Niederalkyl, Benzyloxy, Hydroxy;
X(1) Wasserstoff, Sauerstoff, Schwefel oder NR(7);
   R(7) Wasserstoff, Alkyl, Cycloalkyl, Arylalkyl, Alkenyl, substituiertes Aminoalkyl oder ein aryl oder ein Heteroarylring;
   welche ringe unsubstituiert oder substituiert sind mit 1 - 3 Gruppen ausgewählt aus der Gruppe bestehend aus Halogen, Nitro, Amino, Mono(niederalkyl)amino, Di(niederalkyl)amino, Niederalkyl, Niederalkoxy, Benzyloxy, Phenoxy, Hydroxy und Trifluoromethyl;
   in welchen Substituenten jede Alkylkette oder Alkenylkette durch Sauerstoff, Schwefel oder NR(8) unterbrochen sein kann;
   R(8) Wasserstoff, Alkyl, Cycloalkyl, Arylalkyl, Alkenyl, substituiertes Aminoalkyl oder ein Aryl- oder Heteroarylring,
   welche Ringe unsubstituiert oder substituiert sind mit 1 - 3 Gruppen ausgewählt aus der Gruppe bestehend aus Halogen, Nitro, Amino, Mono(niederalkyl)amino, Di(niederalkyl)amino, Niederalkyl, Niederalkoxy, Benzyloxy, Phenoxy, Hydroxy und Trifluoromethyl;
   und ihre pharmazeutisch akzeptablen Salze;
   (92/F 404 - EP 602 522, NZ 250 438)

h) Verbindungen der Formel I 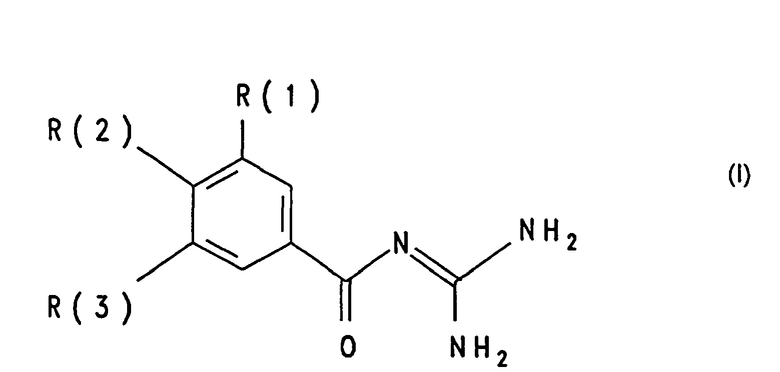 worin bedeuten:
R(1) Wasserstoff, F, Cl, Br, I, -NO₂, -C=N, -CF₃, R(4)-SOₘ oder R(5)R(6)N-SO₂-; m Null, 1 oder 2;
   R(4) und R(5)
   (C₁-C₈)-Alkyl, (C₃-C₆)-Alkenyl, -CₙH₂ₙ-R(7) oder CF₃;
   n Null, 1, 2, 3 oder 4;
   R(7) (C₃-C₇)-Cycloalkyl oder Phenyl,
   welches nicht substituiert ist oder substituiert mit 1 - 3 Substituenten ausgewählt aus der Gruppe bestehend aus F, Cl, CF₃, Methyl, Methoxy und NR(8)R(9); R(8) und R(9)
   H oder C₁-C₄-Alkyl;
   oder
   R(5) H;
   R(6) H oder (C₁-C₄)-Alkyl;
   oder
   R(5) und R(6)
   gemeinsam 4 oder 5 Methylengruppen, von denen eine CH₂-Gruppe durch Sauerstoff, S, NH, N-CH₃ oder N-Benzyl ersetzt sein kann;
R(2) -SR(10), -OR(10), -NHR(10), -NR(10)R(11), -CHR(10)R(12), -[CR(12)R(13)OR(13')], -{C-[CH₂-OR(13')]R(12)(R(13)} oder -[CR(18)R(17)]ₚ-(CO)-[CR(19)R(20)]_{q}-R(14);
   R(10), R(11) gleich oder verschieden
   -[CHR(16)]ₛ-(CH₂)ₚ-(CHOH)_{q}-(CH₂)ᵣ-(CHOH)ₜ-R(21) oder -(CH₂)ₚ-O-(CH₂-CH₂O)_{q}-R(21),
   R(21) Wasserstoff, Methyl,
   p, q, r gleich oder verschieden
   Null, 1, 2, 3 oder 4;
   s Null oder 1;
   t 1, 2, 3 oder 4;
   R(12) und R(13)
   gleich oder verschieden Wasserstoff, (C₁-C₆)-Alkyl oder zusammen mit dem sie tragenden Kohlenstoff-Atom ein (C₃-C₈)-Cycloalkyl,
   R(13') Wasserstoff oder (C₁-C₄)-Alkyl;
   R(14) H, (C₁-C₆)-Alkyl, (C₃-C₈)-Cycloalkyl oder -CₐH₂ₐ-R(15);
   a Null, 1, 2, 3 oder 4;
   R(15) Phenyl,
   das unsubstituiert oder substituiert ist mit 1 - 3 Substituenten ausgewählt aus der Gruppe bestehend aus F, Cl, CF₃, Methyl, Methoxy und NR(8)R(9); R(8) und R(9)
   H oder (C₁-C₄)-Alkyl;
   oder
   R(15) (C₁-C₉)-Heteroaryl,
   das unsubstituiert oder wie Phenyl substituiert ist,
   oder
   R(15) (C₁-C₆)-Alkyl,
   das unsubstituiert oder mit 1 - 3 OH substituiert ist;
   R(16), R(17), R(18), R(19) und R(20)
   Wasserstoff oder (C₁-C₃)-Alkyl;
R(3) wie R(1) definiert,
   oder
R(3) (C₁-C₈)-Alkyl oder -X-R(22);
   X Sauerstoff, S oder NR(16);
   R(16) H oder (C₁-C₃)-Alkyl;
   oder
   R(22) und R(16)
   gemeinsam 4 oder 5 Methylengruppen, von denen eine CH₂-Gruppe durch Sauerstoff, S, NH, N-CH₃ oder N-Benzyl ersetzt sein kann;
   R(22) wie R(14) definiert;
   sowie deren pharmazeutisch verträgliche Salze.
   (HOE 92/F 405 - EP 602 523, NZ 250 437)

i) Benzoylguanidine der Formel I 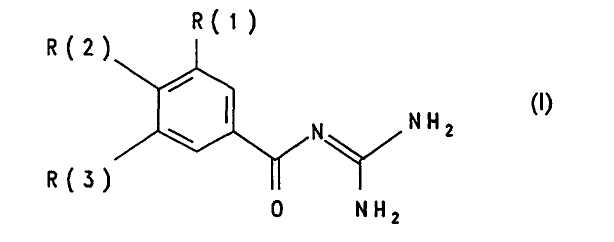 worin bedeuten:
R(1) Wasserstoff, F, Cl, Br, I, -NO₂, -C≡N, R(16)-CₚH₂ₚ-O_{q}, R(4)-SOₘ oder R(5)R(6)N-SO₂-;
   m Null, 1 oder 2;
   p Null oder 1;
   q Null, 1, 2 oder 3;
   R(16) CᵣF₂ᵣ₊₁;
   r 1, 2 oder 3;
   R(4) und R(5)
   (C₁-C₈)-Alkyl, (C₃-C₆)-Alkenyl, -CₙH₂ₙ-R(7) oder CF₃;
   n Null, 1, 2, 3 oder 4;
   R(7) (C₃-C₇)-Cycloalkyl oder Phenyl, welches nicht substituiert ist oder substituiert mit 1 - 3 Substituenten ausgewählt aus der Gruppe bestehend aus F, Cl, CF₃, Methyl, Methoxy und NR(8)R(9);
   R(8) und R(9)
   H oder C₁-C₄-Alkyl;
   oder
   R(5) H;
   R(6) H oder (C₁-C₄)-Alkyl;
   oder
   R(5) und R(6)
   gemeinsam 4 oder 5 Methylengruppen, von denen eine CH₂-Gruppe
   durch Sauerstoff, S, NH, N-CH₃ oder N-Benzyl ersetzt sein kann, R(2) (C₁-C₉)-Heteroaryl,
   das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und
   Dimethylamino;
   oder
R(2) -SR(10), -OR(10), -NR(10)R(11), -CR(10)R(11)R(12);
   R(10) -CₐH₂ₐ-(C₁-C₉)-Heteroaryl,
   das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   a Null, 1 oder 2;
   R(11) und R(12)
   unabhängig voneinander wie R(10) definiert oder Wasserstoff oder
   (C₁-C₄)-Alkyl;
R(3) wie R(1) definiert, oder (C₁-C₆)-Alkyl oder -X-R(13);
   X Sauerstoff, S, oder NR(14);
   R(14) H oder (C₁-C₃)-Alkyl;
   R(13) H, (C₁-C₆)-Alkyl, (C₃-C₈)-Cycloalkyl oder -C_{b}H_{2b}-R(15); b Null, 1, 2, 3 oder 4;
   oder
   R(13) und R(14)
   gemeinsam 4 oder 5 Methylengruppen, von denen CH₂-Gruppe durch Sauerstoff, S, NH, N-CH₃ oder N-Benzyl ersetzt sein kann; R(15) Phenyl,
   das unsubstituiert oder substituiert ist mit 1 - 3 Substituenten ausgewählt aus der Gruppe bestehend aus F, Cl, CF₃, Methyl, Methoxy und NR(8)R(9); R(8) und R(9)
   H oder (C₁-C₄)-Alkyl;
   sowie deren pharmazeutisch verträgliche Salze;
   (HOE 92/F 411 - NZ 250 450, EP 603 650)

k) Benzoylguanidine der Formel I 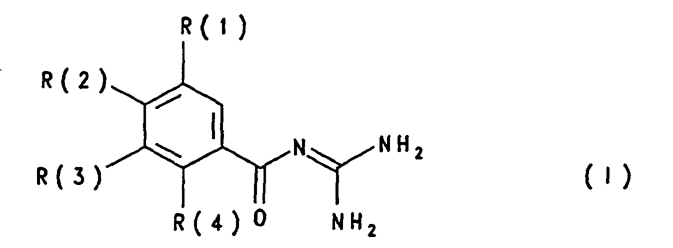 worin bedeuten:
einer der Substituenten R(1), R(2), R(3) oder R(4): eine Aminogruppe 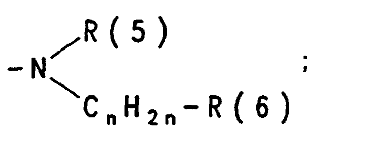 R(5) Wasserstoff oder C₍₁₋₆₎-Alkyl;
n Null, 1, 2, 3 oder 4;
R(6) H oder C₍₁₋₄₎-Alkyl;
worin eine CH₂-Gruppe durch 1 S-Atom oder eine Gruppe NR(7) ersetzt sein kann;
R(7) Wasserstoff, Methyl oder Ethyl;
oder
R(6) C₍₃₋₈₎-Cycloalkyl oder Phenyl,
das unsubstituiert ist oder 1, 2 oder 3 Substituenten ausgewählt aus der Gruppe bestehend aus F, Cl, Br, Methyl, Methoxy, -NR(8)R(9) trägt;
R(8) und R(9)
H, Methyl oder Ethyl;
oder
R(5) und R(6)
gemeinsam mit dem Stickstoffatom einen 5-, 6- oder 7-gliedrigen Ring, in welchem 1 C-Atom durch Sauerstoff, S oder NR(10) ersetzt sein kann;
R(10) H, C₍₁₋₃₎-Alkyl oder Benzyl;
und die jeweils anderen Substituenten R(1), R(2), R(3), R(4):
Wasserstoff, F, Cl, Br, I, CN, CF₃, NO₂, CF₃-O-, CₘF₂ₘ₊₁-CH₂-O- oder R(11)-C_{q}H_{2q}-Xₚ-;
m 1, 2 oder 3;
q Null, 1, 2, 3 oder 4;
p Null oder 1;
X Sauerstoff oder NR(12);
R(12) H oder C₍₁₋₃₎-Alkyl;
R(11) Wasserstoff, C₍₁₋₆₎-Alkyl, C₍₃₋₈₎-Cycloalkyl oder Phenyl,
das unsubstituiert oder substituiert ist mit 1, 2 oder 3 Substituenten ausgewählt aus der Gruppe bestehend aus F, Cl, CH₃, CH₃-O- und NR(13)R(14); R(13), R(14)
H, Methyl oder Ethyl;
sowie deren pharmazeutisch verträgliche Salze.
(HOE 92/F 422 - EP 604 852)
l) Benzoylguanidine der Formel I 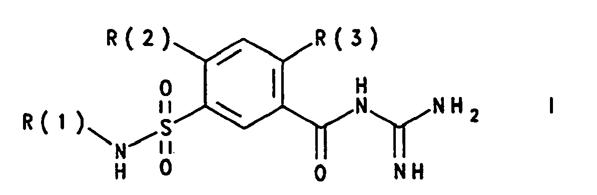 worin bedeuten
R(1) R(4)R(5)N-C(X)-;
X Sauerstoff, S oder N-R(6);
R(4) und R(5)
gleich oder verschieden, H, (C₁-C₈)-Alkyl, (C₃-C₆)-Alkenyl oder -CₙH₂ₙ-R(7);
n Null, 1, 2, 3 oder 4;
R(7) (C₅-C₇)-Cycloalkyl oder Phenyl, welches unsubstituiert ist oder substituiert mit 1 - 3 Substituenten ausgewählt aus der Gruppe bestehend aus F, Cl, CF₃, Methoxy und (C₁-C₄)-Alkyl;
oder
R(4) und R(5) gemeinsam 4 oder 5 Methylengruppen, von denen eine CH₂-Gruppe durch Sauerstoff, S, NH, N-CH₃ oder N-Benzyl ersetzt sein kann; R(6) wie R(4) definiert oder gleich Amidin;
R(2) H, F, Cl, Br, I, (C₁-C₈)-Alkyl, 1-Alkenyl oder 1-Alkinyl, (C₃-C₈)-Cycloalkyl, (C₃-C₈)-Cycloalkyl-(C₁-C₄)-Alkyl, Phenyl, C₆H₅-(C₁-C₄)-Alkyl, Naphthyl, Biphenylyl, 1,1-Diphenyl-(C₁-C₄)-Alkyl, Cyclopentadienyl, Pyridyl, Thiopyridyl, Pyrrolyl, Furanyl, Thienyl, Thiazolyl, Oxazolyl, Indenyl, Chinolyl, Indolyl, Benzofuranyl, Benzothienyl, Benzothiazolyl, Benzoxazolyl oder -W-R(8);
W Sauerstoff, S oder NR(9);
R(8) H, (C₁-C₆)-Alkyl, (C₅-C₇)-Cycloalkyl, Cyclohexylmethyl, Cyclopentylmethyl, -(CH₂)ₘCₚF₂ₚ₊₁ oder -C_{q}H_{2q}-R(10);
m Null oder 1;
p 1, 2 oder 3;
q Null, 1, 2, 3 oder 4;
R(10) Phenyl, das unsubstituiert oder substituiert ist mit 1 - 3 Substituenten ausgewählt aus der Gruppe bestehend aus F, Cl, CF₃, Methyl, Methoxy und NR(11)R(12);
R(11) und R(12)
H oder (C₁-C₄)-Alkyl;
R(9) H oder (C₁-C₃)-Alkyl;
oder
R(8) und R(9) gemeinsam 4 oder 5 Methylengruppen, von denen eine CH₂-Gruppe durch Sauerstoff, S, NH, N-CH₃ oder N-Benzyl ersetzt sein kann;
R(3) H, F, Cl, Br, I, (C₁-C₆)-Alkyl oder -W-R(8) wie für R(2) definiert, sowie deren pharmazeutisch akzeptable Salze;
(93/F 054 - NZ 250 919, EP-OS 612 723)
m) Benzoylguanidine der Formel I worin bedeuten: 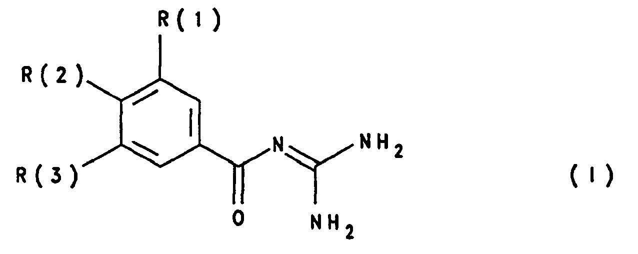 R(1), R(2), R(3)
Wasserstoff, F, Cl, Br, I oder (C₁-C₁₂)-Alkyl; einer der Substituenten R(1), R(2) oder R(3)
N₃, CN, OH oder (C₁-C₁₀)-Alkyloxy, wenn mindestens einer der restlichen Substituenten R(1), R(2) oder R(3) einen hinreichend lipophilen Alkylrest mit 3 bis 12 Kohlenstoff-Atomen bedeutet;
oder
einer der Substituenten R(1), R(2) oder R(3)
R(4)-CₙH₂ₙ-Oₘ-;
m Null oder 1;
n Null, 1, 2 oder 3;
R(4) CₚF₂ₚ₊₁;
p 1, 2 oder 3, sofern n gleich Null oder 1 ist;
oder
R(4) (C₃-C₁₂)-Cycloalkyl, Phenyl, Pyridyl, Chinolyl oder Isochinolyl, wobei die aromatischen und heteroaromatischen Ringsysteme unsubstituiert sind oder substituiert mit einem Substituenten ausgewählt aus der Gruppe bestehend aus F, Cl, CF₃, Methyl, Methoxy und NR(5)R(6);
R(5) und R(6)
Wasserstoff oder (C₁-C₄)-Alkyl;
oder einer der Substituenten R(1), R(2) oder R(3)
-C≡CR(5) oder -C[R(6)] = CR(5);
R(5) Phenyl,
das unsubstituiert oder substituiert ist mit 1 - 3 Substituenten ausgewählt aus der Gruppe bestehend aus F, Cl, CF₃, Methyl, Methoxy, Hydroxy, Amino, Methylamino und Dimethylamino, (C₁-C₉)-Heteroaryl,
das unsubstituiert oder wie Phenyl substituiert ist,
oder
R(5) (C₁-C₆)-Alkyl,
das unsubstituiert oder mit 1 - 3 OH substituiert ist;
oder
R(5) (C₃-C₈)-Cycloalkyl,
R(6) Wasserstoff oder Methyl;
sowie deren pharmakologisch akzeptable Salze;
(93/F 153 - EP-OS 627 413, NZ 260 660)
o) Benzoylguanidine der Formel I 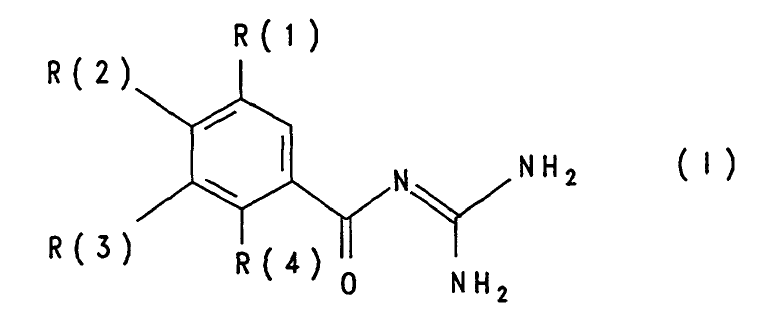 worin bedeuten:
R(1) Wasserstoff, F, Cl, Br, I, -NO₂, -C≡N, Xₒ-(CH₂)ₚ-(CF₂)_{q}-CF₃, R(5)-SOₘ, R(6)-CO- oder R(6)R(7)N-SO₂-, mit
   X Sauerstoff, S oder NR(14);
   m Null, 1 oder 2;
   o Null oder 1;
   p Null, 1 oder 2;
   q Null, 1, 2, 3, 4, 5 oder 6;
   R(5) und R(6)
   (C₁-C₈)-Alkyl, (C₃-C₆)-Alkenyl, -CₙH₂ₙ-R(8) oder CF₃;
   n Null, 1, 2, 3 oder 4;
   R(8) (C₃-C₇)-Cycloalkyl oder Phenyl,
   welches nicht substituiert ist oder substituiert mit 1 - 3 Substituenten ausgewählt aus der Gruppe bestehend aus F, Cl, CF₃, Methyl, Methoxy und NR(9)R(10); R(9) und R(10)
   H oder C₁-C₄-Alkyl;
   oder
   R(6) H;
   R(7) H oder (C₁-C₄)-Alkyl;
   oder
   R(6) und R(7) gemeinsam 4 oder 5 Methylengruppen, von denen eine CH₂-Gruppe durch Sauerstoff, S, NH, N-CH₃ oder N-Benzyl ersetzt sein kann; 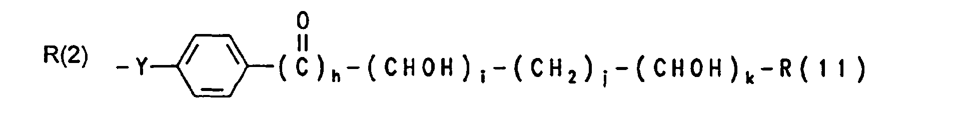 oder 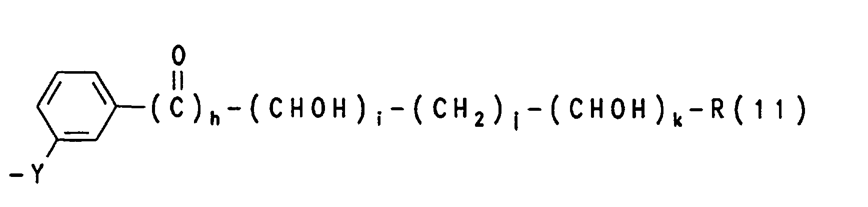 oder 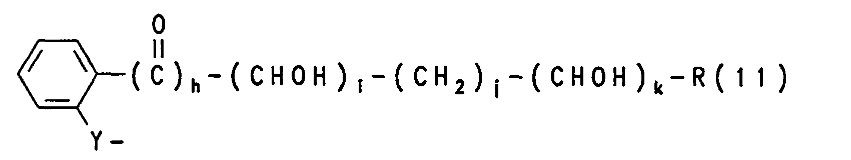 Y Sauerstoff, -S- oder -NR(12)-;
   R(11) und R(12)
   Wasserstoff oder (C₁-C₃)-Alkyl;
   h Null oder 1;
   i, j und k
   unabhängig Null, 1, 2, 3 oder 4;
   wobei jedoch nicht h, i und k gleichzeitig Null sind,
R(3) wie R(1) definiert, oder (C₁-C₆)-Alkyl oder -X-R(13);
   X Sauerstoff, S oder NR(14);
   R(14) H oder (C₁-C₃)-Alkyl;
   R(13) H, (C₁-C₆)-Alkyl, (C₃-C₈)-Cycloalkyl oder -C_{b}H_{2b}-R(15);
   b Null, 1, 2, 3 oder 4;
   oder R(13) und R(14)
   gemeinsam 4 oder 5 Methylengruppen, wobei eine CH₂-Gruppe durch Sauerstoff, S, NH, N-CH₃ oder N-Benzyl ersetzt sein kann;
   R(15) Phenyl,
   das unsubstituiert oder substituiert ist mit 1 - 3 Substituenten ausgewählt aus der Gruppe bestehend aus F, Cl, CF₃, Methyl, Methoxy und NR(9)R(10); R(9) und R(10) H oder (C₁-C₄)-Alkyl;
R(4) Wasserstoff, -OR(16) oder -NR(16)R(17);
   R(16) und R(17)
   unabhängig Wasserstoff oder (C₁-C₃)-Alkyl; sowie deren pharmazeutisch verträgliche Salze;
(HOE 93/F 154 - EP-OS 628 543, NZ 260 681)
p) Benzoylguanidine der Formel I 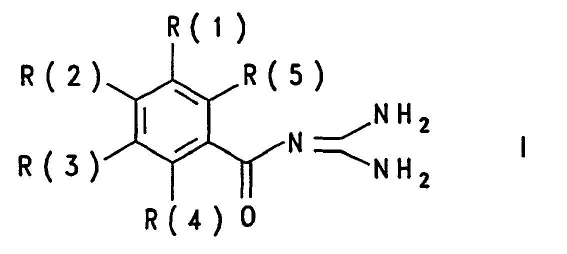 worin bedeuten:
R(1) R(6)-CO oder R(7)R(8)N-CO;
      R(6) (C₁-C₈)-Alkyl, (C₁-C₈)-Perfluoralkyl, (C₃-C₈)-Alkenyl oder -CₙH₂ₙ-R(9);
      n Null, 1, 2, 3 oder 4;
      R(9) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(10)R(11);
      R(10) und R(11)
      H, (C₁-C₄)-Alkyl oder (C₁-C₄)-Perfuoralkyl;
   R(7) H, (C₁-C₈)-Alkyl, (C₁-C₈)-Perfluoralkyl, (C₃-C₈)-Alkenyl oder -CₙH₂ₙ-R(12);
      n Null, 1, 2, 3 oder 4;
      R(12) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(13)R(14); R(13) und R(14)
      H, (C₁-C₄)-Alkyl oder (C₁-C₄)-Perfuoralkyl;
   R(8) H, (C₁-C₄)-Alkyl oder (C₁-C₄)-Perfluoralkyl;
      oder
   R(7) und R(8)
      gemeinsam 4 oder 5 Methylengruppen, von denen eine CH₂-Gruppe durch Sauerstoff, S, NH, N-CH₃ oder N-Benzyl ersetzt sein kann;
R(2) R(1) definiert, oder H, F, Cl, Br, I, CN, NO₂, (C₁-C₈)-Alkyl, (C₁-C₈)-Perfluoralkyl, (C₃-C₈)-Alkenyl oder -CₙH₂ₙR(15);
   n Null, 1, 2, 3, 4;
   R(15) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(16)R(17);
   R(16) und R(17)
   H, (C₁-C₄)-Alkyl oder (C₁-C₄)-Perfuoralkyl;
   oder
R(2) (C₁-C₉)-Heteroaryl,
   das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   oder
R(2) SR(18), -OR(18), -NR(18)R(19), -CR(18)R(19)R(20);
   R(18) -CₐH₂ₐ-(C₁-C₉)-Heteroaryl,
   das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   a Null, 1 oder 2;
   R(19) und R(20)
   unabhängig voneinander wie R(18) definiert oder Wasserstoff, (C₁-C₄)-Alkyl oder (C₁-C₄)-Perfluoralkyl;
   oder
R(2) R(21)-SOₘ oder R(22)R(23)N-SO₂-;
   m 1 oder 2;
   R(21) (C₁-C₈)-Alkyl, (C₁-C₈)-Perfluoralkyl, (C₃-C₈)-Alkenyl, -CₙH₂ₙ-R(24),
   n Null, 1, 2, 3 oder 4;
   R(24) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(27)R(28); R(27) und R(28)
   H, (C₁-C₄)-Alkyl oder (C₁-C₄)-Perfuoralkyl;
   R(22) H, (C₁-C₈)-Alkyl, (C₁-C₈)-Perfluoralkyl, (C₃-C₈)-Alkenyl, -CₙH₂ₙ-R(29);
   n Null, 1, 2, 3 oder 4;
   R(29) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(30)R(31);
   R(30) und R(31)
   H, (C₁-C₄)-Alkyl oder (C₁-C₄)-Perfuoralkyl;
   R(23) H, (C₁-C₄)-Alkyl oder (C₁-C₄)-Perfluoralkyl;
   oder
   R(22) und R(23)
   gemeinsam 4 oder 5 Methylengruppen, von denen eine CH₂-Gruppe
   durch Sauerstoff, S, NH, N-CH₃ oder N-Benzyl ersetzt sein kann;
   oder
R(2) R(33)X-;
   X Sauerstoff, S, NR(34), (D=O)A-, NR(34)C=MN⁽*⁾R(35)-;
   M Sauerstoff oder S;
   A Sauerstoff oder NR(34);
   D C oder SO;
   R(33) (C₁-C₈)-Alkyl, (C₃-C₈)-Alkenyl, (CH₂)_{b}C_{d}F_{2d+1}, -CₙH₂ₙ-R(36),
   b Null oder 1;
   d 1, 2, 3, 4, 5, 6 oder 7;
   n Null, 1, 2, 3, oder 4;
   R(36) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(37)R(38);
   R(37) und R(38)
   H, (C₁-C₄)-Alkyl oder (C₁-C₄)-Perfuoralkyl;
   R(34) H, (C₁-C₄)-Alkyl oder (C₁-C₄)-Perfuoralkyl;
   R(35) definiert wie R(33);
   oder
   R(33) und R(34) gemeinsam 4 oder 5 Methylengruppen, von denen eine CH₂-Gruppe durch Sauerstoff, S, NH, N-CH₃ oder N-Benzyl ersetzt sein kann;
   wobei A und N⁽*⁾ an den Phenylkern des Benzoylguanidin-Grundkörpers gebunden sind;
   oder
R(2) -SR(40), -OR(40), -NHR(40), -NR(40)R(41), -CHR(40)R(42), -C[R(42)R(43)OH], -C≡CR(45), -CR(46)=CHR(45), -[CR(47)R(48)]ᵤ-(CO)-[CR49)R(50)]ᵥ-R(44); R(40), R(41)
   gleich oder verschieden -(CH₂)ₚ-(CHOH)_{q}-(CH₂)ᵣ-(CHOH)ₜ-R(51) oder-(CH₂)ₚ-O-(CH₂-CH₂O)_{q}-R(51);
   R(51) Wasserstoff oder Methyl;
   u 1, 2, 3 oder 4;
   v Null, 1, 2, 3 oder 4;
   p, q, r
   gleich oder verschieden Null, 1, 2, 3 oder 4;
   t 1, 2, 3 oder 4;
   R(42) und R(43)
   gleich oder verschieden Wasserstoff oder (C₁-C₆)-Alkyl;
   oder
   R(42) und R(43)
   zusammen mit dem sie tragenden Kohlenstoff-Atom ein (C₃-C₈)-Cycloalkyl bilden;
   R(44) H, (C₁-C₆)-Alkyl, (C₃-C₈)-Cycloalkyl oder -CₑH₂ₑ-R(45);
   e Null, 1, 2, 3 oder 4;
   R(45) gleich Phenyl,
   das unsubstituiert oder substituiert ist mit 1 - 3 Substituenten aus der Gruppe bestehend aus F, Cl, CF₃, Methyl, Methoxy und NR(52)R(53) mit
   R(52) und R(53) gleich H oder (C₁-C₄)-Alkyl, oder
   R(45) (C₁-C₉)-Heteroaryl,
   das unsubstituiert oder wie Phenyl substituiert ist;
   oder
   R(45) (C₁-C₆)-Alkyl,
   das unsubstituiert oder mit 1 - 3 OH substituiert ist;
   R(46), R(47), R(48), R(49) und R(50)
   Wasserstoff oder Methyl;
   oder
R(2) R(55)-NH-SO₂-;
   R(55) R(56)R(57)N-(C=Y)-;
   Y Sauerstoff, S oder N-R(58);
   R(56) und R(57)
   gleich oder verschieden H, (C₁-C₈)-Alkyl, (C₃-C₆)-Alkenyl oder -C_{f}H_{2f}-R(59);
   f Null, 1, 2, 3 oder 4;
   R(59) (C₅-C₇)-Cycloalkyl oder Phenyl, welches unsubstituiert ist oder substituiert mit 1 - 3 Substituenten ausgewählt aus der Gruppe bestehend aus F, Cl, CF₃, Methoxy und (C₁-C₄)-Alkyl;
   oder
   R(56) und R(57)
   gemeinsam 4 oder 5 Methylengruppen, von denen eine CH₂-Gruppe durch Sauerstoff, S, NH, N-CH₃ oder N-Benzyl ersetzt sein kann;
   R(58) wie R(56) definiert oder Amidin;
R(3), R(4) und R(5)
   unabhängig voneinander wie R(1) oder R(2) definiert; sowie deren pharmazeutisch verträgliche Salze;
   (HOE 93/F 220 - EP-OS 640 593, NZ 264 117)

q) Benzoylguanidine der Formel I 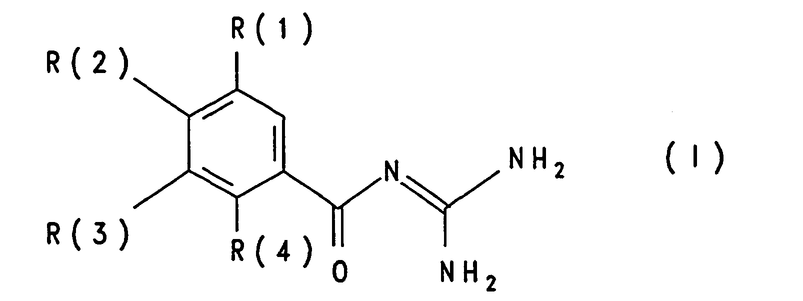 worin bedeuten:
R(1) Wasserstoff, F, Cl, Br, I, -NO₂, -C≡N, -Xₒ-(CH₂)ₚ-(CF₂)_{q}-CF₃, R(5)-SOₘ-, R(6)-CO-, R(6)R(7)N-CO- oder R(6)R(7)N-SO₂-;
   X Sauerstoff, -S- oder NR(14);
   m Null, 1 oder 2;
   o Null oder 1;
   p Null, 1 oder 2;
   q Null, 1, 2, 3, 4, 5 oder 6;
   R(5) und R(6)
   (C₁-C₈)-Alkyl, (C₃-C₆)-Alkenyl, -CₙH₂ₙ-R(8) oder CF₃;
   n Null, 1, 2, 3 oder 4;
   R(8) (C₃-C₇)-Cycloalkyl, Phenyl,
   welches nicht substituiert ist oder substituiert mit 1 bis 3 Substituenten ausgewählt aus der Gruppe bestehend aus F, Cl, CF₃, Methyl, Methoxy und NR(9)R(10); R(9) und R(10)
   H oder (C₁-C₄)-Alkyl;
   oder
   R(6) Wasserstoff;
   R(7) Wasserstoff oder (C₁-C₄)-Alkyl;
   oder
   R(6) und R(7)
   gemeinsam 4 oder 5 Methylengruppen, von denen eine CH₂-Gruppe durch Sauerstoff, S, NH, N-CH₃ oder N-Benzyl ersetzt sein kann; 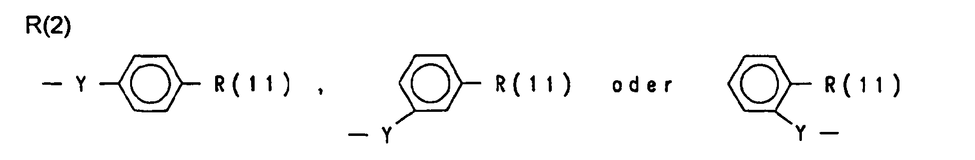 R(11) (C₁-C₉)-Heteroaryl,
   das über C oder N verknüpft ist und das unsubstituiert oder substituiert ist mit 1 bis 3 Substituenten ausgewählt aus der Gruppe bestehend aus F, Cl, CF₃, CH₃, Methoxy, Hydroxy, Amino, Methylamino, Dimethylamino und Benzyl;
   Y Sauerstoff, -S- oder NR(12);
   R(12) H oder (C₁-C₄)-Alkyl;
R(3) wie R(1) definiert;
   oder
R(3) (C₁-C₆)-Alkyl oder -X-R(13);
   X Sauerstoff, -S- oder NR(14);
   R(14) H oder (C₁-C₃)-Alkyl; R(13) H, (C₁-C₆)-Alkyl, (C₃-C₈)-Cycloalkyl oder -C_{b}H_{2b}-R(15);
   b Null, 1, 2, 3 oder 4;
   oder
   R(13) und R(14)
   gemeinsam 4 oder 5 Methylengruppen, von denen eine CH₂-Gruppe durch Sauerstoff, S, NH, N-CH₃ oder N-Benzyl ersetzt sein kann;
   R(15) Phenyl,
   das unsubstituiert oder substituiert ist mit 1 - 3 Substituenten ausgewählt aus der Gruppe bestehend aus F, Cl, CF₃, Methyl, Methoxy und NR(9)R(10); R(9) und R(10)
   H oder (C₁-C₄)-Alkyl;
R(4) Wasserstoff, -OR(16), -NR(16)R(17) oder CᵣF_{2r+1;}
   R(16) und R(17)
   unabhängig Wasserstoff oder (C₁-C₃)-Alkyl;
   r 1, 2, 3 oder 4;
   sowie deren pharmazeutisch verträgliche Salze;
   (HOE 93/F 223 K - EP 639 573, NZ 264 130)

r) Benzokondensierte 5-Ringheterocyclen der Formel I 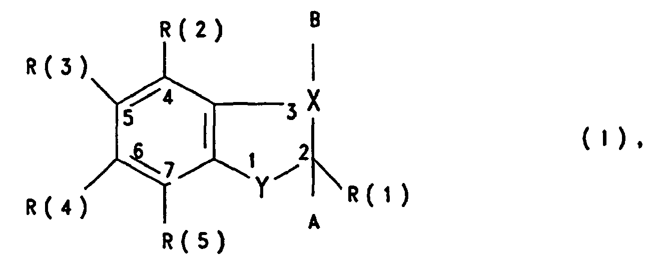 worin bedeuten:
X N oder CR(6);
Y Sauerstoff, S oder NR(7);
A, B gemeinsam eine Bindung
   oder
A, B beide Wasserstoff, sofern gleichzeitig X gleich CR(6) und Y gleich NR(7) sind; einer der Substituenten R(1) bis R(6) eine -CO-N=C(NH₂)₂-Gruppe; die jeweils anderen Substituenten R(1) bis R(6)
   Wasserstoff, F, Cl, Br, I oder (C₁-C₆))-Alkyl;
   bis zu zwei der anderen Substituenten R(1) bis R(6)
   CN, NO₂, N₃, (C₁-C₄)-Alkyloxy oder CF₃;
   bis zu einem der anderen Substituenten
   R(8)-CₙH₂ₙ-Z-;
   n Null bis 10; wobei die Alkylenkette -CₙH₂ₙ- geradkettig oder verzweigt ist und wobei ein C-Atom durch ein Sauerstoff- oder S-Atom oder durch ein N-Atom ersetzt sein kann;
R(8) Wasserstoff, (C₂-C₆)-Alkenyl oder (C₃-C₁₀)-Cycloalkyl, das unsubstituiert oder durch 1 bis 4 Methylgruppen oder eine OH-Gruppe substituiert ist, oder eine Ethylengruppe -CH=CH-enthalten kann, und worin eine Methylengruppe durch ein Sauerstoff- oder S-Atom oder durch ein N-Atom ersetzt sein kann;
   oder
R(8) Phenyl,
   unsubstituiert oder substituiert durch 1 bis 3 Substituenten ausgewählt aus der Gruppe bestehend aus F, Cl, Br, I, CF₃, CH₃-S(O)ₛ- oder R(9)-W_{y}-;
   s Null, 1 oder 2;
   R(9) H, Methyl, Ethyl,
   W Sauerstoff oder NR(10);
   R(10) H oder Methyl;
   y Null oder 1;
   oder
R(8) CₘF₂ₘ₊₁;
   m 1 bis 3;
   oder
R(8) 1- oder 2-Naphthyl, Pyridyl, Chinolyl oder Isochinolyl;
   Z -CO-, -CH₂- oder -[CR(11)(OH)]_{q}-;
   q 1, 2 oder 3;
   R(11) H oder Methyl;
   oder
   Z Sauerstoff oder -NR(12)-;
   R(12) H oder Methyl;
   oder
   Z -S(O)ₛ-;
   s Null, 1 oder 2;
   oder
   Z -SO₂-NR(13)-;
   R(13) H oder (C₁-C₄)-Alkyl;
   R(7) Wasserstoff, (C₁-C₁₀)-Alkyl, (C₂-C₁₀)-Alkenyl oder R(8)-CₙH₂ₙ-; sowie deren pharmazeutisch verträgliche Salze;
   (HOE 93/F 236 - EP-OS 638 548, NZ 264 216)

s) Benzoylguanidine der Formel I 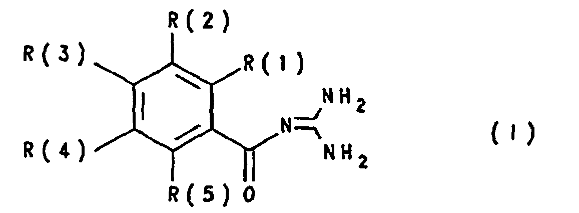 worin bedeuten:
R(1), R(3) oder R(4)
   -NR(6) C=X NR(7)R(8);
   X Sauerstoff oder S;
   R(6) Wasserstoff, (C₁-C₈)-Alkyl, (C₁-C₈)-Perfluoralkyl, (C₃-C₈)-Alkenyl oder -CₙH₂ₙ-R(9);
   n Null, 1, 2, 3 oder 4;
   R(9) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(10)R(11); R(10) und R(11)
   H, (C₁-C₄)-Alkyl oder (C₁-C₄)-Perfluoralkyl;
   R(7) Wasserstoff, (C₁-C₈)-Alkyl, (C₁-C₈)-Perfluoralkyl, (C₃-C₈)-Alkenyl oder -CₒH₂ₒ-R(12);
   o Null, 1, 2, 3 oder 4;
   R(12) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(13)R(14); R(13) und R(14)
   H, (C₁-C₄)-Alkyl oder (C₁-C₄)-Perfluoralkyl;
   R(8) definiert wie R(7);
   oder
   R(7) und R(8)
   gemeinsam 4 oder 5 Methylengruppen, von denen eine CH₂-Gruppe durch Sauerstoff, S, NH, N-CH₃ oder N-Benzyl ersetzt sein kann;
   die jeweils verbleibenden Substituenten R(2), R(3), R(4), R(5) oder R(1), R(2), R(4), R(5) oder R(1), R(2), R(3), R(5)
   unabhängig voneinander Wasserstoff, F, Cl, Br, I, -Oₜₐ(C₁-C₈)-Alkyl, -O_{tb}(C₃-C₈)-Alkenyl,
   -O_{tc}(CH₂)_{b}C_{d}F_{2d+1}, -O_{td}CₚH₂ₚR(18),
   oder bis zu 2 Gruppen CN, NO₂, NR(16)R(17),
   - b: Null oder 1;
   - d: 1, 2, 3, 4, 5, 6 oder 7;
   - ta: Null oder 1;
   - tb: Null oder 1;
   - tc: Null oder 1;
   - td: Null oder 1;
   - p: Null, 1, 2, 3 oder 4;
R(18) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(19)R(20); R(19) und R(20)
Wasserstoff oder (C₁-C₄)-Alkyl oder (C₁-C₄)-Perfluoralkyl;
R(16) Wasserstoff, (C₁-C₈)-Alkyl, (C₁-C₈)-Perfluoralkyl, (C₃-C₈)-Alkenyl, -
C_{q}H_{2q}-R(21),
q Null, 1, 2, 3 oder 4;
R(21) (C₃-C₈)-Cycloalkyl, Phenyl, Biphenylyl oder Naphthyl, wobei die Aromaten nicht substituiert oder substituiert sind mit 1 - 3 Substituenten aus der Gruppe F, Cl, CF₃, Methyl, Methoxy oder NR(22)R(23),
R(22) und R(23) Wasserstoff, (C₁-C₄)-Alkyl oder (C₁-C₄)-Perfluoralkyl;
R(17) Wasserstoff, (C₁-C₈)-Alkyl, (C₁-C₈)-Perfluoralkyl, (C₃-C₈)-Alkenyl, -CᵣH₂ᵣ-R(24);
r Null, 1, 2, 3 oder 4;
R(24) (C₃-C₈)-Cycloalkyl, Phenyl, Biphenylyl oder Naphthyl,
wobei die Aromaten nicht substituiert oder substituiert sind mit 1 - 3 Substituenten ausgewählt aus der Gruppe bestehend aus F, Cl, CF₃, Methyl, Methoxy und NR(25)R(26);
R(25) und R(26)
Wasserstoff, (C₁-C₄)-Alkyl oder (C₁-C₄)-Perfluoralkyl;
oder
R(16) und R(17)
gemeinsam 4 oder 5 Methylengruppen, von denen eine CH₂-Gruppe
durch Sauerstoff, S, NH, N-CH₃ oder N-Benzyl ersetzt sein kann; sowie deren pharmazeutisch verträgliche Salze;
(HOE 93/F 249 - EP-OS 640 587, NZ 264 282)
t) Diacylsubstituierte Guanidine der Formel I 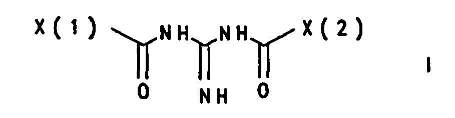 worin bedeuten:
X(1) und X(2) 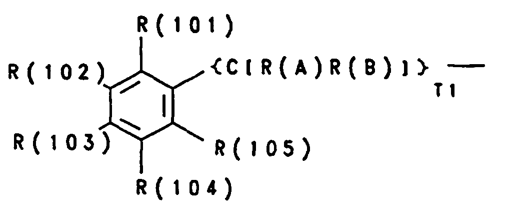
T1 Null, 1, 2, 3 oder 4;
R(A) und R(B)
unabhängig Wasserstoff, F, Cl, Br, I, CN, OR(106), (C₁-C₈)-Alkyl, (C₃-C₈)-Cycloalkyl, O_{zk}(CH₂)_{zl}C_{zm}F_{2zm+1}, NR(107)R(108), Phenyl oder Benzyl, wobei die Aromaten nicht substituiert oder substituiert sind mit 1 - 3 Substituenten ausgewählt aus der Gruppe bestehend aus F, Cl, CF₃, Methyl, Methoxy und NR(109)R(110); R(109) und R(110)
Wasserstoff, (C₁-C₄)-Alkyl oder (C₁-C₄)-Perfluoralkyl;
zl Null, 1, 2, 3 oder 4;
zk Null oder 1;
zm 1, 2, 3, 4, 5, 6, 7 oder 8;
R(106)
Wasserstoff, (C₁-C₈)-Alkyl, (C₁-C₈)-Perfluoralkyl, (C₃-C₈)-Alkenyl, (C₃-C₈)-Cycloalkyl, Phenyl oder Benzyl,
wobei die Aromaten nicht substituiert oder substituiert sind mit 1 - 3 Substituenten ausgewählt aus der Gruppe bestehend aus F, Cl, CF₃, Methyl, Methoxy und NR(111)R(112);
R(111) und R(112)
Wasserstoff, (C₁-C₄)-Alkyl oder (C₁-C₄)-Perfluoralkyl;
R(107) und R(108)
unabhängig voneinander wie R(106) definiert,
oder
R(107) und R(108)
gemeinsam 4 oder 5 Methylengruppen, von denen eine CH₂-Gruppe durch Sauerstoff, S, NH, N-CH₃ oder N-Benzyl ersetzt sein kann;
oder
X(1) und X(2) 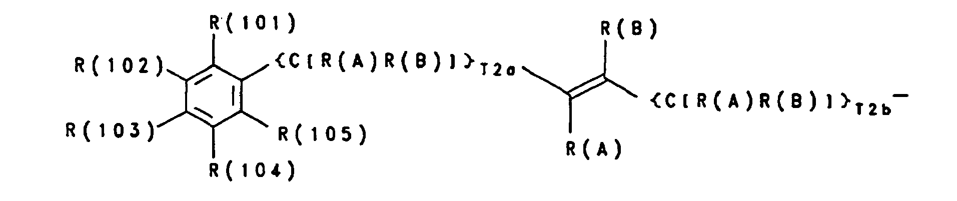 T2a und T2b
unabhängig voneinander Null, 1 oder 2;
wobei die Doppelbindung (E)- oder (Z)-konfiguriert sein kann;
oder
X(1) und X(2) 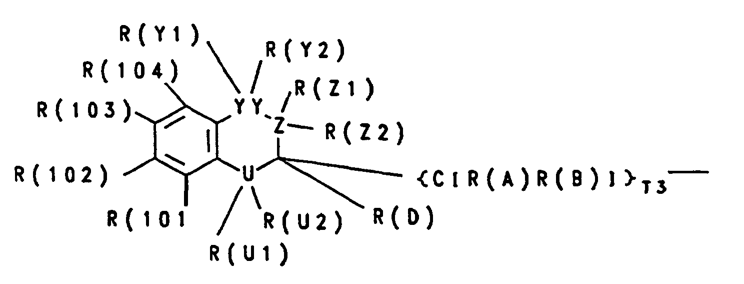 T3 Null, 1 oder 2;
U, YY und Z
unabhängig voneinander C oder N,
wobei U, YY, Z folgende Anzahl von Substituenten tragen können:

| U, YY oder Z | im Ring an eine Doppelbindung gebunden | Anzahl der zugelassenen Substituenten |
|---|---|---|
| C | ja | 1 |
| C | nein | 2 |
| N | ja | 0 |
| N | nein | 1 |

R(D) Wasserstoff, (C₁-C₈)-Alkyl oder (C₁-C₈)-Perfluoralkyl,
R(U1), R(U2), R(Y1), R(Y2), R(Z1), R(Z2)
unabhängig voneinander Wasserstoff, F, Cl, Br, I, CN, OR(114), (C₁-C₈)-Alkyl, (C₃-C₈)-Cycloalkyl, O_{zka}(CH₂)_{zla}C_{zma}F_{2zma+1}, NR(115)R(116), Phenyl oder Benzyl,
wobei die Aromaten nicht substituiert oder substituiert sind mit 1 - 3 Substituenten ausgewählt aus der Gruppe bestehend aus F, Cl, CF₃, Methyl, Methoxy, NR(117)R(118),
R(117) und R(118)
Wasserstoff, (C₁-C₄)-Alkyl oder (C₁-C₄)-Perfluoralkyl,
zka Null oder 1;
zla Null, 1, 2, 3 oder 4;
zma 1, 2, 3, 4, 5, 6, 7 oder 8;
R(114)
Wasserstoff, (C₁-C₈)-Alkyl, (C₁-C₈)-Perfluoralkyl, (C₃-C₈)-Alkenyl, (C₃-C₈)-Cycloalkyl, Phenyl oder Benzyl,
wobei die Aromaten nicht substituiert oder substituiert sind mit 1 - 3 Substituenten ausgewählt aus der Gruppe bestehend aus F, Cl, CF₃, Methyl, Methoxy und NR(119)R(120);
R(119) und R(120)
Wasserstoff, (C₁-C₄)-Alkyl oder (C₁-C₄)-Perfluoralkyl;
R(115) und R(116) unabhängig voneinander wie R(114) definiert;
oder
R(115) und R(116)
gemeinsam 4 oder 5 Methylengruppen, von denen eine CH₂-Gruppe durch Sauerstoff, S, NH, N-CH₃ oder N-Benzyl ersetzt sein kann;
wobei jedoch die Konstitution U gleich Stickstoff (N), YY gleich Stickstoff (N) und Z gleich Kohlenstoff (C) ausgenommen ist,
R(101), R(102), R(103), R(104) und R(105)
unabhängig voneinander Wasserstoff, F, Cl, Br, I, -C≡N, X_{zoa}-(CH₂)_{zpa}-(C_{zqa}F_{2zqa+1}), R(110a)-SO_{zbm}, R(110b)R(110c)N-CO, R(111a)-CO- oder R(112a)R(113a)N-SO₂-,
wobei die Perfluoralkylgruppe geradkettig oder verzweigt ist,
X Sauerstoff, S oder NR(114a);
R(114a)
H oder (C₁-C₃)-Alkyl;
zoa Null oder 1;
zbm Null, 1 oder 2;
zpa Null, 1, 2, 3 oder 4;
zqa 1, 2, 3, 4, 5, 6, 7 oder 8;
R(110a), R(110b), R(111 a) und R(112a)
unabhängig (C₁-C₈)-Alkyl, (C₃-C₈)-Alkenyl, -C_{zn}H_{2zn}-R(115a) oder (C₁-C₈)-Perfluoralkyl;
zn Null, 1, 2, 3 oder 4;
R(115a)
(C₃-C₈)-Cycloalkyl, Phenyl, Biphenylyl oder Naphthyl,
wobei die Aromaten nicht substituiert sind oder substituiert mit 1 - 3 Substituenten ausgewählt aus der Gruppe bestehend aus F, Cl, CF₃, Methyl, Methoxy und NR(116a)R(117a);
R(116a) und R(117a)
Wasserstoff, (C₁-C₄)-Perfluoralkyl oder (C₁-C₄)-Alkyl;
oder
R(110b), R(111a) und R(112a)

Wasserstoff;
R(110c) und R(113a)
unabhängig Wasserstoff, (C₁-C₄)-Perfluoralkyl oder (C₁-C₄)-Alkyl;
oder
R(110b) und R(110c) sowie R(112a) und R(113a)
gemeinsam 4 oder 5 Methylengruppen, von denen eine CH₂-Gruppe durch Sauerstoff, Schwefel, NH, N-CH₃ oder N-Benzyl ersetzt sein kann;
oder
R(101), R(102), R(103), R(104), R(105)
unabhängig voneinander (C₁-C₈)-Alkyl, -C_{zal}H_{2zal}R(118a) oder (C₃-C₈)-Alkenyl, zal Null, 1, 2, 3 oder 4;
R(118a)
(C₃-C₈)-Cycloalkyl, Phenyl, Biphenylyl oder Naphthyl,
wobei die Aromaten nicht substituiert oder substituiert sind mit 1 - 3 Substituenten aus der Gruppe bestehend aus F, Cl, CF₃, Methyl, Methoxy oder NR(119a)R(119b); R(119a) und R(119b)
Wasserstoff, (C₁-C₄)-Alkyl oder (C₁-C₄)-Perfluoralkyl; oder
R(101), R(102), R(103), R(104), R(105)
unabhängig voneinander (C₁-C₉)-Heteroaryl,
das über C oder N verknüpft ist und das unsubstituiert oder substituiert ist mit 1 - 3 Substituenten aus der Gruppe bestehend aus F, Cl, CF₃, CH₃, Methoxy, Hydroxy, Amino, Methylamino und Dimethylamino;
oder
R(101), R(102), R(103), R(104), R(105) unabhängig voneinander -C≡C-R(193);
R(193)
Phenyl, das nicht substituiert oder substituiert ist mit 1 - 3 Substituenten aus der Gruppe bestehend aus F, Cl, CF₃, Methyl, Methoxy oder NR(194)R(195);
R(194) und R(195)
Wasserstoff oder CH₃;
oder
R(101), R(102), R(103), R(104), R(105)
unabhängig voneinander
-Y-para-C₆H₄-(CO)_{zh}-(CHOH)_{zi}-(CH₂)_{zj}-(CHOH)_{zk}-R(123),
- Y-meta-C₆H₄-(CO)_{zad}-(CHOH)_{zae}-(CH₂)_{zaf}-(CHOH)_{zag}-R(124) oder
-Y-ortho-C₆H₄-(CO)_{zah}-(CHOH)_{zao}-(CH₂)_{zap}-(CHOH)_{zak}-R(125);
Y Sauerstoff, -S- oder -NR(122d)-;
zh, zad, zah
unabhängig Null oder 1;
zi, zj, zk, zae, zaf, zag, zao, zap und zak
unabhängig Null, 1, 2, 3 oder 4;
wobei jedoch jeweils
zh, zi und zk nicht gleichzeitig Null,
zad, zae und zag nicht gleichzeitig Null sowie
zah, zao und zak nicht gleichzeitig Null sind,
R(123), R(124) R(125) und R(122d)
unabhängig Wasserstoff oder (C₁-C₃)-Alkyl;
oder
R(101), R(102), R(103), R(104) und R(105)
unabhängig voneinander SR(129), -OR(130), -NR(131)R(132) oder
-CR(133)R(134)R(135);
R(129), R(130), R(131) und R(133)
unabhängig -C_{zab}H_{2zab}-(C₁-C₉)-Heteroaryl, das unsubstituiert oder substituiert ist mit 1 - 3 Substituenten aus der Gruppe bestehend aus F, Cl, CF₃, CH₃, Methoxy, Hydroxy, Amino, Methylamino und Dimethylamino;
zab Null, 1 oder 2;
R(132), R(134) und R(135)
unabhängig voneinander wie R(129) definiert oder Wasserstoff, (C₁-C₄)-Alkyl oder (C₁-C₄)-Perfluoralkyl;
oder
R(101), R(102), R(103), R(104) und R(105)
unabhängig voneinander -W-para-(C₆H₄)-R(196), -W-meta-(C₆H₄)-R(197) oder -W-ortho-(C₆H₄)-R(198);
R(196), R(197) und R(198)
unabhängig (C₁-C₉)-Heteroaryl,
das über C oder N verknüpft ist und das unsubstituiert oder substituiert ist mit 1 bis 3 Substituenten aus der Gruppe bestehend aus F, Cl, CF₃, CH₃, Methoxy, Hydroxy, Amino, Methylamino, Dimethylamino und Benzyl;
W Sauerstoff, S oder NR(136)-;
R(136)
Wasserstoff oder (C₁-C₄)-Alkyl;
oder
R(101), R(102), R(103), R(104) und R(105)
unabhängig voneinander R(146)X(1a)-;
X(1 a)
Sauerstoff, S, NR(147), (D=O)A-, NR(148)C=MN⁽*⁾R(149)-;
M Sauerstoff oder Schwefel;
A Sauerstoff oder NR(150); D C oder SO;
R(146)
(C₁-C₈)-Alkyl, (C₃-C₈)-Alkenyl, (CH₂)_{zbz}C_{zdz}F_{2zdz+1} oder -C_{zxa}H_{2zxa}-R(151);
zbz Null oder 1;
zdz 1, 2, 3, 4, 5, 6 oder 7;
zxa Null, 1, 2, 3 oder 4;
R(151)
(C₃-C₈)-Cycloalkyl, Phenyl, Biphenylyl oder Naphthyl, wobei die Aromaten nicht substituiert oder substituiert sind mit 1 - 3 Substituenten aus der Gruppe bestehend aus F, Cl, CF₃, Methyl, Methoxy und NR(152)R(153); R(152) und R(153)
Wasserstoff, (C₁-C₄)-Alkyl oder (C₁-C₄)-Perfluoralkyl;
R(147), R(148) und R(150)
unabhängig Wasserstoff, (C₁-C₄)-Alkyl, (C₁-C₄)-Perfluoralkyl;
R(149) definiert wie R(146),
oder
R(146) und R(147) beziehungsweise R(146) und R(148)
gemeinsam 4 oder 5 Methylengruppen, von denen eine CH₂-Gruppe durch Sauerstoff, Schwefel, NH, N-CH₃ oder N-Benzyl ersetzt sein kann;
wobei A und N⁽*⁾ an den Phenylkern des Alkanoyl-Grundkörpers gebunden sind;
oder
R(101), R(102), R(103), R(104) und R(105)
unabhängig voneinander -SR(164), -OR(165), -NHR(166), -NR(167)R(168), - CHR(169)R(170), -CR(154)R(155)OH, -C≡CR(156), -CR(158)=CR(157) oder -[CR(159)R(160)]_{zu}-(C=O)-[CR(161)R(162)]_{zv}-R(163); R(164), R(165), R(166), R(167), R(169)
gleich oder verschieden -(CH₂)_{zy}-(CHOH)_{zz}-(CH₂)_{zaa}-(CHOH)_{zt}-R(171) oder-(CH₂)_{zab}-O-(CH₂-CH₂O)_{zac}-R(172);
R(171) und R(172)
Wasserstoff oder Methyl;
zu 1, 2, 3 oder 4;
zv Null, 1, 2, 3 oder 4;
zy, zz, zaa, zab, zac
gleich oder verschieden Null, 1, 2, 3 oder 4;
zt 1, 2, 3 oder 4;
R(168), R(170), R(154), R(155)
gleich oder verschieden Wasserstoff oder (C₁-C₆)-Alkyl, oder
R(169) und R(170) beziehungsweise R(154) und R(155)
zusammen mit dem sie tragenden Kohlenstoff-Atom ein (C₃-C₈)-Cycloalkyl;
R(163)
Wasserstoff, (C₁-C₆)-Alkyl, (C₃-C₈)-Cycloalkyl oder -C_{zeb}H_{2zeb}-R(173);
zeb Null, 1, 2, 3 oder 4;
R(156), R(157) und R(173)
unabhängig Phenyl, das unsubstituiert oder substituiert ist mit 1 - 3 Substituenten aus der Gruppe bestehend aus F, Cl, CF₃, Methyl, Methoxy und NR(174)R(175);
R(174) und R(175)
Wasserstoff oder (C₁-C₄)-Alkyl;
oder
R(156), R(157) und R(173)
unabhängig (C₁-C₉)-Heteroaryl,
das unsubstituiert oder wie Phenyl substituiert ist;
R(158), R(159), R(160), R(161) und R(162)
Wasserstoff oder Methyl,
oder
R(101), R(102), R(103), R(104), R(105)
unabhängig voneinander R(176)-NH-SO₂-;
R(176)
R(177)R(178)N-(C=Y')-;
Y' Sauerstoff, S oder N-R(179);
R(177) und R(178)
gleich oder verschieden Wasserstoff, (C₁-C₈)-Alkyl, (C₃-C₆)-Alkenyl oder -C_{zfa}H_{2zfa}-R(180);
zfa Null, 1, 2, 3 oder 4;
R(180)
(C₅-C₇)-Cycloalkyl oder Phenyl,
welches unsubstituiert mit 1 - 3 Substituenten ausgewählt aus der Gruppe bestehend aus F, Cl, CF₃, Methoxy oder (C₁-C₄)-Alkyl;
oder
R(177) und R(178)
gemeinsam 4 oder 5 Methylengruppen, von denen eine CH₂-Gruppe durch Sauerstoff, Schwefel, NH, N-CH₃ oder N-Benzyl ersetzt sein kann;
R(179)
wie R(177) definiert oder gleich Amidin,
oder
R(101), R(102), R(103), R(104), R(105)
unabhängig voneinander NR(184a)R(185), OR(184b), SR(184c) oder -C_{znx}H_{2znx}-R(184d);
znx Null, 1, 2, 3 oder 4;
R(184d)
(C₃-C₇)-Cycloalkyl oder Phenyl,
welches nicht substituiert ist oder substituiert mit 1 - 3 Substituenten aus der Gruppe bestehend aus F, Cl, CF_{3,} Methyl, Methoxy und NR(116k)R(117k); R(116k) und R(117k)
Wasserstoff oder C₁-C₄-Alkyl;
R(184a), R(184b), R(184c), R(185)
unabhängig voneinander Wasserstoff, (C₁-C₈)-Alkyl, (C₁-C₈)-Perfluoralkyl oder (CH₂)_{zao}-R(184g);
zao Null, 1, 2, 3 oder 4;
184g
(C₃-C₇)-Cycloalkyl oder Phenyl,
welches nicht substituiert ist oder substituiert mit 1 - 3 Substituenten aus der Gruppe bestehend aus F, Cl, CF₃, Methyl, Methoxy und NR(184u)R(184v);
R(184u) und R(184v)
Wasserstoff oder C₁-C₄-Alkyl;
oder
R(184a) und R(185)
gemeinsam 4 oder 5 Methylengruppen, von denen eine CH₂-Gruppe durch Sauerstoff, Schwefel, NH,
N-CH₃ oder N-Benzyl ersetzt sein kann;
sowie deren pharmazeutisch verträgliche Salze;
(HOE 93/F 254 - EP-OS 640 588, NZ 264 307)
u) Benzoylguanidine der Formel I 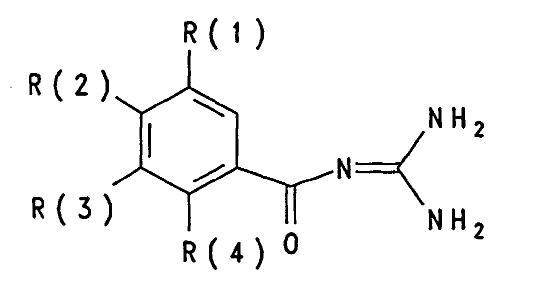 worin bedeuten:
R(1) H, F, Cl, Br, I, CN, NO₂, (C₁-C₈)-Alkyl, (C₃-C₈)-Cycloalkyl oder
   Xₐ-(CH₂)_{b}-(CF₂)_{c}-CF₃;
   X Sauerstoff, S oder NR(5);
   a Null oder 1;
   b Null, 1 oder 2;
   c Null, 1, 2 oder 3;
   R(5) H, (C₁-C₄)-Alkyl oder -C_{d}H_{2d}R(6);
   d Null, 1, 2, 3 oder 4;
   R(6) (C₃-C₈)-Cycloalkyl, Phenyl, Biphenylyl oder Naphthyl, wobei die Aromaten nicht substituiert oder substituiert sind mit 1 bis 3 Substituenten ausgewählt aus der Gruppe bestehend aus F, Cl, CF₃, Methyl, Methoxy und NR(7)R(8);
   R(7) und R(8)
   unabhängig H oder (C₁-C₄)-Alkyl;
   oder
R(1) -SR(10), -OR(10) oder -CR(10)R(11)R(12);
   R(10) -C_{f}H_{2f}-(C₃-C₈)-Cycloalkyl, -(C₁-C₉)-Heteroaryl oder Phenyl, wobei die aromatischen Systeme unsubstituiert oder substituiert sind mit 1 bis 3 Substituenten ausgewählt aus der Gruppe bestehend aus F, Cl, CF₃, CH₃, Methoxy, Hydroxy, Amino, Methylamino und Dimethylamino;
   f Null, 1 oder 2;
   R(11) und R(12)
   unabhängig voneinander wie R(10) definiert oder Wasserstoff oder (C₁-C₄)-Alkyl;
   oder
R(1) Phenyl, Naphthyl, Biphenylyl oder (C₁-C₉)-Heteroaryl,
   letzteres über C oder N verknüpft, und die unsubstituiert oder substituiert sind mit 1 bis 3 Substituenten ausgewählt aus der Gruppe bestehend aus F, Cl, CF₃, CH₃, Methoxy, Hydroxy, Amino, Methylamino und Dimethylamino;
   oder
R(1) -SR(13), -OR(13), -NHR(13), -NR(13)R(14), -CHR(13)R(15), -C[R(15)R(16)]OH, -C≡CR(18), -C[R(19)]=CR(18), -[CR(20)R(21)]ₖ-(CO)-[CR(22)R(23)R(24)], R(13) und R(14)
   gleich oder verschieden -(CH₂)_{g}-(CHOH)ₕ-(CH₂)ᵢ-(CHOH)ⱼ-R(17),
   R(17) Wasserstoff oder Methyl;
   -(CH₂)_{g}-O-(CH₂-CH₂O)ₕ-R(24),
   g, h, i
   gleich oder verschieden Null, 1, 2, 3 oder 4;
   j 1, 2, 3 oder 4;
   R(15) und R(16)
   gleich oder verschieden Wasserstoff, (C₁-C₆)-Alkyl oder zusammen mit dem sie tragenden Kohlenstoff-Atom ein (C₃-C₈)-Cycloalkyl;
   R(18) Phenyl, das unsubstituiert oder substituiert ist mit 1 bis 3 Substituenten ausgewählt aus der Gruppe bestehend aus F, Cl, CF₃, Methyl, Methoxy und NR(25)R(26); R(25) und R(26)
   H oder (C₁-C₄)-Alkyl;
   oder
   R(18) (C₁-C₉)-Heteroaryl,
   das unsubstituiert oder wie Phenyl substituiert ist;
   oder
   R(18) (C₁-C₆)-Alkyl,
   das unsubstituiert oder mit 1 bis 3 OH substituiert ist;
   oder
   R(18) (C₃-C₈)-Cycloalkyl;
   R(19), R(20), R(21), R(22) und R(23)
   Wasserstoff oder Methyl;
   k Null, 1, 2, 3 oder 4;
   l Null, 1, 2, 3 oder 4;
   R(24) H, (C₁-C₆)-Alkyl, (C₃-C₈)-Cycloalkyl oder -CₘH₂ₘ-R(18);
   m 1, 2, 3 oder 4;
R(2) und R(3)
   unabhängig voneinander wie R(1) definiert;
R(4) (C₁-C₃)-Alkyl, F, Cl, Br, I, CN oder -(CH₂)ₙ-(CF₂)ₒ-CF₃;
   n Null oder 1;
   o Null, 1 oder 2;
   sowie deren pharmazeutisch verträgliche Salze; (HOE 93/F 436 - EP-OS 659 748), NZ 270 264) v) Acylguanidine der Formel I 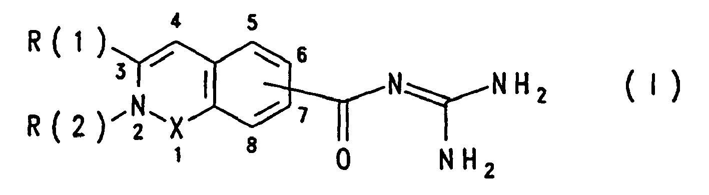 worin bedeuten:
X Carbonyl, Sulfonyl,
R(1) H, (C₁-C₈₎-Alkyl,
   unsubstituiert oder durch Hydroxy substituiert,
   (C₃-C₈)-Cycloalkyl, Phenyl,
   das unsubstituiert oder substituiert ist mit 1 - 3 Substituenten aus der Gruppe F, Cl, CF₃, CH₃, Methoxy, Hydroxy, Amino, Methylamino oder
   Dimethylamino,
R(2) H, (C₁-C₄₎-Alkyl,
   sowie deren pharmazeutisch verträgliche Salze;
   (HOE 94/F 014 K - EP-OS 666 252, NZ 270 370)

w) Perfluoralkylgruppen tragende phenylsubstituierte Alkylcarbonsäure-guanidide der Formel I 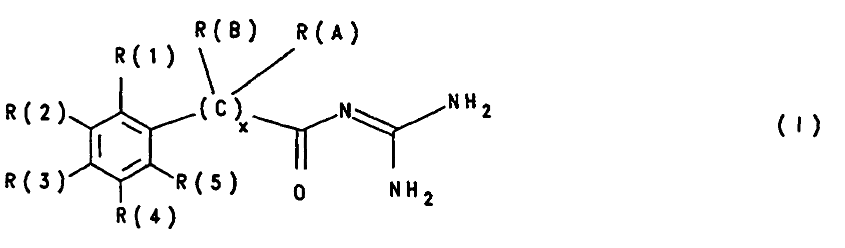 worin bedeuten:
R(A) Wasserstoff, F, Cl, Br, I, CN, OR(6), (C₁-C₈)-Alkyl, (C₃-C₈)-Cycloalkyl,
   Oᵣ(CH₂)ₐC_{b}F_{2b+1} oder NR(7)R(8);
   r Null oder 1;
   a Null, 1, 2, 3 oder 4;
   b 1, 2, 3, 4, 5, 6, 7 oder 8;
   R(6) Wasserstoff, (C₁-C₈)-Alkyl, (C₃-C₈)-Alkenyl, (C₃-C₈)-Cycloalkyl, Phenyl oder Benzyl,
   wobei die Aromaten nicht substituiert oder substituiert sind mit 1 - 3 Substituenten ausgwählt aus der Gruppe bestehend aus F, Cl, CF₃, Methyl, Methoxy und NR(9)R(10); R(9) und R(10)
   H, (C₁-C₄)-Alkyl oder (C₁-C₄)-Perfluoralkyl;
   R(7) und R(8)
   unabhängig voneinander wie R(6) definiert;
R(B) unabhängig wie R(A) definiert;
X 1, 2 oder 3;
R(1 ) Wasserstoff, (C₁-C₈)-Alkyl, (C₃-C₈)-Cycloalkyl, -Oₜ(CH₂)_{d}CₑF_{2e+1,} F, Cl, Br, I
   oder CN;
   t Null oder 1;
   d Null, 1, 2, 3 oder 4;
   e 1, 2, 3, 4, 5, 6, 7 oder 8;
R(2), R(3), R(4) und R(5)
   unabhängig voneinander wie R(1) definiert;
   jedoch unter der Bedingung,
   daß mindestens einer der Substituenten R(1), R(2), R(3), R(4), R(5), R(A) und R(B) eine -Oₜ(CH₂)_{d}CₑF₂ₑ₊₁ oder eine Oᵣ(CH₂)ₐC_{b}F_{2b+1}-Gruppe ist,
   sowie deren pharmazeutisch verträgliche Salze;
   (HOE 94/F 094 - EP-OS 676 395, NZ 270 894)

x) Heteroaroylguanidine der Formel I 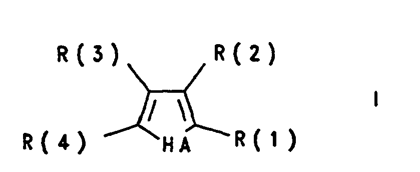 worin bedeuten:
HA SOₘ, O oder NR(5);
   m Null, 1 oder 2;
   R(5) Wasserstoff, (C₁-C₈)-Alkyl oder -CₐₘH₂ₐₘR(81);
   am Null, 1 oder 2;
   R(81) (C₃-C₈)-Cycloalkyl oder Phenyl,
   welches nicht substituiert oder substituiert ist mit 1 - 3 Substituenten ausgewählt aus der Gruppe bestehend aus F, Cl, CF₃, Methyl, Methoxy und NR(82)R(83); R(82) und R(83)
   H oder CH₃;
   oder
   R(81) (C₁-C₉)-Heteroaryl, das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   einer der beiden Substituenten R(1) und R(2)
   -CO-N=C(NH₂)₂;
   und der jeweils andere
   Wasserstoff, F, Cl, Br, I, (C₁-C₃)-Alkyl, -OR(6), CᵣF₂ᵣ₊₁, -CO-N=C(NH₂)_{2 oder}-NR(6)R(7);
   R(6) und R(7)
   unabhängig Wasserstoff oder (C₁-C₃)-Alkyl; r 1, 2, 3 oder 4;
R(3) und R(4)
   unabhängig voneinander Wasserstoff, F, Cl, Br, I, -C≡N, X-(CH₂)ₚ-(C_{q}-F_{2q+1}), R(8)-SO_{bm,} R(9)R(10)N-CO, R(11)-CO- oder R(12)R(13)N-SO₂-, wobei die Perfluoralkylgruppe geradkettig oder verzweigt ist,
   X Sauerstoff, S oder NR(14);
   R(14) H oder (C₁-C₃)-Alkyl;
   bm Null, 1 oder 2;
   p Null, 1 oder 2;
   q Null, 1, 2, 3, 4, 5 oder 6;
   R(8), R(9), R(11) und R(12)
   unabhängig (C₁-C₈)-Alkyl, (C₃-C₆)-Alkenyl, -CₙH₂ₙ-R(15), CF₃;
   n Null, 1, 2, 3 oder 4;
   R(15) (C₃-C₇)-Cycloalkyl oder Phenyl; welches nicht substituiert ist oder substituiert mit 1 - 3 Substituenten ausgewählt aus der Gruppe bestehend aus F, Cl, CF₃, Methyl, Methoxy oder NR(16)R(17); R(16) und R(17)
   H oder C₁-C₄-Alkyl;
   oder
   R(9), R(11) und R(12)
   H;
   R(10) und R(13)
   unabhängig H oder (C₁-C₄)-Alkyl;
   oder
   R(9) und R(10) sowie R(12) und R(13)
   gemeinsam 4 oder 5 Methylengruppen, von denen eine CH₂-Gruppe durch Sauerstoff, S, NH, N-CH₃ oder N-Benzyl ersetzt sein kann,
   oder
R(3) und R(4)
   unabhängig voneinander (C₁-C₈)-Alkyl oder -CₐₗH₂ₐₗR(18);
   al Null, 1 oder 2;
   R(18) (C₃-C₈)-Cycloalkyl oder Phenyl;
   welches nicht substituiert oder substituiert ist mit 1 - 3 Substituenten ausgewählt aus der Gruppe bestehend aus F, Cl, CF₃, Methyl, Methoxy und NR(19)R(20); R(19) und R(20)
   H oder CH₃;
   oder
R(3) und R(4)
   unabhängig voneinander (C₁-C₉)-Heteroaryl,
   das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   oder
R(3) und R(4)
   unabhängig voneinander 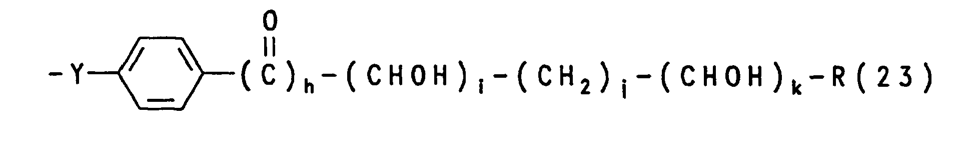 oder 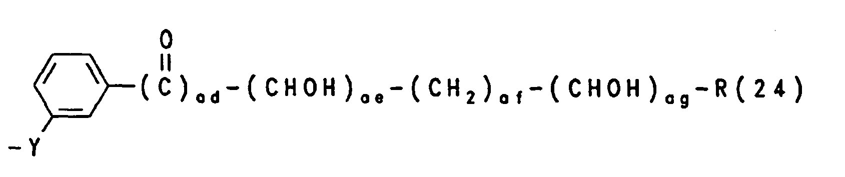 oder 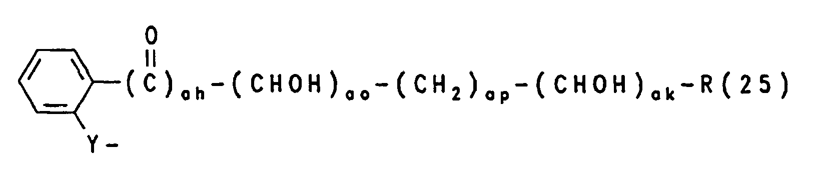 Y Sauerstoff, -S- oder -NR(22)-;
   h, ad, ah unabhängig Null oder 1;
   i, j, k, ae, af, ag, ao, ap und ak unabhängig Null, 1, 2, 3, 4,
   wobei jedoch jeweils
   h, i und k nicht gleichzeitig Null,
   ad, ae und ag nicht gleichzeitig Null sowie
   ah, ao und ak nicht gleichzeitig Null sind,
   R(23), R(24) R(25) und R(22)
   unabhängig Wasserstoff oder (C₁-C₃)-Alkyl;
   oder
R(3) und R(4)
   unabhängig voneinander Wasserstoff, F, Cl, Br, I, CN, (C₁-C₈)-Alkyl, (C₁-C₈)-Perfluoralkyl, (C₃-C₈)-Alkenyl oder -C_{g}H_{2g}R(26);
   g Null, 1, 2, 3 oder 4;
   R(26) (C₃-C₈)-Cycloalkyl, Phenyl, Biphenylyl oder Naphthyl,
   wobei die Aromaten nicht substituiert oder substituiert sind mit 1 - 3 Substituenten ausgewählt aus der Gruppe bestehend aus F, Cl, CF₃, Methyl, Methoxy und NR(27)R(28);
   R(27) und R(28)
   H, (C₁-C₄)-Alkyl oder (C₁-C₄)-Perfluoralkyl;
   oder
R(3) und R(4)
   unabhängig voneinander SR(29), -OR(30), -NR(31)R(32) oder
   -CR(33)R(34)R(35);
   R(29), R(30), R(31) und R(33)
   unabhängig -CₐH₂ₐ-(C₁-C₉)-Heteroaryl,
   das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   a Null, 1 oder 2;
   R(32), R(34) und R(35)
   unabhängig voneinander wie R(29) definiert oder Wasserstoff, (C₁-C₄)-Alkyl oder (C₁-C₄)-Perfluoralkyl;
   oder
   R(3) und R(4)
   unabhängig voneinander 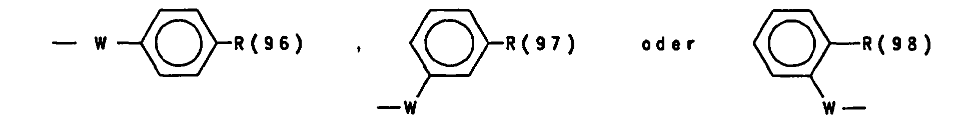 R(96), R(97) und R(98)
   unabhängig (C₁-C₉)-Heteroaryl,
   das über C oder N verknüpft ist und das unsubstituiert oder substituiert ist mit 1 bis 3 Substituenten ausgewählt aus der Gruppe bestehend aus F, Cl, CF₃, CH₃, Methoxy, Hydroxy, Amino, Methylamino, Dimethylamino oder Benzyl;
   W Sauerstoff, S oder NR(36)-;
   R(36) H oder (C₁-C₄)-Alkyl;
   oder
R(3) und R(4)
   unabhängig voneinander R(37)-SO_{cm} oder R(38)R(39)N-SO₂-;
   cm 1 oder 2;
   R(37) (C₁-C₈)-Alkyl, (C₁-C₈)-Perfluoralkyl, (C₃-C₈)-Alkenyl oder -CₛH₂ₛR(40);
   s Null, 1, 2, 3 oder 4;
   R(40) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(41)R(42);
   R(41) und R(42)
   H, (C₁-C₄)-Alkyl oder (C₁-C₄)-Perfluoralkyl;
   R(38) H, (C₁-C₈)-Alkyl, (C₁-C₈)-Perfluoralkyl, (C₃-C₈)-Alkenyl oder -C_{w}H_{2w}-R(43);
   w Null, 1, 2, 3 oder 4;
   R(43) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(44)R(45);
   R(44) und R(45)
   H, (C₁-C₄)-Alkyl oder (C₁-C₄)-Perfluoralkyl;
   R(39) H, (C₁-C₄)-Alkyl oder (C₁-C₄)-Perfluoralkyl;
   oder
   R(38) und R(39)
   gemeinsam 4 oder 5 Methylengruppen, von denen eine CH₂-Gruppe durch Sauerstoff, S, NH, N-CH₃ oder N-Benzyl ersetzt sein kann;
   oder
R(3) und R(4)
   unabhängig voneinander R(46)X(1)-;
   X(1) Sauerstoff, S, NR(47), (D=O)A-, NR(48)C=MN⁽*⁾R(49)-,
   M Sauerstoff oder S;
   A Sauerstoff oder NR(50);
   D C oder SO;
   R(46) (C₁-C₈)-Alkyl, (C₃-C₈)-Alkenyl, (CH₂)_{b}C_{d}F_{2d+1} oder -CₓH₂ₓ-R(51); b Null oder 1;
   d 1, 2, 3, 4, 5, 6 oder 7;
   x Null, 1, 2, 3 oder 4;
   R(51) (C₃-C₈)-Cycloalkyl, Phenyl, Biphenylyl oder Naphthyl,
   wobei die Aromaten nicht substituiert oder substituiert sind mit 1 - 3 Substituenten ausgewählt aus der Gruppe bestehend aus F, Cl, CF₃, Methyl, Methoxy und NR(52)R(53);
   R(52) und R(53)
   H, (C₁-C₄)-Alkyl oder (C₁-C₄)-Perfluoralkyl;
   R(47), R(48) und R(50)
   unabhängig Wasserstoff, (C₁-C₄)-Alkyl oder (C₁-C₄)-Perfluoralkyl;
   R(49) definiert wie R(46);
   oder
   R(46) und R(47) beziehungsweise R(46) und R(48)
   gemeinsam 4 oder 5 Methylengruppen, von denen eine CH₂-Gruppe durch Sauerstoff, S, NH, N-CH₃ oder N-Benzyl ersetzt sein kann,
   wobei A und N⁽*⁾ an den Phenylkern des Benzoylguanidin-Grundkörpers gebunden sind;
   oder
R(3) und R(4)
   unabhängig voneinander -SR(64), -OR(65), -NHR(66), -NR(67)R(68), -CHR(69)R(70), -C(OH)R(54)R(55), -C≡CR(56), -CR(58)=CHR(57), -[CR(59)R(60)]ᵤ-(CO)-[CR(61)R(62)]ᵥ-R(63); R(64), R(65), R(66), R(67) und R(69)
   gleich oder verschieden -(CH₂)_{y}-(CHOH)z-(CH₂)ₐₐ-(CH₂OH)ₜ-R(71) oder -(CH₂)_{ab}-O-(CH₂-CH₂O)_{ac}-R(72), R(71) und R(72)
   Wasserstoff oder Methyl;
   u 1, 2, 3 oder 4;
   v Null, 1, 2, 3 oder 4;
   y, z, aa
   gleich oder verschieden Null, 1, 2, 3 oder 4;
   t 1, 2, 3 oder 4;
   R(68), R(70), R(54) und R(55)
   gleich oder verschieden Wasserstoff, (C₁-C₆)-Alkyl;
   oder
   R(69) und R(70) beziehungsweise R(54) und R(55) zusammen mit dem sie tragenden Kohlenstoff-Atom ein (C₃-C₈)-Cycloalkyl;
   R(63)
   H, (C₁-C₆)-Alkyl, (C₃-C₈)-Cycloalkyl oder -CₑH₂ₑ-R(73);
   e Null, 1, 2, 3 oder 4;
   R(56), R(57) und R(73)
   unabhängig Phenyl,
   das unsubstituiert oder substituiert ist mit 1 - 3 Substituenten ausgewählt aus der Gruppe bestehend aus F, Cl, CF₃, Methyl, Methoxy und NR(74)R(75); R(74) und R(75)
   H oder (C₁-C₄)-Alkyl;
   oder
   R(56), R(57) und R(73)
   unabhängig (C₁-C₉)-Heteroaryl,
   das unsubstituiert oder wie Phenyl substituiert ist;
   R(58), R(59), R(60), R(61) und R(62)
   Wasserstoff oder Methyl,
   oder
R(3) und R(4)
   unabhängig voneinander R(76)-NH-SO₂-;
   R(76) R(77)R(78)N-(C=Y')-;
   Y' Sauerstoff, S oder N-R(79);
   R(77) und R(78)
   gleich oder verschieden H, (C₁-C₈)-Alkyl, (C₃-C₆)-Alkenyl, -C_{f}H_{2f}-R(80);
   f Null, 1, 2, 3 oder 4;
   R(80) (C₅-C₇)-Cycloalkyl oder Phenyl,
   welches unsubstituiert oder substituiert mit 1 - 3 Substituenten ausgewählt aus der Gruppe bestehend aus F, Cl, CF₃, Methoxy und (C₁-C₄)-Alkyl; oder
   R(77) und R(78)
   gemeinsam 4 oder 5 Methylengruppen, von denen eine CH₂-Gruppe durch Sauerstoff, S, NH, N-CH₃ oder N-Benzyl ersetzt sein kann, R(79)
   wie R(77) definiert oder gleich Amidin;
   oder
R(3) und R(4)
   unabhängig voneinander NR(84)R(85);
   R(84) und R(85)
   unabhängig voneinander H, (C₁-C₄)-Alkyl, oder gemeinsam 4 oder 5 Methylengruppen,
   von denen eine CH₂-Gruppe durch Sauerstoff, S, NH, N-CH₃ oder N-Benzyl ersetzt sein kann; oder von denen eine oder zwei CH₂-Gruppen durch CH-C_{dm}H_{2dm+1} ersetzt sein können,
   sowie deren pharmazeutisch verträgliche Salze;
   (HOE 94/F 123 - EP-OS 682 017, NZ 272 058)

y) Bizyklische Heteroaroylguanidine der Formel I 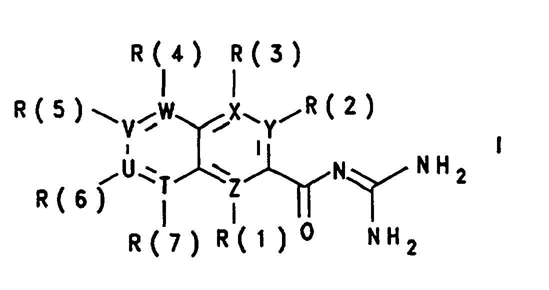 worin bedeuten:
T, U, V, W, X, Y und Z
   unabhängig voneinander Stickstoff oder Kohlenstoff;
   jedoch mit der Einschränkung,
   daß X und Z nicht gleichzeitig Stickstoff sind,
   und daß T, U, V, W, X, Y und Z keinen Substituenten tragen, wenn sie Stickstoff sind,
   und daß nicht mehr als vier von ihnen gleichzeitig Stickstoff sind, R(1) und R(2)
   unabhängig voneinander Wasserstoff, F, Cl, Br, I, (C₁-C₃)-Alkyl, (C₁-C₃)-Perfluoralkyl, OR(8), NR(8)R(9) oder C(=O)N=C(NH₂)₂;
   R(8) und R(9)
   unabhängig voneinander Wasserstoff oder (C₁-C₃)-Alkyl, oder
   R(8) und R(9)
   gemeinsam 4 oder 5 Methylengruppen, von denen eine CH₂-Gruppe
   durch Sauerstoff, S, NH, N-CH₃ oder N-Benzyl ersetzt sein kann; R(3), R(4), R(5), R(6) und R(7)
   unabhängig voneinander Wasserstoff, F, Cl, Br, I, -C≡N, Xₖ-(CH₂)ₚ-(C_{q}F_{2q+1}), R(10a)-SO_{bm}, R(10b)R(10c)N-CO, R(11)-CO- oder R(12)R(13)N-SO₂-,
   wobei die Perfluoralkylgruppe geradkettig oder verzweigt ist;
   X Sauerstoff, S oder NR(14);
   R(14) H oder C₁-C₃)-Alkyl;
   bm Null, 1 oder 2;
   p Null, 1 oder 2;
   k Null oder 1;
   q 1, 2, 3, 4, 5 oder 6;
   R(10a), R(10b), R(11) und R(12) unabhängig voneinander(C₁-C₈)-Alkyl, (C₃-C₆)-Alkenyl, -CₙH₂ₙ-R(15) oder (C₁-C₈)-Perfluoralkyl;
   n Null, 1, 2, 3 oder 4;
   R(15) (C₃-C₇)-Cycloalkyl oder Phenyl, welches nicht substituiert ist oder substituiert mit 1 - 3 Substituenten ausgewählt aus der Gruppe bestehend aus F, Cl, CF₃, Methyl, Methoxy und NR(16)R(17);
   R(16) und R(17)
   H oder C₁-C₄-Alkyl;
   oder
   R(10b), R(11) und R(12)
   Wasserstoff;
   R(10c) und R(13)
   unabhängig Wasserstoff oder (C₁-C₄)-Alkyl;
   oder
   R(10b) und R(10c) sowie R(12) und R(13)
   gemeinsam 4 oder 5 Methylengruppen, von denen eine CH₂-Gruppe durch Sauerstoff, Schwefel, NH, N-CH₃ oder N-Benzyl ersetzt sein kann;
   oder
R(3), R(4), R(5), R(6) und R(7)
   unabhängig voneinander (C₁-C₈)-Alkyl, -CₐₗH₂ₐₗR(18) oder (C₃-C₈)-Alkenyl;
   al Null, 1 oder 2;
   R(18) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(19a)R(19b);
   R(19a) und R(19b)
   Wasserstoff, (C₁-C₄)-Alkyl oder (C₁-C₄)-Perfluoralkyl;
   oder
R(3), R(4), R(5), R(6) und R(7)
   unabhängig voneinander (C₁-C₉)-Heteroaryl, das über C oder N verknüpft ist und das unsubstituiert oder substituiert ist mit 1 - 3 Substituenten aus der Gruppe bestehend aus F, Cl, CF₃, CH₃, Methoxy, Hydroxy, Amino, Methylamino oder Dimethylamino;
   oder
R(3), R(4), R(5), R(6) und R(7)
   unabhängig voneinander 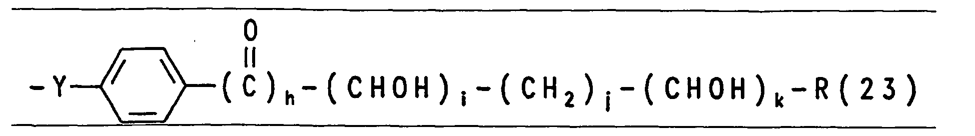 oder 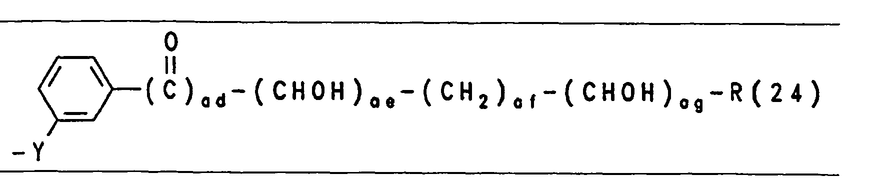 oder 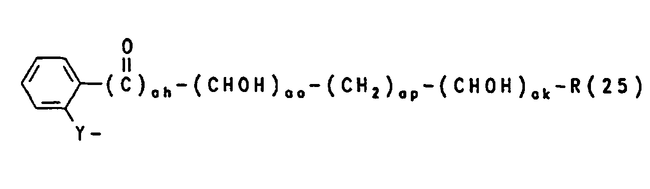 Y Sauerstoff, -S- oder -NR(22)-;
   h, ad, ah
   unabhängig voneinander Null oder 1;
   i, j, k, ae, af, ag, ao, ap und ak
   unabhängig voneinander Null, 1, 2, 3 oder 4;
   wobei jedoch jeweils
   h, i und k nicht gleichzeitig Null,
   ad, ae und ag nicht gleichzeitig Null sowie
   ah, ao und ak nicht gleichzeitig Null sind,
   R(23), R(24) R(25) und R(22)
   unabhängig voneinander Wasserstoff oder (C₁-C₃)-Alkyl;
   oder
R(3), R(4), R(5), R(6) und R(7) unabhängig voneinander SR(29), -OR(30), -NR(31)R(32) oder -CR(33)R(34)R(35);
   R(29), R(30), R(31) und R(33)
   unabhängig voneinander -CₐH₂ₐ-(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   a Null, 1 oder 2;
   R(32), R(34) und R(35)
   unabhängig voneinander wie R(29) definiert oder Wasserstoff, (C₁-C₄)-Alkyl oder (C₁-C₄)-Perfluoralkyl;
   oder
R(3), R(4), R(5), R(6) und R(7) unabhängig voneinander 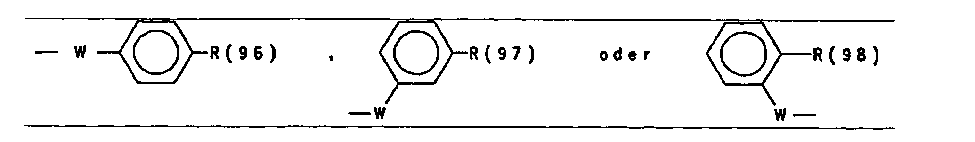 R(96), R(97) und R(98)
   unabhängig voneinander (C₁-C₉)-Heteroaryl, das über C oder N verknüpft ist und das unsubstituiert oder substituiert ist mit 1 bis 3 Substituenten aus der Gruppe bestehend aus F, Cl, CF₃, CH₃, Methoxy, Hydroxy, Amino, Methylamino, Dimethylamino oder Benzyl;
   W Sauerstoff, S oder NR(36)-;
   R(36) H oder (C₁-C₄)-Alkyl;
   oder
R(3), R(4), R(5), R(6) und R(7)
   unabhängig voneinander R(46)X(1)-;
   X(1) Sauerstoff, S, NR(47), (D=O)A- oder NR(48)C=MN⁽*⁾R(49)-;
   M Sauerstoff oder Schwefel;
   A Sauerstoff oder NR(50); D C oder SO;

   R(46) (C₁-C₈)-Alkyl, (C₃-C₈)-Alkenyl, (CH₂)_{b}C_{d}F_{2d+1} oder -CₓH₂ₓ-R(51);
   b Null oder 1;
   d 1, 2, 3, 4, 5, 6 oder 7;
   x Null, 1, 2, 3 oder 4; R(51) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(52)R(53); R(52) und R(53)
   Wasserstoff, (C₁-C₄)-Alkyl oder (C₁-C₄)-Perfluoralkyl;
   R(47), R(48) und R(50) unabhängig
   Wasserstoff, (C₁-C₄)-Alkyl oder (C₁-C₄)-Perfluoralkyl;
   R(49) definiert wie R(46);
   oder
   R(46) und R(47) beziehungsweise R(46) und R(48)
   gemeinsam 4 oder 5 Methylengruppen, von denen eine CH₂-Gruppe durch Sauerstoff, Schwefel, NH, N-CH₃ oder N-Benzyl ersetzt sein kann;
   wobei A und N⁽*⁾ an den Phenylkern des Heteroaroylguanidin-Grundkörpers gebunden sind;
   oder
R(3), R(4), R(5), R(6) und R(7)
   unabhängig voneinander -SR(64), -OR(65), -NHR(66), -NR(67)R(68), -CHR(69)R(70) oder -CR(54)R(55)OH, -C≡CR(56), -CR(58)=CR(57) oder -[CR(59)R(60)]ᵤ-CO-[CR(61)R(62)]ᵥ-R(63); R(64), R(65), R(66), R(67) und R(69)
   gleich oder verschieden
   -(CH₂)_{y}-(CHOH)_{z}-(CH₂)ₐₐ-(CHOH)ₜ-R(71) oder
   -(CH₂)_{ab}-O-(CH₂-CH₂O)_{ac}-R(72);
   R(71), und R(72)
   unabhängig voneinander Wasserstoff oder Methyl;
   u 1, 2, 3 oder 4;
   v Null, 1, 2, 3 oder 4;
   y, z, aa gleich oder verschieden
   Null, 1, 2, 3 oder 4;
   t 1, 2, 3 oder 4;
   R(68), R(70), R(54) und R(55)
   gleich oder verschieden Wasserstoff oder (C₁-C₆)-Alkyl;
   oder
   R(69) und R(70) beziehungsweise R(54) und R(55)
   zusammen mit dem sie tragenden Kohlenstoff-Atom ein (C₃-C₈)-Cycloalkyl;
   R(63)
   Wasserstoff, (C₁-C₆)-Alkyl, (C₃-C₈)-Cycloalkyl oder -CₑH₂ₑ-R(73);
   e Null, 1, 2, 3 oder 4;
   R(56), R(57) und R(73) unabhängig
   Phenyl, das unsubstituiert oder substituiert ist mit 1 - 3 Substituenten ausgewählt aus der Gruppe bestehend aus F, Cl, CF₃, Methyl, Methoxy und NR(74)R(75); R(74) und R(75)
   Wasserstoff oder (C₁-C₄)-Alkyl;
   oder
   R(56), R(57) und R(73) unabhängig
   (C₁-C₉)-Heteroaryl, das unsubstituiert oder wie Phenyl substituiert ist;
   R(58), R(59), R(60), R(61) und R(62)
   Wasserstoff oder Methyl;
   oder
R(3), R(4), R(5), R(6) und R(7)
   unabhängig voneinander R(76)-NH-SO₂-; R(76) R(77)R(78)N-(C=Y')-;
   Y' Sauerstoff, S oder N-R(79);
   R(77) und R(78)
   gleich oder verschieden Wasserstoff, (C₁-C₈)-Alkyl, (C₃-C₆) Alkenyl oder -C_{f}H_{2f}-R(80);
   f Null, 1, 2, 3 oder 4;
   R(80) (C₅-C₇)-Cycloalkyl oder Phenyl, welches unsubstituiert mit 1 - 3 Substituenten ausgewählt aus der Gruppe bestehend aus F, Cl, CF₃, Methoxy und (C₁-C₄)-Alkyl;
   oder
   R(77) und R(78)
   gemeinsam 4 oder 5 Methylengruppen, von denen eine CH₂-Gruppe durch Sauerstoff, Schwefel, NH, N-CH₃ oder N-Benzyl ersetzt sein kann;
   R(79) wie R(77) definiert oder gleich Amidin;
   oder
R(3), R(4), R(5), R(6) und R(7) unabhängig voneinander NR(84a)R(85), OR(84b), SR(84c) oder -CₙH₂ₙ-R(84d);
   n Null, 1, 2, 3 oder 4;
   R(84d) (C₃-C₇)-Cycloalkyl oder Phenyl, welches nicht substituiert ist oder substituiert mit 1 - 3 Substituenten aus der Gruppe bestehend aus F, Cl, CF₃, Methyl, Methoxy und NR(16)R(17);
   R(16) und R(17)
   Wasserstoff, oder C₁-C₄-Alkyl;
   R(84a), R(84b), R(84c) und R(85)
   unabhängig voneinander Wasserstoff, (C₁-C₈)-Alkyl, (C₁-C₈)-Perfluoralkyl oder (CH₂)ₐₓ-R(84g);
   ax Null, 1, 2, 3 oder 4;
   R(84g) (C₃-C₇)-Cycloalkyl oder Phenyl, welches nicht substituiert ist oder substituiert mit 1 - 3 Substituenten ausgewählt aus der Gruppe bestehend aus F, Cl, CF₃, Methyl, Methoxy und NR(84u)R(84v);
   R(84u) und R(84v)
   Wasserstoff oder C₁-C₄-Alkyl;
   oder
   R(84a) und R(85) gemeinsam 4 oder 5 Methylengruppen, von denen eine CH₂-Gruppe durch Sauerstoff, Schwefel, NH, N-CH₃ oder N-Benzyl ersetzt sein
   kann,
   sowie deren pharmazeutisch verträgliche Salze;
   (HOE 94/F 134 - EP-OS 686 627, NZ 272 103)

z) Benzoylguanidine der Formel I 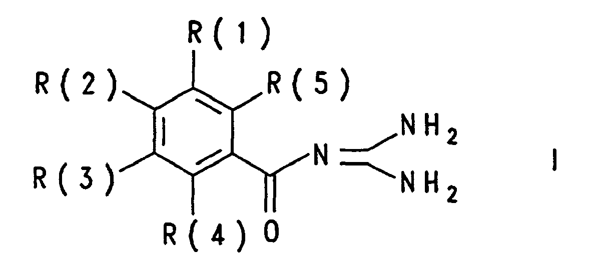 worin bedeuten:
R(1) R(6)-SOₘ;
   m Null, 1 oder 2;
   R(6) Perfluoralkyl mit 1, 2, 3, 4, 5 oder 6 C-Atomen, das geradkettig oder verzweigt ist;
R(2) und R(3)
   unabhängig voneinander Wasserstoff, F, Cl, Br, I, Alkyl mit 1, 2, 3 oder 4 C-Atomen, Alkoxy mit 1, 2, 3 oder 4 C-Atomen oder Phenoxy,
   das unsubstituiert ist oder substituiert ist mit 1 - 3 Substituenten ausgewählt aus der Gruppe bestehend aus F, Cl, Methyl und Methoxy;
   oder
R(2) und R(3)
   unabhängig voneinander Pyrrol-1-yl, Pyrrol-2-yl- oder Pyrrol-3-yl, welches nicht substituiert ist oder substituiert mit 1 bis 4 Substituenten, ausgewählt aus der Gruppe bestehend aus F, Cl, Br, I, CN, Alkanoyl mit 2, 3, 4, 5, 6, 7 oder 8 C-Atomen, Alkoxycarbonyl mit 2, 3, 4, 5, 6, 7 oder 8C-Atomen, Formyl, Carboxy, CF₃, Methyl und Methoxy;
R(4) und R(5)
   unabhängig voneinander Wasserstoff, Alkyl mit 1, 2 oder 3-C-Atomen, F, Cl, Br, I, CN, OR(7), NR(8)R(9) oder -(CH₂)ₙ-(CF₂)ₒ-CF₃;
   R(7), R(8) und R(9)
   unabhängig voneinander Wasserstoff oder Alkyl mit 1, 2, 3 oder 4 C-Atomen;
   n Null oder 1;
   o Null, 1 oder 2;
   sowie deren pharmakologisch akzeptable Salze;
   (HOE 94/F 168 - EP-OS 690 048, NZ 272 373)

ab) Perfluoralkylgruppen tragende phenylsubstituierte Alkenylcarbonsäure-guanidide der Formel I 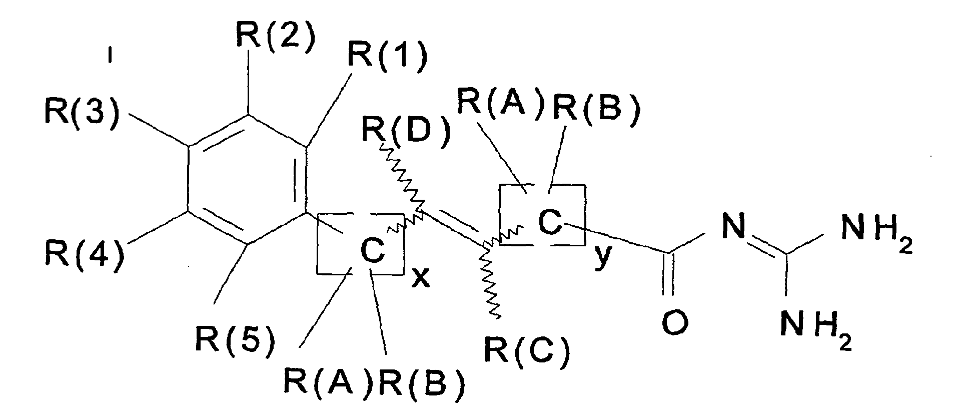 worin bedeuten:
R(A) Wasserstoff, F, Cl, Br, I, CN, OH, OR(6), (C₁-C₈)-Alkyl, Oᵣ(CH₂)ₐC_{b}F_{2b+1}, (C₃-C₈)-Cycloalkyl oder NR(7)R(8);
   r Null oder 1;
   a Null, 1, 2, 3 oder 4;
   b 1, 2, 3, 4, 5, 6, 7 oder 8;
   R(6) (C₁-C₈)-Alkyl, (C₁-C₄)-Perfluoralkyl, (C₃-C₈)-Alkenyl, (C₃-C₈)-Cycloalkyl, Phenyl oder Benzyl; wobei die Aromaten nicht substituiert oder substituiert sind mit 1 - 3 Substituenten aus der Gruppe bestehend aus F, Cl, CF₃, Methyl, Methoxy und NR(9)R(10);
   R(9) und R(10)
   H, (C₁-C₄)-Alkyl oder (C₁-C₄)-Perfluoralkyl;
   R(7) und R(8)
   unabhängig voneinander wie R(6) definiert;
   oder
   R(7) und R(8)
   gemeinsam 4 oder 5 Methylengruppen, von denen eine CH₂-Gruppe durch Sauerstoff, Schwefel, NH, N-CH₃ oder N-Benzyl ersetzt sein
   kann;
R(B) unabhängig wie R(A) definiert;
X Null, 1 oder 2;
Y Null, 1 oder 2;
R(C) Wasserstoff, F, Cl, Br, I, CN, OR(12), (C₁-C₈)-Alkyl, Oₚ(CH₂)_{f}C_{g}F_{2g+1} oder (C₃-C₈)-Cycloalkyl;
   p Null oder 1;
   f Null, 1, 2, 3 oder 4;
   g 1, 2, 3, 4, 5, 6, 7 oder 8;
   R(12)
   (C₁-C₈)-Alkyl, (C₁-C₄)-Perfluoralkyl, (C₃-C₈)-Alkenyl, (C₃-C₈)-Cycloalkyl, Phenyl oder Benzyl;
   wobei die Aromaten Phenyl oder Benzyl nicht substituiert oder substituiert sind mit 1 - 3 Substituenten aus der Gruppe bestehend aus F, Cl, CF₃, Methyl, Methoxy und NR(13)R(14);
   R(13) und R(14)
   unabhängig voneinander H, (C₁-C₄)-Alkyl oder (C₁-C₄)-Perfluoralkyl;
R(D) unabhängig wie R(C) definiert,
R(1) Wasserstoff, (C₁-C₈)-Alkyl, -Oₜ(CH₂)_{d}CₑF₂ₑ₊₁, (C₃-C₈)-Cycloalkyl, F, Cl, Br, I
   oder CN;
   t Null oder 1;
   d Null, 1, 2, 3 oder 4;
   e 1, 2, 3, 4, 5, 6, 7 oder 8;
R(2), R(3), R(4) und R(5)
   unabhängig voneinander wie R(1) definiert;
   jedoch unter der Bedingung,
   daß mindestens einer der Substituenten R(A), R(B), R(C), R(D), R(1), R(2), R(4)
   oder R(5) eine Oᵣ(CH₂)ₐC_{b}F_{2b+1}-, Oₚ(CH₂)_{f}C_{g}F_{2g+1}- oder Oₜ(CH₂)_{d}CₑF₂ₑ₊₁-gruppe und R(3) keine Oₜ(CH₂)dCₑF₂ₑ₊₁-gruppe ist;
   sowie deren pharmazeutisch verträgliche Salze;
   (HOE 94/F 182 - EP-OS 690 048, NZ 272 449)

ac) Ortho-amino-substituierte Benzoylguanidine der Formel I
worin bedeuten: 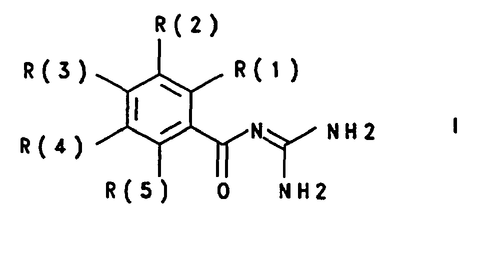
R(1) NR(50)R(6),
   R(50) und R(6)
   unabhängig voneinander Wasserstoff, (C₁-C₈)-Alkyl oder (C₁-C₈)-Perfluoralkyl;
R(2), R(3), R(4) und R(5)
   unabhängig voneinander R(10)-SOₐ-, R(11)R(12)N-CO-, R(13)-CO- oder R(14)R(15)N-SO₂-;
   a Null, 1 oder 2,
   R(10), R(11), R(12), R(13), R(14) und R(15) unabhängig voneinander (C₁-C₈)-Alkyl, (C₁-C₈)-Perfluoralkyl, (C₃-C₆)-Alkenyl oder -C_{ab}H_{2ab}-R(16);
   ab Null, 1, 2, 3 oder 4;
   R(16) (C₃-C₇)-Cycloalkyl, Phenyl,
   welches nicht substituiert ist oder substituiert mit 1 - 3 Substituenten ausgewählt aus der Gruppe bestehend aus F, Cl, CF₃, Methyl, Methoxy oder NR(17)R(18);
   R(17) und R(18)
   unabhängig voneinander H, CF₃ oder (C₁-C₄)-Alkyl;
   oder
   R(11), R(12), sowie R(14) und R(15)
   gemeinsam 4 oder 5 Methylengruppen, von denen eine CH₂-Gruppe durch Sauerstoff, S, NH, N-CH₃ oder N-Benzyl ersetzt sein kann;
   oder
   R(11), R(12), R(14) und R(15)
   unabhängig voneinander Wasserstoff;
   oder
R(2), R(3), R(4) und R(5)
   unabhängig voneinander SR(21), -OR(22), -NR(23)R(24) oder -CR(25)R(26)R(27);
   R(21), R(22), R(23) und R(25)
   unabhängig voneinander -C_{b}H_{2b}-(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   b Null, 1 oder 2;
   R(24), R(26) und R(27)
   unabhängig voneinander Wasserstoff, (C₁-C₄)-Alkyl oder (C₁-C₄)-Perfluoralkyl;
   oder
R(2), R(3), R(4) und R(5) unabhängig voneinander Wasserstoff, F, Cl, Br, I, CN, -(Xa)_{dg}-C_{da}H_{2da+1}, - (Xb)_{dh}-(CH₂)_{db}-C_{d},F_{2de+1}, (C₃-C₈)-Alkenyl oder -C_{df}H_{2df}R(30);
   (Xa) O, S oder NR(33);
   R(33)
   H, (C₁-C₄)-Alkyl oder (C₁-C₄)-Perfluoralkyl;
   dg Null oder 1;
   (Xb) O, S oder NR(34);
   R(34)
   H, (C₁-C₄)-Alkyl oder (C₁-C₄)-Perfluoralkyl;
   dh Null oder 1;
   da Null, 1, 2, 3, 4, 5, 6, 7, 8;
   db Null, 1, 2, 3, 4;
   de Null, 1, 2, 3, 4, 5, 6, 7;
   df Null, 1, 2, 3, 4;
   R(30)
   (C₃-C₈)-Cycloalkyl, Phenyl, Biphenylyl oder Naphthyl, wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(31)R(32);
   R(31) und R(32)
   H, (C₁-C₄)-Alkyl oder (C₁-C₄)-Perfluoralkyl;
   oder
R(2), R(3), R(4) und R(5)
   unabhängig voneinander NR(40)R(41) oder -(Xe)-(CH₂)_{eb}R(45); R(40) und R(41)
   unabhängig voneinander Wasserstoff, (C₁-C₈)-Alkyl, (C₁-C₈)-Perfluoralkyl oder (CH₂)ₑ-R(42);
   e Null, 1, 2, 3 oder 4;
   R(42)
   (C₃-C₇)-Cycloalkyl, Phenyl, welches nicht substituiert ist oder substituiert mit 1 - 3 Substituenten ausgewählt aus der Gruppe bestehend aus F, Cl, CF₃, Methyl, Methoxy und NR(43)R(44); R(43) und R(44)
   unabhängig voneinander H, CF₃ oder (C₁-C₄)-Alkyl;
   oder
R(40) und R(41)
   gemeinsam 4 oder 5 Methylengruppen, von denen eine CH₂-Gruppe durch Sauerstoff, Schwefel, NH, N-CH₃ oder N-Benzyl ersetzt sein kann;
   (Xe) O, S oder NR(47);
   R(47)
   H, (C₁-C₄)-Alkyl oder (C₁-C₄)-Perfluoralkyl;
   eb Null, 1, 2, 3 oder 4;
R(45) (C₃-C₇)-Cycloalkyl, Phenyl, welches nicht substituiert ist oder substituiert mit 1 - 3 Substituenten ausgewählt aus der Gruppe bestehend aus F, Cl, CF₃, Methyl, Methoxy, NR(50)R(51) und -(Xfa)-(CH₂)_{ed}-(Xfb)R(46);
   Xfa CH₂, O, S oder NR(48);
   Xfb O, S oder NR(49);
   ed 1, 2, 3 oder 4;
   R(46)
   H, (C₁-C₄)-Alkyl oder (C₁-C₄)-Perfluoralkyl;
   R(48), R(49), R(50) und R(51)
   unabhängig voneinander H oder (C₁-C₄)-Alkyl oder (C₁-C₄)-Perfluoralkyl;
   wobei R(3) und R(4) jedoch nicht Wasserstoff sein können,
   sowie deren pharmazeutisch verträgliche Salze;
   HOE 94/F 265 - NZ 272 946, EP-OS 700 904)

ad) Benzoylguanidine der Formel I 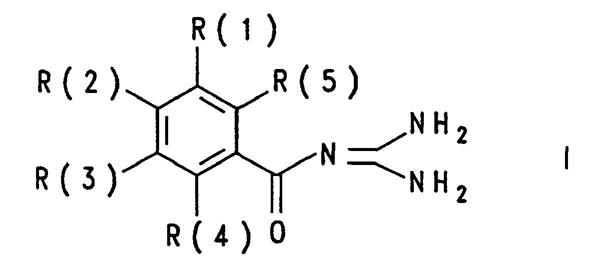 worin bedeuten:
einer der drei Substituenten R(1), R(2) und R(3)
   (C₁-C₉)-Heteroaryl-N-Oxid,
   das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und
   Dimethylamino;
   oder
   einer der drei Substituenten R(1), R(2) und R(3)
   -SR(10), -OR(10), -NR(10)R(11) oder -CR(10)R(11)R(12);
   R(10)
   -CₐH₂ₐ-(C₁-C₉)-Heteroaryl-N-Oxid,
   das unsubstituiert oder substituiert ist mit 1 - 3 Substituenten ausgewählt aus der Gruppe bestehend F, Cl, CF₃, CH₃, Methoxy, Hydroxy, Amino, Methylamino und Dimethylamino;
   a Null, 1 oder 2;
   R(11) und R(12)
   unabhängig voneinander wie R(10) definiert, Wasserstoff oder (C₁-C₄)-Alkyl;
   und die jeweils anderen Substituenten R(1), R(2) und R(3)
   unabhängig voneinander (C₁-C₈)-Alkyl, (C₂-C₈)-Alkenyl oder -CₘH₂ₘR(14);
   m Null, 1 oder 2;
   R(14) (C₃-C₈)-Cycloalkyl oder Phenyl, welches nicht substituiert oder substituiert ist mit 1 - 3 Substituenten ausgewählt aus der Gruppe bestehend aus F, Cl, CF₃, Methyl, Methoxy und NR(15)R(16), R(15) und R(16)
   Wasserstoff oder CH₃;
   oder
   die jeweils anderen Substituenten R(1), R(2) und R(3)
   unabhängig voneinander Wasserstoff, F, Cl, Br, I, -C=N, X-(CH₂)ₚ-(C_{q}F_{2q+1}), R(22)-SOᵤ, R(23)R(24)N-CO, R(25)-CO- oder R(26)R(27)N-SO₂-,
   wobei die Perfluoralkylgruppe geradkettig oder verzweigt ist;
   X eine Bindung, Sauerstoff, S oder NR(28);
   u Null, 1 oder 2;
   p Null, 1 oder 2;
   q Null, 1, 2, 3, 4, 5 oder 6;
   R(22), R(23), R(25) und R(26)
   unabhängig (C₁-C₈)-Alkyl, (C₂-C₆)-Alkenyl, -CₙH₂ₙ-R(29) oder CF₃;
   n Null, 1, 2, 3 oder 4;
   R(28) Wasserstoff oder (C₁-C₃)-Alkyl;
   R(29) (C₃-C₇)-Cycloalkyl oder Phenyl;
   welches nicht substituiert ist oder substituiert mit 1 - 3 Substituenten ausgewählt aus der Gruppe bestehend aus F, Cl, CF₃, Methyl, Methoxy und NR(30)R(31);
   R(30) und R(31)
   Wasserstoff oder C₁-C₄-Alkyl,
   oder
   R(23), R(25) und R(26)
   auch Wasserstoff;
   R(24) und R(27)
   unabhängig voneinander Wasserstoff oder (C₁-C₄)-Alkyl;
   oder R(23) und R(24) sowie R(26) und R(27) gemeinsam 4 oder 5 Methylengruppen, von denen eine CH₂-Gruppe durch Sauerstoff, S, NH, N-CH₃ oder N-Benzyl ersetzt sein kann;
   oder
   die jeweils anderen Substituenten R(1), R(2) und R(3)
   unabhängig voneinander OR(35) oder NR(35)R(36);
   R(35) und R(36)
   unabhängig voneinander Wasserstoff oder (C₁-C₆)-Alkyl;
   oder
   R(35) und R(36)
   gemeinsam 4 - 7 Methylengruppen, von denen eine CH₂-Gruppe durch
   Sauerstoff, S, NH, N-CH₃ oder N-Benzyl ersetzt sein kann, R(4) und R(5)
   unabhängig voneinander Wasserstoff, (C₁-C₄)-Alkyl, F, Cl, -OR(32), - NR(33)R(34) oder CᵣF₂ᵣ₊₁;
   R(32), R(33) und R(34)
   unabhängig voneinander Wasserstoff oder (C₁-C₃)-Alkyl;
   r 1, 2, 3 oder 4;
   sowie deren pharmazeutisch verträgliche Salze;
   (HOE 94/F 266 - EP-OS 702 001, NZ 272 948)

ad) Benzoylguanidine der Formel I 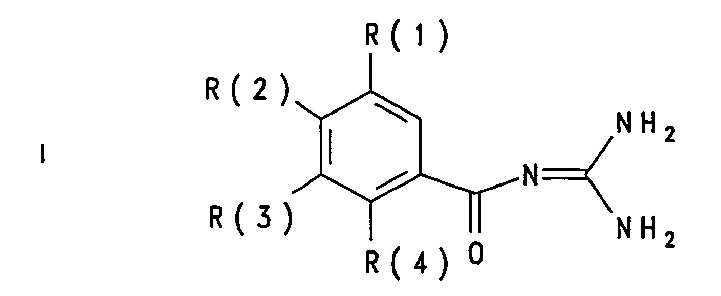 worin bedeuten:
R(1) Wasserstoff, F, Cl, Br, I, CN, NO₂, OH, (C₁-C₈)-Alkyl, (C₃-C₈)-Cycloalkyl, Oₐ-(CH₂)_{b}-(CF₂)_{c}-CF₃;
   a Null oder 1;
   b Null, 1 oder 2;
   c Null, 1, 2 oder 3;
   oder
R(1) R(5)-SOₘ oder R(6)R(7)N-SO₂-;
   m Null, 1 oder 2;
   R(5) und R(6) unabhängig voneinander
   (C₁-C₈)-Alkyl, (C₃-C₆)-Alkenyl, CF₃ oder -CₙH₂ₙ-R(8);
   n Null, 1, 2, 3 oder 4;
   R(7) Wasserstoff oder (C₁-C₄)-Alkyl;
   R(8) (C₃-C₇)-Cycloalkyl oder Phenyl,
   welches nicht substituiert ist oder substituiert mit 1 - 3 Substituenten ausgewählt aus der Gruppe bestehend aus F, Cl, CF₃, Methyl, Methoxy und NR(9)R(10); R(9) und R(10) unabhängig voneinander Wasserstoff oder (C₁-C₄)-Alkyl;
   oder
   R(6) H;
   oder R(6) und R(7)
   gemeinsam 4 oder 5 Methylengruppen, von denen eine CH₂-Gruppe durch Sauerstoff, S, NH, N-CH₃ oder N-Benzyl ersetzt sein kann,
   oder
R(1) -SR(11), -OR(11) oder -CR(11)R(12)R(13);
   R(11) -CₚH₂ₚ-(C₃-C₈)-Cycloalkyl, -(C₁-C₉)-Heteroaryl oder Phenyl,
   wobei die aromatischen Systeme unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino; R(12), R(13) unabhängig voneinander
   wie R(11) definiert oder Wasserstoff oder (C₁-C₄)-Alkyl;
   p Null, 1 oder 2;
   oder
R(1) Phenyl, Naphthyl, Biphenylyl oder (C₁-C₉)-Heteroaryl, letzteres über C oder N verknüpft,
   die unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
R(2) -CF₂R(14), -CF[R(15)][R(16)], -CF[(CF₂)_{q}-CF₃)][R(15)],
   -C[(CF₂)ᵣ-CF₃]=CR(15)R(16);
   R(14) (C₁-C₄)-Alkyl oder (C₃-C₆)-Cycloalkyl;
   R(15) und R(16) unabhängig voneinander
   Wasserstoff oder (C₁-C₄)-Alkyl;
   q Null, 1 oder 2;
   r Null, 1 oder 2;
R(3) wie R(1) definiert;
R(4) Wasserstoff, (C₁-C₃)-Alkyl, F, Cl, Br, I, CN, -(CH₂)ₛ-(CF₂)ₜ-CF₃;
   s Null oder 1;
   t Null, 1 oder 2;
   sowie deren pharmazeutisch verträgliche Salze;
   (HOE 94/F 267 - EP-OS 700 899, NZ 272 947)

ae) Benzoylguanidine der Formel I 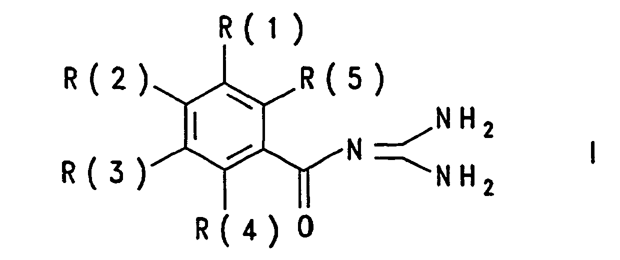 worin bedeuten:
einer der drei Substituenten R(1), R(2) und R(3)
-Y-4-[(CH₂)ₖ-CHR(7)-(C=O)R(8)]-Phenyl,
-Y-3(CH₂)ₖ-CHR(7)-(C=O)R(8)]-Phenyl oder -Y-2-[(CH₂)ₖ-CHR(7)-(C=O)R(8)]-Phenyl,
   wobei das Phenyl jeweils unsubstituiert oder substituiert ist mit 1 - 2 Substituenten aus der Gruppe F, Cl, -CF₃, Methyl, Hydroxy, Methoxy, oder -NR(37)R(38);
   R(37) und R(38)
   unabhängig voneinander Wasserstoff oder -CH₃;
Y eine Bindung, Sauerstoff, -S- oder -NR(9);
   R(9) Wasserstoff oder -(C₁-C₄)-Alkyl;
R(7) -OR(10) oder -NR(10)R(11);
   R(10) und R(11)
   unabhängig voneinander Wasserstoff, -(C₁-C₈)-Alkyl, -(C₁-C₈)-Alkanoyl, -(C₁-C₈)-Alkoxycarbonyl, Benzyl, Benzyloxycarbonyl;
   oder
   R(10) Trityl;
R(8) -OR(12) oder -NR(12)R(13);
   R(12) und R(13)
   unabhängig voneinander Wasserstoff, -(C₁-C₈)-Alkyl oder Benzyl;
   k Null, 1, 2, 3 oder 4;
und die jeweils anderen Reste R(1), R(2) und R(3)
   unabhängig voneinander -(C₁-C₈)-Alkyl, -(C₂-C₈)-Alkenyl oder -(CH₂)ₘR(14);
m Null, 1 oder 2;
R(14) -(C₃-C₈)-Cycloalkyl oder Phenyl,
   welches nicht substituiert oder substituiert ist mit 1 - 3 Substituenten ausgewählt aus der Gruppe bestehend aus F, Cl, -CF₃, Methyl, Methoxy und -NR(15)R(16);
   R(15) und R(16)
   Wasserstoff oder -CH₃;
   oder
   die jeweils anderen Reste R(1), R(2) und R(3)
   unabhängig voneinander R(18)R(19)N-(C=Y')-NH-SO₂-;
   Y' Sauerstoff, -S- oder -N-R(20);
R(18) und R(19)
   unabhängig voneinander Wasserstoff, -(C₁-C₈)-Alkyl, -(C₃-C₆)-Alkenyl oder -(CH₂)ₜ-R(21);
   t Null, 1, 2, 3 oder 4;
   R(21) -(C₅-C₇)-Cycloalkyl oder Phenyl,
   welches unsubstituiert ist oder substituiert ist mit 1 - 3 Substituenten ausgewählt aus der Gruppe bestehend aus F, Cl, -CF₃, Methoxy und -(C₁-C₄)-Alkyl;
   oder
R(18) und R(19)
   gemeinsam 4 oder 5 Methylengruppen, von denen eine CH₂-Gruppe durch Sauerstoff, -S-, -NH-, -N-CH₃ oder -N-Benzyl ersetzt sein kann; R(20)
   wie R(18) definiert oder Amidin;
   oder
   die jeweils anderen Reste R(1), R(2) und R(3)
   unabhängig voneinander Wasserstoff, F, Cl, Br, I, -C≡N, X-(CH₂)p-(C_{q}F_{2q+1}), R(22)-SOᵤ-, R(23)R(24)N-CO-, R(25)-CO- oder R(26)R(27)N-SO₂-, wobei die Perfluoralkylgruppe geradkettig oder verzweigt ist;
   X eine Bindung, Sauerstoff, -S- oder -NR(28);
   u Null, 1 oder 2;
   p Null, 1 oder 2;
   q 1, 2, 3, 4, 5 oder 6;
   R(22), R(23), R(25) und R(26)
   unabhängig voneinander -(C₁-C₈)-Alkyl, -(C₃-C₆)-Alkenyl, -(CH₂)ₙ-R(29) oder -CF₃;
   n Null, 1, 2, 3 oder 4;
   R(28) Wasserstoff oder -(C₁-C₃)-Alkyl;
   R(29) -(C₃-C₇)-Cycloalkyl oder Phenyl,
   welches nicht substituiert ist oder substituiert mit 1 - 3 Substituenten ausgewählt aus der Gruppe bestehend aus F, Cl, -CF₃, Methyl, Methoxy und -NR(30)R(31);
   R(30) und R(31)
   Wasserstoff oder -(C₁-C₄)-Alkyl;
   oder
   R(23), R(25) und R(26)
   Wasserstoff;
   R(24) und R(27)
   unabhängig voneinander Wasserstoff oder -(C₁-C₄)-Alkyl;
   oder
   R(23) und R(24) sowie R(26) und R(27)
   gemeinsam 4 oder 5 Methylengruppen, von denen eine CH₂-Gruppe durch Sauerstoff, -S-, -NH-, - N-CH₃ oder -N-Benzyl ersetzt sein kann;
   oder
   die jeweils anderen Reste R(1), R(2) und R(3)
   unabhängig voneinander -OR(35) oder -NR(35)R(36);
   R(35) und R(36)
   unabhängig voneinander Wasserstoff oder -(C₁-C₆)-Alkyl;
   oder
   R(35) und R(36)
   gemeinsam 4 - 7 Methylengruppen, von denen eine CH₂-Gruppe durch
   Sauerstoff, -S-, -NH-, -N-CH₃ oder -N-Benzyl ersetzt sein kann; R(4) und R(5)
   unabhängig voneinander Wasserstoff, -(C₁-C₄)-Alkyl, F, Cl, -OR(32), - NR(33)R(34) oder -CᵣF₂ᵣ₊₁,
   R(32), R(33) und R(34)
   unabhängig voneinander Wasserstoff oder -(C₁-C₃)-Alkyl;
   r 1, 2, 3 oder 4;
   sowie deren pharmazeutisch verträgliche Salze;
   (HOE 94/F 352 - EP-OS 713 684, NZ 280 517)

af) Benzoylguanidine der Formel I 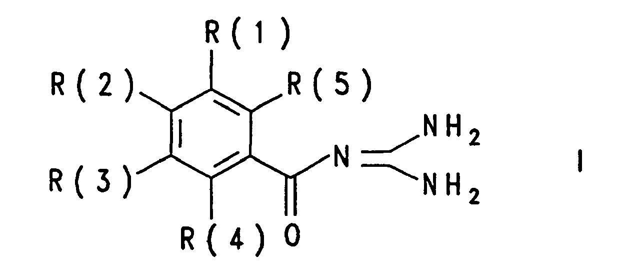 worin bedeuten:
R(1) R(6)-CO oder R(7)R(8)N-CO;
   R(6) (C₁-C₈)-Alkyl, (C₁-C₈)-Perfluoralkyl, (C₃-C₈)-Alkenyl oder -CₙH₂ₙ-R(9),
   n Null, 1, 2, 3 oder 4;
   R(9) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(10)R(11), R(10) und R(11)
   H, (C₁-C₄)-Alkyl oder (C₁-C₄)-Perfluoralkyl;
   R(7) H, (C₁-C₈)-Alkyl, (C₁-C₈)-Perfluoralkyl, (C₃-C₈)-Alkenyl oder
   -CₙH₂ₙ-R(12);
   n Null, 1, 2, 3 oder 4;
   R(12) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(13)R(14);
   R(13) und R(14)
   H, (C₁-C₄)-Alkyl oder (C₁-C₄)-Perfluoralkyl;
   R(8) H, (C₁-C₄)-Alkyl oder (C₁-C₄)-Perfluoralkyl;
   oder
   R(7) und R(8) gemeinsam
   4 oder 5 Methylengruppen, von denen eine CH₂-Gruppe durch Sauerstoff, S, NH, N-CH₃ oder N-Benzyl ersetzt sein kann;
R(2) wie R(1) definiert, oder H, OH, F, Cl, Br, I, CN, NO₂, (C₁-C₈)-Alkyl, (C₁-C₈)-Perfluoralkyl, (C₃-C₈)-Alkenyl oder -CₙH₂ₙR(15);
   n Null, 1, 2, 3 oder 4;
   R(15) (C₃-C₈)-Cycloalkyl, Phenyl, Biphenylyl oder Naphthyl,
   wobei die Aromaten nicht substituiert oder substituiert sind mit 1 - 3 Substituenten ausgewählt aus der Gruppe bestehend aus F, Cl, CF₃, Methyl, Methoxy und NR(16)R(17);
   R(16) und R(17)
   H, (C₁-C₄)-Alkyl oder (C₁-C₄)-Perfluoralkyl;
   oder
R(2) (C₁-C₉)-Heteroaryl, das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   oder
R(2) SR(18), -OR(18), -NR(18)R(19) oder -CR(18)R(19)R(20);
   R(18) -CₐH₂ₐ-(C₁-C₉)-Heteroaryl,
   das unsubstituiert oder substituiert ist mit 1 - 3 Substituenten ausgewählt aus der Gruppe bestehend aus F, Cl, CF₃, CH₃, Methoxy, Hydroxy, Amino, Methylamino, Dimethylamino;
   a Null, 1 oder 2;
   R(19) und R(20)
   unabhängig voneinander wie R(18) definiert oder Wasserstoff, (C₁-C₄)-Alkyl oder (C₁-C₄)-Perfluoralkyl;
   oder
R(2) R(21)-SOₘ oder R(22)R(23)N-SO₂-, m 1 oder 2;
   R(21) (C₁-C₈)-Alkyl, (C₁-C₈)-Perfluoralkyl, (C₃-C₈)-Alkenyl oder -CₙH₂ₙ-R(24);
   n Null, 1, 2, 3 oder 4;
   R(24) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(27)R(28); R(27) und R(28)
   H, (C₁-C₄)-Alkyl oder (C₁-C₄)-Perfluoralkyl;
   R(22) H, (C₁-C₈)-Alkyl, (C₁-C₈)-Perfluoralkyl, (C₃-C₈)-Alkenyl oder -CₙH₂ₙ-R(29);
   n Null, 1, 2, 3 oder 4;
   R(29) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(30)R(31); R(30) und R(31)
   H, (C₁-C₄)-Alkyl oder (C₁-C₄)-Perfluoralkyl;
   R(23) Wasserstoff, (C₁-C₄)-Alkyl oder (C₁-C₄)-Perfluoralkyl;
   oder
   R(22) und R(23)
   gemeinsam 4 oder 5 Methylengruppen, von denen eine CH₂-Gruppe durch Sauerstoff, S, NH, N-CH₃ oder N-Benzyl ersetzt sein kann;
   oder
R(2) R(33)X-;
   X Sauerstoff, S, NR(34), (D=O)A- oder NR(34)C=MN⁽*⁾R(35)-;
   M Sauerstoff oder S;
   A Sauerstoff oder NR(34);
   D C oder SO;
   R(33) (C₁-C₈)-Alkyl, (C₃-C₈)-Alkenyl, (CH₂)_{b}C_{d}F_{2d+1} oder -CₙH₂ₙ-R(36);
   b Null oder 1;
   d 1, 2, 3, 4, 5, 6 oder 7;
   n Null, 1, 2, 3, oder 4;
   R(36) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(37)R(38); R(37) und R(38)
   H, (C₁-C₄)-Alkyl oder (C₁-C₄)-Perfluoralkyl;
   R(34) Wasserstoff, (C₁-C₄)-Alkyl oder (C₁-C₄)-Perfluoralkyl;
   R(35) definiert wie R(33);
   oder
   R(33) und R(34)
   gemeinsam 4 oder 5 Methylengruppen, von denen eine CH₂-Gruppe durch Sauerstoff, S, NH, N-CH₃ oder N-Benzyl ersetzt sein kann;
   wobei A und N⁽*⁾ an den Phenylkern des Benzoylguanidin-Grundkörpers
   gebunden sind;
   oder
R(2) -SR(40), -OR(40), -NHR(40), -NR(40)R(41), -CHR(40)R(42), -CR(42)R(43)OH, -C≡CR(45), -CR(46)=CR(45) oder -[CR(47)R(48)]ᵤ-CO-[C(R49)R(50)]ᵥ-R(44);
   R(40) und R(41)
   unabhängig voneinander -(CH₂)ₚ-(CHOH)_{q}-(CH₂)ᵣ-(CHOH)ₜ-R(51) oder -
   (CH₂)ₚ-O-(CH₂-CH₂O)_{q}-R(51);
   R(51) Wasserstoff oder Methyl;
   u 1, 2, 3 oder 4;
   v Null, 1, 2, 3 oder 4;
   p, q und r
   unabhängig voneinander Null, 1, 2, 3 oder 4;
   t 1, 2, 3 oder 4;
   R(42) und R(43)
   unabhängig voneinander Wasserstoff oder (C₁-C₆)-Alkyl;
   oder
   R(42) und R(43)
   zusammen mit dem sie tragenden Kohlenstoff-Atom ein (C₃-C₈)-Cycloalkyl;
   R(44) Wasserstoff, (C₁-C₆)-Alkyl, (C₃-C₈)-Cycloalkyl, -CₑH₂ₑ-R(45);
   e Null, 1, 2, 3 oder 4;
   R(45) Phenyl,
   das unsubstituiert oder substituiert ist mit 1 - 3 Substituenten ausgewählt aus der Gruppe bestehend aus F, Cl, CF₃, Methyl, Methoxy und NR(52)R(53);
   R(52) und R(53)
   H oder (C₁-C₄)-Alkyl;
   oder
   R(45) (C₁-C₉)-Heteroaryl,
   das unsubstituiert oder wie Phenyl substituiert ist;
   oder
   R(45) (C₁-C₆)-Alkyl,
   das unsubstituiert oder mit 1 - 3 OH substituiert ist;
   R(46), R(47), R(48), R(49) und R(50)
   unabhängig voneinander Wasserstoff oder Methyl;
   oder
R(2) R(55)-NH-SO₂-;
   R(55) R(56)R(57)N-(C=Y)-;
   Y Sauerstoff, S oder N-R(58);
   R(56) und R(57) unabhängig voneinander Wasserstoff, (C₁-C₈)-Alkyl, (C₃-C₆)-Alkenyl oder -C_{f}H_{2f}-R(59);
   f Null, 1, 2, 3 oder 4;
   R(59) (C₅-C₇)-Cycloalkyl, Phenyl,
   welches unsubstituiert ist oder substituiert mit 1 - 3 Substituenten ausgewählt aus der Gruppe bestehend aus F, Cl, CF₃, Methoxy und (C₁-C₄)-Alkyl;
   oder
   R(56) und R(57)
   gemeinsam 4 oder 5 Methylengruppen, von denen eine CH₂-Gruppe durch Sauerstoff, S, NH, N-CH₃ oder N-Benzyl ersetzt sein kann;
   R(58)
   wie R(56) definiert oder gleich Amidin;
R(3), R(4) und R(5) sind unabhängig voneinander wie R(1) oder R(2) definiert, wobei jedoch mindestens einer der Substituenten R(2), R(3), R(4) und R(5) gleich OH sein muß;
   sowie deren pharmazeutisch verträgliche Salze;
   (HOE 95/F 007 K - EP-OS 723 956, NZ 280 887)

ag) Benzoylguanidine der Formel I 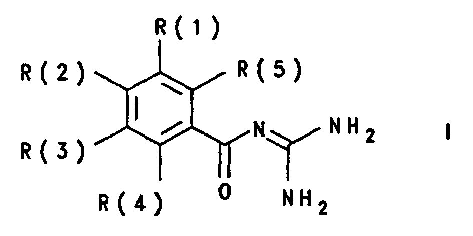 worin bedeuten:
einer der drei Substituenten R(1), R(2) und R(3)
   R(6)-A-B-D-;
   R(6) ein basischer protonierbarer Rest, d.h. eine Aminogruppe -NR(7)R(8), eine Amidinogruppe R(7)R(8)N-C[=N-R(9)]- oder eine Guanidinogruppe 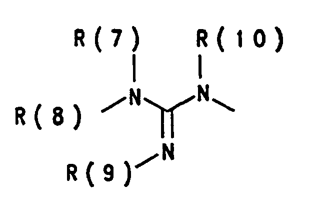 R(7), R(8), R(9) und R(10)
   unabhängig voneinander Wasserstoff oder Alkyl mit 1, 2, 3 oder 4 C-Atomen;
   oder
   R(7) und R(8)
   gemeinsam CₐH₂ₐ;
   a 4, 5, 6 oder 7;
   wobei im Falle von a = 5, 6 oder 7 eine Methylengruppe der Gruppe CₐH₂ₐ durch eine Heteroatomgruppe O, SOₘ oder NR(11) ersetzt sein kann,
   oder
   R(8) und R(9) oder R(9) und R(10) oder R(7) und R(10)
   eine Gruppe CₐH₂ₐ;
   a 2, 3, 4 oder 5;
   wobei im Falle von a = 3, 4 oder 5 eine Methylengruppe der Gruppe CₐH₂ₐ durch eine Heteroatomgruppe O, SOₘ oder NR(11) ersetzt sein kann;
   m Null, 1 oder 2;
   R(11) Wasserstoff oder Methyl;
   oder
   R(6) ein basisches heteroaromatisches Ringsystem mit 1 - 9 C-Atomen;
A C_{b}H_{2b};
   b 1, 2, 3, 4, 5, 6, 7, 8, 9 oder 10;
   wobei in der Gruppe C_{b}H_{2b} ein oder zwei Methylengruppen durch eine der Gruppierungen ausgewählt aus der Gruppe bestehend aus -O-,-CO-, -CH[OR(20)]-, -SOₘ-, -NR(20)-, -NR(20)-CO-, -NR(20)-CO-NH-, -NR(20)-CO-NH-SO₂- 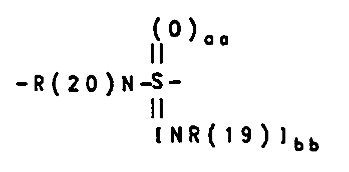 und -SOₐₐ[NR(19)]_{bb-} ersetzt sein können;
   und wobei in der Gruppe C_{b}H_{2b} eine Methylengruppe durch -CH-R(99) ersetzt sein kann, wobei R(99) gemeinsam mit R(7) einen Pyrrolidin- oder Piperidin-Ring bildet;
   aa 1 oder 2;
   bb 0 oder 1;
   aa + bb = 2;
   R(19) Wasserstoff oder Alkyl mit 1, 2, 3 oder 4 C-Atomen
   R(20) Wasserstoff oder Methyl;
B einen Phenylen- oder Naphthylenrest, 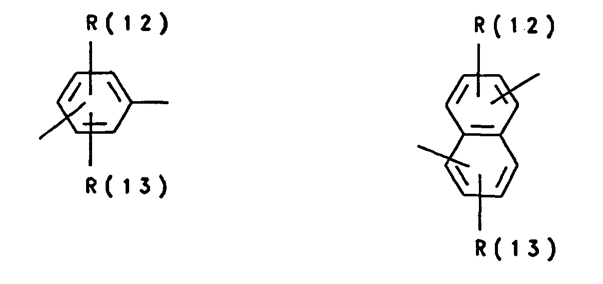 R(12) und R(13)
   unabhängig voneinander Wasserstoff, Methyl, F, Cl, Br, J, CF₃ oder -SO_{w}-R(14);
   R(14) Methyl oder NR(15)R(16);
   R(15) und R(16)
   unabhängig voneinander Wasserstoff oder Alkyl mit 1, 2, 3 oder 4 C-Atomen;
   w Null, 1 oder 2;
D -C_{d}H_{2d}-X_{f}-;
   d Null, 1, 2, 3 oder 4;
   X -O-,-CO-, -CH[OR(21)]-, -SOₘ- oder -NR(21)-;
   f Null oder 1;
   R(21) Wasserstoff oder Methyl;
   m Null, 1 oder 2;
   und die jeweils anderen Substituenten R(1) und R(2) und R(3) unabhängig voneinander Wasserstoff, F, Cl, Br, J, -CN, -(C₁-C₈)-Alkyl, -(C₂-C₈)-Alkenyl, -NR(35)R(36) oder R(17)-C_{g}H_{2g}-Z_{b}-;
   g Null, 1, 2, 3 oder 4;
   h Null oder 1;
   R(35) und R(36)
   unabhängig voneinander Wasserstoff oder Alkyl mit 1, 2, 3, 4, 5 oder 6 C-Atomen;
   oder
   R(35) und R(36)
   gemeinsam 4 - 7 Methylengruppen, von denen eine CH₂-Gruppe durch Sauerstoff, -S-, -NH-, -NCH₃ oder -N-Benzyl ersetzt sein kann;
   Z -O-,-CO-, -SOᵥ- ,-NR(18)-, -NR(18)-CO-, -NR(18)-CO-NH- oder
   -NR(18)-SO₂-;
   R(18) Wasserstoff oder Methyl;
   v Null, 1 oder 2;
   R(17) Wasserstoff, Cycloalkyl mit 3, 5 oder 6 C-Atomen oder CₖF₂ₖ₊₁-;
   k 1, 2 oder 3,
   oder
   R(17) Pyrrol-1-yl, Pyrrol-2-yl oder Pyrrol-3-yl, welches nicht substituiert oder substituiert ist mit 1 - 4 Substituenten ausgewählt aus der Gruppe bestehend aus F, Cl, Br, I, -CN, (C₂-C₈)-Alkanoyl, (C₂-C₈)-Alkoxycarbonyl, Formyl, Carboxy, -CF₃, Methyl und Methoxy;
   oder
   R(17) -(C₃-C₈)-Cycloalkyl oder Phenyl, welches nicht substituiert oder substituiert ist mit 1 - 3 Substituenten ausgewählt aus der Gruppe bestehend aus F und Cl, -CF₃, Methyl, Hydroxy, Methoxy, -NR(37)R(38), CH₃SO₂- und H₂NO₂S-;
   R(37) und R(38)
   Wasserstoff oder -CH₃;
   R(4) und R(5)
      unabhängig voneinander Wasserstoff, Alkyl mit 1, 2, 3 oder 4 C-Atomen, F, Cl, -OR(32), -NR(33)R(34) oder -CᵣF₂ᵣ₊₁;
      R(32), R(33) und R(34)
      unabhängig voneinander Wasserstoff oder Alkyl mit 1, 2 oder 3 C-Atomen;
      r 1, 2, 3 oder 4;
      sowie deren pharmakologisch verträgliche Salze;
      (HOE 95/F 072 - EP-OS 738 712, NZ 286 380

ah) Indenoylguanidine der Formel I 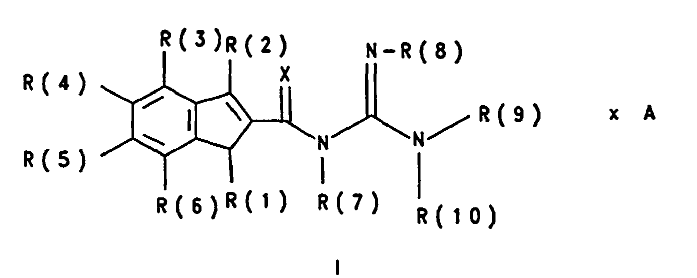 worin bedeuten:
R(1) und R(2) unabhängig voneinander Wasserstoff, Alkyl mit 1, 2, 3, 4, 5, 6, 7, 8, 9 oder 10 C-Atomen, Cycloalkyl mit 3, 4, 5 oder 6 C-Atomen. O-Alkyl mit 1, 2, 3 or 4 C-Atomen, O-C(=O)-Alkyl mit 1, 2, 3 oder 4C-Atomen oder CₘH₂ₘ-NR(12)R(13);
   R(12) und R(13) unabhängig voneinander Wasserstoff oder Alkyl mit 1, 2, 3 or 4 C-Atomen;
   m Nuill, 2, 3 oder 4;
   NH-C(=O)-NH₂, C(=O)-O-Alkyl mit 1, 2, 3 oder 4C-Atomen, C(=O)-NH₂, C(=O)-NH-Alkyl mit 1, 2, 3 oder 4 C-Atomen, C(=O)-N(Alkyl)₂ mit 1, 2, 3 oder 4 C-Atomen in jeder Alkylgruppe, Alkenyl mit 2, 3, 4, 5, 6, 7, 8, 9 oder 10 C-Atomen, alkinyl mit 2, 3, 4, 5, 6, 7, 8, 9 oder 10 C-Atomen, Alkylaryl mit 1, 2, 3 or 4 C-Atomen in der Alkylgruppe, Alkenyl-aryl mit 2, 3, 4, 5, 6, 7, 8, 9 oder 10 C-Atomen in der Alkenylgruppe, Alkinyl-aryl mit 2, 3, 4, 5, 6, 7, 8, 9 oder 10 C-Atomen in der Alkinylgruppe, C₁-C₄-alkyl-substituiertes aryl, C₁-C₄-Alkyl-heteroaryl, C₁-C₄-Alkenyl-heteroaryl, Aminoalkyl-aryl mit 1, 2, 3 oder 4 C-Atomen in der Alkylgruppe, substituiertes Aryl, Heteroaryl und substituiertes heteroaryl;
R(3), R(4), R(5) and R(6)
   unabhängig voneinander Wasserstoff, Alkyl mit 1, 2, 3, 4, 5, 6, 7, 8, 9 oder 10 C-Atomen, O-Alkyl mit 1, 2, 3, 4, 5, 6, 7, 8, 9 oder 10 C-Atomen, Halogen, (wie F, Cl, Br, I), OH, Aryl, substituiertes Aryl, Heteroaryl, substituiertes Heteroaryl, O-Niederalkyl, O-Aryl, O-Niederalkyl-aryl, O-Substituiertes Aryl, O-Niederalkyl-substituiertes Aryl, O-C(=O)-C₁-C₄-Alkyl-aryl, O-C(=O)-NH-C₁-C₄-Alkyl, O-C(=O)-N(C,-C₄-Alkyl)₂, NO₂, CN, CF₃, NH₂, NH-C(=O)-C₁-C₄-Alkyl, NH-C(=O)-NH₂, COOH, C(=O)-O-C₁-C₄-alkyl, C(=O)-NH₂, C(=O)-NH-C₁-C₄-Alkyl, C(=O)-N(C₁-C₄-Alkyl)₂, C₁-C₄-COOH, C₁-C₄-Alkyl-C(=O)-O-C₁-C₄-alkyl, SO₃H, SO₂-Alkyl, SO₂-Alkylaryl, SO₂-N-(Alkyl)₂, SO₂-N(Alkyl)(alkylaryl), C(=O)-R(11), C₁-C₁₀-Alkyl-C(=O)-R(11), C₂-C₁₀-Alkenyl-C(=O)-R(11), C₂-C₁₀-Alkinyl-C(=O)-R(11), NH-C(=O)-C₁-C₁₀-Alkyl-C(=O)-R(11), O-C₁-C₁₁-Alkyl-C(=O)-R(11);
   R(11) C₁-C₄-Alkyl, C₁-C₄-Alkynyl, Aryl, substituiertes Aryl, NH₂, NH-C₁-C₄-Alkyl, N-(C₁-C₄-Alkyl)₂, SO₃H, SO₂-alkyl, SO₂-Alkylaryl, SO₂-N-(Alkyl)₂, SO₂-N(Alkyl)(alkylaryl);
X O, S oder NH;
R(7), R(8), R(9) und R(10)
   unabhängig voneinander Wasserstoff, Alkyl, Cycloalkyl, Aryl, Alkylaryl; oder
R(8) und R(9)
   gemeinsam Teil eines 5, 6 oder 7-gliedrigen heterocyclischen Rings;
A abwesend oder eine ungiftige organische oder Mineralsäure.
   (HOE 95/F 109 - EP 748 795, NZ 286 583)

ai) Benzyloxycarbonylguanidine der Formel I 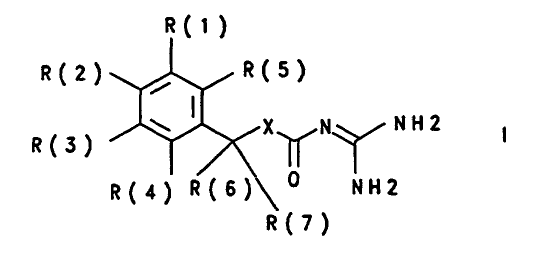 worin bedeuten:
R(1), R(2) und R(3)
   unabhängig voneinander -Y-[4-R(8)-Phenyl], -Y-[3-R(8)-Phenyl] oder -Y-[2-R(8)-Phenyl],
   wobei das Phenyl jeweils unsubstituiert oder substituiert ist mit 1 - 2 Substituenten aus der Gruppe bestehend aus F, Cl, -CF₃, Methyl, Hydroxy, Methoxy und -NR(96)R(97); R(96) und R(97)
   unabhängig voneinander Wasserstoff oder -CH₃;
Y eine Bindung, CH₂, Sauerstoff, -S- oder -NR(9); R(9) Wasserstoff oder Alkyl mit 1, 2, 3 oder 4 C-Atomen;
   R(8) SOₐ[NR(98)]_{b}NR(99)R(10);
   a 1 oder 2;
   b 0 oder 1;
   a + b = 2;
   R(98), R(99) und R(10) unabhängig voneinander Wasserstoff, -(C₁-C₈)-Alkyl, Benzyl, - (C₂-C₈)-Alkylen-NR(11)R(12), (C₂-C₈)-Alkylen-NR(13)-(C₂-C₈)-Alkylen-NR(37)R(38) oder (C₀-C₈)-Alkylen-CR(39)R(40)-CR(41)R(42)(C₀-C₈)-Alkylen-NR(43)R(44);
   R(11), R(12), R(13), R(37), R(38), R(43) und R(44) unabhängig voneinander Wasserstoff, -(C₁-C₈)-Alkyl oder Benzyl:
   R(39), R(40), R(41) und R(42) unabhängig voneinander Wasserstoff, -(C₁-C₈)-Alkyl oder -(C₀-C₃)-Alkylen-Phenyl,
   wobei das Phenyl nicht substituiert oder substituiert ist mit 1 - 3 Substituenten ausgewählt aus der Gruppe bestehend aus F, Cl,-CF₃, Methyl und Methoxy;
   oder
   R(99) und R(10)
   gemeinsam 4 - 6 Methylengruppen, von denen eine CH₂-Gruppe durch Sauerstoff, -S-, -NH-, -N-CH₃ oder -N-Benzyl ersetzt sein kann;
   oder
   R(8) SOₐ[NR(98)]_{b}NR(95)-C[=N-R(94)]-NR(93)R(92); R(92), R(93), R(94) und R(95) unabhängig voneinander Wasserstoff oder Alkyl mit 1, 2, 3 oder 4 C-Atomen;
   oder
R(1), R(2) und R(3) unabhängig voneinander Pyrrol-1-yl, Pyrrol-2-yl oder Pyrrol-3-yl, welches nicht substituiert oder substituiert ist mit 1 - 4 Substituenten ausgewählt aus der Gruppe bestehend aus F, Cl, Br, I, -CN, (C₂-C₈)-Alkanoyl, (C₂-C₈)-Alkoxycarbonyl, Formyl, Carboxy, -CF₃, Methyl, Methoxy;
   oder
R(1), R(2) und R(3)
   unabhängig voneinander Wasserstoff, -(C₁-C₈)-Alkyl, -(C₂-C₈)-Alkenyl oder -(CH₂)ₘR(14);
   m Null, 1 oder 2;
   R(14) -(C₃-C₈)-Cycloalkyl oder Phenyl, welches nicht substituiert oder substituiert ist mit 1 - 3 Substituenten ausgewählt aus der Gruppe bestehend aus F und Cl, -CF₃, Methyl, Methoxy und -NR(15)R(16); R(15) und R(16)
   Wasserstoff oder -CH₃;
   oder
R(1), R(2) und R(3)
   unabhängig voneinander -Q-4-[(CH₂)ₖ-CHR(17)-(C=O)R(20)]-Phenyl, -Q-3-(CH₂)ₖ-CHR(17)-(C=O)R(20)]-Phenyl oder -Q-2-[(CH₂)ₖ-CHR(17)-(C=O)R(20)]-Phenyl,
   wobei das Phenyl jeweils unsubstituiert oder substituiert ist mit 1 - 2 Substituenten aus der Gruppe F, Cl, -CF₃, Methyl, Hydroxy, Methoxy und -NR(35)R(36);
   R(35) und R(36)
   unabhängig voneinander Wasserstoff oder -CH₃;
   Q eine Bindung, Sauerstoff, -S- oder -NR(18);
   R(18) Wasserstoff oder -(C₁-C₄)-Alkyl;
   R(17) -OR(21) oder -NR(21)R(22);
   R(21) und R(22)
   unabhängig voneinander Wasserstoff, -(C₁-C₈)-Alkyl, -(C₁-C₈)-Alkanoyl, -(C₁-C₈)-Alkoxycarbonyl, Benzyl, Benzyloxycarbonyl;
   oder
   R(21) Trityl;
   R(20) -OR(23) oder -NR(23)R(24);
   R(23), R(24) unabhängig voneinander
   Wasserstoff, -(C₁-C₈)-Alkyl oder Benzyl;
   k Null, 1, 2, 3 oder 4;
   oder
R(1), R(2) und R(3)
   unabhängig voneinander (C₁-C₉)-Heteroaryl, das über C oder N verknüpft ist und das unsubstituiert oder substituiert ist mit 1-3 Substituenten aus der Gruppe F, Cl, CF₃, CH₃, Methoxy, Hydroxy, Amino, Methylamino und Dimethylamino;
   oder
R(1), R(2) und R(3)
   -SR(25), -OR(25), -NR(25)R(26), -CR(25)R(26)R(27); R(25) -C_{f}H_{2f}-(C₁-C₉)-Heteroaryl,
   das unsubstituiert oder substituiert ist mit 1-3 Substituenten aus der Gruppe F, Cl, CF₃, CH₃, Methoxy, Hydroxy, Amino, Methylamino und Dimethylamino;
   f Null, 1 oder 2;
   R(26) und R(27)
   unabhängig voneinander wie R(25) definiert oder Wasserstoff oder (C₁-C₄)-Alkyl,
   oder
R(1), R(2) und R(3)
   unabhängig voneinander (C₁-C₉)-Heteroaryl-N-Oxid, das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   oder
R(1), R(2) und R(3) unabhängig voneinander -SR(28), -OR(28), -NR(28)R(29) oder -CR(28)R(29)R(30);
   R(28) -C_{g}H_{2g}-(C₁-C₉)-Heteroaryl-N-Oxid,
   das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   g Null, 1 oder 2;
   R(29), R(30) unabhängig voneinander wie R(28) definiert, Wasserstoff oder (C₁-C₄)-Alkyl;
   oder
R(1), R(2) und R(3) unabhängig voneinander Wasserstoff, F, Cl, Br, I, -C≡N, T-(CH₂)ₕ-(CᵢF₂ᵢ₊₁), R(31)SOₗ-, R(32)R(33)N-CO-, R(34)-CO- oder R(45)R(46)N-SO₂, wobei die Perfluoralkylgruppe geradkettig oder verzweigt ist;
   T eine Bindung, Sauerstoff, -S- oder -NR(47);
   l Null, 1 oder 2;
   h Null, 1 oder 2;
   i 1, 2, 3, 4, 5 oder 6;
   R(31), R(32), R(34) und R(45)
   unabhängig voneinander -(C₁-C₈)-Alkyl, -(C₃-C₆)Alkenyl, (CH₂)ₙR(48) oder -CF₃;
   n Null, 1, 2, 3 oder 4;
   R(47) Wasserstoff oder Alkyl mit 1, 2 oder 3 C-Atomen;
   R(48) -(C₃-C₇)-Cycloalkyl oder Phenyl,
   welches nicht substituiert ist oder substituiert mit 1 - 3 Substituenten ausgewählt aus der Gruppe bestehend aus F, Cl, -CF₃, Methyl, Methoxy und -NR(49)R(50);
   R(49) und R(50)
   Wasserstoff oder Alkyl mit 1, 2, 3 oder 4 C-Atomen;
   oder

   R(32), R(34) und R(45)
   Wasserstoff;
   R(33) und R(46)
   unabhängig voneinander Wasserstoff oder Alkyl mit 1, 2, 3 oder 4 C-Atomen;
   oder
   R(32) und R(33) sowie R(45) und R(46)
   gemeinsam 5 oder 6 Methylengruppen, von denen eine CH₂-Gruppe
   durch Sauerstoff, -S-, -NH-, -NCH₃ oder -N-Benzyl ersetzt sein kann;
   oder
R(1), R(2) und R(3)
   unabhängig voneinander R(51)-A-G-D-;
   R(51) ein basischer protonierbarer Rest, d.h. eine Aminogruppe -NR(52)R(53), eine Amidinogruppe R(52)R(53)N-C[=N-R(54)]- oder eine Guanidinogruppe R(52)R(53)N-C[=N-R(54)]-NR(55)-;
   R(52), R(53), R(54) und R(55)
   unabhängig voneinander Wasserstoff oder Alkyl mit 1, 2, 3 oder 4 C-Atomen;
   oder
   R(52) und R(53)
   eine Gruppe C_{α}H_{2α};
   α 4, 5, 6 oder 7;
   wobei im Falle von α = 5, 6 oder 7 ein C-Atom der Gruppe C_{α}H_{2α} durch eine Heteroatomgruppe O, SO_{d} oder NR(56) ersetzt sein kann,
   oder
   R(53) und R(54) oder R(54) und R(55) oder R(52) und R(55)
   eine Gruppe C_{γ}H_{2γ};
   γ 2, 3, 4 oder 5;
   wobei im Falle von γ = 3, 4 oder 5 ein C-Atom der Gruppe C_{γ}H_{2γ} durch eine Heteroatomgruppe O, SO_{d} oder NR(56) ersetzt sein kann;
   d Null, 1 oder 2;
   R(56) Wasserstoff oder Methyl;
   oder
R(51) ein basisches heteroaromatisches Ringsystem mit 1 - 9 C-Atomen;
A eine Gruppe CₑH₂ₑ;
   e Null, 1, 2, 3, 4, 5, 6, 7, 8, 9 oder 10;
   wobei in der Gruppe CₑH₂ₑ ein C-Atom durch eine der Gruppierungen - O-,-CO-, -CH[OR(57)]-, -SOᵣ-, -NR(57)-, -NR(57)-CO-, -NR(57)-CO-NH-, -NR(57)-CO-NH-SO₂- oder -NR(57)-SO₂- ersetzt sein kann;
   r Null, 1 oder 2;
G einen Phenylenrest, 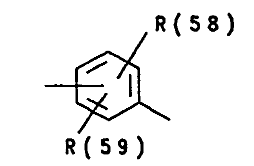 R(58) und R(59)
   unabhängig voneinander Wasserstoff, Methyl, Methoxy, F, Cl, Br, J, CF₃ oder -SOₛ-R(60);
   R(60) Methyl oder NR(61)R(62);
   R(61) und R(62)
   unabhängig voneinander Wasserstoff oder Alkyl
   mit 1, 2, 3 oder 4 C-Atomen;
D -CᵥH₂ᵥ-E_{w}-'
   v Null, 1, 2, 3 oder 4;
   E -O-, -CO-, -CH[OR(63)]-, -SOₐₐ oder -NR(63)-;
   w Null oder 1;
   aa Null, 1 oder 2
   R(63) Wasserstoff oder Methyl, oder
R(1), R(2) und R(3)
   unabhängig voneinander -CF₂R(64), -CF[R(65)][R(66)], -CF[(CF₂)_{q}-CF₃)][R(65)], -C[(CF₂)ₚ-CF₃]=CR(65)R(66);
   R(64) Alkyl mit 1,2,3 oder 4 C-Atomen oder Cycloalkyl mit 3, 4, 5, 6 oder 7 C-Atomen;
   R(65) und R(66) unabhängig voneinander Wasserstoff oder Alkyl mit 1, 2, 3 oder 4 C-Atomen;
   q Null, 1 oder 2;
   p Null, 1 oder 2;
   oder
R(1), R(2) und R(3)
   unabhängig voneinander -OR(67) oder -NR(67)R(68); R(67) und R(68)
   unabhängig voneinander Wasserstoff oder Alkyl mit 1, 2, 3, 4, 5 oder 6 C-Atomen;
   oder
   R(67) und R(68)
   gemeinsam 4, 5, 6 oder 7 Methylengruppen, von denen eine CH₂-Gruppe durch Sauerstoff, -S-, SO₂, -NH-, -NCH₃ oder -N-Benzyl ersetzt
   sein kann;
R(4) und R(5)
   unabhängig voneinander Wasserstoff, Alkyl mit 1, 2, 3 oder 4 C-Atomen, F, Cl, -OR(69), -NR(70)R(71) oder -C_{z}F_{2z+1};
   R(69), R(70) und R(71)
   unabhängig voneinander Wasserstoff oder Alkyl mit 1, 2 oder 3 C-Atomen;
   z 1, 2, 3 oder 4;
R(6) und R(7)
   unabhängig voneinander Wasserstoff oder Alkyl mit 1, 2, 3 oder 4 C-Atomen;
X Sauerstoff oder NR(72);
   R(72) Wasserstoff oder Alkyl mit 1, 2, 3 oder 4 C-Atomen; sowie deren pharmazeutisch verträgliche Salze;
   (HOE 95/F 115 - EP 744 397, NZ 286 622)

ak) Fluorphenylgruppen tragende Alkenylcarbonsäure-guanidide der Formel I 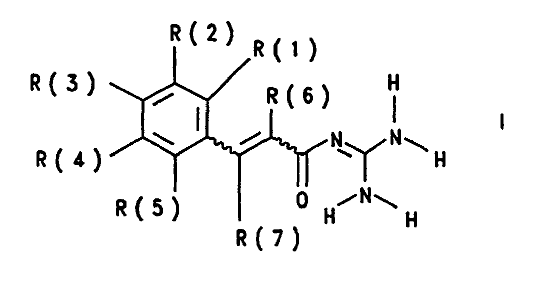 worin bedeuten:
R(6) Wasserstoff, (C₁-C₈)-Alkyl, (C₃-C₈)-Cycloalkyl oder Phenyl,
   wobei die Phenylgruppe nicht substituiert oder substituiert ist mit 1 - 3 Substituenten ausgewählt aus der Gruppe bestehend aus F, Cl, CF₃, Methyl, Methoxy und NR(9)R(10);
   R(9) und R(10)
   Wasserstoff, (C₁-C₄)-Alkyl oder (C₁-C₄)-Perfluoralkyl; R(7) unabhängig wie R(6) definiert;
R(1), R(2), R(3), R(4) und R(5)
   unabhängig voneinander Wasserstoff oder F;
   wobei jedoch mindestens einer der Reste R(1), R(2), R(3), R(4) und R(5) Fluor sein muß;
   sowie deren pharmazeutisch verträgliche Salze;
   (HOE 95/F 167 - NZ 299 015)

al) Benzoylguanidine der Formel I 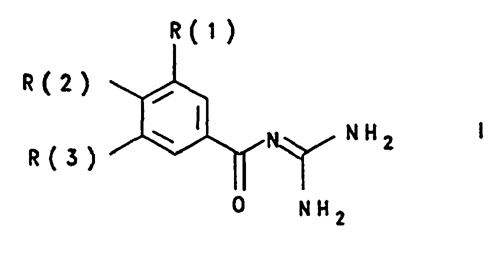 worin bedeuten:
R(1) R(4)-SOₘ oder R(5)R(6)N-SO₂-;
   m 1 oder 2;
   R(4) und R(5)
   unabhängig voneinander Alkyl mit 1, 2, 3, 4, 5, 6, 7 oder 8 C-Atomen, Alkenyl mit 3, 4, 5 oder 6 C-Atomen, CF₃ oder -CₙH₂ₙ-R(7);
   n Null, 1, 2, 3 oder 4;
   R(6) H oder Alkyl mit 1, 2, 3 oder 4 C-Atomen;
   R(7) Cycloalkyl mit 3, 4, 5, 6, 7 oder 8 C-Atomen oder Phenyl,
   welches nicht substituiert ist oder substituiert mit 1 - 3 Substituenten ausgewählt aus der Gruppe bestehend aus F, Cl, CF₃, Methyl, Methoxy und NR(8)R(9);
   R(8) und R(9)
   H oder Alkyl mit 1, 2, 3 oder 4 C-Atomen;
   oder
   R(5) auch Wasserstoff;
   oder
   R(5) und R(6)
   gemeinsam 4 oder 5 Methylengruppen, von denen eine CH₂-Gruppe
   durch Sauerstoff, S, NH, N-CH₃ oder N-Benzyl ersetzt sein kann;
   oder
R(1) -Oₚ-(CH₂)_{q}-(CF₂)ᵣ-CF₃;
   p Null oder 1;
   q Null, 1 oder 2;
   r Null, 1, 2 oder 3;
   oder
R(1) -SR(10), -OR(10) oder -CR(10)R(11)R(12);
   R(10), R(11) und R(12)
   unabhängig voneinander Wasserstoff, Alkyl mit 1, 2, 3, 4, 5, 6, 7 oder 8 C-Atomen, -CₛH₂ₛ-(C₃-C₈)-Cycloalkyl oder ein aromatisches System ausgewählt aus der Gruppe bestehend aus Pyridyl, Pyrrolyl, Chinolyl, Isochinolyl, Imidazolyl oder Phenyl;
   s Null, 1 oder 2;
   wobei die aromatischen Systeme Pyridyl, Pyrrolyl, Chinolyl, Isochinolyl, Imidazolyl und Phenyl unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
R(2) -(CH₂)ᵤ-(CF₂)ₜ-CF₃;
   t Null, 1, 2 oder 3;
   u Null oder 1;
R(3) Wasserstoff oder unabhängig wie R(1) definiert;
   sowie deren pharmazeutisch verträgliche Salze;
   (HOE 95/F 173 - NZ 299 052)

am) Substituierte Zimtsäureguanidide der Formel I 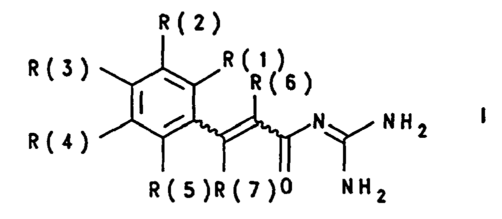 worin bedeuten:
mindestens einer der Substituenten R(1), R(2), R(3), R(4) und R(5)
   -Xₐ-Y_{b}-Lₙ-U;
   X CR(16)R(17), O, S oder NR(18);
   R(16), R(17) und R(18)
   unabhängig voneinander H, Alkyl mit 1, 2, 3 oder 4 C-Atomen oder Perfluoralkyl mit 1, 2, 3 oder 4 C-Atomen;
   a Null oder 1;
   Y Alkylen mit 1, 2, 3, 4, 5, 6, 7 oder 8 C-Atomen, Alkylen-T mit 1, 2, 3, 4, 5, 6, 7 oder 8 C-Atomen in der Alkylengruppe, T, T-Alkylen mit 1, 2, 3, 4, 5, 6, 7 oder 8 C-Atomen in der Alkylengruppe;
   T NR(20), O, S oder Phenylen,
   wobei das Phenylen nicht substituiert oder substituiert ist mit 1 - 3 Substituenten ausgewählt aus der Gruppe bestehend aus F, Cl, CF₃, Methyl, Methoxy und NR(21)R(22);
   R(20), R(21) und R(22)
   unabhängig voneinander H, Alkyl mit 1, 2, 3 oder 4 C-Atomen oder Perfluoralkyl mit 1, 2, 3 oder 4 C-Atomen;
   b Null oder 1;
   L O, S, NR(23) oder CₖH₂ₖ;
   k 1, 2, 3, 4, 5, 6, 7, 8;
   n Null oder 1;
   U NR(24)R(25) oder einen N-haltigen Heterocyclus mit 1, 2, 3, 4, 5, 6, 7, 8 oder 9 C-Atomen;
   R(24) und R(25)
   unabhängig voneinander Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen;
   oder
   R(24) und R(25) gemeinsam 4 oder 5 Methylenguppen, von denen eine CH₂-Gruppe durch Sauerstoff, S, NH, N-CH₃ oder N-Benzyl ersetzt sein kann;
   wobei die N-haltigen Heterocyclen N- oder C-verbrückt sind und nicht substituiert oder substituiert sind mit 1 - 3 Substituenten ausgewählt aus der Gruppe bestehend aus F, Cl, CF₃, Methyl, Methoxy und NR(27)R(28); R(23), R(27) und R(28)
   unabhängig voneinander H, Alkyl mit 1, 2, 3 oder 4 C-Atomen
   oder Perfluoralkyl mit 1, 2, 3 oder 4 C-Atomen;
   und die jeweils anderen Substituenten R(1), R(2), R(3), R(4) und R(5)
   unabhängig voneinander H, F, Cl, Br, I, CN, -Oₙ-CₘH₂ₘ₊₁, -Op-(CH₂)ₛ-C_{q}F_{2q+1} oder -CᵣH₂ᵣR(10);
   n Null oder 1;
   m Null 1, 2, 3, 4, 5, 6, 7 oder 8;
   p Null oder 1;
   q 1, 2, 3, 4, 5, 6, 7 oder 8;
   s Null, 1, 2, 3 oder 4;
   r Null, 1, 2, 3 oder 4;
   R(10)
   Cycloalkyl mit 3, 4, 5, 6, 7 oder 8 C-Atomen, oder Phenyl,
   wobei das Phenyl nicht substituiert oder substituiert ist mit 1 - 3 Substituenten ausgewählt aus der Gruppe bestehend aus F, Cl, CF₃, Methyl, Methoxy und NR(11)R(12);
   R(11) und R(12)
   unabhängig voneinander H, Alkyl mit 1, 2, 3 oder 4 C-Atomen oder Perfluoralkyl mit 1, 2, 3 oder 4 C-Atomen;
R(6) und R(7)
   unabhängig voneinander Wasserstoff, F, Cl, Br, I, CN, Alkyl mit 1, 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen, oder Phenyl,
   das nicht substituiert oder substituiert ist mit 1 - 3 Substituenten ausgewählt aus der Gruppe bestehend aus F, Cl, CF₃, Methyl, Methoxy und NR(14)R(15);
   R(14) und R(15)
   unabhängig voneinander H, Alkyl mit 1, 2, 3 oder 4 C-Atomen
   oder Perfluoralkyl mit 1, 2, 3 oder 4 C-Atomen;
   sowie deren pharmazeutisch verträgliche Salze;
   (HOE 95/F 220 - NZ 299 052)

an) Benzoylguanidine der Formel I 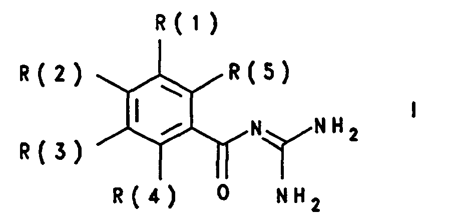 worin bedeuten:
mindestens einer der Substituenten R(1), R(2) und R(3)
   R(6)-C(OH)₂-;
   R(6) Perfluoralkyl mit 1, 2 oder 3-C-Atomen, das geradkettig oder verzweigt ist;
   und die übrigen Substituenten R(1), R(2) und R(3)
   unabhängig voneinander Wasserstoff, OH, F, Cl, Br, I, Alkyl mit 1, 2, 3, 4, 5 oder 6 C-Atomen, Cycloalkyl mit 3, 4, 5 oder 6 C-Atomen, Alkoxy mit 1, 2, 3 oder 4 C-Atomen oder Phenoxy,
   das unsubstituiert ist oder substituiert ist mit 1 - 3 Substituenten ausgewählt auf der Gruppe bestehend aus F, Cl, Methyl und Methoxy;
   oder
   die übrigen Substituenten R(1), R(2) und R(3)
   unabhängig voneinander Alkyl-SOₓ, -CR(7)=CR(8)R(9) oder -C≡CR(9);
   x Null, 1 oder 2;
   R(7) Wasserstoff oder Methyl;
   R(8) und R(9)
   unabhängig voneinander Wasserstoff, Alkyl mit 1, 2, 3 oder 4 C-Atomen, Cycloalkyl mit 3, 4, 5, 6, 7 oder 8 C-Atomen oder Phenyl, das unsubstituiert ist oder substituiert ist mit 1 - 3 Substituenten ausgewählt auf der Gruppe bestehend aus F, Cl, CF₃, Methyl und Methoxy;
   oder
   die übrigen Substituenten R(1), R(2) und R(3)
   unabhängig voneinander Phenyl, C₆H₅-(C₁-C₄)-Alkyl, Naphthyl, Biphenylyl, Chinolinyl, Isochinolinyl oder Imidazolyl,
   wobei Chinolinyl, Isochinolinyl oder Imidazolyl über C oder N gebunden sind, und wobei Phenyl, C₆H₅-(C₁-C₄)-Alkyl, Naphthyl, Biphenylyl, Chinolinyl, Isochinolinyl und Imidazolyl unsubstituiert sind oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
   oder
   die übrigen Substituenten R(1), R(2) und R(3)
   unabhängig voneinander SR(10), -OR(10), -CR(10)R(11)R(12);
   R(10)
   -C_{f}H_{2f}-(C₃-C₈)-Cycloalkyl, Chinolinyl, Isochinolinyl, Pyridinyl, Imidazolyl oder Phenyl,
   wobei die aromatischen Systeme Chinolinyl, Isochinolinyl, Pyridinyl, Imidazolyl und Phenyl unsubstituiert sind oder substituiert sind mit 1 - 3 Substituenten ausgewählt aus der gruppe bestehend aus F, Cl, CF₃, CH₃, Methoxy, Hydroxy, Amino, Methylamino und Dimethylamino;
   f Null, 1 oder 2;
   R(11) und R(12)
   unabhängig voneinander wie R(10) definiert, Wasserstoff oder Alkyl mit 1, 2, 3 oder 4 C-Atomen;
R(4) und R(5)
   unabhängig voneinander Wasserstoff, Alkyl mit 1, 2 oder 3-C-Atomen, F, Cl, Br, I, CN, OR(13), NR(14)R(15), -(CH₂)ₙ-(CF₂)ₒ-CF₃;
   R(13), R(14) und R(15)
   unabhängig voneinander Wasserstoff oder Alkyl mit 1, 2, 3 oder 4 C-Atomen;
   n Null oder 1;
   o Null, 1 oder 2;
   sowie deren pharmakologisch akzeptable Salze;
   (HOE 95/F 253 - NZ 299 682)

ao) Sulfonimidamide der Formel I 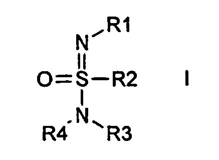 worin bedeuten:
mindestens einer der drei Substituenten R(1), R(2) und R(3)
ein Benzoylguanidin, 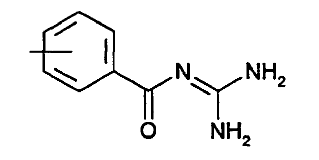 das im Phenylteil unsubstituiert oder substituiert ist mit 1 - 4 Resten ausgewählt aus der Gruppe bestehend aus Alkyl mit 1, 2, 3, 4, 5, 6, 7 oder 8 C-Atomen, Alkenyl mit 2, 3, 4, 5, 6, 7 oder 8 C-Atomen, -(CH₂)ₘ-R(14), F, Cl, Br, I, -C≡N, CF₃, R(22)SO₂-, R(23)R(24)N-CO-, R(25)-CO-, R(26)R(27)N-SO₂, -OR(35), -SR(35) oder -NR(35)R(36);
m Null, 1 oder 2;
R(14)
-(C₃-C₈)-Cycloalkyl oder Phenyl,
welches nicht substituiert oder substituiert ist mit 1 - 3 Substituenten ausgewählt aus der Gruppe bestehend aus F und Cl, -CF₃, Methyl, Methoxy und -NR(15)R(16); R(15) und R(16)
unabhängig voneinander Wasserstoff oder -CH₃; R(22), R(23), R(25) und R(26)
unabhängig voneinander Alkyl mit 1, 2, 3, 4, 5, 6, 7 oder 8 C-Atomen, Alkenyl mit 2, 3, 4, 5, 6, 7 oder 8 C-Atomen, (CH₂)ₙR(29) oder -CF₃;
n Null, 1, 2, 3 oder 4;
R(29) -(C₃-C₇)-Cycloalkyl oder Phenyl,
welches nicht substituiert ist oder substituiert mit 1 - 3 Substituenten ausgewählt aus der Gruppe bestehend aus F, Cl, -CF₃, Methyl, Methoxy und -NR(30)R(31); R(30) und R(31)
Wasserstoff oder Alkyl mit 1, 2, 3 oder 4 C-Atomen;
oder
R(23), R(25) und R(26)
Wasserstoff;
R(24) und R(27)
unabhängig voneinander Wasserstoff oder Alkyl mit 1, 2, 3 oder 4 C-Atomen;
oder
R(23) und R(24) sowie R(26) und R(27)
gemeinsam 5 oder 6 Methylengruppen, von denen eine CH₂-Gruppe durch Sauerstoff, -S-, -NH-, -NCH₃ oder -N-Benzyl ersetzt sein kann;
R(35) und R(36)
unabhängig voneinander Wasserstoff oder Alkyl mit 1, 2, 3, 4, 5 oder 6 C-Atomen;
oder
R(35) und R(36)
gemeinsam 4 - 7 Methylengruppen, von denen eine CH₂-Gruppe durch Sauerstoff, -S-, -NH-, -NCH₃ oder -N-Benzyl ersetzt sein kann;
oder
R(35)
Phenyl,
welches nicht substituiert ist oder substituiert mit 1 - 3 Substituenten ausgewählt aus der Gruppe bestehend aus F, Cl, -CF₃, Methyl, Methoxy, SO₂R(5), SO₂NR(6)R(7) und -NR(32)R(33);
R(5) Alkyl mit 1, 2, 3, 4, 5 oder 6 C-Atomen R(6) und R(7)
unabhängig voneinander Wasserstoff oder Alkyl mit 1, 2, 3 oder 4 C-Atomen;
R(32) und R(33)
unabhängig voneinander Wasserstoff oder Alkyl mit 1, 2, 3 oder 4 C-Atomen;
oder
R(35)
C₁-C₉-Heteroaryl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
und die jeweils anderen Substituenten R(1), R(2) und R(3)
unabhängig voneinander Alkyl mit 1, 2, 3, 4, 5, 6, 7 oder 8 C-Atomen, (CH₂)ₚR(10)
p Null, 1, 2, 3 oder 4;
R(10) Phenyl,
welches nicht substituiert ist oder substituiert mit 1 - 3 Substituenten ausgewählt aus der Gruppe bestehend aus F, Cl, -CF₃, Methyl, Methoxy, -SO₂NR(17)R(8) und -SO₂R(9); R(17) und R(8)
unabhängig voneinander Wasserstoff oder Alkyl mit 1, 2, 3 oder 4 C-Atomen;
R(9) Alkyl mit 1, 2, 3 oder 4 C-Atomen;
oder der jeweils andere Rest R(1) und R(3)
Wasserstoff,
R(4) Wasserstoff oder Alkyl mit 1, 2, 3 oder 4 C-Atomen; sowie deren pharmazeutisch verträgliche Salze;
(HOE 95/F 265 - NZ 299 739)
ap) Benzoylguanidine der Formel I 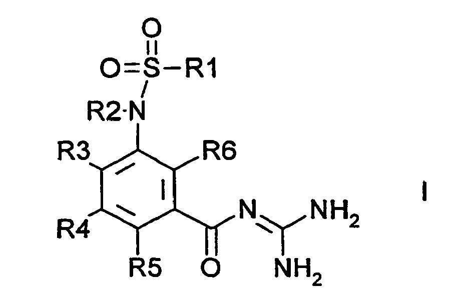 worin bedeuten:
R(1) Alkyl mit 1, 2, 3, 4, 5, 6, 7 oder 8 C-Atomen oder NR(7)R(8); R(7) und R(8)
   unabhängig voneinander Wasserstoff oder Alkyl mit 1, 2, 3, 4, 5, 6, 7 oder 8 C-Atomen
R(2) Wasserstoff, Alkyl mit 1, 2, 3, 4, 5, 6, 7 oder 8 C-Atomen oder -SO₂R(9) R(9) unabhängig wie R(1) definiert;
R(3) Wasserstoff, -SR(25), -OR(25), -NR(25)R(26) oder -CR(25)R(26)R(27); R(25) Wasserstoff, Alkyl mit 1, 2, 3, 4, 5, 6, 7 oder 8 C-Atomen, oder Phenyl,
   das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   oder
   R(25)
   -(C₁-C₉)-Heteroaryl,
   das unsubstituiert oder substituiert ist mit 1 - 3 Substituenten ausgewählt aus der Gruppe bestehend aus der Gruppe F, Cl, CF₃, CH₃, Methoxy, Hydroxy, Amino, Methylamino und Dimethylamino;
   R(26) und R(27)
   unabhängig voneinander wie R(25) definiert oder Wasserstoff oder Alkyl mit 1, 2, 3, 4, 5, 6, 7 oder 8 C-Atomen;
R(4) Wasserstoff, F, Cl, Br, I, OH, -C≡N, CF₃, Alkyl mit 1, 2, 3, 4, 5, 6, 7 oder 8 C-Atomen, Alkenyl mit 2, 3, 4, 5, 6, 7 oder 8 C-Atomen oder -(CH₂)ₘR(14);
   m Null, 1 oder 2;
   R(14) -(C₃-C₈)-Cycloalkyl oder Phenyl,
   welches nicht substituiert oder substituiert ist mit 1 - 3 Substituenten ausgewählt aus der Gruppe bestehend aus F und Cl, -CF₃, Methyl, Methoxy und -NR(15)R(16);
   R(15) und R(16)
   unabhängig voneinander Wasserstoff oder -CH₃;
R(5) und R(6)
   unabhängig voneinander Wasserstoff, Alkyl mit 1, 2, 3 oder 4 C-Atomen, F, Cl, -OR(32), -NR(33)R(34) oder CF₃;
   R(32), R(33) und R(34)
   unabhängig voneinander Wasserstoff oder Alkyl mit 1, 2, 3 oder 4 C-Atomen;
   sowie deren pharmazeutisch verträgliche Salze;
   (HOE 95/F 269 K)

aq) Benzoldicarbonsäure-diguanidide der Formel I 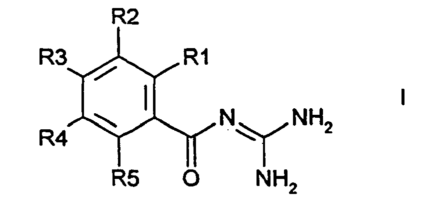 worin bedeuten:
einer der Reste R(1), R(2), R(3) und R(4)
   -CO-N=C(NH₂)₂;
   und die jeweils anderen Reste R(1), R(2), R(3) und R(4):
R(1) Wasserstoff, Alkyl mit 1, 2, 3 oder 4 C-Atomen, F, Cl, Br, I, -OR(32),
   -NR(33)R(34) oder CF₃;
   R(32), R(33) und R(34)
   unabhängig voneinander Wasserstoff oder Alkyl mit 1, 2, 3 oder 4 C-Atomen;
R(2) und R(4)
   unabhängig voneinander Wasserstoff, F, Cl, Br, I, OH, -CN, CF₃, -CO-N=C(NH₂)₂, Alkyl mit 1, 2, 3, 4, 5, 6, 7 oder 8 C-Atomen, Alkenyl mit 2, 3, 4, 5, 6, 7 oder 8 C-Atomen oder -(CH₂)ₘR(14);
   m Null, 1 oder 2;

   R(14) -(C₃-C₈)-Cycloalkyl oder Phenyl, welches nicht substituiert oder substituiert ist mit 1 - 3 Substituenten ausgewählt aus der Gruppe bestehend aus F und Cl, -CF₃, Methyl, Methoxy und -NR(15)R(16); R(15) und R(16)
   Wasserstoff oder -CH₃;
   oder
R(2) und R(4)
   unabhängig voneinander Pyrrol-1-yl, Pyrrol-2-yl oder Pyrrol-3-yl, welches nicht substituiert oder substituiert ist mit 1 - 4 Substituenten ausgewählt aus der Gruppe bestehend aus F, Cl, Br, I, -CN, (C₂-C₈)-Alkanoyl, (C₂-C₈)-Alkoxycarbonyl, Formyl, Carboxy, -CF₃, Methyl, Methoxy;
   oder
R(2) und R(4)
   unabhängig voneinander R(22)-SO₂-, R(23)R(24)N-CO-, R(28)-CO- oder R(29)R(30)N-SO₂;
   R(22) und R(28)
   unabhängig voneinander Methyl oder -CF₃;
   R(23), R(24), R(29) und R(30)
   unabhängig voneinander Wasserstoff oder Methyl;
   oder
R(2) und R(4) unabhängig voneinander -OR(35) oder -NR(35)R(36);
   R(35) und R(36)
   unabhängig voneinander Wasserstoff oder Alkyl mit 1, 2, 3, 4, 5 oder 6 C-Atomen;
   oder
   R(35) und R(36) gemeinsam 4 - 7 Methylengruppen, von denen eine CH₂-Gruppe durch Sauerstoff, -S-, -NH-, -NCH₃ oder -N-Benzyl ersetzt sein kann;
R(3) Wasserstoff, -SR(25), -OR(25), -NR(25)R(26), -CR(25)R(26)R(27);
   R(25) Wasserstoff, Alkyl mit 1, 2, 3, 4, 5, 6, 7 oder 8 C-Atomen oder Phenyl,
   das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   oder
   R(25) -(C₁-C₉)-Heteroaryl,
   das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   R(26) und R(27)
   unabhängig voneinander wie R(25) definiert oder Wasserstoff oder
   Alkyl mit 1, 2, 3, 4, 5, 6, 7 oder 8 C-Atomen;
R(5) Alkyl mit 1, 2, 3 oder 4 C-Atomen, F, Cl, Br, I, X-(CH₂)_{y}-CF₃ oder Phenyl, welches nicht substituiert oder substituiert ist mit 1 - 3 Substituenten ausgewählt aus der Gruppe bestehend aus F und Cl, -CF₃, Methyl, Methoxy und -NR(6)R(7);
   R(6) und R(7)
   unabhängig voneinander Wasserstoff oder -CH₃;
   X eine Bindung oder Sauerstoff;
   y Null, 1 oder 2; sowie deren pharmazeutisch verträgliche Salze;
   (HOE 95/F 269 BK)

ar) Benzoldicarbonsäure-diguanidide der Formel I 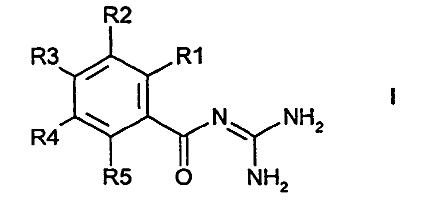 worin bedeuten:
einer der Reste R(1), R(2), R(3) und R(5)
   -CO-N=C(NH₂)₂;
   und die jeweils anderen Reste R(1), R(2), R(3) und R(5):
R(1) und R(5)
   unabhängig voneinander Wasserstoff, Alkyl mit 1, 2, 3 oder 4 C-Atomen, F, Cl, -OR(32), -NR(33)R(34) oder CF₃;
   R(32), R(33) und R(34)
   unabhängig voneinander Wasserstoff oder Alkyl mit 1, 2, 3 oder 4 C-Atomen;
R(2) Wasserstoff, F, Cl, Br, I , OH, -C°N, CF₃, -CO-N=C(NH₂)₂, Alkyl mit 1, 2, 3, 4, 5, 6, 7 oder 8 C-Atomen, Alkenyl mit 2, 3, 4, 5, 6, 7 oder 8 C-Atomen oder -(CH₂)ₘR(14);
   m Null, 1 oder 2;
   R(14) -(C₃-C₈)-Cycloalkyl oder Phenyl,
   welches nicht substituiert oder substituiert ist mit 1 - 3 Substituenten ausgewählt aus der Gruppe bestehend aus F und Cl, -CF₃, Methyl, Methoxy und -NR(15)R(16);
   R(15) und R(16)
   unabhängig voneinander Wasserstoff oder -CH₃;
   oder
R(2) R(22)-SO₂-, R(23)R(24)N-CO-, R(28)-CO- oder R(29)R(30)N-SO₂; R(22) und R(28) unabhängig voneinander Methyl oder -CF₃;
   R(23), R(24), R(29) und R(30)
   unabhängig voneinander Wasserstoff oder Methyl;
   oder
R(2) -OR(35) oder -NR(35)R(36);
   R(35) und R(36)
   unabhängig voneinander Wasserstoff oder Alkyl mit 1, 2, 3, 4, 5 oder 6 C-Atomen;
   oder
   R(35) und R(36)
   gemeinsam 4 - 7 Methylengruppen, von denen eine CH₂-Gruppe durch
   Sauerstoff, -S-, -NH-, -NCH₃ oder -N-Benzyl ersetzt sein kann;
R(3) Wasserstoff, -SR(25), -OR(25), -NR(25)R(26), -CR(25)R(26)R(27);
   R(25) Wasserstoff, Alkyl mit 1, 2, 3, 4, 5, 6, 7 oder 8 C-Atomen oder Phenyl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   oder
   R(25 -(C₁-C₉)-Heteroaryl,
   das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   R(26) und R(27)
   unabhängig voneinander wie R(25) definiert oder Wasserstoff oder Alkyl mit 1, 2, 3, 4, 5, 6, 7 oder 8 C-Atomen;
R(4) CF₃, Alkyl mit 1, 2, 3, 4, 5, 6, 7 oder 8 C-Atomen, Alkenyl mit 2, 3, 4, 5, 6, 7 oder 8 C-Atomen, -(C₃-C₈)-Cycloalkyl oder -(CH₂)ₘR(14);
   m 1 oder 2;
   R(14) -(C₃-C₈)-Cycloalkyl oder Phenyl, welches nicht substituiert oder substituiert ist mit 1 - 3 Substituenten ausgewählt aus der Gruppe bestehend aus F und Cl, -CF₃, Methyl, Methoxy und -NR(15)R(16); R(15) und R(16)
   unabhängig voneinander Wasserstoff oder -CH₃;
   oder
R(4) Phenyl,
   welches substituiert ist mit 2, 3, 4 oder fünf Substituenten ausgewählt aus der Gruppe bestehend aus F, Cl, CF₃, Methyl, Methoxy und -NR(15)R(16);
   R(15) und R(16)
   unabhängig voneinander Wasserstoff oder CH₃;
   sowie deren pharmazeutisch verträgliche Salze;
   (HOE 96/F 013)

as) Diaryldicarbonsäure-diguanidide der Formel I 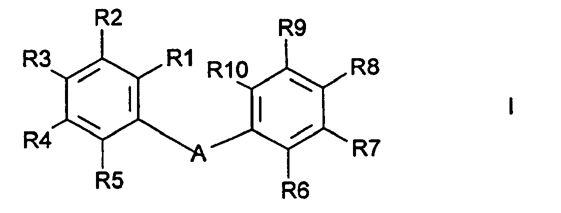 worin bedeuten:
einer der Reste R(1), R(2), R(3), R(4) und R(5)
   -CO-N=C(NH₂)₂;
   die jeweils anderen Reste R(1) und R(5)
   unabhängig voneinander Wasserstoff, Alkyl mit 1, 2, 3 oder 4 C-Atomen, F, Cl, -OR(32), -NR(33)R(34) oder CF₃;
   R(32), R(33) und R(34)
   unabhängig voneinander Wasserstoff oder Alkyl mit 1, 2, 3 oder 4 C-Atomen;
   die jeweils anderen Reste R(2) und R(4)
   unabhängig voneinander Wasserstoff, F, Cl, Br, I, OH, -CN, CF₃, -CO-N=C(NH₂)₂, Alkyl mit 1, 2, 3, 4, 5, 6, 7 oder 8 C-Atomen, Alkenyl mit 2, 3, 4, 5, 6, 7 oder 8 C-Atomen oder -(CH₂)ₘR(14);
   m Null, 1 oder 2;
   R(14) -(C₃-C₈)-Cycloalkyl oder Phenyl,
   welches nicht substituiert oder substituiert ist mit 1 - 3 Substituenten ausgewählt aus der Gruppe bestehend aus F und Cl, -CF₃, Methyl, Methoxy und -NR(15)R(16);
   R(15) und R(16)
   Wasserstoff oder -CH₃;
   oder
   die jeweils anderen Reste R(2) und R(4)
   unabhängig voneinander Pyrrol-1-yl, Pyrrol-2-yl oder Pyrrol-3-yl,
   welches nicht substituiert oder substituiert ist mit 1 - 4 Substituenten ausgewählt aus der Gruppe bestehend aus F, Cl, Br, I, -CN, (C₂-C₈)-Alkanoyl, (C₂-C₈)-Alkoxycarbonyl, Formyl, Carboxy, -CF₃, Methyl, Methoxy;
   oder
   die jeweils anderen Reste R(2) und R(4)
   R(22)-SO₂-, R(23)R(24)N-CO-, R(28)-CO- oder R(29)R(30)N-SO2; R(22) und R(28)
   unabhängig voneinander Methyl oder -CF₃;
   R(23), R(24), R(29) und R(30)
   unabhängig voneinander Wasserstoff oder Methyl;
   oder
   die jeweils anderen Reste R(2) und R(4)
   unabhängig voneinander -OR(35) oder -NR(35)R(36); R(35) und R(36)
   unabhängig voneinander Wasserstoff oder Alkyl mit 1, 2, 3, 4, 5 oder 6 C-Atomen;
   oder
   R(35) und R(36)
   gemeinsam 4 - 7 Methylengruppen, von denen eine CH₂-Gruppe durch
   Sauerstoff, -S-, -NH-, -NCH₃ oder -N-Benzyl ersetzt sein kann; der jeweils andere Rest R(3)
   Wasserstoff, -SR(25), -OR(25), -NR(25)R(26), -CR(25)R(26)R(27);
   R(25) Wasserstoff, Alkyl mit 1, 2, 3, 4, 5, 6, 7 oder 8 C-Atomen oder Phenyl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   oder
   R(25) -(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   R(26) und R(27)
   unabhängig voneinander wie R(25) definiert oder Wasserstoff oder Alkyl mit 1, 2, 3, 4, 5, 6, 7 oder 8 C-Atomen;
   einer der Reste R(6), R(7), R(8), R(9) und R(10)
   -CO-N=C(NH₂)₂;
   die jeweils anderen Reste R(6) und R(10)
   unabhängig voneinander Wasserstoff, Alkyl mit 1, 2, 3 oder 4 C-Atomen, F, Cl, -OR(132), -NR(133)R(134) oder CF₃;
   R(132), R(133) und R(134)
   unabhängig voneinander Wasserstoff oder Alkyl mit 1, 2, 3 oder 4 C-Atomen;
   die jeweils anderen Reste R(7) und R(9)
   unabhängig voneinander Wasserstoff, F, Cl, Br, I, OH, -CN, CF₃, -CO-N=C(NH₂)₂, Alkyl mit 1, 2, 3, 4, 5, 6, 7 oder 8 C-Atomen, Alkenyl mit 2, 3, 4, 5, 6, 7 oder 8 C-Atomen oder -(CH₂)ₘₘR(114);
   mm Null, 1 oder 2;
   R(114)
   -(C₃-C₈)-Cycloalkyl oder Phenyl,
   welches nicht substituiert oder substituiert ist mit 1 - 3 Substituenten ausgewählt aus der Gruppe bestehend aus F und Cl, -CF₃, Methyl, Methoxy und -NR(115)R(116); R(115) und R(116)
   Wasserstoff oder -CH₃;
   oder
   die jeweils anderen Reste R(7) und R(9)
   unabhängig voneinander Pyrrol-1-yl, Pyrrol-2-yl oder Pyrrol-3-yl,
   welches nicht substituiert oder substituiert ist mit 1 - 4 Substituenten ausgewählt aus der Gruppe bestehend aus F, Cl, Br, I, -CN, (C₂-C₈)-Alkanoyl, (C₂-C₈)-Alkoxycarbonyl, Formyl, Carboxy, -CF₃, Methyl und Methoxy;
   oder
   die jeweils anderen Reste R(7) und R(9)
   R(122)-SO₂-, R(123)R(124)N-CO-, R(128)-CO- oder R(129)R(130)N-SO₂; R(122) und R(128)
   unabhängig voneinander Methyl oder -CF₃;
   R(123), R(124), R(129) und R(130)
   unabhängig voneinander Wasserstoff oder Methyl;
   oder
   die jeweils anderen Reste R(7) und R(9)
   unabhängig voneinander -OR(135) oder -NR(135)R(136);
   R(135) und R(136)
   unabhängig voneinander Wasserstoff oder Alkyl mit 1, 2, 3, 4, 5 oder 6 C-Atomen;
   oder
   R(135) und R(136)
   gemeinsam 4 - 7 Methylengruppen, von denen eine CH₂-Gruppe durch Sauerstoff, -S-, -NH-, -NCH₃ oder -N-Benzyl ersetzt sein kann;
   der jeweils andere Rest R(8)
   Wasserstoff, -SR(125), -OR(125), -NR(125)R(126) oder -CR(125)R(126)R(127);
   R(125)
   Wasserstoff, Alkyl mit 1, 2, 3, 4, 5, 6, 7 oder 8 C-Atomen oder Phenyl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   oder
   R(125)
   -(C₁-C₉)-Heteroaryl,
   das unsubstituiert oder substituiert ist mit 1 - 3 Substituenten ausgewählt aus der Gruppe bestehend aus der Gruppe F, Cl, CF₃, CH₃, Methoxy, Hydroxy, Amino, Methylamino und Dimethylamino;
   R(126) und R(127)
   unabhängig voneinander wie R(125) definiert oder Wasserstoff oder Alkyl mit 1, 2, 3, 4, 5, 6, 7 oder 8 C-Atomen;
A abwesend, -NR(11)-CO-, -NR(12)-CO-NR(13)-, -NR(17)-CO-NR(18)-SO₂-, - NR(19)-SO₂-, -SO₂-NR(19)-SO₂-, -SO₂-NR(19)-CO-, -O-CO-NR(19)-SO₂- oder -CR(20)=CR(21)-;
   R(11), R(12), R(13), R(17), R(18), R(19), R(20) und R(21) unabhängig voneinander Wasserstoff oder Alkyl mit 1, 2, 3, 4, 5, 6, 7 oder 8 C-Atomen
   sowie deren pharmazeutisch verträgliche Salze;
   (HOE 96/F 026)

at) Substituierte Thiophenylalkenylcarbonsäureguanidide der Formel I 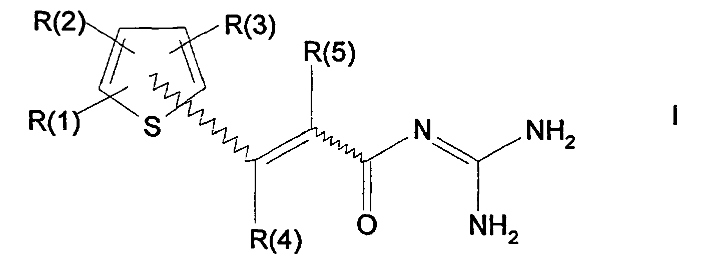 worin bedeuten:
mindestens einer der Substituenten R(1), R(2) und R(3)
   -Op-(CH₂)ₛ-C_{q}F_{2q+1}, R(40)CO- oder R(31)SOₖ-;
   p Null oder 1;
   s Null, 1, 2, 3 oder 4;
   q 1, 2, 3, 4, 5, 6, 7 oder 8;
   k Null, 1 oder 2;
   R(40) Alkyl mit 1, 2, 3, 4, 5, 6, 7 oder 8 C-Atomen, Perfluoralkyl mit 1, 2, 3, 4,
   5, 6, 7 oder 8 C-Atomen, Cycloalkyl mit 3, 4, 5, 6, 7 oder 8 C-Atomen, oder Phenyl,
   das nicht substituiert oder substituiert ist mit 1 - 3 Substituenten ausgewählt aus der Gruppe bestehend aus F, Cl, CF₃, Methyl und Methoxy;
   R(31) Alkyl mit 1, 2, 3, 4, 5, 6, 7 oder 8 C-Atomen, Perfluoralkyl mit 1, 2, 3, 4,
   5, 6, 7 oder 8 C-Atomen, Cycloalkyl mit 3, 4, 5, 6, 7 oder 8 C-Atomen, oder Phenyl,
   das nicht substituiert oder substituiert ist mit 1 - 3 Substituenten ausgewählt aus der Gruppe bestehend aus F, Cl, CF₃, Methyl oder Methoxy;
   oder
   R(31) NR(41)R(42);
   R(41) und R(42) unabhängig voneinander Wasserstoff, Alkyl mit 1, 2, 3 oder 4 C-Atomen, Perfluoralkyl mit 1, 2, 3 oder 4 C-Atomen, oder
   R(41) und R(42)
   gemeinsam 4 oder 5 Methylengruppen, von denen eine CH₂-Gruppe durch Sauerstoff, S, NH, N-CH₃ oder N-Benzyl ersetzt sein kann;
   und die jeweils anderen Substituenten R(1), R(2) und R(3)
   unabhängig voneinander H, F, Cl, Br, I, CN, -Oₙₐ-CₘₐH₂ₘₐ₊₁ oder - O_{ga}CᵣₐH₂ᵣₐR(10);
   na Null oder 1;
   ma Null, 1, 2, 3, 4, 5, 6, 7 oder 8;
   ga Null oder 1;
   ra Null, 1, 2, 3 oder 4;
   R(10) Cycloalkyl mit 3, 4, 5, 6, 7 oder 8 C-Atomen oder Phenyl, wobei das Phenyl nicht substituiert oder substituiert ist mit 1 - 3 Substituenten ausgewählt aus der Gruppe bestehend aus F, Cl, CF₃, Methyl und Methoxy;
R(4) und R(5) unabhängig voneinander Wasserstoff, F, Cl, Br, I, CN, Alkyl mit 1, 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen oder Phenyl,
   das nicht substituiert oder substituiert ist mit 1 - 3 Substituenten ausgewählt aus der Gruppe bestehend aus F, Cl, CF₃, Methyl, Methoxy und NR(14)R(15);
   R(14) und R(15)
   unabhängig voneinander H, Alkyl mit 1, 2, 3 oder 4 C-Atomen oder Perfluoralkyl mit 1, 2, 3 oder 4 C-Atomen;
   sowie deren pharmazeutisch verträgliche Salze;
   (HOE 96/F 032)

au) Ortho-substituierte Benzoylguanidine der Formel I 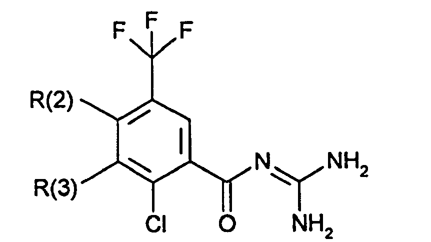 worin bedeuten:
R(2) und R(3)
   unabhängig voneinander Wasserstoff, Cl, Br, I, (C₁-C₈)-Alkyl, (C₃-C₈)-Cycloalkyl oder -OR(5);
   R(5) (C₁-C₈)-Alkyl oder -C_{d}H_{2d}-(C₃-C₈)-Cycloalkyl;
   d Null, 1 oder 2;
   wobei stets einer der beiden Substituenten R(2) und R(3) Wasserstoff ist, jedoch nicht beide Substituenten R(2) und R(3) gleichzeitig Wasserstoff sind,
   sowie deren pharmazeutisch verträgliche Salze;
   (HOE 96/F 042)

av) Benzoylguanidine der Formel I 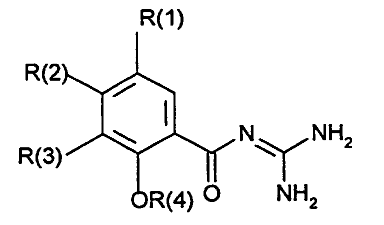 worin bedeuten:
R(1) H, F, Cl, Br, I, CN, NO₂, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder Xₐ-(CH₂)_{b}-(CF₂)_{c}-CF₃;
   X Sauerstoff, S, NR(5),
   a Null oder 1;
   b Null, 1 oder 2;
   c Null, 1, 2 oder 3;
   R (5) H, Alkyl mit 1, 2, 3 oder 4 C-Atomen oder -C_{d}H_{2d}R(6);
   d Null, 1, 2, 3 oder 4;
   R(6) Cycloalkyl mit 3, 4, 5, 6, 7 oder 8 C-Atomen, Phenyl, Biphenylyl oder Naphthyl,
   wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(7)R(8);
   R(7) und R(8)
   unabhängig H oder Alkyl mit 1, 2, 3 oder 4 C-Atomen;
   oder
R(1) -SR(10), -OR(10) oder -CR(10)R(11)R(12);
   R(10) -C_{f}H₂f-Cycloalkyl mit 3, 4, 5, 6, 7 oder 8 C-Atomen im Cycloalkylring, oder Phenyl,
   wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   f Null, 1 oder 2;
   R(11) und R(12)
   unabhängig voneinander wie R(10) definiert oder Wasserstoff oder Alkyl mit 1, 2, 3 oder 4 C-Atomen;
   oder
R(1) Phenyl, Naphthyl, Biphenylyl oder Heteroaryl mit 1, 2, 3, 4, 5, 6, 7, 8 oder 9 C-Atomen, letzteres über ein C- oder ein N-Atom des Rings verknüpft,
   die jeweils unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
   oder
R(1) -SR(13), -OR(13), -NHR(13), -NR(13)R(14), -CHR(13)R(15), -C[R(15)R(16)OH], -C≡CR(18), -C[R(19)]=CHR(18), -C[R(20)R(21)]ₖ-(CO)-[CR(22)R(23)]ₗ-R(24),
   k Null, 1, 2, 3 oder 4;
   l Null, 1, 2, 3 oder 4;
   R(13) und R(14)
   gleich oder verschieden -(CH₂)_{g}-(CHOH)ₕ-(CH₂)ᵢ-(CHOH)ⱼ-R(17) oder -(CH₂)_{g}-O-(CH₂-CH₂O)ₕ-R(24);
   R(17) Wasserstoff oder Methyl,
   g, h und i
   gleich oder verschieden Null, 1, 2, 3 oder 4;
   j 1, 2, 3 oder 4;
   R(15) und R(16)
   gleich oder verschieden Wasserstoff, Alkyl mit 1, 2, 3, 4, 5 oder 6 C-Atomen, oder zusammen mit dem sie tragenden Kohlenstoffatom Cycloalkyl mit 3, 4, 5, 6, 7 oder 8 C-Atomen;
   R(18)
   Phenyl,
   das unsubstituiert oder substituiert ist mit 1 - 3 Substituenten ausgewählt aus der Gruppe bestehend aus F, Cl, CF₃, Methyl, Methoxy und NR(25)R(26);
   R(25) und R(26)
   H oder Alkyl mit 1, 2, 3 oder 4 C-Atomen;
   oder
   R(18) Heteroaryl mit 1, 2, 3, 4, 5, 6, 7, 8 oder 9 Kohlenstoffatomen, das unsubstituiert oder wie Phenyl substituiert ist;
   oder
   R(18) Alkyl mit 1, 2, 3, 4, 5 oder 6 C-Atomen,
   das unsubstituiert oder mit 1 - 3 OH substituiert ist;
   oder
   R (18)
   Cycloalkyl mit 3, 4, 5, 6, 7 oder 8 C-Atomen;
   R(19), R(20), R(21), R(22) und R(23)
   gleich oder verschieden Wasserstoff oder Methyl;
   R(24) H, Alkyl mit 1, 2, 3, 4, 5 oder 6 C-Atomen, Cycloalkyl mit 3, 4, 5, 6, 7
   oder 8 C-Atomen oder -CₘH₂m-R(18);
   m 1, 2, 3 oder 4;
R(2) und R(3)
   wie R(1) definiert;
R(4) Alkyl mit 1, 2, 3 oder 4 C-Atomen;
   sowie deren pharmazeutisch verträgliche Salze;
   (HOE 96/F 043)

aw) Ortho-substituierte Benzoylguanidine der Formel I 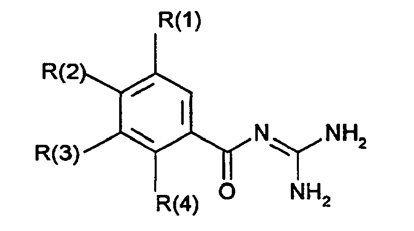 worin bedeuten:
R(1) H, F, Cl, Br, I, CN, NO₂, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder Xₐ-(CH₂)_{b}-(CF₂)_{c}-CF₃;
   X Sauerstoff, S, NR(5),
   a Null oder 1;
   b Null, 1 oder 2;
   c Null, 1, 2 oder 3;
   R (5) H, Alkyl mit 1, 2, 3 oder 4 C-Atomen oder -C_{d}H_{2d}R(6);
   d Null, 1, 2, 3 oder 4;
   R(6) Cycloalkyl mit 3, 4, 5, 6, 7 oder 8 C-Atomen, Phenyl, Biphenylyl oder Naphthyl,
   wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(7)R(8);
   R(7) und R(8)
   unabhängig H oder Alkyl mit 1, 2, 3 oder 4 C-Atomen;
   oder
R(1) -SR(10), -OR(10) oder -CR(10)R(11)R(12);
   R(10) -C_{f}H_{2f}-Cycloalkyl mit 3, 4, 5, 6, 7 oder 8 C-Atomen im Cycloalkylring, oder Phenyl,
   wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   f Null, 1 oder 2;
   R(11) und R(12)
   unabhängig voneinander wie R(10) definiert oder Wasserstoff oder Alkyl mit 1, 2, 3 oder 4 C-Atomen;
   oder
R(1) Phenyl, Naphthyl, Biphenylyl oder Heteroaryl mit 1, 2, 3, 4, 5, 6, 7, 8 oder 9 C-Atomen, letzteres über ein C- oder ein N-Atom des Rings verknüpft,
   die jeweils unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
   oder
R(1) -SR(13), -OR(13), -NHR(13), -NR(13)R(14), -CHR(13)R(15), -C[R(15)R(16)OH], -C≡CR(18), -C[R(19)]=CHR(18), C[R(20)R(21)]ₖ-(CO)-[CR(22)R(23)]ₗ-R(24),
   k Null, 1, 2, 3 oder 4;
   l Null, 1, 2, 3 oder 4;
   R(13) und R(14)
   gleich oder verschieden -(CH₂)_{g}-(CHOH)ₕ-(CH₂)ᵢ-(CHOH)ⱼ-R(17) oder -(CH₂)_{g}-O-(CH₂-CH₂O)ₕ-R(24);
   R(17) Wasserstoff oder Methyl,
   g, h und i
   gleich oder verschieden Null, 1, 2, 3 oder 4;
   j 1, 2, 3 oder 4;
R(15) und R(16)
   gleich oder verschieden Wasserstoff, Alkyl mit 1, 2, 3, 4, 5 oder 6 C-Atomen, oder zusammen mit dem sie tragenden Kohlenstoffatom Cycloalkyl mit 3, 4, 5, 6, 7 oder 8 C-Atomen;
   R(18)
   Phenyl,
   das unsubstituiert oder substituiert ist mit 1 - 3 Substituenten ausgewählt aus der Gruppe bestehend aus F, Cl, CF₃, Methyl, Methoxy und NR(25)R(26);
   R(25) und R(26)
   H oder Alkyl mit 1, 2, 3 oder 4 C-Atomen;
   oder
   R(18) Heteroaryl mit 1, 2, 3, 4, 5, 6, 7, 8 oder 9 Kohlenstoffatomen,
   das unsubstituiert oder wie Phenyl substituiert ist;
   oder
   R(18) Alkyl mit 1, 2, 3, 4, 5 oder 6 C-Atomen,
   das unsubstituiert oder mit 1 - 3 OH substituiert ist;
   oder
   R (18)
   Cycloalkyl mit 3, 4, 5, 6, 7 oder 8 C-Atomen;
   R(19), R(20), R(21), R(22) und R(23)
   gleich oder verschieden Wasserstoff oder Methyl;
   R(24) H, Alkyl mit 1, 2, 3, 4, 5 oder 6 C-Atomen, Cycloalkyl mit 3, 4, 5, 6, 7
   oder 8 C-Atomen oder -CₘH₂ₘ-R(18);
   m 1, 2, 3 oder 4;
   einer der beiden Substituenten R(2) und R(3)
   Hydroxyl;
   und
   der jeweils andere der Substituenten R(2) und R(3)
   definiert ist wie R(1);
R(4) Alkyl mit 1, 2, 3 oder 4 C-Atomen; Alkoxy mit 1, 2, 3 oder 4 C-Atomen, F, Cl, Br, I oder -(CH₂)ₙ-(CF₂)ₒ-CF₃;
   n Null oder 1;
   o Null oder 1;
   sowie deren pharmazeutisch verträgliche Salze.
   (HOE 96/F 135)

ax) Bis-ortho-substituierte Benzoylguanidine der Formel I 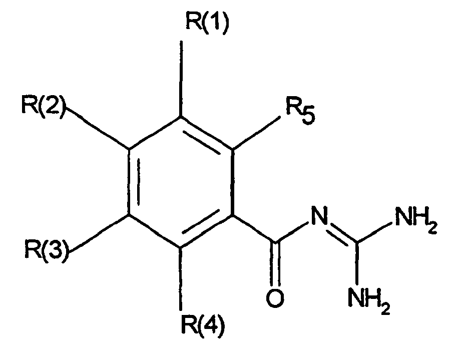 worin bedeuten:
R(1), R(2) und R(3)
   unabhängig voneinander R(10)-SOₐ- oder R(14)R(15)N-SO₂-;
   a Null, 1 oder 2,
   R(10), R(14) und R(15) unabhängig voneinander Alkyl mit 1, 2, 3, 4, 5, 6, 7 oder 8 C-Atomen, Perfluoralkyl mit 1, 2, 3, 4, 5, 6, 7 oder 8 C-Atomen, Alkenyl mit 3, 4, 5 oder 6 C-Atomen oder -C_{ab}H_{2ab}-R(16);
   ab Null, 1, 2, 3 oder 4;
   R(16) Cycloalkyl mit 3, 4, 5, 6 oder 7 C-Atomen oder Phenyl,
   welches nicht substituiert ist oder substituiert mit 1 - 3 Substituenten ausgewählt aus der Gruppe bestehend aus F, Cl, CF₃, Methyl, Methoxy und NR(17)R(18);
   R(17) und R(18)
   unabhängig voneinander Wasserstoff, CF₃ oder Alkyl mit 1, 2, 3 oder 4 C-Atomen;
   oder
   R(14) und R(15)
   gemeinsam 4 oder 5 Methylengruppen, von denen eine CH₂-Gruppe durch Sauerstoff, Schwefel, NH, N-CH₃ oder N-Benzyl ersetzt sein kann;
   oder
   R(14) und R(15)
   Wasserstoff;
   oder
R(1), R(2) und R(3)
   unabhängig voneinander SR(21), -OR(22), -NR(23)R(24) oder -CR(25)R(26)R(27);
   R(21), R(22), R(23) und R(25)
   unabhängig voneinander -C_{b}H_{2b}-(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   b Null, 1 oder 2;
   R(24), R(26) und R(27)
   unabhängig voneinander Wasserstoff, Alkyl mit 1, 2, 3 oder 4 C-Atomen oder Perfluoralkyl mit 1, 2, 3 oder 4 C-Atomen;
   oder
R(1), R(2) und R(3) unabhängig voneinander Wasserstoff, F, Cl, Br, I, CN, -(Xa)_{dg}-C_{da}H_{2da+1},-(Xb)_{dh}-(CH₂)_{db}-C_{de}F_{2de+1}, Alkenyl mit 3, 4, 5, 6, 7 oder 8 C-Atomen oder -C_{df}H_{2df}R(30);
   (Xa) Sauerstoff, Schwefel oder NR(33);
   R(33) Wasserstoff, Alkyl mit 1, 2, 3 oder 4 C-Atomen oder Perfluoralkyl mit 1, 2, 3 oder 4 C-Atomen;
   dg Null oder 1;
   (Xb) Sauerstoff, Schwefel oder NR(34);
   R(34) Wasserstoff, Alkyl mit 1, 2, 3 oder 4 C-Atomen oder Perfluoralkyl mit 1, 2, 3 oder 4 C-Atomen;
   dh Null oder 1;
   da Null, 1, 2, 3, 4, 5, 6, 7 oder 8;
   db Null, 1, 2, 3 oder 4;
   de Null, 1, 2, 3, 4, 5, 6 oder 7;
   df Null, 1, 2, 3 oder 4;
   R(30) Cycloalkyl mit 3, 4, 5, 6, 7 oder 8 C-Atomen, Phenyl, Biphenylyl oder Naphthyl,
   wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(31)R(32);
   R(31) und R(32)
   Wasserstoff, Alkyl mit 1, 2, 3 oder 4 C-Atomen oder Perfluoralkyl mit 1, 2, 3 oder 4 C-Atomen;
   oder
R(1), R(2) und R(3)
   unabhängig voneinander NR(40)R(41) oder -(Xe)-(CH₂)_{eb}R(45); R(40) und R(41)
   unabhängig voneinander Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen oder (CH₂)ₑ-R(42);
   e Null, 1, 2, 3 oder 4;
   R(42) Cycloalkyl mit 3, 4, 5, 6 oder 7 C-Atomen oder Phenyl,
   welches nicht substituiert ist oder substituiert mit 1 - 3 Substituenten ausgewählt aus der Gruppe bestehend aus F, Cl, CF₃, Methyl, Methoxy und NR(43)R(44);
   R(43) und R(44)
   unabhängig voneinander Wasserstoff, CF₃ oder Alkyl mit 1, 2, 3 oder 4 C-Atomen;
   oder
   R(40) und R(41)
   gemeinsam 4 oder 5 Methylengruppen, von denen eine CH₂-Gruppe durch Sauerstoff, Schwefel, NH, N-CH₃ oder N-Benzyl ersetzt sein kann;
   (Xe) Sauerstoff, Schwefel oder NR(47);
   R(47) Wasserstoff, Alkyl mit 1, 2, 3 oder 4 C-Atomen oder Perfluoralkyl mit 1, 2, 3 oder 4 C-Atomen;
   eb Null, 1, 2, 3 oder 4;
   R(45) Cycloalkyl mit 3, 4, 5, 6 oder 7 C-Atomen oder Phenyl,
   welches nicht substituiert ist oder substituiert mit 1 - 3 Substituenten ausgewählt aus der Gruppe bestehend aus F, Cl, CF₃, Methyl, Methoxy, NR(50)R(51) und -(Xfa)-(CH₂)_{ed}-(Xfb)R(46);
   Xfa CH₂, Sauerstoff, Schwefel oder NR(48);
   Xfb Sauerstoff, Schwefel oder NR(49);
   R(48), R(49), R(50) und R(51) unabhängig voneinander Wasserstoff, Alkyl mit 1, 2, 3 oder 4 C-Atomen oder Perfluoralkyl mit 1, 2, 3 oder 4 C-Atomen;
   ed 1, 2, 3 oder 4;
   R(46) Wasserstoff, Alkyl mit 1, 2, 3 oder 4 C-Atomen oder Perfluoralkyl mit 1, 2, 3 oder 4 C-Atomen;
   oder
R(1), R(2) und R(3)
   unabhängig voneinander -CHR(52)R(53);
   R(52) -(CH₂)_{g}-(CHOH)ₕ-(CH)ᵢ-(CHOH)ₖ-R(54) oder -(CH₂)_{g}-O-(CH₂-CH₂O)ₕ-R(54);
   R(54) Wasserstoff oder Methyl;
   g, h, i
   gleich oder verschieden Null, 1, 2, 3 oder 4;
   k 1, 2, 3 oder 4;
   R(53) Wasserstoff oder Alkyl mit 1, 2, 3 oder 4 C-Atomen;
   oder
R(1), R(2) und R(3)
   unabhängig voneinander -C(OH)R(55)R(56);
   R(55) und R(56)
   gleich oder verschieden Wasserstoff oder Alkyl mit 1, 2, 3 oder 4 C-Atomen;
   oder
   R(55) und R(56)
   gemeinsam Cycloalkyl mit 3, 4, 5 oder 6 C-Atomen;
   oder
   R(55) -CH₂OH;
   und
R(4) und R(5)
   unabhängig voneinander Alkyl mit 1, 2, 3 oder 4 C-Atomen, Alkoxy mit 1, 2, 3 oder 4 C-Atomen, OH, F, Cl, Br, I, CN, -Oₙ-(CH₂)ₒ-(CF₂)ₚ-CF₃;
   n Null oder 1;
   o Null, 1 oder 2;
   p Null, 1 oder 2;
   sowie deren pharmazeutisch verträgliche Salze.
   (96/F 136)

ay) Substituierte 1-Naphthoylguanidine der Formel I 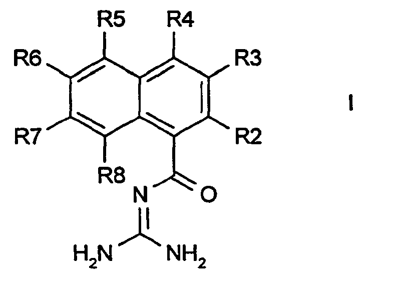 worin bedeuten
R2, R3, R4, R5, R6, R7 und R8
   unabhängig voneinander H, F, Cl, Br, I, CN, NO₂, CF₃, C₂F₅ oder XₐY_{b}Z;
   X O, S, NR(10), CR(11)R(12), C=O, C(=O)NR(10), C(=O)O, SO, SO₂, SO₂NR(10), OC=O, NR(10)C=O oder NR(10)SO₂,
   wobei die Verknüpfung mit dem Naphthalinring jeweils über das links stehende Atom erfolgt;
   R(10), R(11) und R(12)
   unabhängig voneinander H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
   a Null oder 1;
   Y Alkylen mit 1, 2, 3, 4, 5, 6, 7 oder 8 CH₂-Gruppen, wobei eine dieser CH₂-Gruppen durch O, S, NR(13) oder o-, p-oder m-Phenylen ersetzt sein kann;
   R(13) H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;
   b Null oder 1;
   Z H, Alkyl mit 1, 2, 3, 4, 5, 6 oder 7 C-Atomen, Cycloalkyl mit 3, 4, 5, 6 oder 7 C-Atomen, C(=O)R(15), SO₂R(15), NR(16)R(17) oder Phenyl, das nicht substituiert oder substituiert ist mit 1 - 3 Substituenten ausgewählt aus der Gruppe bestehend aus F, Cl, Br, CF₃, Methyl, Methoxy, NR(21)R(22);
   R(21) und R(22)
   unabhängig voneinander H oder Alkyl mit 1, 2, 3 oder 4 C-Atomen oder Perfluoralkyl mit 1, 2, 3 oder 4 C-Atomen;
   R(15) N=C(NH₂)₂, NR(18)R(19), N(CH₂)_{c}NR(18)R(19) oder OR(20);
   c 2 oder 3;
   R(18) und R(19)
   unabhängig voneinander H, Alkyl mit 1, 2, 3, 4, 5, 6, 7 oder 8 C-Atomen oder Perfluoralkyl mit 1, 2, 3 oder 4 C-Atomen;
   oder
   R(18) und R(19)
   gemeinsam 4 oder 5 Methylengruppen,
   von denen eine CH₂-Gruppe durch Sauerstoff, S, NH, N-CH₃, N-Benzyl oder N-(p-Chlorphenyl) ersetzt sein kann;
   R(20) H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
   R(16) und R(17)
   unabhängig voneinander H, Alkyl mit 1, 2, 3, 4, 5, 6, 7 oder 8 C-Atomen oder Perfluoralkyl mit 1, 2, 3 oder 4 C-Atomen;
   oder
   R(16) und R(17)
   gemeinsam 4 oder 5 Methylengruppen, von denen eine CH₂-Gruppe durch Sauerstoff, S, NH, N-CH₃, N-Benzyl oder N-(p-Chlorphenyl) ersetzt sein kann;
   oder
   Z ein N-haltiger Heterocyclus mit 1, 2, 3, 4, 5, 6, 7, 8 oder 9 C-Atomen, wobei der N-haltige Heterocyclus über N oder C verknüpft ist und nicht substituiert oder substituiert ist mit 1 - 3 Substituenten ausgewählt aus der Gruppe bestehend aus F, Cl, Br, CF₃, Methyl, Methoxy und NR(21)R(22);
   wobei jedoch für den Fall, daß R(4) für einen Alkoxyrest steht, mindestens einer der Substituenten R(2), R(3), R(5), R(6), R(7) und R(8) ungleich Wasserstoff ist;
   sowie deren pharmazeutisch verträgliche Salze.
   (96/F 137)

az) Substituierte 2-Naphthoylguanidine der Formel I 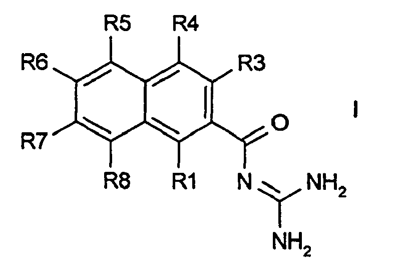 worin bedeuten:
mindestens einer der Substituenten R1, R3, R4, R5, R6, R7 und R8 XYₐWZ oder X'YₐWZ';
   X O,S, NR(10) oder CR(11)R(12);
   R(10), R(11) und R(12)
   unabhängig voneinander H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
   Y Alkylen mit 1, 2, 3, 4, 5, 6, 7 oder 8 CH₂-Gruppen, wobei eine dieser CH₂-Gruppen durch O, S, NR(13) oder o-, p-oder m-Phenylen ersetzt sein kann;
   R(13) H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;
   a Null oder 1;
   W CH₂, SO₂, S(=O)(=NH) oder - falls W nicht unmittelbar auf ein Heteroatom der Gruppe XYₐ folgt - auch O oder NR(14); R(14) H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;
   Z C(=O)R(15), SO₂R(15) oder - falls W nicht O oder NR(14) bedeutet - auch NR(16)R(17);
   R(15)
   N=C(NH₂)₂, NR(18)R(19), N(CH₂)_{b}NR(18)R(19) oder OR(20);
   b 2 oder 3;
   R(18) und R(19)
   unabhängig voneinander H, Alkyl mit 1, 2, 3, 4, 5, 6, 7 oder 8 C-Atomen oder Perfluoralkyl mit 1, 2, 3 oder 4 C-Atomen;
   oder
   R(18) und R(19)
   gemeinsam 4 oder 5 Methylengruppen,
   von denen eine CH₂-Gruppe durch Sauerstoff, S, NH, N-CH₃, N-Benzyl oder N-(p-Chlorphenyl) ersetzt sein kann;
   R(20)
   H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
   R(16) und R(17)

   unabhängig voneinander H, Alkyl mit 1, 2, 3, 4, 5, 6, 7 oder 8 C-Atomen oder Perfluoralkyl mit 1, 2, 3 oder 4 C-Atomen;
   oder
   R(16) und R(17)
   gemeinsam 4 oder 5 Methylengruppen, von denen eine CH₂-Gruppe durch Sauerstoff, S, NH, N-CH₃, N-Benzyl oder N-(p-Chlorphenyl) ersetzt sein kann;
   X' C=O, C(=O)NR(30), C(=O)O, SO, SO₂, SO₂NR(30), OC=O,
   NR(30)C=O oder NR(30)SO₂,
   wobei die Verknüpfung mit dem Naphthalinring jeweils über das links stehende Atom erfolgt;
   R(30) H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
   Z' C(=O)R(15), SO₂R(15), einen N-haltigen Heterocyclus mit 1, 2, 3, 4, 5, 6, 7, 8 oder 9 C-Atomen,
   wobei der N-haltige Heterocyclus über N oder C verknüpft ist und nicht substituiert oder substituiert ist mit 1 - 3 Substituenten ausgewählt aus der Gruppe bestehend aus F, Cl, Br, CF₃, Methyl, Methoxy und NR(21)R(22);
   R(21) und R(22)
   unabhängig voneinander H, Alkyl mit 1, 2, 3 oder 4 C-Atomen oder Perfluoralkyl mit 1, 2, 3 oder 4 C-Atomen;
   R(15)
   N=C(NH₂)₂, NR(18)R(19), N(CH₂)_{b}NR(18)R(19) oder OR(20); R(18) und R(19)
   unabhängig voneinander H, Alkyl mit 1, 2, 3, 4, 5, 6, 7 oder 8 C-Atomen oder Perfluoralkyl mit 1, 2, 3 oder 4 C-Atomen;
   oder
   R(18) und R(19)
   gemeinsam 4 oder 5 Methylengruppen,
   von denen eine CH₂-Gruppe durch Sauerstoff, S, NH, N-CH₃, N-Benzyl oder N-(p-Chlorphenyl) ersetzt sein kann;
   b 2 oder 3;
   R(20) H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen; oder
   Z' - falls W nicht O oder NR(14) bedeutet - NR(16)R(17);
   R(16) und R(17)
   unabhängig voneinander H, Alkyl mit 1, 2, 3, 4, 5, 6, 7 oder 8 C-Atomen oder Perfluoralkyl mit 1, 2, 3 oder 4 C-Atomen;
   oder
   R(16) und R(17)
   gemeinsam 4 oder 5 Methylengruppen,
   von denen eine CH₂-Gruppe durch Sauerstoff, S, NH, N-CH₃, N-Benzyl oder N-(p-Chlorphenyl) ersetzt sein kann;
   und die jeweils übrigen Substituenten R1, R3, R4, R5, R6, R7 und R8, die noch nicht durch die zuvor gegebenen Definitionen vergeben sind, unabhängig voneinander H, F, Cl, Br, I, CN, NO₂, CF₃, C₂F₅ oder VₚQ_{q}U;
   V O, S, SO, SO₂, NR(60), OC=O, C=O, C(=O)NR(60), C(=O)O oder CR(66)R(67);
   R(60), R(66) und R(67)
   unabhängig voneinander H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
   p Null oder 1;
   Q Alkylen mit 1, 2, 3, 4, 5, 6, 7 oder 8 CH₂-Gruppen, wobei eine dieser CH₂-Gruppen durch O, S, NR(68) oder o-, p- oder m-Phenylen ersetzt sein kann;
   R(68)
   H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;
   q Null oder 1;
   U H, Alkyl mit 1, 2, 3, 4, 5, 6 oder 7 C-Atomen, Cycloalkyl mit 3, 4, 5, 6
   oder 7 C-Atomen, C(=O)R(65), SO₂R(65), NR(61)R(62) oder Phenyl, das nicht substituiert oder substituiert ist mit 1 - 3 Substituenten ausgewählt aus der Gruppe bestehend aus F, Cl, Br, CF₃, Methyl, Methoxy und NR(63)R(64); R(63) und R(64)
   unabhängig voneinander H, Alkyl mit 1, 2, 3 oder 4 C-Atomen oder Perfluoralkyl mit 1, 2, 3 oder 4 C-Atomen;
   R(65) N=C(NH₂)₂, NR(61)R(62) oder OR(60);
   R(61) und R(62)
   unabhängig voneinander H, Alkyl mit 1, 2, 3, 4, 5, 6, 7 oder 8 C-Atomen oder Perfluoralkyl mit 1, 2, 3 oder 4 C-Atomen;
   oder
   R(61) und R(62)
   gemeinsam 4 oder 5 Methylengruppen,
   von denen eine CH₂-Gruppe durch Sauerstoff, S, NH, N-CH₃, N-Benzyl oder N-(p-Chlorphenyl) ersetzt sein kann;
   oder
   U einen N-haltigen Heterocyclus mit 1, 2, 3, 4, 5, 6, 7, 8 oder 9 C-Atomen,
   wobei der N-haltige Heterocyclus über N oder C verknüpft ist und nicht substituiert oder substituiert ist mit 1 - 3 Substituenten ausgewählt aus der Gruppe bestehend aus F, Cl, Br, CF₃, Methyl, Methoxy und NR(63)R(64);
   wobei jedoch mindestens einer der Substituenten R5, R6, R7 und R8 ungleich Wasserstoff ist; sowie deren pharmazeutisch verträgliche Salze.
   (96/F 141)

ba) Ortho-substituierte Benzoylguanidine der Formel I 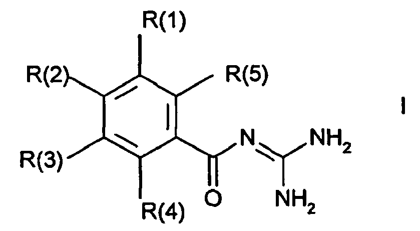 worin bedeuten:
R(1) H, F, Cl, Br, I, CN, NO₂, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder Xₐ-(CH₂)_{b}-(CF₂)_{c}-CF₃;
   X Sauerstoff, Schwefel oder NR(9),
   a Null oder 1;
   b Null, 1 oder 2;
   c Null, 1, 2 oder 3;
   R(9) H, Alkyl mit 1, 2, 3 oder 4 C-Atomen oder -C_{d}H_{2d}R(6);
   d Null, 1, 2, 3 oder 4;
   R(6) Cycloalkyl mit 3, 4, 5, 6, 7 oder 8 C-Atomen, Phenyl, Biphenylyl oder Naphthyl,
   wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(7)R(8);
   R(7) und R(8)
   unabhängig H oder Alkyl mit 1, 2, 3 oder 4 C-Atomen;
   oder
R(1) -SR(10), -OR(10) oder -CR(10)R(11)R(12);
   R(10) -C_{f}H_{2f}-Cycloalkyl mit 3, 4, 5, 6, 7 oder 8 C-Atomen im Cycloalkylring, Heteroaryl mit 1, 2, 3, 4, 5, 6, 7, 8 oder 9 C-Atomen oder Phenyl,
   wobei Heteroaryl und Phenyl unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
   f Null, 1 oder 2;
   R(11) und R(12)
   unabhängig voneinander wie R(10) definiert oder Wasserstoff oder Alkyl mit 1, 2, 3 oder 4 C-Atomen;
   oder
R(1) Phenyl, Naphthyl, Biphenylyl oder Heteroaryl mit 1, 2, 3, 4, 5, 6, 7, 8 oder 9 C-Atomen, letzteres über ein C- oder ein N-Atom des Rings verknüpft,
   die jeweils unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
   oder
R(1) -SR(13), -OR(13), -NHR(13), -NR(13)R(14), -CHR(13)R(15), -C[R(15)R(16)]OH, -C≡CR(18), -C[R(19)]=CHR(18), -C[R(20)R(21)]ₖ-(CO)-[CR(22)R(23)]ₗ-R(24),
   k Null, 1, 2, 3 oder 4;
   l Null, 1, 2, 3 oder 4;
   R(13) und R(14)
   gleich oder verschieden -(CH₂)_{g}-(CHOH)ₕ-(CH₂)ᵢ-(CHOH)ₖₖ-R(17) oder -(CH₂)_{g}-O-(CH₂-CH₂O)ₕ-R(24);
   R(17) Wasserstoff oder Methyl,
   g, h und i
   gleich oder verschieden Null, 1, 2, 3 oder 4;
   kk 1, 2, 3 oder 4;
   R(15) und R(16)
   gleich oder verschieden Wasserstoff, Alkyl mit 1, 2, 3, 4, 5 oder 6 C-Atomen, oder zusammen mit dem sie tragenden Kohlenstoffatom Cycloalkyl mit 3, 4, 5, 6, 7 oder 8 C-Atomen;
   R(18)
   Phenyl,
   das unsubstituiert oder substituiert ist mit 1 - 3 Substituenten ausgewählt aus der Gruppe bestehend aus F, Cl, CF₃, Methyl, Methoxy und NR(25)R(26);
   R(25) und R(26)
   H oder Alkyl mit 1, 2, 3 oder 4 C-Atomen;
   oder
   R(18) Heteroaryl mit 1, 2, 3, 4, 5, 6, 7, 8 oder 9 Kohlenstoffatomen,
   das unsubstituiert oder wie Phenyl substituiert ist;
   oder
   R(18) Alkyl mit 1, 2, 3, 4, 5 oder 6 C-Atomen,
   das unsubstituiert oder mit 1 - 3 OH substituiert ist;
   oder
   R (18)
   Cycloalkyl mit 3, 4, 5, 6, 7 oder 8 C-Atomen;
   R(19), R(20), R(21), R(22) und R(23)
   gleich oder verschieden Wasserstoff oder Methyl;
   R(24) H, Alkyl mit 1, 2, 3, 4, 5 oder 6 C-Atomen, Cycloalkyl mit 3, 4, 5, 6, 7
   oder 8 C-Atomen oder -CₘH₂ₘ-R(18);
   m 1, 2, 3 oder 4;
   einer der beiden Substituenten R(2) und R(3)
   -O-CO-R(27);
   R(27) Alkyl mit 1, 2, 3, 4, 5, 6, 7 oder 8 C-Atomen, Cycloalkyl mit 3, 4, 5, 6, 7
   oder 8 C-Atomen, Phenyl, Biphenylyl, Naphthyl, Pyridyl oder Chinolinyl,
   wobei Phenyl, Biphenylyl, Naphthyl, Pyridyl oder Chinolinyl unsubstituiert oder substituiert sind mit 1 - 3 Substituenten ausgewählt aus der Gruppe bestehend aus F, Cl, CF₃, Methyl, Methoxy und NR(7)R(8);
   R(7) und R(8)
   unabhängig voneinander Wasserstoff oder Alkyl mit 1, 2, 3 oder 4 C-Atomen;

   wobei stets einer der Substituenten R(2) und R(3)
   definiert ist wie R(1);
R(4) und R(5)
   unabhängig voneinander Wasserstoff, Alkyl mit 1, 2, 3 oder 4 C-Atomen; Alkoxy mit 1, 2, 3 oder 4 C-Atomen, F, Cl, Br, I, CN oder -(CH₂)ₙ-(CF₂)ₒ-CF₃;
   n Null oder 1;
   o Null oder 1;
   sowie deren pharmazeutisch verträgliche Salze.
   (96/F 154)

bb) Benzoylguanidine der Formel I 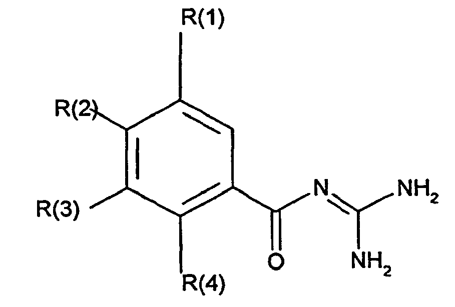 worin bedeuten:
R(1) R(13)-SOₘ oder R(14)R(15)N-SO₂-;
   m 1 oder 2;
   R(13) Alkyl mit 1, 2, 3, 4, 5, 6, 7 oder 8 C-Atomen, Perfluoralkyl mit 1, 2, 3, 4, 5, 6, 7 oder 8 C-Atomen, Alkenyl mit 3, 4, 5, 6, 7 oder 8 C-Atomen oder -CₙH₂ₙ-R(16),
   n Null, 1, 2, 3 oder 4;
   R(16) Cycloalkyl mit 3, 4, 5, 6, 7 oder 8 C-Atomen, Phenyl, Biphenylyl oder Naphthyl,
   wobei Phenyl, Biphenylyl und Naphthyl nicht substituiert oder substituiert sind mit 1 - 3 Substituenten ausgewählt aus der Gruppe bestehend aus F, Cl, CF₃, Methyl, Methoxy und NR(25)R(26); R(25) und R(26)
   unabhängig voneinander Wasserstoff, Alkyl mit 1, 2, 3 oder 4 C-Atomen oder Perfluoralkyl mit 1, 2, 3 oder 4 C-Atomen;
R(14) Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen, Alkenyl mit 3, 4, 5, 6, 7 oder 8 C-Atomen oder -CₙH₂ₙ-R(27),
   n Null, 1, 2, 3 oder 4;
   R(27) Cycloalkyl mit 3, 4, 5, 6, 7 oder 8 C-Atomen, Phenyl, Biphenylyl oder Naphthyl,
   wobei Phenyl, Biphenylyl und Naphthyl nicht substituiert oder substituiert sind mit 1 - 3 Substituenten ausgewählt aus der Gruppe bestehend aus F, Cl, CF₃, Methyl, Methoxy und NR(28)R(29); R(28) und R(29)
   unabhängig voneinander Wasserstoff, Alkyl mit 1, 2, 3 oder 4 C-Atomen oder Perfluoralkyl mit 1, 2, 3 oder 4 C-Atomen;
R(15) Wasserstoff, Alkyl mit 1, 2, 3 oder 4 C-Atomen oder Perfluoralkyl mit 1, 2, 3 oder 4 C-Atomen;
   oder
   R(14) und R(15)
   gemeinsam 4 oder 5 Methylengruppen, von denen eine CH₂-Gruppe durch Sauerstoff, S, NH, N-CH₃ oder N-Benzyl ersetzt sein kann;
   einer der Substituenten R(2) und R(3) Wasserstoff;
   und der jeweils andere Substituent R(2) und R(3) -CHR(30)R(31);
   R(30)
   -(CH₂)_{g}-(CHOH)ₕ-(CH₂)ᵢ-(CHOH)ₖ-R(32) oder -(CH₂)_{g}-O-(CH₂-CH₂O)ₕ-R(24); R(24 und R(32)
   unabhängig voneinander Wasserstoff oder Methyl; g, h, i
   gleich oder verschieden Null, 1, 2, 3 oder 4;
   k 1, 2, 3 oder 4;
   oder der jeweils andere Substituent R(2) und R(3)
   -C(OH)R(33)R(34);
   R(31), R(33) und R(34)
   gleich oder verschieden Wasserstoff oder Alkyl mit 1, 2, 3 oder 4 C-Atomen
   oder
   R(33) und R(34)
   gemeinsam Cycloalkyl mit 3, 4, 5 oder 6 C-Atomen;
   oder
   R(33) -CH₂OH;
R(4) Alkyl mit 1, 2, 3 oder 4 C-Atomen, Alkoxy mit 1, 2, 3 oder 4 C-Atomen, F, Cl, Br, I, CN, -(CH₂)ₙ-(CF₂)ₒ-CF₃;
   n Null oder 1;
   o Null, 1 oder 2;
   sowie deren pharmazeutisch verträgliche Salze. (96/F 202)

bc) Indanylidinacetylguanidine der Formel I 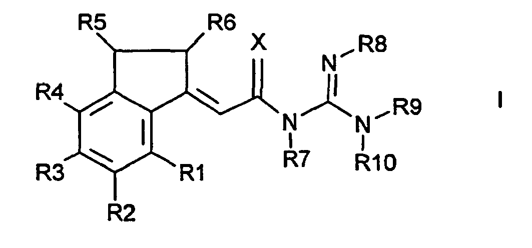 worin bedeuten:
R1, R2, R3, R4, R5 und R6
   unabhängig voneinander H, C₁-C₁₀-Alkyl; Haloalkyl mit 1 - 6 Kohlenstoffatomen, O-C₁-C₁₀-Alkyl, Haloalkoxy mit 1 - 6 C-Atomen, F, Cl, Br, I, Aryl, substituiertes Aryl, Heteroaryl, substituiertes Heteroaryl, OH, O-Niederalkyl, O-Aryl, O-Niederalkylaryl, O-substituiertes Aryl, O-Niederalkylsubstituiertes Aryl, O-C(=O)-C₁-C₄-Alkyl-Aryl, O-C(=O)-NH-C₁-C₄-Alkyl, O-C(=O)-N(C₁-C₄-Alkyl)₂, NO₂, CN, CF₃, NH₂, NH-C(=O)-C₁-C₄-Alkyl, NH-C(=O)-NH₂, COOH, C(=O)-O-C₁-C₄-Alkyl, C(=O)-NH₂, C(=O)-NH-C₁-C₄-Alkyl, C(=O)-N(C₁-C₄-Alkyl)₂, C₁-C₄-COOH, C₁-C₄-Alkyl-C(=O)-O-C₁-C₄-Alkyl, SO₃H, SO₂-Alkyl; SO₂-Alkylaryl, SO₂-N-(Alkyl)₂, SO₂-N(Alkyl)(Alkylaryl), C(=O)-R11, C₁-C₁₀-Alkyl-C(=O)-R11, C₂-C₁₀-Alkenyl-C(=O)-R11, C₂-C₁₀-Alkinyl-C(=O)-R11, NH-C(=O)-C₁-C₁₀-Alkyl-C(=O)-R11 oder O-C₁-C₁₁-Alkyl-C(=O)-R11;
   R11C₁-C₄-Alkyl, C₁-C₄-Alkinyl, Aryl, substituiertes Aryl, NH₂, NH-C₁-C₄-Alkyl, N-(C₁-C₄-Alkyl)₂, SO₃H, SO₂-Alkyl, SO₂-Alkylaryl, SO₂-N-(Alkyl)₂ oder SO₂-N(Alkyl)(Alkylaryl);
X O, S oder NH;
   R7, R8, R9 und R10
   unabhängig voneinander H, Alkyl, Cycloalkyl, Aryl, Alkylaryl, oder
   R8 und R9
   zusammen Teil eines a 5-, 6- oder 7-gliedrigen heterocyclischen Rings; oder ihre pharmazeutisch akzeptablen Salze.
   (96/F 226)

bd) Phenylsubstituierte Alkenylcarbonsäure-guanidide der Formel I 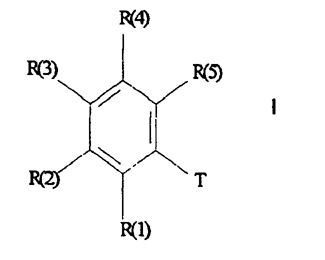 worin bedeuten:
T 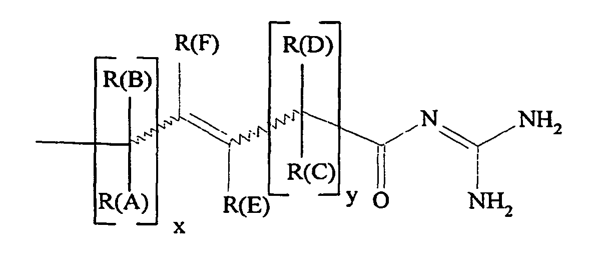 R(A) Wasserstoff, F, Cl, Br, I, CN, OH, OR(6), (C₁-C₄)-Alkyl, Oᵣ(CH₂)ₐC_{b}F_{2b+1}, (C₃-C₈)-Cycloalkyl oder NR(7)R(8)
   r Null oder 1;
   a Null, 1, 2, 3 oder 4;
   b 1, 2, 3 oder 4;
   R(6) (C₁-C₄)-Alkyl, (C₁-C₄)-Perfluoralkyl, (C₃-C₆)-Alkenyl, (C₃-C₈)-Cycloalkyl, Phenyl oder Benzyl,
   wobei der Phenylkern nicht substituiert oder substituiert ist mit 1 - 3 Substituenten ausgewählt aus der Gruppe bestehend aus F, Cl, CF₃, Methyl, Methoxy und NR(9)R(10);
   R(9) und R(10)
   H, (C₁-C₄)-Alkyl oder (C₁-C₄)-Perfluoralkyl;
   R(7) und R(8)
   unabhängig voneinander wie R(6) definiert;
   oder
   R(7) und R(8)
   gemeinsam 4 oder 5 Methylengruppen, von denen eine CH₂-Gruppe durch Sauerstoff, Schwefel, NH, N-CH₃ oder N-Benzyl ersetzt sein kann;
   R(B), R(C) und R(D)
   unabhängig wie R(A) definiert;
   x Null, 1 oder 2;
   y Null, 1 oder 2;
   R(F) Wasserstoff, F, Cl, Br, I, CN, OR(12), (C₁-C₈)-Alkyl, Oₚ(CH₂)_{f}C_{g}F_{2g+1},
   (C₃-C₈)-Cycloalkyl oder (C₁-C₉)-Heteroaryl-,
   p Null oder 1;
   f Null, 1, 2, 3 oder 4;
   g 1, 2, 3, 4, 5, 6, 7 oder 8;
   R(12) (C₁-C₈)-Alkyl, (C₁-C₄)-Perfluoralkyl, (C₃-C₈)-Alkenyl, (C₃-C₈)-Cycloalkyl, Phenyl oder Benzyl,
   wobei der Phenylkern nicht substituiert ist oder substituiert 1 - 3 Substituenten ausgewählt aus der Gruppe bestehend aus F, Cl, CF₃, Methyl, Methoxy und NR(13)R(14);
   R(13) und R(14)
   H, (C₁-C₄)-Alkyl oder (C₁-C₄)-Perfluoralkyl;
   R(E) unabhängig wie R(F) definiert;
R(1) unabhängig wie T definiert;
   oder
R(1) Wasserstoff, -OₖCₘH₂ₘ₊₁, -Oₙ(CH₂)ₚC_{q}F_{2q+1}, F, Cl, Br, I, CN, -(C=O)-N=C(NH₂)₂, -SOᵣR(17), -SOᵣ₂NR(31)R(32); -Oᵤ(CH₂)ᵥC₆H₅, -Oᵤ₂-(C₁-C₉)-Heteroaryl oder -Sᵤ₂-(C₁-C₉)-Heteroaryl,
   k Null oder 1;
   m Null, 1, 2, 3, 4, 5, 6, 7 oder 8;
   n Null oder 1;
   p Null, 1, 2, 3 oder 4;
   q 1, 2, 3, 4, 5, 6, 7 oder 8;
   r Null, 1, 2;
   r2 Null, 1, 2;
   R(31) und R(32)
   unabhängig voneinander Wasserstoff, (C₁-C₈)-Alkyl oder (C₁-C₈)-Perfluoralkyl;
   oder
   R(31) und R(32) gemeinsam 4 oder 5 Methylengruppen, von denen eine CH₂-Gruppe durch Sauerstoff, S, NH, N-CH₃ oder N-Benzyl ersetzt sein kann;
   R(17) (C₁-C₈)-Alkyl;
   u Null oder 1;
   u2 Null oder 1;
   v Null, 1, 2, 3 oder 4;
   wobei der Phenylkern substituiert ist oder substituiert mit 1 - 3 Substituenten ausgewählt aus der Gruppe bestehend aus F, Cl, CF₃, Methyl, Methoxy, -(CH₂)_{w}NR(21)R(22), NR(18)R(19) und (C₁-C₉)-Heteroaryl;
   R(18), R(19), R(21) und R(22)
   unabhängig voneinander (C₁-C₄)-Alkyl oder (C₁-C₄)-Perfluoralkyl;
   w 1, 2, 3 oder 4; wobei der Heterocyclus des (C₁-C₉)-Heteroaryl unsubstituiert oder substituiert ist mit 1 - 3 Substituenten ausgewählt aus der Gruppe bestehend aus F, Cl, CF₃, Methyl oder Methoxy;
R(2), R(3), R(4) und R(5)
   unabhängig voneinander wie R(1) definiert,
   oder
R(1) und R(2) oder R(2) und R(3) jeweils gemeinsam -CH-CH=CH-CH-,
   das nicht substituiert oder substituiert ist mit 1 - 3 Substituenten ausgewählt aus der Gruppe bestehend aus F, CI, CF₃, Methyl, Methoxy, -(CH₂)_{w2}NR(24)R(25) und NR(26)R(27); R(24), R(25), R(26) und R(27)
   H, (C₁-C₄)-Alkyl oder (C₁-C₄)-Perfluoralkyl;
   w2 1, 2, 3 oder 4;
   wobei der Rest T im Molekül mindestens zweimal, jedoch nur höchstens dreimal vorhanden ist;
   sowie deren pharmazeutisch verträgliche Salze.
   (97/ F 082)

be) Benzoylguanidine der Formel I 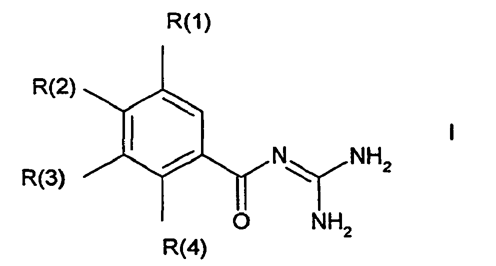 worin bedeuten:
R(1) CF₃;
   einer der Substituenten R(2) und R(3) Wasserstoff;
   und der jeweils andere Substituent R(2) und R(3)
   -C(OH)(CH₃)-CH₂OH, -CH(CH₃)-CH₂OH oder -C(OH)(CH₃)₂;
R(4) Methyl, Methoxy, Cl oder CF₃;
   sowie deren pharmazeutisch verträgliche Salze.

(DE 195 02 895, DE 44 30 212, EP 667 341, DE 44 04 183, EP 708 088, EP 723 963, EP 0 694 537, DE 44 21 495, EP 699 660, EP 699 663, EP 699 666, DE 43 37 611, EP 0719 766, WO 94/26709, WO 96 04 241, EP 726 254, US 4 251 545, DE 35 02 629, WO 84/00875, Kumamoto et al., Pharm. Bull. [1966], 7 - 13; US 3 780 027, JP 8225513; EP 743 301)

### II. Geeignet sind auch Verbindungen der Formel

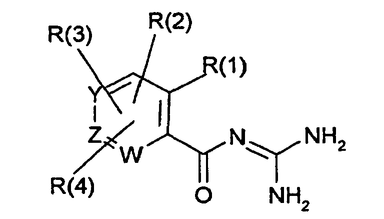

worin bedeuten:
W, Y und Z
   ein Stickstoffatom oder ein mit R(2) oder R(3) oder R(4) substituiertes C-atom;
R(1) Wasserstoff, A, Hal, -CF₃, -CH₂F, -CHF₂, -CH₂CF₃, -C₂F₅, -CN, -NO₂, - Ethinyl, oder einen X-R';
   A Alkyl mit 1 bis 6 C-Atomen;
   Hal F, Cl, Br oder I;
   X Sauerstoff, S oder NR";
   R" Wasserstoff, A oder eine cyclische Methylenkette mit 3 bis 7 C-Atomen;
   R' H, A, HO-A-, HOOC-A-, (C₃-C₇)-Cycloalkyl, (C₆-C₈)-Cycloalkyl-alkyl, CF₃, CH₂F, CHF₂, CH₂-CF₃, Ph, -CH₂-Ph oder Het;
   Ph unsubstituiertes oder ein-, zwei-, oder dreifach durch A, OA, NR'R", Hal, CF₃ substituiertes Phenyl, Naphthyl, oder Biphenylyl;
   Het einen ein- oder zweikernigen gesättigten, ungesättigten oder aromatischen Heterocyclus mit 1 bis 4 N-, O-, und/oder S-Atomen,
   der unsubstituiert oder ein-, zwei- oder dreifach substitiert ist durch Hal, CF₃, A, OH, OA, -X-R', -CN, -NO₂, und/oder Carbonylsauerstoff,
   wobei Het über N oder oder eine Alkylenkette CₘH₂ₘ mit m = Null bis 6 gebunden ist;
   oder
   R' und R"
   zusammen Alkylen mit 4 - 5 C-Atomen, worin eine CH₂-Gruppe auch durch Sauerstoff, S, NH, N-A, N-Ph und N-CH₂-Ph ersetzt sein kann; R(2) und R(3)
   unabhängig voneinander Wasserstoff, Hal, A, HO-A-, X-R', -C(=N-OH)-A, A-O-CO-(C₁-C₄)-Alkyl-, CN, NO₂, COOH, halogensubstituiertes A, insbesondere CF₃, CH₂F, CHF₂, C₂F₅, CH₂CF₃, oder S(O)ₙR"';
   R'" A, Ph oder -Het;
   n Null, 1 oder 2;
   oder
R(2) und R(3)
   unabhängig voneinander SO₂NR'R", Ph oder -O-Ph, -O-CH₂-Ph, -CO-A, -CHO, -COOA, -CSNR'R", CONR'R", -CH=CH-COOH, -CH=CH-COOA, Indenyl, Indanyl, Decahydronaphthyl, Cyclopentenyl, Dihydrothienyl, Dihydrofuryl, Heterobicyclyl, Alkylthienyl, Halothienyl, Haloalkylthienyl, Acylthienyl, Halofuryl, Haloalkylfuryl oder Pyrrolyl;
   oder
R(2) und R(3)
   unabhängig voneinander R(5)-O-;
   R(5) Wasserstoff, A, (C₁-C₆)-Alkenyl oder (C₃-C₇)-Cycloalkyl;
R(4) Ph, Het, -O-Het; CF₃, S(O)ₙR"', -SO₂NR'R", Alk; 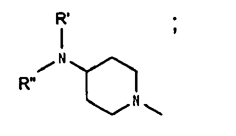 oder
   zwei der Substituenten R(1) bis R(4)
   miteinander eine Gruppe -O-CR(6)R(7)-CO-NR(8)-,
   oder 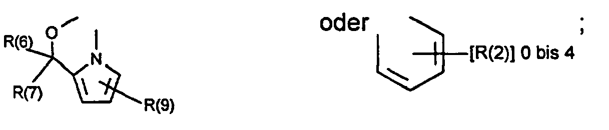 mit R(2) in der angegebenen Bedeutung;
   R(6), R(7), R(8) und R(9)
   unabhängig voneinander H oder A;
   oder
   R(8) (C₅-C₇)-Cycloalkyl;
   oder
   R(9) Cyano;
Alk geradkettiges oder verzweigtes (C₁-C₈)-Alkyl oder (C₃-C₈)-Cycloalkyl, welches unsubstituiert oder durch A ein-, zwei- oder dreifach substituiert ist;
   oder
Alk ein durch H, A, Ph oder Het substituierter Ethenyl oder Ethinylrest;

(DE 41 27 026, DE 43 37 609, JP 07025768, Edward J. Cragoe, Jr., DIURETICS [Chemistry, Pharmacology and Medicine), J. Wiley & Sons (1983), 303 - 341];

### III. Verbindungen der Formel

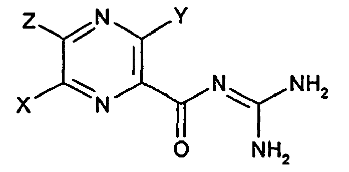

worin bedeuten:
X H, Hal, (Hal)₃C-, (C₁-C₆)-Alkyl, (C₃-C₆)-Cycloalkyl, substituiertes Phenyl, (C₁-C₅)-Alkyl-S- oder (C₁-C₅)-Alkyl-SO₂-;
Y NH₂ oder substituiertes Amino;
   oder
X und Z
   zusammen eine -(CH₂)₄- oder eine 1,3-Butadienylen-Kette;
   oder
Z H, Hal, OH, HS, (C₁-C₅)-Alkyl, (C₃-C₆)-Cycloalkyl, substituiertes Phenyl; oder
Z eine Aminogruppe -NR(1)R(2);
   R(1) H, gerad- und verzweigtkettiges, ggf. substituiertes (C₁-C₈)-Alkyl, das unterbrochen sein kann durch Sauerstoff;
   oder
   R(1) (C₃-C₈)-Alkenyl, (C₃-C₈)-Alkinyl, (C₃-C₇)-Cycloalkyl oder OH-substituiertes Phenyl oder OH-substituiertes Phenyl-(C₁-C₄)-alkyl oder OH-substituiertes (C₃-C₇)-Cycloalkyl;
   R(2) 1-Morpholino, Wasserstoff oder eine gerade oder verzweigte (C₁-C₈)-Alkylkette,
   die unterbrochen sein kann durch Sauerstoff, eine Aminogruppe,
   welche gerade oder verzweigte (C₁-C₈)-Alkylkette unsubstituiert oder substituiert ist durch
   einen substituierten oder unsubstituierten ein- oder mehrkernigen Heterocyclus, der Stickstoff, Sauerstoff oder Schwefeleatomen enthält;
   oder
   welche Alkylkette substituiert ist durch Phenyl,
   ggf ein- oder mehrfach substituiert mit (C₁-C₄) Alkoxy, gegebenenfalls substituiert durch OH, Alkylamino, Alkyl oder Phenyl;
   oder
   durch eine Aminocarbonylgruppe
   oder
   durch Hydroxy-, (C₁-C₄)-Alkoxygruppen,
   oder
R(2) Phenyl,
   unsubstituiert oder substituiert durch Alkyl, Alkoxy, eine Aminogruppe, die als Substituenten trägt:
   H, einen ein- oder mehrkernigen Heterocyclus, der Stickstoff, Sauerstoff oder Schwefelatome enthält, der unsubstituiert oder substituiert ist mit H, Hal oder (C₁-C₄)-Alkyl;
   einen Phenylrest, unsubstituiert oder substituiert durch einen Substituenten, ausgewählt aus der Gruppe bestehend aus (C₁-C₄)-Alkyl, (C₁-C₄)-Alkoxy, Hal und OH;
   oder
R(2) 1-Piperidino,
   unsubstituiert oder substituiert in 4-Stellung durch einen Acylrest einer aliphatischen, alicyclischen, aromatischen oder heteroaromatischen Carbonsäure, (C₁-C₈) Alkyl, das seinerseits substituiert sein kann durch OH oder (C1-C4)-Alkoxy oder einen (C₁-C₄)-alkoxysubstituierten Phenylrest;
   oder
R(2) Amidino, das unsubstituiert oder substituiert ist mit Phenyl, das unsubstituiert oder substituiert ist mit Hal oder Alkyl;
   oder
R(2) ein Acylrest einer aliphatischen, alicyclischen, aromatischen oder heteroaromatischen Carbonsäure,
   oder
R(2) eine (C₁-C₈) Alkylkette, die substituiert sein kann durch einen OH, Alkoxy oder Alkylreste tragenden Phenylrest.
   oder
R(1) und R(2) gemeinsam mit dem N-Atom, an das sie gebunden sind, einen Piperazinring,
   der unsubstituiert ist oder über eine (C₁-C₆)-Methylenkette einen ein- oder mehrkernigen Heterocyclus trägt,
   der Stickstoff, Sauerstoff oder Schwefele enthält (DE 41 27 026 und DE 43 37 609).
Hal F, Cl, Br oder I;

( EP 708 091, EP 622 356, JP 5-125085)

### IV. Geeignet sind ebenfalls Indoloylguanidin-Derivate der Formel

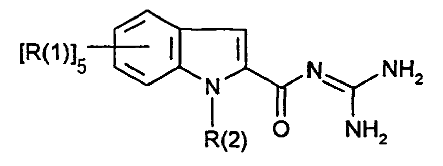

worin bedeuten
R(2) Wasserstoff, unsubstituiertes oder substituiertes (C₁-C₈)-Alkyl, (C₃-C₇)-Cycloalkyl, OH, (C₁-C₆)-Alkyl-O-, einen aromatischen Rest oder eine Gruppe - CH₂-R(20); R(20) (C₂-C₆)-Alkenyl oder (C₂-C₆)-Alkinyl;
R(1) 1 bis 5 gleiche oder verschiedene Substituenten, die bedeuten: Wasserstoff, unsubstituiertes oder substituiertes (C₁-C₈)-Alkyl, (C₂-C₆)-Alkenyl, (C₂-C₆)-Alkinyl, (C₃-C₇)-Cycloalkyl, Halogen, -NO_{2,} (C₂-C₈)-Alkanoyl, Arylalkanoyl mit bis zu 10 C-Atomen, Aroyl mit bis zu 11 C-Atomen, -COOH, (C₂-C₆)-Alkoxycarbonyl, eine aromatische Gruppe, eine der nachfolgend aufgeführten Gruppen: -OR(3), -NR(6)R(7) oder -S(O)ₙR(40);
R(3) Wasserstoff, (C₁-C₈)-Alkyl, substituiertes (C₁-C₈)-Alkyl, (C₃-C₇)-Cycloalkyl, einen aromatischen Rest oder eine Gruppe -CH₂-R(30) R(30) Alkenyl oder Alkinyl;
R(6) und R(7) unabhängig voneinander Wasserstoff, unsubstituiertes oder substituiertes (C₁-C₈)-Alkyl, (C₃-C₇)-Cycloalkyl, (C₂-C₈)-Alkanoyl, eine Arylalkanoylgruppe mit bis zu 10 C-Atomen, eine Aroylgruppe mit bis zu 11 C-Atomen, eine aromatische Gruppe oder -CH₂-R(60); R(60) (C₂-C₆)-Alkenyl oder (C₂-C₆)-Alkinyl;
oder
R(6) und R(7) gemeinsam mit dem N-Atom ein 5 - 7-gliedriges cyclisches Amin, das noch weitere Heteroatome im Ring enthalten kann;
n Null, 1 oder 2;
R(40) unsubstituiertes oder substituiertes (C₁-C₈)-Alkyl, oder eine aromatische Gruppe, oder eine Gruppe 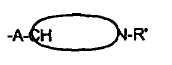 A Sauerstoff, -S(O)ₙ- oder -N(R50)-;
R(50) Wasserstoff oder (C₁-C₈)-Alkyl;
R' Wasserstoff, unsubstituiertes oder substituiertes (C₁-C₈)-Alkyl, worin der Ring einen gesättigten 3 - 8 gliedrigen Heterocyclus mit einem N-atom repräsentiert,
besagtes substituiertes Alkyl eine oder mehrere Gruppen trägt ausgewählt aus der Gruppe bestehend aus Halogen, -OH, (C₁-C₆)-Alkoxy, -CN, -COOH, (C₂-C₆)-Alkoxycarbonyl, (C₂-C₈)-Alkanoyl, Arylalkanoyl mit bis zu 10 C-Atomen, Aroyl mit bis zu 11 C-Atomen, eine aromatische Gruppe, - CONR(4)(R5),
R(4) und R(5)
gleich oder verschieden Wasserstoff oder (C₁-C₈)-Alkyl;
oder
R(4) und R(5) miteinander verbunden und bilden zusammen ein 5 - 7-gliedriges cyclisches Amin, das noch weitere Heteroatome im Ring enthalten kann,
oder besagtes substituiertes Alkyl eine Gruppe 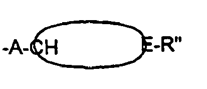 trägt, worin bedeuten:
E ein N-Atom oder eine CH-Gruppe;
R" Wasserstoff, unsubstituiertes oder mit OH substituiertes (C₁-C₈)-Alkyl oder substituiertes (C₁-C₈)-Alkyl, (C₁-C₆)-Alkoxy, -CN, -COOH, (C₂-C₆)-Alkoxycarbonyl, (C₂-C₈)-Alkanoyl, Aralkanoyl mit bis zu 10 C-Atomen, Aroyl mit bis zu 11 C-Atomen, eine aromatische Gruppe, -NR(6)R(7), -CONR(4)R(5);
   R(4) und R(5)
   unabhängig voneinander Wasserstoff oder (C₁-C₈)-Alkyl; wobei das cyclische System der Formel 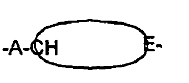 ein 3 - 8 gliedriges gesättigtes aliphatisches oder heterocyclisches Ringsystem mit einem N-Atom bedeutet,
und wobei die erwähnten aromatischen Gruppen einen Arylrest mit bis zu 10 C-Atomen, einen 5- oder 6-gliedrigen Heteroarylrest mit 1-4 N-Atomen, eine 5- oder 6-gliedrige Heteroarylgruppe enthaltend 1 oder 2 N-Atome und ein Heteroatom in der Bedeutung von Sauerstoff oder Schwefel, oder Furyl bedeuten,
und wobei die erwähnten Arylreste unsubstituiert oder substituiert sein können durch unsubstituiertes (C₁-C₈)-Alkyl oder substituiertes (C₁-C₈)-Alkyl, Halogen, -NO₂, (C₂-C₆)-Alkoxycarbonyl, COOH, -OR(3), NR(6)R(7), -CONR(4)R(5), -SO₂NR(6)R(7) oder S(O)ₙR(40),
wobei R(1) und der Guanidinocarbonylrest an einer beliebigen Stelle des 5- oder 6-gliedrigen Rings des Indolsystems stehen können,
sowie die zugehörigen pharmazeutisch verträglichen Salze.

(WO 95 04052)

### V. Weiterhin geeignet sind heterocyclische Guanidin-Derivate der Formel

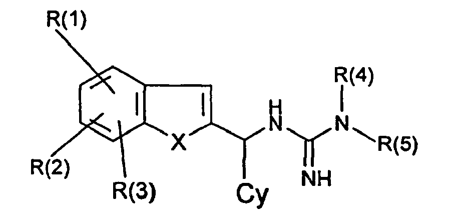

worin bedeuten:
X -O-, -S-, -NH-, -N[(C₁-C₄)-Alkyl]- oder -N(Phenyl)-;
R(1), R(2) und R(3)
   Wasserstoff, Halogen, (C₁-C₄)-Alkyl, (C₁-C₄)-Alkyl-O-, Phenyl, Benzyl; oder
   zwei der Substituenten R(1), R(2) und R(3)
   zusammen mit einer Seite des Benzosystems einen 4 - 6-gliedrigen carbocyclischen Ring;
R(4) und R(5)
   unabhängig voneinander Wasserstoff, (C₁-C₁₂)-Alkyl, Benzhydryl, Aralkyl, das unsubstituiert oder substituiert ist durch einen oder mehrere Substituenten aus den Gruppen Halogen, (C₁-C₄)-Alkyl, (C₁-C₄)-Alkyl-O- oder -CF₃, -(CH₂)ₘ-CH₂-T,
   m Null bis 3;
   T -CO-O-T(1);
   T(1) Wasserstoff oder (C1-C4)-Alkyl;
Cy einen benzokondensierten ungesättigten oder Dihydro-5-Ringheterocyclus 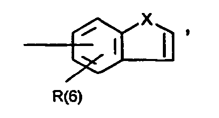 einen Pyrazol- oder Imidazolring der Formel 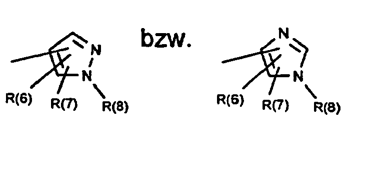 einen Naphthylrest oder ein Dihydro- oder Tetrahydronaphthylrest 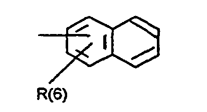 einen 2-, 3- oder 4-Pyridylrest 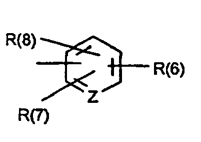 Z N- oder CH;
   einen Thienylrest 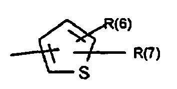 R(6) Wasserstoff, Halogen, Hydroxy, (C₁-C₁₀)-Alkyl, (C₁-C₁₀)-Alkyl-O-, Phenoxy, (C₁-C₁₀)-Alkyloxymethyloxy- oder -(O)ₙS-R(9);
   R(9) (C₁-C₁₀)-Alkyl, Thienyl, Pyridyl-, Thiazolyl, Thiadiazolyl, Imidazolyl, Pyrazolyl oder Phenyl,
   die unsubstituiert oder ein- oder zweifach substituiert sind mit Halogen, (C₁-C₄)-Alkyl oder (C₁-C₄)-Alkyl-O-;
   R(7) und R(8)
   Wasserstoff, Halogen, Hydroxy, (C₁-C₁₀)-Alkyl, (C₁-C₁₀)-Alkyl-O-, Phenyl, Phenoxy oder (C₁-C₁₀)-Alkoxymethyloxy;
   oder
Cy Phenyl, das unsubstituiert ist oder ein- oder zweifach substituiert ist mit Halogen, (C₁-C₄)-Alkyl oder (C₁-C₄)-Alkyl-O-;
   oder
Cy -Gr-Am;
   Gr -R(13)-R(12)-(CH₂)_{q}-C[W][W(1)]-(CH₂)_{q'}-; R(13)R(14)- oder -R(15)-; R(12) eine Einfachbindung, -O-, -(O)ₙS-, -CO- oder -CONH-; R(13) eine Einfachbindung, Phenyl, Thienyl, Pyridyl, Thiazolyl, Thiadiazolyl, Imidazolyl oder Pyrazolyl;
   R(14) eine Einfachbindung SO₂-;
   R(15) (C₂ - C₁₀)-Alkenyl- oder (C₂-C₁₀)-Alkinyl;
   W und W(1)
   unabhängig voneinander Wasserstoff, (C₁-C₄)-Alkyl;
   oder
   WundW(1)
   miteinander cyclisch verbunden einen (C₃-C₈)-Kohlenwasserstoffring;
   q und q'
   Null bis 9;
   Am -NR(10)R(11);
   R(10) Wasserstoff, (C₁-C₄)-Alkyl oder Benzyl,
   R(11) (C₁-C₄)-Alkyl, Phenyl oder Benzyl;
   oder
   R(10) und R(11)
   gemeinsam eine (C₃-C₁₀)-Alkylengruppe,
   die unsubstituiert ist oder substituiert mit -COOH, (C₁-C₅)-Alkoxycarbonyl, (C₂-C₄)-Hydroxyalkylen oder Benzyl;
   oder
   Am Pyrrolyl, Pyridyl, Pyrazolyl, Morpholinyl, Dihydropyridyl, Tetrahydropyridyl, Chinuclidinyl, Imidazolyl, 3-Azabicyclo[3.2.1]octyl, das unsubstituiert ist oder substituiert mit (C₁-C₄)-Alkyl,
   oder
   Am Azabicyclo[3.2.2]nonyl;
   oder
   Am eine Piperazingruppe der Formel 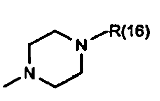 R(16) Wasserstoff, (C₁-C₄)-Alkyl, (C₃-C₆)-Cycloalkyl, Phenyl, Tolyl, Methoxyphenyl, Halophenyl, Diphenylmethylen, Benzyl oder Pyridyl;
   oder
   Am eine Azidogruppe -(O)t-(CH2)q-C[W][W(1)]-(CH2)q'-N₃;
   t Null oder 1;
   wobei W und W(1) die vorher angegebene Bedeutung besitzen; sowie die optischen Enantiomeren und die pharmakologisch verträglichen Salze.

### VI. Geeignet sind ferner die Guanidin-Verbindungen, wie sie beschrieben werden in EP- 743 301 (DE 195 17 848), EP 758 644 (DE 195 29 612), EP 760 365 (DE 195 31 138)

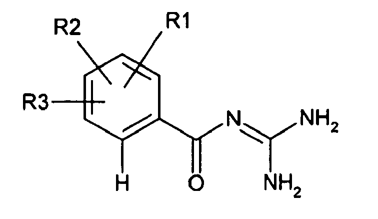

mit R1=R2 gleich H, Halo, Alkyl, CN, NO₂, Perfluoralkyl, SOₙCF₃; R3 = CH=CH₂, CH₂-CH=CH₂, CH₂-CH₂-CH=CH₂, Cycloalkenyl, Cycloalkenylalkyl; R4= Alkyl, (substituiertes) Phenyl,

Herzkreislaufaktive Wirkstoffklassen, die sich therapeutisch vorteilhaft mit NHE-Inhibitoren kombinieren lassen, sind Beta-Rezeptoren-Blocker, Calcium-Antagonisten, Angiotensin-Conversions-Enzym-Hemmer, Angiotensin-Rezeptorblocker, Schleifendiuretika, Thiaziddiuretika, kaliumsparende Diuretika, Aldosteronantagonisten, wie sie beispielsweise in der Blutdrucksenkung eingesetzt werden, sowie Herzglykoside oder andere kontraktionskraftverstärkende Mittel in der Behandlung der Herzinsuffizienz und des Congestive Heart Failures, sowie Antiarrhythmika der Klassen I - IV, Nitrate, K_{ATP}-Öffner, K_{ATP}-Blocker, oder_ Hemmer des Veratridin-aktivierbaren Natriumkanals. So sind zum Beispiel geeignet: die Betablocker Propanolol, Atenolol, Metoprolol; die Calciumantagonisten Diltiazem-Hydrochlorid, Verapamil-Hydrochlorid, Nifedipin; die ACE-Hemmer Captopril, Enalapril, Ramipril; der Angiotensin-Rezeptorantagonist Losartan; die Schleifendiruetika Furosemid, Piretanid, Torasemid; die Thiazid-Diuretika Hydrochlorothiazid, Metolazon, Indapamid; die kaliumsparenden Diuretika Amilorid, Triamteren, Spironolacton; die Herzglykoside Digoxin, Digitoxin, Strophantin; die Antiarrhythmika Amiodaron, Sotalol, Bretylium, Flecainid; das Nitrat Glyceroltrinitrat; die K⁺(ATP)-Öffner Cromakalim, Lemakalim, Nocorandil, Pinacidil, Minoxidil; die Hemmer des veratridin-aktivierbaren Na⁺-Kanals.

Ein Beispiel eines solchen besonders vorteilhaften Kombinationspartners mit NHE-Inhibitoren sind Blocker des nicht-inaktivierenden-Natriumkanals (Veratridin-aktivierbarer Natriumkanal). Beide Wirkstoffklassen zeigen in der kombinierten therapeutischen Anwendung überraschenderweise solche oben genannten synergistischen Effekte bei der Behandlung von Krankheitsbildern, die auf ischämische Zustände und Reperfusionsereignisse zurückzuführen sind. Somit sind die Kombinationen eines NHE-Inhibitors mit einem Blocker des nicht-inaktivierenden-Natriumkanals (Veratridin-aktivierbarer Natriumkanal) hervorragend zur Infarkt- und Re-infarktprophylaxe und der Infarktbehandlung sowie zur Behandlung der angina pectoris und der Inhibierung ischämisch induzierter Herzarrhythmien, der Tachykardie und der Entstehung und Aufrechterhaltung des Kammerflimmerns geeignet, wobei die Kombinationen eines NHE-Inhibitors mit einem Blocker des nicht-inaktivierenden-Natriumkanals auch präventiv die pathophysiologischen Vorgänge beim Entstehen ischämisch induzierter Schäden inhibieren oder stark vermindern. Wegen ihrer verstärkten schützenden Wirkungen gegen pathologische hypoxische und ischämische Situationen können die erfindungsgemäßen Kombinationen eines NHE-Inhibitors mit einem Blocker des nicht-inaktivierenden-Natriumkanals infolge verstärkter Inhibierung des Na+ Einstroms in die Zelle als Arzneimittel zur Behandlung aller akuten oder chronischen, durch Ischämie ausgelösten Schäden oder dadurch primär oder sekundäre induzierter Krankheiten verwendet werden. Dies betrifft ihre Verwendung als Arzneimittel für operative Eingriffe, z. B. bei Organtransplantationen, wobei die Kombinationen eines NHE-Inhibitors mit einem Blocker des nicht-inaktivierenden-Natriumkanals sowohl für den Schutz der Organe im Spender vor und während der Entnahme, zum Schutz entnommener Organe beispielweise auch bei deren Lagerung in physiologischen Badflüssigkeiten, wie auch bei der Überführung in den Empfängerorganismus verwendet werden können. Die Kombinationen eines NHE-Inhibitors mit einem Blocker des nicht-inaktivierenden-Natriumkanals sind ebenfalls wertvolle, protektiv wirkende Arzneimittel bei der Durchführung angioplastischer operativer Eingriffe beispielweise am Herzen wie auch an peripheren Gefäßen. Entsprechend ihrer protektiven Wirkung gegen ischämisch induzierte Schäden sind die Kombinationen eines NHE-Inhibitors mit einem Blocker des nicht-inaktivierenden-Natriumkanals auch als Arzneimittel zur Behandlung von Ischämien des Nervensystems, insbesondere des Zentralnervensystems geeignet, wobei sie zur Behandlung des Schlaganfalls oder des Hirnödems geeignet sind. Darüber hinaus eignen sich die erfindungsgemäßen Kombinationen eines NHE-Inhibitors mit einem Blocker des nicht-inaktivierenden-Natriumkanals ebenfalls zur Behandlung von Formen des Schocks, wie beispielsweise des allergischen, cardiogenen, hypovolämischen und des bakteriellen Schocks.

So wurde beispielsweise überraschend gefunden, daß die Kombination des NHE-Inhibitors Cariporide (HOE 642) 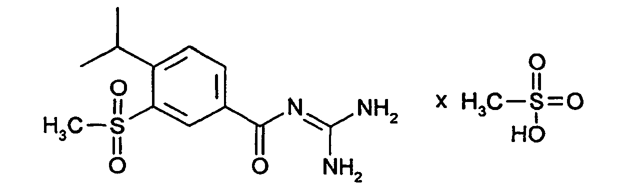 das zum Beispiel in der US-Patentschrift US 5 591 754 beschrieben ist, mit dem Hemmer des nicht-inaktivierenden-Natriumkanals (Veratridin-aktivierbarer Natriumkanal) R56865 [N-[1-[4-(4-fluorophenoxy)butyl]-4-piperidinyl]-N-methyl-2-benzothiazolamine (siehe Verdonck F, Bielen F, Ver Donck L: Preferential block of the veratridine-induced, noninactivating Na+current by R56865 in single cardiac Purkinje cells. Eur J Pharmacol 1991; 203: 371 - 378)] eine wesentlich größere als additive Wirkung in einem Modell des experimentellen Herzinfarktes an der Ratte zeigte. Die Verminderung der Infarktgröße, die mit der Kombination aus Cariporide und R56865 erzielt wurde, überstieg den Maximaleffekt der Einzelsubstanzen in diesem Modell bei weitem. Die Studie wurde wie im folgenden beschrieben durchgeführt.

### Präparation der Tiere

Männliche Ratten mit einem Körpergewicht von 280 bis 410 g wurden in vier Gruppen eingeteilt und anästhesiert. Nach Eröffnen des Brustkorbes nahe des Brustbeins wurde ein Seidenfaden (Ethicon^{®}; 1.0 metric, 5-0) um die linke Herzkranzarterie gelegt und die beiden losen Enden des Faden durch einen kleinen Plastikschlauch gezogen.

Für die Substanzapplikation wurde ein Katheter in der linke Jugularvene plaziert. Der systemische Blutdruck wurde in der linken Karotisarterie mit einem Druckaufnehmer (Combitrans®, B. Braun Melsungen AG) aufgenommen während die EKG-Erfassung mittels subkutaner Elektroden erfolgte. Die Daten wurden nach digitaler Umwandlung auf einem Computer registriert und das EKG entsprechend den Richtlinien der Lambeth Convention (6. Walker MJA, Curtis MJ, Hearse DJ, Campbell RWF, Janse MJ, Yellon DM, Cobbe SM, Cokes SJ, Harness JB, Harron DWG, Higgins SJ, Julian DG, Lab MJ, Manning AS, Northover BJ, Parratt JR, Riemersma RA, Rieva E, Russell DG, Sheridan DJ, Winslow E, Woodward B: The Lambeth convention: Guidelines for the study of arrhythmias in ischemia, infarction and reperfusion. Cardiovasx. Res 1988; 22: 447 - 455) ausgewertet.

### Protokoll des Experiments

Hoe 642 wurde in bidestilliertem Wasser und R56865 in Weinsäure (0.6%) gelöst, verdünnt mit bidestilliertem Wasser und 4 % Mannitol zugegeben. Nach der Präparation der Tiere wurden die Substanzen intravenös einzeln oder in Kombination gegeben. Die Kontrolltiere erhielten lediglich das Lösungsmittel. Das Injektionsvolumen betrug 1 ml pro kg Körpergewicht. Fünf Minuten später wurde der Seidenfaden durch Aufschieben des Plastikschlauches zugezogen und die Koronararterie für eine Stunde verschlossen. Eine Klemme fixierte den Plastikschlauch. Nach der Wiedereröffnung der Koronararterie erfolgte eine zweistündige Reperfusion des Gewebes.

### Bestimmung der Infarktgröße

Die zuvor abgebundene Kranzarterie wurde erneut verschlossen und über die Herzspitze wurde Tusche in die linke Herzkammer injiziert, um das normaldurchblutete Herzgewebe zu markieren. Nach dem Töten der Tiere wurden die Herzen entnommen, die linke Herzkammer präpariert und senkrecht zur Herzachse in Scheiben geschnitten. Die Scheiben wurden in Kochsalzlösung gewaschen und anschließend in Triphenyltetrazoliumchlorid für 5 - 7 min bei 37°C inkubiert, um das noch lebende Gewebe zu färben. Anschließend wurden die Scheiben gewogen und planimetrisch vermessen (Imaging Res. Inc. Brock. University, St. Catherines, Ontario, Canada, Phoenix Technologies, Matrox Electronic Systems OS/2). Bestimmt wurde erstens die Größe des normaldurchbluteten Gewebes, zweitens das Gebiet, welches nach dem Zuziehen der Schlinge nicht durchblutet war (Risikogebiet) und drittens die Größe des Gebietes, in dem die Zellen abgestorben waren (Infarktgröße).
Die statistische Auswertung der Unterschiede zwischen den einzelnen Gruppen erfolgte mittels Student's t-Test.

### Ergebnisse

Die Gabe der Substanzen veränderte nicht den Blutfluß in dem normaldurchbluteten Gewebe. Somit ergab sich auch keine Größenänderung des Risikogebietes von durchschnittlich 59,3 % des linken Ventrikels. In der Kontrollgruppe kam es infolge der einstündigen Blockade der Durchblutung und der Wiederdurchblutung zu einem Absterben des Gewebes. Die Infarktgröße betrug in der Kontrollgruppe 63,4 ± 4,3 % (n = 8) der Risikogebietes. Gabe von 10 mg/kg Körpergewicht Hoe 642 verringerte die Infarktgröße auf 33,2 ± 3,7 % (n = 7) und 3 mg/kg Körpergewicht an R56865 auf 38,9 ± 3,1 % (n = 8). Dagegen war die Kombination der beiden Substanzen in der jeweiligen Dosierung in der Lage, den Infarkt auf 10,5 ± 2,6 % (n = 10) des Risikogebietes abzusenken. Die Angaben sind in Durchschnitt ± S.E.M. angeben.

## Patentansprüche

1. Pharmazeutisches Kombinationspräparat, **dadurch gekennzeichnet, daß** es einen Inhibitor des Na⁺/H⁺-Austauschers und eine Herz-Kreislauf-aktive Substanz enthält und daß der Inhibitor des Na⁺/H⁺-Austauscher ausgewählt ist aus folgenden Verbindungen:
I.
a) Benzoylguanidine der Formel I 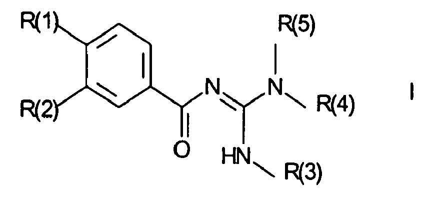 worin bedeuten:
R(1) oder R(2)
R(6)-S(O)ₙ- oder R(7)R(8)N-O₂S-;
und der jeweils andere Substituent R(1) oder R(2)
H, F, Cl, Br, (C₁-C₄)-Alkyl, (C₁-C₄)-Alkoxy oder Phenoxy,
das unsubstituiert ist oder substituiert mit 1 - 3 Substituenten ausgewählt
aus der Gruppe bestehend aus Fluor, Chlor, Methyl und Methoxy; oder der jeweils andere Substituent R(1) oder R(2)
R(6)-S(O)ₙ oder R(7)R(8)N-;
n Null, 1 oder 2;
R(6) (C₁-C₆)-Alkyl, (C₅-C₇)-Cycloalkyl, Cyclopentylmethyl, Cyclohexylmethyl oder Phenyl,
das unsubstituiert ist oder substituiert mit 1 - 3 Substituenten ausgewählt aus der Gruppe bestehend aus Fluor, Chlor, Methyl und Methoxy;
R(7) und R(8)
gleich oder verschieden H oder (C₁-C₆)-Alkyl;
oder
R(7) Phenyl-(CH₂)ₘ;
m 1 - 4;
oder
R(7) Phenyl,
das unsubstituiert ist oder substituiert mit 1 - 2 Substituenten ausgewählt aus der Gruppe bestehend aus Fluor, Chlor, Methyl und Methoxy;
oder
R(7) und R(8)
gemeinsam eine geradkettige oder verzweigte (C₄-C₇)-Kette, wobei die Kette zusätzlich durch O, S oder NR(9) unterbrochen sein kann;
R(9) H oder Methyl;
oder
R(7) und R(8)
gemeinsam mit dem Stickstoffatom, an das sie gebunden sind, ein
Dihydroindol-, Tetrahydrochinolin- oder Tetrahydroisochinolin-System;
R(3), R(4) und R(5)
unabhängig voneinander H oder (C₁-C₂)-Alkyl,
oder
R(3) und R(4)
gemeinsam eine (C₂-C₄)-Alkylenkette;
oder
R(4) und R(5)
gemeinsam eine (C₄-C₇)-Alkylenkette;
sowie deren pharmazeutisch verträgliche Salze;
b) Benzoylguanidine der Formel I 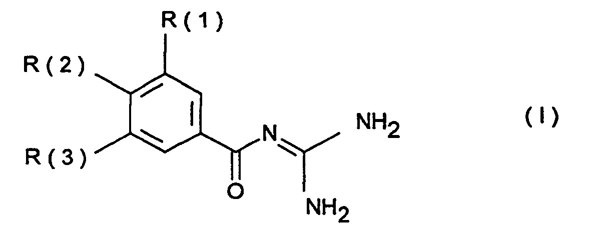 worin bedeuten:
R(1) R(4)-SOₘ oder R(5)R(6)N-SO₂-;
m Null, 1 oder 2;
R(4) und R(5)
C₁-C₈-Alkyl, C₃-C₆-Alkenyl oder -CₙH₂ₙ-R(7);
n Null, 1, 2, 3 oder 4;
R(7) C₅-C₇-Cycloalkyl oder Phenyl,
welches unsubstituiert ist oder substituiert mit 1 - 3 Substituenten ausgewählt aus der Gruppe bestehend aus F, Cl, CF₃, Methyl, Methoxy und NR(8)R(9); R(8) und R(9)
H oder C₁-C₄-Alkyl;
oder
R(5) H;
R(6) H oder C₁-C₄-Alkyl,
oder
R(5) und R(6)
gemeinsam 4 oder 5 Methylengruppen, von denen eine CH₂-Gruppe durch ein O, S, NH, N-CH₃ oder N-Benzyl ersetzt sein kann;
R(2) Wasserstoff, F, Cl, Br, (C₁-C₄)-Alkyl-, O-(CH₂)ₘCₚF₂ₚ₊₁ oder -X-R(10);
m Null oder 1;
p 1, 2 oder 3;
X O, S oder NR(11);
R(10) H, C₁-C₆-Alkyl, C₅-C₇-Cycloalkyl, Cyclohexylmethyl, Cyclopentylmethyl oder -CₙH₂ₙ-R(12);
n Null, 1, 2, 3 oder 4;
R(12) Phenyl,
das unsubstituiert oder substituiert ist mit 1 - 3 Substituenten ausgewählt aus der Gruppe bestehend aus F, Cl, CF₃, Methyl, Methoxy und NR(8)R(9); R(8) und R(9)
gleich H oder C₁-C₄-Alkyl;
R(11) Wasserstoff oder C₁-C₃-Alkyl;
oder
R(10) und R(11)
gemeinsam 4 oder 5 Methylengruppen, von denen eine CH₂-Gruppe durch O, S, NH, N-CH₃ oder N-Benzyl ersetzt sein kann;
R(3) wie R(1) definiert, oder C₁-C₆-Alkyl, Nitro, Cyano, Trifluormethyl, F, Cl, Br, J oder -X-R(10);
X O, S oder NR(11);
R(10) gleich H, C₁-C₆-Alkyl, C₅-C₇-Cycloalkyl, Cyclohexylmethyl, Cyclopentylmethyl oder -CₙH₂ₙ-R(12);
n null bis 4;
R(12) Phenyl,
das unsubstituiert ist oder substituiert mit 1 - 3 Substituenten ausgewählt aus der Gruppe bestehend aus F, Cl, CF₃, Methyl, Methoxy und NR(8)R(9);
R(8) und R(9)
H oder C₁-C₄-Alkyl;
R(11) C₁-C₃-Alkyl, oder
R(10) und R(11)
gemeinsam 4 oder 5 Methylengruppen, von denen eine CH₂-Gruppe durch O, S, NH, N-CH₃ oder N-Benzyl ersetzt sein kann;
sowie deren pharmazeutisch verträgliche Salze;
c) Ortho-substituierte Benzoylguanidine der Formel I 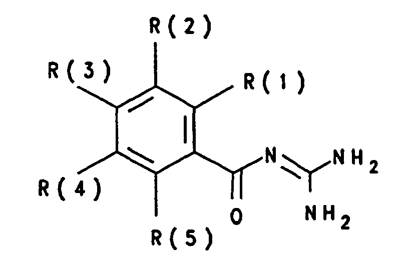 worin bedeuten:
R(1 ) F, Cl, Br, J, C₁-C₆-Alkyl oder -X-R(6);
X O, S, NR(7) oder Y-ZO;
Y O oder NR(7);
Z C oder SO;
R(6) H, C₁-C₆-Alkyl, C₅-C₇-Cycloalkyl, Cyclohexylmethyl, Cyclopentylmethyl, -(CH₂)ₘCₚF₂ₚ₊₁ oder -CₙH₂ₙ-R(8);
m Null oder 1;
p 1 - 3;
n Null bis 4;
R(8) Phenyl,
das unsubstituiert ist oder substituiert mit 1 - 3 Substituenten ausgewählt aus der Gruppe bestehend aus den Gruppen F, Cl, CF₃, Methyl, Methoxy und NR(9)R(10);
R(9) und R(10)
H oder C₁-C₄-Alkyl;
R(7) H oder C₁-C₃-Alkyl;
oder
R(6) und R(7)
gemeinsam 4 oder 5 Methylengruppen, von denen eine CH₂-Gruppe durch ein O, S, NH, N-CH₃ oder N-Benzyl ersetzt sein kann;
R(3) H oder -X-R(6);
X O, S, NR(7) oder Y-ZO;
R(7) H oder C₁-C₃-Alkyl;
Y O oder NR(7);
wobei Y am Phenylrest der Formel I gebunden ist,
Z C oder SO;
R(6) H, C₁-C₆-Alkyl, C₅-C₇-Cycloalkyl, Cyclohexylmethyl, Cyclopentylmethyl,
-(CH₂)ₘCₚF₂ₚ₊₁ oder -CₙH₂ₙ-R(8);
m null oder 1;
p 1 - 3;
n null bis 4;
R(8) Phenyl,
das unsubstituiert ist oder substituiert mit 1 - 3 Substituenten ausgewählt aus der Gruppe bestehend aus F, Cl, CF₃, Methyl, Methoxy und NR(9)R(10); R(9) und R(10)
H oder C₁-C₄-Alkyl;
oder
R(6) und R(7)
gemeinsam 4 oder 5 Methylengruppen, von denen eine CH₂-Gruppe durch ein O, S, NH, N-CH₃ oder N-Benzyl ersetzt sein kann;
R(2) und R(4)
gleich oder verschieden R(11)-SO_{q}- oder R(12)R(13)N-SO₂-;
q null - 2;
R(11) C₁-C₄-Alkyl,
das unsubstituiert ist oder Phenyl als Substituenten trägt, wobei Phenyl unsubstituiert ist oder substituiert mit 1 - 3 Substituenten ausgewählt aus der Gruppe bestehend aus F, Cl, CF₃, Methyl, Methoxy und NR(9)R(10);
R(9) und R(10)
H oder C₁-C₄-Alkyl;
R(12) und R(13)
wie R(6) und R(7) definiert;
oder einer der beiden Reste R(2) oder R(4)
Wasserstoff oder wie R(1) definiert;
R(5) H, Methyl, F, Cl oder Methoxy,
sowie deren pharmazeutisch verträgliche Salze.
d) Benzoylguanidine der Formel I 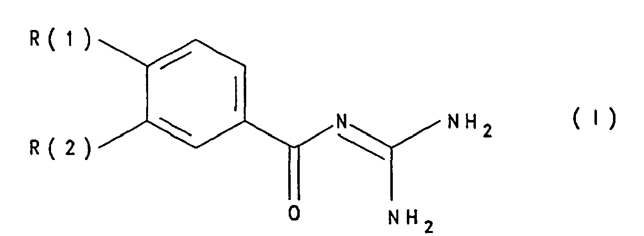 worin bedeuten:
R(1) oder R(2)
eine Aminogruppe -NR(3)R(4);
R(3) und R(4)
gleich oder verschieden H, C₁-C₆-Alkyl oder C₃-C₇-Cycloalkyl;
oder
R(3) Phenyl-(CH₂)ₚ-;
p 0, 1, 2, 3 oder 4;
oder
R(3) Phenyl,
wobei das Phenyl jeweils unsubstituiert ist oder einen bis zwei Substituenten ausgewählt aus der Gruppe bestehend aus Fluor, Chlor, Methyl und Methoxy trägt;
oder
R(3) und R(4)
gemeinsam eine geradkettige oder verzweigte C₄-C₇-Methylenkette sein können, wobei ein -CH₂-Glied der Methylenkette durch Sauerstoff, S oder NR(5) ersetzt sein kann;
R(5) H oder niedriges Alkyl;
der jeweils andere Substituent R(1) oder R(2)
H, F, Cl, C₁-C₄-Alkyl, C₁-C₄-Alkoxy, CF₃, CₘF₂ₘ₊₁-CH₂-, Benzyl oder Phenoxy, wobei der jeweilige Phenylrest unsubstituiert ist oder einen bis zwei Substituenten ausgewählt aus der Gruppe bestehend aus Methyl, Methoxy, Fluor und Chlor trägt;
m 1, 2 oder 3;
sowie deren pharmazeutisch verträgliche Salze;
e) Benzoylguanidine der Formel I 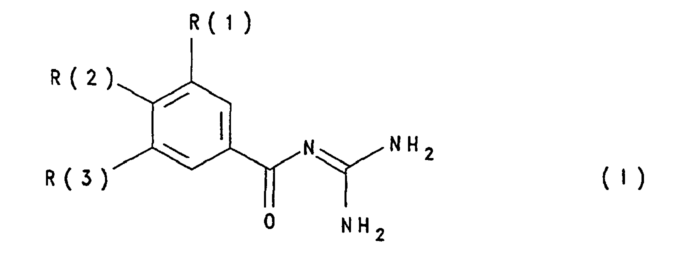 worin bedeuten:
R(1) R(4)-SOₘ oder R(5)R(6)N-SO₂-;
m Null, 1 oder 2;
R(4) und R(5)
C₁-C₈-Alkyl, C₃-C₆-Alkenyl oder -CₙH₂ₙ-R(7);
n Null, 1, 2, 3 oder 4;
R(7) C₅-C₇-Cycloalkyl oder Phenyl, welches unsubstituiert ist oder substituiert mit 1 - 3 Substituenten ausgewählt aus der Gruppe bestehend aus F, Cl, CF₃, Methyl, Methoxy und NR(8)R(9);
R(8) und R(9)
H oder C₁-C₄-Alkyl;
oder
R(5) H;
R(6) H oder C₁-C₄-Alkyl;
oder
R(5) und R(6)
gemeinsam 4 oder 5 Methylengruppen, von denen eine CH₂-Gruppe durch ein O, S, NH, N-CH₃ oder N-Benzyl ersetzt sein kann;
R(2) Wasserstoff, geradkettiges oder verzweigtes (C₅-C₈)-Alkyl, -CR(13)=CHR(12) oder -C≡CR(12);
R(12) Phenyl,
das unsubstituiert oder substituiert ist mit 1 - 3 Substituenten ausgewählt aus der Gruppe bestehend aus F, Cl, CF₃, Methyl, Methoxy und NR(14)R(15);
R(14) und R(15)
H oder (C₁-C₄)-Alkyl;
oder
R(12) (C₁-C₉)-Heteroaryl,
das unsubstituiert oder wie Phenyl substituiert ist, oder
R(12) (C₁-C₆)-Alkyl,
das unsubstituiert oder mit 1 - 3 OH substituiert ist, oder
R(12) (C₃-C₈)-Cycloalkyl;
R(13) Wasserstoff oder Methyl,
oder
R(12) (C₃-C₈)-Cycloalkyl, (C₃-C₈)-Cycloalkyl-(C₁-C₄)-alkyl, Phenyl, C₆H₅-(C₁-C₄)-Alkyl, Naphthyl, Biphenylyl, 1,1-Diphenyl-(C₁-C₄)-Alkyl, Cyclopentadienyl, Pyridyl, Pyrrolyl, Furanyl, Thienyl, Thiazolyl, Oxazolyl, Indenyl, Chinolyl, Indolyl, Benzofuranyl, Benzothienyl, Benzothiazolyl, Benzoxazolyl, Imidazolyl, Pyrazolyl, Triazolyl, Tetrazolyl, Isoxazolyl, Isothiazolyl, Pyrazinyl, Pyrimidinyl, Pyridazinyl, Indazolyl, Isochinolyl, Phthalazinyl, Chinoxalinyl, Chinazolinyl oder Cinnolinyl;
R(3) wie R(2) definiert;
und wobei die aromatischen Substituenten R(2) bzw. R(3) unsubstituiert oder mit 1-3 Substituenten aus den Gruppen F, Cl, CF₃, (C₁-C₄)-Alkyl, -Alkoxy, oder NR(10)R(11) mit R(10) und R(11) in der Bedeutung von H oder (C₁-C₄)-Alkyl substituiert sind;
sowie deren pharmazeutisch verträgliche Salze.
f) Benzoylguanidine der Formel I 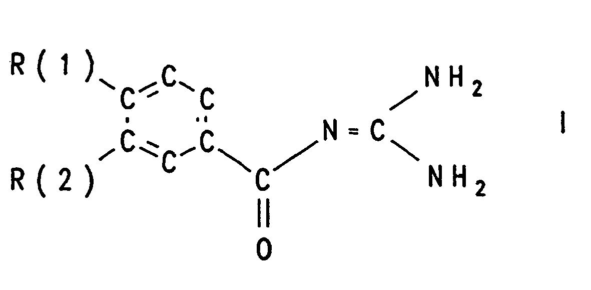 worin bedeuten:
R(1) oder R(2)
R(3)-S(O)ₙ- oder 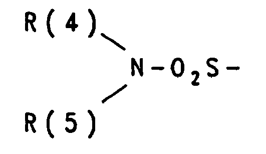 der jeweils andere Substituent R(1) oder R(2)
H, OH, F, Cl, Br, J, C₁-C₄-Alkyl, C₁-C₄-Alkoxy, Benzyloxy oder Phenoxy, das unsubstituiert ist oder einen bis drei Substituenten ausgewählt aus der Gruppe bestehend aus Fluor, Chlor, Methyl, Methoxy, Hydroxy oder Benzyloxy trägt,
R(3)-S(O)ₙ, -NR4)R(5) oder 3,4-Dehydropiperidin
R(3) C₁-C₆-Alkyl, C₅-C₇-Cycloalkyl, Cyclopentylmethyl, Cyclohexylmethyl oder Phenyl,
das unsubstituiert ist oder einen bis drei Substituenten ausgewählt aus der Gruppe bestehend aus Fluor, Chlor, Methyl und Methoxy trägt;
R(4) und R(5)
gleich oder verschieden, H oder C₁-C₆-Alkyl;
oder
R(4) Phenyl-(CH₂)ₘ-;
m 1, 2, 3 oder 4;
oder
R(4) Phenyl,
das unsubstituiert ist oder einen bis zwei Substituenten ausgewählt aus der Gruppe bestehend aus Fluor, Chlor, Methyl und Methoxy trägt;
oder
R(4) und R(5)
gemeinsam eine geradkettige oder verzweigte C₄-C₇-Kette, wobei die Kette zusätzlich durch O, S oder NR(6) unterbrochen sein kann,
R(6) H oder Methyl;
oder
R(4) und R(5)
gemeinsam mit dem Stickstoffatom, an das sie gebunden sind, ein Dihydroindol, Tetrahydrochinolin oder Tetrahydroisochinolin-System;
n Null, 1 oder 2;
sowie deren pharmazeutisch verträgliche Salze.
g) Isochinoline der Formel I 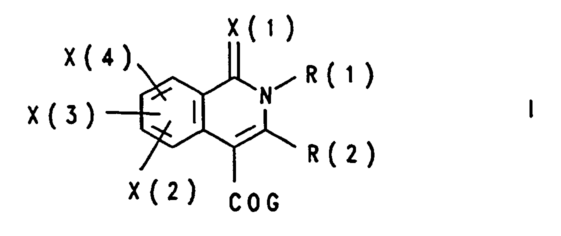 worin bedeuten:
R(1) Wasserstoff, Alkyl, Cycloalkyl, Arylalkyl, Alkenyl, substituiertes Aminoalkyl oder ein Aryl- oder Heteroarylring;
wobei die ringe unsubstituiert oder substituiert sind mit 1 - 3 Gruppen ausgewählt aus der Gruppe bestehend aus Halogen, Nitro, Amino, Mono(niederalkyl)amino, Di(niederalkyl)amino, Nieder alkyl, Niederalkoxy, Benzyloxy, Phenoxy, Hydroxy, Trifluoromethyl,
R(2) Wasserstoff, Halogen, Alkyl, oder Aryl;
welches unsubstituiert ist oder substituiert mit 1 - 3 Gruppen ausgewählt aus der Gruppe bestehend aus Halogen, Nitro, Amino, Mono(niederalkyl)amino, Di(niederalkyl)amino, Niederalkyl, Niederalkoxy, Benzyloxy, Phenoxy, Hydroxy,
G 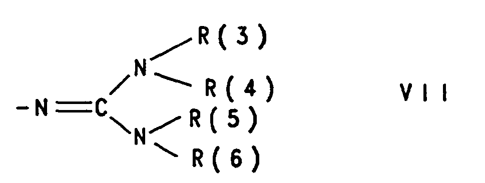
X(2), X(3) and X(4)
unabhängig voneinander Wasserstoff, Halogen, Nitro, Amino, Alkyl, Sulfonamid, Mono(niederalkyl)amino, Di(niederalkyl)amino, Niederalkyl, Benzyloxy, Hydroxy;
X(1) Wasserstoff, Sauerstoff, Schwefel oder NR(7);
R(7) Wasserstoff, Alkyl, Cycloalkyl, Arylalkyl, Alkenyl, substituiertes Aminoalkyl oder ein aryl oder ein Heteroarylring; welche ringe unsubstituiert oder substituiert sind mit 1 - 3 Gruppen ausgewählt aus der Gruppe bestehend aus Halogen, Nitro, Amino, Mono(niederalkyl)amino, Di(niederalkyl)amino, Niederalkyl, Niederalkoxy, Benzyloxy, Phenoxy, Hydroxy und Trifluoromethyl;
in welchen Substituenten jede Alkylkette oder Alkenylkette durch Sauerstoff, Schwefel oder NR(8) unterbrochen sein kann;
R(8) Wasserstoff, Alkyl, Cycloalkyl, Arylalkyl, Alkenyl, substituiertes Aminoalkyl oder ein Aryl- oder Heteroarylring,
welche Ringe unsubstituiert oder substituiert sind mit 1 - 3 Gruppen ausgewählt aus der Gruppe bestehend aus Halogen, Nitro, Amino, Mono(niederalkyl)amino, Di(niederalkyl)amino, Niederalkyl, Niederalkoxy, Benzyloxy, Phenoxy, Hydroxy und Trifluoromethyl;
und ihre pharmazeutisch akzeptablen Salze;
h) Verbindungen der Formel I 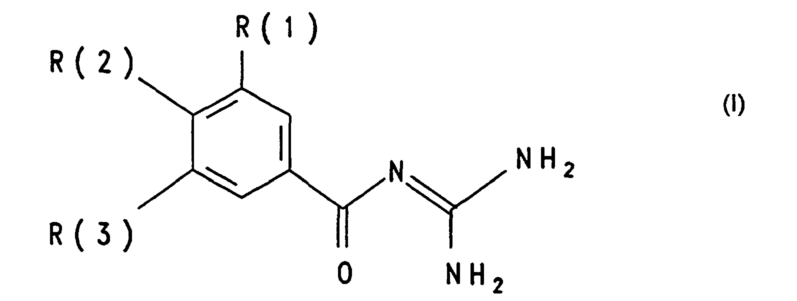 worin bedeuten:
R(1 ) Wasserstoff, F, Cl, Br, I, -NO₂, -C≡N, -CF₃, R(4)-SOₘ oder R(5)R(6)N-SO₂-; m Null, 1 oder 2;
R(4) und R(5)
(C₁-C₈)-Alkyl, (C₃-C₆)-Alkenyl, -CₙH₂ₙ-R(7) oder CF₃;
n Null, 1, 2, 3 oder 4;
R(7) (C₃-C₇)-Cycloalkyl oder Phenyl, welches nicht substituiert ist oder substituiert mit 1 - 3 Substituenten ausgewählt aus der Gruppe bestehend aus F, Cl, CF₃, Methyl, Methoxy und NR(8)R(9);
R(8) und R(9)
H oder C₁-C₄-Alkyl;
oder
R(5) H;
R(6) H oder (C₁-C₄)-Alkyl;
oder
R(5) und R(6)
gemeinsam 4 oder 5 Methylengruppen, von denen eine CH₂-Gruppe durch Sauerstoff, S, NH, N-CH₃ oder N-Benzyl ersetzt sein kann;
R(2) -SR(10), -OR(10), -NHR(10), -NR(10)R(11), -CHR(10)R(12), -[CR(12)R(13)OR(13')], -{C-[CH₂-OR(13')]R(12)(R(13)} oder -[CR(18)R(17)]ₚ-(CO)-[CR(19)R(20)]_{q}-R(14);
R(10), R(11)
gleich oder verschieden
-[CHR(16)]ₛ(CH₂)ₚ-(CHOH)_{q}-(CH₂)ᵣ-(CHOH)ₜ-R(21) oder
-(CH₂)ₚ-O-(CH₂-CH₂O)_{q}-R(21),
R(21) Wasserstoff, Methyl,
p, q, r gleich oder verschieden
Null, 1, 2, 3 oder 4;
s Null oder 1;
t 1, 2, 3 oder 4;
R(12) und R(13)
gleich oder verschieden Wasserstoff, (C₁-C₆)-Alkyl oder zusammen mit dem sie tragenden Kohlenstoff-Atom ein (C₃-C₈)-Cycloalkyl,
R(13') Wasserstoff oder (C₁-C₄)-Alkyl;
R(14) H, (C₁-C₆)-Alkyl, (C₃-C₈)-Cycloalkyl oder -CₐH₂ₐ-R(15);
a Null, 1, 2, 3 oder 4;
R(15) Phenyl, das unsubstituiert oder substituiert ist mit 1 - 3 Substituenten ausgewählt aus der Gruppe bestehend aus F, Cl, CF₃, Methyl, Methoxy und NR(8)R(9);
R(8) und R(9)
H oder (C₁-C₄)-Alkyl;
oder
R(15) (C₁-C₉)-Heteroaryl,
das unsubstituiert oder wie Phenyl substituiert ist, oder
R(15) (C₁-C₆)-Alkyl,
das unsubstituiert oder mit 1 - 3 OH substituiert ist;
R(16), R(17), R(18), R(19) und R(20)
Wasserstoff oder (C₁-C₃)-Alkyl;
R(3) wie R(1) definiert,
oder
R(3) (C₁-C₆)-Alkyl oder -X-R(22);
X Sauerstoff, S oder NR(16); R(16) H oder (C₁-C₃)-Alkyl;
oder
R(22) und R(16)
gemeinsam 4 oder 5 Methylengruppen, von denen eine CH₂-Gruppe durch Sauerstoff, S, NH, N-CH₃ oder N-Benzyl ersetzt sein kann;
R(22) wie R(14) definiert;
sowie deren pharmazeutisch verträgliche Salze.
i) Benzoylguanidine der Formel I 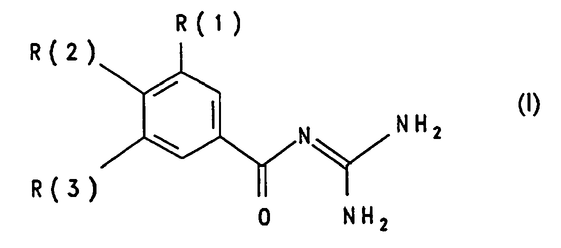 worin bedeuten:
R(1) Wasserstoff, F, Cl, Br, I, -NO₂, -C≡N, R(16)-CₚH₂ₚ-O_{q}, R(4)-SOₘ oder R(5)R(6)N-SO₂-;
m Null, 1 oder 2;
p Null oder 1;
q Null, 1, 2 oder 3;
R(16) CᵣF₂ᵣ₊₁;
r 1, 2 oder 3;
R(4) und R(5)
(C₁-C₈)-Alkyl, (C₃-C₆)-Alkenyl, -CₙH₂ₙ-R(7) oder CF₃;
n Null, 1, 2, 3 oder 4;
R(7) (C₃-C₇)-Cycloalkyl oder Phenyl,
welches nicht substituiert ist oder substituiert mit 1 - 3 Substituenten ausgewählt aus der Gruppe bestehend aus F, Cl, CF₃, Methyl, Methoxy und NR(8)R(9); R(8) und R(9)
H oder C₁-C₄-Alkyl;
oder
R(5) H;
R(6) H oder (C₁-C₄)-Alkyl;
oder
R(5) und R(6)
gemeinsam 4 oder 5 Methylengruppen, von denen eine CH₂-Gruppe durch Sauerstoff, S, NH, N-CH₃ oder N-Benzyl ersetzt sein kann,
R(2) (C₁-C₉)-Heteroaryl,
das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und
Dimethylamino;
oder
R(2) -SR(10), -OR(10), -NR(10)R(11), -CR(10)R(11)R(12); R(10) -CₐH₂ₐ-(C₁-C₉)-Heteroaryl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
a Null, 1 oder 2;
R(11) und R(12)
unabhängig voneinander wie R(10) definiert oder Wasserstoff oder (C₁-C₄)-Alkyl;
R(3) wie R(1) definiert, oder (C₁-C₆)-Alkyl oder -X-R(13);
X Sauerstoff, S, oder NR(14);
R(14) H oder (C₁-C₃)-Alkyl;
R(13) H, (C₁-C₆)-Alkyl, (C₃-C₈)-Cycloalkyl oder -C_{b}H_{2b}-R(15);
b Null, 1, 2, 3 oder 4;
oder
R(13) und R(14)
gemeinsam 4 oder 5 Methylengruppen, von denen CH₂-Gruppe durch Sauerstoff, S, NH, N-CH₃ oder N-Benzyl ersetzt sein kann;
R(15) Phenyl,
das unsubstituiert oder substituiert ist mit 1 - 3 Substituenten ausgewählt aus der Gruppe bestehend aus F, Cl, CF₃, Methyl, Methoxy und NR(8)R(9);
R(8) und R(9)
H oder (C₁-C₄)-Alkyl;
sowie deren pharmazeutisch verträgliche Salze;
k) Benzoylguanidine der Formel I 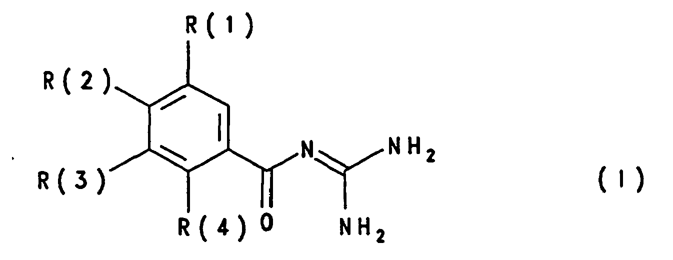 worin bedeuten:
einer der Substituenten R(1), R(2), R(3) oder R(4):
eine Aminogruppe 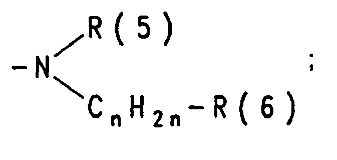 R(5) Wasserstoff oder C₍₁₋₆₎-Alkyl;
n Null, 1, 2, 3 oder 4;
R(6) H oder C₍₁₋₄₎-Alkyl;
worin eine CH₂-Gruppe durch 1 S-Atom oder eine Gruppe NR(7) ersetzt sein kann;
R(7) Wasserstoff, Methyl oder Ethyl;
oder
R(6) C₍₃₋₈₎-Cycloalkyl oder Phenyl,
das unsubstituiert ist oder 1, 2 oder 3 Substituenten ausgewählt aus der Gruppe bestehend aus F, Cl, Br, Methyl, Methoxy, -NR(8)R(9) trägt;
R(8) und R(9)
H, Methyl oder Ethyl;
oder
R(5) und R(6)
gemeinsam mit dem Stickstoffatom einen 5-, 6- oder 7-gliedrigen Ring, in welchem 1 C-Atom durch Sauerstoff, S oder NR(10) ersetzt sein kann;
R(10) H, C₍₁₋₃₎-Alkyl oder Benzyl;
und die jeweils anderen Substituenten R(1), R(2), R(3), R(4):
Wasserstoff, F, Cl, Br, I, CN, CF₃, NO₂, CF₃-O-, CₘF₂ₘ₊₁-CH₂-O- oder R(11)-C_{q}H_{2q}-Xₚ-;
m 1, 2 oder 3;
q Null, 1, 2, 3 oder 4;
p Null oder 1;
X Sauerstoff oder NR(12);
R(12) H oder C₍₁₋₃₎-Alkyl;
R(11) Wasserstoff, C₍₁₋₆₎-Alkyl, C₍₃₋₈₎-Cycloalkyl oder Phenyl,
das unsubstituiert oder substituiert ist mit 1, 2 oder 3 Substituenten ausgewählt aus der Gruppe bestehend aus F, Cl, CH₃, CH₃-O- und NR(13)R(14);
R(13), R(14)
H, Methyl oder Ethyl;
sowie deren pharmazeutisch verträgliche Salze.
l) Benzoylguanidine der Formel I 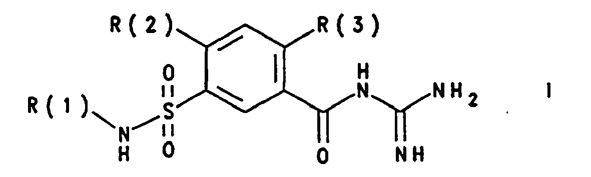 worin bedeuten
R(1) R(4)R(5)N-C(X)-;
X Sauerstoff, S oder N-R(6);
R(4) und R(5)
gleich oder verschieden, H, (C₁-C₈)-Alkyl, (C₃-C₆)-Alkenyl oder -CₙH₂ₙ-R(7);
n Null, 1, 2, 3 oder 4;
R(7) (C₅-C₇)-Cycloalkyl oder Phenyl, welches unsubstituiert ist oder substituiert mit 1 - 3 Substituenten ausgewählt aus der Gruppe bestehend aus F, Cl, CF₃, Methoxy und (C₁-C₄)-Alkyl;
oder
R(4) und R(5)
gemeinsam 4 oder 5 Methylengruppen, von denen eine CH₂-Gruppe durch Sauerstoff, S, NH, N-CH₃ oder N-Benzyl ersetzt sein kann; R(6) wie R(4) definiert oder gleich Amidin;
R(2) H, F, Cl, Br, I, (C₁-C₈)-Alkyl, 1-Alkenyl oder 1-Alkinyl, (C₃-C₈)-Cycloalkyl, (C₃-C₈)-Cycloalkyl-(C₁-C₄)-Alkyl, Phenyl, C₆H₅-(C₁-C₄)-Alkyl, Naphthyl, Biphenylyl, 1,1-Diphenyl-(C₁-C₄)-Alkyl, Cyclopentadienyl, Pyridyl, Thiopyridyl, Pyrrolyl, Furanyl, Thienyl, Thiazolyl, Oxazolyl, Indenyl, Chinolyl, Indolyl, Benzofuranyl, Benzothienyl, Benzothiazolyl, Benzoxazolyl oder -W-R(8);
W Sauerstoff, S oder NR(9);
R(8) H, (C₁-C₆)-Alkyl, (C₅-C₇)-Cycloalkyl, Cyclohexylmethyl, Cyclopentylmethyl, -(CH₂)ₘCpF₂ₚ₊₁ oder -C_{q}H_{2q}-R(10);
m Null oder 1;
p 1, 2 oder 3;
q Null, 1, 2, 3 oder 4;
R(10) Phenyl,
das unsubstituiert oder substituiert ist mit 1 - 3 Substituenten ausgewählt aus der Gruppe bestehend aus F, Cl, CF₃, Methyl, Methoxy und NR(11)R(12);
R(11) und R(12)
H oder (C₁-C₄)-Alkyl;
R(9) H oder (C₁-C₃)-Alkyl;
oder
R(8) und R(9)
gemeinsam 4 oder 5 Methylengruppen, von denen eine CH₂-Gruppe durch Sauerstoff, S, NH, N-CH₃ oder N-Benzyl ersetzt sein kann;
R(3) H, F, Cl, Br, I, (C₁-C₆)-Alkyl oder -W-R(8) wie für R(2) definiert, sowie deren pharmazeutisch akzeptable Salze;
m) Benzoylguanidine der Formel I 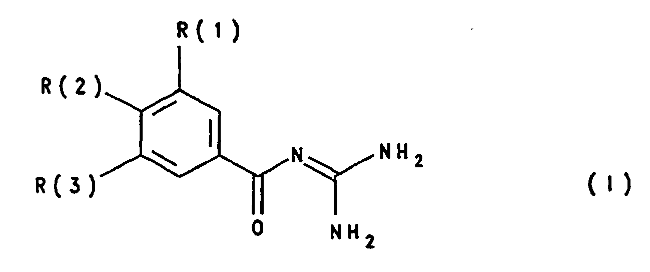 worin bedeuten:
R(1), R(2), R(3)
Wasserstoff, F, Cl, Br, I oder (C₁-C₁₂)-Alkyl;
einer der Substituenten R(1), R(2) oder R(3)
N₃, CN, OH oder (C₁-C₁₀)-Alkyloxy, wenn mindestens einer der restlichen Substituenten R(1), R(2) oder R(3) einen hinreichend lipophilen Alkylrest mit 3 bis 12 Kohlenstoff-Atomen bedeutet;
oder
einer der Substituenten R(1), R(2) oder R(3)
R(4)-CₙH₂ₙ-Oₘ-;
m Null oder 1;
n Null, 1, 2 oder 3;
R(4) CₚF₂ₚ₊₁;
p 1, 2 oder 3, sofern n gleich Null oder 1 ist;
oder
R(4) (C₃-C₁₂)-Cycloalkyl, Phenyl, Pyridyl, Chinolyl oder Isochinolyl, wobei die aromatischen und heteroaromatischen Ringsysteme unsubstituiert sind oder substituiert mit einem Substituenten ausgewählt aus der Gruppe bestehend aus F, Cl, CF₃, Methyl, Methoxy und NR(5)R(6); R(5) und R(6)
Wasserstoff oder (C₁-C₄)-Alkyl;
oder einer der Substituenten R(1), R(2) oder R(3) -C≡CR(5) oder -C[R(6)] = CR(5);
R(5) Phenyl, das unsubstituiert oder substituiert ist mit 1 - 3 Substituenten ausgewählt aus der Gruppe bestehend aus F, Cl, CF₃, Methyl, Methoxy, Hydroxy, Amino, Methylamino und Dimethylamino, (C₁-C₉)-Heteroaryl,
das unsubstituiert oder wie Phenyl substituiert ist,
oder
R(5) (C₁-C₆)-Alkyl,
das unsubstituiert oder mit 1 - 3 OH substituiert ist;
oder
R(5) (C₃-C₈)-Cycloalkyl,
R(6) Wasserstoff oder Methyl;
sowie deren pharmakologisch akzeptable Salze;
o) Benzoylguanidine der Formel I 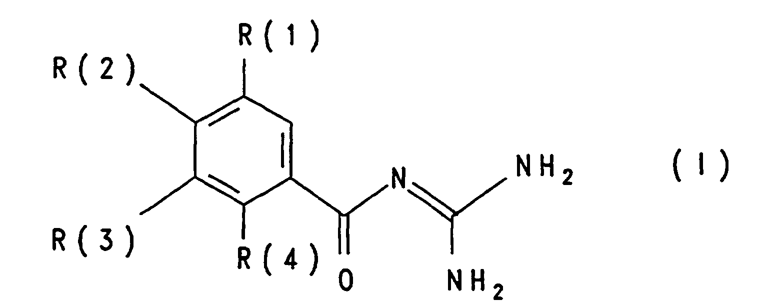 worin bedeuten:
R(1) Wasserstoff, F, Cl, Br, I, -NO₂, -C≡N, Xo (CH₂)p-(CF₂)_{q}-CF₃, R(5)-SOₘ, R(6)-CO- oder R(6)R(7)N-SO₂-, mit
X Sauerstoff, S oder NR(14);
m Null, 1 oder 2;
o Null oder 1;
p Null, 1 oder 2;
q Null, 1, 2, 3, 4, 5 oder 6;
R(5) und R(6)
(C₁-C₈)-Alkyl, (C₃-C₆)-Alkenyl, -CₙH₂ₙ-R(8) oder CF₃;
n Null, 1, 2, 3 oder 4;
R(8) (C₃-C₇)-Cycloalkyl oder Phenyl,
welches nicht substituiert ist oder substituiert mit 1 - 3 Substituenten ausgewählt aus der Gruppe bestehend aus F, Cl, CF₃, Methyl, Methoxy und NR(9)R(10); R(9) und R(10)
H oder C₁-C₄-Alkyl;
oder
R(6) H;
R(7) H oder (C₁-C₄)-Alkyl;
oder
R(6) und R(7)
gemeinsam 4 oder 5 Methylengruppen, von denen eine CH₂-Gruppe durch Sauerstoff, S, NH, N-CH₃ oder N-Benzyl ersetzt sein kann; 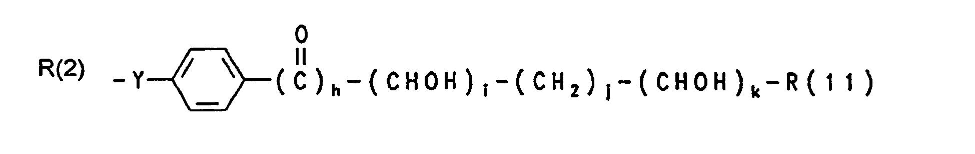 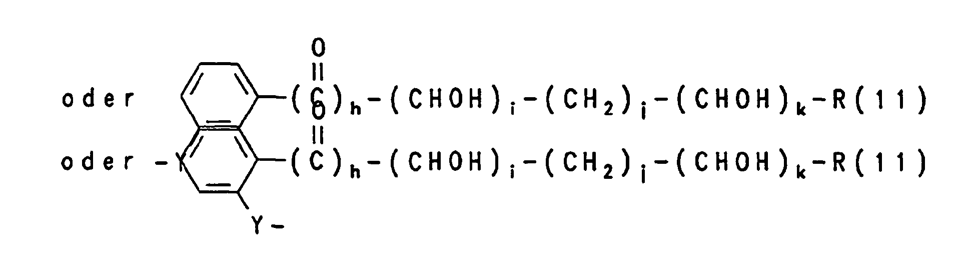 Y Sauerstoff, -S- oder -NR(12)-;
R(11) und R(12)
Wasserstoff oder (C₁-C₃)-Alkyl;
h Null oder 1;
i, j und k
unabhängig Null, 1, 2, 3 oder 4;
wobei jedoch nicht h, i und k gleichzeitig Null sind,
R(3) wie R(1) definiert, oder (C₁-C₆)-Alkyl oder -X-R(13);
X Sauerstoff, S oder NR(14);
R(14) H oder (C₁-C₃)-Alkyl;
R(13) H, (C₁-C₆)-Alkyl, (C₃-C₈)-Cycloalkyl oder -C_{b}H_{2b}-R(15);
b Null, 1, 2, 3 oder 4;
oder
R(13) und R(14)
gemeinsam 4 oder 5 Methylengruppen, wobei eine CH₂-Gruppe durch Sauerstoff, S, NH, N-CH₃ oder N-Benzyl ersetzt sein kann;
R(15) Phenyl,
das unsubstituiert oder substituiert ist mit 1 - 3 Substituenten ausgewählt aus der Gruppe bestehend aus F, Cl, CF₃, Methyl, Methoxy und NR(9)R(10); R(9) und R(10)
H oder (C₁-C₄)-Alkyl;
R(4) Wasserstoff, -OR(16) oder -NR(16)R(17);
R(16) und R(17)
unabhängig Wasserstoff oder (C₁-C₃)-Alkyl; sowie deren pharmazeutisch verträgliche Salze;
p) Benzoylguanidine der Formel I 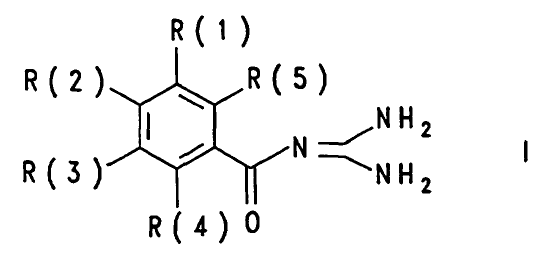 worin bedeuten:
R(1) R(6)-CO oder R(7)R(8)N-CO;
R(6) (C₁-C₈)-Alkyl, (C₁-C₈)-Perfluoralkyl, (C₃-C₈)-Alkenyl oder -CₙH₂ₙ-R(9);
n Null, 1, 2, 3 oder 4;
R(9) (C₃-C₈)-Cycloalkyl, Phenyl, Biphenylyl oder Naphthyl,
wobei die Aromaten nicht substituiert oder substituiert sind mit 1 - 3 Substituenten ausgewählt aus der Gruppe bestehend aus F, Cl, CF₃, Methyl, Methoxy und NR(10)R(11);
R(10) und R(11)
H, (C₁-C₄)-Alkyl oder (C₁-C₄)-Perfuoralkyl;
R(7) H, (C₁-C₈)-Alkyl, (C₁-C₈)-Perfluoralkyl, (C₃-C₈)-Alkenyl oder -CₙH₂ₙ-R(12);
n Null, 1, 2, 3 oder 4;
R(12) (C₃-C₈)-Cycloalkyl, Phenyl, Biphenylyl oder Naphthyl,
wobei die Aromaten nicht substituiert oder substituiert sind mit 1 - 3 Substituenten ausgewählt aus der Gruppe bestehend aus F, Cl, CF₃, Methyl, Methoxy und NR(13)R(14);
R(13) und R(14)
H, (C₁-C₄)-Alkyl oder (C₁-C₄)-Perfuoralkyl;
R(8) H, (C₁-C₄)-Alkyl oder (C₁-C₄)-Perfluoralkyl;
oder
R(7) und R(8)
gemeinsam 4 oder 5 Methylengruppen, von denen eine CH₂-Gruppe durch Sauerstoff, S, NH, N-CH₃ oder N-Benzyl ersetzt sein kann;
R(2) R(1) definiert, oder H, F, Cl, Br, I, CN, NO₂, (C₁-C₈)-Alkyl, (C₁-C₈)-Perfluoralkyl, (C₃-C₈)-Alkenyl oder -CₙH₂ₙR(15);
n Null, 1, 2, 3, 4; R(15) (C₃-C₈)-Cycloalkyl, Phenyl, Biphenylyl oder Naphthyl,
wobei die Aromaten nicht substituiert oder substituiert sind mit 1 - 3 Substituenten ausgewählt aus der Gruppe bestehend aus F, Cl, CF₃, Methyl, Methoxy und NR(16)R(17);
R(16) und R(17)
H, (C₁-C₄)-Alkyl oder (C₁-C₄)-Perfuoralkyl;
oder
R(2) (C₁-C₉)-Heteroaryl, das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(2) SR(18), -OR(18), -NR(18)R(19), -CR(18)R(19)R(20);
R(18) -CₐH₂ₐ-(C₁-C₉)-Heteroaryl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
a Null, 1 oder 2;
R(19) und R(20)
unabhängig voneinander wie R(18) definiert oder Wasserstoff, (C₁-C₄)-Alkyl oder (C₁-C₄)-Perfluoralkyl;
oder
R(2) R(21)-SOₘ oder R(22)R(23)N-SO₂-;
m 1 oder 2;
R(21) (C₁-C₈)-Alkyl, (C₁-C₈)-Perfluoralkyl, (C₃-C₈)-Alkenyl, -CₙH₂ₙ-R(24), n Null, 1, 2, 3 oder 4;
R(24) (C₃-C₈)-Cycloalkyl, Phenyl, Biphenylyl oder Naphthyl,
wobei die Aromaten nicht substituiert oder substituiert sind mit 1 - 3 Substituenten ausgewählt aus der Gruppe bestehend aus F, Cl, CF₃, Methyl, Methoxy und NR(27)R(28);
R(27) und R(28)
H, (C₁-C₄)-Alkyl oder (C₁-C₄)-Perfuoralkyl;
R(22) H, (C₁-C₈)-Alkyl, (C₁-C₈)-Perfluoralkyl, (C₃-C₈)-Alkenyl, -CₙH₂ₙ-R(29);
n Null, 1, 2, 3 oder 4;
R(29) (C₃-C₈)-Cycloalkyl, Phenyl, Biphenylyl oder Naphthyl,
wobei die Aromaten nicht substituiert oder substituiert sind mit 1 - 3 Substituenten ausgewählt aus der Gruppe bestehend aus F, Cl, CF₃, Methyl, Methoxy und NR(30)R(31);
R(30) und R(31)
H, (C₁-C₄)-Alkyl oder (C₁-C₄)-Perfuoralkyl;
R(23) H, (C₁-C₄)-Alkyl oder (C₁-C₄)-Perfluoralkyl;
oder
R(22) und R(23)
gemeinsam 4 oder 5 Methylengruppen, von denen eine CH₂-Gruppe durch Sauerstoff, S, NH, N-CH₃ oder N-Benzyl ersetzt sein kann;
oder
R(2) R(33)X-;
X Sauerstoff, S, NR(34), (D=O)A-, NR(34)C=MN⁽*⁾R(35)-;
M Sauerstoff oder S;
A Sauerstoff oder NR(34);
D C oder SO;
R(33) (C₁-C₈)-Alkyl, (C₃-C₈)-Alkenyl, (CH₂)_{b}C_{d}F_{2d+1}, -CₙH₂ₙ-R(36),
b Null oder 1;
d 1, 2, 3, 4, 5, 6 oder 7;
n Null, 1, 2, 3, oder 4;
R(36) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(37)R(38);
R(37) und R(38)
H, (C₁-C₄)-Alkyl oder (C₁-C₄)-Perfuoralkyl;
R(34) H, (C₁-C₄)-Alkyl oder (C₁-C₄)-Perfuoralkyl;
R(35) definiert wie R(33);
oder
R(33) und R(34)
gemeinsam 4 oder 5 Methylengruppen, von denen eine CH₂-Gruppe durch Sauerstoff, S, NH, N-CH₃ oder N-Benzyl ersetzt sein kann;
wobei A und N⁽*⁾ an den Phenylkern des Benzoylguanidin-Grundkörpers gebunden sind;
oder
R(2) -SR(40), -OR(40), -NHR(40), -NR(40)R(41), -CHR(40)R(42), -C[R(42)R(43)OH], -C≡CR(45), -CR(46)=CHR(45), -[CR(47)R(48)]ᵤ-(CO)-[CR49)R(50)]ᵥ-R(44); R(40), R(41)
gleich oder verschieden -(CH₂)ₚ-(CHOH)_{q}-(CH₂)ᵣ-(CHOH)ₜ-R(51) oder - (CH₂)ₚ-O-(CH₂-CH₂O)_{q}-R(51);
R(51) Wasserstoff oder Methyl;
u 1, 2, 3 oder 4;
v Null, 1, 2, 3 oder 4;
p, q, r
gleich oder verschieden Null, 1, 2, 3 oder 4;
t 1, 2, 3 oder 4;
R(42) und R(43) gleich oder verschieden Wasserstoff oder (C₁-C₆)-Alkyl;
oder
R(42) und R(43)
zusammen mit dem sie tragenden Kohlenstoff-Atom ein (C₃-C₈)-Cycloalkyl bilden;
R(44) H, (C₁-C₆)-Alkyl, (C₃-C₈)-Cycloalkyl oder -CₑH₂ₑ-R(45);
e Null, 1, 2, 3 oder 4;
R(45) gleich Phenyl,
das unsubstituiert oder substituiert ist mit 1 - 3 Substituenten aus der Gruppe bestehend aus F, Cl, CF₃, Methyl, Methoxy und NR(52)R(53) mit
R(52) und R(53) gleich H oder (C₁-C₄)-Alkyl, oder R(45) (C₁-C₉)-Heteroaryl,
das unsubstituiert oder wie Phenyl substituiert ist;
oder
R(45) (C₁-C₆)-Alkyl,
das unsubstituiert oder mit 1 - 3 OH substituiert ist;
R(46), R(47), R(48), R(49) und R(50)
Wasserstoff oder Methyl;
oder
R(2) R(55)-NH-SO₂-;
R(55) R(56)R(57)N-(C=Y)-;
Y Sauerstoff, S oder N-R(58);
R(56) und R(57)
gleich oder verschieden H, (C₁-C₈)-Alkyl, (C₃-C₆)-Alkenyl oder -C_{f}H_{2f}-R(59);
f Null, 1, 2, 3 oder 4;
R(59) (C₅-C₇)-Cycloalkyl oder Phenyl, welches unsubstituiert ist oder substituiert mit 1 - 3 Substituenten ausgewählt aus der Gruppe bestehend aus F, Cl, CF₃, Methoxy und (C₁-C₄)-Alkyl;
oder
R(56) und R(57)
gemeinsam 4 oder 5 Methylengruppen, von denen eine CH₂-Gruppe durch Sauerstoff, S, NH, N-CH₃ oder N-Benzyl ersetzt sein kann;
R(58) wie R(56) definiert oder Amidin;
R(3), R(4) und R(5)
unabhängig voneinander wie R(1) oder R(2) definiert; sowie deren pharmazeutisch verträgliche Salze;
q) Benzoylguanidine der Formel I 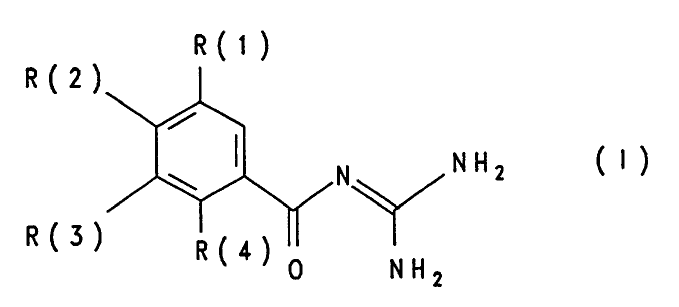 worin bedeuten:
R(1) Wasserstoff, F, Cl, Br, I, -NO₂, -C≡N, -Xₒ-(CH₂)ₚ-(CF₂)_{q}-CF₃, R(5)-SOₘ-, R(6)-CO-, R(6)R(7)N-CO- oder R(6)R(7)N-SO₂-;
X Sauerstoff, -S- oder NR(14);
m Null, 1 oder 2;
o Null oder 1;
p Null, 1 oder 2;
q Null, 1, 2, 3, 4, 5 oder 6;
R(5) und R(6)
(C₁-C₈)-Alkyl, (C₃-C₆)-Alkenyl, -CₙH₂ₙ-R(8) oder CF₃;
n Null, 1, 2, 3 oder 4;
R(8) (C₃-C₇)-Cycloalkyl, Phenyl,
welches nicht substituiert ist oder substituiert mit 1 bis 3 Substituenten ausgewählt aus der Gruppe bestehend aus F, Cl, CF₃, Methyl, Methoxy und NR(9)R(10);
R(9) und R(10)
H oder (C₁-C₄)-Alkyl;
oder
R(6) Wasserstoff;
R(7) Wasserstoff oder (C₁-C₄)-Alkyl;
oder
R(6) und R(7)
gemeinsam 4 oder 5 Methylengruppen, von denen eine CH₂-Gruppe durch Sauerstoff, S, NH, N-CH₃ oder N-Benzyl ersetzt sein kann; 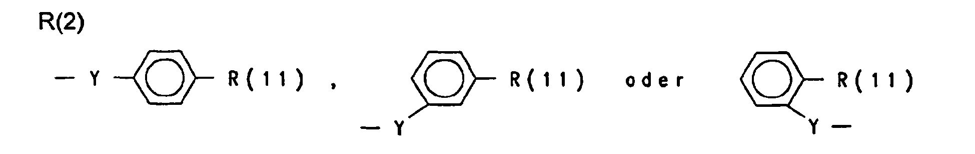 R(11) (C₁-C₉)-Heteroaryl,
das über C oder N verknüpft ist und das unsubstituiert oder substituiert ist mit 1 bis 3 Substituenten ausgewählt aus der Gruppe bestehend aus F, Cl, CF₃, CH₃, Methoxy, Hydroxy, Amino, Methylamino, Dimethylamino und Benzyl;
Y Sauerstoff, -S- oder NR(12);
R(12) H oder (C₁-C₄)-Alkyl;
R(3) wie R(1) definiert;
oder
R(3) (C₁-C₆)-Alkyl oder -X-R(13);
X Sauerstoff, -S- oder NR(14);
R(14) H oder (C₁-C₃)-Alkyl;
R(13) H, (C₁-C₆)-Alkyl, (C₃-C₈)-Cycloalkyl oder -C_{b}H_{2b}-R(15);
b Null, 1, 2, 3 oder 4;
oder
R(13) und R(14)
gemeinsam 4 oder 5 Methylengruppen, von denen eine CH₂-Gruppe durch Sauerstoff, S, NH, N-CH₃ oder N-Benzyl ersetzt sein kann;
R(15) Phenyl,
das unsubstituiert oder substituiert ist mit 1 - 3 Substituenten ausgewählt aus der Gruppe bestehend aus F, Cl, CF₃, Methyl, Methoxy und NR(9)R(10); R(9) und R(10)
H oder (C₁-C₄)-Alkyl;
R(4) Wasserstoff, -OR(16), -NR(16)R(17) oder CᵣF₂ᵣ₊₁;
R(16) und R(17)
unabhängig Wasserstoff oder (C₁-C₃)-Alkyl;
r 1, 2, 3 oder 4;
sowie deren pharmazeutisch verträgliche Salze;
r) Benzokondensierte 5-Ringheterocyclen der Formel I 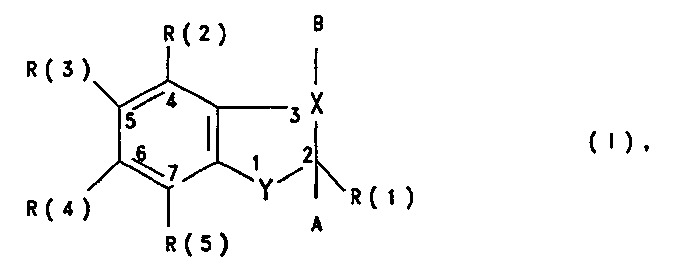 worin bedeuten:
X N oder CR(6);
Y Sauerstoff, S oder NR(7);
A, B gemeinsam eine Bindung oder
A, B beide Wasserstoff, sofern gleichzeitig X gleich CR(6) und Y gleich NR(7) sind; einer der Substituenten R(1) bis R(6) eine -CO-N=C(NH₂)₂-Gruppe;
die jeweils anderen Substituenten R(1) bis R(6)
Wasserstoff, F, Cl, Br, I oder (C₁-C₆)-Alkyl;
bis zu zwei der anderen Substituenten R(1) bis R(6)
CN, NO₂, N₃, (C₁-C₄)-Alkyloxy oder CF₃;
bis zu einem der anderen Substituenten
R(8)-CₙH₂ₙ-Z-;
n Null bis 10;
wobei die Alkylenkette -CₙH₂ₙ- geradkettig oder verzweigt ist und wobei ein C-Atom durch ein Sauerstoff- oder S-Atom oder durch ein N-Atom ersetzt sein kann;
R(8) Wasserstoff, (C₂-C₆)-Alkenyl oder (C₃-C₁₀)-Cycloalkyl,
das unsubstituiert oder durch 1 bis 4 Methylgruppen oder eine OH-Gruppe substituiert ist, oder eine Ethylengruppe -CH=CH-enthalten kann, und worin eine Methylengruppe durch ein Sauerstoff- oder S-Atom oder durch ein N-Atom ersetzt sein kann;
oder
R(8) Phenyl,
unsubstituiert oder substituiert durch 1 bis 3 Substituenten ausgewählt aus der Gruppe bestehend aus F, Cl, Br, I, CF₃, CH₃-S(O)ₛ- oder R(9)-W_{y}-;
s Null, 1 oder 2;
R(9) H, Methyl, Ethyl,
W Sauerstoff oder NR(10);
R(10) H oder Methyl;
y Null oder 1;
oder
R(8) CₘF₂ₘ₊₁;
m 1 bis 3;
oder
R(8) 1- oder 2-Naphthyl, Pyridyl, Chinolyl oder Isochinolyl;
Z -CO-, -CH₂- oder -[CR(11)(OH)]_{q}-;
q 1, 2 oder 3;
R(11) H oder Methyl;
oder
Z Sauerstoff oder -NR(12)-;
R(12) H oder Methyl;
oder
Z -S(O)ₛ-;
s Null, 1 oder 2;
oder
Z -SO₂-NR(13)-;
R(13) H oder (C₁-C₄)-Alkyl;
R(7) Wasserstoff, (C₁-C₁₀)-Alkyl, (C₂-C₁₀)-Alkenyl oder R(8)-CₙH₂ₙ-; sowie deren pharmazeutisch verträgliche Salze;
s) Benzoylguanidine der Formel I 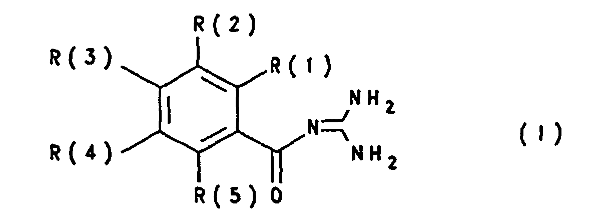 worin bedeuten:
R(1), R(3) oder R(4)
-NR(6) C=X NR(7)R(8);
X Sauerstoff oder S;
R(6) Wasserstoff, (C₁-C₈)-Alkyl, (C₁-C₈)-Perfluoralkyl, (C₃-C₈)-Alkenyl oder -CₙH₂ₙ-R(9);
n Null, 1, 2, 3 oder 4;
R(9) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(10)R(11);
R(10) und R(11)
H, (C₁-C₄)-Alkyl oder (C₁-C₄)-Perfluoralkyl;
R(7) Wasserstoff, (C₁-C₈)-Alkyl, (C₁-C₈)-Perfluoralkyl, (C₃-C₈)-Alkenyl oder -CₒH₂ₒ-R(12);
o Null, 1, 2, 3 oder 4;
R(12) (C₃-C₈)-Cycloalkyl, Phenyl, Biphenylyl oder Naphthyl,
wobei die Aromaten nicht substituiert oder substituiert sind mit 1 - 3 Substituenten ausgewählt aus der Gruppe bestehend aus F, Cl, CF₃, Methyl, Methoxy und NR(13)R(14); R(13) und R(14)
H, (C₁-C₄)-Alkyl oder (C₁-C₄)-Perfluoralkyl;
R(8) definiert wie R(7);
oder
R(7) und R(8)
gemeinsam 4 oder 5 Methylengruppen, von denen eine CH₂-Gruppe durch Sauerstoff, S, NH, N-CH₃ oder N-Benzyl ersetzt sein kann;
die jeweils verbleibenden Substituenten R(2), R(3), R(4), R(5) oder R(1), R(2), R(4), R(5) oder R(1), R(2), R(3), R(5)
unabhängig voneinander Wasserstoff, F, Cl, Br, I, -Oₜₐ(C₁-C₈)-Alkyl, -O_{tb}(C₃-C₈)-Alkenyl,
-O_{tc}(CH₂)_{b}C_{d}F_{2d+1}, -O_{td}CₚH₂ₚR(18),
oder bis zu 2 Gruppen CN, NO₂, NR(16)R(17),
b Null oder 1;
d 1, 2, 3, 4, 5, 6 oder 7;
ta Null oder 1;
tb Null oder 1;
tc Null oder 1;
td Null oder 1;
p Null, 1, 2, 3 oder 4;
R(18) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(19)R(20); R(19) und R(20)
Wasserstoff oder (C₁-C₄)-Alkyl oder (C₁-C₄)-Perfluoralkyl; R(16) Wasserstoff, (C₁-C₈)-Alkyl, (C₁-C₈)-Perfluoralkyl, (C₃-C₈)-Alkenyl, -
C_{q}H_{2q}-R(21),
q Null, 1, 2, 3 oder 4;
R(21) (C₃-C₈)-Cycloalkyl, Phenyl, Biphenylyl oder Naphthyl,
wobei die Aromaten nicht substituiert oder substituiert sind mit 1 - 3 Substituenten aus der Gruppe F, Cl, CF₃, Methyl, Methoxy oder NR(22)R(23),
R(22) und R(23) Wasserstoff, (C₁-C₄)-Alkyl oder (C₁-C₄)-Perfluoralkyl;
R(17) Wasserstoff, (C₁-C₈)-Alkyl, (C₁-C₈)-Perfluoralkyl, (C₃-C₈)-Alkenyl, -CᵣH₂ᵣ-R(24);
r Null, 1, 2, 3 oder 4;
R(24) (C₃-C₈)-Cycloalkyl, Phenyl, Biphenylyl oder Naphthyl,
wobei die Aromaten nicht substituiert oder substituiert sind mit 1 - 3 Substituenten ausgewählt aus der Gruppe bestehend aus F, Cl, CF₃, Methyl, Methoxy und NR(25)R(26);
R(25) und R(26)
Wasserstoff, (C₁-C₄)-Alkyl oder (C₁-C₄)-Perfluoralkyl;
oder
R(16) und R(17)
gemeinsam 4 oder 5 Methylengruppen, von denen eine CH₂-Gruppe
durch Sauerstoff, S, NH, N-CH₃ oder N-Benzyl ersetzt sein kann; sowie deren pharmazeutisch verträgliche Salze;
t) Diacylsubstituierte Guanidine der Formel I 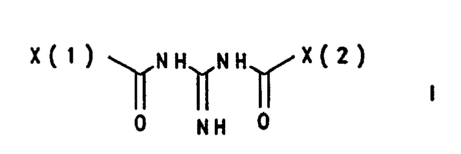 worin bedeuten:
X(1) und X(2) 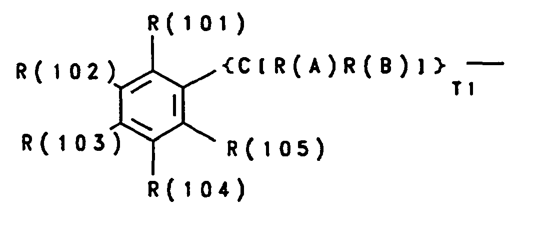
T1 Null, 1, 2, 3 oder 4;
R(A) und R(B)
unabhängig Wasserstoff, F, Cl, Br, I, CN, OR(106), (C₁-C₈)-Alkyl, (C₃-C₈)-Cycloalkyl, O_{zk}(CH₂)_{zl}C_{zm}F_{2zm+1}, NR(107)R(108), Phenyl oder Benzyl,
wobei die Aromaten nicht substituiert oder substituiert sind mit 1 - 3 Substituenten ausgewählt aus der Gruppe bestehend aus F, Cl, CF₃, Methyl, Methoxy und NR(109)R(110);
R(109) und R(110)
Wasserstoff, (C₁-C₄)-Alkyl oder (C₁-C₄)-Perfluoralkyl;
zl Null, 1, 2, 3 oder 4;
zk Null oder 1;
zm 1, 2, 3, 4, 5, 6, 7 oder 8;
R(106)
Wasserstoff, (C₁-C₈)-Alkyl, (C₁-C₈)-Perfluoralkyl, (C₃-C₈)-Alkenyl, (C₃-C₈)-Cycloalkyl, Phenyl oder Benzyl,
wobei die Aromaten nicht substituiert oder substituiert sind mit 1 - 3 Substituenten ausgewählt aus der Gruppe bestehend aus F, Cl, CF₃, Methyl, Methoxy und NR(111)R(112); R(111) und R(112)
Wasserstoff, (C₁-C₄)-Alkyl oder (C₁-C₄)-Perfluoralkyl;
R(107) und R(108)
unabhängig voneinander wie R(106) definiert,
oder
R(107) und R(108) gemeinsam 4 oder 5 Methylengruppen, von denen eine CH₂-Gruppe
durch Sauerstoff, S, NH, N-CH₃ oder N-Benzyl ersetzt sein kann;
oder
X(1) und X(2) 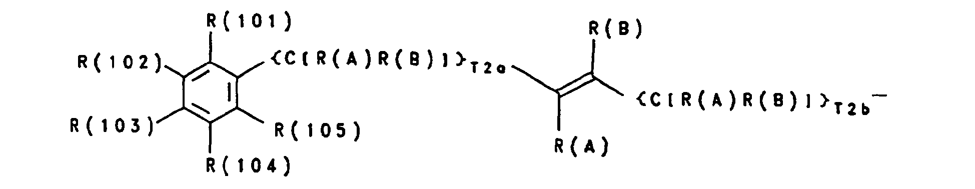 T2a und T2b
unabhängig voneinander Null, 1 oder 2;
wobei die Doppelbindung (E)- oder (Z)-konfiguriert sein kann; oder
X(1) und X(2) 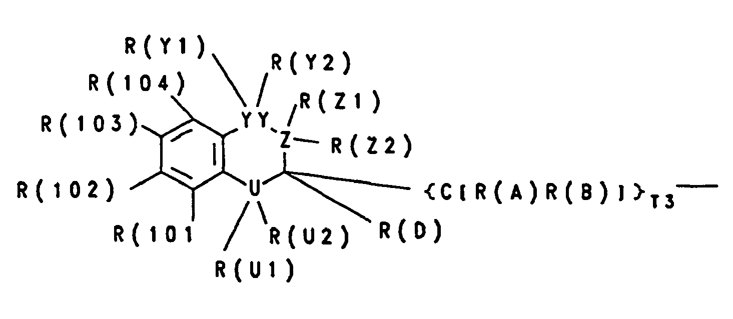 T3 Null, 1 oder 2;
U, YY und Z
unabhängig voneinander C oder N,
wobei U, YY, Z folgende Anzahl von Substituenten tragen können:
| U, YY oder Z | im Ring an eine Doppelbindung gebunden | Anzahl der zugelassenen Substituenten |
|---|---|---|
| C | ja | 1 |
| C | nein | 2 |
| N | ja | 0 |
| N | nein | 1 |
R(D) Wasserstoff, (C₁-C₈)-Alkyl oder (C₁-C₈)-Perfluoralkyl,
R(U1), R(U2), R(Y1), R(Y2), R(Z1), R(Z2)
unabhängig voneinander Wasserstoff, F, Cl, Br, I, CN, OR(114), (C₁-C₈)-Alkyl, (C₃-C₈)-Cycloalkyl, O_{zka}(CH₂)_{zla}C_{zma}F_{2zma+1}, NR(115)R(116), Phenyl oder Benzyl,
wobei die Aromaten nicht substituiert oder substituiert sind mit 1 - 3 Substituenten ausgewählt aus der Gruppe bestehend aus F, Cl, CF₃, Methyl, Methoxy, NR(117)R(118),
R(117) und R(118)
Wasserstoff, (C₁-C₄)-Alkyl oder (C₁-C₄)-Perfluoralkyl,
zka Null oder 1;
zla Null, 1, 2, 3 oder 4;
zma 1, 2, 3, 4, 5, 6, 7 oder 8;
R(114)
Wasserstoff, (C₁-C₈)-Alkyl, (C₁-C₈)-Perfluoralkyl, (C₃-C₈)-Alkenyl, (C₃-C₈)-Cycloalkyl, Phenyl oder Benzyl,
wobei die Aromaten nicht substituiert oder substituiert sind mit 1 - 3 Substituenten ausgewählt aus der Gruppe bestehend aus F, Cl, CF₃, Methyl, Methoxy und NR(119)R(120);
R(119) und R(120)
Wasserstoff, (C₁-C₄)-Alkyl oder (C₁-C₄)-Perfluoralkyl;
R(115) und R(116) unabhängig voneinander wie R(114) definiert;
oder
R(115) und R(116)
gemeinsam 4 oder 5 Methylengruppen, von denen eine CH₂-Gruppe durch Sauerstoff, S, NH, N-CH₃ oder N-Benzyl ersetzt sein kann; wobei jedoch die Konstitution U gleich Stickstoff (N), YY gleich Stickstoff (N) und Z gleich Kohlenstoff (C) ausgenommen ist,
R(101), R(102), R(103), R(104) und R(105)
unabhängig voneinander Wasserstoff, F, Cl, Br, I, -C≡N, X_{zoa}-(CH₂)_{zpa}-(C_{zqa}F_{2zqa+1}), R(110a)-SO_{zbm}, R(110b)R(110c)N-CO, R(111a)-CO- oder R(112a)R(113a)N-SO₂-,
wobei die Perfluoralkylgruppe geradkettig oder verzweigt ist,
X Sauerstoff, S oder NR(114a);
R(114a)
H oder (C₁-C₃)-Alkyl;
zoa Null oder 1;
zbm Null, 1 oder 2;
zpa Null, 1, 2, 3 oder 4;
zqa 1, 2, 3, 4, 5, 6, 7 oder 8;
R(110a), R(110b), R(111a) und R(112a) unabhängig (C₁-C₈)-Alkyl, (C₃-C₈)-Alkenyl, -C_{zn}H_{2zn}-R(115a) oder (C,-C₈)-Perfluoralkyl;
zn Null, 1, 2, 3 oder 4;
R(115a)
(C₃-C₈)-Cycloalkyl, Phenyl, Biphenylyl oder Naphthyl, wobei die Aromaten nicht substituiert sind oder substituiert mit 1 - 3 Substituenten ausgewählt aus der Gruppe bestehend aus F, Cl, CF₃, Methyl, Methoxy und NR(116a)R(117a);
R(116a) und R(117a)
Wasserstoff, (C₁-C₄)-Perfluoralkyl oder (C₁-C₄)-Alkyl;
oder
R(110b), R(111a) und R(112a)
Wasserstoff;
R(110c) und R(113a)
unabhängig Wasserstoff, (C₁-C₄)-Perfluoralkyl oder (C₁-C₄)-Alkyl;
oder
R(110b) und R(110c) sowie R(112a) und R(113a)
gemeinsam 4 oder 5 Methylengruppen, von denen eine CH₂-Gruppe durch Sauerstoff, Schwefel, NH, N-CH₃ oder N-Benzyl ersetzt sein kann;
oder
R(101), R(102), R(103), R(104), R(105)
unabhängig voneinander (C₁-C₈)-Alkyl, -C_{zal}H_{2zal}R(118a) oder (C₃-C₈)-Alkenyl, zal Null, 1, 2, 3 oder 4;
R(118a)
(C₃-C₈)-Cycloalkyl, Phenyl, Biphenylyl oder Naphthyl,
wobei die Aromaten nicht substituiert oder substituiert sind mit 1 - 3 Substituenten aus der Gruppe bestehend aus F, Cl, CF₃, Methyl, Methoxy oder NR(119a)R(119b); R(119a) und R(119b)
Wasserstoff, (C₁-C₄)-Alkyl oder (C₁-C₄)-Perfluoralkyl; oder
R(101), R(102), R(103), R(104), R(105)
unabhängig voneinander (C₁-C₉)-Heteroaryl, das über C oder N verknüpft ist und das unsubstituiert oder substituiert ist mit 1 - 3 Substituenten aus der Gruppe bestehend aus F, Cl, CF₃, CH₃, Methoxy, Hydroxy, Amino, Methylamino und Dimethylamino;
oder
R(101), R(102), R(103), R(104), R(105)
unabhängig voneinander -C≡C-R(193);
R(193)
Phenyl, das nicht substituiert oder substituiert ist mit 1 - 3 Substituenten aus der Gruppe bestehend aus F, Cl, CF₃, Methyl, Methoxy oder NR(194)R(195);
R(194) und R(195)
Wasserstoff oder CH₃;
oder
R(101), R(102), R(103), R(104), R(105)
unabhängig voneinander
-Y-para-C₆H₄-(CO)_{zh}-(CHOH)_{zi}-(CH2)_{zj}-(CHOH)_{zk}-R(123),
-Y-meta-C₆H₄-(CO)_{zad}-(CHOH)_{zae}-(CH₂)_{zaf}-(CHOH)_{zag}-R(124) oder
-Y-ortho-C6H4-(CO)_{zah}-(CHOH)_{zao}-(CH₂)_{zap}-(CHOH)_{zak}-R(125);
Y Sauerstoff, -S- oder -NR(122d)-;
zh, zad, zah
unabhängig Null oder 1;
zi, zj, zk, zae, zaf, zag, zao, zap und zak
unabhängig Null, 1, 2, 3 oder 4;
wobei jedoch jeweils
zh, zi und zk nicht gleichzeitig Null,
zad, zae und zag nicht gleichzeitig Null sowie
zah, zao und zak nicht gleichzeitig Null sind,
R(123), R(124) R(125) und R(122d)
unabhängig Wasserstoff oder (C₁-C₃)-Alkyl;
oder
R(101), R(102), R(103), R(104) und R(105)
unabhängig voneinander SR(129), -OR(130), -NR(131)R(132) oder -CR(133)R(134)R(135);
R(129), R(130), R(131) und R(133)
unabhängig -C_{zab}H_{2zab}-(C₁-C₉)-Heteroaryl,
das unsubstituiert oder substituiert ist mit 1 - 3 Substituenten aus der Gruppe bestehend aus F, Cl, CF₃, CH₃, Methoxy, Hydroxy, Amino, Methylamino und Dimethylamino;
zab Null, 1 oder 2;
R(132), R(134) und R(135)
unabhängig voneinander wie R(129) definiert oder Wasserstoff, (C₁-C₄)-Alkyl oder (C₁-C₄)-Perfluoralkyl;
oder
R(101), R(102), R(103), R(104) und R(105) unabhängig voneinander -W-para-(C₆H₄)-R(196), -W-meta-(C₆H₄)-R(197) oder -W ortho-(C₆H₄)-R(198);
R(196), R(197) und R(198)
unabhängig (C₁-C₉)-Heteroaryl,
das über C oder N verknüpft ist und das unsubstituiert oder substituiert ist mit 1 bis 3 Substituenten aus der Gruppe bestehend aus F, Cl, CF₃, CH₃, Methoxy, Hydroxy, Amino, Methylamino, Dimethylamino und Benzyl;
W Sauerstoff, S oder NR(136)-;
R(136)
Wasserstoff oder (C₁-C₄)-Alkyl;
oder
R(101), R(102), R(103), R(104) und R(105)
unabhängig voneinander R(146)X(1a)-;
X(1a)
Sauerstoff, S, NR(147), (D=O)A-, NR(148)C=MN⁽*⁾R(149)-;
M Sauerstoff oder Schwefel;
A Sauerstoff oder NR(150);
D C oder SO;
R(146)
(C₁-C₈)-Alkyl, (C₃-C₈)-Alkenyl, (CH₂)_{zbz}C_{zdz}F_{2zdz+1} oder -C_{zxa}H_{2zxa}-R(151); zbz Null oder 1;
zdz 1, 2, 3,4, 5, 6 oder 7;
zxa Null, 1, 2, 3 oder 4;
R(151)
(C₃-C₈)-Cycloalkyl, Phenyl, Biphenylyl oder Naphthyl, wobei die Aromaten nicht substituiert oder substituiert sind mit 1 - 3 Substituenten aus der Gruppe bestehend aus F, Cl, CF₃, Methyl, Methoxy und NR(152)R(153); R(152) und R(153)
Wasserstoff, (C₁-C₄)-Alkyl oder (C₁-C₄)-Perfluoralkyl;
R(147), R(148) und R(150)
unabhängig Wasserstoff, (C₁-C₄)-Alkyl, (C₁-C₄)-Perfluoralkyl;
R(149) definiert wie R(146),
oder
R(146) und R(147) beziehungsweise R(146) und R(148) gemeinsam 4 oder 5 Methylengruppen, von denen eine CH₂-Gruppe durch Sauerstoff, Schwefel, NH, N-CH₃ oder N-Benzyl ersetzt sein kann;
wobei A und N^{(*)} an den Phenylkern des Alkanoyl-Grundkörpers gebunden sind;
oder
R(101), R(102), R(103), R(104) und R(105) unabhängig voneinander -SR(164), -OR(165), -NHR(166), -NR(167)R(168), - CHR(169)R(170), -CR(154)R(155)OH, -C≡CR(156), -CR(158)=CR(157) oder -[CR(159)R(160)]_{zu}-(C=O)-[CR(161)R(162)]_{zv}-R(163);
R(164), R(165), R(166), R(167), R(169)
gleich oder verschieden -(CH₂)_{zy}-(CHOH)_{zz}-(CH₂)_{zaa}-(CHOH)_{zt}-R(171) oder -(CH₂)_{zab}-O-(CH₂-CH₂O)_{zac}-R(172);
R(171) und R(172)
Wasserstoff oder Methyl;
zu 1, 2, 3 oder 4;
zv Null, 1, 2, 3 oder 4;
zy, zz, zaa, zab, zac
gleich oder verschieden Null, 1, 2, 3 oder 4;
zt 1, 2, 3 oder 4;
R(168), R(170), R(154), R(155)
gleich oder verschieden Wasserstoff oder (C₁-C₆)-Alkyl,
oder
R(169) und R(170) beziehungsweise R(154) und R(155)
zusammen mit dem sie tragenden Kohlenstoff-Atom ein (C₃-C₈)-Cycloalkyl;
R(163)
Wasserstoff, (C₁-C₆)-Alkyl, (C₃-C₈)-Cycloalkyl oder -C_{zeb}H_{2zeb}-R(173);
zeb Null, 1, 2, 3 oder 4;
R(156), R(157) und R(173)
unabhängig Phenyl, das unsubstituiert oder substituiert ist mit 1 - 3 Substituenten aus der Gruppe bestehend aus F, Cl, CF₃, Methyl, Methoxy und NR(174)R(175);
R(174) und R(175)
Wasserstoff oder (C₁-C₄)-Alkyl;
oder
R(156), R(157) und R(173)
unabhängig (C₁-C₉)-Heteroaryl, das unsubstituiert oder wie Phenyl substituiert ist;
R(158), R(159), R(160), R(161) und R(162)
Wasserstoff oder Methyl,
oder
R(101), R(102), R(103), R(104), R(105)
unabhängig voneinander R(176)-NH-SO₂-;
R(176)
R(177)R(178)N-(C=Y')-;
Y' Sauerstoff, S oder N-R(179);
R(177) und R(178)
gleich oder verschieden Wasserstoff, (C₁-C₈)-Alkyl, (C₃-C₆)-Alkenyl oder -C_{zfa}H_{2zfa}-R(180);
zfa Null, 1, 2, 3 oder 4;
R(180)
(C₅-C₇)-Cycloalkyl oder Phenyl,
welches unsubstituiert mit 1 - 3 Substituenten ausgewählt aus der Gruppe bestehend aus F, Cl, CF₃, Methoxy oder (C₁-C₄)-Alkyl;
oder
R(177) und R(178)
gemeinsam 4 oder 5 Methylengruppen, von denen eine CH₂-Gruppe durch Sauerstoff, Schwefel, NH, N-CH₃ oder N-Benzyl ersetzt sein kann;
R(179)
wie R(177) definiert oder gleich Amidin,
oder
R(101), R(102), R(103), R(104), R(105) unabhängig voneinander NR(184a)R(185), OR(184b), SR(184c) oder -C_{znx}H_{2znx}-R(184d);
znx Null, 1, 2, 3 oder 4;
R(184d)
(C₃-C₇)-Cycloalkyl oder Phenyl,
welches nicht substituiert ist oder substituiert mit 1 - 3 Substituenten aus der Gruppe bestehend aus F, Cl, CF₃, Methyl, Methoxy und NR(116k)R(117k); R(116k) und R(117k)
Wasserstoff oder C₁-C₄-Alkyl;
R(184a), R(184b), R(184c), R(185) unabhängig voneinander Wasserstoff, (C₁-C₈)-Alkyl, (C₁-C₈)-Perfluoralkyl oder (CH₂)_{zao}-R(184g);
zao Null, 1, 2, 3 oder 4;
184g
(C₃-C₇)-Cycloalkyl oder Phenyl, welches nicht substituiert ist oder substituiert mit 1 - 3 Substituenten aus der Gruppe bestehend aus F, Cl, CF₃, Methyl, Methoxy und NR(184u)R(184v); R(184u) und R(184v)
Wasserstoff oder C₁-C₄-Alkyl;
oder
R(184a) und R(185)
gemeinsam 4 oder 5 Methylengruppen, von denen eine CH₂-Gruppe durch Sauerstoff, Schwefel, NH,
N-CH₃ oder N-Benzyl ersetzt sein kann;
sowie deren pharmazeutisch verträgliche Salze;
u) Benzoylguanidine der Formel I 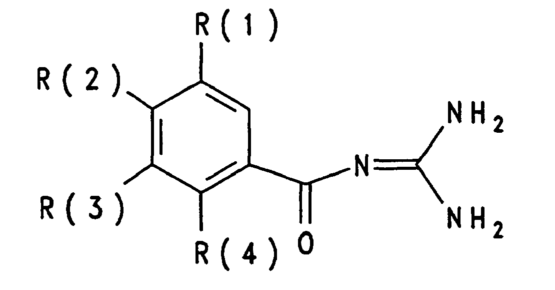 worin bedeuten:
R(1) H, F, Cl, Br, I, CN, NO₂, (C₁-C₈)-Alkyl, (C₃-C₈)-Cycloalkyl oder Xₐ-(CH₂)_{b}-(CF₂)_{c}-CF₃;
X Sauerstoff, S oder NR(5);
a Null oder 1;
b Null, 1 oder 2;
c Null, 1, 2 oder 3;
R(5) H, (C₁-C₄)-Alkyl oder -C_{d}H_{2d}R(6);
d Null, 1, 2, 3 oder 4;
R(6) (C₃-C₈)-Cycloalkyl, Phenyl, Biphenylyl oder Naphthyl,
wobei die Aromaten nicht substituiert oder substituiert sind mit 1 bis 3 Substituenten ausgewählt aus der Gruppe bestehend aus F, Cl, CF₃, Methyl, Methoxy und NR(7)R(8); R(7) und R(8)
unabhängig H oder (C₁-C₄)-Alkyl;
oder
R(1) -SR(10), -OR(10) oder -CR(10)R(11)R(12);
R(10) -C_{f}H_{2f}-(C₃-C₈)-Cycloalkyl, -(C₁-C₉)-Heteroaryl oder Phenyl,
wobei die aromatischen Systeme unsubstituiert oder substituiert sind mit 1 bis 3 Substituenten ausgewählt aus der Gruppe bestehend aus F, Cl, CF₃, CH₃, Methoxy, Hydroxy, Amino, Methylamino und Dimethylamino;
f Null, 1 oder 2;
R(11) und R(12)
unabhängig voneinander wie R(10) definiert oder Wasserstoff oder
(C₁-C₄)-Alkyl;
oder
R(1) Phenyl, Naphthyl, Biphenylyl oder (C₁-C₉)-Heteroaryl, letzteres über C oder N verknüpft,
und die unsubstituiert oder substituiert sind mit 1 bis 3 Substituenten ausgewählt aus der Gruppe bestehend aus F, Cl, CF₃, CH₃, Methoxy, Hydroxy, Amino, Methylamino und Dimethylamino;
oder
R(1) -SR(13), -OR(13), -NHR(13), -NR(13)R(14), -CHR(13)R(15), -C[R(15)R(16)]OH, -C≡CR(18), -C[R(19)]=CR(18), -[CR(20)R(21)]ₖ-(CO)-[CR(22)R(23)R(24)],
R(13) und R(14)
gleich oder verschieden -(CH₂)_{g}-(CHOH)ₕ-(CH₂)ᵢ-(CHOH)ⱼ-R(17), R(17) Wasserstoff oder Methyl;
-(CH₂)_{g}-O-(CH₂-CH₂O)ₕ-R(24),
g, h, i
gleich oder verschieden Null, 1, 2, 3 oder 4;
j 1, 2, 3 oder 4;
R(15) und R(16)
gleich oder verschieden Wasserstoff, (C₁-C₆)-Alkyl oder zusammen mit dem sie tragenden Kohlenstoff-Atom ein (C₃-C₈)-Cycloalkyl;
R(18) Phenyl,
das unsubstituiert oder substituiert ist mit 1 bis 3 Substituenten ausgewählt aus der Gruppe bestehend aus F, Cl, CF₃, Methyl, Methoxy und NR(25)R(26);
R(25) und R(26)
H oder (C₁-C₄)-Alkyl;
oder
R(18) (C₁-C₉)-Heteroaryl,
das unsubstituiert oder wie Phenyl substituiert ist;
oder
R(18) (C₁-C₆)-Alkyl,
das unsubstituiert oder mit 1 bis 3 OH substituiert ist;
oder
R(18) (C₃-C₈)-Cycloalkyl;
R(19), R(20), R(21), R(22) und R(23)
Wasserstoff oder Methyl;
k Null, 1, 2, 3 oder 4;
l Null, 1, 2, 3 oder 4;
R(24) H, (C₁-C₆)-Alkyl, (C₃-C₈)-Cycloalkyl oder -CₘH₂ₘ-R(18);
m 1, 2, 3 oder 4;
R(2) und R(3)
unabhängig voneinander wie R(1) definiert;
R(4) (C₁-C₃)-Alkyl, F, Cl, Br, I, CN oder -(CH₂)ₙ-(CF₂)ₒ-CF₃;
n Null oder 1;
o Null, 1 oder 2;
sowie deren pharmazeutisch verträgliche Salze;
v) Acylguanidine der Formel I 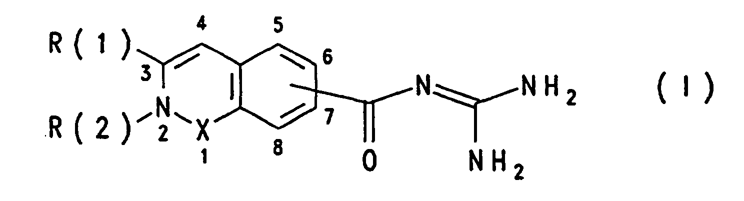
worin bedeuten:
X Carbonyl, Sulfonyl,
R(1) H, (C₁-C₈)-Alkyl,
unsubstituiert oder durch Hydroxy substituiert, (C₃-C₈)-Cycloalkyl, Phenyl, das unsubstituiert oder substituiert ist mit 1 - 3 Substituenten aus der Gruppe F, Cl, CF₃, CH₃, Methoxy, Hydroxy, Amino, Methylamino oder Dimethylamino,
R(2) H, (C₁-C₄)-Alkyl,
sowie deren pharmazeutisch verträgliche Salze;
w) Perfluoralkylgruppen tragende phenylsubstituierte Alkylcarbonsäure-guanidide der Formel I 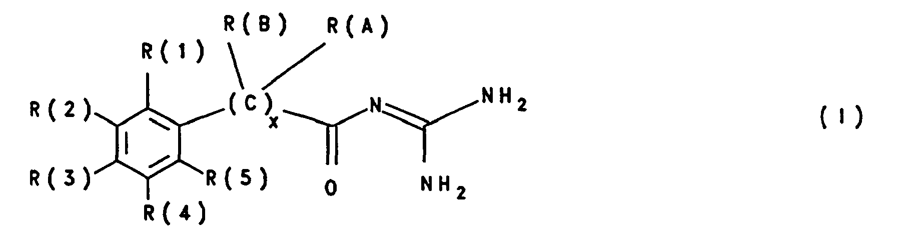 worin bedeuten:
R(A) Wasserstoff, F, Cl, Br, I, CN, OR(6), (C₁-C₈)-Alkyl, (C₃-C₈)-Cycloalkyl,
Oᵣ(CH₂)ₐC_{b}F_{2b+1} oder NR(7)R(8);
r Null oder 1;
a Null, 1, 2, 3 oder 4;
b 1, 2, 3, 4, 5, 6, 7 oder 8;
R(6) Wasserstoff, (C₁-C₈)-Alkyl, (C₃-C₈)-Alkenyl, (C₃-C₈)-Cycloalkyl, Phenyl oder Benzyl,
wobei die Aromaten nicht substituiert oder substituiert sind mit 1 - 3 Substituenten ausgwählt aus der Gruppe bestehend aus F, Cl, CF₃, Methyl, Methoxy und NR(9)R(10); R(9) und R(10)
H, (C₁-C₄)-Alkyl oder (C₁-C₄)-Perfluoralkyl;
R(7) und R(8)
unabhängig voneinander wie R(6) definiert;
R(B) unabhängig wie R(A) definiert;
X 1, 2 oder 3;
R(1) Wasserstoff, (C₁-C₈)-Alkyl, (C₃-C₈)-Cycloalkyl, -Oₜ(CH₂)_{d}CₑF₂ₑ₊₁, F, Cl, Br, I oder CN;
t Null oder 1;
d Null, 1, 2, 3 oder 4;
e 1, 2, 3, 4, 5, 6, 7 oder 8;
R(2), R(3), R(4) und R(5)
unabhängig voneinander wie R(1) definiert;
jedoch unter der Bedingung,
daß mindestens einer der Substituenten R(1), R(2), R(3), R(4), R(5), R(A) und R(B) eine -Oₜ(CH₂)_{d}CₑF₂ₑ₊₁ oder eine Oᵣ(CH₂)ₐC_{b}F_{2b+1}-Gruppe ist, sowie deren pharmazeutisch verträgliche Salze;
x) Heteroaroylguanidine der Formel I 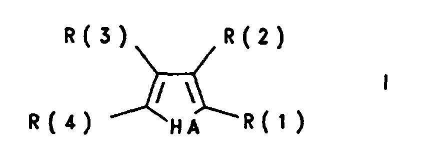 worin bedeuten:
HA SOₘ, O oder NR(5);
m Null, 1 oder 2;
R(5) Wasserstoff, (C₁-C₈)-Alkyl oder -CₐₘH₂ₐₘR(81);
am Null, 1 oder 2;
R(81) (C₃-C₈)-Cycloalkyl oder Phenyl, welches nicht substituiert oder substituiert ist mit 1 - 3 Substituenten ausgewählt aus der Gruppe bestehend aus F, Cl, CF₃, Methyl, Methoxy und NR(82)R(83); R(82) und R(83)
H oder CH₃;
oder
R(81) (C₁-C₉)-Heteroaryl,
das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
einer der beiden Substituenten R(1) und R(2)
-CO-N=C(NH₂)₂;
und der jeweils andere
Wasserstoff, F, Cl, Br, I, (C₁-C₃)-Alkyl, -OR(6), CᵣF₂ᵣ₊₁, -CO-N=C(NH₂)_{2 oder} - NR(6)R(7);
R(6) und R(7)
unabhängig Wasserstoff oder (C₁-C₃)-Alkyl;
r 1, 2, 3 oder 4;
R(3) und R(4)
unabhängig voneinander Wasserstoff, F, Cl, Br, I, -C=N, X-(CH₂)ₚ-(C_{q}-F_{2q+1}), R(8)-SO_{bm}, R(9)R(10)N-CO, R(11)-CO- oder R(12)R(13)N-SO₂-,
wobei die Perfluoralkylgruppe geradkettig oder verzweigt ist,
X Sauerstoff, S oder NR(14);
R(14) H oder (C₁-C₃)-Alkyl;
bm Null, 1 oder 2;
p Null, 1 oder 2;
q Null, 1, 2, 3, 4, 5 oder 6;
R(8), R(9), R(11) und R(12)
unabhängig (C₁-C₈)-Alkyl, (C₃-C₆)-Alkenyl, -CₙH₂ₙ-R(15), CF₃;
n Null, 1, 2, 3 oder 4;
R(15) (C₃-C₇)-Cycloalkyl oder Phenyl;
welches nicht substituiert ist oder substituiert mit 1 - 3 Substituenten ausgewählt aus der Gruppe bestehend aus F, Cl, CF₃, Methyl, Methoxy oder NR(16)R(17); R(16) und R(17)
H oder C₁-C₄-Alkyl;
oder
R(9), R(11) und R(12)
H;
R(10) und R(13)
unabhängig H oder (C₁-C₄)-Alkyl;
oder
R(9) und R(10) sowie R(12) und R(13)
gemeinsam 4 oder 5 Methylengruppen, von denen eine CH₂-Gruppe durch Sauerstoff, S, NH, N-CH₃ oder N-Benzyl ersetzt sein kann,
oder
R(3) und R(4) unabhängig voneinander (C₁-C₈)-Alkyl oder -CₐₗH₂ₐₗR(18);
al Null, 1 oder 2;
R(18) (C₃-C₈)-Cycloalkyl oder Phenyl; welches nicht substituiert oder substituiert ist mit 1 - 3 Substituenten ausgewählt aus der Gruppe bestehend aus F, Cl, CF₃, Methyl, Methoxy und NR(19)R(20); R(19) und R(20)
H oder CH₃;
oder
R(3) und R(4)
unabhängig voneinander (C₁-C₉)-Heteroaryl,
das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(3) und R(4)
unabhängig voneinander 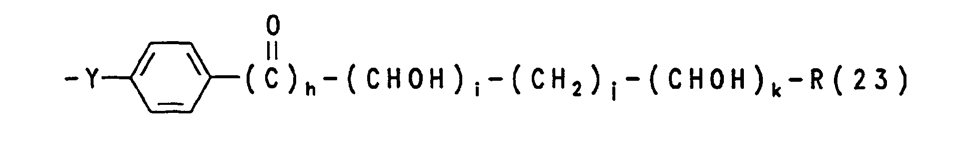 oder 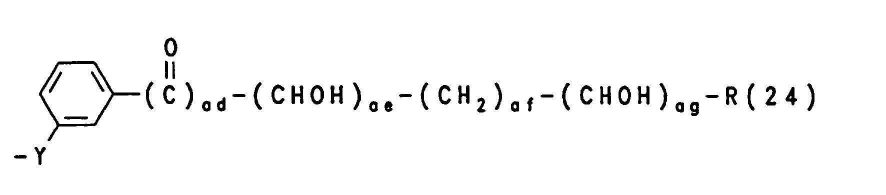 oder 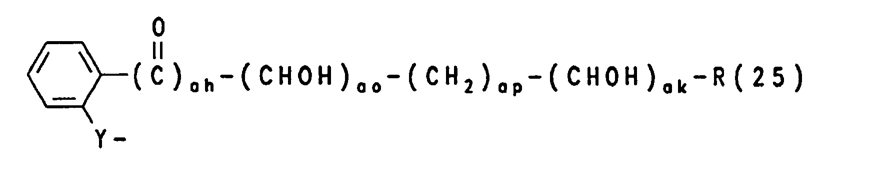 Y Sauerstoff, -S- oder -NR(22)-;
h, ad, ah unabhängig Null oder 1;
i, j, k, ae, af, ag, ao, ap und ak unabhängig Null, 1, 2, 3, 4,
wobei jedoch jeweils
h, i und k nicht gleichzeitig Null,
ad, ae und ag nicht gleichzeitig Null sowie
ah, ao und ak nicht gleichzeitig Null sind,
R(23), R(24) R(25) und R(22)
unabhängig Wasserstoff oder (C₁-C₃)-Alkyl;
oder
R(3) und R(4)
unabhängig voneinander Wasserstoff, F, Cl, Br, I, CN, (C₁-C₈)-Alkyl, (C₁-C₈)-Perfluoralkyl, (C₃-C₈)-Alkenyl oder -C_{g}H_{2g}R(26);
g Null, 1, 2, 3 oder 4;
R(26) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(27)R(28);
R(27) und R(28)
H, (C₁-C₄)-Alkyl oder (C₁-C₄)-Perfluoralkyl;
oder
R(3) und R(4)
unabhängig voneinander SR(29), -OR(30), -NR(31)R(32) oder -CR(33)R(34)R(35);
R(29), R(30), R(31) und R(33)
unabhängig -CₐH₂ₐ-(C₁-C₉)-Heteroaryl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
a Null, 1 oder 2;
R(32), R(34) und R(35)
unabhängig voneinander wie R(29) definiert oder Wasserstoff, (C₁-C₄)-Alkyl oder (C₁-C₄)-Perfluoralkyl;
oder
R(3) und R(4)
unabhängig voneinander 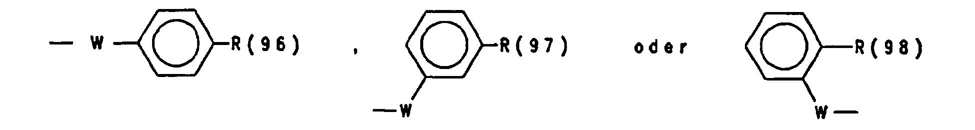 R(96), R(97) und R(98)
unabhängig (C₁-C₉)-Heteroaryl,
das über C oder N verknüpft ist und das unsubstituiert oder substituiert ist mit 1 bis 3 Substituenten ausgewählt aus der Gruppe bestehend aus F, Cl, CF₃, CH₃, Methoxy, Hydroxy, Amino, Methylamino, Dimethylamino oder Benzyl;
W Sauerstoff, S oder NR(36)-;
R(36) H oder (C₁-C₄)-Alkyl;
oder
R(3) und R(4) unabhängig voneinander R(37)-SO_{cm} oder R(38)R(39)N-SO₂-;
cm 1 oder 2;
R(37) (C₁-C₈)-Alkyl, (C₁-C₈)-Perfluoralkyl, (C₃-C₈)-Alkenyl oder -CₛH₂ₛR(40);
s Null, 1, 2, 3 oder 4;
R(40) (C₃-C₈)-Cycloalkyl, Phenyl, Biphenylyl oder Naphthyl,
wobei die Aromaten nicht substituiert oder substituiert sind mit 1 - 3 Substituenten ausgewählt aus der Gruppe bestehend aus F, Cl, CF₃, Methyl, Methoxy und NR(41)R(42);
R(41) und R(42)
H, (C₁-C₄)-Alkyl oder (C₁-C₄)-Perfluoralkyl;
R(38) H, (C₁-C₈)-Alkyl, (C₁-C₈)-Perfluoralkyl, (C₃-C₈)-Alkenyl oder -C_{w}H_{2w}-R(43);
w Null, 1, 2, 3 oder 4;
R(43) (C₃-C₈)-Cycloalkyl, Phenyl, Biphenylyl oder Naphthyl,
wobei die Aromaten nicht substituiert oder substituiert sind mit 1 - 3 Substituenten ausgewählt aus der Gruppe bestehend aus F, Cl, CF₃, Methyl, Methoxy und NR(44)R(45);
R(44) und R(45)
H, (C₁-C₄)-Alkyl oder (C₁-C₄)-Perfluoralkyl;
R(39) H, (C₁-C₄)-Alkyl oder (C₁-C₄)-Perfluoralkyl;
oder
R(38) und R(39)
gemeinsam 4 oder 5 Methylengruppen, von denen eine CH₂-Gruppe
durch Sauerstoff, S, NH, N-CH₃ oder N-Benzyl ersetzt sein kann;
oder
R(3) und R(4) unabhängig voneinander R(46)X(1)-;
X(1) Sauerstoff, S, NR(47), (D=O)A-, NR(48)C=MN⁽*⁾R(49)-,
M Sauerstoff oder S;
A Sauerstoff oder NR(50);
D C oder SO;
R(46) (C₁-C₈)-Alkyl, (C₃-C₈)-Alkenyl, (CH₂)_{b}C_{d}F_{2d+1} oder -CₓH₂ₓ-R(51);
b Null oder 1;
d 1, 2, 3, 4, 5, 6 oder 7;
x Null, 1, 2, 3 oder 4;
R(51) (C₃-C₈)-Cycloalkyl, Phenyl, Biphenylyl oder Naphthyl,
wobei die Aromaten nicht substituiert oder substituiert sind mit 1 - 3 Substituenten ausgewählt aus der Gruppe bestehend aus F, Cl, CF₃, Methyl, Methoxy und NR(52)R(53);
R(52) und R(53)
H, (C₁-C₄)-Alkyl oder (C₁-C₄)-Perfluoralkyl;
R(47), R(48) und R(50)
unabhängig Wasserstoff, (C₁-C₄)-Alkyl oder (C₁-C₄)-Perfluoralkyl;
R(49) definiert wie R(46);
oder R(46) und R(47) beziehungsweise R(46) und R(48)
gemeinsam 4 oder 5 Methylengruppen, von denen eine CH₂-Gruppe durch Sauerstoff, S, NH, N-CH₃ oder N-Benzyl ersetzt sein kann,
wobei A und N^{(*)} an den Phenylkern des Benzoylguanidin-Grundkörpers gebunden sind;
oder
R(3) und R(4)
unabhängig voneinander -SR(64), -OR(65), -NHR(66), -NR(67)R(68), -CHR(69)R(70), -C(OH)R(54)R(55), -C≡CR(56), -CR(58)=CHR(57), -[CR(59)R(60)]ᵤ-(CO)-[CR(61)R(62)]ᵥ-R(63); R(64), R(65), R(66), R(67) und R(69)
gleich oder verschieden -(CH₂)_{y}-(CHOH)_{z}-(CH₂)ₐₐ-(CH₂OH)ₜ-R(71) oder -(CH₂)_{ab}-O-(CH₂-CH₂O)_{ac}-R(72),
R(71) und R(72)
Wasserstoff oder Methyl;
u 1, 2, 3 oder 4;
v Null, 1, 2, 3 oder 4;
y, z, aa
gleich oder verschieden Null, 1, 2, 3 oder 4;
t 1, 2, 3 oder 4;
R(68), R(70), R(54) und R(55)
gleich oder verschieden Wasserstoff, (C₁-C₆)-Alkyl;
oder
R(69) und R(70) beziehungsweise R(54) und R(55)
zusammen mit dem sie tragenden Kohlenstoff-Atom ein (C₃-C₈)-Cycloalkyl;
R(63)
H, (C₁-C₆)-Alkyl, (C₃-C₈)-Cycloalkyl oder -CₑH₂ₑ-R(73);
e Null, 1, 2, 3 oder 4;
R(56), R(57) und R(73)
unabhängig Phenyl,
das unsubstituiert oder substituiert ist mit 1 - 3 Substituenten ausgewählt aus der Gruppe bestehend aus F, Cl, CF₃, Methyl, Methoxy und NR(74)R(75);
R(74) und R(75)
H oder (C₁-C₄)-Alkyl;
oder
R(56), R(57) und R(73)
unabhängig (C₁-C₉)-Heteroaryl,
das unsubstituiert oder wie Phenyl substituiert ist;
R(58), R(59), R(60), R(61) und R(62)
Wasserstoff oder Methyl,
oder
R(3) und R(4)
unabhängig voneinander R(76)-NH-SO₂-;
R(76) R(77)R(78)N-(C=Y')-;
Y' Sauerstoff, S oder N-R(79);
R(77) und R(78)
gleich oder verschieden H, (C₁-C₈)-Alkyl, (C₃-C₆)-Alkenyl, -C_{f}H_{2f}-R(80);
f Null, 1, 2, 3 oder 4;
R(80) (C₅-C₇)-Cycloalkyl oder Phenyl,
welches unsubstituiert oder substituiert mit 1 - 3 Substituenten ausgewählt aus der Gruppe bestehend aus F, Cl, CF₃, Methoxy und (C₁-C₄)-Alkyl;
oder
R(77) und R(78)
gemeinsam 4 oder 5 Methylengruppen, von denen eine CH₂-Gruppe durch Sauerstoff, S, NH, N-CH₃ oder N-Benzyl ersetzt sein kann, R(79)
wie R(77) definiert oder gleich Amidin;
oder
R(3) und R(4)
unabhängig voneinander NR(84)R(85);
R(84) und R(85)
unabhängig voneinander H, (C₁-C₄)-Alkyl, oder gemeinsam 4 oder 5 Methylengruppen, von denen eine CH₂-Gruppe durch Sauerstoff, S, NH, N-CH₃ oder N-Benzyl ersetzt sein kann; oder von denen eine oder zwei
CH₂-Gruppen durch CH-C_{dm}H_{2dm+1} ersetzt sein können, sowie deren pharmazeutisch verträgliche Salze;
y) Bizyklische Heteroaroylguanidine der Formel I 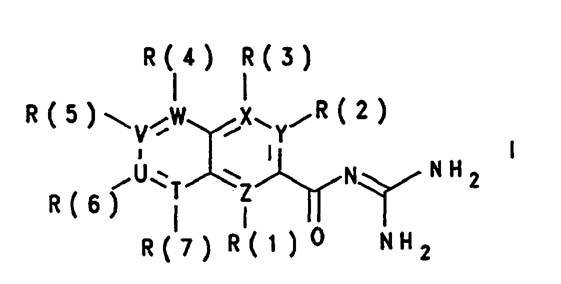 worin bedeuten:
T, U, V, W, X, Y und Z
unabhängig voneinander Stickstoff oder Kohlenstoff; jedoch mit der Einschränkung,
daß X und Z nicht gleichzeitig Stickstoff sind,
und daß T, U, V, W, X, Y und Z keinen Substituenten tragen, wenn sie Stickstoff sind,
und daß nicht mehr als vier von ihnen gleichzeitig Stickstoff sind, R(1) und R(2)
unabhängig voneinander Wasserstoff, F, Cl, Br, I, (C₁-C₃)-Alkyl, (C₁-C₃)-Perfluoralkyl, OR(8), NR(8)R(9) oder C(=O)N=C(NH₂)₂;
R(8) und R(9)
unabhängig voneinander Wasserstoff oder (C₁-C₃)-Alkyl, oder
R(8) und R(9)
gemeinsam 4 oder 5 Methylengruppen, von denen eine CH₂-Gruppe durch Sauerstoff, S, NH, N-CH₃ oder N-Benzyl ersetzt sein kann;
R(3), R(4), R(5), R(6) und R(7)
unabhängig voneinander Wasserstoff, F, Cl, Br, I, -C≡N, Xₖ-(CH₂)ₚ-(C_{q}F_{2q+1}), R(10a)-SO_{bm}, R(10b)R(10c)N-CO, R(11)-CO- oder R(12)R(13)N-SO₂-, wobei die Perfluoralkylgruppe geradkettig oder verzweigt ist;
X Sauerstoff, S oder NR(14);
R(14) H oder C₁-C₃)-Alkyl;
bm Null, 1 oder 2;
p Null, 1 oder 2;
k Null oder 1;
q 1, 2, 3, 4, 5 oder 6;
R(10a), R(10b), R(11) und R(12)
unabhängig voneinander (C₁-C₈)-Alkyl, (C₃-C₆)-Alkenyl, -CₙH₂ₙ-R(15) oder (C₁-C₈)-Perfluoralkyl;
n Null, 1, 2, 3 oder 4;
R(15) (C₃-C₇)-Cycloalkyl oder Phenyl, welches nicht substituiert ist oder substituiert mit 1 - 3 Substituenten ausgewählt aus der Gruppe bestehend aus F, Cl, CF₃, Methyl, Methoxy und NR(16)R(17);
R(16) und R(17)
H oder C₁-C₄-Alkyl;
oder
R(10b), R(11) und R(12)
Wasserstoff;
R(10c) und R(13)
unabhängig Wasserstoff oder (C₁-C₄)-Alkyl;
oder
R(10b) und R(10c) sowie R(12) und R(13)
gemeinsam 4 oder 5 Methylengruppen, von denen eine CH₂-Gruppe durch Sauerstoff, Schwefel, NH, N-CH₃ oder N-Benzyl ersetzt sein kann;
oder
R(3), R(4), R(5), R(6) und R(7)
unabhängig voneinander (C₁-C₈)-Alkyl, -CₐₗH₂ₐₗR(18) oder (C₃-C₈)-Alkenyl;
al Null, 1 oder 2;
R(18) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(19a)R(19b);
R(19a) und R(19b)
Wasserstoff, (C₁-C₄)-Alkyl oder (C₁-C₄)-Perfluoralkyl;
oder
R(3), R(4), R(5), R(6) und R(7)
unabhängig voneinander (C₁-C₉)-Heteroaryl, das über C oder N verknüpft ist und das unsubstituiert oder substituiert ist mit 1 - 3 Substituenten aus der Gruppe bestehend aus F, Cl, CF₃, CH₃, Methoxy, Hydroxy, Amino,
Methylamino oder Dimethylamino;
oder
R(3), R(4), R(5), R(6) und R(7)
unabhängig voneinander 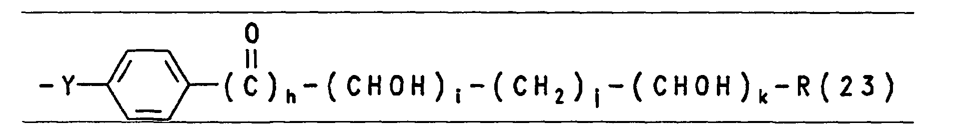 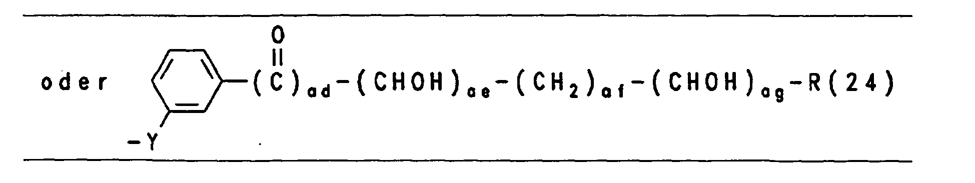 oder 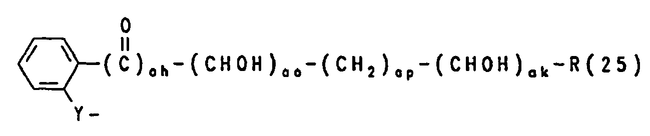 Y Sauerstoff, -S- oder -NR(22)-;
h, ad, ah
unabhängig voneinander Null oder 1;
i, j, k, ae, af, ag, ao, ap und ak
unabhängig voneinander Null, 1, 2, 3 oder 4;
wobei jedoch jeweils
h, i und k nicht gleichzeitig Null,
ad, ae und ag nicht gleichzeitig Null sowie
ah, ao und ak nicht gleichzeitig Null sind,
R(23), R(24) R(25) und R(22)
unabhängig voneinander Wasserstoff oder (C₁-C₃)-Alkyl; oder
R(3), R(4), R(5), R(6) und R(7)
unabhängig voneinander SR(29), -OR(30), -NR(31)R(32) oder -CR(33)R(34)R(35);
R(29), R(30), R(31) und R(33)
unabhängig voneinander -CₐH₂ₐ-(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
a Null, 1 oder 2;
R(32), R(34) und R(35)
unabhängig voneinander wie R(29) definiert oder Wasserstoff, (C₁-C₄)-Alkyl oder (C₁-C₄)-Perfluoralkyl;
oder
R(3), R(4), R(5), R(6) und R(7)
unabhängig voneinander 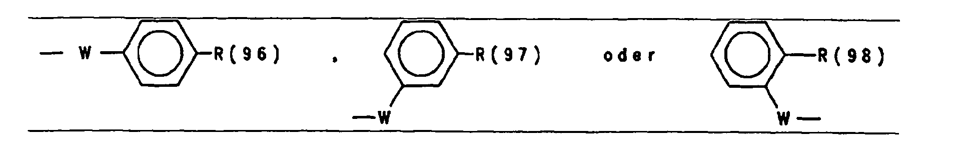 R(96), R(97) und R(98)
unabhängig voneinander (C₁-C₉)-Heteroaryl, das über C oder N verknüpft ist und das unsubstituiert oder substituiert ist mit 1 bis 3 Substituenten aus der Gruppe bestehend aus F, Cl, CF₃, CH₃, Methoxy, Hydroxy, Amino, Methylamino, Dimethylamino oder Benzyl;
W Sauerstoff, S oder NR(36)-;
R(36) H oder (C₁-C₄)-Alkyl;
oder
R(3), R(4), R(5), R(6) und R(7)
unabhängig voneinander R(46)X(1)-;
X(1) Sauerstoff, S, NR(47), (D=O)A- oder NR(48)C=MN⁽*⁾R(49)-;
M Sauerstoff oder Schwefel;
A Sauerstoff oder NR(50);
D C oder SO;
R(46) (C₁-C₈)-Alkyl, (C₃-C₈)-Alkenyl, (CH₂)_{b}C_{d}F_{2d+1} oder -CₓH₂ₓ-R(51);
b Null oder 1;
d 1, 2, 3, 4, 5, 6 oder 7;
x Null, 1, 2, 3 oder 4;
R(51) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(52)R(53); R(52) und R(53)
Wasserstoff, (C₁-C₄)-Alkyl oder (C₁-C₄)-Perfluoralkyl;
R(47), R(48) und R(50) unabhängig
Wasserstoff, (C₁-C₄)-Alkyl oder (C₁-C₄)-Perfluoralkyl;
R(49) definiert wie R(46);
oder
R(46) und R(47) beziehungsweise R(46) und R(48)
gemeinsam 4 oder 5 Methylengruppen, von denen eine CH₂-Gruppe durch Sauerstoff, Schwefel, NH, N-CH₃ oder N-Benzyl ersetzt sein kann;
wobei A und N⁽*⁾ an den Phenylkern des Heteroaroylguanidin-Grundkörpers gebunden sind;
oder
R(3), R(4), R(5), R(6) und R(7)
unabhängig voneinander -SR(64), -OR(65), -NHR(66), -NR(67)R(68), -CHR(69)R(70) oder -CR(54)R(55)OH, -C≡CR(56), -CR(58)=CR(57) oder -[CR(59)R(60)]ᵤ-CO-[CR(61)R(62)]ᵥ-R(63); R(64), R(65), R(66), R(67) und R(69) gleich oder verschieden
-(CH2)_{y}-(CHOH)_{z}-(CH₂)ₐₐ-(CHOH)ₜ-R(71) oder
-(CH₂)_{ab}-O-(CH₂-CH₂O)_{ac}-R(72);
R(71), und R(72)
unabhängig voneinander Wasserstoff oder Methyl;
u 1, 2, 3 oder 4;
v Null, 1, 2, 3 oder 4;
y, z, aa gleich oder verschieden
Null, 1, 2, 3 oder 4;
t 1, 2, 3 oder 4;
R(68), R(70), R(54) und R(55)
gleich oder verschieden Wasserstoff oder (C₁-C₆)-Alkyl;
oder
R(69) und R(70) beziehungsweise R(54) und R(55)
zusammen mit dem sie tragenden Kohlenstoff-Atom ein (C₃-C₈)-Cycloalkyl; R(63)
Wasserstoff, (C₁-C₆)-Alkyl, (C₃-C₈)-Cycloalkyl oder -CₑH₂ₑ-R(73);
e Null, 1, 2, 3 oder 4;
R(56), R(57) und R(73) unabhängig
Phenyl, das unsubstituiert oder substituiert ist mit 1 - 3 Substituenten ausgewählt aus der Gruppe bestehend aus F, Cl, CF₃, Methyl, Methoxy und NR(74)R(75);
R(74) und R(75)
Wasserstoff oder (C₁-C₄)-Alkyl;
oder
R(56), R(57) und R(73) unabhängig
(C₁-C₉)-Heteroaryl, das unsubstituiert oder wie Phenyl substituiert ist;
R(58), R(59), R(60), R(61) und R(62)
Wasserstoff oder Methyl;
oder
R(3), R(4), R(5), R(6) und R(7)
unabhängig voneinander R(76)-NH-SO₂-;
R(76) R(77)R(78)N-(C=Y')-;
Y' Sauerstoff, S oder N-R(79);
R(77) und R(78) gleich oder verschieden Wasserstoff, (C₁-C₈)-Alkyl, (C₃-C₆)-Alkenyl oder -C_{f}H_{2f}-R(80);
f Null, 1, 2, 3 oder 4;
R(80) (C₅-C₇)-Cycloalkyl oder Phenyl, welches unsubstituiert mit 1 - 3 Substituenten ausgewählt aus der Gruppe bestehend aus F, Cl, CF₃, Methoxy und (C₁-C₄)-Alkyl;
oder
R(77) und R(78)
gemeinsam 4 oder 5 Methylengruppen, von denen eine CH₂-Gruppe durch Sauerstoff, Schwefel, NH, N-CH₃ oder N-Benzyl ersetzt sein kann;
R(79) wie R(77) definiert oder gleich Amidin;
oder
R(3), R(4), R(5), R(6) und R(7)
unabhängig voneinander NR(84a)R(85), OR(84b), SR(84c) oder -CₙH₂ₙ-R(84d);
n Null, 1, 2, 3 oder 4;
R(84d) (C₃-C₇)-Cycloalkyl oder Phenyl, welches nicht substituiert ist oder substituiert mit 1 - 3 Substituenten aus der Gruppe bestehend aus F, Cl, CF₃, Methyl, Methoxy und NR(16)R(17);
R(16) und R(17)
Wasserstoff, oder C₁-C₄-Alkyl;
R(84a), R(84b), R(84c) und R(85)
unabhängig voneinander Wasserstoff, (C₁-C₈)-Alkyl, (C₁-C₈)-Perfluoralkyl oder (CH₂)ₐₓ-R(84g);
ax Null, 1, 2, 3 oder 4;
R(84g) (C₃-C₇)-Cycloalkyl oder Phenyl, welches nicht substituiert ist oder substituiert mit 1 - 3 Substituenten ausgewählt aus der Gruppe bestehend aus F, Cl, CF₃, Methyl, Methoxy und NR(84u)R(84v);
R(84u) und R(84v)
Wasserstoff oder C₁-C₄-Alkyl;
oder
R(84a) und R(85)
gemeinsam 4 oder 5 Methylengruppen, von denen eine CH₂-Gruppe durch Sauerstoff, Schwefel, NH, N-CH₃ oder N-Benzyl ersetzt sein kann,
sowie deren pharmazeutisch verträgliche Salze;
z) Benzoylguanidine der Formel I 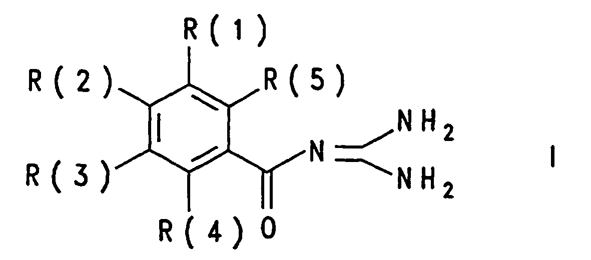 worin bedeuten:
R(1) R(6)-SOₘ;
m Null, 1 oder 2;
R(6) Perfluoralkyl mit 1, 2, 3, 4, 5 oder 6 C-Atomen, das geradkettig oder verzweigt ist;
R(2) und R(3)
unabhängig voneinander Wasserstoff, F, Cl, Br, I, Alkyl mit 1, 2, 3 oder 4 C-Atomen, Alkoxy mit 1, 2, 3 oder 4 C-Atomen oder Phenoxy,
das unsubstituiert ist oder substituiert ist mit 1 - 3 Substituenten
ausgewählt aus der Gruppe bestehend aus F, Cl, Methyl und Methoxy; oder
R(2) und R(3)
unabhängig voneinander Pyrrol-1-yl, Pyrrol-2-yl- oder Pyrrol-3-yl, welches nicht substituiert ist oder substituiert mit 1 bis 4 Substituenten, ausgewählt aus der Gruppe bestehend aus F, Cl, Br, I, CN, Alkanoyl mit 2, 3, 4, 5, 6, 7 oder 8 C-Atomen, Alkoxycarbonyl mit 2, 3, 4, 5, 6, 7 oder 8 C-Atomen, Formyl, Carboxy, CF₃,Methyl und Methoxy;
R(4) und R(5)
unabhängig voneinander Wasserstoff, Alkyl mit 1, 2 oder 3-C-Atomen, F, Cl, Br, I, CN, OR(7), NR(8)R(9) oder -(CH₂)ₙ-(CF₂)ₒ-CF₃; R(7), R(8) und R(9)
unabhängig voneinander Wasserstoff oder Alkyl mit 1, 2, 3 oder 4 C-Atomen;
n Null oder 1;
o Null, 1 oder 2;
sowie deren pharmakologisch akzeptable Salze;
ab) Perfluoralkylgruppen tragende phenylsubstituierte Alkenylcarbonsäure-guanidide der Formel I 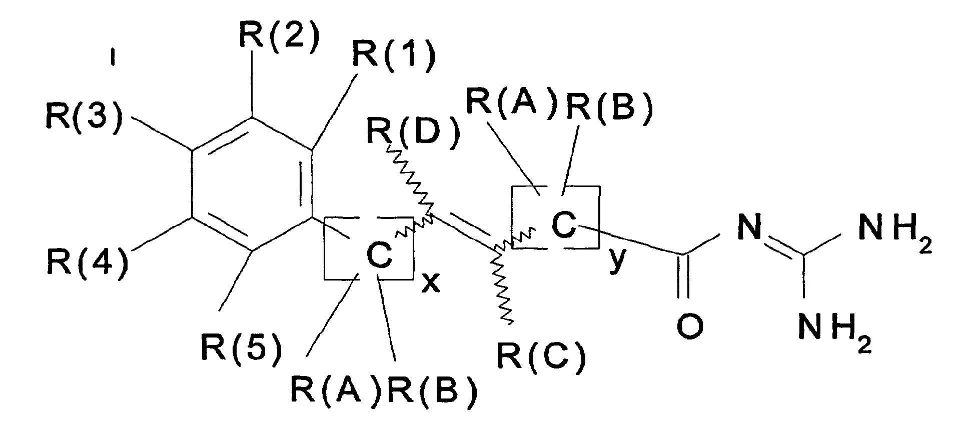 worin bedeuten:
R(A) Wasserstoff, F, Cl, Br, I, CN, OH, OR(6), (C₁-C₈)-Alkyl, Oᵣ(CH₂)ₐC_{b}F_{2b+1}, (C₃-C₈)-Cycloalkyl oder NR(7)R(8);
r Null oder 1;
a Null, 1, 2, 3 oder 4;
b 1, 2, 3, 4, 5, 6, 7 oder 8;
R(6) (C₁-C₈)-Alkyl, (C₁-C₄)-Perfluoralkyl, (C₃-C₈)-Alkenyl, (C₃-C₈)-Cycloalkyl,
Phenyl oder Benzyl;
wobei die Aromaten nicht substituiert oder substituiert sind mit 1 - 3 Substituenten aus der Gruppe bestehend aus F, Cl, CF₃, Methyl, Methoxy und NR(9)R(10);
R(9) und R(10)
H, (C₁-C₄)-Alkyl oder (C₁-C₄)-Perfluoralkyl;
R(7) und R(8)
unabhängig voneinander wie R(6) definiert;
oder
R(7) und R(8) gemeinsam 4 oder 5 Methylengruppen, von denen eine CH₂-Gruppe durch Sauerstoff, Schwefel, NH, N-CH₃ oder N-Benzyl ersetzt sein kann;
R(B) unabhängig wie R(A) definiert;
X Null, 1 oder 2;
Y Null, 1 oder 2;
R(C) Wasserstoff, F, Cl, Br, I, CN, OR(12), (C₁-C₈)-Alkyl, Oₚ(CH₂)_{f}C_{g}F_{2g+1} oder (C₃-C₈)-Cycloalkyl;
p Null oder 1;
f Null, 1, 2, 3 oder 4;
g 1, 2, 3, 4, 5, 6, 7 oder 8;
R(12)
(C₁-C₈)-Alkyl, (C₁-C₄)-Perfluoralkyl, (C₃-C₈)-Alkenyl, (C₃-C₈)-Cycloalkyl, Phenyl oder Benzyl;
wobei die Aromaten Phenyl oder Benzyl nicht substituiert oder substituiert sind mit 1 - 3 Substituenten aus der Gruppe bestehend aus F, Cl, CF₃, Methyl, Methoxy und NR(13)R(14); R(13) und R(14)
unabhängig voneinander H, (C₁-C₄)-Alkyl oder (C₁-C₄)-Perfluoralkyl;
R(D) unabhängig wie R(C) definiert,
R(1) Wasserstoff, (C₁-C₈)-Alkyl, -Oₜ(CH₂)_{d}CₑF₂ₑ₊₁, (C₃-C₈)-Cycloalkyl, F, Cl, Br, I oder CN;
t Null oder 1;
d Null, 1, 2, 3 oder 4;
e 1, 2, 3, 4, 5, 6, 7 oder 8;
R(2), R(3), R(4) und R(5)
unabhängig voneinander wie R(1) definiert;
jedoch unter der Bedingung, daß mindestens einer der Substituenten R(A), R(B), R(C), R(D), R(1), R(2), R(4) oder R(5) eine Oᵣ(CH₂)ₐC_{b}F_{2b+1}-, Oₚ(CH₂)_{f}C₉F_{2g+1}- oder Oₜ(CH₂)_{d}CₑF₂ₑ₊₁-gruppe und R(3) keine Oₜ(CH₂)_{d}CₑF₂ₑ₊₁-gruppe ist; sowie deren pharmazeutisch verträgliche Salze;
ac) Ortho-amino-substituierte Benzoylguanidine der Formel I 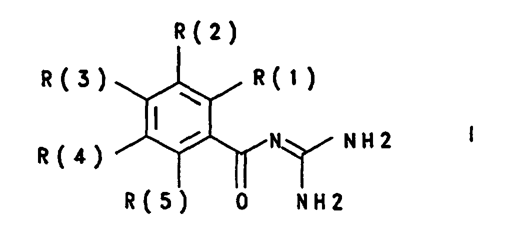 worin bedeuten:
R(1) NR(50)R(6),
R(50) und R(6)
unabhängig voneinander Wasserstoff, (C₁-C₈)-Alkyl oder (C₁-C₈)-Perfluoralkyl;
R(2), R(3), R(4) und R(5)
unabhängig voneinander R(10)-SOₐ-, R(11)R(12)N-CO-, R(13)-CO- oder R(14)R(15)N-SO₂-;
a Null, 1 oder 2,
R(10), R(11), R(12), R(13), R(14) und R(15)
unabhängig voneinander (C₁-C₈)-Alkyl, (C₁-C₈)-Perfluoralkyl, (C₃-C₆)-Alkenyl oder -C_{ab}H_{2ab}-R(16);
ab Null, 1, 2, 3 oder 4;
R(16) (C₃-C₇)-Cycloalkyl, Phenyl,
welches nicht substituiert ist oder substituiert mit 1 - 3 Substituenten ausgewählt aus der Gruppe bestehend aus F, Cl, CF₃, Methyl, Methoxy oder NR(17)R(18);
R(17) und R(18)
unabhängig voneinander H, CF₃ oder (C₁-C₄)-Alkyl;
oder
R(11), R(12), sowie R(14) und R(15)
gemeinsam 4 oder 5 Methylengruppen, von denen eine CH₂-Gruppe durch Sauerstoff, S, NH, N-CH₃ oder N-Benzyl ersetzt sein kann;
oder
R(11), R(12), R(14) und R(15)
unabhängig voneinander Wasserstoff;
oder
R(2), R(3), R(4) und R(5)
unabhängig voneinander SR(21), -OR(22), -NR(23)R(24) oder -CR(25)R(26)R(27);
R(21), R(22), R(23) und R(25) unabhängig voneinander -C_{b}H_{2b}-(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
b Null, 1 oder 2;
R(24), R(26) und R(27)
unabhängig voneinander Wasserstoff, (C₁-C₄)-Alkyl oder (C₁-C₄)-Perfluoralkyl;
oder
R(2), R(3), R(4) und R(5)
unabhängig voneinander Wasserstoff, F, Cl, Br, I, CN, -(Xa)_{dg}-C_{da}H_{2da+1}, -
(Xb)_{dh}-(CH₂)_{db}-C_{de}F_{2de+1}, (C₃-C₈)-Alkenyl oder -C_{df}H_{2df}R(30);
(Xa) O, S oder NR(33);
R(33)
H, (C₁-C₄)-Alkyl oder (C₁-C₄)-Perfluoralkyl;
dg Null oder 1;
(Xb) O, S oder NR(34); R(34)
H, (C₁-C₄)-Alkyl oder (C₁-C₄)-Perfluoralkyl;
dh Null oder 1;
da Null, 1, 2, 3, 4, 5, 6, 7, 8;
db Null, 1, 2, 3, 4;
de Null, 1, 2, 3, 4, 5, 6, 7;
df Null, 1, 2, 3, 4;
R(30) (C₃-C₈)-Cycloalkyl, Phenyl, Biphenylyl oder Naphthyl, wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(31)R(32); R(31) und R(32)
H, (C₁-C₄)-Alkyl oder (C₁-C₄)-Perfluoralkyl;
oder
R(2), R(3), R(4) und R(5)
unabhängig voneinander NR(40)R(41) oder -(Xe)-(CH₂)_{eb}R(45); R(40) und R(41)
unabhängig voneinander Wasserstoff, (C₁-C₈)-Alkyl, (C₁-C₈)-Perfluoralkyl oder (CH₂)ₑ-R(42);
e Null, 1, 2, 3 oder 4;
R(42) (C₃-C₇)-Cycloalkyl, Phenyl, welches nicht substituiert ist oder substituiert mit 1 - 3 Substituenten ausgewählt aus der Gruppe bestehend aus F, Cl, CF₃, Methyl, Methoxy und NR(43)R(44); R(43) und R(44)
unabhängig voneinander H, CF₃ oder (C₁-C₄)-Alkyl;
oder
R(40) und R(41) gemeinsam 4 oder 5 Methylengruppen, von denen eine CH₂-Gruppe durch Sauerstoff, Schwefel, NH, N-CH₃ oder N-Benzyl ersetzt sein kann;
(Xe) O, S oder NR(47);
R(47)
H, (C₁-C₄)-Alkyl oder (C₁-C₄)-Perfluoralkyl;
eb Null, 1, 2, 3 oder 4;
R(45) (C₃-C₇)-Cycloalkyl, Phenyl, welches nicht substituiert ist oder substituiert mit 1 - 3 Substituenten ausgewählt aus der Gruppe bestehend aus F, Cl, CF₃, Methyl, Methoxy, NR(50)R(51) und -(Xfa)-(CH₂)_{ed}-(Xfb)R(46);
Xfa CH₂, O, S oder NR(48);
Xfb O, S oder NR(49);
ed 1, 2, 3 oder 4;
R(46)
H, (C₁-C₄)-Alkyl oder (C₁-C₄)-Perfluoralkyl;
R(48), R(49), R(50) und R(51)
unabhängig voneinander H oder (C₁-C₄)-Alkyl oder (C₁-C₄)-Perfluoralkyl;
wobei R(3) und R(4) jedoch nicht Wasserstoff sein können,
sowie deren pharmazeutisch verträgliche Salze;
ad) Benzoylguanidine der Formel I 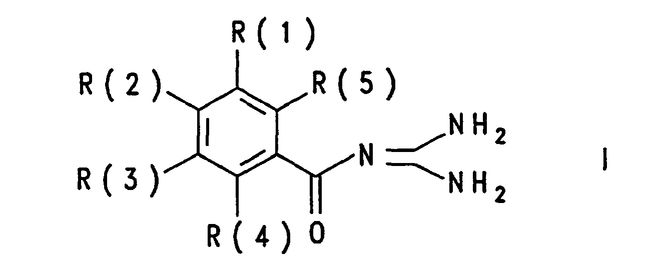 worin bedeuten:
einer der drei Substituenten R(1), R(2) und R(3)
(C₁-C₉)-Heteroaryl-N-Oxid,
das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
einer der drei Substituenten R(1), R(2) und R(3)
-SR(10), -OR(10), -NR(10)R(11) oder -CR(10)R(11)R(12);
R(10)
-CₐH₂ₐ-(C₁-C₉)-Heteroaryl-N-Oxid,
das unsubstituiert oder substituiert ist mit 1 - 3 Substituenten ausgewählt aus der Gruppe bestehend F, Cl, CF₃, CH₃, Methoxy, Hydroxy, Amino, Methylamino und Dimethylamino;
a Null, 1 oder 2;
R(11) und R(12)
unabhängig voneinander wie R(10) definiert, Wasserstoff oder (C₁-C₄)-Alkyl;
und die jeweils anderen Substituenten R(1), R(2) und R(3)
unabhängig voneinander (C₁-C₈)-Alkyl, (C₂-C₈)-Alkenyl oder -CₘH₂ₘR(14);
m Null, 1 oder 2;
R(14) (C₃-C₈)-Cycloalkyl oder Phenyl,
welches nicht substituiert oder substituiert ist mit 1 - 3 Substituenten ausgewählt aus der Gruppe bestehend aus F, Cl, CF₃, Methyl, Methoxy und NR(15)R(16), R(15) und R(16)
Wasserstoff oder CH₃;
oder
die jeweils anderen Substituenten R(1), R(2) und R(3)
unabhängig voneinander Wasserstoff, F, Cl, Br, I, -C=N, X-(CH₂)ₚ-(C_{q}F_{2q+1}), R(22)-SOᵤ, R(23)R(24)N-CO, R(25)-CO- oder R(26)R(27)N-SO₂-, wobei die Perfluoralkylgruppe geradkettig oder verzweigt ist;
X eine Bindung, Sauerstoff, S oder NR(28);
u Null, 1 oder 2;
p Null, 1 oder 2;
q Null, 1, 2, 3, 4, 5 oder 6;
R(22), R(23), R(25) und R(26)
unabhängig (C₁-C₈)-Alkyl, (C₂-C₆)-Alkenyl, -CₙH₂ₙ-R(29) oder CF₃;
n Null, 1, 2, 3 oder 4;
R(28) Wasserstoff oder (C₁-C₃)-Alkyl;
R(29) (C₃-C₇)-Cycloalkyl oder Phenyl;
welches nicht substituiert ist oder substituiert mit 1 - 3 Substituenten ausgewählt aus der Gruppe bestehend aus F, Cl, CF₃, Methyl, Methoxy und NR(30)R(31);
R(30) und R(31)
Wasserstoff oder C₁-C₄-Alkyl,
oder
R(23), R(25) und R(26)
auch Wasserstoff;
R(24) und R(27)
unabhängig voneinander Wasserstoff oder (C₁-C₄)-Alkyl;
oder
R(23) und R(24) sowie R(26) und R(27) gemeinsam 4 oder 5 Methylengruppen, von denen eine CH₂-Gruppe durch Sauerstoff, S, NH, N-CH₃ oder N-Benzyl ersetzt sein kann;
oder
die jeweils anderen Substituenten R(1), R(2) und R(3) unabhängig voneinander OR(35) oder NR(35)R(36);
R(35) und R(36)
unabhängig voneinander Wasserstoff oder (C₁-C₆)-Alkyl;
oder
R(35) und R(36) gemeinsam 4 - 7 Methylengruppen, von denen eine CH₂-Gruppe durch Sauerstoff, S, NH, N-CH₃ oder N-Benzyl ersetzt sein kann,
R(4) und R(5)
unabhängig voneinander Wasserstoff, (C₁-C₄)-Alkyl, F, Cl, -OR(32), -
NR(33)R(34) oder CᵣF₂ᵣ₊₁;
R(32), R(33) und R(34)
unabhängig voneinander Wasserstoff oder (C₁-C₃)-Alkyl;
r 1, 2, 3 oder 4;
sowie deren pharmazeutisch verträgliche Salze;
ad) Benzoylguanidine der Formel I 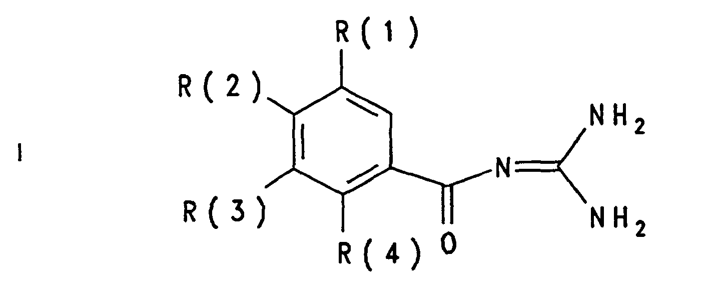 worin bedeuten:
R(1) Wasserstoff, F, Cl, Br, I, CN, NO₂, OH, (C₁-C₈)-Alkyl, (C₃-C₈)-Cycloalkyl,
Oₐ-(CH₂)_{b}-(CF₂)_{c}-CF₃;
a Null oder 1;
b Null, 1 oder 2;
c Null, 1, 2 oder 3;
oder
R(1) R(5)-SOₘ oder R(6)R(7)N-SO₂-;
m Null, 1 oder 2;
R(5) und R(6) unabhängig voneinander
(C₁-C₈)-Alkyl, (C₃-C₆)-Alkenyl, CF₃ oder -CₙH₂ₙ-R(8);
n Null, 1, 2, 3 oder 4;
R(7) Wasserstoff oder (C₁-C₄)-Alkyl;
R(8) (C₃-C₇)-Cycloalkyl oder Phenyl,
welches nicht substituiert ist oder substituiert mit 1 - 3 Substituenten ausgewählt aus der Gruppe bestehend aus F, Cl, CF₃, Methyl, Methoxy und NR(9)R(10); R(9) und R(10) unabhängig voneinander
Wasserstoff oder (C₁-C₄)-Alkyl;
oder
R(6) H;
oder R(6) und R(7)
gemeinsam 4 oder 5 Methylengruppen, von denen eine CH₂-Gruppe durch Sauerstoff, S, NH, N-CH₃ oder N-Benzyl ersetzt sein kann,
oder
R(1) -SR(11), -OR(11) oder -CR(11)R(12)R(13);
R(11) -CₚH₂ₚ-(C₃-C₈)-Cycloalkyl, -(C₁-C₉)-Heteroaryl oder Phenyl,
wobei die aromatischen Systeme unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino; R(12), R(13) unabhängig voneinander
wie R(11) definiert oder Wasserstoff oder (C₁-C₄)-Alkyl;
p Null, 1 oder 2;
oder
R(1) Phenyl, Naphthyl, Biphenylyl oder (C₁-C₉)-Heteroaryl, letzteres über C oder N verknüpft,
die unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
R(2) -CF₂R(14), -CF[R(15)][R(16)], -CF[(CF2)_{q}-CF₃)][R(15)],
-C[(CF₂)ᵣ-CF₃]=CR(15)R(16);
R(14) (C₁-C₄)-Alkyl oder (C₃-C₆)-Cycloalkyl;
R(15) und R(16) unabhängig voneinander
Wasserstoff oder (C₁-C₄)-Alkyl;
q Null, 1 oder 2;
r Null, 1 oder 2;
R(3) wie R(1) definiert;
R(4) Wasserstoff, (C₁-C₃)-Alkyl, F, Cl, Br, I, CN, -(CH₂)ₛ-(CF₂)ₜ-CF₃;
s Null oder 1;
t Null, 1 oder 2;
sowie deren pharmazeutisch verträgliche Salze;
ae) Benzoylguanidine der Formel I 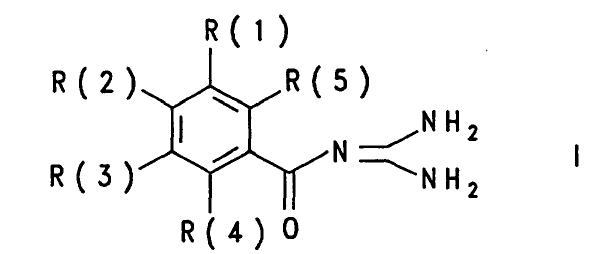 worin bedeuten:
einer der drei Substituenten R(1), R(2) und R(3)
-Y-4-[(CH₂)ₖ-CHR(7)-(C=O)R(8)]-Phenyl,
-Y-3-(CH₂)ₖ-CHR(7)-(C=O)R(8)]-Phenyl oder -Y-2-[(CH₂)ₖ-CHR(7)-(C=O)R(8)]-Phenyl,
wobei das Phenyl jeweils unsubstituiert oder substituiert ist mit 1 - 2 Substituenten aus der Gruppe F, Cl, -CF₃, Methyl, Hydroxy, Methoxy, oder -NR(37)R(38);
R(37) und R(38)
unabhängig voneinander Wasserstoff oder -CH₃;
Y eine Bindung, Sauerstoff, -S- oder -NR(9);
R(9) Wasserstoff oder -(C₁-C₄)-Alkyl;
R(7) -OR(10) oder -NR(10)R(11);
R(10) und R(11)
unabhängig voneinander Wasserstoff, -(C₁-C₈)-Alkyl, -(C₁-C₈)-Alkanoyl, -(C₁-C₈)-Alkoxycarbonyl, Benzyl, Benzyloxycarbonyl;
oder
R(10) Trityl;
R(8) -OR(12) oder -NR(12)R(13);
R(12) und R(13)
unabhängig voneinander Wasserstoff, -(C₁-C₈)-Alkyl oder Benzyl;
k Null, 1, 2, 3 oder 4;
und die jeweils anderen Reste R(1), R(2) und R(3)
unabhängig voneinander -(C₁-C₈)-Alkyl, -(C₂-C₈)-Alkenyl oder -(CH₂)ₘR(14);
m Null, 1 oder 2;
R(14) -(C₃-C₈)-Cycloalkyl oder Phenyl,
welches nicht substituiert oder substituiert ist mit 1 - 3 Substituenten ausgewählt aus der Gruppe bestehend aus F, Cl, -CF₃, Methyl, Methoxy und -NR(15)R(16); R(15) und R(16)
Wasserstoff oder -CH₃;
oder
die jeweils anderen Reste R(1), R(2) und R(3)
unabhängig voneinander R(18)R(19)N-(C=Y')-NH-SO₂-;
Y' Sauerstoff, -S- oder -N-R(20);
R(18) und R(19)
unabhängig voneinander Wasserstoff, -(C₁-C₈)-Alkyl, -(C₃-C₆)-Alkenyl oder -(CH₂)ₜ-R(21);
t Null, 1, 2, 3 oder 4;
R(21) -(C₅-C₇)-Cycloalkyl oder Phenyl, welches unsubstituiert ist oder substituiert ist mit 1 - 3 Substituenten ausgewählt aus der Gruppe bestehend aus F, Cl, -CF₃, Methoxy und -(C₁-C₄)-Alkyl;
oder
R(18) und R(19)
gemeinsam 4 oder 5 Methylengruppen, von denen eine CH₂-Gruppe durch Sauerstoff, -S-, -NH-, -N-CH₃ oder -N-Benzyl ersetzt sein kann; R(20)
wie R(18) definiert oder Amidin;
oder
die jeweils anderen Reste R(1), R(2) und R(3)
unabhängig voneinander Wasserstoff, F, Cl, Br, I, -C≡N, X-(CH₂)ₚ-(C_{q}F_{2q+1}), R(22)-SOᵤ-, R(23)R(24)N-CO-, R(25)-CO- oder R(26)R(27)N-SO₂-, wobei die Perfluoralkylgruppe geradkettig oder verzweigt ist;
X eine Bindung, Sauerstoff, -S- oder -NR(28);
u Null, 1 oder 2;
p Null, 1 oder 2;
q 1, 2, 3, 4, 5 oder 6;
R(22), R(23), R(25) und R(26)
unabhängig voneinander -(C₁-C₈)-Alkyl, -(C₃-C₆)-Alkenyl, -(CH₂)ₙ-R(29) oder -CF₃;
n Null, 1, 2, 3 oder 4;
R(28) Wasserstoff oder -(C₁-C₃)-Alkyl;
R(29) -(C₃-C₇)-Cycloalkyl oder Phenyl,
welches nicht substituiert ist oder substituiert mit 1 - 3 Substituenten ausgewählt aus der Gruppe bestehend aus F, Cl, -CF₃, Methyl, Methoxy und -NR(30)R(31); R(30) und R(31)
Wasserstoff oder -(C₁-C₄)-Alkyl;
oder
R(23), R(25) und R(26)
Wasserstoff;
R(24) und R(27)
unabhängig voneinander Wasserstoff oder -(C₁-C₄)-Alkyl;
oder
R(23) und R(24) sowie R(26) und R(27)
gemeinsam 4 oder 5 Methylengruppen, von denen eine CH₂-Gruppe durch Sauerstoff, -S-, -NH-, - N-CH₃ oder -N-Benzyl ersetzt sein kann;
oder
die jeweils anderen Reste R(1), R(2) und R(3)
unabhängig voneinander -OR(35) oder -NR(35)R(36);
R(35) und R(36)
unabhängig voneinander Wasserstoff oder -(C₁-C₆)-Alkyl;
oder
R(35) und R(36)
gemeinsam 4 - 7 Methylengruppen, von denen eine CH₂-Gruppe durch Sauerstoff, -S-, -NH-, -N-CH₃ oder -N-Benzyl ersetzt sein kann;
R(4) und R(5)
unabhängig voneinander Wasserstoff, -(C₁-C₄)-Alkyl, F, Cl, -OR(32), - NR(33)R(34) oder -CᵣF₂ᵣ₊₁;
R(32), R(33) und R(34)
unabhängig voneinander Wasserstoff oder -(C₁-C₃)-Alkyl;
r 1, 2, 3 oder 4;
sowie deren pharmazeutisch verträgliche Salze;
af) Benzoylguanidine der Formel I 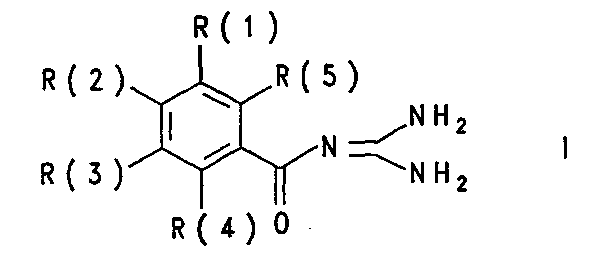 worin bedeuten:
R(1) R(6)-CO oder R(7)R(8)N-CO;
R(6) (C₁-C₈)-Alkyl, (C₁-C₈)-Perfluoralkyl, (C₃-C₈)-Alkenyl oder -CₙH₂ₙ-R(9),
n Null, 1, 2, 3 oder 4;
R(9) (C₃-C₈)-Cycloalkyl, Phenyl, Biphenylyl oder Naphthyl,
wobei die Aromaten nicht substituiert oder substituiert sind mit 1 - 3 Substituenten ausgewählt aus der Gruppe bestehend aus F, Cl, CF₃, Methyl, Methoxy und NR(10)R(11), R(10) und R(11)
H, (C₁-C₄)-Alkyl oder (C₁-C₄)-Perfluoralkyl;
R(7) H, (C₁-C₈)-Alkyl, (C₁-C₈)-Perfluoralkyl, (C₃-C₈)-Alkenyl oder -CₙH₂ₙ-R(12);
n Null, 1, 2, 3 oder 4;
R(12) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(13)R(14); R(13) und R(14)
H, (C₁-C₄)-Alkyl oder (C₁-C₄)-Perfluoralkyl;
R(8) H, (C₁-C₄)-Alkyl oder (C₁-C₄)-Perfluoralkyl;
oder
R(7) und R(8) gemeinsam
4 oder 5 Methylengruppen, von denen eine CH₂-Gruppe durch
Sauerstoff, S, NH, N-CH₃ oder N-Benzyl ersetzt sein kann;
R(2) wie R(1) definiert, oder H, OH, F, Cl, Br, I, CN, NO₂, (C₁-C₈)-Alkyl, (C₁-C₈)-Perfluoralkyl, (C₃-C₈)-Alkenyl oder -CₙH₂ₙR(15);
n Null, 1, 2, 3 oder 4;
R(15) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(16)R(17);
R(16) und R(17)
H, (C₁-C₄)-Alkyl oder (C₁-C₄)-Perfluoralkyl;
oder
R(2) (C₁-C₉)-Heteroaryl,
das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(2) SR(18), -OR(18), -NR(18)R(19) oder -CR(18)R(19)R(20); R(18) -CₐH₂ₐ-(C₁-C₉)-Heteroaryl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino, Dimethylamino;
a Null, 1 oder 2;
R(19) und R(20)
unabhängig voneinander wie R(18) definiert oder Wasserstoff,
(C₁-C₄)-Alkyl oder (C₁-C₄)-Perfluoralkyl;
oder
R(2) R(21)-SOₘ oder R(22)R(23)N-SO₂-;
m 1 oder 2;
R(21) (C₁-C₈)-Alkyl, (C₁-C₈)-Perfluoralkyl, (C₃-C₈)-Alkenyl oder -CₙH₂ₙ-R(24);
n Null, 1, 2, 3 oder 4;
R(24) (C₃-C₈)-Cycloalkyl, Phenyl, Biphenylyl oder Naphthyl,
wobei die Aromaten nicht substituiert oder substituiert sind mit 1 - 3 Substituenten ausgewählt aus der Gruppe bestehend aus F, Cl, CF₃, Methyl, Methoxy und NR(27)R(28);
R(27) und R(28)
H, (C₁-C₄)-Alkyl oder (C₁-C₄)-Perfluoralkyl;
R(22) H, (C₁-C₈)-Alkyl, (C₁-C₈)-Perfluoralkyl, (C₃-C₈)-Alkenyl oder -CₙH₂ₙ-R(29);
n Null, 1, 2, 3 oder 4;
R(29) (C₃-C₈)-Cycloalkyl. Phenyl, Biphenylyl oder Naphthyl,
wobei die Aromaten nicht substituiert oder substituiert sind mit 1 - 3 Substituenten ausgewählt aus der Gruppe bestehend aus F, Cl, CF₃, Methyl, Methoxy und NR(30)R(31); R(30) und R(31)
H, (C₁-C₄)-Alkyl oder (C₁-C₄)-Perfluoralkyl;
R(23) Wasserstoff, (C₁-C₄)-Alkyl oder (C₁-C₄)-Perfluoralkyl;
oder
R(22) und R(23) gemeinsam 4 oder 5 Methylengruppen, von denen eine CH₂-Gruppe durch Sauerstoff, S, NH, N-CH₃ oder N-Benzyl ersetzt sein kann;
oder
R(2) R(33)X-;
X Sauerstoff, S, NR(34), (D=O)A- oder NR(34)C=MN⁽*⁾R(35)-;
M Sauerstoff oder S;
A Sauerstoff oder NR(34);
D C oder SO;
R(33) (C₁-C₈)-Alkyl, (C₃-C₈)-Alkenyl, (CH₂)_{b}C_{d}F_{2d+1} oder -CₙH₂ₙ-R(36);
b Null oder 1;
d 1, 2, 3, 4, 5, 6 oder 7;
n Null, 1, 2, 3, oder 4;
R(36) (C₃-C₈)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, CF₃, Methyl, Methoxy und NR(37)R(38);
R(37) und R(38)
H, (C₁-C₄)-Alkyl oder (C₁-C₄)-Perfluoralkyl;
R(34) Wasserstoff, (C₁-C₄)-Alkyl oder (C₁-C₄)-Perfluoralkyl;
R(35) definiert wie R(33);
oder
R(33) und R(34)
gemeinsam 4 oder 5 Methylengruppen, von denen eine CH₂-Gruppe durch Sauerstoff, S, NH, N-CH₃ oder N-Benzyl ersetzt sein kann;
wobei A und N⁽*⁾ an den Phenylkern des Benzoylguanidin-Grundkörpers gebunden sind;
oder
R(2) -SR(40), -OR(40), -NHR(40), -NR(40)R(41), -CHR(40)R(42), -CR(42)R(43)OH, -C≡CR(45), -CR(46)=CR(45) oder -[CR(47)R(48)]ᵤ-CO-[C(R49)R(50)]ᵥ-R(44); R(40) und R(41) unabhängig voneinander -(CH₂)ₚ-(CHOH)_{q}-(CH₂)ᵣ-(CHOH)ₜ-R(51) oder-(CH₂)ₚ-O-(CH₂-CH₂O)_{q}-R(51);
R(51) Wasserstoff oder Methyl;
u 1, 2, 3 oder 4;
v Null, 1, 2, 3 oder 4;
p, q und r
unabhängig voneinander Null, 1, 2, 3 oder 4;
t 1, 2, 3 oder 4;
R(42) und R(43)
unabhängig voneinander Wasserstoff oder (C₁-C₆)-Alkyl;
oder R(42) und R(43)
zusammen mit dem sie tragenden Kohlenstoff-Atom ein (C₃-C₈)-Cycloalkyl;
R(44) Wasserstoff, (C₁-C₆)-Alkyl, (C₃-C₈)-Cycloalkyl, -CₑH₂ₑ-R(45);
e Null, 1, 2, 3 oder 4;
R(45) Phenyl,
das unsubstituiert oder substituiert ist mit 1 - 3 Substituenten ausgewählt aus der Gruppe bestehend aus F, Cl, CF₃, Methyl, Methoxy und NR(52)R(53);
R(52) und R(53)
H oder (C₁-C₄)-Alkyl;
oder
R(45) (C₁-C₉)-Heteroaryl,
das unsubstituiert oder wie Phenyl substituiert ist;
oder
R(45) (C₁-C₆)-Alkyl,
das unsubstituiert oder mit 1 - 3 OH substituiert ist;
R(46), R(47), R(48), R(49) und R(50)
unabhängig voneinander Wasserstoff oder Methyl; oder
R(2) R(55)-NH-SO₂-;
R(55) R(56)R(57)N-(C=Y)-;
Y Sauerstoff, S oder N-R(58);
R(56) und R(57)
unabhängig voneinander Wasserstoff, (C₁-C₈)-Alkyl, (C₃-C₆)-Alkenyl oder -C_{f}H_{2f}-R(59);
f Null, 1, 2, 3 oder 4;
R(59) (C₅-C₇)-Cycloalkyl, Phenyl,
welches unsubstituiert ist oder substituiert mit 1 - 3 Substituenten ausgewählt aus der Gruppe bestehend aus F, Cl, CF₃, Methoxy und (C₁-C₄)-Alkyl;
oder
R(56) und R(57)
gemeinsam 4 oder 5 Methylengruppen, von denen eine CH₂-Gruppe durch Sauerstoff, S, NH, N-CH₃ oder N-Benzyl ersetzt sein kann;
R(58)
wie R(56) definiert oder gleich Amidin;
R(3), R(4) und R(5) sind unabhängig voneinander wie R(1) oder R(2) definiert, wobei jedoch mindestens einer der Substituenten R(2), R(3), R(4) und R(5) gleich OH sein muß;
sowie deren pharmazeutisch verträgliche Salze;
ag) Benzoylguanidine der Formel I 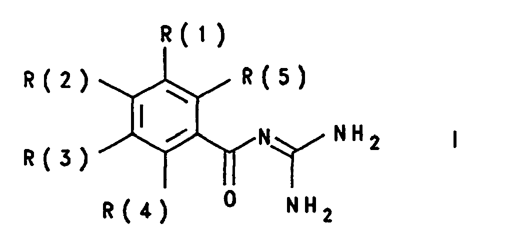 worin bedeuten:
einer der drei Substituenten R(1), R(2) und R(3)
R(6)-A-B-D-;
R(6) ein basischer protonierbarer Rest, d.h. eine Aminogruppe -NR(7)R(8), eine Amidinogruppe R(7)R(8)N-C[=N-R(9)]- oder eine Guanidinogruppe 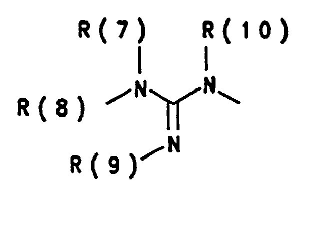 R(7), R(8), R(9) und R(10)
unabhängig voneinander Wasserstoff oder Alkyl mit 1, 2, 3 oder 4 C-Atomen;
oder
R(7) und R(8)
gemeinsam CₐH₂ₐ;
a 4, 5, 6 oder 7;
wobei im Falle von a = 5, 6 oder 7 eine Methylengruppe der Gruppe CₐH₂ₐ durch eine Heteroatomgruppe O, SOₘ oder NR(11) ersetzt sein kann,
oder
R(8) und R(9) oder R(9) und R(10) oder R(7) und R(10)
eine Gruppe CₐH₂ₐ;
a 2, 3, 4 oder 5;
wobei im Falle von a = 3, 4 oder 5 eine Methylengruppe der Gruppe CₐH₂ₐ durch eine Heteroatomgruppe O, SOₘ oder NR(11) ersetzt sein kann;
m Null, 1 oder 2;
R(11) Wasserstoff oder Methyl;
oder
R(6) ein basisches heteroaromatisches Ringsystem mit 1 - 9 C-Atomen;
A C_{b}H_{2b};
b 1, 2, 3, 4, 5, 6, 7, 8, 9 oder 10;
wobei in der Gruppe C_{b}H_{2b} ein oder zwei Methylengruppen durch eine der Gruppierungen ausgewählt aus der Gruppe bestehend aus -O-, -CO-, -CH[OR(20)]-, -SOₘ-, -NR(20)-, -NR(20)-CO-, -NR(20)-CO-NH-, -NR(20)-CO-NH-SO₂- 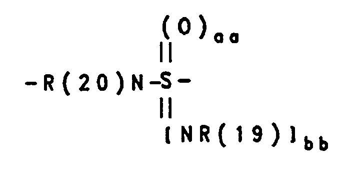 und -SOₐₐ[NR(19)]_{bb}- ersetzt sein können; und wobei in der Gruppe C_{b}H_{2b} eine Methylengruppe durch -CH-R(99) ersetzt sein kann, wobei R(99) gemeinsam mit R(7) einen Pyrrolidin- oder Piperidin-Ring bildet;
aa 1 oder 2;
bb 0 oder 1;
aa+bb=2;
R(19) Wasserstoff oder Alkyl mit 1, 2, 3 oder 4 C-Atomen
R(20) Wasserstoff oder Methyl;
B einen Phenylen- oder Naphthylenrest, 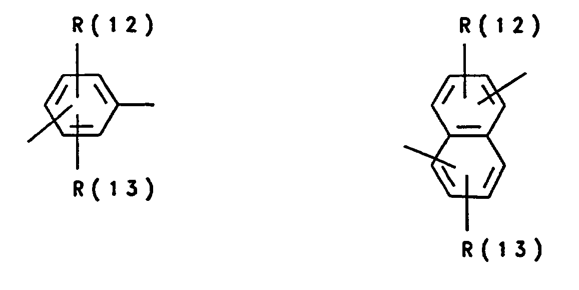 R(12) und R(13)
unabhängig voneinander Wasserstoff, Methyl, F, Cl, Br, J, CF₃ oder -SO_{w}-R(14);
R(14) Methyl oder NR(15)R(16);
R(15) und R(16)
unabhängig voneinander Wasserstoff oder Alkyl mit 1, 2, 3 oder 4 C-Atomen;
w Null, 1 oder 2;
D -CₐH_{2d}-X_{f}-;
d Null, 1, 2, 3 oder 4;
X -O-, -CO-, -CH[OR(21)]-, SOₘ oder -NR(21)-;
f Null oder 1;
R(21) Wasserstoff oder Methyl;
m Null, 1 oder 2;
und die jeweils anderen Substituenten R(1) und R(2) und R(3) unabhängig voneinander Wasserstoff, F, Cl, Br, J, -CN, -(C₁-C₈)-Alkyl, -(C₂-C₈)-Alkenyl, -NR(35)R(36) oder R(17)-C_{g}H_{2g}-Zₕ-;
g Null, 1, 2, 3 oder 4;
h Null oder 1;
R(35) und R(36)
unabhängig voneinander Wasserstoff oder Alkyl mit 1, 2, 3, 4, 5 oder 6 C-Atomen;
oder
R(35) und R(36)
gemeinsam 4 - 7 Methylengruppen, von denen eine CH₂-Gruppe durch Sauerstoff, -S-, -NH-, -NCH₃ oder -N-Benzyl ersetzt sein kann;
Z -O-, -CO-, -SOᵥ- ,-NR(18)-, -NR(18)-CO-, -NR(18)-CO-NH- oder
-NR(18)-SO₂-;
R(18) Wasserstoff oder Methyl;
v Null, 1 oder 2;
R(17) Wasserstoff, Cycloalkyl mit 3, 5 oder 6 C-Atomen oder CₖF₂ₖ₊₁-;
k 1, 2 oder 3,
oder
R(17) Pyrrol-1-yl, Pyrrol-2-yl oder Pyrrol-3-yl, welches nicht substituiert oder substituiert ist mit 1 - 4 Substituenten ausgewählt aus der Gruppe bestehend aus F, Cl, Br, I, -CN, (C₂-C₈)-Alkanoyl, (C₂-C₈)-Alkoxycarbonyl, Formyl, Carboxy, -CF₃, Methyl und Methoxy;
oder
R(17) -(C₃-C₈)-Cycloalkyl oder Phenyl, welches nicht substituiert oder substituiert ist mit 1 - 3 Substituenten ausgewählt aus der Gruppe bestehend aus F und Cl, -CF₃, Methyl, Hydroxy, Methoxy, -NR(37)R(38), CH₃SO₂- und H₂NO₂S-;
R(37) und R(38)
Wasserstoff oder -CH₃;
R(4) und R(5) unabhängig voneinander Wasserstoff, Alkyl mit 1, 2, 3 oder 4 C-Atomen, F, Cl, -OR(32), -NR(33)R(34) oder -CᵣF₂ᵣ₊₁;
R(32), R(33) und R(34)
unabhängig voneinander Wasserstoff oder Alkyl mit 1, 2 oder 3 C-Atomen;
r 1, 2, 3 oder 4;
sowie deren pharmakologisch verträgliche Salze;
ah) Indenoylguanidine der Formel I 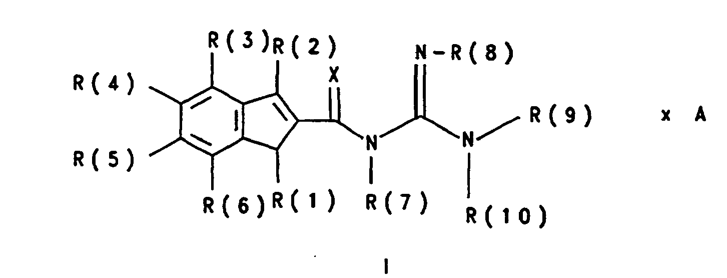 worin bedeuten:
R(1) und R(2)
unabhängig voneinander Wasserstoff, Alkyl mit 1, 2, 3, 4, 5, 6, 7, 8, 9 oder 10 C-Atomen, Cycloalkyl mit 3, 4, 5 oder 6 C-Atomen. O-Alkyl mit 1, 2, 3 or 4 C-Atomen, O-C(=O)-Alkyl mit 1, 2, 3 oder 4 C-Atomen oder CₘH₂ₘ-NR(12)R(13); R(12) und R(13)
unabhängig voneinander Wasserstoff oder Alkyl mit 1, 2, 3 or 4 C-Atomen;
m Nuill, 2, 3 oder 4;
NH-C(=O)-NH₂, C(=O)-O-Alkyl mit 1, 2, 3 oder 4 C-Atomen, C(=O)-NH₂, C(=O)-NH-Alkyl mit 1, 2, 3 oder 4 C-Atomen, C(=O)-N(Alkyl)₂ mit 1, 2, 3 oder 4 C-Atomen in jeder Alkylgruppe, Alkenyl mit 2, 3, 4, 5, 6, 7, 8, 9 oder 10 C-Atomen, alkinyl mit 2, 3, 4, 5, 6, 7, 8, 9 oder 10 C-Atomen, Alkylaryl mit 1, 2, 3 or 4 C-Atomen in der Alkylgruppe, Alkenyl-aryl mit 2, 3, 4, 5, 6, 7, 8, 9 oder 10 C-Atomen in der Alkenylgruppe, Alkinyl-aryl mit 2, 3, 4, 5, 6, 7, 8, 9 oder 10 C-Atomen in der Alkinylgruppe, C₁-C₄-alkyl-substituiertes aryl, C₁-C₄-Alkyl-heteroaryl, C₁-C₄-Alkenyl-heteroaryl, Aminoalkyl-aryl mit 1, 2, 3 oder 4 C-Atomen in der Alkylgruppe, substituiertes Aryl, Heteroaryl und substituiertes heteroaryl;
R(3), R(4), R(5) and R(6)
unabhängig voneinander Wasserstoff, Alkyl mit 1, 2, 3, 4, 5, 6, 7, 8, 9 oder 10 C-Atomen, O-Alkyl mit 1, 2, 3, 4, 5, 6, 7, 8, 9 oder 10 C-Atomen, Halogen, (wie F, Cl, Br, I), OH, Aryl, substituiertes Aryl, Heteroaryl, substituiertes Heteroaryl, O-Niederalkyl, O-Aryl, O-Niederalkyl-aryl, O-Substituiertes Aryl, O-Niederalkyl-substituiertes Aryl, O-C(=O)-C₁-C₄-Alkyl-aryl, O-C(=O)-NH-C₁-C₄-Alkyl, O-C(=O)-N(C₁-C₄-Alkyl)₂, NO₂, CN, CF₃, NH₂, NH-C(=O)-C₁-C₄-Alkyl, NH-C(=O)-NH₂, COOH, C(=O)-O-C₁-C₄-alkyl, C(=O)-NH₂, C(=O)-NH-C₁-C₄-Alkyl, C(=O)-N(C₁-C₄-Alkyl)₂, C₁-C₄-COOH, C₁-C₄-Alkyl-C(=O)-O-C₁-C₄-alkyl, SO₃H, SO₂-Alkyl, SO₂-Alkylaryl, SO₂-N-(Alkyl)₂, SO₂-N(Alkyl)(alkylaryl), C(=O)-R(11), C₁-C₁₀-Alkyl-C(=O)-R(11), C₂-C₁₀-Alkenyl-C(=O)-R(11), C₂-C₁₀-Alkinyl-C(=O)-R(11), NH-C(=O)-C₁-C₁₀-Alkyl-C(=O)-R(11), O-C₁-C₁₁-Alkyl-C(=O)-R(11);
R(11) C₁-C₄-Alkyl, C₁-C₄-Alkynyl, Aryl, substituiertes Aryl, NH₂, NH-C₁-C₄-Alkyl, N-(C₁-C₄-Alkyl)₂, SO₃H, SO₂-alkyl, SO₂-Alkylaryl, SO₂-N-(Alkyl)₂, SO₂-N(Alkyl)(alkylaryl);
X O, S oder NH;
R(7), R(8), R(9) und R(10)
unabhängig voneinander Wasserstoff, Alkyl, Cycloalkyl, Aryl, Alkylaryl; oder
R(8) und R(9)
gemeinsam Teil eines 5, 6 oder 7-gliedrigen heterocyclischen Rings;
A abwesend oder eine ungiftige organische oder Mineralsäure.
ai) Benzyloxycarbonylguanidine der Formel I 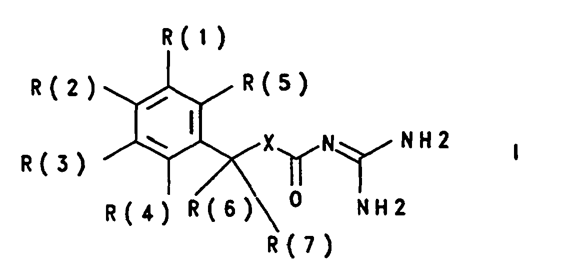 worin bedeuten:
R(1), R(2) und R(3)
unabhängig voneinander -Y-[4-R(8)-Phenyl], -Y-[3-R(8)-Phenyl] oder -Y-[2-R(8)-Phenyl],
wobei das Phenyl jeweils unsubstituiert oder substituiert ist mit 1 - 2 Substituenten aus der Gruppe bestehend aus F, Cl, -CF₃, Methyl, Hydroxy, Methoxy und -NR(96)R(97);
R(96) und R(97)
unabhängig voneinander Wasserstoff oder -CH₃;
Y eine Bindung, CH₂, Sauerstoff, -S- oder -NR(9); R(9) Wasserstoff oder Alkyl mit 1, 2, 3 oder 4 C-Atomen;
R(8) SOₐ[NR(98)]_{b}NR(99)R(10);
a 1 oder 2;
b 0 oder 1;
a + b = 2;
R(98), R(99) und R(10)
unabhängig voneinander Wasserstoff, -(C₁-C₈)-Alkyl, Benzyl, - (C₂-C₈)-Alkylen-NR(11)R(12), (C₂-C₈)-Alkylen-NR(13)-(C₂-C₈)-Alkylen-NR(37)R(38) oder (C₀-C₈)-Alkylen-CR(39)R(40)-CR(41)R(42)(C₀-C₈)-Alkylen-NR(43)R(44);
R(11), R(12), R(13), R(37), R(38), R(43) und R(44)
unabhängig voneinander Wasserstoff, -(C₁-C₈)-Alkyl oder Benzyl:
R(39), R(40), R(41) und R(42)
unabhängig voneinander Wasserstoff, -(C₁-C₈)-Alkyl oder -(C₀-C₃)-Alkylen-Phenyl,
wobei das Phenyl nicht substituiert oder substituiert ist mit 1 - 3 Substituenten ausgewählt aus der Gruppe bestehend aus F, Cl,-CF₃, Methyl und Methoxy;
oder
R(99) und R(10)
gemeinsam 4 - 6 Methylengruppen, von denen eine CH₂-Gruppe durch Sauerstoff, -S-, -NH-, -N-CH₃ oder -N-Benzyl ersetzt sein kann;
oder
R(8) SOₐ[NR(98)]_{b}NR(95)-C[=N-R(94)]-NR(93)R(92);
R(92), R(93), R(94) und R(95)
unabhängig voneinander Wasserstoff oder Alkyl mit 1, 2, 3 oder 4 C-Atomen;
oder
R(1), R(2) und R(3)
unabhängig voneinander Pyrrol-1-yl, Pyrrol-2-yl oder Pyrrol-3-yl,
welches nicht substituiert oder substituiert ist mit 1 - 4 Substituenten ausgewählt aus der Gruppe bestehend aus F, Cl, Br, I, -CN, (C₂-C₈)-Alkanoyl, (C₂-C₈)-Alkoxycarbonyl, Formyl, Carboxy, -CF₃, Methyl, Methoxy;
oder
R(1), R(2) und R(3) unabhängig voneinander Wasserstoff, -(C₁-C₈)-Alkyl, -(C₂-C₈)-Alkenyl oder -(CH₂)ₘR(14);
m Null, 1 oder 2;
R(14) -(C₃-C₈)-Cycloalkyl oder Phenyl,
welches nicht substituiert oder substituiert ist mit 1 - 3 Substituenten ausgewählt aus der Gruppe bestehend aus F und Cl, -CF₃, Methyl, Methoxy und -NR(15)R(16);
R(15) und R(16)
Wasserstoff oder -CH₃;
oder
R(1), R(2) und R(3)
unabhängig voneinander -Q-4-[(CH₂)ₖ-CHR(17)-(C=O)R(20)]-Phenyl, -Q-3-(CH₂)ₖ-CHR(17)-(C=O)R(20)]-Phenyl oder -Q-2-[(CH₂)ₖ-CHR(17)-(C=O)R(20)]-Phenyl,
wobei das Phenyl jeweils unsubstituiert oder substituiert ist mit 1 - 2 Substituenten aus der Gruppe F, Cl, -CF₃, Methyl, Hydroxy, Methoxy und -NR(35)R(36);
R(35) und R(36)
unabhängig voneinander Wasserstoff oder -CH₃;
Q eine Bindung, Sauerstoff, -S- oder -NR(18);
R(18) Wasserstoff oder -(C₁-C₄)-Alkyl;
R(17) -OR(21) oder -NR(21)R(22);
R(21) und R(22)
unabhängig voneinander Wasserstoff, -(C₁-C₈)-Alkyl, -(C₁-C₈)-Alkanoyl, -(C₁-C₈)-Alkoxycarbonyl, Benzyl, Benzyloxycarbonyl;
oder
R(21) Trityl;
R(20) -OR(23) oder -NR(23)R(24);
R(23), R(24) unabhängig voneinander
Wasserstoff, -(C₁-C₈)-Alkyl oder Benzyl;
k Null, 1, 2, 3 oder 4;
oder
R(1), R(2) und R(3)
unabhängig voneinander (C₁-C₉)-Heteroaryl, das über C oder N verknüpft ist und das unsubstituiert oder substituiert ist mit 1-3 Substituenten aus der Gruppe F, Cl, CF₃, CH₃, Methoxy, Hydroxy, Amino, Methylamino und Dimethylamino;
oder
R(1), R(2) und R(3)
-SR(25), -OR(25), -NR(25)R(26), -CR(25)R(26)R(27); R(25) -C_{f}H_{2f}-(C₁-C₉)-Heteroaryl,
das unsubstituiert oder substituiert ist mit 1-3 Substituenten aus der Gruppe F, Cl, CF₃, CH₃, Methoxy, Hydroxy, Amino, Methylamino und Dimethylamino;
f Null, 1 oder 2;
R(26) und R(27)
unabhängig voneinander wie R(25) definiert oder Wasserstoff oder (C₁-C₄)-Alkyl,
oder
R(1), R(2) und R(3)
unabhängig voneinander (C₁-C₉)-Heteroaryl-N-Oxid, das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(1), R(2) und R(3)
unabhängig voneinander -SR(28), -OR(28), -NR(28)R(29) oder -CR(28)R(29)R(30); R(28) -C_{g}H_{2g}-(C₁-C₉)-Heteroaryl-N-Oxid, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
g Null, 1 oder 2;
R(29), R(30)
unabhängig voneinander wie R(28) definiert, Wasserstoff oder (C₁-C₄)-Alkyl;
oder
R(1), R(2) und R(3)
unabhängig voneinander Wasserstoff, F, Cl, Br, I, -C≡N, T-(CH₂)ₕ-(CᵢF₂ᵢ₊₁), R(31)SOₗ-, R(32)R(33)N-CO-, R(34)-CO- oder R(45)R(46)N-SO₂, wobei die Perfluoralkylgruppe geradkettig oder verzweigt ist;
T eine Bindung, Sauerstoff, -S- oder -NR(47); l Null, 1 oder 2;
h Null, 1 oder 2;
i 1, 2, 3, 4, 5 oder 6;
R(31), R(32), R(34) und R(45)
unabhängig voneinander -(C₁-C₈)-Alkyl, -(C₃-C₆)Alkenyl, (CH₂)ₙR(48) oder -CF₃;
n Null, 1, 2, 3 oder 4;
R(47) Wasserstoff oder Alkyl mit 1, 2 oder 3 C-Atomen;
R(48) -(C₃-C₇)-Cycloalkyl oder Phenyl,
welches nicht substituiert ist oder substituiert mit 1 - 3 Substituenten ausgewählt aus der Gruppe bestehend aus F, Cl, -CF₃, Methyl, Methoxy und -NR(49)R(50);
R(49) und R(50)
Wasserstoff oder Alkyl mit 1, 2, 3 oder 4 C-Atomen;
oder
R(32), R(34) und R(45)
Wasserstoff;
R(33) und R(46)
unabhängig voneinander Wasserstoff oder Alkyl mit 1, 2, 3 oder 4 C-Atomen;
oder
R(32) und R(33) sowie R(45) und R(46)
gemeinsam 5 oder 6 Methylengruppen, von denen eine CH₂-Gruppe durch Sauerstoff, -S-, -NH-, -NCH₃ oder -N-Benzyl ersetzt sein kann;
oder
R(1), R(2) und R(3)
unabhängig voneinander R(51)-A-G-D-;
R(51) ein basischer protonierbarer Rest, d.h. eine Aminogruppe -NR(52)R(53), eine Amidinogruppe R(52)R(53)N-C[=N-R(54)]- oder eine Guanidinogruppe R(52)R(53)N-C[=N-R(54)]-NR(55)-;
R(52), R(53), R(54) und R(55) unabhängig voneinander Wasserstoff oder Alkyl mit 1, 2, 3 oder 4 C-Atomen;
oder
R(52) und R(53)
eine Gruppe C_{α}H_{2α};
α 4, 5, 6 oder 7;
wobei im Falle von α = 5, 6 oder 7 ein C-Atom der Gruppe C_{α}H_{2α} durch eine Heteroatomgruppe O, SO_{d} oder NR(56) ersetzt sein kann,
oder
R(53) und R(54) oder R(54) und R(55) oder R(52) und R(55)
eine Gruppe C_{γ}H_{2γ};
γ 2, 3, 4 oder 5;
wobei im Falle von γ = 3, 4 oder 5 ein C-Atom der Gruppe C_{γ}H_{2γ} durch eine Heteroatomgruppe O, SO_{d} oder NR(56) ersetzt sein kann;
d Null, 1 oder 2;
R(56) Wasserstoff oder Methyl;
oder
R(51) ein basisches heteroaromatisches Ringsystem mit 1 - 9 C-Atomen;
A eine Gruppe CₑH₂ₑ;
e Null, 1, 2, 3, 4, 5, 6, 7, 8, 9 oder 10;
wobei in der Gruppe CₑH₂ₑ ein C-Atom durch eine der Gruppierungen - O-,-CO-, -CH[OR(57)]-, -SOᵣ-, -NR(57)-, -NR(57)-CO-, -NR(57)-CO-NH-, -NR(57)-CO-NH-SO₂- oder -NR(57)-SO₂- ersetzt sein kann;
r Null, 1 oder 2;
G einen Phenylenrest, 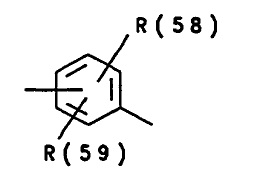 R(58) und R(59)
unabhängig voneinander Wasserstoff, Methyl, Methoxy, F, Cl, Br, J, CF₃ oder -SOₛ-R(60);
R(60) Methyl oder NR(61)R(62);
R(61) und R(62)
unabhängig voneinander Wasserstoff oder Alkyl mit 1, 2, 3 oder 4 C-Atomen;
D -CᵥH₂ᵥ-E_{w}-;
v Null, 1, 2, 3 oder 4;
E -O-, -CO-, -CH[OR(63)]-, -SOₐₐ- oder -NR(63)-;
w Null oder 1;
aa Null, 1 oder 2
R(63) Wasserstoff oder Methyl,
oder
R(1), R(2) und R(3)
unabhängig voneinander -CF₂R(64), -CF[R(65)][R(66)], -CF[(CF₂)_{q}-CF₃)][R(65)], -C[(CF₂)ₚ-CF₃]=CR(65)R(66);
R(64) Alkyl mit 1, 2, 3 oder 4 C-Atomen oder Cycloalkyl mit 3, 4, 5, 6 oder 7 C-Atomen;
R(65) und R(66) unabhängig voneinander Wasserstoff oder Alkyl mit 1, 2, 3 oder 4 C-Atomen;
q Null, 1 oder 2;
p Null, 1 oder 2;
oder
R(1), R(2) und R(3)
unabhängig voneinander -OR(67) oder -NR(67)R(68);
R(67) und R(68)
unabhängig voneinander Wasserstoff oder Alkyl mit 1, 2, 3, 4, 5 oder 6 C-Atomen;
oder
R(67) und R(68)
gemeinsam 4, 5, 6 oder 7 Methylengruppen, von denen eine CH₂-Gruppe durch Sauerstoff, -S-, SO₂, -NH-, -NCH₃ oder -N-Benzyl ersetzt sein kann;
R(4) und R(5) unabhängig voneinander Wasserstoff, Alkyl mit 1, 2, 3 oder 4 C-Atomen, F, Cl, -OR(69), -NR(70)R(71) oder -C_{z}F_{2z+1};
R(69), R(70) und R(71)
unabhängig voneinander Wasserstoff oder Alkyl mit 1, 2 oder 3 C-Atomen;
z 1, 2, 3 oder 4;
R(6) und R(7)
unabhängig voneinander Wasserstoff oder Alkyl mit 1, 2, 3 oder 4 C-Atomen;
X Sauerstoff oder NR(72);
R(72) Wasserstoff oder Alkyl mit 1, 2, 3 oder 4 C-Atomen; sowie deren pharmazeutisch verträgliche Salze;
ak) Fluorphenylgruppen tragende Alkenylcarbonsäure-guanidide der Formel I 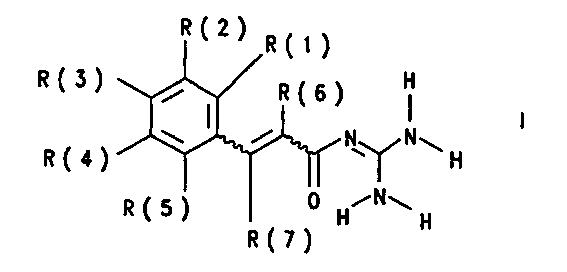 worin bedeuten:
R(6) Wasserstoff, (C₁-C₈)-Alkyl, (C₃-C₈)-Cycloalkyl oder Phenyl,
wobei die Phenylgruppe nicht substituiert oder substituiert ist mit 1 - 3 Substituenten ausgewählt aus der Gruppe bestehend aus F, Cl, CF₃, Methyl, Methoxy und NR(9)R(10);
R(9) und R(10)
Wasserstoff, (C₁-C₄)-Alkyl oder (C₁-C₄)-Perfluoralkyl;
R(7) unabhängig wie R(6) definiert;
R(1), R(2), R(3), R(4) und R(5)
unabhängig voneinander Wasserstoff oder F;
wobei jedoch mindestens einer der Reste R(1), R(2), R(3), R(4) und R(5) Fluor sein muß;
sowie deren pharmazeutisch verträgliche Salze;
al) Benzoylguanidine der Formel I 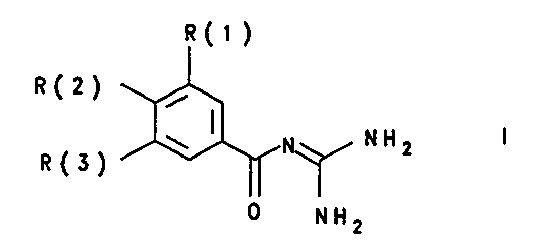 worin bedeuten:
R(1) R(4)-SOₘ oder R(5)R(6)N-SO₂-;
m 1 oder 2;
R(4) und R(5)
unabhängig voneinander Alkyl mit 1, 2, 3, 4, 5, 6, 7 oder 8 C-Atomen, Alkenyl mit 3, 4, 5 oder 6 C-Atomen, CF₃ oder -CₙH₂ₙ-R(7);
n Null, 1, 2, 3 oder 4;
R(6) H oder Alkyl mit 1, 2, 3 oder 4 C-Atomen;
R(7) Cycloalkyl mit 3, 4, 5, 6, 7 oder 8 C-Atomen oder Phenyl,
welches nicht substituiert ist oder substituiert mit 1 - 3 Substituenten ausgewählt aus der Gruppe bestehend aus F, Cl, CF₃, Methyl, Methoxy und NR(8)R(9);
R(8) und R(9)
H oder Alkyl mit 1, 2, 3 oder 4 C-Atomen;
oder
R(5) auch Wasserstoff;
oder
R(5) und R(6)
gemeinsam 4 oder 5 Methylengruppen, von denen eine CH₂-Gruppe durch Sauerstoff, S, NH, N-CH₃ oder N-Benzyl ersetzt sein kann;
oder
R(1) Oₚ-(CH₂)_{q}-(CF₂)ᵣ-CF₃;
p Null oder 1;
q Null, 1 oder 2;
r Null, 1, 2 oder 3;
oder
R(1) -SR(10), -OR(10) oder -CR(10)R(11)R(12);
R(10), R(11) und R(12)
unabhängig voneinander Wasserstoff, Alkyl mit 1, 2, 3, 4, 5, 6, 7 oder 8 C-Atomen, -CₛH₂ₛ-(C₃-C₈)-Cycloalkyl oder ein aromatisches System ausgewählt aus der Gruppe bestehend aus Pyridyl, Pyrrolyl, Chinolyl, Isochinolyl, Imidazolyl oder Phenyl;
s Null, 1 oder 2;
wobei die aromatischen Systeme Pyridyl, Pyrrolyl, Chinolyl, Isochinolyl, Imidazolyl und Phenyl unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
R(2) -(CH₂)ᵤ-(CF₂)ₜ-CF₃;
t Null, 1, 2 oder 3;
u Null oder 1;
R(3) Wasserstoff oder unabhängig wie R(1) definiert;
sowie deren pharmazeutisch verträgliche Salze;
am) Substituierte Zimtsäureguanidide der Formel I 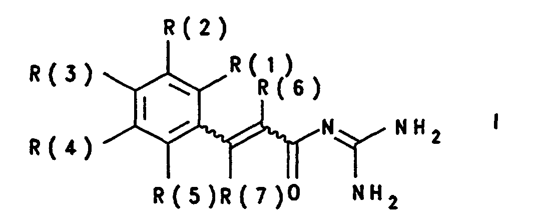 worin bedeuten:
mindestens einer der Substituenten R(1), R(2), R(3), R(4) und R(5)
-Xₐ-Y_{b}-Lₙ-U;
X CR(16)R(17), O, S oder NR(18);
R(16), R(17) und R(18)
unabhängig voneinander H, Alkyl mit 1, 2, 3 oder 4 C-Atomen oder Perfluoralkyl mit 1, 2, 3 oder 4 C-Atomen;
a Null oder 1;
Y Alkylen mit 1, 2, 3, 4, 5, 6, 7 oder 8 C-Atomen, Alkylen-T mit 1, 2, 3, 4, 5, 6, 7 oder 8 C-Atomen in der Alkylengruppe, T, T-Alkylen mit 1, 2, 3, 4, 5, 6, 7 oder 8 C-Atomen in der Alkylengruppe;
T NR(20), O, S oder Phenylen,
wobei das Phenylen nicht substituiert oder substituiert ist mit 1 - 3 Substituenten ausgewählt aus der Gruppe bestehend aus F, Cl, CF₃, Methyl, Methoxy und NR(21)R(22);
R(20), R(21) und R(22)
unabhängig voneinander H, Alkyl mit 1, 2, 3 oder 4 C-Atomen oder Perfluoralkyl mit 1, 2, 3 oder 4 C-Atomen;
b Null oder 1;
L O, S, NR(23) oder CₖH₂ₖ;
k 1, 2, 3, 4, 5, 6, 7, 8;
n Null oder 1;
U NR(24)R(25) oder einen N-haltigen Heterocyclus mit 1, 2, 3, 4, 5, 6, 7, 8 oder 9 C-Atomen;
R(24) und R(25)
unabhängig voneinander Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen;
oder
R(24) und R(25)
gemeinsam 4 oder 5 Methylenguppen, von denen eine CH₂-Gruppe durch Sauerstoff, S, NH, N-CH₃ oder N-Benzyl ersetzt sein kann;
wobei die N-haltigen Heterocyclen N- oder C-verbrückt sind und nicht substituiert oder substituiert sind mit 1 - 3 Substituenten ausgewählt aus der Gruppe bestehend aus F, Cl, CF₃, Methyl, Methoxy und NR(27)R(28);
R(23), R(27) und R(28)
unabhängig voneinander H, Alkyl mit 1, 2, 3 oder 4 C-Atomen oder Perfluoralkyl mit 1, 2, 3 oder 4 C-Atomen; und die jeweils anderen Substituenten R(1), R(2), R(3), R(4) und R(5) unabhängig voneinander H, F, Cl, Br, I, CN, -Oₙ-CₘH₂ₘ₊₁, -Oₚ-(CH₂)ₛ-C_{q}F_{2q+1} oder -CᵣH₂ᵣR(10);
n Null oder 1;
m Null 1, 2, 3, 4, 5, 6, 7 oder 8;
p Null oder 1; q 1, 2, 3, 4, 5, 6, 7 oder 8;
s Null, 1, 2, 3 oder 4;
r Null, 1, 2, 3 oder 4;
R(10)
Cycloalkyl mit 3, 4, 5, 6, 7 oder 8 C-Atomen, oder Phenyl, wobei das Phenyl nicht substituiert oder substituiert ist mit 1 - 3 Substituenten ausgewählt aus der Gruppe bestehend aus F, Cl, CF₃, Methyl, Methoxy und NR(11)R(12); R(11) und R(12)
unabhängig voneinander H, Alkyl mit 1, 2, 3 oder 4 C-Atomen oder Perfluoralkyl mit 1, 2, 3 oder 4 C-Atomen;
R(6) und R(7)
unabhängig voneinander Wasserstoff, F, Cl, Br, I, CN, Alkyl mit 1, 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen, oder Phenyl,
das nicht substituiert oder substituiert ist mit 1 - 3 Substituenten ausgewählt aus der Gruppe bestehend aus F, Cl, CF₃, Methyl, Methoxy und NR(14)R(15);
R(14) und R(15)
unabhängig voneinander H, Alkyl mit 1, 2, 3 oder 4 C-Atomen oder Perfluoralkyl mit 1, 2, 3 oder 4 C-Atomen;
sowie deren pharmazeutisch verträgliche Salze;
an) Benzoylguanidine der Formel I 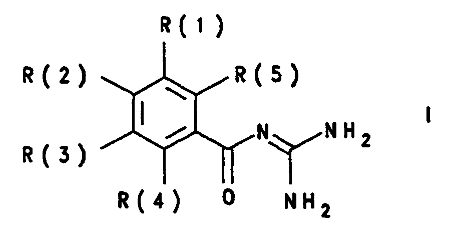 worin bedeuten:
mindestens einer der Substituenten R(1), R(2) und R(3)
R(6)-C(OH)₂-;
R(6) Perfluoralkyl mit 1, 2 oder 3-C-Atomen, das geradkettig oder verzweigt ist;
und die übrigen Substituenten R(1), R(2) und R(3)
unabhängig voneinander Wasserstoff, OH, F, Cl, Br, I, Alkyl mit 1, 2, 3, 4, 5 oder 6 C-Atomen, Cycloalkyl mit 3, 4, 5 oder 6 C-Atomen, Alkoxy mit 1, 2, 3 oder 4 C-Atomen oder Phenoxy,
das unsubstituiert ist oder substituiert ist mit 1 - 3 Substituenten
ausgewählt auf der Gruppe bestehend aus F, Cl, Methyl und Methoxy;
oder
die übrigen Substituenten R(1), R(2) und R(3)
unabhängig voneinander Alkyl-SOₓ, -CR(7)=CR(8)R(9) oder -C≡CR(9);
x Null, 1 oder 2;
R(7) Wasserstoff oder Methyl;
R(8) und R(9)
unabhängig voneinander Wasserstoff, Alkyl mit 1, 2, 3 oder 4 C-Atomen, Cycloalkyl mit 3, 4, 5, 6, 7 oder 8 C-Atomen oder Phenyl, das unsubstituiert ist oder substituiert ist mit 1 - 3 Substituenten ausgewählt auf der Gruppe bestehend aus F, Cl, CF₃, Methyl und Methoxy;
oder
die übrigen Substituenten R(1), R(2) und R(3) unabhängig voneinander Phenyl, C₆H₅-(C₁-C₄)-Alkyl, Naphthyl, Biphenylyl, Chinolinyl, Isochinolinyl oder Imidazolyl,
wobei Chinolinyl, Isochinolinyl oder Imidazolyl über C oder N gebunden sind, und wobei Phenyl, C₆H₅-(C₁-C₄)-Alkyl, Naphthyl, Biphenylyl, Chinolinyl, Isochinolinyl und Imidazolyl unsubstituiert sind oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
oder
die übrigen Substituenten R(1), R(2) und R(3) unabhängig voneinander SR(10), -OR(10), -CR(10)R(11)R(12);
R(10)
-C_{f}H_{2f}-(C₃-C₈)-Cycloalkyl, Chinolinyl, Isochinolinyl, Pyridinyl, Imidazolyl oder Phenyl,
wobei die aromatischen Systeme Chinolinyl, Isochinolinyl, Pyridinyl, Imidazolyl und Phenyl unsubstituiert sind oder substituiert sind mit 1 - 3 Substituenten ausgewählt aus der gruppe bestehend aus F, Cl, CF₃, CH₃, Methoxy, Hydroxy, Amino, Methylamino und Dimethylamino;
f Null, 1 oder 2;
R(11) und R(12)
unabhängig voneinander wie R(10) definiert, Wasserstoff oder Alkyl mit 1, 2, 3 oder 4 C-Atomen;
R(4) und R(5)
unabhängig voneinander Wasserstoff, Alkyl mit 1, 2 oder 3-C-Atomen, F, Cl, Br, I, CN, OR(13), NR(14)R(15), -(CH₂)ₙ-(CF₂)ₒ-CF₃; R(13), R(14) und R(15)
unabhängig voneinander Wasserstoff oder Alkyl mit 1, 2, 3 oder 4 C-Atomen;
n Null oder 1;
o Null, 1 oder 2;
sowie deren pharmakologisch akzeptable Salze;
ao) Sulfonimidamide der Formel I 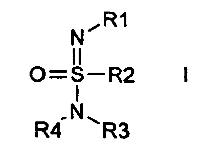 worin bedeuten:
mindestens einer der drei Substituenten R(1), R(2) und R(3)
ein Benzoylguanidin, 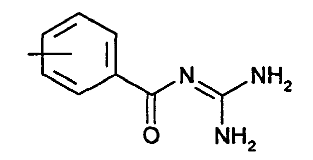 das im Phenylteil unsubstituiert oder substituiert ist mit 1 - 4 Resten ausgewählt aus der Gruppe bestehend aus Alkyl mit 1, 2, 3, 4, 5, 6, 7 oder 8 C-Atomen, Alkenyl mit 2, 3, 4, 5, 6, 7 oder 8 C-Atomen, -(CH₂)ₘ-R(14), F, Cl, Br, I, -C≡N, CF₃, R(22)SO₂-, R(23)R(24)N-CO-, R(25)-CO-, R(26)R(27)N-SO₂, -OR(35), -SR(35) oder -NR(35)R(36); m Null, 1 oder 2;
R(14)
-(C₃-C₈)-Cycloalkyl oder Phenyl,
welches nicht substituiert oder substituiert ist mit 1 - 3 Substituenten ausgewählt aus der Gruppe bestehend aus F und Cl, -CF₃, Methyl, Methoxy und -NR(15)R(16); R(15) und R(16)
unabhängig voneinander Wasserstoff oder -CH₃;
R(22), R(23), R(25) und R(26)
unabhängig voneinander Alkyl mit 1, 2, 3, 4, 5, 6, 7 oder 8 C-Atomen, Alkenyl mit 2, 3, 4, 5, 6, 7 oder 8 C-Atomen, (CH₂)ₙR(29) oder -CF₃;
n Null, 1, 2, 3 oder 4;
R(29) -(C₃-C₇)-Cycloalkyl oder Phenyl, welches nicht substituiert ist oder substituiert mit 1 - 3 Substituenten ausgewählt aus der Gruppe bestehend aus F, Cl, -CF₃, Methyl, Methoxy und -NR(30)R(31); R(30) und R(31)
Wasserstoff oder Alkyl mit 1, 2, 3 oder 4 C-Atomen;
oder
R(23), R(25) und R(26)
Wasserstoff;
R(24) und R(27)
unabhängig voneinander Wasserstoff oder Alkyl mit 1, 2, 3 oder 4 C-Atomen;
oder
R(23) und R(24) sowie R(26) und R(27)
gemeinsam 5 oder 6 Methylengruppen, von denen eine CH₂-Gruppe durch Sauerstoff, -S-, -NH-, -NCH₃ oder -N-Benzyl ersetzt sein kann;
R(35) und R(36)
unabhängig voneinander Wasserstoff oder Alkyl mit 1, 2, 3, 4, 5 oder 6 C-Atomen;
oder
R(35) und R(36)
gemeinsam 4 - 7 Methylengruppen, von denen eine CH₂-Gruppe durch Sauerstoff, -S-, -NH-, -NCH₃ oder -N-Benzyl ersetzt sein kann;
oder
R(35)
Phenyl, welches nicht substituiert ist oder substituiert mit 1 - 3 Substituenten ausgewählt aus der Gruppe bestehend aus F, Cl, -CF₃, Methyl, Methoxy, SO₂R(5), SO₂NR(6)R(7) und -NR(32)R(33);
R(5) Alkyl mit 1, 2, 3, 4, 5 oder 6 C-Atomen
R(6) und R(7)
unabhängig voneinander Wasserstoff oder Alkyl mit 1, 2, 3 oder 4 C-Atomen;
R(32) und R(33)
unabhängig voneinander Wasserstoff oder Alkyl mit 1, 2, 3 oder 4 C-Atomen;
oder
R(35)
C₁-C₉-Heteroaryl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
und die jeweils anderen Substituenten R(1), R(2) und R(3) unabhängig voneinander Alkyl mit 1, 2, 3, 4, 5, 6, 7 oder 8 C-Atomen, (CH₂)ₚR(10)
p Null, 1, 2, 3 oder 4;
R(10) Phenyl,
welches nicht substituiert ist oder substituiert mit 1 - 3 Substituenten ausgewählt aus der Gruppe bestehend aus F, Cl, -CF₃, Methyl, Methoxy, -SO₂NR(17)R(8) und -SO₂R(9); R(17) und R(8)
unabhängig voneinander Wasserstoff oder Alkyl mit 1, 2, 3 oder 4 C-Atomen;
R(9) Alkyl mit 1, 2, 3 oder 4 C-Atomen;
oder der jeweils andere Rest R(1) und R(3)
Wasserstoff,
R(4) Wasserstoff oder Alkyl mit 1, 2, 3 oder 4 C-Atomen; sowie deren pharmazeutisch verträgliche Salze;
ap) Benzoylguanidine der Formel I 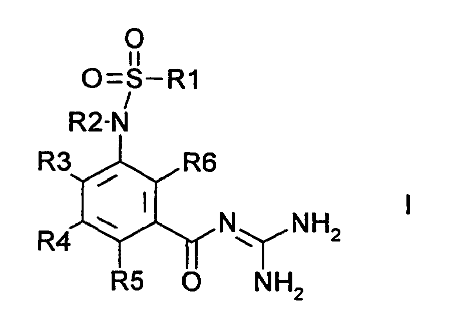 worin bedeuten:
R(1) Alkyl mit 1, 2, 3, 4, 5, 6, 7 oder 8 C-Atomen oder NR(7)R(8); R(7) und R(8)
unabhängig voneinander Wasserstoff oder Alkyl mit 1, 2, 3, 4, 5, 6, 7 oder 8 C-Atomen
R(2) Wasserstoff, Alkyl mit 1, 2, 3, 4, 5, 6, 7 oder 8 C-Atomen oder -SO₂R(9) R(9) unabhängig wie R(1) definiert;
R(3) Wasserstoff, -SR(25), -OR(25), -NR(25)R(26) oder -CR(25)R(26)R(27); R(25) Wasserstoff, Alkyl mit 1, 2, 3, 4, 5, 6, 7 oder 8 C-Atomen, oder Phenyl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(25) -(C₁-C₉)-Heteroaryl,
das unsubstituiert oder substituiert ist mit 1 - 3 Substituenten ausgewählt aus der Gruppe bestehend aus der Gruppe F, Cl, CF₃, CH₃, Methoxy, Hydroxy, Amino, Methylamino und Dimethylamino;
R(26) und R(27) unabhängig voneinander wie R(25) definiert oder Wasserstoff oder Alkyl mit 1, 2, 3, 4, 5, 6, 7 oder 8 C-Atomen;
R(4) Wasserstoff, F, Cl, Br, I, OH, -C≡N, CF₃, Alkyl mit 1, 2, 3, 4, 5, 6, 7 oder 8 C-Atomen, Alkenyl mit 2, 3, 4, 5, 6, 7 oder 8 C-Atomen oder -(CH₂)ₘR(14);
m Null, 1 oder 2;
R(14) -(C₃-C₈)-Cycloalkyl oder Phenyl,
welches nicht substituiert oder substituiert ist mit 1 - 3 Substituenten ausgewählt aus der Gruppe bestehend aus F und Cl, -CF₃, Methyl, Methoxy und -NR(15)R(16);
R(15) und R(16)
unabhängig voneinander Wasserstoff oder -CH₃;
R(5) und R(6)
unabhängig voneinander Wasserstoff, Alkyl mit 1, 2, 3 oder 4 C-Atomen, F, Cl, -OR(32), -NR(33)R(34) oder CF₃;
R(32), R(33) und R(34)
unabhängig voneinander Wasserstoff oder Alkyl mit 1, 2, 3 oder 4 C-Atomen;
sowie deren pharmazeutisch verträgliche Salze;
aq) Benzoldicarbonsäure-diguanidide der Formel I 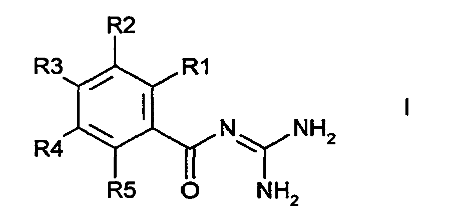 worin bedeuten:
einer der Reste R(1), R(2), R(3) und R(4)
-CO-N=C(NH₂)₂;
und die jeweils anderen Reste R(1), R(2), R(3) und R(4):
R(1) Wasserstoff, Alkyl mit 1, 2, 3 oder 4 C-Atomen, F, Cl, Br, I, -OR(32),
-NR(33)R(34) oder CF₃;
R(32), R(33) und R(34)
unabhängig voneinander Wasserstoff oder Alkyl mit 1, 2, 3 oder 4 C-Atomen;
R(2) und R(4)
unabhängig voneinander Wasserstoff, F, Cl, Br, I, OH, -CN, CF₃, -CO-N=C(NH₂)₂, Alkyl mit 1, 2, 3, 4, 5, 6, 7 oder 8 C-Atomen, Alkenyl mit 2, 3, 4, 5, 6, 7 oder 8 C-Atomen oder -(CH₂)ₘR(14);
m Null, 1 oder 2;
R(14) -(C₃-C₈)-Cycloalkyl oder Phenyl,
welches nicht substituiert oder substituiert ist mit 1 - 3 Substituenten ausgewählt aus der Gruppe bestehend aus F und Cl, -CF₃, Methyl, Methoxy und -NR(15)R(16);
R(15) und R(16)
Wasserstoff oder -CH₃;
oder
R(2) und R(4)
unabhängig voneinander Pyrrol-1-yl, Pyrrol-2-yl oder Pyrrol-3-yl, welches nicht substituiert oder substituiert ist mit 1 - 4 Substituenten ausgewählt aus der Gruppe bestehend aus F, Cl, Br, I,-CN, (C₂-C₈)-Alkanoyl, (C₂-C₈)-Alkoxycarbonyl, Formyl, Carboxy, -CF₃, Methyl, Methoxy;
oder
R(2) und R(4)
unabhängig voneinander R(22)-SO₂-, R(23)R(24)N-CO-, R(28)-CO- oder R(29)R(30)N-SO₂;
R(22) und R(28)
unabhängig voneinander Methyl oder -CF₃;
R(23), R(24), R(29) und R(30)
unabhängig voneinander Wasserstoff oder Methyl;
oder
R(2) und R(4)
unabhängig voneinander -OR(35) oder -NR(35)R(36);
R(35) und R(36)
unabhängig voneinander Wasserstoff oder Alkyl mit 1, 2, 3, 4, 5 oder 6 C-Atomen;
oder
R(35) und R(36)
gemeinsam 4 - 7 Methylengruppen, von denen eine CH₂-Gruppe durch
Sauerstoff, -S-, -NH-, -NCH₃ oder -N-Benzyl ersetzt sein kann;
R(3) Wasserstoff, -SR(25), -OR(25), -NR(25)R(26), -CR(25)R(26)R(27);
R(25) Wasserstoff, Alkyl mit 1, 2, 3, 4, 5, 6, 7 oder 8 C-Atomen oder Phenyl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(25) -(C₁-C₉)-Heteroaryl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
R(26) und R(27)
unabhängig voneinander wie R(25) definiert oder Wasserstoff oder Alkyl mit 1, 2, 3, 4, 5, 6, 7 oder 8 C-Atomen;
R(5) Alkyl mit 1, 2, 3 oder 4 C-Atomen, F, Cl, Br, I, X-(CH₂)_{y}-CF₃ oder Phenyl, welches nicht substituiert oder substituiert ist mit 1 - 3 Substituenten ausgewählt aus der Gruppe bestehend aus F und Cl, -CF₃, Methyl, Methoxy und -NR(6)R(7);
R(6) und R(7)
unabhängig voneinander Wasserstoff oder -CH₃;
X eine Bindung oder Sauerstoff;
y Null, 1 oder 2;
sowie deren pharmazeutisch verträgliche Salze;
ar) Benzoldicarbonsäure-diguanidide der Formel I 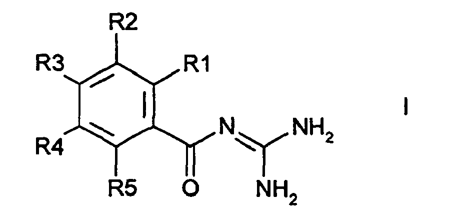 worin bedeuten:
einer der Reste R(1), R(2), R(3) und R(5)
-CO-N=C(NH₂)₂;
und die jeweils anderen Reste R(1), R(2), R(3) und R(5):
R(1) und R(5)
unabhängig voneinander Wasserstoff, Alkyl mit 1, 2, 3 oder 4 C-Atomen, F, Cl, -OR(32), -NR(33)R(34) oder CF₃;
R(32), R(33) und R(34)
unabhängig voneinander Wasserstoff oder Alkyl mit 1, 2, 3 oder 4 C-Atomen;
R(2) Wasserstoff, F, Cl, Br, I, OH, -C°N, CF₃, -CO-N=C(NH₂)₂, Alkyl mit 1, 2, 3, 4, 5, 6, 7 oder 8 C-Atomen, Alkenyl mit 2, 3, 4, 5, 6, 7 oder 8 C-Atomen oder -(CH₂)ₘR(14);
m Null, 1 oder 2;
R(14) -(C₃-C₈)-Cycloalkyl oder Phenyl,
welches nicht substituiert oder substituiert ist mit 1 - 3 Substituenten ausgewählt aus der Gruppe bestehend aus F und Cl, -CF₃, Methyl, Methoxy und -NR(15)R(16);
R(15) und R(16)
unabhängig voneinander Wasserstoff oder -CH₃;
oder
R(2) R(22)-SO₂-, R(23)R(24)N-CO-, R(28)-CO- oder R(29)R(30)N-SO₂;
R(22) und R(28)
unabhängig voneinander Methyl oder -CF₃;
R(23), R(24), R(29) und R(30)
unabhängig voneinander Wasserstoff oder Methyl;
oder
R(2) -OR(35) oder -NR(35)R(36);
R(35) und R(36)
unabhängig voneinander Wasserstoff oder Alkyl mit 1, 2, 3, 4, 5 oder 6 C-Atomen;
oder
R(35) und R(36)
gemeinsam 4 - 7 Methylengruppen, von denen eine CH₂-Gruppe durch
Sauerstoff, -S-, -NH-, -NCH₃ oder -N-Benzyl ersetzt sein kann;
R(3) Wasserstoff, -SR(25), -OR(25), -NR(25)R(26), -CR(25)R(26)R(27);
R(25) Wasserstoff, Alkyl mit 1, 2, 3, 4, 5, 6, 7 oder 8 C-Atomen oder Phenyl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(25) -(C₁-C₉)-Heteroaryl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
R(26) und R(27)
unabhängig voneinander wie R(25) definiert oder Wasserstoff oder
Alkyl mit 1, 2, 3, 4, 5, 6, 7 oder 8 C-Atomen;
R(4) CF₃, Alkyl mit 1, 2, 3, 4, 5, 6, 7 oder 8 C-Atomen, Alkenyl mit 2, 3, 4, 5, 6, 7 oder 8 C-Atomen, -(C₃-C₈)-Cycloalkyl oder -(CH₂)ₘR(14);
m 1 oder 2;
R(14) -(C₃-C₈)-Cycloalkyl oder Phenyl, welches nicht substituiert oder substituiert ist mit 1 - 3 Substituenten ausgewählt aus der Gruppe bestehend aus F und Cl, -CF₃, Methyl, Methoxy und -NR(15)R(16);
R(15) und R(16)
unabhängig voneinander Wasserstoff oder -CH₃;
oder
R(4) Phenyl,
welches substituiert ist mit 2, 3, 4 oder fünf Substituenten ausgewählt aus der Gruppe bestehend aus F, Cl, CF₃, Methyl, Methoxy und -NR(15)R(16);
R(15) und R(16)
unabhängig voneinander Wasserstoff oder CH₃; sowie deren pharmazeutisch verträgliche Salze;
as) Diaryldicarbonsäure-diguanidide der Formel I 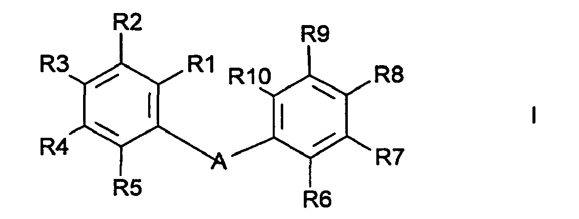 worin bedeuten:
einer der Reste R(1), R(2), R(3), R(4) und R(5)
-CO-N=C(NH₂)₂;
die jeweils anderen Reste R(1) und R(5)
unabhängig voneinander Wasserstoff, Alkyl mit 1, 2, 3 oder 4 C-Atomen, F, Cl, -OR(32), -NR(33)R(34) oder CF₃;
R(32), R(33) und R(34)
unabhängig voneinander Wasserstoff oder Alkyl mit 1, 2, 3 oder 4 C-Atomen;
die jeweils anderen Reste R(2) und R(4) unabhängig voneinander Wasserstoff, F, Cl, Br, I, OH, -CN, CF₃, -CO-N=C(NH₂)₂, Alkyl mit 1, 2, 3, 4, 5, 6, 7 oder 8 C-Atomen, Alkenyl mit 2, 3, 4, 5, 6, 7 oder 8 C-Atomen oder -(CH₂)ₘR(14);
m Null, 1 oder 2;
R(14) -(C₃-C₈)-Cycloalkyl oder Phenyl,
welches nicht substituiert oder substituiert ist mit 1 - 3 Substituenten ausgewählt aus der Gruppe bestehend aus F und Cl, -CF₃, Methyl, Methoxy und -NR(15)R(16);
R(15) und R(16)
Wasserstoff oder -CH₃;
oder
die jeweils anderen Reste R(2) und R(4)
unabhängig voneinander Pyrrol-1-yl, Pyrrol-2-yl oder Pyrrol-3-yl,
welches nicht substituiert oder substituiert ist mit 1 - 4 Substituenten ausgewählt aus der Gruppe bestehend aus F, Cl, Br, I,-CN, (C₂-C₈)-Alkanoyl, (C₂-C₈)-Alkoxycarbonyl, Formyl, Carboxy, -CF₃, Methyl, Methoxy;
oder
die jeweils anderen Reste R(2) und R(4)
R(22)-SO₂-, R(23)R(24)N-CO-, R(28)-CO- oder R(29)R(30)N-SO2;
R(22) und R(28)
unabhängig voneinander Methyl oder -CF₃;
R(23), R(24), R(29) und R(30)
unabhängig voneinander Wasserstoff oder Methyl;
oder
die jeweils anderen Reste R(2) und R(4)
unabhängig voneinander -OR(35) oder -NR(35)R(36);
R(35) und R(36)
unabhängig voneinander Wasserstoff oder Alkyl mit 1, 2, 3, 4, 5 oder 6 C-Atomen;
oder
R(35) und R(36)
gemeinsam 4 - 7 Methylengruppen, von denen eine CH₂-Gruppe durch Sauerstoff, -S-, -NH-, -NCH₃ oder -N-Benzyl ersetzt sein kann;
der jeweils andere Rest R(3)
Wasserstoff, -SR(25), -OR(25), -NR(25)R(26), -CR(25)R(26)R(27);
R(25) Wasserstoff, Alkyl mit 1, 2, 3, 4, 5, 6, 7 oder 8 C-Atomen oder Phenyl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(25) -(C₁-C₉)-Heteroaryl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
R(26) und R(27)
unabhängig voneinander wie R(25) definiert oder Wasserstoff oder Alkyl mit 1, 2, 3, 4, 5, 6, 7 oder 8 C-Atomen;
einer der Reste R(6), R(7), R(8), R(9) und R(10)
-CO-N=C(NH₂)₂;
die jeweils anderen Reste R(6) und R(10)
unabhängig voneinander Wasserstoff, Alkyl mit 1, 2, 3 oder 4 C-Atomen, F, Cl, -OR(132), -NR(133)R(134) oder CF₃; R(132), R(133) und R(134)
unabhängig voneinander Wasserstoff oder Alkyl mit 1, 2, 3 oder 4 C-Atomen;
die jeweils anderen Reste R(7) und R(9)
unabhängig voneinander Wasserstoff, F, Cl, Br, I, OH, -CN, CF₃, -CO-N=C(NH₂)₂, Alkyl mit 1, 2, 3, 4, 5, 6, 7 oder 8 C-Atomen, Alkenyl mit 2, 3, 4, 5, 6, 7 oder 8 C-Atomen oder -(CH₂)ₘₘR(114);
mm Null, 1 oder 2;
R(114)
-(C₃-C₈)-Cycloalkyl oder Phenyl,
welches nicht substituiert oder substituiert ist mit 1 - 3 Substituenten ausgewählt aus der Gruppe bestehend aus F und Cl, -CF₃, Methyl, Methoxy und -NR(115)R(116); R(115) und R(116)
Wasserstoff oder -CH₃;
oder
die jeweils anderen Reste R(7) und R(9)
unabhängig voneinander Pyrrol-1-yl, Pyrrol-2-yl oder Pyrrol-3-yl,
welches nicht substituiert oder substituiert ist mit 1 - 4 Substituenten ausgewählt aus der Gruppe bestehend aus F, Cl, Br, I,-CN, (C₂-C₈)-Alkanoyl, (C₂-C₈)-Alkoxycarbonyl, Formyl, Carboxy, -CF₃, Methyl und Methoxy;
oder
die jeweils anderen Reste R(7) und R(9)
R(122)-SO₂-, R(123)R(124)N-CO-, R(128)-CO- oder R(129)R(130)N-SO₂; R(122) und R(128)
unabhängig voneinander Methyl oder -CF₃;
R(123), R(124), R(129) und R(130)
unabhängig voneinander Wasserstoff oder Methyl;
oder
die jeweils anderen Reste R(7) und R(9)
unabhängig voneinander -OR(135) oder -NR(135)R(136);
R(135) und R(136)
unabhängig voneinander Wasserstoff oder Alkyl mit 1, 2, 3, 4, 5 oder 6 C-Atomen;
oder
R(135) und R(136)
gemeinsam 4 - 7 Methylengruppen, von denen eine CH₂-Gruppe durch Sauerstoff, -S-, -NH-, -NCH₃ oder -N-Benzyl ersetzt sein kann;
der jeweils andere Rest R(8)
Wasserstoff, -SR(125), -OR(125), -NR(125)R(126) oder
-CR(125)R(126)R(127);
R(125)
Wasserstoff, Alkyl mit 1, 2, 3, 4, 5, 6, 7 oder 8 C-Atomen oder Phenyl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(125)
-(C₁-C₉)-Heteroaryl,
das unsubstituiert oder substituiert ist mit 1 - 3 Substituenten ausgewählt aus der Gruppe bestehend aus der Gruppe F, Cl, CF₃, CH₃, Methoxy, Hydroxy, Amino, Methylamino und Dimethylamino;
R(126) und R(127)
unabhängig voneinander wie R(125) definiert oder Wasserstoff oder Alkyl mit 1, 2, 3, 4, 5, 6, 7 oder 8 C-Atomen;
A abwesend, -NR(11)-CO-, -NR(12)-CO-NR(13)-, -NR(17)-CO-NR(18)-SO₂-, - NR(19)-SO₂-, -SO₂-NR(19)-SO₂-, -SO₂-NR(19)-CO-, -O-CO-NR(19)-SO₂- oder -CR(20)=CR(21)-;
R(11), R(12), R(13), R(17), R(18), R(19), R(20) und R(21)
unabhängig voneinander Wasserstoff oder Alkyl mit 1, 2, 3, 4, 5, 6, 7 oder 8 C-Atomen
sowie deren pharmazeutisch verträgliche Salze;
at) Substituierte Thiophenylalkenylcarbonsäureguanidide der Formel I 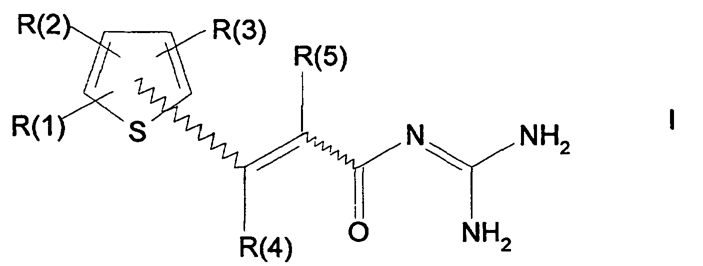 worin bedeuten:
mindestens einer der Substituenten R(1), R(2) und R(3)
-Oₚ(CH₂)ₛ-C_{q}F_{2q+1}, R(40)CO- oder R(31)SOₖ-;
p Null oder 1;
s Null, 1, 2, 3 oder 4;
q 1, 2, 3, 4, 5, 6, 7 oder 8;
k Null, 1 oder 2; R(40) Alkyl mit 1, 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen, oder Phenyl,
das nicht substituiert oder substituiert ist mit 1 - 3 Substituenten ausgewählt aus der Gruppe bestehend aus F, Cl, CF₃, Methyl und Methoxy;
R(31) Alkyl mit 1, 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen, oder Phenyl,
das nicht substituiert oder substituiert ist mit 1 - 3 Substituenten ausgewählt aus der Gruppe bestehend aus F, Cl, CF₃, Methyl oder Methoxy;
oder
R(31) NR(41)R(42);
R(41) und R(42) unabhängig voneinander Wasserstoff, Alkyl mit 1, 2, 3 oder 4 C-Atomen, Perfluoralkyl mit 1, 2, 3 oder 4 C-Atomen, oder
R(41) und R(42)
gemeinsam 4 oder 5 Methylengruppen, von denen eine CH₂-Gruppe durch Sauerstoff, S, NH, N-CH₃ oder N-Benzyl ersetzt sein kann;
und die jeweils anderen Substituenten R(1), R(2) und R(3)
unabhängig voneinander H, F, Cl, Br, I, CN, -Oₙₐ-CₘₐH₂ₘₐ₊₁ oder -
O_{ga}CᵣₐH₂ᵣₐR(10);
na Null oder 1;
ma Null, 1, 2, 3, 4, 5, 6, 7 oder 8;
ga Null oder 1;
ra Null, 1, 2, 3 oder 4;
R(10) Cycloalkyl mit 3, 4, 5, 6, 7 oder 8 C-Atomen oder Phenyl,
wobei das Phenyl nicht substituiert oder substituiert ist mit 1 - 3 Substituenten ausgewählt aus der Gruppe bestehend aus F, Cl, CF₃, Methyl und Methoxy;
R(4) und R(5)
unabhängig voneinander Wasserstoff, F, Cl, Br, I, CN, Alkyl mit 1, 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen oder Phenyl,
das nicht substituiert oder substituiert ist mit 1 - 3 Substituenten ausgewählt aus der Gruppe bestehend aus F, Cl, CF₃, Methyl, Methoxy und NR(14)R(15);
R(14) und R(15)
unabhängig voneinander H, Alkyl mit 1, 2, 3 oder 4 C-Atomen oder Perfluoralkyl mit 1, 2, 3 oder 4 C-Atomen;
sowie deren pharmazeutisch verträgliche Salze;
au) Ortho-substituierte Benzoylguanidine der Formel I worin bedeuten: 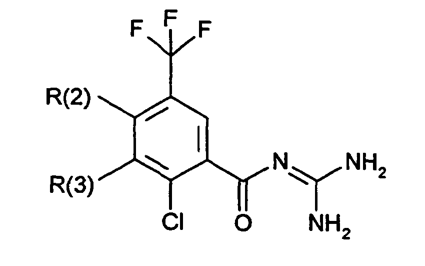
R(2) und R(3)
unabhängig voneinander Wasserstoff, Cl, Br, I, (C₁-C₈)-Alkyl, (C₃-C₈)-Cycloalkyl oder -OR(5);
R(5) (C₁-C₈)-Alkyl oder -C_{d}H_{2d}-(C₃-C₈)-Cycloalkyl;
d Null, 1 oder 2;
wobei stets einer der beiden Substituenten R(2) und R(3) Wasserstoff ist, jedoch nicht beide Substituenten R(2) und R(3) gleichzeitig Wasserstoff sind, sowie deren pharmazeutisch verträgliche Salze;
av) Benzoylguanidine der Formel I 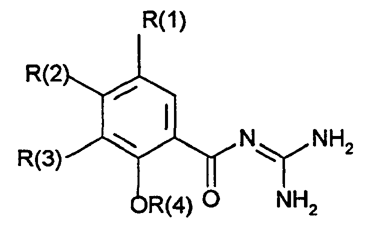 worin bedeuten:
R(1) H, F, Cl, Br, I, CN, NO₂, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder Xₐ-(CH₂)_{b}-(CF₂)_{c}-CF₃;
X Sauerstoff, S, NR(5),
a Null oder 1;
b Null, 1 oder 2;
c Null, 1, 2 oder 3;
R (5) H, Alkyl mit 1, 2, 3 oder 4 C-Atomen oder -C_{d}H_{2d}R(6);
d Null, 1, 2, 3 oder 4;
R(6) Cycloalkyl mit 3, 4, 5, 6, 7 oder 8 C-Atomen, Phenyl, Biphenylyl oder Naphthyl,
wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(7)R(8);
R(7) und R(8)
unabhängig H oder Alkyl mit 1, 2, 3 oder 4 C-Atomen;
oder
R(1) -SR(10), -OR(10) oder -CR(10)R(11)R(12);
R(10) -C_{f}H₂f-Cycloalkyl mit 3, 4, 5, 6, 7 oder 8 C-Atomen im Cycloalkylring,
oder Phenyl,
wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
f Null, 1 oder 2;
R(11) und R(12)
unabhängig voneinander wie R(10) definiert oder Wasserstoff oder
Alkyl mit 1, 2, 3 oder 4 C-Atomen;
oder
R(1) Phenyl, Naphthyl, Biphenylyl oder Heteroaryl mit 1, 2, 3, 4, 5, 6, 7, 8 oder 9 C-Atomen, letzteres über ein C- oder ein N-Atom des Rings verknüpft,
die jeweils unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
oder
R(1) -SR(13), -OR(13), -NHR(13), -NR(13)R(14), -CHR(13)R(15), -C[R(15)R(16)OH], -C≡CR(18), -C[R(19)]=CHR(18), -C[R(20)R(21)]ₖ-(CO)-[CR(22)R(23)]ₗ-R(24),
k Null, 1, 2, 3 oder 4;
l Null, 1, 2, 3 oder 4;
R(13) und R(14)
gleich oder verschieden -(CH₂)_{g}-(CHOH)ₕ-(CH₂)ᵢ-(CHOH)ⱼ-R(17) oder -(CH₂)_{g}-O-(CH₂-CH₂O)ₕ-R(24); R(17) Wasserstoff oder Methyl,
g, h und i
gleich oder verschieden Null, 1, 2, 3 oder 4;
j 1, 2, 3 oder 4;
R(15) und R(16)
gleich oder verschieden Wasserstoff, Alkyl mit 1, 2, 3, 4, 5 oder 6 C-Atomen, oder zusammen mit dem sie tragenden Kohlenstoffatom Cycloalkyl mit 3, 4, 5, 6, 7 oder 8 C-Atomen;
R(18)
Phenyl,
das unsubstituiert oder substituiert ist mit 1 - 3 Substituenten ausgewählt aus der Gruppe bestehend aus F, Cl, CF₃, Methyl, Methoxy und NR(25)R(26);
R(25) und R(26)
H oder Alkyl mit 1, 2, 3 oder 4 C-Atomen;
oder
R(18) Heteroaryl mit 1, 2, 3, 4, 5, 6, 7, 8 oder 9 Kohlenstoffatomen, das unsubstituiert oder wie Phenyl substituiert ist;
oder
R(18) Alkyl mit 1, 2, 3, 4, 5 oder 6 C-Atomen,
das unsubstituiert oder mit 1 - 3 OH substituiert ist;
oder
R (18)
Cycloalkyl mit 3, 4, 5, 6, 7 oder 8 C-Atomen;
R(19), R(20), R(21), R(22) und R(23)
gleich oder verschieden Wasserstoff oder Methyl;
R(24) H, Alkyl mit 1, 2, 3, 4, 5 oder 6 C-Atomen, Cycloalkyl mit 3, 4, 5, 6, 7 oder 8 C-Atomen oder -CₘH₂mR-(18); ?? sehr breit mit allen R(18) m 1, 2, 3 oder 4;
R(2) und R(3)
wie R(1) definiert;
R(4) Alkyl mit 1, 2, 3 oder 4 C-Atomen;
sowie deren pharmazeutisch verträgliche Salze;
aw) Ortho-substituierte Benzoylguanidine der Formel I 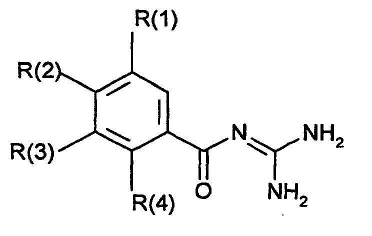 worin bedeuten:
R(1) H, F, Cl, Br, I, CN, NO₂, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder Xₐ-(CH₂)_{b}-(CF₂)_{c}-CF₃;
X Sauerstoff, S, NR(5),
a Null oder 1;
b Null, 1 oder 2;
c Null, 1, 2 oder 3;
R (5) H, Alkyl mit 1, 2, 3 oder 4 C-Atomen oder -C_{d}H_{2d}R(6);
d Null, 1, 2, 3 oder 4;
R(6) Cycloalkyl mit 3, 4, 5, 6, 7 oder 8 C-Atomen, Phenyl, Biphenylyl oder Naphthyl,
wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(7)R(8);
R(7) und R(8)
unabhängig H oder Alkyl mit 1, 2, 3 oder 4 C-Atomen;
oder
R(1) -SR(10), -OR(10) oder -CR(10)R(11)R(12);
R(10) -C_{f}H_{2f}-Cycloalkyl mit 3, 4, 5, 6, 7 oder 8 C-Atomen im Cycloalkylring, oder Phenyl,
wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
f Null, 1 oder 2;
R(11) und R(12)
unabhängig voneinander wie R(10) definiert oder Wasserstoff oder Alkyl mit 1, 2, 3 oder 4 C-Atomen;
oder
R(1) Phenyl, Naphthyl, Biphenylyl oder Heteroaryl mit 1, 2, 3, 4, 5, 6, 7, 8 oder 9 C-Atomen, letzteres über ein C- oder ein N-Atom des Rings verknüpft,
die jeweils unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
oder
R(1) -SR(13), -OR(13), -NHR(13), -NR(13)R(14), -CHR(13)R(15), -C[R(15)R(16)OH], -C≡CR(18), -C[R(19)]=CHR(18), -C[R(20)R(21)]ₖ-(CO)-[CR(22)R(23)]ₗ-R(24),
k Null, 1, 2, 3 oder 4;
l Null, 1, 2, 3 oder 4;
R(13) und R(14)
gleich oder verschieden -(CH₂)_{g}-(CHOH)ₕ-(CH₂)ᵢ-(CHOH)ⱼ-R(17) oder -(CH₂)_{g}-O-(CH₂-CH₂O)ₕ-R(24); R(17) Wasserstoff oder Methyl,
g, h und i
gleich oder verschieden Null, 1, 2, 3 oder 4;
j 1, 2, 3 oder 4;
R(15) und R(16)
gleich oder verschieden Wasserstoff, Alkyl mit 1, 2, 3, 4, 5 oder 6 C-Atomen, oder zusammen mit dem sie tragenden Kohlenstoffatom Cycloalkyl mit 3, 4, 5, 6, 7 oder 8 C-Atomen;
R(18)
Phenyl,
das unsubstituiert oder substituiert ist mit 1 - 3 Substituenten ausgewählt aus der Gruppe bestehend aus F, Cl, CF₃, Methyl, Methoxy und NR(25)R(26);
R(25) und R(26)
H oder Alkyl mit 1, 2, 3 oder 4 C-Atomen;
oder
R(18) Heteroaryl mit 1, 2, 3, 4, 5, 6, 7, 8 oder 9 Kohlenstoffatomen,
das unsubstituiert oder wie Phenyl substituiert ist;
oder
R(18) Alkyl mit 1, 2, 3, 4, 5 oder 6 C-Atomen,
das unsubstituiert oder mit 1 - 3 OH substituiert ist;
oder
R (18)
Cycloalkyl mit 3, 4, 5, 6, 7 oder 8 C-Atomen;
R(19), R(20), R(21), R(22) und R(23)
gleich oder verschieden Wasserstoff oder Methyl;
R(24) H, Alkyl mit 1, 2, 3, 4, 5 oder 6 C-Atomen, Cycloalkyl mit 3, 4, 5, 6, 7
oder 8 C-Atomen oder -CₘH₂ₘ-R(18);
m 1, 2, 3 oder 4;
einer der beiden Substituenten R(2) und R(3)
Hydroxyl;
und
der jeweils andere der Substituenten R(2) und R(3)
definiert ist wie R(1);
R(4) Alkyl mit 1, 2, 3 oder 4 C-Atomen; Alkoxy mit 1, 2, 3 oder 4 C-Atomen, F, Cl,
Br, I oder -(CH₂)ₙ-(CF₂)ₒ-CF₃;
n Null oder 1;
o Null oder 1;
sowie deren pharmazeutisch verträgliche Salze.
ax) Bis-ortho-substituierte Benzoylguanidine der Formel I 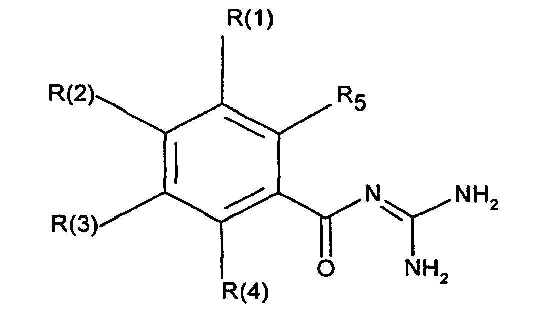 worin bedeuten:
R(1), R(2) und R(3)
unabhängig voneinander R(10)-SOₐ- oder R(14)R(15)N-SO₂-;
a Null, 1 oder 2,
R(10), R(14) und R(15)
unabhängig voneinander Alkyl mit 1, 2, 3, 4, 5, 6, 7 oder 8 C-Atomen, Perfluoralkyl mit 1, 2, 3, 4, 5, 6, 7 oder 8 C-Atomen, Alkenyl mit 3, 4, 5 oder 6 C-Atomen oder -C_{ab}H_{2ab}-R(16);
ab Null, 1, 2, 3 oder 4;
R(16) Cycloalkyl mit 3, 4, 5, 6 oder 7 C-Atomen oder Phenyl,
welches nicht substituiert ist oder substituiert mit 1 - 3 Substituenten ausgewählt aus der Gruppe bestehend aus F, Cl, CF₃, Methyl, Methoxy und NR(17)R(18);
R(17) und R(18)
unabhängig voneinander Wasserstoff, CF₃ oder Alkyl mit 1, 2, 3 oder 4 C-Atomen;
oder
R(14) und R(15)
gemeinsam 4 oder 5 Methylengruppen, von denen eine CH₂-Gruppe durch Sauerstoff, Schwefel, NH, N-CH₃ oder N-Benzyl ersetzt sein kann;
oder
R(14) und R(15)
Wasserstoff;
oder
R(1), R(2) und R(3)
unabhängig voneinander SR(21), -OR(22), -NR(23)R(24) oder -CR(25)R(26)R(27);
R(21), R(22), R(23) und R(25)
unabhängig voneinander -C_{b}H_{2b}-(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
b Null, 1 oder 2;
R(24), R(26) und R(27)
unabhängig voneinander Wasserstoff, Alkyl mit 1, 2, 3 oder 4 C-Atomen oder Perfluoralkyl mit 1, 2, 3 oder 4 C-Atomen;
oder
R(1), R(2) und R(3)
unabhängig voneinander Wasserstoff, F, Cl, Br, I, CN, -(Xa)_{dg}-C_{da}H_{2da+1}, - (Xb)_{dh}-(CH₂)_{db}-C_{de}F_{2de+1}, Alkenyl mit 3, 4, 5, 6, 7 oder 8 C-Atomen oder -C_{df}H_{2df}R(30);
(Xa) Sauerstoff, Schwefel oder NR(33);
R(33) Wasserstoff, Alkyl mit 1, 2, 3 oder 4 C-Atomen oder Perfluoralkyl mit 1, 2, 3 oder 4 C-Atomen;
dg Null oder 1;
(Xb) Sauerstoff, Schwefel oder NR(34);
R(34) Wasserstoff, Alkyl mit 1, 2, 3 oder 4 C-Atomen oder Perfluoralkyl mit 1, 2, 3 oder 4 C-Atomen;
dh Null oder 1;
da Null, 1, 2, 3, 4, 5, 6, 7 oder 8;
db Null, 1, 2, 3 oder 4;
de Null, 1, 2, 3, 4, 5, 6 oder 7;
df Null, 1, 2, 3 oder 4;
R(30) Cycloalkyl mit 3, 4, 5, 6, 7 oder 8 C-Atomen, Phenyl, Biphenylyl oder Naphthyl,
wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(31)R(32);
R(31) und R(32)
Wasserstoff, Alkyl mit 1, 2, 3 oder 4 C-Atomen oder Perfluoralkyl mit 1, 2, 3 oder 4 C-Atomen;
oder
R(1), R(2) und R(3)
unabhängig voneinander NR(40)R(41) oder -(Xe)-(CH₂)_{eb}R(45);
R(40) und R(41)
unabhängig voneinander Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen oder (CH₂)ₑ-R(42);
e Null, 1, 2, 3 oder 4;
R(42) Cycloalkyl mit 3, 4, 5, 6 oder 7 C-Atomen oder Phenyl, welches nicht substituiert ist oder substituiert mit 1 - 3 Substituenten ausgewählt aus der Gruppe bestehend aus F, Cl, CF₃, Methyl, Methoxy und NR(43)R(44);
R(43) und R(44)
unabhängig voneinander Wasserstoff, CF₃ oder Alkyl mit 1, 2, 3 oder 4 C-Atomen;
oder
R(40) und R(41)
gemeinsam 4 oder 5 Methylengruppen, von denen eine CH₂-Gruppe durch Sauerstoff, Schwefel, NH, N-CH₃ oder N-Benzyl ersetzt sein kann;
(Xe) Sauerstoff, Schwefel oder NR(47);
R(47) Wasserstoff, Alkyl mit 1, 2, 3 oder 4 C-Atomen oder Perfluoralkyl mit 1, 2, 3 oder 4 C-Atomen;
eb Null, 1, 2, 3 oder 4;
R(45) Cycloalkyl mit 3, 4, 5, 6 oder 7 C-Atomen oder Phenyl,
welches nicht substituiert ist oder substituiert mit 1 - 3 Substituenten ausgewählt aus der Gruppe bestehend aus F, Cl, CF₃, Methyl, Methoxy, NR(50)R(51) und -(Xfa)-(CH₂)_{ed}-(Xfb)R(46);
Xfa CH₂, Sauerstoff, Schwefel oder NR(48);
Xfb Sauerstoff, Schwefel oder NR(49); R(48), R(49), R(50) und R(51) unabhängig voneinander Wasserstoff, Alkyl mit 1, 2, 3 oder 4 C-Atomen oder Perfluoralkyl mit 1, 2, 3 oder 4 C-Atomen;
ed 1, 2, 3 oder 4;
R(46) Wasserstoff, Alkyl mit 1, 2, 3 oder 4 C-Atomen oder Perfluoralkyl mit 1, 2, 3 oder 4 C-Atomen;
oder
R(1), R(2) und R(3)
unabhängig voneinander -CHR(52)R(53);
R(52) -(CH₂)_{g}-(CHOH)ₕ-(CH)ᵢ-(CHOH)ₖ-R(54) oder -(CH₂)_{g}-O-(CH₂-CH₂O)ₕ-R(54);
R(54) Wasserstoff oder Methyl;
g, h, i
gleich oder verschieden Null, 1, 2, 3 oder 4;
k 1, 2, 3 oder 4;
R(53) Wasserstoff oder Alkyl mit 1, 2, 3 oder 4 C-Atomen;
oder
R(1), R(2) und R(3)
unabhängig voneinander -C(OH)R(55)R(56);
R(55) und R(56)
gleich oder verschieden Wasserstoff oder Alkyl mit 1, 2, 3 oder 4 C-Atomen;
oder
R(55) und R(56)
gemeinsam Cycloalkyl mit 3, 4, 5 oder 6 C-Atomen;
oder
R(55) -CH₂OH;
und
R(4) und R(5)
unabhängig voneinander Alkyl mit 1, 2, 3 oder 4 C-Atomen, Alkoxy mit 1, 2, 3 oder 4C-Atomen, OH, F, Cl, Br, I, CN, -Oₙ-(CH₂)ₒ-(CF₂)ₚ-CF₃;
n Null oder 1;
o Null, 1 oder 2;
p Null, 1 oder 2;
sowie deren pharmazeutisch verträgliche Salze.
ay) Substituierte 1-Naphthoylguanidine der Formel I 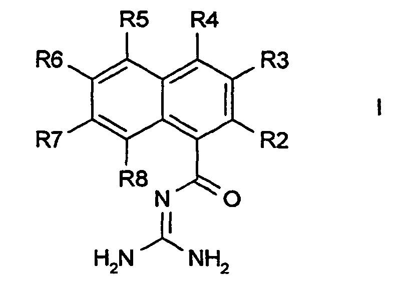 worin bedeuten
R2, R3, R4, R5, R6, R7 und R8
unabhängig voneinander H, F, Cl, Br, I, CN, NO₂, CF₃, C₂F₅ oder XₐY_{b}Z;
X O, S, NR(10), CR(11)R(12), C=O, C(=O)NR(10), C(=O)O, SO, SO₂, SO₂NR(10), OC=O, NR(10)C=O oder NR(10)SO₂,
wobei die Verknüpfung mit dem Naphthalinring jeweils über das links stehende Atom erfolgt;
R(10), R(11) und R(12)
unabhängig voneinander H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
a Null oder 1;
Y Alkylen mit 1, 2, 3, 4, 5, 6, 7 oder 8 CH₂-Gruppen,
wobei eine dieser CH₂-Gruppen durch O, S, NR(13) oder o-, p- oder m-Phenylen ersetzt sein kann;
R(13) H, Alkyl mit 1, 2, 3, 4, 5 oder 6 C-Atomen, Perfluoralkyl mit 1, 2, 3 oder 4C-Atomen oder Cycloalkyl mit 3, 4, 5 oder 6 C-Atomen;
b Null oder 1;
Z H, Alkyl mit 1, 2, 3, 4, 5, 6 oder 7 C-Atomen, Cycloalkyl mit 3, 4, 5, 6
oder 7 C-Atomen, C(=O)R(15), SO₂R(15), NR(16)R(17) oder Phenyl, das nicht substituiert oder substituiert ist mit 1 - 3 Substituenten ausgewählt aus der Gruppe bestehend aus F, Cl, Br, CF₃, Methyl, Methoxy, NR(21)R(22);
R(21) und R(22)
unabhängig voneinander H oder Alkyl mit 1, 2, 3 oder 4 C-Atomen oder Perfluoralkyl mit 1, 2, 3 oder 4 C-Atomen;
R(15) N=C(NH₂)₂, NR(18)R(19), N(CH₂)_{c}NR(18)R(19) oder OR(20);
c 2 oder 3;
R(18) und R(19)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(18) und R(19)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
R(20) H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
R(16) und R(17)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(16) und R(17)
gemeinsam 4 oder 5 Methylengruppen, von denen eine CH₂-Gruppe durch Sauerstoff, S, NH, N-CH₃, N-Benzyl oder N-(p-Chlorphenyl) ersetzt sein kann;
oder
Z ein N-haltiger Heterocyclus mit 1, 2, 3, 4, 5, 6, 7, 8 oder 9 C-Atomen, wobei der N-haltige Heterocyclus über N oder C verknüpft ist und nicht substituiert oder substituiert ist mit 1 - 3 Substituenten ausgewählt aus der Gruppe bestehend aus F, Cl, Br, CF₃, Methyl, Methoxy und NR(21)R(22); wobei jedoch für den Fall, daß R(4) für einen Alkoxyrest steht, mindestens einer der Substituenten R(2), R(3), R(5), R(6), R(7) und R(8) ungleich Wasserstoff ist;
sowie deren pharmazeutisch verträgliche Salze.
az) Substituierte 2-Naphthoylguanidine der Formel I 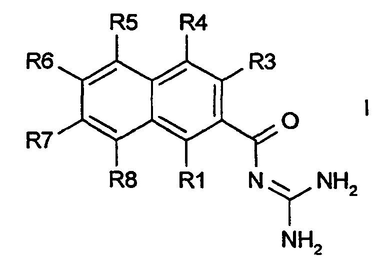 worin bedeuten:
mindestens einer der Substituenten R1, R3, R4, R5, R6, R7 und R8
XYₐ WZ oder X'YₐWZ';
X O, S, NR(10) oder CR(11)R(12);
R(10), R(11) und R(12)
unabhängig voneinander H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
Y Alkylen mit 1, 2, 3, 4, 5, 6, 7 oder 8 CH₂-Gruppen, wobei eine dieser CH₂-Gruppen durch O, S, NR(13) oder o-, p- oder m-Phenylen ersetzt sein kann;
R(13) H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;
a Null oder 1;
W CH₂, SO₂, S(=O)(=NH) oder - falls W nicht unmittelbar auf ein Heteroatom der Gruppe XYₐ folgt - auch O oder NR(14);
R(14) H, Alkyl mit 1, 2, 3, 4, 5 oder 6 C-Atomen, Perfluoralkyl mit 1, 2,
3 oder 4C-Atomen oder Cycloalkyl mit 3, 4, 5 oder 6 C-Atomen;
Z C(=O)R(15), SO₂R(15) oder - falls W nicht O oder NR(14) bedeutet - auch NR(16)R(17);
R(15)
N=C(NH₂)₂, NR(18)R(19), N(CH₂)_{b}NR(18)R(19) oder OR(20);
b 2 oder 3;
R(18) und R(19)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(18) und R(19)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
R(20)
H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
R(16) und R(17)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(16) und R(17)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
X' C=O, C(=O)NR(30), C(=O)O, SO, SO₂, SO₂NR(30), OC=O, NR(30)C=O oder NR(30)SO₂,
wobei die Verknüpfung mit dem Naphthalinring jeweils über das links stehende Atom erfolgt;
R(30) H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
Z' C(=O)R(15), SO₂R(15), einen N-haltigen Heterocyclus mit 1, 2, 3, 4, 5, 6, 7, 8 oder 9 C-Atomen,
wobei der N-haltige Heterocyclus über N oder C verknüpft ist und nicht substituiert oder substituiert ist mit 1 - 3 Substituenten ausgewählt aus der Gruppe bestehend aus F, Cl, Br, CF₃, Methyl, Methoxy und NR(21)R(22);
R(21) und R(22)
unabhängig voneinander H, Alkyl mit 1, 2, 3 oder 4 C-Atomen oder Perfluoralkyl mit 1, 2, 3 oder 4 C-Atomen;
R(15)
N=C(NH₂)₂, NR(18)R(19), N(CH₂)_{b}NR(18)R(19) oder OR(20); R(18) und R(19)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(18) und R(19)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
b 2 oder 3;
R(20) H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
oder
Z' - falls W nicht O oder NR(14) bedeutet - NR(16)R(17); R(16) und R(17)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(16) und R(17)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
und die jeweils übrigen Substituenten R1, R3, R4, R5, R6, R7 und R8, die noch nicht durch die zuvor gegebenen Definitionen vergeben sind, unabhängig voneinander H, F, Cl, Br, I, CN, NO₂, CF₃, C₂F₅ oder VₚQ_{q}U;
V O, S, SO, SO₂, NR(60), OC=O, C=O, C(=O)NR(60), C(=O)O oder CR(66)R(67);
R(60), R(66) und R(67)
unabhängig voneinander H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
p Null oder 1;
Q Alkylen mit 1, 2, 3, 4, 5, 6, 7 oder 8 CH₂-Gruppen, wobei eine dieser CH₂-Gruppen durch O, S, NR(68) oder o-, p-oder m-Phenylen ersetzt sein kann;
R(68)
H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;
q Null oder 1;
U H, Alkyl mit 1, 2, 3, 4, 5, 6 oder 7 C-Atomen, Cycloalkyl mit 3, 4, 5, 6 oder 7 C-Atomen, C(=O)R(65), SO₂R(65), NR(61)R(62) oder Phenyl, das nicht substituiert oder substituiert ist mit 1 - 3 Substituenten ausgewählt aus der Gruppe bestehend aus F, Cl, Br, CF₃, Methyl, Methoxy und NR(63)R(64); R(63) und R(64)
unabhängig voneinander H, Alkyl mit 1, 2, 3 oder 4 C-Atomen oder Perfluoralkyl mit 1, 2, 3 oder 4 C-Atomen;
R(65) N=C(NH₂)₂, NR(61)R(62) oder OR(60);
R(61) und R(62)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(61) und R(62)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann; oder
U einen N-haltigen Heterocyclus mit 1, 2, 3, 4, 5, 6, 7, 8 oder 9 C-Atomen,
wobei der N-haltige Heterocyclus über N oder C verknüpft ist und nicht substituiert oder substituiert ist mit 1 - 3 Substituenten ausgewählt aus der Gruppe bestehend aus F, Cl, Br, CF₃, Methyl, Methoxy und NR(63)R(64);
wobei jedoch mindestens einer der Substituenten R5, R6, R7 und R8 ungleich Wasserstoff ist;
sowie deren pharmazeutisch verträgliche Salze.
ba) Ortho-substituierte Benzoylguanidine der Formel I 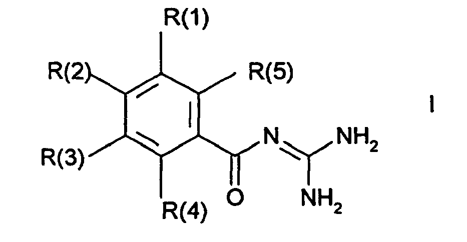 worin bedeuten:
R(1) H, F, Cl, Br, I, CN, NO₂, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder Xₐ-(CH₂)_{b}-(CF₂)_{c}-CF₃;
X Sauerstoff, Schwefel oder NR(9),
a Null oder 1;
b Null, 1 oder 2;
c Null, 1, 2 oder 3;
R(9) H, Alkyl mit 1, 2, 3 oder 4 C-Atomen oder -C_{d}H_{2d}R(6);
d Null, 1, 2, 3 oder 4;
R(6) Cycloalkyl mit 3, 4, 5, 6, 7 oder 8 C-Atomen, Phenyl, Biphenylyl oder Naphthyl,
wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(7)R(8);
R(7) und R(8) unabhängig H oder Alkyl mit 1, 2, 3 oder 4 C-Atomen;
oder
R(1) -SR(10), -OR(10) oder -CR(10)R(11)R(12);
R(10) -C_{f}H_{2f}-Cycloalkyl mit 3, 4, 5, 6, 7 oder 8 C-Atomen im Cycloalkylring, Heteroaryl mit 1, 2, 3, 4, 5, 6, 7, 8 oder 9 C-Atomen oder Phenyl,
wobei Heteroaryl und Phenyl unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
f Null, 1 oder 2;
R(11) und R(12)
unabhängig voneinander wie R(10) definiert oder Wasserstoff oder Alkyl mit 1, 2, 3 oder 4 C-Atomen;
oder
R(1) Phenyl, Naphthyl, Biphenylyl oder Heteroaryl mit 1, 2, 3, 4, 5, 6, 7, 8 oder 9 C-Atomen, letzteres über ein C- oder ein N-Atom des Rings verknüpft,
die jeweils unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
oder
R(1) -SR(13), -OR(13), -NHR(13), -NR(13)R(14), -CHR(13)R(15), -C[R(15)R(16)]OH, -C≡CR(18), -C[R(19)]=CHR(18), -C[R(20)R(21)]ₖ-(CO)-[CR(22)R(23)]ₗ-R(24),
k Null, 1, 2, 3 oder 4;
l Null, 1, 2, 3 oder 4;
R(13) und R(14)
gleich oder verschieden -(CH₂)_{g}-(CHOH)ₕ-(CH₂)ᵢ-(CHOH)ₖₖ-R(17) oder -(CH₂)_{g}-O-(CH₂-CH₂O)ₕ-R(24);
R(17) Wasserstoff oder Methyl,
g, h und i gleich oder verschieden Null, 1, 2, 3 oder 4;
kk 1, 2, 3 oder 4;
R(15) und R(16)
gleich oder verschieden Wasserstoff, Alkyl mit 1, 2, 3, 4, 5 oder 6 C-Atomen, oder zusammen mit dem sie tragenden Kohlenstoffatom Cycloalkyl mit 3, 4, 5, 6, 7 oder 8 C-Atomen;
R(18)
Phenyl,
das unsubstituiert oder substituiert ist mit 1 - 3 Substituenten ausgewählt aus der Gruppe bestehend aus F, Cl, CF₃, Methyl, Methoxy und NR(25)R(26);
R(25) und R(26)
H oder Alkyl mit 1, 2, 3 oder 4 C-Atomen;
oder
R(18) Heteroaryl mit 1, 2, 3, 4, 5, 6, 7, 8 oder 9 Kohlenstoffatomen, das unsubstituiert oder wie Phenyl substituiert ist;
oder
R(18) Alkyl mit 1, 2, 3, 4, 5 oder 6 C-Atomen,
das unsubstituiert oder mit 1 - 3 OH substituiert ist;
oder
R (18)
Cycloalkyl mit 3, 4, 5, 6, 7 oder 8 C-Atomen;
R(19), R(20), R(21), R(22) und R(23)
gleich oder verschieden Wasserstoff oder Methyl;
R(24) H, Alkyl mit 1, 2, 3, 4, 5 oder 6 C-Atomen, Cycloalkyl mit 3, 4, 5, 6, 7 oder 8 C-Atomen oder -CₘH₂ₘ-R(18);
m 1, 2, 3 oder 4;
einer der beiden Substituenten R(2) und R(3) -O-CO-R(27);
R(27) Alkyl mit 1, 2, 3, 4, 5, 6, 7 oder 8 C-Atomen, Cycloalkyl mit 3, 4, 5, 6, 7 oder 8 C-Atomen, Phenyl, Biphenylyl, Naphthyl, Pyridyl oder Chinolinyl,
wobei Phenyl, Biphenylyl, Naphthyl, Pyridyl oder Chinolinyl unsubstituiert oder substituiert sind mit 1 - 3 Substituenten ausgewählt aus der Gruppe bestehend aus F, Cl, CF₃, Methyl, Methoxy und NR(7)R(8);
R(7) und R(8)
unabhängig voneinander Wasserstoff oder Alkyl mit 1, 2, 3 oder 4 C-Atomen;
wobei stets einer der Substituenten R(2) und R(3)
definiert ist wie R(1);
R(4) und R(5) unabhängig voneinander Wasserstoff, Alkyl mit 1, 2, 3 oder 4 C-Atomen; Alkoxy mit 1, 2, 3 oder 4C-Atomen, F, Cl, Br, I, CN oder -(CH₂)ₙ-(CF₂)ₒ-CF₃;
n Null oder 1;
o Null oder 1;
sowie deren pharmazeutisch verträgliche Salze.
bb) Benzoylguanidine der Formel I 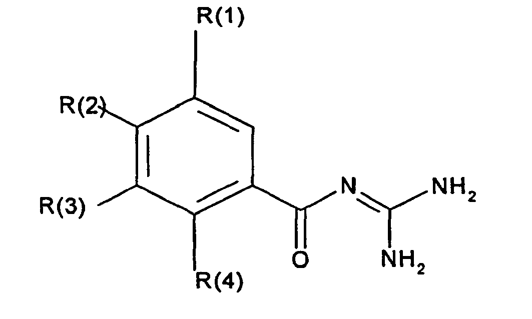 worin bedeuten:
R(1) R(13)-SOm oder R(14)R(15)N-SO₂-_{;}
m 1 oder 2;
R(13) Alkyl mit 1, 2, 3, 4, 5, 6, 7 oder 8 C-Atomen, Perfluoralkyl mit 1, 2, 3, 4, 5, 6, 7 oder 8C-Atomen, Alkenyl mit 3, 4, 5, 6, 7 oder 8 C-Atomen oder -CₙH₂ₙ-R(16),
n Null, 1, 2, 3 oder 4;
R(16) Cycloalkyl mit 3, 4, 5, 6, 7 oder 8 C-Atomen, Phenyl, Biphenylyl oder Naphthyl,
wobei Phenyl, Biphenylyl und Naphthyl nicht substituiert oder substituiert sind mit 1 - 3 Substituenten ausgewählt aus der Gruppe bestehend aus F, Cl, CF₃, Methyl, Methoxy und NR(25)R(26);
R(25) und R(26)
unabhängig voneinander Wasserstoff, Alkyl mit 1, 2, 3 oder 4 C-Atomen oder Perfluoralkyl mit 1, 2, 3 oder 4 C-Atomen;
R(14) Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen, Alkenyl mit 3, 4, 5, 6, 7 oder 8 C-Atomen oder -CₙH₂ₙ-R(27),
n Null, 1, 2, 3 oder 4;
R(27) Cycloalkyl mit 3, 4, 5, 6, 7 oder 8 C-Atomen, Phenyl, Biphenylyl oder Naphthyl,
wobei Phenyl, Biphenylyl und Naphthyl nicht substituiert oder substituiert sind mit 1 - 3 Substituenten ausgewählt aus der Gruppe bestehend aus F, Cl, CF₃, Methyl, Methoxy und NR(28)R(29);
R(28) und R(29)
unabhängig voneinander Wasserstoff, Alkyl mit 1, 2, 3 oder 4 C-Atomen oder Perfluoralkyl mit 1, 2, 3 oder 4 C-Atomen;
R(15) Wasserstoff, Alkyl mit 1, 2, 3 oder 4 C-Atomen oder Perfluoralkyl mit 1, 2, 3 oder 4 C-Atomen;
oder
R(14) und R(15) gemeinsam 4 oder 5 Methylengruppen, von denen eine CH₂-Gruppe
durch Sauerstoff, S, NH, N-CH₃ oder N-Benzyl ersetzt sein kann;
einer der Substituenten R(2) und R(3) Wasserstoff;
und der jeweils andere Substituent R(2) und R(3) -CHR(30)R(31);
R(30)
-(CH₂)_{g}-(CHOH)ₕ-(CH₂)ᵢ-(CHOH)ₖ-R(32) oder -(CH₂)_{g}-O-(CH₂-CH₂O)ₕ-R(24);
R(24 und R(32)
unabhängig voneinander Wasserstoff oder Methyl; g, h, i
gleich oder verschieden Null, 1, 2, 3 oder 4;
k 1, 2, 3 oder 4;
oder der jeweils andere Substituent R(2) und R(3)
-C(OH)R(33)R(34);
R(31), R(33) und R(34)
gleich oder verschieden Wasserstoff oder Alkyl mit 1, 2, 3 oder 4 C-Atomen
oder
R(33) und R(34)
gemeinsam Cycloalkyl mit 3, 4, 5 oder 6 C-Atomen;
oder
R(33) -CH₂OH;
R(4) Alkyl mit 1, 2, 3 oder 4 C-Atomen, Alkoxy mit 1, 2, 3 oder 4 C-Atomen, F, Cl, Br, I, CN, -(CH₂)ₙ-(CF₂)ₒ-CF₃;
n Null oder 1;
o Null, 1 oder 2;
sowie deren pharmazeutisch verträgliche Salze.
bc) Indanylidinacetylguanidine der Formel I 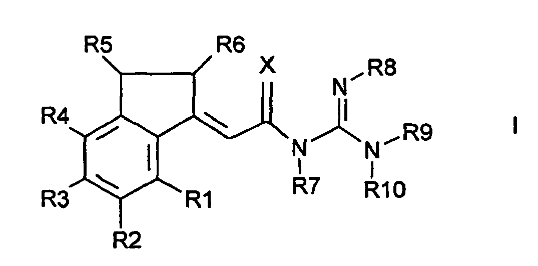 worin bedeuten:
R1, R2, R3, R4, R5 und R6
unabhängig voneinander H, C₁-C₁₀-Alkyl; Haloalkyl mit 1 - 6 Kohlenstoffatomen, O-C₁-C₁₀-Alkyl, Haloalkoxy mit 1 - 6C-Atomen, F, Cl, Br, I, Aryl, substituiertes Aryl, Heteroaryl, substituiertes Heteroaryl, OH, O-Niederalkyl, O-Aryl, O-Niederalkylaryl, O-substituiertes Aryl, O-Niederalkylsubstituiertes Aryl, O-C(=O)-C₁-C₄-Alkyl-Aryl, O-C(=O)-NH-C₁-C₄-Alkyl, O-C(=O)-N(C₁-C₄-Alkyl)₂, NO₂, CN, CF₃, NH₂, NH-C(=O)-C₁-C₄-Alkyl, NH-C(=O)-NH₂, COOH, C(=O)-O-C₁-C₄-Alkyl, C(=O)-NH₂, C(=O)-NH-C₁-C₄-Alkyl, C(=O)-N(C₁-C₄-Alkyl)₂, C₁-C₄-COOH, C₁-C₄-Alkyl-C(=O)-O-C₁-C₄-Alkyl, SO₃H, SO₂-Alkyl; SO₂-Alkylaryl, SO₂-N-(Alkyl)₂, SO₂-N(Alkyl)(Alkylaryl), C(=O)-R11, C₁-C₁₀-Alkyl-C(=O)-R11, C₂-C₁₀-Alkenyl-C(=O)-R11, C₂-C₁₀-Alkinyl-C(=O)-R11, NH-C(=O)-C₁-C₁₀-Alkyl-C(=O)-R11 oder O-C₁-C₁₁-Alkyl-C(=O)-R11;
R11C₁-C₄-Alkyl, C₁-C₄-Alkinyl, Aryl, substituiertes Aryl, NH₂, NH-C₁-C₄-Alkyl, N-(C₁-C₄-Alkyl)₂, SO₃H, SO₂-Alkyl, SO₂-Alkylaryl, SO₂-N-(Alkyl)₂ oder SO₂-N(Alkyl)(Alkylaryl);
X O, S oder NH;
R7, R8, R9 und R10
unabhängig voneinander H, Alkyl, Cycloalkyl, Aryl, Alkylaryl, oder
R8 und R9
zusammen Teil eines a 5-, 6- oder 7-gliedrigen heterocyclischen Rings; oder ihre pharmazeutisch akzeptablen Salze.
bd) Phenylsubstituierte Alkenylcarbonsäure-guanidide der Formel I 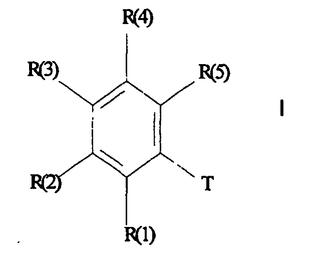 worin bedeuten:
T 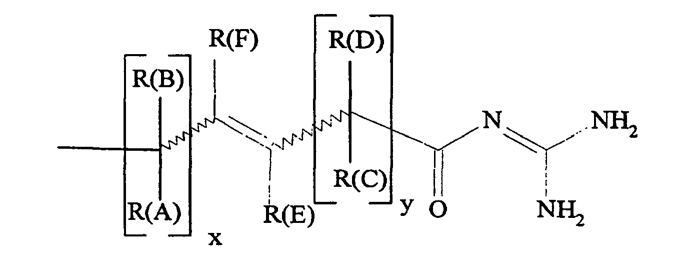 R(A) Wasserstoff, F, Cl, Br, I, CN, OH, OR(6), (C₁-C₄)-Alkyl, Oᵣ(CH₂)ₐC_{b}F_{2b+1}, (C₃-C₈)-Cycloalkyl oder NR(7)R(8)
r Null oder 1;
a Null, 1, 2, 3 oder 4;
b 1, 2, 3 oder 4;
R(6) (C₁-C₄)-Alkyl, (C₁-C₄)-Perfluoralkyl, (C₃-C₆)-Alkenyl, (C₃-C₈)-Cycloalkyl, Phenyl oder Benzyl,
wobei der Phenylkern nicht substituiert oder substituiert ist mit 1 - 3 Substituenten ausgewählt aus der Gruppe bestehend aus F, Cl, CF₃, Methyl, Methoxy und NR(9)R(10);
R(9) und R(10)
H, (C₁-C₄)-Alkyl oder (C₁-C₄)-Perfluoralkyl;
R(7) und R(8)
unabhängig voneinander wie R(6) definiert;
oder
R(7) und R(8)
gemeinsam 4 oder 5 Methylengruppen, von denen eine CH₂-Gruppe durch Sauerstoff, Schwefel, NH, N-CH₃ oder N-Benzyl ersetzt sein kann;
R(B), R(C) und R(D)
unabhängig wie R(A) definiert;
x Null, 1 oder 2;
y Null, 1 oder 2;
R(F) Wasserstoff, F, Cl, Br, I, CN, OR(12), (C₁-C₈)-Alkyl, Oₚ(CH₂)_{f}C_{g}F_{2g+1}, (C₃-C₈)-Cycloalkyl oder (C₁-C₉)-Heteroaryl,
p Null oder 1;
f Null, 1, 2, 3 oder 4;
g 1, 2, 3, 4, 5, 6, 7 oder 8;
R(12) (C₁-C₈)-Alkyl, (C₁-C₄)-Perfluoralkyl, (C₃-C₈)-Alkenyl, (C₃-C₈)-Cycloalkyl, Phenyl oder Benzyl,
wobei der Phenylkern nicht substituiert ist oder substituiert 1 - 3 Substituenten ausgewählt aus der Gruppe bestehend aus F, Cl, CF₃, Methyl, Methoxy und NR(13)R(14);
R(13) und R(14)
H, (C₁-C₄)-Alkyl oder (C₁-C₄)-Perfluoralkyl;
R(E) unabhängig wie R(F) definiert;
R(1) unabhängig wie T definiert;
oder
R(1) Wasserstoff, -OₖCₘH₂ₘ₊₁, -Oₙ(CH₂)ₚC_{q}F_{2q+1}, F, Cl, Br, I, CN, -(C=O)-N=C(NH₂)₂, -SOᵣR(17), -SOᵣ₂NR(31)R(32); -Oᵤ(CH₂)ᵥC₆H₅, -Oᵤ₂-(C₁-C₉)-Heteroaryl oder -Sᵤ₂-(C₁-C₉)-Heteroaryl;
k Null oder 1;
m Null, 1, 2, 3, 4, 5, 6, 7 oder 8;
n Null oder 1;
p Null, 1, 2, 3 oder 4;
q 1, 2, 3, 4, 5, 6, 7 oder 8;
r Null, 1, 2;
r2 Null, 1, 2;
R(31) und R(32)
unabhängig voneinander Wasserstoff, (C₁-C₈)-Alkyl oder (C₁-C₈)-Perfluoralkyl;
oder
R(31) und R(32)
gemeinsam 4 oder 5 Methylengruppen, von denen eine CH₂-Gruppe durch Sauerstoff, S, NH, N-CH₃ oder N-Benzyl ersetzt sein kann;
R(17) (C₁-C₈)-Alkyl;
u Null oder 1;
u2 Null oder 1;
v Null, 1, 2, 3 oder 4;
wobei der Phenylkern substituiert ist oder substituiert mit 1 - 3 Substituenten ausgewählt aus der Gruppe bestehend aus F, Cl, CF₃, Methyl, Methoxy, -(CH₂)_{w}NR(21)R(22), NR(18)R(19) und (C₁-C₉)-Heteroaryl;
R(18), R(19), R(21) und R(22)
unabhängig voneinander (C₁-C₄)-Alkyl oder (C₁-C₄)-Perfluoralkyl;
w 1, 2, 3 oder 4;
wobei der Heterocyclus des (C₁-C₉)-Heteroaryl unsubstituiert oder substituiert ist mit 1 - 3 Substituenten ausgewählt aus der Gruppe bestehend aus F, Cl, CF₃, Methyl oder Methoxy;
R(2), R(3), R(4) und R(5)
unabhängig voneinander wie R(1) definiert, oder
R(1) und R(2) oder R(2) und R(3)
jeweils gemeinsam -CH-CH=CH-CH-,
das nicht substituiert oder substituiert ist mit 1 - 3 Substituenten ausgewählt aus der Gruppe bestehend aus F, Cl, CF₃, Methyl, Methoxy, -(CH₂)_{w2}NR(24)R(25) und NR(26)R(27); R(24), R(25), R(26) und R(27)
H, (C₁-C₄)-Alkyl oder (C₁-C₄)-Perfluoralkyl;
w2 1, 2, 3 oder 4;
wobei der Rest T im Molekül mindestens zweimal, jedoch nur höchstens dreimal vorhanden ist;
sowie deren pharmazeutisch verträgliche Salze.
be) Benzoylguanidine der Formel I 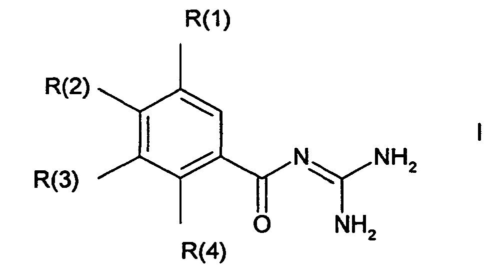 worin bedeuten:
R(1) CF₃;
einer der Substituenten R(2) und R(3)
Wasserstoff;
und der jeweils andere Substituent R(2) und R(3)
-C(OH)(CH₃)-CH₂OH, -CH(CH₃)-CH₂OH oder -C(OH)(CH₃)₂;
R(4) Methyl, Methoxy, Cl oder CF₃;
sowie deren pharmazeutisch verträgliche Salze;
II. oder Verbindungen der Formel 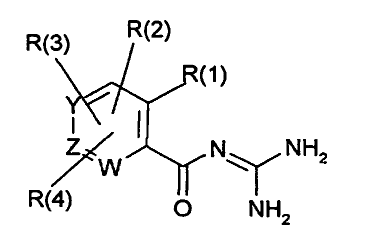 worin bedeuten:
W, Y und Z
ein Stickstoffatom oder ein mit R(2) oder R(3) oder R(4) substituiertes C-atom;
R(1) Wasserstoff, A, Hal, -CF₃, -CH₂F, -CHF₂, -CH₂CF₃, -C₂F₅, -CN, -NO₂, - Ethinyl, oder einen X-R';
A Alkyl mit 1 bis 6 C-Atomen;
Hal F, Cl, Br oder I;
X Sauerstoff, S oder NR";
R" Wasserstoff, A oder eine cyclische Methylenkette mit 3 bis 7 C-Atomen;
R' H, A, HO-A-, HOOC-A-, (C₃-C₇)-Cycloalkyl, (C₆-C₈)-Cycloalkyl-alkyl, CF₃, CH₂F, CHF₂, CH₂-CF₃, Ph, -CH₂-Ph oder Het;
Ph unsubstituiertes oder ein-, zwei-, oder dreifach durch A, OA, NR'R", Hal, CF₃ substituiertes Phenyl, Naphthyl, oder Biphenylyl;
Het einen ein- oder zweikernigen gesättigten, ungesättigten oder aromatischen Heterocyclus mit 1 bis 4 N-, O-, und/oder S-Atomen,
der unsubstituiert oder ein-, zwei- oder dreifach substitiert ist durch Hal, CF₃, A, OH, OA, -X-R', -CN, -NO₂, und/oder Carbonylsauerstoff,
wobei Het über N oder oder eine Alkylenkette CₘH₂ₘ mit m = Null bis 6 gebunden ist;
oder
R' und R" zusammen Alkylen mit 4 - 5 C-Atomen, worin eine CH₂-Gruppe auch durch Sauerstoff, S, NH, N-A, N-Ph und N-CH₂-Ph ersetzt sein kann;
R(2) und R(3)
unabhängig voneinander Wasserstoff, Hal, A, HO-A-, X-R', -C(=N-OH)-A, A-O-CO-(C₁-C₄)-Alkyl-, CN, NO₂, COOH, halogensubstituiertes A, insbesondere CF₃, CH₂F, CHF₂, C₂F₅, CH₂CF₃, oder S(O)ₙR"';
R'" A, Ph oder -Het;
n Null, 1 oder 2;
oder
R(2) und R(3)
unabhängig voneinander SO₂NR'R", Ph oder -O-Ph, -O-CH₂-Ph, -CO-A, -CHO, -COOA, -CSNR'R", CONR'R", -CH=CH-COOH, -CH=CH-COOA, Indenyl, Indanyl, Decahydronaphthyl, Cyclopentenyl, Dihydrothienyl, Dihydrofuryl, Heterobicyclyl, Alkylthienyl, Halothienyl, Haloalkylthienyl, Acylthienyl, Halofuryl, Haloalkylfuryl oder Pyrrolyl;
oder
R(2) und R(3)
unabhängig voneinander R(5)-O-;
R(5) Wasserstoff, A, (C₁-C₆)-Alkenyl oder (C₃-C₇)-Cycloalkyl;
R(4) Ph, Het, -O-Het; CF₃, S(O)ₙR"', -SO₂NR'R", Alk; 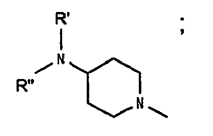 oder
zwei der Substituenten R(1) bis R(4)
miteinander eine Gruppe -O-CR(6)R(7)-CO-NR(8)-, oder 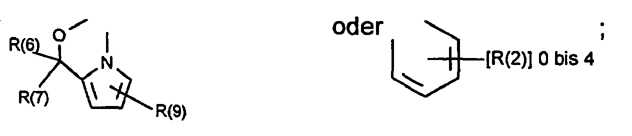 mit R(2) in der angegebenen Bedeutung;
R(6), R(7), R(8) und R(9) unabhängig voneinander H oder A;
oder
R(8) (C₅-C₇)-Cycloalkyl;
oder
R(9) Cyano;
Alk geradkettiges oder verzweigtes (C₁-C₈)-Alkyl oder (C₃-C₈)-Cycloalkyl, welches unsubstituiert oder durch A ein-, zwei- oder dreifach substituiert ist;
oder
Alk ein durch H, A, Ph oder Het substituierter Ethenyl oder Ethinylrest; oder
III. Verbindungen der Formel 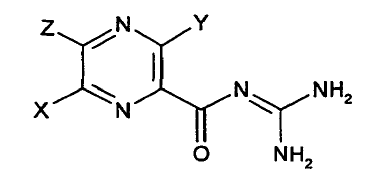 worin bedeuten:
X H, Hal, (Hal)₃C-, (C₁-C₆)-Alkyl, (C₃-C₆)-Cycloalkyl, substituiertes Phenyl, (C₁-C₅)-Alkyl-S- oder (C₁-C₅)-Alkyl-SO₂-;
Y NH₂ oder substituiertes Amino;
oder
X und Z
zusammen eine -(CH₂)₄- oder eine 1,3-Butadienylen-Kette;
oder
Z H, Hal, OH, HS, (C₁-C₅)-Alkyl, (C₃-C₆)-Cycloalkyl, substituiertes Phenyl; oder
Z eine Aminogruppe -NR(1)R(2);
R(1) H, gerad- und verzweigtkettiges, ggf. substituiertes (C₁-C₈)-Alkyl, das unterbrochen sein kann durch Sauerstoff;
oder
R(1) (C₃-C₈)-Alkenyl, (C₃-C₈)-Alkinyl, (C₃-C₇)-Cycloalkyl oder OH-substituiertes Phenyl oder OH-substituiertes Phenyl-(C₁-C₄)-alkyl oder OH-substituiertes (C₃-C₇)-Cycloalkyl;
R(2) 1-Morpholino, Wasserstoff oder eine gerade oder verzweigte (C₁-C₈)-Alkylkette,
die unterbrochen sein kann durch Sauerstoff, eine Aminogruppe,
welche gerade oder verzweigte (C₁-C₈)-Alkylkette unsubstituiert oder substituiert ist durch
einen substituierten oder unsubstituierten ein- oder mehrkernigen Heterocyclus, der Stickstoff, Sauerstoff oder Schwefeleatomen enthält;
oder
welche Alkylkette substituiert ist durch Phenyl,
unsubstituiert oder ein- oder mehrfach substituiert mit (C₁-C₄) Alkoxy, gegebenenfalls substituiert durch OH, Alkylamino, Alkyl oder Phenyl;
oder
durch eine Aminocarbonylgruppe
oder
durch Hydroxy-, (C₁-C₄)-Alkoxygruppen,
oder
R(2) Phenyl,
unsubstituiert oder substituiert durch Alkyl, Alkoxy, eine Aminogruppe, die als Substituenten trägt:
H, einen ein- oder mehrkernigen Heterocyclus, der Stickstoff, Sauerstoff oder Schwefelatome enthält,
der unsubstituiert oder substituiert ist mit H, Hal oder (C₁-C₄)-Alkyl;
einen Phenylrest, unsubstituiert oder substituiert durch einen Substituenten, ausgewählt aus der Gruppe bestehend aus (C₁-C₄)-Alkyl, (C₁-C₄)-Alkoxy, Hal und OH;
oder
R(2) 1-Piperidino,
unsubstituiert oder substituiert in 4-Stellung durch einen Acylrest einer aliphatischen, alicyclischen, aromatischen oder heteroaromatischen Carbonsäure, (C₁-C₈) Alkyl,
das seinerseits substituiert sein kann durch OH oder (C1-C4)-Alkoxy oder einen (C₁-C₄)-alkoxysubstituierten Phenylrest;
oder
R(2) Amidino,
das unsubstituiert oder substituiert ist mit Phenyl,
das unsubstituiert oder substituiert ist mit Hal oder Alkyl;
oder
R(2) ein Acylrest einer aliphatischen, alicyclischen, aromatischen oder heteroaromatischen Carbonsäure,
oder
R(2) eine (C₁-C₈) Alkylkette, die substituiert sein kann durch einen OH, Alkoxy oder Alkylreste tragenden Phenylrest.
oder
R(1) und R(2) gemeinsam mit dem N-Atom, an das sie gebunden sind, einen Piperazinring,
der unsubstituiert ist oder über eine (C₁-C₆)-Methylenkette einen ein- oder mehrkernigen Heterocyclus trägt,
der Stickstoff, Sauerstoff oder Schwefele enthält.
Hal F, Cl, Br oder I; oder
IV. Indoloylguanidin-Derivate der Formel 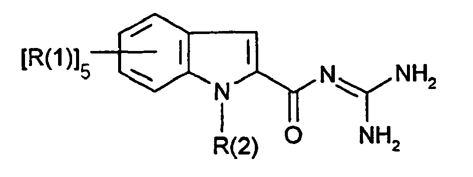 worin bedeuten
R(2) Wasserstoff, unsubstituiertes oder substituiertes (C₁-C₈)-Alkyl, (C₃-C₇)-Cycloalkyl, OH, (C₁-C₆)-Alkyl-O-, einen aromatischen Rest oder eine Gruppe - CH₂-R(20);
R(20) (C₂-C₆)-Alkenyl oder (C₂-C₆)-Alkinyl;
R(1) 1 bis 5 gleiche oder verschiedene Substituenten, die bedeuten:
Wasserstoff, unsubstituiertes oder substituiertes (C₁-C₈)-Alkyl, (C₂-C₆)-Alkenyl, (C₂-C₆)-Alkinyl, (C₃-C₇)-Cycloalkyl, Halogen, -NO₂, (C₂-C₈)-Alkanoyl, Arylalkanoyl mit bis zu 10 C-Atomen, Aroyl mit bis zu 11 C-Atomen, -COOH, (C₂-C₆)-Alkoxycarbonyl, eine aromatische Gruppe, eine der nachfolgend aufgeführten Gruppen: -OR(3), -NR(6)R(7) oder -S(O)ₙR(40);
R(3) Wasserstoff, (C₁-C₈)-Alkyl, substituiertes (C₁-C₈)-Alkyl, (C₃-C₇)-Cycloalkyl, einen aromatischen Rest oder eine Gruppe -CH₂-R(30) R(30) Alkenyl oder Alkinyl;
R(6) und R(7)
unabhängig voneinander Wasserstoff, unsubstituiertes oder substituiertes (C₁-C₈)-Alkyl, (C₃-C₇)-Cycloalkyl, (C₂-C₈)-Alkanoyl, eine Arylalkanoylgruppe mit bis zu 10 C-Atomen, eine Aroylgruppe mit bis zu 11 C-Atomen, eine aromatische Gruppe oder -CH₂-R(60); R(60) (C₂-C₆)-Alkenyl oder (C₂-C₆)-Alkinyl;
oder
R(6) und R(7)
gemeinsam mit dem N-Atom ein 5 - 7-gliedriges cyclisches Amin, das noch weitere Heteroatome im Ring enthalten kann;
n Null, 1 oder 2;
R(40) unsubstituiertes oder substituiertes (C₁-C₈)-Alkyl, oder eine aromatische Gruppe, oder eine Gruppe 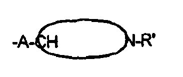 A Sauerstoff, -S(O)ₙ- oder -N(R50)-;
R(50) Wasserstoff oder (C₁-C₈)-Alkyl;
R' Wasserstoff, unsubstituiertes oder substituiertes (C₁-C₈)-Alkyl, worin der Ring einen gesättigten 3 - 8 gliedrigen Heterocyclus mit einem N-atom repräsentiert,
besagtes substituiertes Alkyl eine oder mehrere Gruppen trägt ausgewählt aus der Gruppe bestehend aus Halogen, -OH, (C₁-C₆)-Alkoxy, -CN, -COOH, (C₂-C₆)-Alkoxycarbonyl, (C₂-C₈)-Alkanoyl, Arylalkanoyl mit bis zu 10 C-Atomen, Aroyl mit bis zu 11 C-Atomen, eine aromatische Gruppe, - CONR(4)(R5),
R(4) und R(5)
gleich oder verschieden Wasserstoff oder (C₁-C₈)-Alkyl;
oder
R(4) und R(5)
miteinander verbunden und bilden zusammen ein 5 - 7-gliedriges cyclisches Amin, das noch weitere Heteroatome im Ring enthalten kann,
oder besagtes substituiertes Alkyl eine Gruppe 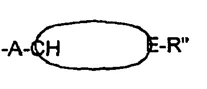 trägt, worin bedeuten:
E ein N-Atom oder eine CH-Gruppe;
R" Wasserstoff, unsubstituiertes oder mit OH substituiertes (C₁-C₈)-Alkyl oder substituiertes (C₁-C₈)-Alkyl, (C₁-C₆)-Alkoxy, -CN, -COOH, (C₂-C₆)-Alkoxycarbonyl, (C₂-C₈)-Alkanoyl, Aralkanoyl mit bis zu 10 C-Atomen, Aroyl mit bis zu 11 C-Atomen, eine aromatische Gruppe, -NR(6)R(7), -CONR(4)R(5);
R(4) und R(5)
unabhängig voneinander Wasserstoff oder (C₁-C₈)-Alkyl; wobei das cyclische System der Formel 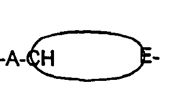 ein 3 - 8 gliedriges gesättigtes aliphatisches oder heterocyclisches Ringsystem mit einem N-Atom bedeutet,
und wobei die erwähnten aromatischen Gruppen einen Arylrest mit bis zu 10C-Atomen, einen 5- oder 6-gliedrigen Heteroarylrest mit 1-4 N-Atomen, eine 5- oder 6-gliedrige Heteroarylgruppe enthaltend 1 oder 2 N-Atome und ein Heteroatom in der Bedeutung von Sauerstoff oder Schwefel, oder Furyl bedeuten,
und wobei die erwähnten Arylreste unsubstituiert oder substituiert sein können durch unsubstituiertes (C₁-C₈)-Alkyl oder substituiertes (C₁-C₈)-Alkyl, Halogen, -NO₂, (C₂-C₆)-Alkoxycarbonyl, COOH, -OR(3), NR(6)R(7), -CONR(4)R(5), -SO₂NR(6)R(7) oder S(O)ₙR(40),
wobei R(1) und der Guanidinocarbonylrest an einer beliebigen Stelle des 5- oder 6-gliedrigen Rings des Indolsystems stehen können,
sowie die zugehörigen pharmazeutisch verträglichen Salze.
oder
V. Heterocyclische Guanidin-Derivate der Formel 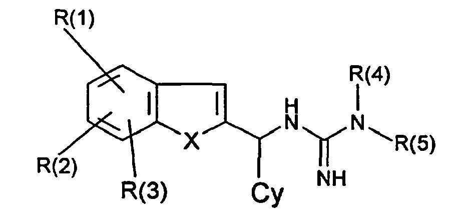 worin bedeuten:
X -O-, -S-, -NH-, -N[(C₁-C₄)-Alkyl]- oder -N(Phenyl)-;
R(1), R(2) und R(3)
Wasserstoff, Halogen, (C₁-C₄)-Alkyl, (C₁-C₄)-Alkyl-O-, Phenyl, Benzyl; oder
zwei der Substituenten R(1), R(2) und R(3) zusammen mit einer Seite des Benzosystems einen 4 - 6-gliedrigen carbocyclischen Ring;
R(4) und R(5)
unabhängig voneinander Wasserstoff, (C₁-C₁₂)-Alkyl, Benzhydryl, Aralkyl, das unsubstituiert oder substituiert ist durch einen oder mehrere Substituenten aus den Gruppen Halogen, (C₁-C₄)-Alkyl, (C₁-C₄)-Alkyl-O- oder -CF₃, -(CH₂)ₘ-CH₂-T,
m Null bis 3;
T -CO-O-T(1);
T(1) Wasserstoff oder (C1-C4)-Alkyl;
Cy einen benzokondensierten ungesättigten oder Dihydro-5-Ringheterocyclus 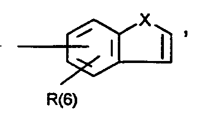 einen Pyrazol- oder Imidazolring der Formel 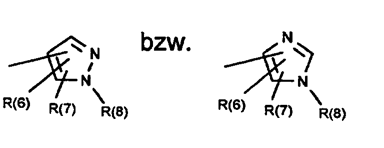 einen Naphthylrest oder ein Dihydro- oder Tetrahydronaphthylrest 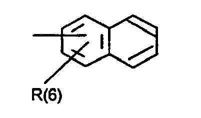 einen 2-, 3- oder 4-Pyridylrest 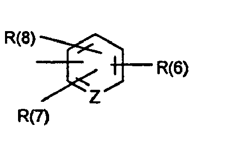 Z N- oder CH;
einen Thienylrest 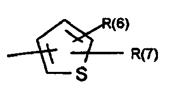 R(6) Wasserstoff, Halogen, Hydroxy, (C₁-C₁₀)-Alkyl, (C₁-C₁₀)-Alkyl-O-, Phenoxy, (C₁-C₁₀)-Alkyloxymethyloxy- oder -(O)ₙS-R(9); R(9) (C₁-C₁₀)-Alkyl, Thienyl, Pyridyl-, Thiazolyl, Thiadiazolyl,
Imidazolyl, Pyrazolyl oder Phenyl, die unsubstituiert oder ein- oder zweifach substituiert sind mit Halogen, (C₁-C₄)-Alkyl oder (C₁-C₄)-Alkyl-O-;
R(7) und R(8)
Wasserstoff, Halogen, Hydroxy, (C₁-C₁₀)-Alkyl, (C₁-C₁₀)-Alkyl-O-, Phenyl, Phenoxy oder (C₁-C₁₀)-Alkoxymethyloxy;
oder
Cy Phenyl,
das unsubstituiert ist oder ein- oder zweifach substituiert ist mit Halogen, (C₁-C₄)-Alkyl oder (C₁-C₄)-Alkyl-O-;
oder
Cy -Gr-Am;
Gr -R(13)-R(12)-(CH₂)_{q}-C[W][W(1)]-(CH₂)_{q}-; R(13)R(14)- oder -R(15)-; R(12) eine Einfachbindung, -O-, -(O)ₙS-, -CO- oder -CONH-; R(13) eine Einfachbindung, Phenyl, Thienyl, Pyridyl, Thiazolyl, Thiadiazolyl, Imidazolyl oder Pyrazolyl;
R(14) eine Einfachbindung SO₂-;
R(15) (C₂ - C₁₀)-Alkenyl- oder (C₂-C₁₀)-Alkinyl;
W und W(1)
unabhängig voneinander Wasserstoff, (C₁-C₄)-Alkyl;
oder
W und W(1)
miteinander cyclisch verbunden einen (C₃-C₈)-Kohlenwasserstoffring;
q und q'
Null bis 9;
Am -NR(10)R(11);
R(10) Wasserstoff, (C₁-C₄)-Alkyl oder Benzyl,
R(11) (C₁-C₄)-Alkyl, Phenyl oder Benzyl;
oder
R(10) und R(11)
gemeinsam eine (C₃-C₁₀)-Alkylengruppe, die unsubstituiert ist oder substituiert mit -COOH, (C₁-C₅)-Alkoxycarbonyl, (C₂-C₄)-Hydroxyalkylen oder Benzyl;
oder
Am Pyrrolyl, Pyridyl, Pyrazolyl, Morpholinyl, Dihydropyridyl, Tetrahydropyridyl, Chinuclidinyl, Imidazolyl, 3-Azabicyclo[3.2.1]octyl, das unsubstituiert ist oder substituiert mit (C₁-C₄)-Alkyl,
oder
Am Azabicyclo[3.2.2]nonyl;
oder
Am eine Piperazingruppe der Formel 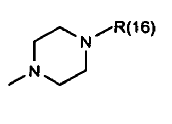 R(16) Wasserstoff, (C₁-C₄)-Alkyl, (C₃-C₆)-Cycloalkyl, Phenyl, Tolyl, Methoxyphenyl, Halophenyl, Diphenylmethylen, Benzyl oder Pyridyl;
oder
Am eine Azidogruppe -(O)t-(CH2)q-C[W][W(1)]-(CH2)q'-N3;
t Null oder 1;
wobei W und W(1) die vorher angegebene Bedeutung besitzen; sowie die optischen Enantiomeren und die pharmakologisch verträglichen Salze. oder
VI. Guanidin-Verbindungen der folgenden Formel 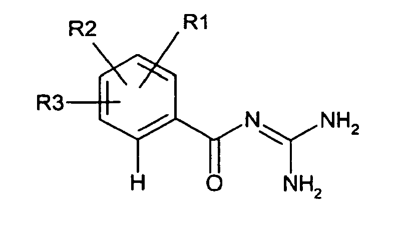 mit R1 = R2 gleich H, Halo, Alkyl, CN, NO₂, Perfluoralkyl, SOₙCF₃; R3 = CH=CH₂, CH₂-CH=CH₂, CH₂-CH₂-CH=CH₂, Cycloalkenyl, Cycloalkenylalkyl; R4= Alkyl, (substituiertes) Phenyl und das die Herz-Kreislauf-aktive Substanz ein blutdrucksenkendes Medikament zur Bluthochdruckbehandlung unter cardioprotektiven Bedingungen, ein Herzglykosid zur Behandlung der Herzinsuffizienz und des Congestive Heart failures unter cardioprotektiven Bedingungen, ein Antiarrhythmikum zur Behandlung von Herzarrhythmien verschiedener Genese unter cardioprotektiven Bedingungen, ein Herz-Kreislauf-aktives Nitrat, einen Öffner des K(ATP)-Kanals oder einen K(ATP)-Blocker ist.

2. Pharmazeutisches Kombinationspräparat nach Anspruch 1, **dadurch gekennzeichnet, daß** es einen NHE-Inhibitor und einen Betarezeptoren-Blocker zur Bluthochdruckbehandlung und Arrhythmiebehandlung unter cardioprotektiven Bedingungen enthält.

3. Pharmazeutisches Kombinationspräparat nach Anspruch 1, **dadurch gekennzeichnet, daß** es NHE-Inhibitor und einen Calciumantagonisten zur Bluthochdruckbehandlung unter cardioprotektiven Bedingungen enthält

4. Pharmazeutisches Kombinationspräparat nach Anspruch 1, **dadurch gekennzeichnet, daß** es einen NHE-Inhibitor und einen Angiotensin-Conversions-Enzym-Hemmer zur Bluthochdruckbehandlung unter cardioprotektiven Bedingungen enthält.

5. Pharmazeutisches Kombinationspräparat nach Anspruch 1, **dadurch gekennzeichnet, daß** es einen NHE-Inhibitor und ein Diuretikum oder einen Aldosteronantagonisten zur Bluthochdruck- und Herzinsuffizienzbehandlung unter cardioprotektiven Bedingungen enthält

6. Pharmazeutisches Kombinationspräparat nach Anspruch 1, **dadurch gekennzeichnet, daß** es einen NHE-Inhibitor und ein Antiarrhythmikum der Klassen I, II, III oder IV zur Behandlung von Herzarrhythmien verschiedener Genese unter cardioprotektiven Bedingungen enthält.

7. Pharmazeutisches Kombinationspräparat nach Anspruch 1, **dadurch gekennzeichnet, daß** es den NHE-Inhibitor Cariporide (Hoe 642) in Kombination mit einem Inhibitor des nicht-inaktivierenden-Natriumkanals enthält.

## Claims

1. Pharmaceutical combination preparation, **characterized in that** it comprises an inhibitor of the Na⁺/H⁺ exchanger and a substance having cardiovascular activity, the inhibitor of the Na⁺/H⁺ exchanger being selected from the following compounds:
I
a) benzoylguanidines of the formula I 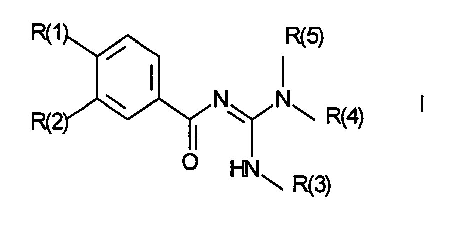 in which:
R(1) or R(2)
is R(6)-S(O)ₙ- or R(7)R(8)N-O₂S-;
and the other substituent R(1) or R(2) in each case is H, F, Cl, Br, (C₁-C₄)-alkyl, (C₁-C₄) -alkoxy or phenoxy,
which is unsubstituted or substituted by 1 - 3 substituents selected from the group consisting of fluorine, chlorine, methyl and methoxy;
or the other substituent R(1) or R(2) in each case
is R(6)-S(O)ₙ or R(7)R(8)N-;
n is zero, 1 or 2;
R(6) is (C₁-C₆)-alkyl, (C₅-C₇-cycloalkyl, cyclopentylmethyl, cyclohexylmethyl or phenyl,
which is unsubstituted or substituted by 1 - 3 substituents selected from the group consisting of fluorine, chlorine, methyl and methoxy;
R(7) and R(8)
identically or differently are H or (C₁-C₆)-alkyl;
or
R (7) is phenyl-(CH₂)_{m;}
m is 1 - 4;
or
R(7) is phenyl,
which is unsubstituted or substituted by 1 - 2 substituents selected from the group consisting of fluorine, chlorine, methyl and methoxy;
or
R(7) and R(8)
together are a straight-chain or branched (C₄-C₇)-chain,
where the chain can additionally be interrupted by O, S or NR(9);
R(9) is H or methyl;
or
R(7) and R(8)
together with the nitrogen atom to which they are bonded, are a dihydroindole, tetrahydroquinoline or tetrahydroisoquinoline system;
R(3), R(4) and R(5)
independently of one another are H or (C₁-C₂)-alkyl,
or
R(3) and R(4)
together are a (C₂-C₉)-alkylene chain; or
R(4) and R(5)
together are a (C₄-C₇)-alkylene chain; and their pharmaceutically tolerable salts;
b) benzoylguanidines of the formula I 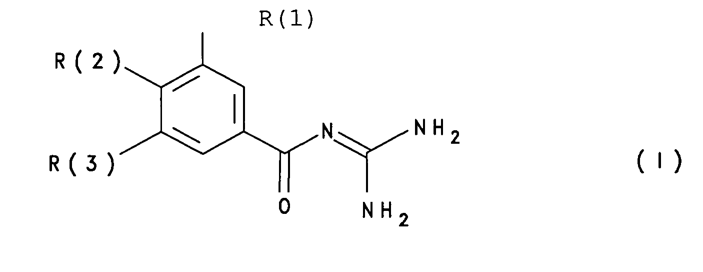 in which:
R(1) is R(4)-SOₘ or R(5)R(6)N-SO₂-;
m is zero, 1 or 2;
R(4) and R(5)
are C₁-C₈-alkyl, C₃-C₆-alkenyl or -CₙH₂ₙ-R (7) ;
n is zero, 1, 2, 3 or 4;
R(7) is C₅-C₇-cycloalkyl or phenyl,
which is unsubstituted or substituted by 1 - 3 substituents selected from the group consisting of F, Cl, CF₃, methyl, methoxy and NR(8)R(9) ;
R(8) and R(9)
are H or C₁-C₄-alkyl;
or
R(5) is H;
R(6) is H or C₁-C₄-alkyl,
or
R(5) and R(6)
together are 4 or 5 methylene groups, of which one CH₂ group can be replaced by an 0, S, NH, N-CH₃ or N-benzyl;
R(2) is hydrogen, F, Cl, Br, (C₁-C₄)-alkyl-, O-(CH₂)ₘCₚF₂ₚ₊₁ or -X-R(10) ;
m is zero or 1;
p is 1, 2 or 3;
X is 0, S or NR(11);
R(10) is H, C₁-C₆-alkyl, C₅-C₇-cycloalkyl, cyclohexylmethyl, cyclopentylmethyl or -CₙH₂ₙ-R (12) ;
n is zero, 1, 2, 3 or 4;
R(12) is phenyl, which is unsubstituted or substituted by 1 - 3 substituents selected from the group consisting of F, Cl, CF₃, methyl, methoxy und NR(8)R(9) ;
R(8) and R(9)
are H or C₁-C₄-alkyl;
R(11) is hydrogen or C₁-C₃-alkyl;
or
R(10) and R(11)
together are 4 or 5 methylene groups, of which one CH₂ group can be replaced by O, S, NH, N-CH₃ or N-benzyl;
R (3) is defined as R(1), or is C₁-C₆-alkyl, nitro, cyano, trifluoromethyl, F, Cl, Br, I or -X-R(10);
X is O, S or NR(11);
R (10) is H, C₁-C₆-alkyl, C₅-C₇-cycloalkyl, cyclohexylmethyl, cyclopentylmethyl or -CₙH₂ₙ-R (12) ;
n is zero to 4;
R(12) is phenyl,
which is unsubstituted or substituted by 1 - 3 substituents selected from the group consisting of F, Cl, CF₃, methyl, methoxy und NR(8)R(9);
R(8) and R(9)
are H or C₁-C₄-alkyl;
R (11) is C₁-C₃-alkyl,
or
R(10) and R(11)
together are 4 or 5 methylene groups, of which one CH₂ group can be replaced by O, S, NH, N-CH₃ or N-benzyl;
and their pharmaceutically tolerable salts;
c) ortho-substituted benzoylguanidines of the formula I 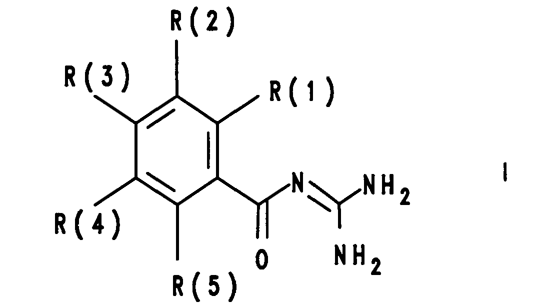 in which:
R (1) is F, Cl, Br, I, C₁-C₆-alkyl or -X-R(6);
X is O, S, NR(7) or Y-ZO;
Y is O or NR(7);
Z is C or SO;
R (6) is H, C₁-C₆-alkyl, C₅-C₇-cycloalkyl, cyclohexylmethyl, cyclopentylmethyl, - (CH₂)ₘCₚF₂ₚ₊₁ or -CₙH₂ₙ-R (8);
m is zero or 1;
p is 1 - 3;
n is zero to 4;
R(8) is phenyl,
which is unsubstituted or substituted by 1 - 3 substituents selected from the group consisting of the groups F, Cl, CF₃, methyl, methoxy and NR(9)R(10);
R(9) and R(10)
are H or C₁-C₄-alkyl;
R(7) is H or C₁-C₃-alkyl; or
R(6) and R(7)
together are 4 or 5 methylene groups, of which one CH₂ group can be replaced by O, S, NH, N-CH₃ or N-benzyl;
R(3) is H or -X-R(6);
X is O, S, NR(7) or Y-ZO;
R(7) is H or C₁-C₃-alkyl;
Y is O or NR(7);
where Y is bonded to the phenyl radical of the formula I,
Z is C or SO;
R(6) is H, C₁-C₆-alkyl, C₅-C₇-cycloalkyl, cyclohexylmethyl, cyclopentylmethyl, - (CH₂)ₘCₚF₂ₚ₊₁ or -CₙH₂ₙ-R (8) ;
m is zero or 1;
p is 1 - 3;
n is zero to 4;
R(8) is phenyl,
which is unsubstituted or substituted by 1 - 3 substituents selected from the group consisting of F, Cl, CF₃, methyl, methoxy and NR(9)R(10);
R(9) and R(10)
are H or C₁-C₄-alkyl;
or
R(6) and R(7)
together are 4 or 5 methylene groups, of which one CH₂ group can be replaced by O, S, NH, N-CH₃ or N-benzyl;
R(2) and R(4)
identically or differently are R(11)-SOq- or R(12)R(13)N-SO₂-;
q is zero - 2;
R(11) is C₁-C₄-alkyl,
which is unsubstituted or carries phenyl as a substituent, where phenyl is unsubstituted or substituted by 1 - 3 substituents selected from the group consisting of F, Cl, CF₃, methyl, methoxy and NR(9)R(10);
R(9) and R(10)
are H or C₁-C₄-alkyl;
R(12) and R(13)
are defined as R(6) and R(7);
or one of the two radicals R(2) or R(4) is hydrogen or is defined as R(1);
R(5) is H, methyl, F, Cl or methoxy,
and their pharmaceutically tolerable salts;
d) benzoylguanidines of the formula I 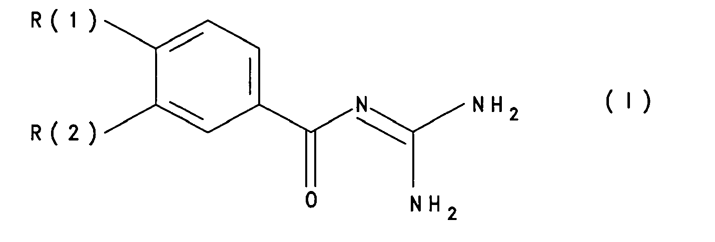 in which:
R(1) or R(2)
is an amino group -NR(3)R(4);
R(3) and R(4)
identically or differently are H, C₁-C₆-alkyl or C₃-C₇-cycloalkyl;
or
R(3) is phenyl-(CH₂)ₚ-;
p is 0, 1, 2, 3 or 4;
or
R(3) is phenyl,
where the phenyl in each case is unsubstituted or carries one to two substituents selected from the group consisting of fluorine, chlorine, methyl and methoxy; or
R(3) and R(4)
together can be a straight-chain or branched C₄-C₇-methylene chain, where one -CH₂- member of the methylene chain can be replaced by oxygen, S or NR(5);
R(5) is H or lower alkyl;
the other substituent R(1) or R(2) in each case
is H, F, Cl, C₁-C₄-alkyl, C₁-C₄-alkoxy, CF₃, CₘF₂ₘ₊₁-CH₂-, benzyl or phenoxy,
where the respective phenyl radical is unsubstituted or carries one to two substituents selected from the group consisting of methyl, methoxy, fluorine and chlorine;
m is 1, 2 or 3;
and their pharmaceutically tolerable salts;
e) benzoylguanidines of the formula I 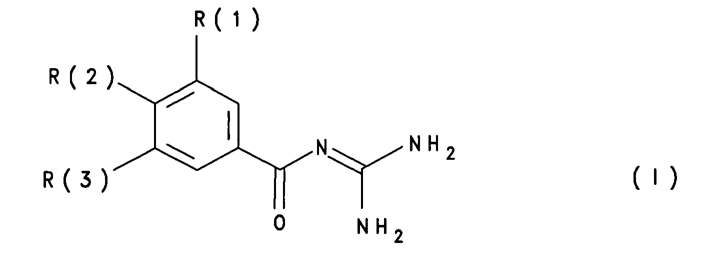 in which:
R(1) is R(4)-SOₘ or R(5)R(6)N-SO₂-;
m is zero, 1 or 2;
R(4) and R(5)
are C₁-C₈-alkyl, C₃-C₆-alkenyl or -CₙH₂ₙ-R(7) ;
n is zero, 1, 2, 3 or 4;
R(7) is C₅-C₇-cycloalkyl or phenyl, which is unsubstituted or substituted by 1 - 3 substituents selected from the group consisting of F, Cl, CF₃, methyl, methoxy and NR(8)R(9) ;
R(8) and R(9)
are H or C₁-C₄-alkyl;
or
R(5) is H;
R(6) is H or C₁-C₄-alkyl; or
R(5) and R(6) together are 4 or 5 methylene groups, of which one CH₂ group can be replaced by an O, S, NH, N-CH₃ or N-benzyl;
R(2) is hydrogen, straight-chain or branched (C₅-C₈)-alkyl,
-CR(13)=CHR(12) or -C≡CR(12);
R(12) is phenyl,
which is unsubstituted or substituted by 1 - 3 substituents selected from the group consisting of F, Cl, CF₃, methyl, methoxy and NR(14)R(15);
R(14) and R(15)
are H or (C₁-C₄)-alkyl;
or
R(12) is (C₁-C₉)-heteroaryl, which is unsubstituted or substituted as phenyl,
or
R(12) is (C₁-C₆)-alkyl,
which is unsubstituted or substituted by 1 - 3 OH,
or
R (12) is (C₃-C₈)-cycloalkyl;
R(13) is hydrogen or methyl,
or
R(12) is (C₃-C₈)-cycloalkyl, (C₃-C₈)-cycloalkyl-(C₁-C₄)-alkyl, phenyl, C₆H₅- (C₁-C₄)-alkyl, naphthyl, biphenylyl, 1,1-diphenyl-(C₁-C₄) - alkyl, cyclopentadienyl, pyridyl, pyrrolyl, furanyl, thienyl, thiazolyl, oxazolyl, indenyl, quinolyl, indolyl, benzofuranyl, benzothienyl, benzothiazolyl, benzoxazolyl, imidazolyl, pyrazolyl, triazolyl, tetrazolyl, isoxazolyl, isothiazolyl, pyrazinyl, pyrimidinyl, pyridazinyl, indazolyl, isoquinolyl, phthalazinyl, quinoxalinyl, quinazolinyl or cinnolinyl;
R(3) is defined as R(2);
and where the aromatic substituents R(2) and R(3) are unsubstituted or substituted by 1 - 3 substituents from the groups F, Cl, CF₃, (C₁-C₉)-alkyl or -alkoxy, or NR(10)R(11) with R(10) and R(11) being H or (C₁-C₄) - alkyl;
and their pharmaceutically tolerable salts;
f) benzoylguanidines of the formula I 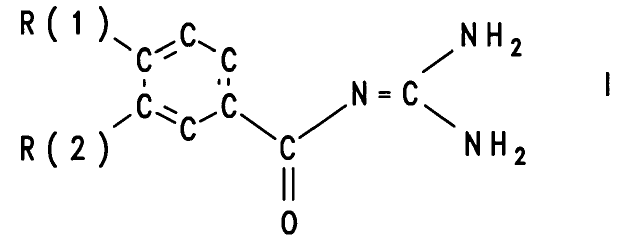 in which:
R(1) or R(2)
is R(3)-S(O)ₙ- or 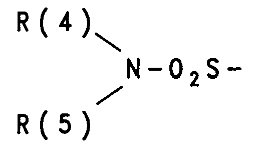 the other substituent R(1) or R(2) in each case is H, OH, F, Cl, Br, I, C₁-C₄-alkyl, C₁-C₄-alkoxy, benzyloxy or phenoxy,
which is unsubstituted or carries one to three substituents selected from the group consisting of fluorine, chlorine, methyl, methoxy, hydroxyl and benzyloxy,
R(3)-S(O)ₙ, -NR(4)R(5) or 3,4-dehydropiperidine R(3) is C₁-C₆-alkyl, C₅-C₇-cycloalkyl, cyclopentylmethyl, cyclohexylmethyl or phenyl,
which is unsubstituted or substituted by one to three substituents selected from the group consisting of fluorine, chlorine, methyl and methoxy;
R(4) and R(5)
identically or differently, are H or C₁-C₆-alkyl;
or
R(4) is phenyl-(CH₂)ₘ-;
m is 1, 2, 3 or 4;
or
R(4) is phenyl,
which is unsubstituted or carries one to two substituents selected from the group consisting of fluorine, chlorine, methyl and methoxy;
or
R(4) and R(5)
together are a straight-chain or branched C₄-C₇-chain, where the chain can additionally be interrupted by O, S or NR(6),
R(6) is H or methyl;
or
R(4) and R(5)
together with the nitrogen atom to which they are bonded, are a dihydroindole, tetrahydroquinoline or tetrahydroisoquinoline system;
n is zero, 1 or 2;
and their pharmaceutically tolerable salts;
g) isoquinolines of the formula I 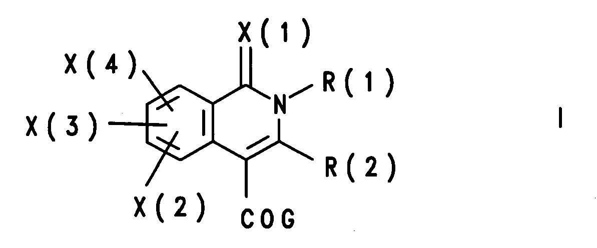 in which:
R(1) is hydrogen, alkyl, cycloalkyl, arylalkyl, alkenyl, substituted aminoalkyl or an aryl or heteroaryl ring; where the rings are unsubstituted or substituted by 1 - 3 groups selected from the group consisting of halogen, nitro, amino, mono(lower alkyl)amino, di(lower alkyl)amino, lower alkyl, lower alkoxy, benzyloxy, phenoxy, hydroxyl, trifluoromethyl,
R(2) is hydrogen, halogen, alkyl or aryl;
which is unsubstituted or substituted by 1 - 3 groups selected from the group consisting of halogen, nitro, amino, mono(lower alkyl)amino, di(lower alkyl)amino, lower alkyl, lower alkoxy, benzyloxy, phenoxy, hydroxyl,
G 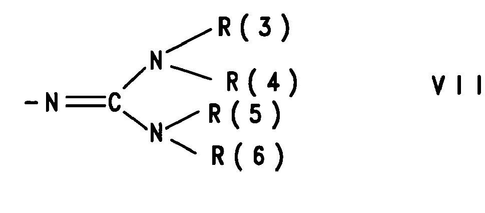 is
X(2), X(3) and X(4)
independently of one another are hydrogen, halogen, nitro, amino, alkyl, sulfonamide, mono(lower alkyl)amino, di(lower alkyl)amino, lower alkyl, benzyloxy, hydroxyl;
X(1) is hydrogen, oxygen, sulfur or NR(7);
R(7) is hydrogen, alkyl, cycloalkyl, arylalkyl, alkenyl, substituted aminoalkyl or an aryl or a heteroaryl ring;
which rings are unsubstituted or substituted by 1 - 3 groups selected from the group consisting of halogen, nitro, amino, mono(lower alkyl)amino, di(lower alkyl)amino, lower alkyl, lower alkoxy, benzyloxy, phenoxy, hydroxyl and trifluoromethyl;
in which substituents each alkyl chain or alkenyl chain can be interrupted by oxygen, sulfur or NR(8);
R(8) is hydrogen, alkyl, cycloalkyl, arylalkyl, alkenyl, substituted aminoalkyl or an aryl or heteroaryl ring,
which rings are unsubstituted or substituted by 1 - 3 groups selected from the group consisting of halogen, nitro, amino, mono(lower alkyl)amino, di(lower alkyl) amino, lower alkyl, lower alkoxy, benzyloxy, phenoxy, hydroxyl and trifluoromethyl;
and their pharmaceutically acceptable salts;
h) compounds of the formula I 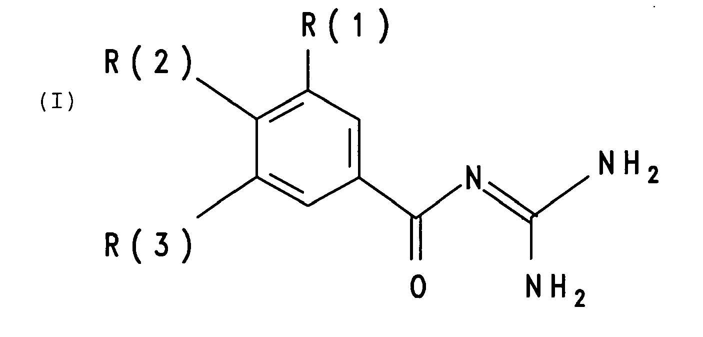 in which:
R(1) is hydrogen, F, Cl, Br, I, -NO₂, -C≡N, -CF₃, R(4)-SOₘ or R(5)R(6)N-SO₂-;
m is zero, 1 or 2;
R(4) and R(5)
are (C₁-C₈)-alkyl, (C₃-C₆)-alkenyl, -CₙH₂ₙ-R(7) or CF₃;
n is zero, 1, 2, 3 or 4;
R(7) is (C₃-C₇)-cycloalkyl or phenyl,
which is not substituted or is substituted by 1 - 3 substituents selected from the group consisting of F, Cl, CF₃, methyl, methoxy and NR(8)R(9);
R(8) and R(9)
are H or C₁-C₄-alkyl;
or
R(5) is H;
R(6) is H or (C₁-C₄)-alkyl;
or
R(5) and R(6)
together are 4 or 5 methylene groups, of which one CH₂ group can be replaced by oxygen, S, NH, N-CH₃ or N-benzyl;
R(2) is -SR(10), -OR(10), -NHR(10), -NR(10)R(11), - CHR(10)R(12), -[CR(12)R(13)OR(13')], -{C-[CH₂-OR(13')]R(12)R(13)} or -[CR(18)R(17)]ₚ-(CO)-[CR(19)R(20)]_{q}-R(14); R(10), R(11)
identically or differently are -[CHR(16)]ₛ-(CH₂)ₚ-(CHOH)_{q}-(CH₂)ᵣ-(CHOH)ₜ-R(21) or
- (CH₂)ₚ-O- (CH₂-CH₂O)_{q}-R(21),
R(21) is hydrogen, methyl,
p, q, r identically or differently
are zero, 1, 2, 3 or 4;
s is zero or 1;
t is 1, 2, 3 or 4;
R(12) and R(13) identically or differently are hydrogen, (C₁-C₆)-alkyl or, together with the carbon atom carrying them, are a (C₃-C₈)-cycloalkyl, R(13') is hydrogen or (C₁-C₄)-alkyl;
R(14) is H, (C₁-C₆)-alkyl, (C₃-C₈)-cycloalkyl or
-CₐH₂ₐ-R (15) ;
a is zero, 1, 2, 3 or 4;
R(15) is phenyl,
which is unsubstituted or substituted by 1 - 3 substituents selected from the group consisting of F, Cl, CF₃, methyl, methoxy and NR(8)R(9);
R(8) and R(9)
are H or (C₁-C₄)-alkyl;
or
R(15) is (C₁-C₉)-heteroaryl,
which is unsubstituted or substituted as phenyl,
or
R(15) is (C₁-C₆)-alkyl,
which is unsubstituted or substituted by 1 - 3 OH;
R(16), R(17), R(18), R(19) and R(20)
are hydrogen or (C₁-C₃)-alkyl;
R(3) is defined as R(1),
or
R(3) is (C₁-C₆)-alkyl or -X-R(22);
X is oxygen, S or NR(16);
R(16) is H or (C₁-C₃)-alkyl;
or
R(22) and R(16)
together are 4 or 5 methylene groups, of which one CH₂ group can be replaced by oxygen, S, NH, N-CH₃ or N-benzyl;
R(22) is defined as R(14);
and their pharmaceutically tolerable salts;
i) benzoylguanidines of the formula I 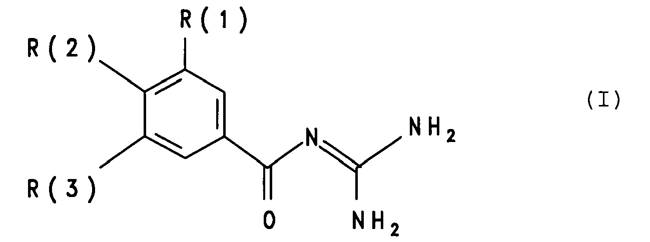 in which:
R(1) is hydrogen, F, Cl, Br, I, -NO₂, -C=N, R(16)-CₚH₂ₚ-Oq, R(4)-SOₘ or R(5)R(6)N-SO₂-;
m is zero, 1 or 2;
p is zero or 1;
q is zero, 1, 2 or 3;
R(16) is CᵣF₂ᵣ₊₁;
r is 1, 2 or 3;
R(4) and R(5)
are (C₁-C₈)-alkyl, (C₃-C₆) -alkenyl, -CₙH₂ₙ-R(7) or CF₃;
n is zero, 1, 2, 3 or 4;
R(7) is (C₃-C₇)-cycloalkyl or phenyl,
which is not substituted or is substituted by 1 - 3 substituents selected from the group consisting of F, Cl, CF₃, methyl, methoxy and NR(8)R(9) ;
R(8) and R(9)
are H or C₁-C₄-alkyl;
or
R(5) is H;
R(6) is H or (C₁-C₄)-alkyl;
or
R(5) and R(6)
together are 4 or 5 methylene groups, of which one CH₂ group can be replaced by oxygen,
S, NH, N-CH₃ or N-benzyl,
R(2) is (C₁-C₉)-heteroary,
which is linked via C or N and which is unsubstituted or substituted by 1 - 3 substituents selected from the group consisting of F, Cl, CF₃, CH₃, methoxy, hydroxyl, amino, methylamino and dimethylamino;
or
R(2) is -SR(10), -OR(10), -NR(10)R(11), - CR(10)R(11)R(12);
R(10) is -CₐH₂ₐ-(C₁-C₉)-heteroaryl,
which is unsubstituted or substituted by 1 - 3 substituents selected from the group consisting of F, Cl, CF₃, CH₃, methoxy, hydroxyl, amino, methylamino and dimethylamino;
a is zero, 1 or 2;
R(11) and R(12)
independently of one another are defined as R(10) or are hydrogen or (C₁-C₄)-alkyl;
R(3) is defined as R(1), or is (C₁-C₆)-alkyl or -X-R(13);
X is oxygen, S, or NR(14); R(14) is H or (C₁-C₃)-alkyl;
R(13) is H, (C₁-C₆)-alkyl, (C₃-C₈)-cycloalkyl or -C_{b}H_{2b}-R(15) ;
b is zero, 1, 2, 3 or 4;
or
R(13) and R(14)
together are 4 or 5 methylene groups, of which one CH₂ group can be replaced by oxygen, S, NH, N-CH₃ or N-benzyl;
R(15) is phenyl,
which is unsubstituted or substituted by 1 - 3 substituents selected from the group consisting of F, Cl, CF₃, methyl, methoxy and NR(8)R(9); R(8) and R(9)
are H or (C₁-C₄)-alkyl;
and their pharmaceutically tolerable salts;
k) benzoylguanidines of the formula I 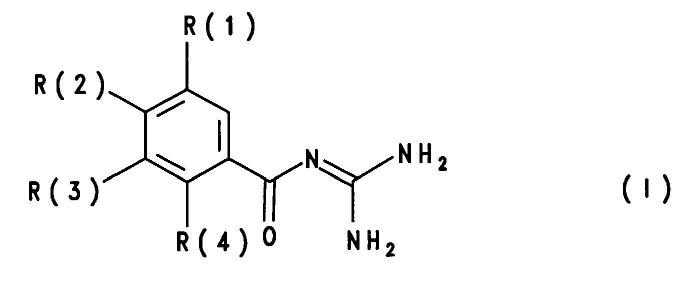 in which:
one of the substituents R(1), R(2), R(3) or R(4) is an amino group 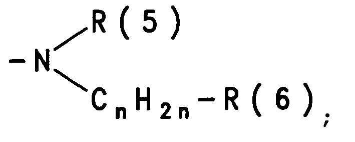 R(5) is hydrogen or C₍₁₋₆₎-alkyl;
n is zero, 1, 2, 3 or 4;
R(6) is H or C₍₁₋₄₎-alkyl;
in which one CH₂ group can be replaced by 1 sulfur atom or a group NR(7);
R(7) is hydrogen, methyl or ethyl;
or
R(6) is C₍₃₋₈₎-cycloalkyl or phenyl,
which is unsubstituted or carries 1, 2 or 3 substituents selected from the group consisting of F, Cl, Br, methyl, methoxy, -NR(8)R(9);
R(8) and R(9)
are H, methyl or ethyl;
or
R(5) and R(6)
together with the nitrogen atom are a 5-, 6-or 7-membered ring, in which 1 carbon atom can be replaced by oxygen, S or NR(10);
R(10) is H, C₍₁₋₃₎-alkyl or benzyl; and the other substituents R(1), R(2), R(3), R(4) in each case are:
hydrogen, F, Cl, Br, I, CN, CF₃, NO₂, CF₃-O-, CₘF₂ₘ₊₁-CH₂-O- or R (11)-C_{q}H_{2q}-Xₚ-;
m is 1, 2 or 3;
q is zero, 1, 2, 3 or 4;
p zero or 1;
X is oxygen or NR(12);
R(12) is H or C(₁₋₃)-alkyl;
R(11) is hydrogen, C(₁₋₆)-alkyl, C(₃₋₈)-cycloalkyl or phenyl,
which is unsubstituted or substituted by 1, 2 or 3 substituents selected from the group consisting of F, Cl, CH₃, CH₃-O- and NR(13)R(14);
R(13), R(14)
are H, methyl or ethyl;
and their pharmaceutically tolerable salts; 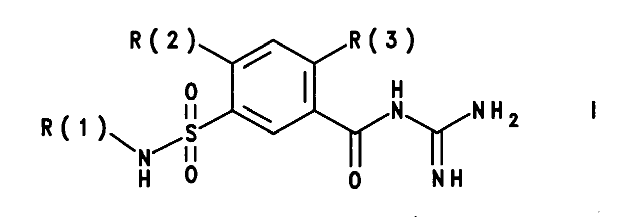
1) benzoylguanidines of the formula I
in which
R(1) is R(4)R(5)N-C(X)-;
X is oxygen, S or N-R(6);
R(4) and R(5)
identically or differently, are H, (C₁-C₈)-alkyl, (C₃-C₆)-alkenyl or -CₙH₂ₙ-R(7) ;
n is zero, 1, 2, 3 or 4;
R(7) is (C₅-C₇)-cycloalkyl or phenyl,
which is unsubstituted or substituted by 1 - 3 substituents selected from the group consisting of F, Cl, CF₃, methoxy and (C₁-C₄)-alkyl;
or
R(4) and R(5)
together are 4 or 5 methylene groups, of which one CH₂ group can be replaced by oxygen, S, NH, N-CH₃ or N-benzyl;
R(6) is defined as R(4) or is amidine;
R(2) is H, F, Cl, Br, I, (C₁-C₈)-alkyl, 1-alkenyl or 1-alkynyl, (C₃-C₈)-cycloalkyl, (C₃-C₈)-cycloalkyl-(C₁-C₄)-alkyl, phenyl, C₆H₅- (C₁-C₄) -alkyl, naphthyl, biphenylyl, 1,1-diphenyl-(C₁-C₄)-alkyl, cyclopentadienyl, pyridyl, thiopyridyl, pyrrolyl, furanyl, thienyl, thiazolyl, oxazolyl, indenyl, quinolyl, indolyl, benzofuranyl, benzothienyl, benzothiazolyl, benzoxazolyl or -W-R(8);
W is oxygen, S or NR(9);
R(8) is H, (C₁-C₆)-alkyl, (C₅-C₇)-cycloalkyl, cyclohexylmethyl, cyclopentylmethyl, -(CH₂)ₘCₚF₂ₚ₊₁ or -C_{q}H_{2q}-R(10) ;
m is zero or 1;
p is 1, 2 or 3;
q is zero, 1, 2, 3 or 4;
R(10) is phenyl,
which is unsubstituted or substituted by 1 - 3 substituents selected from the group consisting of F, Cl, CF₃, methyl, methoxy and NR(11)R(12);
R(11) and R(12)
are H or (C₁-C₄)-alkyl;
R(9) is H or (C₁-C₃)-alkyl;
or
R(8) and R(9)
together are 4 or 5 methylene groups, of which one CH₂ group can be replaced by oxygen, S, NH, N-CH₃ or N-benzyl;
R(3) is H, F, Cl, Br, I, (C₁-C₆)-alkyl or -W-R(8) as defined for R(2),
and their pharmaceutically acceptable salts;
m) benzoylguanidines of the formula I 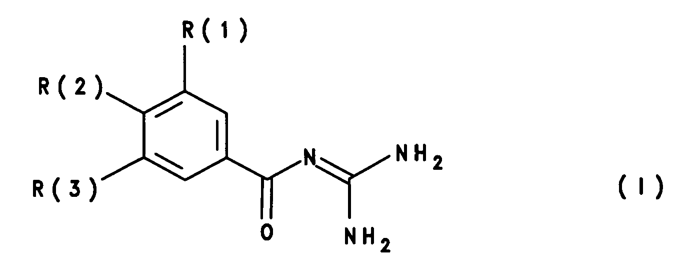 in which:
R(1), R(2), R(3)
are hydrogen, F, Cl, Br, I or (C₁-C₁₂)-alkyl; one of the substituents R(1), R(2) or R(3) is N₃, CN, OH or (C₁-C₁₀)-alkyloxy, if at least one of the remaining substituents R(1), R(2) or R(3) is a sufficiently lipophilic alkyl radical having 3 to 12 carbon atoms;
or
one of the substituents R(1), R(2) and R(3)
is R (4) -CₙH₂ₙ-Oₘ- ;
m is zero or 1;
n is zero, 1, 2 or 3;
R (4) is CₚF₂ₚ₊₁;
p is 1, 2 or 3, if n is zero or 1;
or
R (4) is (C₃-C₁₂) -cycloalkyl, phenyl, pyridyl, quinolyl or isoquinolyl, where the aromatic and heteroaromatic ring systems are unsubstituted or substituted by a substituent selected from the group consisting of F, Cl, CF₃, methyl, methoxy and NR(5)R(6);
R(5) and R(6)
are hydrogen or (C₁-C₄)-alkyl;
or one of the substituents R(1), R(2) and R(3) is -C=CR(5) or -C[R(6)] = CR(5);
R(5) is phenyl, which is unsubstituted or substituted by 1 - 3 substituents selected from the group consisting of F, Cl, CF₃, methyl, methoxy, hydroxyl, amino, methylamino and dimethylamino, (C₁-C₉)-heteroaryl,
which is unsubstituted or substituted as phenyl,
or
R(5) is (C₁-C₆)-alkyl,
which is unsubstituted or substituted by 1 - 3 OH;
or
R(5) is (C₃-C₈)-cycloalkyl,
R(6) is hydrogen or methyl;
and their pharmacologically acceptable salts; o) benzoylguanidines of the formula I 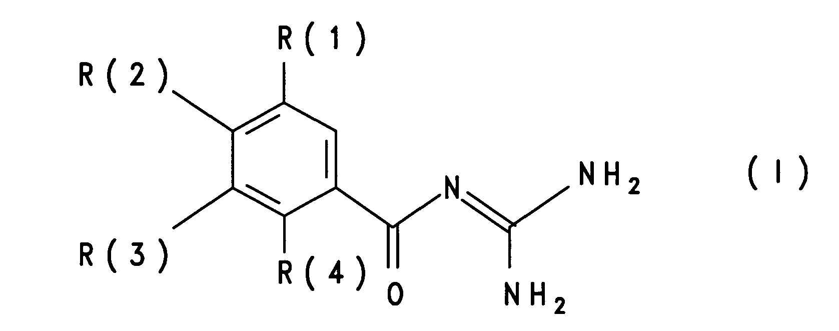
in which:
R (1) is hydrogen, F, Cl, Br, I, -NO₂, -C≡N, Xₒ-(CH₂)ₚ-(CF₂)_{q}-CF₃, R(5)-SOₘ, R(6)-CO- or R(6)R(7)N-SO₂-, where
X is oxygen, S or NR(14);
m is zero, 1 or 2;
o is zero or 1;
p is zero, 1 or 2;
q is zero, 1, 2, 3, 4, 5 or 6;
R(5) and R(6) are (C₁-C₈)-alkyl, (C₃-C₆)-alkenyl, -CₙH₂ₙ-R(8) or CF₃;
n is zero, 1, 2, 3 or 4;
R(8) is (C₃-C₇)-cycloalkyl or phenyl, which is not substituted or is substituted by 1 - 3 substituents selected from the group consisting of F, Cl, CF₃, methyl, methoxy and NR(9)R(10);
R(9) and R(10) are H or C₁-C₄-alkyl;
or
R(6) is H;
R(7) is H or (C₁-C₄)-alkyl;
or
R(6) and R(7)
together are 4 or 5 methylene groups, of which one CH₂ group can be replaced by oxygen, S, NH, N-CH₃ or N-benzyl;
R(2) is 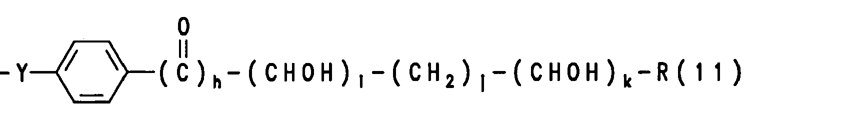 or 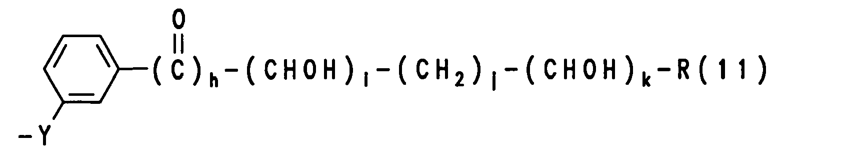 or 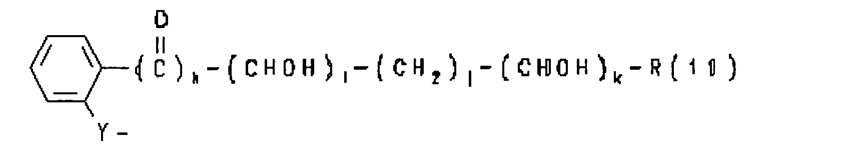 Y is oxygen, -S- or -NR(12)-;
R(11) and R(12)
are hydrogen or (C₁-C₃)-alkyl;
h is zero or 1;
i, j and k
independently are zero, 1, 2, 3 or 4;
but where h, i and k are not simultaneously zero,
R(3) is defined as R(1), or is (C₁-C₆)-alkyl or -X-R(13);
X is oxygen, S or NR(14);
R(14) is H or (C₁-C₃)-alkyl;
R(13) is H, (C₁-C₆)-alkyl, (C₃-C₈)-cycloalkyl or -C_{b}H_{2b}-R(15);
b is zero, 1, 2, 3 or 4;
or
R(13) and R(14)
together are 4 or 5 methylene groups, where one CH₂ group can be replaced by oxygen, S, NH, N-CH₃ or N-benzyl;
R(15) is phenyl,
which is unsubstituted or substituted by 1 - 3 substituents selected from the group consisting of F, Cl, CF₃, methyl, methoxy and NR(9)R(10);
R(9) and R(10)
are H or (C₁-C₄) -alkyl;
R(4) is hydrogen, -OR(16) or -NR(16)R(17);
R(16) and R(17)
independently are hydrogen or (C₁-C₃)-alkyl; and their pharmaceutically tolerable salts;
p) benzoylguanidines of the formula I 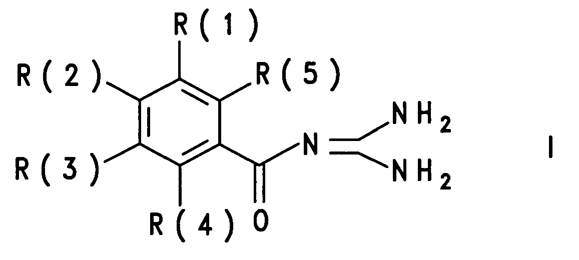 in which:
R(1) is R(6)-CO or R(7)R(8)N-CO;
R(6) is (C₁-C₈)-alkyl, (C₁-C₈) -perfluoroalkyl, (C₃-C₈)-alkenyl or -CₙH₂ₙ-R(9) ;
n is zero, 1, 2, 3 or 4;
R(9) is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR(10)R(11);
R(10) and R(11)
are H, (C₁-C₄-alkyl or (C₁-C₄)-perfluoroalkyl;
R (7) is H, (C₁-C₈)-alkyl, (C₁-C₈)-perfluoroalkyl, (C₃-C₈)-alkenyl or -CₙH₂ₙ-R(12);
n is zero, 1, 2, 3 or 4;
R(12) is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR(13)R(14);
R(13) and R(14)
are H, (C₁-C₄-alkyl or (C₁-C₄)-perfluoroalkyl;
R(8) is H, (C₁-C₄)-alkyl or (C₁-C₄)-perfluoroalkyl;
or
R(7) and R(8)
together are 4 or 5 methylene groups, of which one CH₂ group can be replaced by oxygen, S, NH, N-CH₃ or N-benzyl;
R(2) is defined as R(1), or is H, F, Cl, Br, I, CN, NO₂, (C₁-C₈)-alkyl, (C₁-C₈) -perfluoroalkyl, (C₃-C₈)-alkenyl or -CₙH₂ₙR(15);
n is zero 1, 2, 3, 4;
R (15) is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR(16)R(17);
R(16) and R(17)
are H, (C₁-C₄-alkyl or (C₁-C₄) - perfluoroalkyl;
or
R(2) is (C₁-C₉)-heteroaryl, which is linked via C or N and which is unsubstituted or substituted by 1 - 3 substituents selected from the group consisting of F, Cl, CF₃, CH₃, methoxy, hydroxyl, amino, methylamino and dimethylamino;
or
R(2) is SR(18), -OR(18), -NR(18)R(19), - CR(18)R(19)R(20);
R (18) is -CₐH₂ₐ-(C₁-C₉)-heteroaryl,
which is unsubstituted or substituted by 1 - 3 substituents selected from the group consisting of F, Cl, CF₃, CH₃, methoxy, hydroxyl, amino, methylamino and dimethylamino;
a is zero, 1 or 2;
R(19) and R(20)
independently of one another are defined as R(18) or are hydrogen, (C₁-C₄)-alkyl or (C₁-C₄)-perfluoroalkyl;
or
R(2) is R(21)-SOₘ or R(22)R(23)N-SO₂-;
m is 1 or 2;
R (21) is (C₁-C₈)-alkyl, (C₁-C₈)-perfluoroalkyl, (C₃-C₈)-alkenyl, -CₙH₂ₙ-R(24),
n is zero, 1, 2, 3 or 4;
R(24) is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR(27)R(28);
R(27) and R(28)
are H, (C₁-C₄)-alkyl or (C₁-C₉) - perfluoroalkyl;
R(22) is H, (C₁-C₈)-alkyl, (C₁-C₈) - perfluoroalkyl, (C₃-C₈)-alkenyl, -CₙH₂ₙ-R(29) ;
n is zero, 1, 2, 3 or 4;
R(29) is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR(30)R(31);
R(30) and R(31)
are H, (C₁-C₄)-alkyl or (C₁-C₄)-perfluoroalkyl;
R(23) is H, (C₁-C₄)-alkyl or (C₁-C₄)-perfluoroalkyl;
or
R(22) and R(23)
together are 4 or 5 methylene groups, of which one CH₂ group can be replaced by
oxygen, S, NH, N-CH₃ or N-benzyl;
or
R(2) is R(33)X-;
X is oxygen, S, NR(34), (D=O)A-,
NR(34)C=MN⁽*⁾R(35)-;
M is oxygen or S;
A is oxygen or NR(34);
D is C or SO;
R(33) is (C₁-C₈)-alkyl, (C₃-C₈) -alkenyl, (CH₂)_{b}C_{d}F_{2d+1}, -CₙH₂ₙ-R (36),
b is zero or 1;
d is 1, 2, 3, 4, 5, 6 or 7;
n is zero, 1, 2 , 3 or 4;
R(36) is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR(37)R(38);
R(37) and R(38)
are H, (C₁-C₄)-alkyl or (C₁-C₄)-perfluoroalkyl;
R(34) is H, (C₁-C₄)-alkyl or (C₁-C₄) - perfluoroalkyl;
R(35) is defined as R(33);
or
R(33) and R(34)
together are 4 or 5 methylene groups, of which one CH₂ group can be replaced by oxygen, S, NH, N-CH₃ or N-benzyl;
where A and N⁽*⁾ are bonded to the phenyl nucleus of the benzoylguanidine parent structure;
or
R(2) is -SR(40), -OR(40), -NHR(40), -NR(40)R(41), - CHR(40)R(42), -C[R(42)R(43)OH], -C≡CR(45), - CR(46)=CHR(45), -[CR(47)R(48)]ᵤ-(CO)-[CR49)R(50)]ᵥ-R(44);
R(40), R(41)
identically or differently are -(CH₂)ₚ-(CHOH)q-(CH₂)ᵣ-(CHOH)ₜ-R(51) or -(CH₂)ₚ-O-(CH₂-CH₂O)_{q}-R(51) ;
R(51) is hydrogen or methyl;
u is 1, 2, 3 or 4;
v is zero, 1, 2, 3 or 4;
p, q, r
identically or differently are zero, 1, 2, 3 or 4;
t is 1, 2, 3 or 4;
R(42) and R(43)
identically or differently are hydrogen or (C₁-C₆)-alkyl;
or
R(42) and R(43)
together with the carbon atom carrying them form a (C₃-C₈)-cycloalkyl;
R(44) is H, (C₁-C₆)-alkyl, (C₃-C₈)-cycloalkyl or -CₑH₂ₑ-R (45) ;
e is zero, 1, 2, 3 or 4;
R(45) is phenyl, which is unsubstituted or substituted by 1 - 3 substituents from the group consisting of F, Cl, CF₃, methyl, methoxy and NR(52)R(53) where
R(52) and R(53) are H or (C₁-C₄)-alkyl, or
R(45) is (C₁-C₉)-heteroaryl,
which is unsubstituted or substituted as phenyl;
or
R(45) is (C₁-C₆)-alkyl,
which is unsubstituted or substituted by 1 - 3 OH;
R(46), R(47), R(48), R(49) and R(50)
are hydrogen or methyl;
or
R(2) is R(55)-NH-SO₂-;
R(55) is R(56)R(57)N-(C=Y)-;
Y is oxygen, S or N-R(58);
R(56) and R(57)
identically or differently are H, (C₁-C₈)-alkyl, (C₃-C₆) -alkenyl or -C_{f}H_{2f}-R(59);
f is zero, 1, 2, 3 or 4;
R(59) is (C₅-C₇)-cycloalkyl or phenyl,
which is unsubstituted or substituted by 1 - 3 substituents selected from the group consisting of F, Cl, CF₃, methoxy and (C₁-C₄)-alkyl;
or
R(56) and R(57)
together are 4 or 5 methylene groups, of which one CH₂ group can be replaced by oxygen, S, NH, N-CH₃ or N-benzyl;
R(58) is defined as R(56) or is amidine;
R(3), R(4) and R(5)
independently of one another are defined as R(1) or R(2);
and their pharmaceutically tolerable salts;
q) benzoylguanidines of the formula I 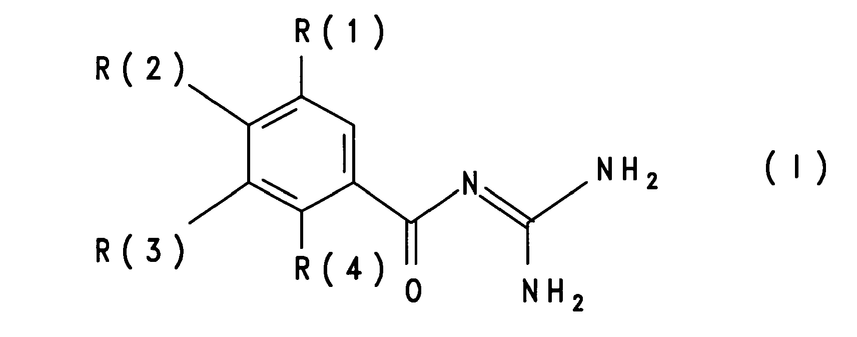 in which:
R(1) is hydrogen, F, Cl, Br, I, -NO₂, -C≡N, -Xₒ-(CH₂)ₚ-(CF₂)q-CF₃, R(5)-SOₘ-, R(6)-CO-, R(6)R(7)N-CO- or R(6)R(7)N-SO₂-;
X is oxygen, -S- or NR(14);
m is zero, 1 or 2;
o is zero or 1;
p is zero, 1 or 2;
q is zero, 1, 2, 3, 4, 5 or 6;
R(5) and R(6)
are (C₁-C₈)-alkyl, (C₃-C₆) -alkenyl, -CₙH₂ₙ-R(8) or CF₃;
n is zero, 1, 2, 3 or 4;
R(8) is (C₃-C₇)-cycloalkyl, phenyl,
which is not substituted or is substituted by 1 to 3 substituents selected from the group consisting of F, Cl, CF₃, methyl, methoxy and NR(9)R(10);
R(9) and R(10)
are H or (C₁-C₄) -alkyl;
or
R(6) is hydrogen; R(7) is hydrogen or (C₁-C₄)-alkyl;
or
R(6) and R(7)
together are 4 or 5 methylene groups, of which one CH₂ group can be replaced by oxygen, S, NH, N-CH₃ or N-benzyl; 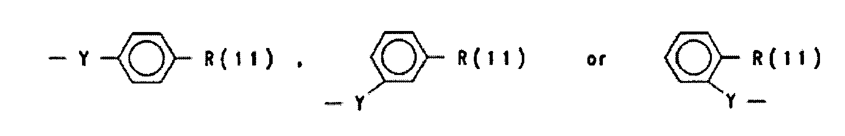
R(2) is
R(11) is (C₁-C₉)-heteroaryl,
which is linked via C or N and which is unsubstituted or substituted by 1 to 3 substituents selected from the group consisting of F, Cl, CF₃, CH₃, methoxy, hydroxyl, amino, methylamino, dimethylamino and benzyl;
Y is oxygen, -S- or NR(12);
R(12) is H or (C₁-C₄)-alkyl;
R(3) is defined as R(1);
or
R(3) is (C₁-C₆)-alkyl or -X-R(13);
X is oxygen, -S- or NR(14);
R(14) is H or (C₁-C₃)-alkyl;
R(13) is H, (C₁-C₆)-alkyl, (C₃-C₈) - cycloalkyl or -C_{b}H_{2b}-R(15);
b is zero, 1, 2, 3 or 4;
or
R(13) and R(14)
together are 4 or 5 methylene groups, of which one CH₂ group can be replaced by oxygen, S, NH, N-CH₃ or N-benzyl;
R(15) is phenyl,
which is unsubstituted or substituted by 1 - 3 substituents selected from the group consisting of F, Cl, CF₃, methyl, methoxy and NR(9)R(10);
R(9) and R(10) are H or (C₁-C₄)-alkyl;
R(4) is hydrogen, -OR(16), -NR(16)R(17) or CᵣF₂ᵣ₊₁; R(16) and R(17)
independently are hydrogen or (C₁-C₃)-alkyl;
r is 1, 2, 3 or 4;
and their pharmaceutically tolerable salts;
r) benzo-fused 5-membered ring heterocycles of the formula I 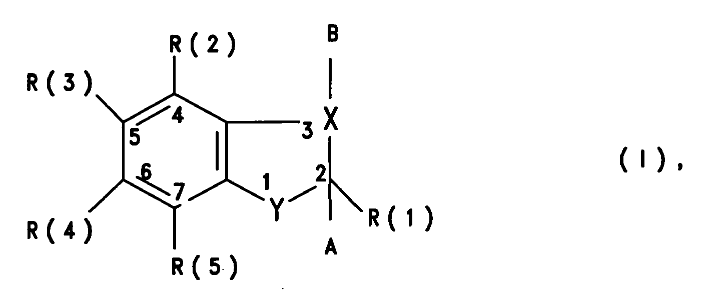 in which:
X is N or CR(6);
Y is oxygen, S or NR(7);
A, B together are a bond
or
A, B are both hydrogen, if X is simultaneously CR(6) and Y is NR(7);
one of the substituents R(1) to R(6) is a -CO-N=C(NH₂)₂ group;
the other substituents R(1) to R(6) in each case are hydrogen, F, Cl, Br, I or (C₁-C₆)-alkyl;
up to two of the other substituents R(1) to R(6) are CN, NO₂, N₃, (C₁-C₄)-alkyloxy or CF₃;
up to one of the other substituents is R(8)-CₙH₂ₙ-Z-;
n is zero to 10;
where the alkylene chain -CₙH₂ₙ- is straight-chain or branched and where one carbon atom can be replaced by an oxygen or sulfur atom or by a nitrogen atom;
R (8) is hydrogen, (C₂-C₆)-alkenyl or (C₃-C₁₀)-cycloalkyl,
which is unsubstituted or substituted by 1 to 4 methyl groups or an OH group, or can contain an ethylene group -CH=CH-, and in which one methylene group can be replaced by an oxygen or sulfur atom or by a nitrogen atom;
or
R(8) is phenyl,
which is unsubstituted or substituted by 1 to 3 substituents selected from the group consisting of F, Cl, Br, I, CF₃, CH₃-S(O)ₛ- or R(9)-W_{y}-;
s is zero, 1 or 2;
R(9) is H, methyl, ethyl,
W is oxygen or NR(10);
R(10) is H or methyl;
y is zero or 1;
or
R(8) is CₘF₂ₘ₊₁;
m is 1 to 3;
or
R(8) is 1- or 2-naphthyl, pyridyl, quinolyl or isoquinolyl;
Z is -CO-, -CH₂- or -[CR(11)(OH)]_{q}-;
q is 1, 2 or 3;
R(11) is H or methyl;
or
Z is oxygen or -NR(12)-;
R(12) is H or methyl;
or
Z is -S(O)ₛ-;
s is zero, 1 or 2;
or
Z is -SO₂-NR(13)-;
R(13) is H or (C₁-C₉)-alkyl; R(7) is hydrogen, (C₁-C₁₀)-alkyl, (C₂-C₁₀)-alkenyl or R(8)-CₙH₂ₙ-;
and their pharmaceutically tolerable salts;
s) benzoylguanidines of the formula I 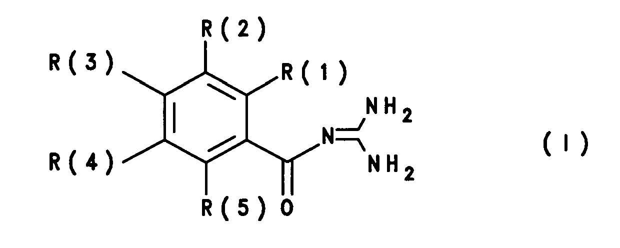 in which:
R (1) R(3) or R(4)
is -NR(6) C=X NR(7)R(8);
X is oxygen or S;
R(6) is hydrogen, (C₁-C₈)-alkyl, (C₁-C₈)-perfluoroalkyl, (C₃-C₈)-alkenyl or -CₙH₂ₙ-R(9);
n is zero, 1, 2, 3 or 4;
R(9) is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR (10) R (11) ;
R(10) and R(11)
are H, (C₁-C₄)-alkyl or (C₁-C₄)-perfluoroalkyl;
R(7) is hydrogen, (C₁-C₈)-alkyl, (C₁-C₈)-perfluoroalkyl, (C₃-C₈)-alkenyl or -CₒH₂ₒ-R(12);
o is zero, 1, 2, 3 or 4;
R(12) is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR(13)R(14);
R(13) and R(14)
are H, (C₁-C₄)-alkyl or (C₁-C₄)-perfluoroalkyl;
R(8) is defined as R(7);
or
R(7) and R(8)
together are 4 or 5 methylene groups, of which one CH₂ group can be replaced by
oxygen, S, NH, N-CH₃ or N-benzyl; the remaining substituents R(2), R(3), R(4), R(5) or R(1), R(2), R(4), R(5) or R(1), R(2), R(3), R(5) in each case
independently of one another are hydrogen, F, Cl, Br, I, -Oₜₐ(C₁-C₈)-alkyl, -O_{tb}(C₃-C₈)-alkenyl, -O_{tc} (CH₂)_{b}C_{d}F_{2d+1}, -O_{td}CₚH₂ₚR (18),
or up to 2 groups CN, NO₂, NR(16)R(17),
b is zero or 1;
d is 1, 2, 3, 4, 5, 6 or 7;
ta is zero or 1;
tb is zero or 1;
tc is zero or 1;
td is zero or 1;
p is zero, 1, 2, 3 or 4;
R(18) is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR(19)R(20);
R(19) and R(20)
are hydrogen or (C₁-C₄)-alkyl or (C₁-C₄)-perfluoroalkyl;
R(16) is hydrogen, (C₁-C₈)-alkyl, (C₁-C₈) - perfluoroalkyl, (C₃-C₈)-alkenyl, -C_{q}H_{2q}-R(21),
q is zero, 1, 2, 3 or 4;
R (21) is (C₃-C₈)-cycloalkyl, phenyl, biphenylyl or naphthyl,
where the aromatics are not substituted or are substituted by 1 - 3 substituents from the group F, Cl, CF₃, methyl, methoxy or NR(22)R(23),
R(22) and R(23) are hydrogen, (C₁-C₄)-alkyl or (C₁-C₄)-perfluoroalkyl;
R(17) is hydrogen, (C₁-C₈)-alkyl, (C₁-C₈)-perfluoroalkyl, (C₃-C₈) -alkenyl, -CᵣH₂ᵣ-R(24);
r is zero, 1, 2, 3 or 4;
R(24) is (C₃-C₈)-cycloalkyl, phenyl,
biphenylyl or naphthyl,
where the aromatics are not substituted or are substituted by 1 - 3 substituents selected from the group consisting of F, Cl, CF₃, methyl, methoxy and NR(25)R(26);
R(25) and R(26)
are hydrogen, (C₁-C₄)-alkyl or (C₁-C₄)-perfluoroalkyl;
or
R(16) and R(17)
together are 4 or 5 methylene groups, of which one CH₂ group can be replaced by oxygen, S, NH, N-CH₃ or N-benzyl;
and their pharmaceutically tolerable salts;
t) diacyl-substituted guanidines of the formula I 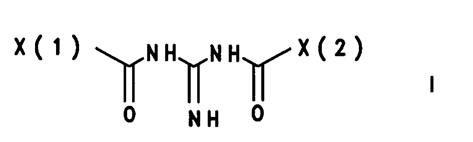 in which:
X(1) and X(2) are 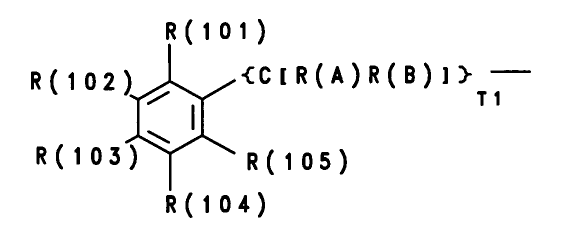
T1 is zero, 1, 2, 3 or 4;
R (A) and R(B)
independently of one another are hydrogen, F, Cl, Br, I, CN, OR(106), (C₁-C₈)-alkyl, (C₃-C₈)-cycloalkyl, O_{zk}(CH₂)_{zl}C_{zm}F_{2zm+1}, NR(107)R(108), phenyl or benzyl,
where the aromatics are not substituted or are substituted by 1 - 3 substituents selected from the group consisting of F, Cl, CF₃, methyl, methoxy and NR(109)R(110);
R(109) and R(110)
are hydrogen, (C₁-C₄)-alkyl or (C₁-C₄) - perfluoroalkyl;
zl is zero, 1, 2, 3 or 4;
zk is zero or 1;
zm is 1, 2, 3, 4, 5, 6, 7 or 8;
R(106)
is hydrogen, (C₁-C₈)-alkyl, (C₁-C₈)-perfluoroalkyl, (C₃-C₈)-alkenyl, (C₃-C₈)-cycloalkyl, phenyl or benzyl,
where the aromatics are not substituted or are substituted by 1 - 3 substituents selected from the group consisting of F, Cl, CF₃, methyl, methoxy and NR(111) R(112) ;
R(111) and R(112)
are hydrogen, (C₁-C₄)-alkyl or (C₁-C₄)-perfluoroalkyl;
R(107) and R(108)
independently of one another are defined as R(106),
or
R(107) and R(108)
together are 4 or 5 methylene groups, of which one CH₂ group can be replaced by
oxygen, S, NH, N-CH₃ or N-benzyl;
or
X(1) and X(2) are 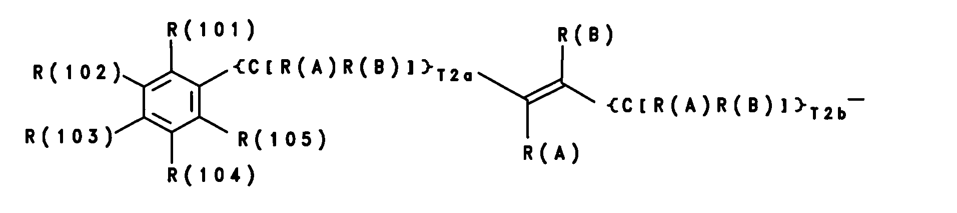 T2a and T2b
independently of one another are zero, 1 or 2;
where the double bond can have the (E)- or (Z)-configuration;
or
X(1) and X(2) are 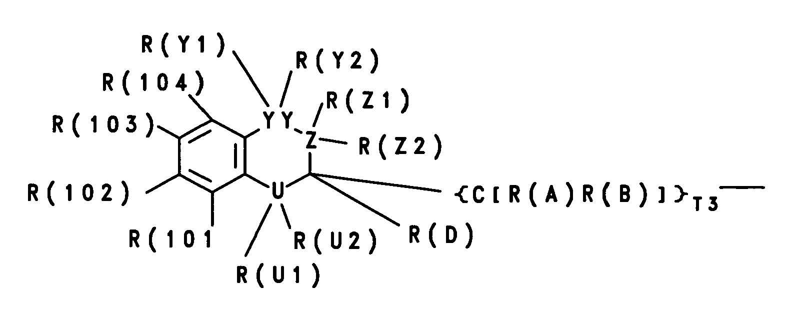 T3 is zero, 1 or 2;
U, YY and Z
independently of one another are C or N, where U, YY, Z can carry the following number of substituents:
| U, YY or Z | Bonded in the ring to a double bond | Number of permitted substituents |
|---|---|---|
| C | yes | 1 |
| C | no | 2 |
| N | yes | 0 |
| N | no | 1 |
R (D) is hydrogen, (C₁-C₈)-alkyl or (C₁-C₈)-perfluoroalkyl,
R(U1), R(U2), R(Y1), R(Y2), R(Z1), R(Z2)
independently of one another are hydrogen, F, Cl, Br, I, CN, OR(114), (C₁-C₈)-alkyl, (C₃-C₈)-Cycloalkyl, O_{zka}(CH₂)_{zla}C_{zma}F_{2zma+1}, NR(115)R(116), phenyl or benzyl,
where the aromatics are not substituted or are substituted by 1 - 3 substituents selected from the group consisting of F, Cl, CF₃, methyl, methoxy, NR(117)R(118),
R(117) and R(118)
are hydrogen, (C₁-C₄)-alkyl or (C₁-C₄) - perfluoroalkyl,
zka is zero or 1;
zla is zero, 1, 2, 3 or 4;
zma is 1, 2, 3, 4, 5, 6, 7 or 8;
R(114)
is hydrogen, (C₁-C₈)-alkyl, (C₁-C₈)-perfluoroalkyl, (C₃-C₈)-alkenyl, (C₃-C₈)-cycloalkyl, phenyl or benzyl,
where the aromatics are not substituted or are substituted by 1 - 3 substituents selected from the group consisting of F, Cl, CF₃, methyl, methoxy and NR(119)R(120);
R(119) and R(120)
are hydrogen, (C₁-C₄)-alkyl or (C₁-C₄)-perfluoroalkyl;
R(115) and R(116) independently of one another are defined as R(114);
or
R(115) and R(116)
together are 4 or 5 methylene groups, of which one CH₂ group can be replaced by oxygen, S, NH, N-CH₃ or N-benzyl;
but where the constitution of U is nitrogen (N), YY is nitrogen (N) and Z is carbon (C) is
excluded,
R(101), R(102), R(103), R(104) and R(105)
independently of one another are hydrogen, F, Cl, Br, I, -C=N, X_{zoa}- (CH₂)_{zpa}- (C_{zqa}F_{2zqa+1}), R (110a) -SO_{zbm}, R(110b)R(110c)N-CO, R(111a)-CO- or R(112a)R(113a)N-SO₂-,
where the perfluoroalkyl group is straight-chain or branched,
X is oxygen, S or NR(114a);
R(114a)
is H or (C₁-C₃)-alkyl;
zoa is zero or 1;
zbm is zero, 1 or 2;
zpa is zero, 1, 2, 3 or 4;
zqa is 1, 2, 3, 4, 5, 6, 7 or 8;
R(110a), R(110b), R(111a) and R(112a)
independently of one another are (C₁-C₈)-alkyl, (C₃-C₈)-alkenyl, -C_{zn}H_{2zn}-R(115a) or (C₁-C₈)-perfluoroalkyl;
zn is zero, 1, 2, 3 or 4;
R(115a)
is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR(116a)R(117a);
R(116a) and R(117a)
are hydrogen, (C₁-C₄)-perfluoroalkyl or (C₁-C₄)-alkyl;
or
R(110b), R(111a) and R(112a)
are hydrogen;
R(110c) and R(113a)
independently are hydrogen, (C₁-C₄)-perfluoroalkyl or (C₁-C₄)-alkyl;
or
R(110b) and R(110c) and R(112a) and R(113a) together are 4 or 5 methylene groups, of which one CH₂ group can be replaced by oxygen, sulfur, NH, N-CH₃ or N-benzyl;
or
R(101), R(102), R(103), R(104), R(105) independently of one another are (C₁-C₈)-alkyl, - C_{zal}H_{2zal}R(118a) or (C₃-C₈)-alkenyl, zal is zero, 1, 2, 3 or 4;
R(118a)
is (C₃-C₈)-cycloalkyl, phenyl, biphenylyl or naphthyl,
where the aromatics are not substituted or are substituted by 1 - 3 substituents from the group consisting of F, Cl, CF₃, methyl, methoxy or NR(119a)R(119b);
R(119a) and R(119b)
are hydrogen, (C₁-C₄)-alkyl or (C₁-C₄)-perfluoroalkyl;
or
R(101), R(102), R(103), R(104), R(105)
independently of one another are (C₁-C₉)-heteroaryl, which is linked via C or N and which is unsubstituted or substituted by 1 - 3 substituents from the group consisting of F, Cl, CF₃, CH₃, methoxy, hydroxyl, amino, methylamino and dimethylamino;
or
R(101), R(102), R(103), R(104), R(105) independently of one another are -C≡C-R(193);
R(193) is phenyl which is not substituted or is substituted by 1 - 3 substituents from the group consisting of F, Cl, CF₃, methyl, methoxy or NR(194)R(195);
R(194) and R(195)
are hydrogen or CH₃;
or
R(101), R(102), R(103), R(104), R(105) independently of one another are -Y-para-C₆H₄- (CO)_{zh}- (CHOH)_{zi}-(CH₂)_{zj}-(CHOH) _{zk}-R(123), -Y-meta-C₆H₄- (CO)_{zad}- (CHOH)_{zae}- (CH₂)_{zaf}- (CHOH)_{zag}-R(124)
or
-Y-ortho-C₆H₄- (CO)_{zah}- (CHOH)_{zao}- (CH₂)_{zap}- (CHOH)_{zak}-R(125);
Y is oxygen, -S- or -NR(122d)-;
zh, zad, zah
independently are zero or 1;
zi, zj, zk, zae, zaf, zag, zao, zap and zak
independently are zero, 1, 2, 3 or 4;
but where in each case
zh, zi and zk are not simultaneously zero, zad, zae and zag are not simultaneously zero, and
zah, zao and zak are not simultaneously zero,
R(123), R(124) R(125) and R(122d)
independently are hydrogen or (C₁-C₃)-alkyl;
or
R(101), R(102), R(103), R(104) and R(105) independently of one another are SR(129), - OR(130), -NR(131)R(132) or -CR(133)R(134)R(135); R(129), R(130), R(131) and R(133)
independently are -C_{zab}H_{2zab}- (C₁-C₉) -heteroaryl,
which is unsubstituted or substituted by 1 - 3 substituents from the group consisting of F, Cl, CF₃, CH₃, methoxy, hydroxyl, amino, methylamino and dimethylamino;
zab is zero, 1 or 2;
R(132), R(134) and R(135)
independently of one another are defined as R(129) or are hydrogen, (C₁-C₄)-alkyl or (C₁-C₄)-perfluoroalkyl;
or
R(101), R(102), R(103), R(104) and R(105) independently of one another are -W-para-(C₆H₄)-R(196), -W-meta-(C₆H₄)-R(197) or -W-ortho-(C₆H₄)-R(198);
R(196), R(197) and R(198)
independently are (C₁-C₉)-heteroaryl,
which is linked via C or N and which is unsubstituted or substituted by 1 to 3 substituents from the group consisting of F, Cl, CF₃, CH₃, methoxy, hydroxyl, amino, methylamino, dimethylamino and benzyl;
W is oxygen, S or NR(136)-;
R(136)
is hydrogen or (C₁-C₄)-alkyl;
or
R(101), R(102), R(103), R(104) and R(105) independently of one another are R(146)X(1a) -;
X(1a)
is oxygen, S, NR(147), (D=O)A-, NR(148)C=MN⁽*⁾R(149)-;
M is oxygen or sulfur;
A is oxygen or NR(150);
D is C or SO;
R(146)
is (C₁-C₈)-alkyl, (C₃-C₈)-alkenyl, (CH₂)_{zbz}C_{zdz}F_{2zdz+1} or -C_{zxa}H_{2zxa}-R(151) ;
zbz is zero or 1;
zdz is 1, 2, 3, 4, 5, 6 or 7;
zxa is zero, 1, 2, 3 or 4;
R(151)
is (C₃-C₈)-cycloalkyl, phenyl, biphenylyl or naphthyl,
where the aromatics are not substituted or are substituted by 1 - 3 substituents from the group consisting of F, Cl, CF₃, methyl, methoxy and NR(152)R(153); R(152) and R(153)
are hydrogen, (C₁-C₄)-alkyl or (C₁-C₄)-perfluoroalkyl;
R(147), R(148) and R(150)
independently are hydrogen, (C₁-C₄)-alkyl, (C₁-C₄)-perfluoroalkyl;
R(149) is defined as R(146),
or
R(146) and R(147), or R(146) and R(148)
together are 4 or 5 methylene groups, of which one CH₂ group can be replaced by oxygen, sulfur, NH, N-CH₃ or N-benzyl;
where A and N⁽*⁾ are bonded to the phenyl nucleus of the alkanoyl parent structure;
or
R(101), R(102), R(103), R(104) and R(105) independently of one another are -SR(164), - OR(165), -NHR(166), -NR(167)R(168), - CHR(169)R(170), -CR(154)R(155)OH, -C≡CR(156), -CR(158)=CR(157) or -[CR(159)R(160)]_{zu}-(C=O)-[CR(161)R(162)]_{zv}-R(163); R(164), R(165), R(166), R(167), R(169)
identically or differently are -(CH₂)_{zy}-(CHOH)_{zz}-(CH₂)_{zaa}-(CHOH)_{zt}-R(171) or -(CH₂)_{zab}-O-(CH₂-CH₂O)_{zac}-R(172) ;
R(171) and R(172)
are hydrogen or methyl;
zu is 1, 2, 3 or 4;
zv is zero, 1, 2, 3 or 4;
zy, zz, zaa, zab, zac
identically or differently are zero, 1, 2, 3 or 4;
zt is 1, 2, 3 or 4;
R(168), R(170), R(154), R(155)
identically or differently are hydrogen or (C₁-C₆)-alkyl,
or
R(169) and R(170), or R(154) and R(155)
together with the carbon atom carrying them are a (C₃-C₈)-cycloalkyl;
R(163)
is hydrogen, (C₁-C₆)-alkyl, (C₃-C₈)-cycloalkyl Or -C_{zeb}H_{2zeb}-R(173) ;
zeb is zero, 1, 2, 3 or 4;
R(156), R(157) and R(173)
independently are phenyl which is unsubstituted or is substituted by 1 - 3 substituents from the group consisting of F, Cl, CF₃, methyl, methoxy and NR(174)R(175);
R(174) and R(175)
are hydrogen or (C₁-C₄)-alkyl ;
or
R(156), R(157) and R(173)
independently are (C₁-C₉)-heteroaryl,
which is unsubstituted or substituted as phenyl;
R(158), R(159), R(160), R(161) and R(162)
are hydrogen or methyl,
or
R(101), R(102), R(103), R(104), R(105) independently of one another are R(176)-NH-SO₂-; R(176)
is R(177)R(178)N-(C=Y')-;
Y' is oxygen, S or N-R(179);
R(177) and R(178)
identically or differently are hydrogen, (C₁-C₈)-alkyl, (C₃-C₆)-alkenyl or - C_{zfa}H_{2zfa}-R(180);
zfa is zero, 1, 2, 3 or 4;
R(180)
is (C₅-C₇)-cycloalkyl or phenyl, which is unsubstituted or substituted by 1 - 3 substituents selected from the group consisting of F, Cl, CF₃, methoxy or (C₁-C₄)-alkyl;
or
R(177) and R(178)
together are 4 or 5 methylene groups, of which one CH₂ group can be replaced by oxygen, sulfur, NH, N-CH₃ or N-benzyl;
R(179)
is defined as R(177) or is amidine,
or
R(101), R(102), R(103), R(104), R(105) independently of one another are NR(184a)R(185), OR(184b), SR(184c) or -C_{znx}H_{2znx}-R(184d); znx is zero, 1, 2, 3 or 4;
R(184d) is (C₃-C₇)-cycloalkyl or phenyl, which is not substituted or substituted by 1 - 3 substituents from the group consisting of F, Cl, CF₃, methyl, methoxy and NR(116k)R(117k); R(116k) and R(117k)
are hydrogen or C₁-C₄-alkyl;
R(184a), R(184b), R(184c), R(185)
independently of one another are hydrogen, (C₁-C₈) -alkyl, (C₁-C₈) -perfluoroalkyl or (CH₂)_{zao}-R(184g);
zao is zero, 1, 2, 3 or 4;
R(184g)
is (C₃-C₇)-cycloalkyl or phenyl, which is not substituted or substituted by 1 - 3 substituents from the group consisting of F, Cl, CF₃, methyl, methoxy and NR(184u)R(184v); R(184u) and R(184v)
are hydrogen or C₁-C₄-alkyl;
or
R(184a) and R(185)
together are 4 or 5 methylene groups, of which one CH₂ group can be replaced by oxygen, sulfur, NH, N-CH₃ or N-benzyl;
and their pharmaceutically tolerable salts;
u) benzoylguanidines of the formula I 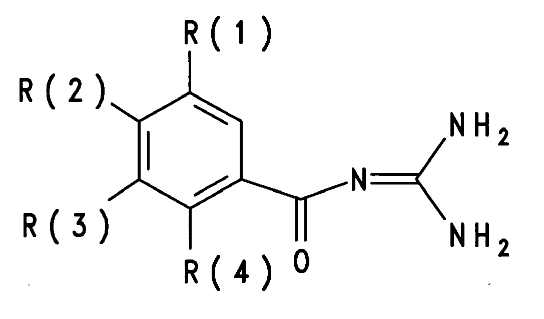 in which:
R(1) is H, F, Cl, Br, I, CN, NO₂, (C₁-C₈)-alkyl, (C₃-C₈) - cycloalkyl or
Xₐ- (CH₂)_{b}- (CF₂) c-CF₃;
X is oxygen, S or NR(5);
a is zero or 1;
b is zero, 1 or 2;
c is zero, 1, 2 or 3;
R(5) is H, (C₁-C₄-alkyl or -C_{d}H_{2d}R (6) ;
d is zero, 1, 2, 3 or 4;
R(6) is (C₃-C₈)-cycloalkyl, phenyl, biphenylyl or naphthyl,
where the aromatics are not substituted or are substituted by 1 to 3 substituents selected from the group consisting of F, Cl, CF₃, methyl, methoxy and NR(7)R(8); R(7) and R(8)
independently are H or (C₁-C₄) -alkyl;
or
R(1) is -SR(10), -OR(10) or -CR(10)R(11)R(12); R(10) is -C_{f}H_{2f}- (C₃-C₈) -cycloalkyl, -(C₁-C₉)-heteroaryl or phenyl,
where the aromatic systems are unsubstituted or substituted by one to 3 substituents selected from the group consisting of F, Cl, CF₃, CH₃, methoxy, hydroxyl, amino, methylamino and dimethylamino;
f is zero, 1 or 2;
R(11) and R(12)
independently of one another are defined as
R(10) or are hydrogen or (C₁-C₄)-alkyl;
or
R(1) is phenyl, naphthyl, biphenylyl or (C₁-C₉) - heteroaryl,
the latter linked via C or N,
and which are unsubstituted or substituted by 1 to 3 substituents selected from the group consisting of F, Cl, CF₃, CH₃, methoxy, hydroxyl, amino, methylamino and
dimethylamino;
or
R(1) is -SR(13), -OR(13), -NHR(13), -NR(13)R(14), - CHR(13)R(15),
-C[R(15)R(16)]OH, -C≡CR(18), -C[R(19)]=CR(18), - [CR(20)R(21)]ₖ-(CO)-[CR(22)R(23)R(24)];
R(13) and R(14)
identically or differently are -(CH₂)_{g}-(CHOH)ₕ-(CH₂)ᵢ-(CHOH)ⱼ-R(17),
R(17) is hydrogen or methyl; - (CH₂)g-O-(CH₂-CH₂O)ₕ-R(24),
g, h, i
identically or differently are zero, 1, 2, 3 or 4;
j is 1, 2, 3 or 4;
R(15) and R(16)
identically or differently are hydrogen, (C₁-C₆)-alkyl or together with the carbon atom carrying them are a (C₃-C₈)-cycloalkyl;
R(18) is phenyl,
which is unsubstituted or substituted by 1 to 3 substituents selected from the group consisting of F, Cl, CF₃, methyl, methoxy and NR(25)R(26) ;
R(25) and R(26)
are H or (C₁-C₄)-alkyl;
or
R(18) is (C₁-C₉)-heteroaryl,
which is unsubstituted or substituted as phenyl;
or
R(18) is (C₁-C₆)-alkyl,
which is unsubstituted or substituted by 1 to 3 OH;
or
R (18) is (C₃-C₈)-cycloalkyl;
R(19), R(20), R(21), R(22) and R(23)
are hydrogen or methyl;
k is zero, 1, 2, 3 or 4;
1 is zero, 1, 2, 3 or 4;
R(24) is H, (C₁-C₆)-alkyl, (C₃-C₈)-cycloalkyl or -CₘH₂ₘ-R(18) ;
m is 1, 2, 3 or 4;
R(2) and R(3)
independently of one another are defined as R(1);
R(4) is (C₁-C₃)-alkyl, F, Cl, Br, I, CN or -(CH₂)ₙ-(CF₂)ₒ-CF₃;
n is zero or 1;
o is zero, 1 or 2;
and their pharmaceutically tolerable salts;
v) acylguanidines of the formula I 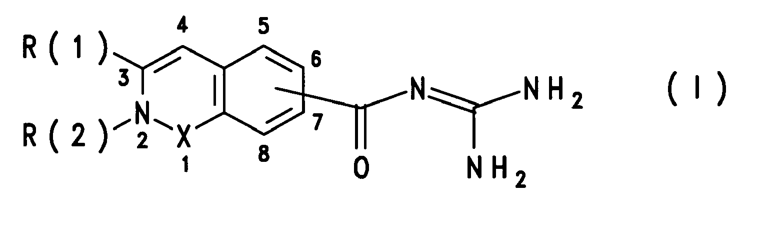 in which:
X is carbonyl, sulfonyl,
R(1) is H, (C₁-C₈)-alkyl,
unsubstituted or substituted by hydroxyl,
(C₃-C₈)-cycloalkyl, phenyl,
which is unsubstituted or substituted by 1 - 3 substituents from the group F, Cl, CF₃, CH₃, methoxy, hydroxyl, amino, methylamino or dimethylamino,
R(2) is H, (C₁-C₄)-alkyl,
and their pharmaceutically tolerable salts;
w) phenyl-substituted alkylcarboxylic acid guanidides, carrying perfluoroalkyl groups, of the formula I 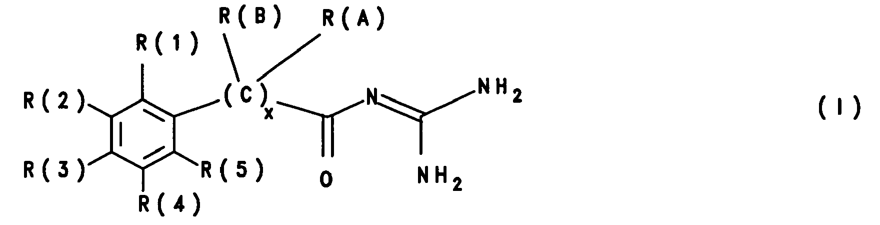 in which:
R(A) is hydrogen, F, Cl, Br, I, CN, OR(6), (C₁-C₈)-alkyl, (C₃-C₈)-cycloalkyl, Oᵣ(CH₂)ₐC_{b}F_{2b+1} or NR(7)R(8);
r is zero or 1;
a is zero, 1, 2, 3 or 4;
b is 1, 2, 3, 4, 5, 6, 7 or 8;
R(6) is hydrogen, (C₁-C₈)-alkyl, (C₃-C₈)-alkenyl, (C₃-C₈)-cycloalkyl, phenyl or benzyl,
where the aromatics are not substituted or are substituted by 1 - 3 substituents selected from the group consisting of F, Cl, CF₃, methyl, methoxy and NR(9)R(10);
R(9) and R(10)
are H, (C₁-C₄)-alkyl or (C₁-C₄)-perfluoroalkyl;
R(7) and R(8)
independently of one another are defined as R(6);
R(B) independently is defined as R(A);
X is 1, 2 or 3;
R(1) is hydrogen, (C₁-C₈)-alkyl, (C₃-C₈)-cycloalkyl, - Oₜ(CH₂)_{d}CₑF₂ₑ₊₁, F, Cl, Br, I or CN;
t is zero or 1;
d is zero, 1, 2, 3 or 4;
e is 1, 2, 3, 4, 5, 6, 7 or 8;
R(2), R(3), R(4) and R(5)
independently of one another are defined as R(1); but with the condition
that at least one of the substituents R(1), R(2), R(3), R(4), R(5), R(A) and R(B) is an -Oₜ(CH₂)_{d}CₑF₂ₑ₊₁ or an Oᵣ(CH₂)C_{b}F_{2b+1} group,
and their pharmaceutically tolerable salts;
x) heteroaroylguanidines of the formula I 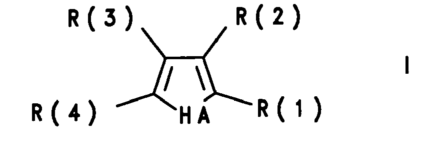 in which:
HA is SOₘ, O or NR(5);
m is zero, 1 or 2;
R(5) is hydrogen, (C₁-C₈)-alkyl or - CₐₘH₂ₐₘR (81) ;
am is zero, 1 or 2;
R(81) is (C₃-C₈)-cycloalkyl or phenyl,
which is not substituted or is substituted by 1 - 3 substituents selected from the group consisting of F, Cl, CF₃, methyl, methoxy and NR(82)R(83);
R(82) and R(83)
is H or CH₃;
or
R(81) is (C₁-C₉)-heteroaryl, which is linked via C or N and which is unsubstituted or substituted by 1 - 3 substituents selected from the group consisting of F, Cl, CF₃, CH₃, methoxy, hydroxyl, amino, methylamino and dimethylamino;
one of the two substituents R(1) and R(2) is -CO-N=C(NH₂)₂;
and the other in each case
is hydrogen, F, Cl, Br, I, (C₁-C₃)-alkyl, -OR(6), CᵣF₂ᵣ₊₁, -CO-N=C(NH₂)₂ or -NR(6)R(7);
R(6) and R(7)
independently are hydrogen or (C₁-C₃)-alkyl;
r is 1, 2, 3 or 4;
R(3) and R(4)
independently of one another are hydrogen, F, Cl, Br, I, -C=N, X-(CH₂)ₚ-(C_{q}-F_{2q+1}), R(8)-SO_{bm}, R(9)R(10)N-CO, R(11)-CO- or R(12)R(13)N-SO₂-, where the perfluoroalkyl group is straight-chain or branched,
X is oxygen, S or NR(14);
R(14) is H or (C₁-C₃)-alkyl;
bm is zero, 1 or 2;
p is zero, 1 or 2;
q is zero, 1, 2, 3, 4, 5 or 6;
R(8), R(9), R(11) and R(12)
independently are (C₁-C₈)-alkyl, (C₃-C₆)-alkenyl, -CₙH₂ₙ-R(15), CF₃;
n is zero, 1, 2, 3 or 4;
R(15) is (C₃-C₇)-cycloalkyl or phenyl;
which is not substituted or is substituted by 1 - 3 substituents selected from the group consisting of F, Cl, CF₃, methyl, methoxy or NR(16)R(17) ;
R(16) and R(17)
are H or C₁-C₄-alkyl;
or
R(9), R(11) and R(12)
are H;
R(10) and R(13)
independently are H or (C₁-C₄)-alkyl;
or
R(9) and R(10), and R(12) and R(13)
together are 4 or 5 methylene groups, of which one CH₂ group can be replaced by oxygen,
S, NH, N-CH₃ or N-benzyl,
or
R(3) and R(4)
independently of one another are (C₁-C₈)-alkyl or - CₐₗH₂ₐₗR(18) ;
al is zero, 1 or 2;
R(18) is (C₃-C₈)-cycloalkyl or phenyl;
which is not substituted or is substituted by 1 - 3 substituents selected from the group consisting of F, Cl, CF₃, methyl, methoxy and NR(19)R(20);
R(19) and R(20)
are H or CH₃;
or
R(3) and R(4)
independently of one another are (C₁-C₉)-heteroaryl,
which is linked via C or N and which is unsubstituted or substituted by 1 - 3 substituents selected from the group consisting of F, Cl, CF₃, CH₃, methoxy, hydroxyl, amino, methylamino and dimethylamino;
or
R(3) and R(4) independently of one another are 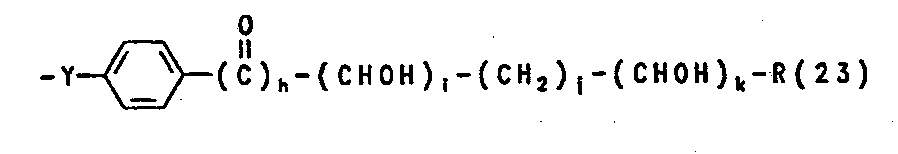 or 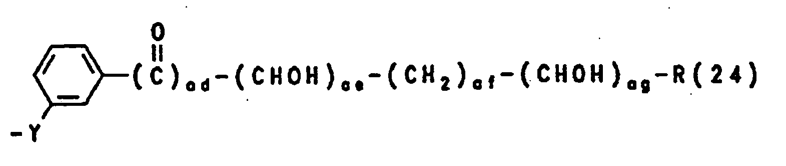 or 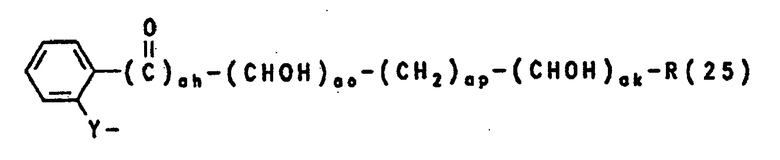 Y is oxygen, -S- or -NR(22)-;
h, ad, ah independently are zero or 1;
i, j, k, ae, af, ag, ao, ap and ak independently are zero, 1, 2, 3, 4,
but where in each case
h, i and k are not simultaneously zero,
ad, ae and ag are not simultaneously zero, ah, ao and ak are not simultaneously zero,
R(23), R(24) R(25) and R(22)
independently are hydrogen or (C₁-C₃)-alkyl;
or
R(3) and R(4)
independently of one another are hydrogen, F, Cl, Br, I, CN, (C₁-C₈)-alkyl, (C₁-C₈)-perfluoroalkyl, (C₃-C₈)-alkenyl or -C_{g}H_{2g}R(26) ;
g is zero, 1, 2, 3 or 4;
R(26) is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR(27)R(28); R(27) and R(28)
are H, (C₁-C₄)-alkyl or (C₁-C₄) - pefluoroalkyl;
or
R(3) and R(4) independently of one another are SR(29), -OR(30), -NR(31)R(32) or -CR(33)R(34)R(35);
R(29), R(30), R(31) and R(33)
independently are -CₐH₂ₐ-(C₁-C₉)-heteroaryl, which is unsubstituted or substituted by 1 - 3 substituents selected from the group consisting of F, Cl, CF₃, CH₃, methoxy, hydroxyl, amino, methylamino and dimethylamino;
a is zero, 1 or 2;
R(32), R(34) and R(35)
independently of one another are defined as R(29) or are hydrogen, (C₁-C₄)-alkyl or (C₁-C₄)-perfluoroalkyl;
or
R(3) and R(4)
independently of one another are 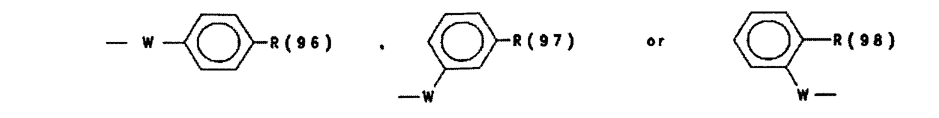 R(96), R(97) and R(98)
independently are (C₁-C₉)-heteroaryl, which is linked via C or N and which is unsubstituted or substituted by 1 to 3 substituents selected from the group consisting of F, Cl, CF₃, CH₃, methoxy, hydroxyl, amino, methylamino, dimethylamino or benzyl;
W is oxygen, S or NR(36)-;
R(36) is H or (C₁-C₄)-alkyl;
or
R(3) and R(4)
independently of one another are R(37)-SO_{cm} or R(38)R(39)N-SO₂-;
cm is 1 or 2;
R(37) is (C₁-C₈)-alkyl, (C₁-C₈)-perfluoroalkyl,
(C₃-C₈)-alkenyl or -CₛH₂ₛR(40);
s is zero, 1, 2, 3 or 4;
R(40) is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR(41)R(42); R(41) and R(42)
are H, (C₁-C₄)-alkyl or (C₁-C₄)-perfluoroalkyl;
R(38) is H, (C₁-C₈)-alkyl, (C₁-C₈)-perfluoroalkyl, (C₃-C₈)-alkenyl or -C_{w}H_{2w}-R(43);
w is zero, 1, 2, 3 or 4;
R (43) is (C₃-C₈) -cycloalkyl, phenyl, biphenylyl or naphthyl, where the aromatics are not substituted or are substituted by 1 - 3 substituents selected from the group consisting of F, Cl, CF₃, methyl, methoxy and NR(44)R(45);
R(44) and R(45)
are H, (C₁-C₄) -alkyl or (C₁-C₄)-perfluoroalkyl;
R(39) is H, (C₁-C₉)-alkyl or (C₁-C₄)-perfluoroalkyl;
or
R(38) and R(39)
together are 4 or 5 methylene groups, of which one CH₂ group can be replaced by oxygen,
S, NH, N-CH₃ or N-benzyl;
or
R(3) and R(4) independently of one another are R(46)X(1)-;
X(1) is oxygen, S, NR(47), (D=O)A-,
NR(48)C=MN⁽*⁾R(49)-,
M is oxygen or S;
A is oxygen or NR(50);
D is C or SO;
R(46) is (C₁-C₈)-alkyl, (C₃-C₈)-alkenyl, (CH₂)_{b}C_{d}F_{2d+1} or -CₓH₂ₓ-R(51) ;
b is zero or 1;
d is 1, 2, 3, 4, 5, 6 or 7;
x is zero, 1, 2, 3 or 4;
R(51) is (C₃-C₈)-cycloalkyl, phenyl,
biphenylyl or naphthyl,
where the aromatics are not substituted or are substituted by 1 - 3 substituents selected from the group consisting of F, Cl, CF₃, methyl, methoxy and NR(52)R(53); R(52) and R(53)
are H, (C₁-C₄)-alkyl or (C₁-C₄)-perfluoroalkyl;
R(47), R(48) and R(50) independently are hydrogen, (C₁-C₄)-alkyl or (C₁-C₄)-perfluoroalkyl:
R(49) is defined as R(46);
or
R(46) and R(47), or R(46) and R(48)
together are 4 or 5 methylene groups, of which one CH₂ group can be replaced by oxygen, S, NH, N-CH₃ or N-benzyl,
where A and N⁽*⁾ are bonded to the phenyl nucleus of the benzoylguanidine parent structure;
or
R(3) and R(4) independently of one another are -SR(64), -OR(65), -NHR(66), -NR(67)R(68), -CHR(69)R(70), -C(OH)R(54)R(55), -C=CR(56), -CR(58)=CHR(57), -[CR(59)R(60)]ᵤ-(CO)-[CR(61)R(62)]ᵥ-R(63);
R(64), R(65), R(66), R(67) and R(69) identically or differently are -(CH₂)_{y}-(CHOH)_{z}- (CH₂)ₐₐ-(CH₂OH)ₜ-R(71) or -(CH₂)_{ab}-O-(CH₂-CH₂O)_{ac}-R(72),
R(71) and R(72)
are hydrogen or methyl;
u is 1, 2, 3 or 4;
v is zero, 1, 2, 3 or 4;
y, z, aa
identically or differently are zero, 1, 2, 3 or 4;
t is 1, 2, 3 or 4;
R(68), R(70), R(54) and R(55)
identically or differently are hydrogen, (C₁-C₆)-alkyl;
or
R(69) and R(70), or R(54) and R(55)
together with the carbon atom carrying them are a (C₃-C₈)-cycloalkyl;
R(63)
is H, (C₁-C₆)-alkyl, (C₃-C₈)-cycloalkyl or - CₑH₂ₑ-R (73) ;
e is zero, 1, 2, 3 or 4;
R(56), R(57) and R(73)
independently are phenyl, which are unsubstituted or substituted by 1 - 3 substituents selected from the group consisting of F, Cl, CF₃, methyl, methoxy and NR(74)R(75);
R(74) and R(75) are H or (C₁-C₄)-alkyl;
or
R(56), R(57) and R(73)
independently are (C₁-C₉)-heteroaryl,
which is unsubstituted or substitued as phenyl;
R(58), R(59), R(60), R(61) and R(62)
are hydrogen or methyl,
or
R(3) and R(4)
independently of one another are R(76)-NH-SO₂-;
R(76) is R(77)R(78)N-(C=Y')-;
Y' is oxygen, S or N-R(79);
R(77) and R(78)
identically or differently are H, (C₁-C₈)-alkyl, (C₃-C₆)-alkenyl, -C_{f}H_{2f}-R(80);
f is zero, 1, 2, 3 or 4;
R(80) is (C₅-C₇)-cycloalkyl or phenyl,
which is unsubstituted or substituted by 1 - 3 substituents selected from the group consisting of F, Cl, CF₃, methoxy and (C₁-C₄)-alkyl;
or
R(77) and R(78)
together are 4 or 5 methylene groups, of which one CH₂ group can be replaced by oxygen, S, NH, N-CH₃ or N-benzyl,
R(79)
is defined as R(77) or is amidine;
or
R(3) and R(4)
independently of one another are NR(84)R(85); R(84) and R(85)
independently of one another are H, (C₁-C₄)-alkyl, or together are 4 or 5 methylene groups,
of which one CH₂ group can be replaced by oxygen, S, NH, N-CH₃ or N-benzyl; or of which one or two CH₂ groups can be replaced by CH-C_{dm}H_{2dm+1},
and their pharmaceutically tolerable salts;
y) bicyclic heteroaroylguanidines of the formula I 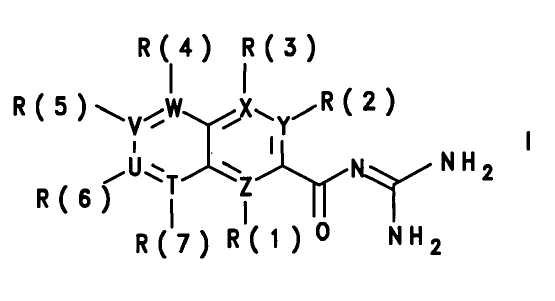 in which:
T, U, V, W, X, Y and Z
independently of one another are nitrogen or carbon;
but with the restriction
that X and Z are not simultaneously nitrogen, and that T, U, V, W, X, Y and Z carry no substituents if they are nitrogen,
and that no more than four of them are simultaneously nitrogen,
R(1) and R(2)
independently of one another are hydrogen, F, Cl, Br, I, (C₁-C₃)-alkyl, (C₁-C₃)-perfluoroalkyl, OR(8), NR(8)R(9) or C(=O)N=C(NH₂)₂;
R(8) and R(9)
independently of one another are hydrogen or (C₁-C₃)-alkyl,
or
R(8) and R(9)
together are 4 or 5 methylene groups, of which one CH₂ group can be replaced by oxygen, S, NH, N-CH₃ or N-benzyl;
R(3), R(4), R(5), R(6) and R(7) independently of one another are hydrogen, F, Cl, Br, I, -C≡N, Xₖ-(CH₂)ₚ-(CqF_{2q+1}), R(10a)-SO_{bm}, R(10b)R(10c)N-CO, R(11)-CO- or R(12)R(13)N-SO₂-,
where the perfluoroalkyl group is straight-chain or branched;
X is oxygen, S or NR(14);
R(14) is H or (C₁-C₃)-alkyl;
bm is zero, 1 or 2;
p is zero, 1 or 2;
k is zero or 1;
q 1, 2, 3, 4, 5 or 6;
R(10a), R(10b), R(11) and R(12)
independently of one another are (C₁-C₈) - alkyl, (C₃-C₆)-alkenyl, -CₙH₂ₙ-R(15) or (C₁-C₈) - perfluoroalkyl;
n is zero, 1, 2, 3 or 4;
R(15) is (C₃-C₇)-cycloalkyl or phenyl, which is not substituted or is substituted by 1 - 3 substituents selected from the group consisting of F, Cl, CF₃, methyl, methoxy and NR(16)R(17);
R(16) and R(17)
are H or C₁-C₄-alkyl;
or
R(10b), R(11) and R(12)
are hydrogen;
R(10c) and R(13)
independently are hydrogen or (C₁-C₄)-alkyl;
or
R(10b) and R(10c) and R(12) and R(13) together are 4 or 5 methylene groups, of which one CH₂ group can be replaced by oxygen, sulfur, NH, N-CH₃ or N-benzyl;
or
R(3), R(4), R(5), R(6) and R(7)
independently of one another are (C₁-C₈)-alkyl, - CₐₗH₂ₐₗR(18) or (C₃-C₈)-alkenyl;
al is zero, 1 or 2;
R(18) is (C₃-C₈)-cycloalkyl, phenyl, biphenylyl or naphthyl, where the aromatics are not substituted or are substituted by 1 - 3 substituents selected from the group consisting of F, Cl, CF₃, methyl, methoxy and NR(19a)R(19b);
R(19a) and R(19b)
are hydrogen, (C₁-C₄)-alkyl or (C₁-C₄) - perfluoroalkyl;
or
R(3), R(4), R(5), R(6) and R(7) independently of one another are (C₁-C₉)-heteroaryl, which is linked via C or N and which is unsubstituted or substituted by 1 - 3 substituents from the group consisting of F, Cl, CF₃, CH₃, methoxy, hydroxyl, amino, methylamino or dimethylamino;
or
R(3), R(4), R(5), R(6) and R(7)
independently of one another are 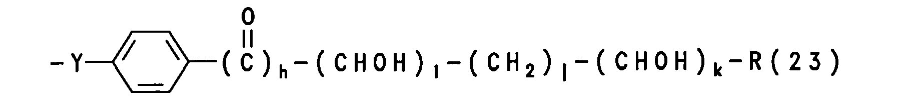 or 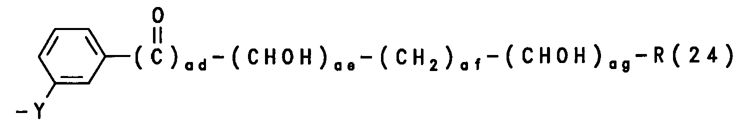 or 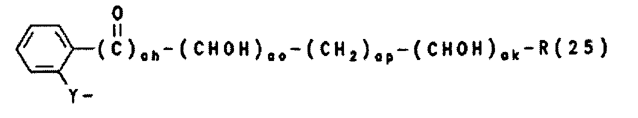 Y is oxygen, -S- or -NR(22)-;
h, ad, ah
independently of one another are zero or 1;
i, j, k, ae, af, ag, ao, ap and ak independently of one another are zero, 1, 2, 3 or 4;
but where in each case
h, i and k are not simultaneously zero, ad, ae and ag are not simultaneously zero, and
ah, ao and ak are not simultaneously zero,
R(23), R(24) R(25) and R(22)
independently of one another are hydrogen or (C₁-C₃)-alkyl;
or
R(3), R(4), R(5), R(6) and R(7) independently of one another are SR(29), -OR(30), -NR(31)R(32) or -CR(33)R(34)R(35);
R(29), R(30), R(31) and R(33)
independently of one another are -CₐH₂ₐ-(C₁-C₉) - heteroaryl, which is unsubstituted or substituted by 1 - 3 substituents selected from the group consisting of F, Cl, CF₃, CH₃, methoxy, hydroxyl, amino, methylamino and dimethylamino;
a is zero, 1 or 2;
R(32), R(34) and R(35)
independently of one another are defined as R(29) or are hydrogen, (C₁-C₄)-alkyl or (C₁-C₄)-perfluoroalkyl;
or
R(3), R(4), R(5), R(6) and R(7)
independently of one another are 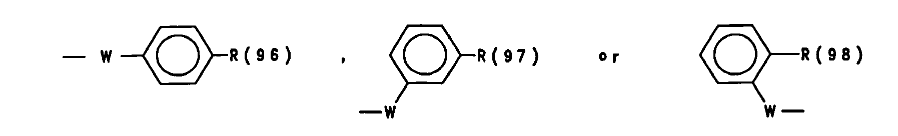 R(96), R(97) and R(98) independently of one another are (C₁-C₉)-heteroaryl, which is linked via C or N and which is unsubstitued or substituted by 1 to 3 substituents from the group consisting of F, Cl, CF₃, CH₃, methoxy, hydroxyl, amino, methylamino, dimethylamino or benzyl;
W is oxygen, S or NR(36)-;
R(36) is H or (C₁-C₄)-alkyl;
or
R(3), R(4), R(5), R(6) and R(7)
independently of one another are R(46)X(1)-; X(1) is oxygen, S, NR(47), (D=O)A- or NR(48)C=MN⁽*⁾R(49)-;
M is oxygen or sulfur;
A is oxygen or NR(50);
D is C or SO;
R(46) is (C₁-C₈)-alkyl, (C₃-C₈)-alkenyl, (CH₂)_{b}C_{d}F_{2d+1} or -CₓH₂ₓ-R(51) ;
b is zero or 1;
d is 1, 2, 3, 4, 5, 6 or 7;
x is zero, 1, 2, 3 or 4;
R (51) is (C₃-C₈) -cycloalkyl, phenyl, biphenylyl or naphthyl, where the aromatics are not substituted or are substituted by 1 - 3 substituents selected from the group consisting of F, Cl, CF₃, methyl, methoxy and NR(52)R(53);
R(52) and R(53)
are hydrogen, (C₁-C₄)-alkyl or (C₁-C₄)-perfluoroalkyl;
R(47), R(48) and R(50) independently
are hydrogen, (C₁-C₄)-alkyl or (C₁-C₄)-perfluoroalkyl;
R(49) is defined as R(46);
or
R(46) and R(47), or R(46) and R(48)
together are 4 or 5 methylene groups, of which one CH₂ groups can be replaced by oxygen, sulfur, NH, N-CH₃ or N-benzyl; where A and N⁽*⁾ are bonded to the phenyl nucleus
of the heteroaroylguanidine parent structure; or
R(3), R(4), R(5), R(6) and R(7) independently of one another are -SR(64), -OR(65), -NHR(66), -NR(67)R(68), -CHR(69)R(70) or -CR(54)R(55)OH, -C≡CR(56), -CR(58)=CR(57) or -[CR(59)R(60)]ᵤ-CO-[CR(61)R(62)]ᵥ-R(63) ;
R(64), R(65), R(66), R(67) and R(69) identically or differently are - (CH₂)_{y}- (CHOH)_{z}- (CH₂)ₐₐ- (CHOH) ₜ-R (71) or - (CH₂)_{ab}-O-(CH₂-CH₂O)_{ac}-R(72) ;
R(71) and R(72)
independently of one another are hydrogen or methyl;
u is 1, 2, 3 or 4;
v is zero, 1, 2, 3 or 4;
y, z, aa identically or differently are zero, 1, 2, 3 or 4;
t is 1, 2, 3 or 4;
R(68), R(70), R(54) and R(55)
identically or differently are hydrogen or (C₁-C₆)-alkyl;
or
R(69) and R(70), or R(54) and R(55)
together with the carbon atom carrying them are (C₃-C₈)-cycloalkyl;
R(63)
is hydrogen, (C₁-C₆)-alkyl, (C₃-C₈)-cycloalkyl or -CₑH₂ₑ-R(73);
e is zero, 1, 2, 3 or 4;
R(56), R(57) and R(73) independently are phenyl, which is unsubstituted or substituted by 1 - 3 substituents selected from the group consisting of F, Cl, CF₃, methyl, methoxy and NR(74)R(75);
R(74) and R(75)
are hydrogen or (C₁-C₄)-alkyl;
or
R(56), R(57) and R(73) independently are (C₁-C₉)-heteroaryl, which is unsubstituted or substituted as phenyl;
R(58), R(59), R(60), R(61) and R(62) are hydrogen or methyl;
or
R(3), R(4), R(5), R(6) and R(7) independently of one another are R(76)-NH-SO₂-;
R(76) is R(77)R(78)N-(C=Y')-;
Y' is oxygen, S or N-R(79);
R(77) and R(78)
identically or differently are hydrogen, (C₁-C₈)-alkyl, (C₃-C₆)-alkenyl or -C_{f}H_{2f}-R (80) ;
f is zero, 1, 2, 3 or 4;
R(80) is (C₅-C₇)-cycloalkyl or phenyl, which is unsubstituted or substituted by 1 - 3 substituents selected from the group consisting of F, Cl, CF₃, methoxy and (C₁-C₄) - alkyl;
or
R(77) and R(78)
together are 4 or 5 methylene groups, of which one CH₂ group can be replaced by oxygen, sulfur, NH, N-CH₃ or N-benzyl;
R(79) is defined as R(77) or is amidine;
or
R(3), R(4), R(5), R(6) and R(7)
independently of one another are NR(84a)R(85), OR(84b), SR(84c) or -CₙH₂ₙ-R(84d);
n is zero, 1, 2, 3 or 4;
R(84d) is (C₃-C₇)-cycloalkyl or phenyl, which is not substituted or is substituted by 1 - 3 substituents from the group consisting of F, Cl, CF₃, methyl, methoxy and NR(16)R(17);
R(16) and R(17)
are hydrogen or C₁-C₄-alkyl;
R(84a), R(84b), R(84c) and R(85) independently of one another are hydrogen, (C₁-C₈)-alkyl, (C₁-C₈)-perfluoroalkyl or (CH₂)ₐₓ-R(84g) ;
ax is zero, 1, 2, 3 or 4;
R(84g) is (C₃-C₇)-cycloalkyl or phenyl, which is not substituted or is substituted by 1 - 3 substituents selected from the group consisting of F, Cl, CF₃, methyl, methoxy and NR(84u)R(84v);
R(84u) and R(84v)
are hydrogen or C₁-C₄-alkyl;
or
R(84a) and R(85)
together are 4 or 5 methylene groups, of which one CH₂ group can be replaced by oxygen, sulfur, NH, N-CH₃ or N-benzyl,
and their pharmaceutically tolerable salts;
z) benzoylguanidines of the formula I 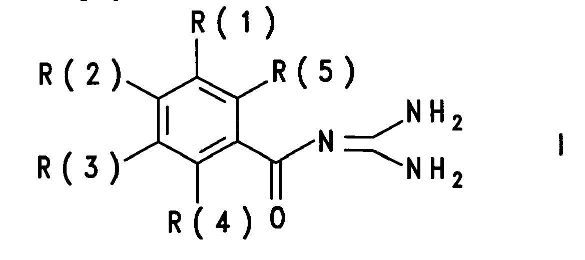 in which:
R(1) is R(6)-SOₘ;
m is zero, 1 or 2;
R(6) is perfluoroalkyl having 1, 2, 3, 4, 5 or 6 carbon atoms, which is straight-chain or branched;
R(2) and R(3)
independently of one another are hydrogen, F, Cl, Br, I, alkyl having 1, 2, 3 or 4 carbon atoms, alkoxy having 1, 2, 3 or 4 carbon atoms or phenoxy,
which is unsubstituted or substituted by 1 - 3 substituents selected from the group consisting of F, Cl, methyl and methoxy;
or
R(2) and R(3)
independently of one another are pyrrol-1-yl, pyrrol-2-yl or pyrrol-3-yl,
which is not substituted or is substituted by 1 to 4 substituents selected from the group
consisting of F, Cl, Br, I, CN, alkanoyl having 2, 3, 4, 5, 6, 7 or 8 carbon atoms, alkoxycarbonyl having 2, 3, 4, 5, 6, 7 or 8 carbon atoms, formyl, carboxyl, CF₃, methyl
and methoxy;
R(4) and R(5)
independently of one another are hydrogen, alkyl having 1, 2 or 3 carbon atoms, F, Cl, Br, I, CN, OR(7), NR(8)R(9) or -(CH₂)ₙ-(CF₂)ₒ-CF₃;
R(7), R(8) and R(9)
independently of one another are hydrogen or alkyl having 1, 2, 3 or 4 carbon atoms;
n is zero or 1;
o is zero, 1 or 2;
and their pharmacologically acceptable salts;
aa) phenyl-substituted alkenylcarboxylic acid guanidides, carrying perfluoroalkyl groups, of the formula I 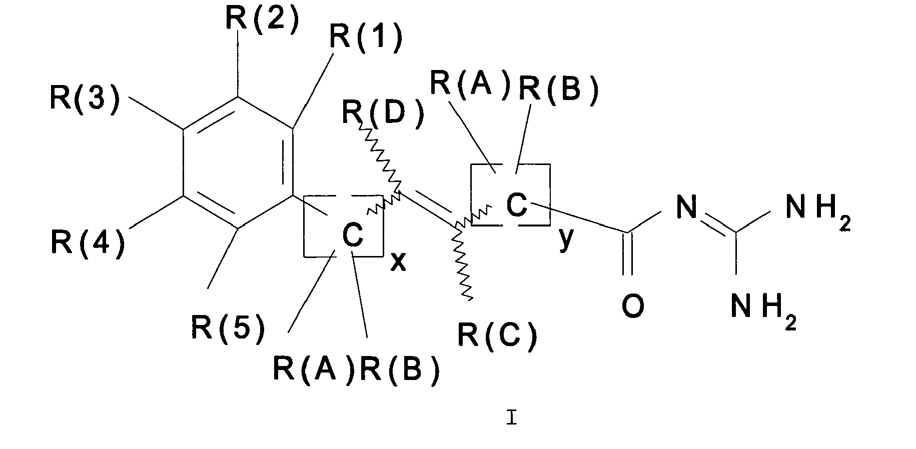 in which:
R(A) is hydrogen, F, Cl, Br, I, CN, OH, OR(6), (C₁-C₈)-alkyl, Oᵣ(CH₂)ₐC_{b}F_{2b+1}, (C₃-C₈) -cycloalkyl or NR(7)R(8);
r is zero or 1;
a is zero, 1, 2, 3 or 4;
b is 1, 2, 3, 4, 5, 6, 7 or 8;
R(6) is (C₁-C₈)-alkyl, (C₁-C₄)-perfluoroalkyl, (C₃-C₈)-alkenyl, (C₃-C₈)-cycloalkyl, phenyl or benzyl;
where the aromatics are not substituted or are substituted by 1 - 3 substituents from the group consisting of F, Cl, CF₃, methyl, methoxy and NR(9)R(10);
R(9) and R(10)
are H, (C₁-C₄)-alkyl or (C₁-C₄)-perfluoroalkyl;
R(7) and R(8)
independently of one another are defined as R(6);
or
R(7) and R(8)
together are 4 or 5 methylene groups, of which one CH₂ group can be replaced by oxygen, sulfur, NH, N-CH₃ or N-benzyl;
R(B) independently is defined as R(A);
x is zero, 1 or 2;
y is zero, 1 or 2;
R(C) is hydrogen, F, Cl, Br, I, CN, OR(12), (C₁-C₈)-alkyl, Oₚ(CH₂)_{f}C_{g}F_{2g+1} or (C₃-C₈)-cycloalkyl;
p is zero or 1;
f is zero, 1, 2, 3 or 4;
g is 1, 2, 3, 4, 5, 6, 7 or 8;
R(12)
is (C₁-C₈-alkyl, (C₁-C₄)-perfluoroalkyl, (C₃-C₈)-alkenyl, (C₃-C₈)-cycloalkyl, phenyl or benzyl;
where the aromatics phenyl or benzyl are not substituted or are substituted by 1 - 3 substituents from the group consisting of F, Cl, CF₃, methyl, methoxy and NR(13)R(14); R(13) and R(14)
independently of one another are H, (C₁-C₄)-alkyl or (C₁-C₄)-perfluoroalkyl;
R(D) independently is defined as R(C),
R(1) is hydrogen, (C₁-C₈)-alkyl, -Oₜ(CH₂)_{d}CₑF₂ₑ₊₁, (C₃-C₈)-cycloalkyl, F, Cl, Br, I or CN;
t is zero or 1;
d is zero, 1, 2, 3 or 4;
e is 1, 2, 3, 4, 5, 6, 7 or 8;
R(2), R(3), R(4) and R(5)
independently of one another are defined as R(1); but with the condition that at least one of the substituents R(A), R(B), R(C), R(D), R(1), R(2), R(4) or R(5) is a Oᵣ(CH₂)ₐC_{b}F_{2b+1}, Op(CH_{2)f}C_{g}F_{2g+1} or Oₜ(CH₂)_{d}CₑF₂ₑ₊₁ group and R (3) is not a Oₜ(CH₂)_{d}CeF₂ₑ₊₁ group:
and their pharmaceutically tolerable salts;
ab) ortho-amino-substituted benzoylguanidines of the formula I 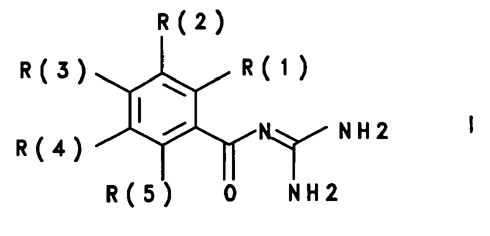 in which:
R(1) is NR(50)R(6),
R(50) and R(6)
independently of one another are hydrogen, (C₁-C₈)-alkyl or (C₁-C₈)-perfluoroalkyl;
R(2), R(3), R(4) and R(5)
independently of one another are R(10)-SOₐ-, R(11)R(12)N-CO-, R(13)-CO- or R(14)R(15)N-SO₂-;
a is zero, 1 or 2,
R(10), R(11), R(12), R(13), R(14) and R(15)
independently of one another are (C₁-C₈)-alkyl, (C₁-C₈)-perfluoroalkyl, (C₃-C₆)-alkenyl or -C_{ab}H_{2ab}-R(16) ;
ab is zero, 1, 2, 3 or 4;
R(16) is (C₃-C₇)-cycloalkyl, phenyl, which is not substituted or is substituted by 1 - 3 substituents selected from the group consisting of F, Cl, CF₃, methyl, methoxy and NR(17)R(18);
R(17) and R(18)
independently of one another are H, CF₃ or (C₁-C₄)-alkyl;
or
R(11), R(12), and also R(14) and R(15) together are 4 or 5 methylene groups, of which one CH₂ group can be replaced by oxygen, S, NH, N-CH₃ or N-benzyl;
or
R(11), R(12), R(14) and R(15)
independently of one another are hydrogen;
or
R(2), R(3), R(4) and R(5)
independently of one another are SR(21), -OR(22), -NR(23)R(24) or -CR(25)R(26)R(27);
R(21), R(22), R(23) and R(25) independently of one another are -C_{b}H_{2b}-(C₁-C₉)-heteroaryl, which is unsubstituted or substituted by 1 - 3 substituents selected from the group consisting of F, Cl, CF₃, CH₃, methoxy, hydroxyl, amino, methylamino and dimethylamino;
b is zero, 1 or 2;
R(24), R(26) and R(27)
independently of one another are hydrogen, (C₁-C₄)-alkyl or (C₁-C₉)-perfluoroalkyl;
or
R(2), R(3), R(4) and R(5)
independently of one another are hydrogen, F, Cl, Br, I, CN, -(Xa)_{dg}-C_{da}H_{2da+1}, -(Xb)_{dh}-(CH₂)_{db}-C_{de}F_{2de+1}, (C₃-C₈)-alkenyl or -C_{df}H_{2df}R(30);
(Xa) is O, S or NR(33);
R(33)
is H, (C₁-C₄)-alkyl or (C₁-C₄)-perfluoroalkyl; dg is zero or 1;
(Xb) is O, S or NR(34);
R(34)
is H, (C₁-C₄) -alkyl or (C₁-C₄)-perfluoroalkyl;
dh is zero or 1;
da is zero, 1, 2, 3, 4, 5, 6, 7, 8;
db is zero, 1, 2, 3, 4;
de is zero, 1, 2, 3, 4, 5, 6, 7;
df is zero, 1, 2, 3, 4;
R(30)
is (C₃-C₈)-cycloalkyl, phenyl, biphenylyl or naphthyl, where the aromatics phenyl, biphenylyl or naphthyl are not substituted or are substituted by 1 - 3 substituents selected from the group consisting of F, Cl, CF₃, methyl, methoxy and NR(31)R(32);
R(31) and R(32) are H, (C₁-C₄)-alkyl or (C₁-C₄)-perfluoroalkyl;
or
R(2), R(3), R(4) and R(5)
independently of one another are NR(40)R(41) or - (Xe)-(CH₂)_{eb}R(45);
R(40) and R(41)
independently of one another are hydrogen, (C₁-C₈)-alkyl, (C₁-C₈)-perfluoroalkyl or (CH₂)ₑ-R(42) ;
e is zero, 1, 2, 3 or 4;
R(42) is (C₃-C₇)-cycloalkyl, phenyl, which is not substituted or is substituted by 1 - 3 substituents selected from the group consisting of F, Cl, CF₃, methyl, methoxy and NR(43)R(44);
R(43) and R(44)
independently of one another are H, CF₃ or (C₁-C₄)-alkyl;
or
R(40) and R(41)
together are 4 or 5 methylene groups, of which one CH₂ group can be replaced by oxygen, sulfur, NH, N-CH₃ or N-benzyl;
(Xe) is O, S or NR(47);
R(47) is H, (C₁-C₄) -alkyl or (C₁-C₄)-perfluoroalkyl;
eb is zero, 1, 2, 3 or 4;
R(45) is (C₃-C₇)-cycloalkyl, phenyl, which is not substituted or is substituted by 1 - 3 substituents selected from the group consisting of F, Cl, CF₃, methyl, methoxy, NR(50)R(51) and -(Xfa)-(CH₂)_{ed}-(Xfb)R(46);
Xfa is CH₂, O, S or NR(48);
Xfb is O, S or NR(49);
ed is 1, 2, 3 or 4;
R(46) is H, (C₁-C₄-alkyl or (C₁-C₄)-perfluoroalkyl;
R(48), R(49), R(50) and R(51) independently of one another are H or (C₁-C₄)-alkyl or (C₁-C₄)-perfluoroalkyl;
where R(3) and R(4), however, cannot be hydrogen, and their pharmaceutically tolerable salts;
ac) benzoylguanidines of the formula I 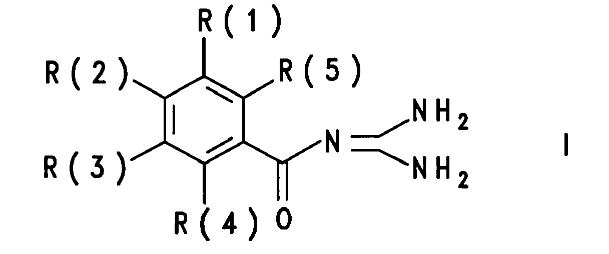 in which:
one of the three substituents R(1), R(2) and R(3) is (C₁-C₉)-heteroaryl-N-oxide,
which is linked via C or N and which is unsubstituted or substituted by 1 - 3 substituents selected from the group consisting of F, Cl, CF₃, CH₃, methoxy, hydroxyl, amino, methylamino and dimethylamino;
or
one of the three substituents R(1), R(2) and R(3) is -SR(10), -OR(10), -NR(10)R(11) or -CR(10)R(11)R(12);
R(10)
is -CₐH₂ₐ-(C₁-C₉)-heteroaryl-N-oxide, which is unsubstituted or substituted by 1 - 3 substituents selected from the group consisting of F, Cl, CF₃, CH₃, methoxy, hydroxyl, amino, methylamino and dimethylamino;
a is zero, 1 or 2;
R(11) and R(12)
independently of one another are defined as R(10), are hydrogen or (C₁-C₄)-alkyl;
and the other substituents R(1), R(2) and R(3) in each case
independently of one another are (C₁-C₈)-alkyl, (C₂-C₈)-alkenyl or -CₘH₂ₘR(14) ;
m is zero, 1 or 2;
R(14) is (C₃-C₈)-cycloalkyl or phenyl,
which is not substituted or is substituted by 1 - 3 substituents selected from the group consisting of F, Cl, CF₃, methyl, methoxy and NR(15)R(16),
R(15) and R(16)
are hydrogen or CH₃;
or
the other substituents R(1), R(2) and R(3) in each case independently of one another are hydrogen, F, Cl, Br, I, -C≡N, X-(CH₂)ₚ-(C_{q}F_{2q+1}), R(22)-SOᵤ, R(23)R(24)N-CO, R(25)-CO- or R(26)R(27)N-SO₂-,
where the perfluoroalkyl group is straight-chain or branched;
X is a bond, oxygen, S or NR(28);
u is zero, 1 or 2;
p is zero, 1 or 2;
q is zero, 1, 2, 3, 4, 5 or 6;
R(22), R(23), R(25) and R(26)
independently are (C₁-C₈)-alkyl, (C₂-C₆)-alkenyl, -CₙH₂ₙ-R(29) or CF₃;
n is zero, 1, 2, 3 or 4;
R(28) is hydrogen or (C₁-C₃) -alkyl;
R(29) is (C₃-C₇)-cycloalkyl or phenyl;
which is not substituted or is substituted by 1 - 3 substituents selected from the group consisting of F, Cl, CF₃, methyl, methoxy and NR(30)R(31);
R(30) and R(31)
are hydrogen or C₁-C₄-alkyl,
or
R(23), R(25) and R(26)
are also hydrogen;
R(24) and R(27)
independently of one another are hydrogen or (C₁-C₄)-alkyl;
or
R(23) and R(24), and also R(26) and R(27) together are 4 or 5 methylene groups, of which one CH₂ group can be replaced by oxygen, S, NH, N-CH₃ or
N-benzyl;
or
the other substituents R(1), R(2) and R(3) in each case independently of one another are OR(35) or NR(35)R(36);
R(35) and R(36)
independently of one another are hydrogen or (C₁-C₆)-alkyl;
or
R(35) and R(36)
together are 4 - 7 methylene groups, of which one CH₂ group can be replaced by oxygen, S, NH, N-CH₃ or N-benzyl,
R(4) and R(5)
independently of one another are hydrogen, (C₁-C₄)-alkyl, F, Cl, -OR(32), -NR(33)R(34) or CᵣF₂ᵣ₊₁; R(32), R(33) and R(34)
independently of one another are hydrogen or (C₁-C₃)-alkyl;
r is 1, 2, 3 or 4;
and their pharmaceutically tolerable salts;
ad) benzoylquanidines of the formula I 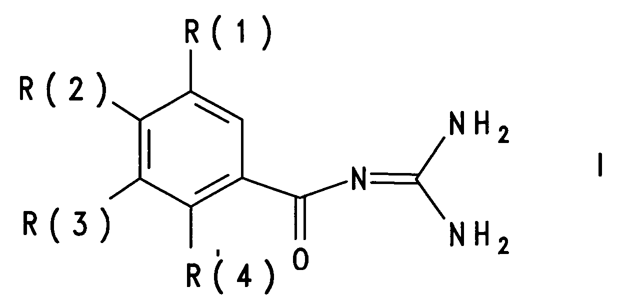 in which:
R(1) is hydrogen, F, Cl, Br, I, CN, NO₂, OH, (C₁-C₈)-alkyl, (C₃-C₈)-cycloalkyl, Oₐ-(CH₂)_{b}-(CF₂)_{c}-CF₃;
a is zero or 1;
b is zero, 1 or 2;
c is zero, 1, 2 or 3;
or
R(1) is R(5)-SOₘ or R(6)R(7)N-SO₂-;
m is zero, 1 or 2;
R(5) and R(6) independently of one another are (C₁-C₈)-alkyl, (C₃-C₆)-alkenyl, CF₃ or - CₙH₂ₙ-R(8);
n is zero, 1, 2, 3 or 4;
R(7) is hydrogen or (C₁-C₄)-alkyl;
R(8) is (C₃-C₇)-cycloalkyl or phenyl,
which is not substituted or is substituted by 1 - 3 substituents selected from the group consisting of F, Cl, CF₃, methyl, methoxy and NR(9)R(10); R(9) and R(10) independently of one another
are hydrogen or (C₁-C₄)-alkyl;
or
R(6) is H;
or R(6) and R(7)
together are 4 or 5 methylene groups, of which one CH₂ group can be replaced by oxygen, S, NH, N-CH₃ or N-benzyl,
or
R(1) is -SR(11), -OR(11) or -CR(11)R(12)R(13);
R(11) is -CₚH₂ₚ-(C₃-C₈) -cycloalkyl, -(C₁-C₉)-heteroaryl or phenyl,
where the aromatic systems are unsubstituted or substituted by 1 - 3 substituents selected from the group consisting of F, Cl, CF₃, CH₃, methoxy, hydroxyl, amino, methylamino and dimethylamino; R(12), R(13) independently of one another are defined as R(11) or are hydrogen or (C₁-C₄) -alkyl;
p is zero, 1 or 2;
or
R(1) is phenyl, naphthyl, biphenylyl or (C₁-C₉)-heteroaryl, the latter linked via C or N,
which are unsubstituted or substituted by 1 - 3 substituents selected from the group consisting of F, Cl, CF₃, CH₃, methoxy, hydroxyl, amino, methylamino and dimethylamino;
R(2) is -CF₂R(14), -CF[R(15) [R(16)], -CF[(CF₂)_{q}-CF₃)][R(15)], -C[(CF₂)ᵣ-CF₃]=CR(15)R(16);
R(14) is (C₁-C₉)-alkyl or (C₃-C₆)-cycloalkyl;
R(15) and R(16) independently of one another are hydrogen or (C₁-C₄)-alkyl;
q is zero, 1 or 2;
r is zero, 1 or 2;
R(3) is defined as R(1);
R(4) is hydrogen, (C₁-C₃)-alkyl, F, Cl, Br, I, CN, - (CH₂)ₛ-(CF₂)ₜ-CF₃;
s is zero or 1;
t is zero, 1 or 2;
and their pharmaceutically tolerable salts;
ae) benzoylguanidines of the formula I 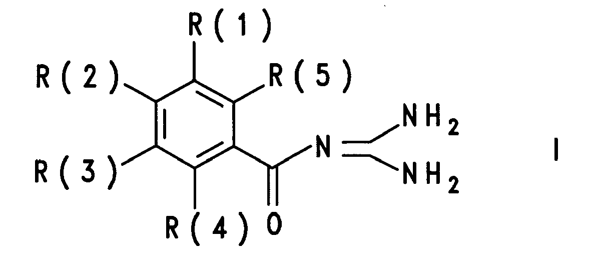 in which:
one of the three substituents R(1), R(2) and R(3) is -Y-4-[(CH₂)ₖ-CHR(7)-(C=O)R(8)]-phenyl, -Y-3-[(CH₂)ₖ-CHR(7)-(C=O)R(8)]-phenyl or -Y-2-[(CH₂)ₖ-CHR(7)-(C=O)R(8)-phenyl,
where the phenyl in each case is unsubstituted or substituted by 1 - 2 substituents from the group F, Cl, -CF₃, methyl, hydroxyl, methoxy, or -NR(37)R(38); R(37) and R(38)
independently of one another are hydrogen or -CH₃;
Y is a bond, oxygen, -S- or -NR(9);
R(9) is hydrogen or -(C₁-C₄)-alkyl;
R(7) is -OR(10) or -NR(10)R(11);
R(10) and R(11)
independently of one another are hydrogen, -(C₁-C₈)-alkyl, -(C₁-C₈)-alkanoyl, -(C₁-C₈)-alkoxycarbonyl, benzyl, benzyloxycarbonyl;
or
R(10) is trityl;
R(8) is -OR(12) or -NR(12)R(13);
R(12) and R(13)
independently of one another are hydrogen, -(C₁-C₈)-alkyl or benzyl;
k is zero, 1, 2, 3 or 4;
and the other radicals R(1), R(2) and R(3) in each case independently of one another are -(C₁-C₈)-alkyl, - (C₂-C₈)-alkenyl or -(CH₂)ₘR(14);
m is zero, 1 or 2;
R(14) is -(C₃-C₈)-cycloalkyl or phenyl, which is not substituted or is substituted by 1 - 3 substituents selected from the group consisting of F, Cl, -CF₃, methyl, methoxy and -NR(15)R(16);
R(15) and R(16) are hydrogen or -CH₃;
or
the other radicals R(1), R(2) and R(3) in each case independently of one another are R(18)R(19)N-( C=Y')-NH-SO₂-;
Y' is oxygen, -S- or -N-R(20);
R(18) and R(19)
independently of one another are hydrogen, - (C₁-C₈)-alkyl, -(C₃-C₆)-alkenyl or -(CH₂)ₜ-R(21);
t is zero, 1, 2, 3 or 4;
R (21) is -(C₅-C₇)-cycloalkyl or phenyl,
which is unsubstituted or substituted by 1 - 3 substituents selected from the groups consisting of F, Cl, -CF₃, methoxy and -(C₁-C₄)-alkyl;
or
R(18) and R(19)
together are 4 or 5 methylene groups, of which one CH₂ group can be replaced by oxygen, -S-, -NH-, -N-CH₃ or -N-benzyl;
R(20) is defined as R(18) or is amidine;
or
the other radicals R(1), R(2) and R(3) in each case independently of one another are hydrogen, F, Cl, Br, I, -C≡N, X-(CH₂)ₚ-(C_{q}F₂q₊₁), R(22)-SOᵤ-, R(23)R(24)N-CO-, R(25)-CO- or R(26)R(27)N-SO₂-, where the perfluoroalkyl group is straight-chain or branched;
X is a bond, oxygen, -S- or -NR(28);
u is zero, 1 or 2;
p is zero, 1 or 2;
q is 1, 2, 3, 4, 5 or 6;
R(22), R(23), R(25) and R(26)
independently of one another are -(C₁-C₈)-alkyl, -(C₃-C₆)-alkenyl, -(CH₂)ₙ-R(29) or -CF₃; n is zero, 1, 2, 3 or 4;
R(28) is hydrogen or -(C₁-C₃)-alkyl;
R(29) is -(C₃-C₇)-cycloalkyl or phenyl,
which is not substituted or is substituted by 1 - 3 substituents selected from the group consisting of F, Cl, -CF₃, methyl, methoxy and - NR(30)R(31);
R(30) and R(31)
are hydrogen or -(C₁-C₄)-alkyl;
or
R(23), R(25) and R(26)
are hydrogen;
R(24) and R(27)
independently of one another are hydrogen or -(C₁-C₄)-alkyl;
or
R(23) and R(24), and also R(26) and R(27)
together are 4 or 5 methylene groups, of which one CH₂ group can be replaced by oxygen, -S-, -NH-, - N-CH₃ or -N-benzyl;
or
the other radicals R(1), R(2) and R(3) in each case independently of one another are -OR(35) or - NR(35)R(36);
R(35) and R(36)
independently of one another are hydrogen or -(C₁-C₆)-alkyl;
or
R(35) and R(36) together are 4 - 7 methylene groups, of which one CH₂ group can be replaced by oxygen, -S-, -NH-, -N-CH₃ or -N-benzyl;
R(4) and R(5)
independently of one another are hydrogen, -(C₁-C₄)-alkyl, F, Cl, -OR(32), -NR(33)R(34) or -CᵣF₂ᵣ₊₁; R(32), R(33) and R(34)
independently of one another are hydrogen or -(C₁-C₃)-alkyl;
r is 1, 2, 3 or 4;
and their pharmaceutically tolerable salts;
af) benzoylguanidines of the formula I 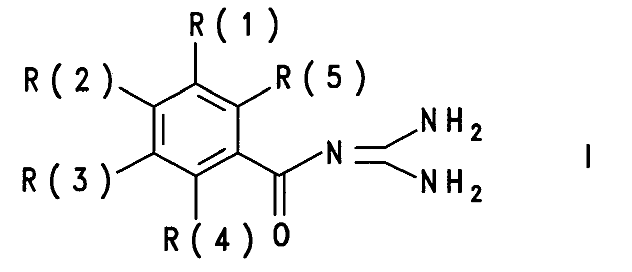 in which:
R(1) is R(6)-CO or R(7)R(8)N-CO;
R(6) is (C₁-C₈)-alkyl, (C₁-C₈)-perfluoroalkyl, (C₃-C₈)-alkenyl or -CₙH₂ₙ-R(9),
n is zero, 1, 2, 3 or 4;
R(9) is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR(10)R(11),
R(10) and R(11)
are H, (C₁-C₉)-alkyl or (C₁-C₄)-perfluoroalkyl;
R (7) is H, (C₁-C₈)-alkyl, (C₁-C₈)-perfluoroalkyl, (C₃-C₈)-alkenyl or -CₙH₂ₙ-R (12) ;
n is zero, 1, 2, 3 or 4;
R(12) is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR(13)R(14);
R(13) and R(14)
are H, (C₁-C₄)-alkyl or (C₁-C₄)-perfluoroalkyl;
R(8) is H, (C₁-C₄)-alkyl or (C₁-C₄)-perfluoroalkyl;
or
R(7) and R(8) together
are 4 or 5 methylene groups, of which one CH₂ group can be replaced by oxygen, S, NH, N-CH₃ or N-benzyl;
R(2) is defined as R(1), or is H, OH, F, Cl, Br, I, CN, NO₂, (C₁-C₈)-alkyl, (C₁-C₈)-perfluoroalkyl, (C₃-C₈)-alkenyl or -CₙH₂ₙR(15);
n is zero, 1, 2, 3 or 4;
R(15) is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR(16)R(17);
R(16) and R(17)
are H, (C₁-C₄) -alkyl or (C₁-C₄)-perfluoroalkyl;
or
R(2) is (C₁-C₉)-heteroaryl,
which is linked via C or N and which is unsubstituted or substituted by 1 - 3 substituents selected from the group consisting of F, Cl, CF₃, CH₃, methoxy, hydroxyl, amino, methylamino and dimethylamino;
or
R(2) is SR(18), -OR(18), -NR(18)R(19) or - CR(18)R(19)R(20);
R(18) -is CₐH₂ₐ-(C₁-C₉)-heteroaryl, which is unsubstituted or substituted by 1 - 3 substituents selected from the group consisting of F, Cl, CF₃, CH₃, methoxy, hydroxyl, amino, methylamino, dimethylamino;
a is zero, 1 or 2;
R(19) and R(20)
independently of one another are defined as R(18) or are hydrogen, (C₁-C₄)-alkyl or (C₁-C₄)-perfluoroalkyl;
or
R(2) is R(21)-SOₘ or R(22)R(23)N-SO₂-;
m is 1 or 2;
R(21) is (C₁-C₈)-alkyl, (C₁-C₈)-perfluoroalkyl, (C₃-C₈)-alkenyl or -CₙH₂ₙ-R(24);
n is zero, 1, 2, 3 or 4;
R(24) is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR(27)R(28); R(27) and R(28)
are H, (C₁-C₉)-alkyl or (C₁-C₄)-perfluoroalkyl;
R(22) is H, (C₁-C₈)-alkyl, (C₁-C₈)-perfluoroalkyl, (C₃-C₈)-alkenyl or -CₙH₂ₙ-R(29);
n is zero, 1, 2, 3 or 4;
R (29) is (C₃-C₈)-cycloalkyl, phenyl, biphenylyl or naphthyl,
where the aromatics are not substituted or are substituted by 1 - 3 substituents selected from the group consisting of F, Cl, CF₃, methyl, methoxy and NR(30)R(31);
R(30) and R(31)
are H, (C₁-C₄)-alkyl or (C₁-C₄)-perfluoroalkyl;
R(23) is hydrogen, (C₁-C₄)-alkyl or (C₁-C₄)-perfluoroalkyl;
or
R(22) and R(23)
together are 4 or 5 methylene groups, of which one CH₂ group can be replaced by oxygen,
S, NH, N-CH₃ or N-benzyl;
or
R(2) is R(33)X-;
X is oxygen, S, NR(34), (D=O)A- or NR(34)C=MN⁽*⁾R(35)-;
M is oxygen or S;
A is oxygen or NR(34);
D is C or SO;
R(33) is (C₁-C₈)-alkyl, (C₃-C₈)-alkenyl, (CH₂)_{b}C_{d}F_{2d+1} or -CₙH₂ₙ-R(36);
b is zero or 1;
d is 1, 2, 3, 4, 5, 6 or 7;
n is zero, 1, 2, 3, or 4;
R(36) is (C₃-C₈)-cycloalkyl, phenyl, biphenylyl or naphthyl, where the aromatics are not substituted or are substituted by 1 - 3 substituents selected from the group consisting of F, Cl, CF₃, methyl, methoxy and NR(37)R(38); R(37) and R(38) are H, (C₁-C₄)-alkyl or (C₁-C₄)-perfluoroalkyl;
R(34) is hydrogen, (C₁-C₄)-alkyl or (C₁-C₄)-perfluoroalkyl;
R(35) is defined as R(33);
or
R(33) and R(34) together are 4 or 5 methylene groups, of which one CH₂ group can be replaced by oxygen, S, NH, N-CH₃ or N-benzyl;
where A and N⁽*⁾ are bonded to the phenyl nucleus of the benzoylguanidine parent structure;
or
R(2) is -SR(40), -OR(40), -NHR(40), -NR(40)R(41), - CHR(40)R(42), -CR(42)R(43)OH, -C≡CR(45), -CR(46)=CR(45) or -[CR(47)R(48)]ᵤ-CO-[C(R49)R(50)]ᵥ-R(44); R(40) and R(41) independently of one another are -(CH₂)ₚ-(CHOH)_{q}-(CH₂)ᵣ-(CHOH)ₜ-R(51) or -(CH₂)ₚ-O-(CH₂-CH₂O)_{q}-R(51);
R(51) is hydrogen or methyl;
u is 1, 2, 3 or 4;
v is zero, 1, 2, 3 or 4;
p, q and r
independently of one another are zero, 1, 2, 3 or 4;
t is 1, 2, 3 or 4;
R(42) and R(43)
independently of one another are hydrogen or (C₁-C₆)-alkyl;
or
R(42) and R(43)
together with the carbon atom carrying them are a (C₃-C₈)-cycloalkyl;
R(44) is hydrogen, (C₁-C₆)-alkyl, (C₃-C₈)-cycloalkyl, -CₑH₂ₑ-R(45);
e is zero, 1, 2, 3 or 4;
R(45) is phenyl,
which is unsubstituted or substituted by 1 - 3 substituents selected from the group consisting of F, Cl, CF₃, methyl, methoxy and NR(52)R(53); R(52) and R(53)
are H or (C₁-C₄)-alkyl;
or
R(45) is (C₁-C₉)-heteroaryl,
which is unsubstituted or substituted as phenyl;
or
R(45) is (C₁-C₆)-alkyl,
which is unsubstituted or substituted by 1 - 3 OH;
R(46), R(47), R(48), R(49) and R(50)
independently of one another are hydrogen or methyl;
or
R(2) is R(55)-NH-SO₂-;
R(55) is R(56)R(57)N-(C=Y)-;
Y is oxygen, S or N-R(58);
R(56) and R(57)
independently of one another are hydrogen, (C₁-C₈)-alkyl, (C₃-C₆)-alkenyl or -C_{f}H_{2f}-R(59); f is zero, 1, 2, 3 or 4;
R(59) is (C₅-C₇)-cycloalkyl, phenyl, which is unsubstituted or substituted by 1 - 3 substituents selected from the group consisting of F, Cl, CF₃, methoxy and (C₁-C₄)-alkyl;
or
R(56) and R(57)
together are 4 or 5 methylene groups, of which one CH₂ group can be replaced by oxygen, S, NH, N-CH₃ or N-benzyl;
R(58)
is defined as R(56) or is amidine;
R(3), R(4) and R(5) are independently of one another defined as R(1) or R(2), but where at least one of the substituents R(2), R(3), R(4) and R(5) must be OH;
and their pharmaceutically tolerable salts;
ag) benzoylguanidines of the formula I 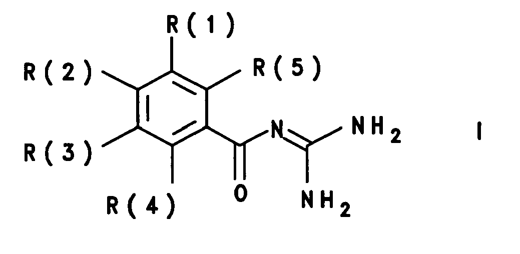 in which:
one of the three substituents R(1), R(2) and R(3) is R(6)-A-B-D-;
R(6) is a basic protonatable radical, i.e. an amino group -NR(7)R(8), an amidino group R(7)R(8)N-C[=N-R(9)]- or a guanidino group 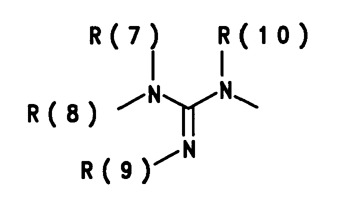 R(7), R(8), R(9) and R(10)
independently of one another are hydrogen or alkyl having 1, 2, 3 or 4 carbon atoms;
or
R(7) and R(8)
together are CₐH₂ₐ;
a is 4, 5, 6 or 7;
where if a = 5, 6 or 7 a methylene group of the group CₐH₂ₐ can be replaced by a heteroatom group 0, SOₘ or NR(11),
or
R(8) and R(9) or R(9) and R(10) or R(7) and R(10)
are a group CₐH₂ₐ;
a is 2, 3, 4 or 5;
where if a = 3, 4 or 5 a methylene group of the group CₐH₂ₐ can be replaced by a heteroatom group O, SOₘ or NR(11);
m is zero, 1 or 2;
R(11) is hydrogen or methyl;
or
R(6) is a basic heteroaromatic ring system having 1 - 9 carbon atoms;
A is C_{b}H_{2b};
b is 1, 2, 3, 4, 5, 6, 7, 8, 9 or 10; where in the group C_{b}H_{2b} one or two methylene groups can be replaced by one of the groupings selected from the group consisting of -O-, -CO-, -CH[OR(20)]-, -SOₘ-, -NR(20)-, -NR(20)-CO-, -NR(20)-CO-NH-, -NR(20)-CO-NH-SO₂- 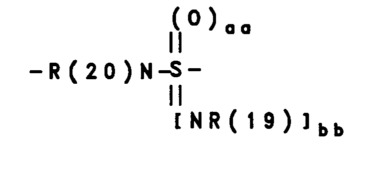 and -SOₐₐ[NR(19)]_{bb}-;
and where in the group C_{b}H_{2b} a methylene group can be replaced by -CH-R(99), where R(99) together with R(7) forms a pyrrolidine or piperidine ring;
aa is 1 or 2;
bb is 0 or 1;
aa + bb = 2;
R(19) is hydrogen or alkyl having 1, 2, 3 or 4 carbon atoms;
R(20) is hydrogen or methyl;
B is a phenylene or naphthylene radical 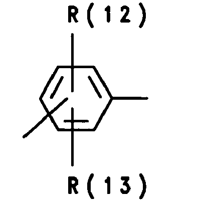 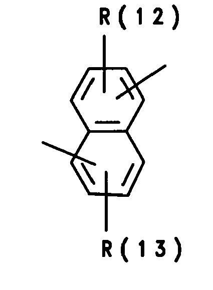 R(12) and R(13)
independently of one another are hydrogen, methyl, F, Cl, Br, I, CF₃ or -SO_{w}-R(14);
R(14) is methyl or NR(15)R(16);
R(15) and R(16)
independently of one another are hydrogen or alkyl having 1, 2, 3 or 4 carbon atoms;
w is zero, 1 or 2;
D is -C_{d}H_{2d}-X_{f}-;
d is zero, 1, 2, 3 or 4;
X is -O-, -CO-, -CH[OR(21)]-, -SOₘ- or - NR(21)-;
f is zero or 1;
R(21) is hydrogen or methyl;
m is zero, 1 or 2;
and the other substituents R(1) and R(2) and R(3) in each case independently of one another are hydrogen, F, Cl, Br, I, -CN, -(C₁-C₈-alkyl, -(C₂-C₈)-alkenyl, -NR(35)R(36) or R(17)-C_{g}H_{2g}-Zₕ-;
g is zero, 1, 2, 3 or 4;
h is zero or 1;
R(35) and R(36)
independently of one another are hydrogen or alkyl having 1, 2, 3, 4, 5 or 6 carbon atoms;
or
R(35) and R(36)
together are 4 - 7 methylene groups, of which one CH₂ group can be replaced by oxygen, -S-, -NH-, -NCH₃ or -N-benzyl;
Z is -0-, -CO-, -SOᵥ- ,-NR(18)-, -NR(18)-CO-, -
NR(18)-CO-NH- or -NR(18)-SO₂-;
R(18) is hydrogen or methyl;
v is zero, 1 or 2;
R(17) is hydrogen, cycloalkyl having 3, 5 or 6 carbon atoms or CₖF₂ₖ₊₁-;
k is 1, 2 or 3,
or
R(17) is pyrrol-1-yl, pyrrol-2-yl or pyrrol-3-yl,
which is not substituted or is substituted by 1 - 4 substituents selected from the group consisting of F, Cl, Br, I, -CN, (C₂-C₈)-alkanoyl, (C₂-C₈)-alkoxycarbonyl, formyl, carboxyl, -CF₃, methyl and methoxy;
or
R(17) -is (C₃-C₈)-cycloalkyl or phenyl,
which is not substituted or is substituted by 1 - 3 substituents selected from the group consisting of F and Cl, -CF₃, methyl, hydroxyl, methoxy, -NR(37)R(38), CH₃SO₂- and H₂NO₂S-;
R(37) and R(38)
are hydrogen or -CH₃;
R(4) and R(5) independently of one another are hydrogen, alkyl having 1, 2, 3 or 4 carbon atoms, F, Cl, -OR(32), -NR(33)R(34) or -CᵣF₂ᵣ₊₁;
R(32), R(33) and R(34)
independently of one another are hydrogen or alkyl having 1, 2 or 3 carbon atoms;
r is 1, 2, 3 or 4;
and their pharmacologically tolerable salts;
ah) indenoylguanidines of the formula I 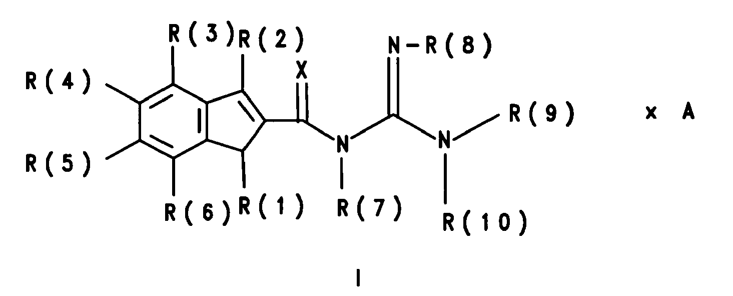 in which:
R(1) and R(2) independently of one another are hydrogen, alkyl having 1, 2, 3, 4, 5, 6, 7, 8, 9 or 10 carbon atoms, cycloalkyl having 3, 4, 5 or 6 carbon atoms, O-alkyl having 1, 2, 3 or 4 carbon atoms, O-C(=O)-alkyl having 1, 2, 3 or 4 carbon atoms or CₘH₂ₘ-NR(12)R(13);
R(12) and R(13)
independently of one another are hydrogen or alkyl having 1, 2, 3 or 4 carbon atoms;
m is zero, 2, 3 or 4;
NH-C(=O)-NH₂, C(=O)-O-alkyl having 1, 2, 3 or 4 carbon atoms, C(=O)-NH₂, C(=O)-NH-alkyl having 1, 2, 3 or 4 carbon atoms, C(=O)-N(alkyl)₂ having 1, 2, 3 or 4 carbon atoms in each alkyl group, alkenyl having 2, 3, 4, 5, 6, 7, 8, 9 or 10 carbon atoms, alkynyl having 2, 3, 4, 5, 6, 7, 8, 9 or 10 carbon atoms, alkylaryl having 1, 2, 3 or 4 carbon atoms in the alkyl group, alkenylaryl having 2, 3, 4, 5, 6, 7, 8, 9 or 10 carbon atoms in the alkenyl group, alkynylaryl having 2, 3, 4, 5, 6, 7, 8, 9 or 10 carbon atoms in the alkynyl group, C₁-C₄-alkyl-substituted aryl, C₁-C₉-alkylheteroaryl, C₁-C₄-alkenylheteroaryl, aminoalkylaryl having 1, 2, 3 or 4 carbon atoms in the alkyl group, substituted aryl, heteroaryl and substituted heteroaryl;
R(3), R(4), R(5) and R(6)
independently of one another are hydrogen, alkyl having 1, 2, 3, 4, 5, 6, 7, 8, 9 or 10 carbon atoms, O-alkyl having 1, 2, 3, 4, 5, 6; 7, 8, 9 or 10 carbon atoms, halogen, (such as F, Cl, Br, I),
OH, aryl, substituted aryl, heteroaryl, substituted heteroaryl, O-lower alkyl, O-aryl, O-lower alkylaryl, O-substituted aryl, O-lower alkyl-substituted aryl, O-C(=O)-C₁-C₄-alkylaryl, 0-C(=O)-NH-C₁-C₄-alkyl, O-C(=O)-N(C₁-C₄-alkyl)₂, NO₂, CN, CF₃, NH₂, NH-C (=O) -C₁-C₄-alkyl, NH-C(=O)-NH₂, COOH, C(=O)-O-C₁-C₄-alkyl, C(=O)-NH₂, C(=O)-NH-C₁-C₄-alkyl, C (=O) -N (C₁-C₄-alkyl)₂, C₁-C₄-COOH, C₁-C₄-alkyl-C (=O) -O-C₁-C₄-alkyl, SO₃H, SO₂-alkyl, SO₂-alkylaryl, SO₂-N-(alkyl)₂, SO₂-N(alkyl)(alkylaryl), C(=O)-R(11), C₁-C₁₀-alkyl-C(=O)-R(11), C₂-C₁₀-alkenyl-C(=O)-R(11), C₂-C₁₀-alkynyl-C(=O)-R(11), NH-C (=O) -C₁-C₁₀-alkyl-C(=O)-R(11), O-C₁-C₁₁-alkyl-C(=O)-R(11);
R(11) is C₁-C₄-alkyl, C₁-C₄-alkynyl, aryl, substituted aryl, NH₂, NH-C₁-C₄-alkyl, N-(C₁-C₄-alkyl)₂, SO₃H, SO₂-alkyl, SO₂-alkylaryl, SO₂-N-(alkyl)2, SO₂-N (alkyl) (alkylaryl) ;
X is O, S or NH;
R(7), R(8), R(9) and R(10)
independently of one another are hydrogen, alkyl, cycloalkyl, aryl, alkylaryl;
or
R(8) and R(9) together are part of a 5, 6 or 7-membered heterocyclic ring;
A is absent or is a nontoxic organic or inorganic acid.
ai) benzyloxycarbonylguanidines of the formula I
in which: 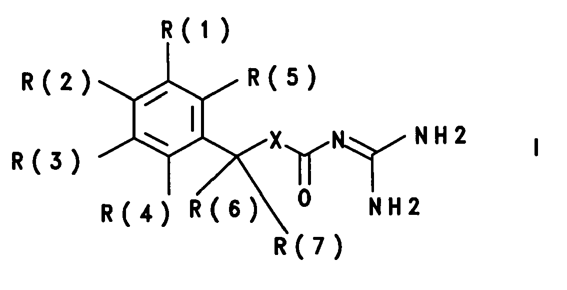
R(1), R(2) and R(3) independently of one another are -Y-[4-R(8)-phenyl], -Y-[3-R(8)-phenyl] or -Y-[2-R(8)-phenyl],
where the phenyl is in each case unsubstituted or substituted by 1 - 2 substituents from the group consisting of F, Cl, -CF₃, methyl, hydroxyl, methoxy and - NR(96)R(97);
R(96) and R(97)
independently of one another are hydrogen or -CH₃;
Y is a bond, CH₂, oxygen, -S- or -NR(9);
R(9) is hydrogen or alkyl having 1, 2, 3 or 4 carbon atoms;
R(8) is SOₐ[NR(98)]_{b}NR(99)R(10);
a is 1 or 2;
b is 0 or 1;
a + b = 2;
R(98), R(99) and R(10)
independently of one another are hydrogen, -(C₁-C₈)-alkyl, benzyl, -(C₂-C₈)-alkylene-NR(11)R(12), (C₂-C₈)-alkylene-NR(13)-(C₂-C₈)-alkylene-NR(37)R(38) or (C₀-C₈)-alkylene-CR(39)R(40)CR(41)R(42)(C₀-C₈)-alkylene-NR(43)R(44);
R(11), R(12), R(13), R(37), R(38), R(43) and R(44)
independently of one another are hydrogen, -(C₁-C₈)-alkyl or benzyl:
R(39), R(40), R(41) and R(42)
independently of one another are hydrogen, -(C₁-C₈)-alkyl or -(C₀-C₃)-alkylenephenyl,
where the phenyl is not substituted or is substituted by 1 - 3 substituents selected from the group consisting of F, Cl, -CF₃, methyl and methoxy;
or
R(99) and R(10)
together are 4 - 6 methylene groups, of which one CH₂ group can be replaced by oxygen, -S-, -NH-, -N-CH₃ or -N-benzyl;
or
R(8) is SOₐ[NR(98)]_{b}NR(95)-C[=N-R(94)]-NR(93)R(92); R(92), R(93), R(94) and R(95)
independently of one another are hydrogen or alkyl having 1, 2, 3 or 4 carbon atoms;
or
R(1), R(2) and R(3)
independently of one another are pyrrol-1-yl, pyrrol-2-yl or pyrrol-3-yl,
which is not substituted or is substituted by 1 - 4 substituents selected from the group consisting of F, Cl, Br, I, -CN, (C₂-C₈)-alkanoyl, (C₂-C₈)-alkoxycarbonyl, formyl, carboxyl, -CF₃, methyl, methoxy;
or
R(1), R(2) and R(3)
independently of one another are hydrogen, -(C₁-C₈)-alkyl, -(C₂-C₈₎-alkenyl or -(CH₂)ₘR(14);
m is zero, 1 or 2;
R(14) is -(C₃-C₈)-cycloalkyl or phenyl,
which is not substituted or is substituted by 1 - 3 substituents selected from the group consisting of F and -Cl, -CF₃, methyl, methoxy and - NR (15) R (16) ;
R(15) and R(16)
are hydrogen or -CH₃;
or
R(1), R(2) and R(3)
independently of one another are -Q-4-[(CH₂)ₖ-CHR(17)-(C=O)R(20)]-phenyl, -Q-3-(CH₂)ₖ-CHR(17)-(C=O)R(20)]-phenyl or -Q-2-[(CH₂)ₖ-CHR(17)-(C=O)R(20)]-phenyl, where the phenyl in each case is unsubstituted or substituted by 1 - 2 substituents from the group F, Cl, -CF₃, methyl, hydroxyl, methoxy and -NR(35)R(36);
R(35) and R(36)
independently of one another are hydrogen or -CH₃;
Q is a bond, oxygen, -S- or -NR(18);
R(18) is hydrogen or -(C₁-C₄)-alkyl;
R(17) is -OR(21) or -NR(21)R(22);
R(21) and R(22)
independently of one another are hydrogen, -(C₁-C₈)-alkyl, -(C₁-C₈)-alkanoyl, -(C₁-C₈)-alkoxycarbonyl, benzyl, benzyloxycarbonyl;
or
R(21) is trityl;
R(20) is -OR(23) or -NR(23)R(24);
R(23), R(24) independently of one another are hydrogen, -(C₁-C₈)-alkyl or benzyl;
k is zero, 1, 2, 3 or 4;
or
R(1), R(2) and R(3)
independently of one another are (C₁-C₉)-heteroaryl,
which is linked via C or N and which is unsubstituted or substituted by 1 - 3 substituents from the group F, Cl, CF₃, CH₃, methoxy, hydroxyl, amino, methylamino and
dimethylamino;
or
R(1), R(2) and R(3)
are -SR(25), -OR(25), -NR(25)R(26), - CR(25)R(26)R(27);
R(25) is -C_{f}H_{2f}-(C₁-C₉)-heteroaryl, which is unsubstituted or substituted by 1 - 3 substituents from the group F, Cl, CF₃, CH₃, methoxy, hydroxyl, amino, methylamino and dimethylamino;
f is zero, 1 or 2;
R(26) and R(27)
independently of one another are defined as R(25) or are hydrogen or (C₁-C₄)-alkyl,
or
R(1), R(2) and R(3) independently of one another are (C₁-C₉)-heteroaryl-N-oxide,
which is linked via C or N and which is unsubstituted or substituted by 1 - 3 substituents selected from the group consisting of F, Cl, CF₃, CH₃, methoxy, hydroxyl, amino, methylamino and
dimethylamino;
or
R(1), R(2) and R(3) independently of one another are -SR(28), -OR(28), -NR(28)R(29) or -CR(28)R(29)R(30);
R(28) is -CgH_{2g}-(C₁-C₉)-heteroaryl-N-oxide, which is unsubstituted or substituted by 1 - 3 substituents selected from the group consisting of F, Cl, CF₃, CH₃, methoxy, hydroxyl, amino, methylamino and dimethylamino;
g is zero, 1 or 2;
R(29), R(30)
independently of one another are defined as R(28), hydrogen or (C₁-C₉)-alkyl;
or
R(1), R(2) and R(3) independently of one another are hydrogen, F, Cl, Br, I, -C=N, T-(CH₂)ₕ-(CᵢF₂ᵢ₊₁), R(31)SO₁-, R(32)R(33)N-CO-, R(34)-CO- or R(45)R(46)N-SO₂, where the perfluoroalkyl group is straight-chain or branched;
T is a bond, oxygen, -S- or -NR(47);
1 is zero, 1 or 2;
h is zero, 1 or 2;
i is 1, 2, 3, 4, 5 or 6;
R(31), R(32), R(34) and R(45) independently of one another are -(C₁-C₈)-alkyl, -(C₃-C₆)-alkenyl, (CH₂)ₙR(48) or -CF₃;
n is zero, 1, 2, 3 or 4;
R(47) is hydrogen or alkyl with 1, 2 or 3 carbon atoms;
R(48) is -(C₃-C₇)-cycloalkyl or phenyl,
which is not substituted or is substituted by 1 - 3 substituents selected from the group consisting of F, Cl, -CF₃, methyl, methoxy and -NR(49)R(50);
R(49) and R(50)
are hydrogen or alkyl having 1, 2, 3 or 4 carbon atoms;
or
R(32), R(34) and R(45)
are hydrogen;
R(33) and R(46)
independently of one another are hydrogen or alkyl having 1, 2, 3 or 4 carbon atoms;
or
R(32) and R(33), and R(45) and R(46) together are 5 or 6 methylene groups, of which one CH₂ group can be replaced by oxygen, -S-, -NH-, -NCH₃ or -N-benzyl;
or
R(1), R(2) and R(3) independently of one another are R(51)-A-G-D-;
R(51) is a basic protonatable radical, i.e. an amino group -NR(52)R(53), an amidino group R(52)R(53)N-C[=N-R(54)]- or a guanidino group R(52)R(53)N-C[=N-R(54)]-NR(55)-;
R(52), R(53), R(54) and R(55)
independently of one another are hydrogen or alkyl having 1, 2, 3 or 4 carbon atoms;
or
R(52) and R(53) are
a group C_{α}H_{2α}
α is 4, 5, 6 or 7;
where if α = 5, 6 or 7 a carbon atom of the group C_{α}H_{2α} can be replaced by a heteroatom group 0, SO_{d} or NR(56),
or
R(53) and R(54) or R(54) and R(55) or R(52) and R(55) are
a group C_{γ}H_{2γ};
γ is 2, 3, 4 or 5;
where if γ = 3, 4 or 5 a carbon atom of the group C_{γ}H_{2γ} can be replaced by a heteroatom group O, SO_{d} or NR(56);
d is zero, 1 or 2;
R(56) is hydrogen or methyl;
or
R(51) is a basic heteroaromatic ring system having 1 - 9 carbon atoms;
A is a group CₑH₂ₑ;
e is zero, 1, 2, 3, 4, 5, 6, 7, 8, 9 or 10;
where in the group CₑH₂ₑ a carbon atom can be replaced by one of the groupings -0-, -CO-, -CH[OR(57)]-, -SOᵣ-, -NR(57)-, -NR(57)-CO-, -NR(57)-CO-NH-, -NR(57)-CO-NH-SO₂- or -NR(57)-SO₂-;
r is zero, 1 or 2;
G is a phenylene radical 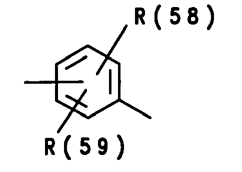 R(58) and R(59)
independently of one another are hydrogen, methyl, methoxy, F, Cl, Br, I, CF₃ or -SOₛ-R(60);
R(60) is methyl or NR(61)R(62); R(61) and R(62)
independently of one another are hydrogen or alkyl having 1, 2, 3 or 4 carbon atoms;
D is -CᵥH₂ᵥ-E_{w}-;
v is zero, 1, 2, 3 or 4;
E is -O-, -CO-, -CH[OR(63)]-, -SOₐₐ- or - NR(63)-;
w is zero or 1;
aa is zero, 1 or 2
R(63) is hydrogen or methyl,
or
R(1), R(2) and R(3)
independently of one another are -CF₂R(64), - CF[R(65)] [R(66)], -CF[(CF₂)_{q}-CF₃] [R(65)], -C[(CF₂)ₚ-CF₃]=CR(65)R(66) ;
R(64) is alkyl having 1, 2, 3 or 4 carbon atoms or cycloalkyl having 3, 4, 5, 6 or 7 carbon atoms;
R(65) and R(66) independently of one another are hydrogen or alkyl having 1, 2, 3 or 4 carbon atoms;
q is zero, 1 or 2;
p is zero, 1 or 2;
or
R(1), R(2) and R(3)
independently of one another are -OR(67) or - NR(67)R(68);
R(67) and R(68)
independently of one another are hydrogen or alkyl having 1, 2, 3, 4, 5 or 6 carbon atoms;
or
R(67) and R(68)
together are 4, 5, 6 or 7 methylene groups, of which one CH₂ group can be replaced by oxygen, -S-, SO₂, -NH-, -NCH₃ or -N-benzyl;
R(4) and R(5)
independently of one another are hydrogen, alkyl having 1, 2, 3 or 4 carbon atoms, F, Cl, -OR(69), -NR(70)R(71) or -C₂F_{2z+1};
R(69), R(70) and R(71)
independently of one another are hydrogen or alkyl having 1, 2 or 3 carbon atoms;
z is 1, 2, 3 or 4;
R(6) and R(7)
independently of one another are hydrogen or alkyl having 1, 2, 3 or 4 carbon atoms;
X is oxygen or NR(72);
R(72) is hydrogen or alkyl having 1, 2, 3 or 4
carbon atoms;
and their pharmaceutically tolerable salts;
ak) alkenylcarboxylic acid guanidides, carrying fluorophenyl groups, of the formula I 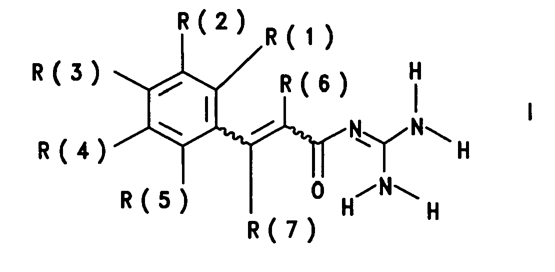 in which:
R(6) is hydrogen, (C₁-C₈))-alkyl, (C₃-C₈)-cycloalkyl or phenyl,
where the phenyl group is not substituted or is substituted by 1 - 3 substituents selected from the group consisting of F, Cl, CF₃, methyl, methoxy and NR(9)R(10);
R(9) and R(10)
are hydrogen, (C₁-C₄)-alkyl or (C₁-C₄)-perfluoroalkyl;
R(7) independently is defined as R(6);
R(1), R(2), R(3), R(4) and R(5) independently of one another are hydrogen or F; where, however, at least one of the radicals R(1), R(2), R(3), R(4) and R(5) must be fluorine;
and their pharmaceutically tolerable salts;
al) benzoylguanidines of the formula I 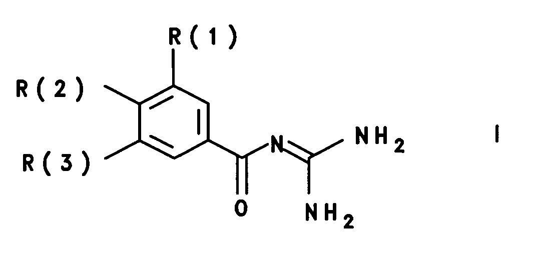 in which:
R(1) is R(4)-SOₘ,
or R(5)R(6)N-SO₂-;
m is 1 or 2;
R(4) and R(5)
independently of one another are alkyl having 1, 2, 3, 4, 5, 6, 7 or 8 carbon atoms, alkenyl having 3, 4, 5 or 6 carbon atoms, CF₃ or -CₙH₂ₙ-R(7);
n is zero, 1, 2, 3 or 4;
R(6) is H or alkyl having 1, 2, 3 or 4 carbon atoms;
R(7) is cycloalkyl having 3, 4, 5, 6, 7 or 8 carbon atoms or phenyl,
which is not substituted or is substituted by 1 - 3 substituents selected from the group consisting of F, Cl, CF₃, methyl, methoxy and NR(8)R(9);
R(8) and R(9)
are H or alkyl having 1, 2, 3 or 4 carbon atoms;
or
R(5) is also hydrogen;
or
R(5) and R(6)
together are 4 or 5 methylene groups, of which a CH₂ group can be replaced by oxygen,
S, NH, N-CH₃ or N-benzyl;
or
R(1) is -Oₚ-(CH₂)_{q}-(CF₂)ᵣ-CF₃;
p is zero or 1;
q is zero, 1 or 2;
r is zero, 1, 2 or 3;
or
R(1) is -SR(10), -OR(10) or -CR(10)R(11)R(12); R(10), R(11) and R(12)
independently of one another are hydrogen, alkyl having 1, 2, 3, 4, 5, 6, 7 or 8 carbon atoms, -CₛH₂ₛ-(C₃-C₈)-cycloalkyl or an aromatic system selected from the group consisting of pyridyl, pyrrolyl, quinolyl, isoquinolyl, imidazolyl or phenyl;
s is zero, 1 or 2; where the aromatic systems pyridyl, pyrrolyl, quinolyl, isoquinolyl, imidazolyl and phenyl are unsubstituted or substituted by 1 - 3 substituents selected from the group consisting of F, Cl, CF₃, CH₃, methoxy, hydroxyl, amino, methylamino and dimethylamino;
R(2) is -(CH₂)ᵤ-(CF₂)ₜ-CF₃;
t is zero, 1, 2 or 3;
u is zero or 1;
R(3) is hydrogen or independently is defined as R(1); and their pharmaceutically tolerable salts;
am) substituted cinnamic acid guanidides of the formula 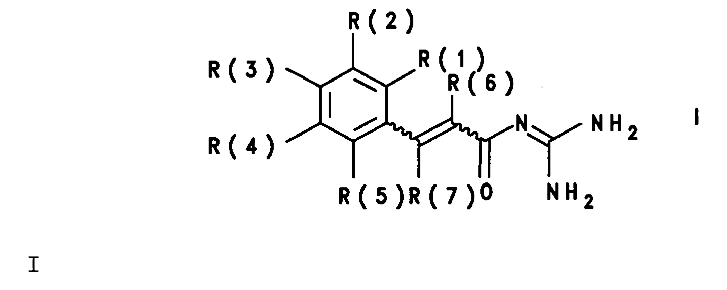 in which:
at least one of the substituents R(1), R(2), R(3), R(4) and R(5) is
-Xₐ-Y_{b}-Lₙ-U;
X is CR(16)R(17), O, S or NR(18);
R(16), R(17) and R(18)
independently of one another are H, alkyl having 1, 2, 3 or 4 carbon atoms or perfluoroalkyl having 1, 2, 3 or 4 carbon atoms;
a is zero or 1;
Y is alkylene having 1, 2, 3, 4, 5, 6, 7 or 8 carbon atoms, alkylene-T having 1, 2, 3, 4, 5, 6, 7 or 8 carbon atoms in the alkylene group, T, T-alkylene having 1, 2, 3, 4, 5, 6, 7 or 8 carbon atoms in the alkylene group;
T is NR(20), 0, S or phenylene,
where the phenylene is not substituted or is substituted by 1 - 3 substituents selected from the group consisting of F, Cl, CF₃, methyl, methoxy and NR(21)R(22); R(20), R(21) and R(22)
independently of one another are H, alkyl having 1, 2, 3 or 4 carbon atoms or perfluoroalkyl having 1, 2, 3 or 4 carbon atoms; b is zero or 1;
L is O, S, NR(23) or CₖH₂ₖ;
k is 1, 2, 3, 4, 5, 6, 7, 8;
n is zero or 1;
U is NR(24)R(25) or an N-containing heterocycle having 1, 2, 3, 4, 5, 6, 7, 8 or 9 carbon atoms;
R(24) and R(25)
independently of one another are hydrogen, alkyl having 1, 2, 3, 4, 5, 6, 7 or 8 carbon atoms or perfluoroalkyl having 1, 2, 3, 4, 5, 6, 7 or 8 carbon atoms;
or
R(24) and R(25)
together are 4 or 5 methylene groups, of which one CH₂ group can be replaced by oxygen, S, NH, N-CH₃ or N-benzyl;
where the N-containing heterocycles are N- or C-bridged and are not substituted or are substituted by 1 - 3 substituents selected from the group consisting of F, Cl, CF₃, methyl, methoxy and NR(27)R(28);
R(23), R(27) and R(28)
independently of one another are H, alkyl having 1, 2, 3 or 4 carbon atoms
or perfluoroalkyl having 1, 2, 3 or 4 carbon atoms;
and the other substituents R(1), R(2), R(3), R(4) and R(5) in each case
independently of one another are H, F, Cl, Br, I, CN, -Oₙ-CₘH₂ₘ₊₁, -Oₚ-(CH₂)ₛ-C_{q}F_{2q+1} or -CᵣH₂ᵣR(10);
n is zero or 1;
m is zero 1, 2, 3, 4, 5, 6, 7 or 8;
p is zero or 1;
q is 1, 2, 3, 4, 5, 6, 7 or 8;
s is zero, 1, 2, 3 or 4;
r is zero, 1, 2, 3 or 4;
R(10)
is cycloalkyl having 3, 4, 5, 6, 7 or 8 carbon atoms, or phenyl,
where the phenyl is not substituted or is substituted by 1 - 3 substituents selected from the group consisting of F, Cl, CF₃, methyl, methoxy and NR(11)R(12); R(11) and R(12)
independently of one another are H, alkyl having 1, 2, 3 or 4 carbon atoms or perfluoroalkyl having 1, 2, 3 or 4 carbon atoms;
R(6) and R(7) independently of one another are hydrogen, F, Cl, Br, I, CN, alkyl having 1, 2, 3, 4, 5, 6, 7 or 8 carbon atoms, perfluoroalkyl having 1, 2, 3, 4, 5, 6, 7 or 8 carbon atoms, cycloalkyl having 3, 4, 5, 6, 7 or 8 carbon atoms, or phenyl,
which is not substituted or is substituted by 1 - 3 substituents selected from the group consisting of F, Cl, CF₃, methyl, methoxy and NR(14)R(15);
R(14) and R(15)
independently of one another are H, alkyl having 1, 2, 3 or 4 carbon atoms or perfluoroalkyl having 1, 2, 3 or 4 carbon atoms;
and their pharmaceutically tolerable salts;
an) benzoylguanidines of the formula I 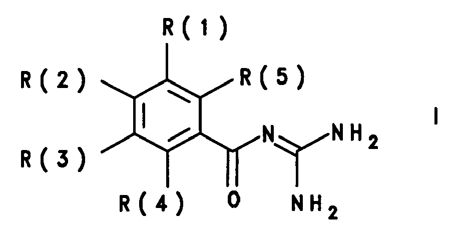 in which:
at least one of the substituents R(1), R(2) and R(3) is R(6)-C(OH)₂-;
R(6) is perfluoroalkyl having 1, 2 or 3 carbon atoms, which is straight-chain or branched; and the other substituents R(1), R(2) and R(3)
independently of one another are hydrogen, OH, F, Cl, Br, I, alkyl having 1, 2, 3, 4, 5 or 6 carbon atoms, cycloalkyl with 3, 4, 5 or 6 carbon atoms, alkoxy having 1, 2, 3 or 4 carbon atoms or phenoxy,
which is unsubstituted or is substituted by 1 - 3 substituents selected from the group consisting of F, Cl, methyl and methoxy;
or
the other substituents R(1), R(2) and R(3)
independently of one another are alkyl-SOₓ, - CR(7)=CR(8)R(9) or -C≡CR(9);
x is zero, 1 or 2;
R(7) is hydrogen or methyl;
R(8) and R(9)
independently of one another are hydrogen, alkyl having 1, 2, 3 or 4 carbon atoms, cycloalkyl having 3, 4, 5, 6, 7 or 8 carbon atoms or phenyl,
which is unsubstituted or is substituted by 1 - 3 substituents selected from the group consisting of F, Cl, CF₃, methyl and methoxy;
or
the other substituents R(1), R(2) and R(3) independently of one another are phenyl, C₆H₅-(C₁-C₄)-alkyl, naphthyl, biphenylyl, quinolinyl, isoquinolinyl or imidazolyl,
where quinolinyl, isoquinolinyl or imidazolyl are bonded via C or N and where phenyl, C₆H₅-(C₁-C₄)-alkyl, naphthyl, biphenylyl, quinolinyl, isoquinolinyl and imidazolyl are unsubstituted or are substituted by 1 - 3 substituents selected from the group consisting of F, Cl, CF₃, CH₃, methoxy, hydroxyl, amino, methylamino and dimethylamino;
or
the other substituents R(1), R(2) and R(3)
independently of one another are SR(10), -OR(10), -CR(10)R(11)R(12);
R(10)
is -C_{f}H_{2f}-(C₃-C₈)-cycloalkyl, quinolinyl, isoquinolinyl, pyridinyl, imidazolyl or phenyl,
where the aromatic systems quinolinyl, isoquinolinyl, pyridinyl, imidazolyl and phenyl are unsubstituted or are substituted by 1 - 3 substituents selected from the group consisting of F, Cl, CF₃, CH₃, methoxy, hydroxyl, amino, methylamino and dimethylamino;
f is zero, 1 or 2;
R(11) and R(12)
independently of one another are defined as R(10), hydrogen or alkyl having 1, 2, 3 or 4 carbon atoms;
R(4) and R(5) independently of one another are hydrogen, alkyl having 1, 2 or 3 carbon atoms, F, Cl, Br, I, CN, OR(13), NR(14)R(15), -(CH₂)ₙ-(CF₂)ₒ-CF₃;
R(13), R(14) and R(15)
independently of one another are hydrogen or alkyl having 1, 2, 3 or 4 carbon atoms;
n is zero or 1;
o is zero, 1 or 2;
and their pharmacologically acceptable salts;
ao) sulfonimidamides of the formula I 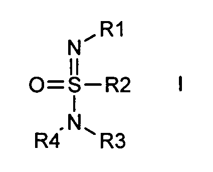 in which:
at least one of the three substituents R(1), R(2) and R(3)
is a benzoylguanidine, 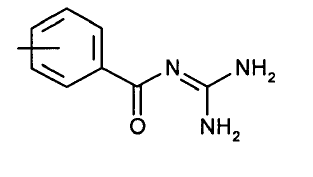 which is unsubstituted or substituted in the phenyl moiety by 1 - 4 radicals selected from the group consisting of alkyl having 1, 2, 3, 4, 5, 6, 7 or 8 carbon atoms, alkenyl having 2, 3, 4, 5, 6, 7 or 8 carbon atoms, -(CH₂)ₘ-R(14), F, Cl, Br, I, -C=N, CF₃, R(22)SO₂-, R(23)R(24)N-CO-, R(25)-CO-, R(26)R(27)N-SO₂, - OR(35), -SR(35) or -NR(35)R(36);
m is zero, 1 or 2;
R(14)
is -(C₃-C₈)-cycloalkyl or phenyl, which is not substituted or is substituted by 1 - 3 substituents selected from the group consisting of F and Cl, -CF₃, methyl, methoxy and -NR(15)R(16);
R(15) and R(16)
independently of one another are hydrogen or -CH₃;
R(22), R(23), R(25) and R(26) independently of one another are alkyl having 1, 2, 3, 4, 5, 6, 7 or 8 carbon atoms, alkenyl having 2, 3, 4, 5, 6, 7 or 8 carbon atoms, (CH₂)ₙR(29) or -CF₃;
n is zero, 1, 2, 3 or 4;
R(29) is -(C₃-C₇)-cycloalkyl or phenyl,
which is not substituted or is substituted by 1 - 3 substituents selected from the group consisting of F, Cl, -CF₃, methyl, methoxy and -NR(30)R(31);
R(30) and R(31)
are hydrogen or alkyl having 1, 2, 3 or 4 carbon atoms;
or
R(23), R(25) and R(26)
are hydrogen;
R(24) and R(27)
independently of one another are hydrogen or alkyl having 1, 2, 3 or 4 carbon atoms;
or
R(23) and R(24), and also R(26) and R(27)
together are 5 or 6 methylene groups, of which one CH₂ group can be replaced by oxygen, -S-, -NH-, -NCH₃ or -N-benzyl;
R(35) and R(36) independently of one another are hydrogen or alkyl having 1, 2, 3, 4, 5 or 6 carbon atoms;
or
R(35) and R(36)
together are 4 - 7 methylene groups, of which one CH₂ group can be replaced by oxygen, -S-, -NH-, -NCH₃ or -N-benzyl;
or
R(35)
is phenyl,
which is not substituted or is substituted by 1 - 3 substituents selected from the group consisting of F, Cl, -CF₃, methyl, methoxy, SO₂R(5), SO₂NR(6)R(7) and
-NR(32)R(33) ;
R(5) is alkyl having 1, 2, 3, 4, 5 or 6 carbon atoms
R(6) and R(7) independently of one another are hydrogen or alkyl having 1, 2, 3 or 4 carbon atoms;
R(32) and R(33)
independently of one another are hydrogen or alkyl having 1, 2, 3 or 4 carbon atoms;
or
R(35)
is C₁-C₉-heteroaryl,
which is unsubstituted or substituted by 1 - 3 substituents selected from the group consisting of F, Cl, CF₃, CH₃, methoxy, hydroxyl, amino, methylamino and dimethylamino;
and the other substituents R(1), R(2) and R(3) in each case
independently of one another are alkyl having 1, 2, 3, 4, 5, 6, 7 or 8 carbon atoms, (CH₂)ₚR(10)
p is zero, 1, 2, 3 or 4;
R(10) is phenyl,
which is not substituted or is substituted by 1 - 3 substituents selected from the group consisting of F, Cl, -CF₃, methyl, methoxy, -SO₂NR(17)R(8) and -SO₂R(9)
R(17) and R(8)
independently of one another are hydrogen or alkyl having 1, 2, 3 or 4 carbon atoms;
R(9) is alkyl having 1, 2, 3 or 4 carbon atoms;
or the other radicals R(1) and R(3) in each case are hydrogen,
R(4) is hydrogen or alkyl having 1, 2, 3 or 4 carbon atoms;
and their pharmaceutically tolerable salts;
ap) benzoylguanidines of the formula I 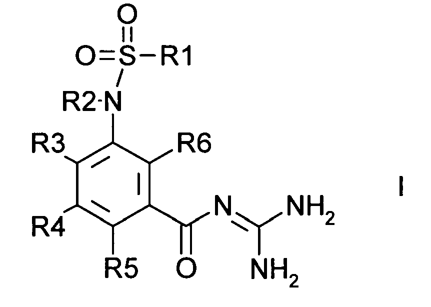 in which:
R(1) is alkyl having 1, 2, 3, 4, 5, 6, 7 or 8 carbon atoms or NR(7)R(8);
R(7) and R(8)
independently of one another are hydrogen or alkyl having 1, 2, 3, 4, 5, 6, 7 or 8 carbon atoms;
R(2) is hydrogen, alkyl having 1, 2, 3, 4, 5, 6, 7 or 8 carbon atoms or -SO₂R(9);
R(9) independently is defined as R(1);
R(3) is hydrogen, -SR(25), -OR(25), -NR(25)R(26) or - CR(25)R(26)R(27);
R(25) is hydrogen, alkyl having 1, 2, 3, 4, 5, 6, 7 or 8 carbon atoms, or phenyl,
which is unsubstituted or substituted by 1 - 3 substituents selected from the group consisting of F, Cl, CF₃, CH₃, methoxy, hydroxyl, amino, methylamino and dimethylamino;
or
R(25)
is - (C₁-C₉)-heteroaryl,
which is unsubstituted or substituted by 1 - 3 substituents selected from the group consisting of F, Cl, CF₃, CH₃, methoxy, hydroxyl, amino, methylamino and dimethylamino;
R(26) and R(27)
independently of one another are defined as R(25) or are hydrogen or alkyl having 1, 2, 3, 4, 5, 6, 7 or 8 carbon atoms;
R(4) is hydrogen, F, Cl, Br, I, OH, -C=N, CF₃, alkyl having 1, 2, 3, 4, 5, 6, 7 or 8 carbon atoms, alkenyl having 2, 3, 4, 5, 6, 7 or 8 carbon atoms or -(CH₂)ₘR(14);
m is zero, 1 or 2;
R(14) is -(C₃-C₈)-cycloalkyl or phenyl, which is not substituted or is substituted by 1 - 3 substituents selected from the group consisting of F and Cl, -CF₃, methyl, methoxy and - NR(15)R(16);
R(15) and R(16)
independently of one another are hydrogen or -CH₃;
R(5) and R(6) independently of one another are hydrogen, alkyl having 1, 2, 3 or 4 carbon atoms, F, Cl, -OR(32), -NR(33)R(34) or CF₃; R(32), R(33) and R(34)
independently of one another are hydrogen or alkyl having 1, 2, 3 or 4 carbon atoms; and their pharmaceutically tolerable salts;
aq) benzenedicarboxylic acid diguanidides of the formula I 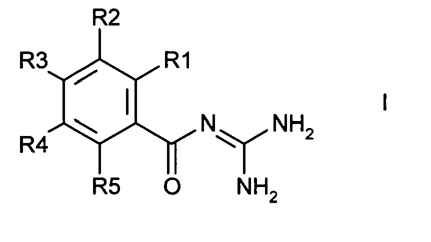 in which:
one of the radicals R(1), R(2), R(3) and R(4) is -CO-N=C(NH₂)₂;
and the other radicals R(1), R(2), R(3) and R(4) in each case are:
R(1) is hydrogen, alkyl having 1, 2, 3 or 4 carbon atoms, F, Cl, Br, I, -OR(32), -NR(33)R(34) or CF₃; R(32), R(33) and R(34)
independently of one another are hydrogen or alkyl having 1, 2, 3 or 4 carbon atoms;
R(2) and R(4) independently of one another are hydrogen, F, Cl, Br, I, OH, -CN, CF₃, -CO-N=C(NH₂)₂, alkyl having 1, 2, 3, 4, 5, 6, 7 or 8 carbon atoms, alkenyl having 2, 3, 4, 5, 6, 7 or 8 carbon atoms or -(CH₂)ₘR(14); m is zero, 1 or 2;
R(14) is -(C₃-C₈)-cycloalkyl or phenyl,
which is not substituted or is substituted by 1 - 3 substituents selected from the group consisting of F and Cl, -CF₃, methyl, methoxy and - NR(15)R(16);
R(15) and R(16)
are hydrogen or -CH₃;
or
R(2) and R(4) independently of one another are pyrrol-1-yl, pyrrol-2-yl or pyrrol-3-yl,
each of which is not substituted or is substituted by 1 - 4 substituents selected from the group consisting of F, Cl, Br, I, - CN, (C₂-C₈)-alkanoyl, (C₂-C₈)-alkoxycarbonyl, formyl, carboxyl, -CF₃, methyl, methoxy;
or
R(2) and R(4) independently of one another are R(22)-SO₂-, R(23)R(24)N-CO-, R(28)-CO- or R(29)R(30)N-SO₂; R(22) and R(28)
independently of one another are methyl or - CF₃;
R(23), R(24), R(29) and R(30)
independently of one another are hydrogen or methyl;
or
R(2) and R(4)
independently of one another are -OR(35) or - NR(35)R(36);
R(35) and R(36)
independently of one another are hydrogen or alkyl having 1, 2, 3, 4, 5 or 6 carbon atoms;
or
R(35) and R(36)
together are 4 - 7 methylene groups, of which one CH₂ group can be replaced by oxygen, -S-, -NH-, -NCH₃ or -N-benzyl;
R(3) is hydrogen, -SR(25), -OR(25), -NR(25)R(26), - CR(25)R(26)R(27);
R(25) is hydrogen, alkyl having 1, 2, 3, 4, 5, 6, 7 or 8 carbon atoms or phenyl,
which is unsubstituted or substituted by 1 - 3 substituents selected from the group consisting of F, Cl, CF₃, CH₃, methoxy, hydroxyl, amino, methylamino and dimethylamino; or
R (25) is -(C₁-C₉)-heteroaryl,
which is unsubstituted or substituted by 1 - 3 substituents selected from the group consisting of F, Cl, CF₃, CH₃, methoxy, hydroxyl, amino, methylamino and dimethylamino;
R(26) and R(27)
independently of one another are defined as R(25) or are hydrogen or alkyl having 1, 2, 3, 4, 5, 6, 7 or 8 carbon atoms;
R(5) is alkyl having 1, 2, 3 or 4 carbon atoms, F, Cl, Br, I, X-(CH₂)_{y}-CF₃ or phenyl,
which is not substituted or is substituted by 1 - 3 substituents selected from the group consisting of F and Cl, -CF₃, methyl, methoxy and -NR(6)R(7);
R(6) and R(7)
independently of one another are hydrogen or -CH₃;
X is a bond or oxygen;
y is zero, 1 or 2;
and their pharmaceutically tolerable salts;
ar) benzenedicarboxylic acid diguanidides of the formula I 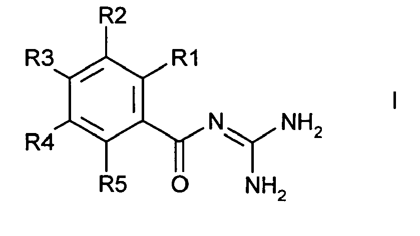 in which:
one of the radicals R(1), R(2), R(3) and R(5) is -CO-N=C( NH₂)₂;
and the other radicals R(1), R(2), R(3) and R(5) in each case are:
R(1) and R(5)
independently of one another are hydrogen, alkyl having 1, 2, 3 or 4 carbon atoms, F, Cl, -OR(32), -NR(33)R(34) or CF₃;
R(32), R(33) and R(34)
independently of one another are hydrogen or alkyl having 1, 2, 3 or 4 carbon atoms;
R(2) is hydrogen, F, Cl, Br, I, OH, -CN, CF₃, -CON=C(NH₂)₂, alkyl having 1, 2, 3, 4, 5, 6, 7 or 8 carbon atoms, alkenyl having 2, 3, 4, 5, 6, 7 or 8 carbon atoms or -(CH₂)ₘR(14);
m is zero, 1 or 2;
R(14) is -(C₃-C₈)-cycloalkyl or phenyl,
which is not substituted or is substituted by 1 - 3 substituents selected from the group consisting of F and Cl, -CF₃, methyl, methoxy and - NR(15)R(16);
R(15) and R(16)
independently of one another are
hydrogen or -CH₃;
or
R(2) is R(22)-SO₂-, R(23)R(24)N-CO-, R(28)-CO- or R(29)R(30)N-SO₂;
R(22) and R(28)
independently of one another are methyl or - CF₃;
R(23), R(24), R(29) and R(30)
independently of one another are hydrogen or methyl;
or
R(2) is -OR(35) or -NR(35)R(36); R(35) and R(36)
independently of one another are hydrogen or alkyl having 1, 2, 3, 4, 5 or 6 carbon atoms;
or
R(35) and R(36)
together are 4 - 7 methylene groups, of which one CH₂ group can be replaced by oxygen, -S-, -NH-, -NCH₃ or -N-benzyl;
R(3) is hydrogen, -SR(25), -OR(25), -NR(25)R(26), - CR(25)R(26)R(27);
R(25) is hydrogen, alkyl having 1, 2, 3, 4, 5, 6, 7 or 8 carbon atoms or phenyl,
which is unsubstituted or substituted by 1 - 3 substituents selected from the group consisting of F, Cl, CF₃, CH₃, methoxy, hydroxyl, amino, methylamino and dimethylamino;
or
R(25) is -(C₁-C₉)-heteroaryl,
which is unsubstituted or substituted by 1 - 3 substituents selected from the group consisting of F, Cl, CF₃, CH₃, methoxy, hydroxyl, amino, methylamino and dimethylamino;
R(26) and R(27)
independently of one another are defined as R(25) or are hydrogen or alkyl having 1, 2, 3, 4, 5, 6, 7 or 8 carbon atoms;
R(4) is CF₃, alkyl having 1, 2, 3, 4, 5, 6, 7 or 8 carbon atoms, alkenyl having 2, 3, 4, 5, 6, 7 or 8 carbon atoms, -(C₃-C₈)-cycloalkyl or -(CH₂)ₘR(14);
m is 1 or 2;
R(14) is -(C₃-C₈)-cycloalkyl or phenyl,
which is not substituted or is substituted by 1 - 3 substituents selected from the group consisting of F and Cl, -CF₃, methyl, methoxy and - NR(15) R(16) ;
R(15) and R(16)
independently of one another are hydrogen or -CH₃;
or
R(4) is phenyl, which is substituted by 2, 3, 4 or five substituents selected from the group consisting of F, Cl, CF₃, methyl, methoxy and -NR(15)R(16);
R(15) and R(16)
independently of one another are hydrogen or CH₃;
and their pharmaceutically tolerable salts;
as) diaryldicarboxylic acid diguanidides of the formula I 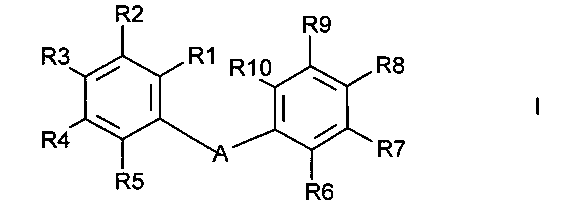 in which:
one of the radicals R(1), R(2), R(3), R(4) and R(5) is -CO-N=C(NH₂)₂;
the other radicals R(1) and R(5) in each case independently of one another are hydrogen, alkyl having 1, 2, 3 or 4 carbon atoms, F, Cl, -OR(32), -NR(33)R(34) or CF₃;
R(32), R(33) and R(34)
independently of one another are hydrogen or alkyl having 1, 2, 3 or 4 carbon atoms;
the other radicals R(2) and R(4) in each case independently of one another are hydrogen, F, Cl, Br, I, OH, -CN, CF₃, -CO-N=C(NH₂)₂, alkyl having 1, 2, 3, 4, 5, 6, 7 or 8 carbon atoms, alkenyl having 2, 3, 4, 5, 6, 7 or 8 carbon atoms or -(CH₂)ₘR(14); m is zero, 1 or 2;
R(14) is -(C₃-C₈)-cycloalkyl or phenyl,
which is not substituted or is substituted by 1 - 3 substituents selected from the group consisting of F and Cl, -CF₃, methyl, methoxy and - NR(15) R(16) ;
R(15) and R(16)
are hydrogen or -CH₃;
or
the other radicals R(2) and R(4) in each case independently of one another are pyrrol-1-yl, pyrrol-2-yl or pyrrol-3-yl, which is not substituted or is substituted by 1 - 4 substituents selected from the group consisting of F, Cl, Br, I, -CN, (C₂-C₈)-alkanoyl, (C₂-C₈)-alkoxycarbonyl, formyl,
carboxyl, -CF₃, methyl, methoxy;
or
the other radicals R(2) and R(4) in each case
are R(22)-SO₂-, R(23)R(24)N-CO-, R(28)-CO- or R(29)R(30)N-SO₂;
R(22) and R(28)
independently of one another are methyl or - CF₃;
R(23), R(24), R(29) and R(30)
independently of one another are hydrogen or methyl;
or
the other radicals R(2) and R(4) in each case independently of one another are -OR(35) or - NR(35)R(36);
R(35) and R(36)
independently of one another are hydrogen or alkyl having 1, 2, 3, 4, 5 or 6 carbon atoms;
or
R(35) and R(36)
together are 4 - 7 methylene groups, of which one CH₂ group can be replaced by oxygen, -S-, -NH-, -NCH₃ or -N-benzyl;
the other radical R(3) in each case
is hydrogen, -SR(25), -OR(25), -NR(25)R(26), - CR(25)R(26)R(27);
R(25) is hydrogen, alkyl having 1, 2, 3, 4, 5, 6, 7 or 8 carbon atoms or phenyl,
which is unsubstituted or substituted by 1 - 3 substituents selected from the group consisting of F, Cl, CF₃, CH₃, methoxy, hydroxyl, amino, methylamino and dimethylamino;
or
R (25) is -(C₁-C₉)-heteroaryl,
which is unsubstituted or substituted by 1 - 3 substituents selected from the group consisting of F, Cl, CF₃, CH₃, methoxy, hydroxyl, amino, methylamino and dimethylamino;
R(26) and R(27)
independently of one another are defined as R(25) or are hydrogen or alkyl having 1, 2, 3, 4, 5, 6, 7 or 8 carbon atoms;
one of the radicals R(6), R(7), R(8), R(9) and R(10) is -CO-N=C(NH₂)₂;
the other radicals R(6) and R(10) in each case independently of one another are hydrogen, alkyl having 1, 2, 3 or 4 carbon atoms, F, Cl, -OR(132), -NR(133)R(134) or CF₃;
R(132), R(133) and R(134)
independently of one another are hydrogen or alkyl having 1, 2, 3 or 4 carbon atoms;
the other radicals R(7) and R(9) in each case independently of one another are hydrogen, F, Cl, Br, I, OH, -CN, CF₃, -CO-N=C(NH₂)₂, alkyl having 1, 2, 3, 4, 5, 6, 7 or 8 carbon atoms, alkenyl having 2, 3, 4, 5, 6, 7 or 8 carbon atoms or -(CH₂)ₘₘR(114);
mm is zero, 1 or 2;
R(114)
is -(C₃-C₈)-cycloalkyl or phenyl, which is not substituted or is substituted by 1 - 3 substituents selected from the group consisting of F and Cl, -CF₃, methyl, methoxy and - NR(115)R(116) ;
R(115) and R(116)
are hydrogen or -CH₃;
or
the other radicals R(7) and R(9) in each case independently of one another are pyrrol-1-yl, pyrrol-2-yl or pyrrol-3-yl,
which is not substituted or is substituted by 1 - 4 substituents selected from the group consisting of F, Cl, Br, I, -CN, (C₂-C₈) - alkanoyl, (C₂-C₈)-alkoxycarbonyl, formyl, carboxyl, -CF₃, methyl and methoxy;
or
the other radicals R(7) and R(9) in each case
are R(122)-SO₂-, R(123)R(124)N-CO-, R(128)-CO- or R(129)R(130)N-SO₂;
R(122) and R(128)
independently of one another are methyl or - CF₃;
R(123), R(124), R(129) and R(130)
independently of one another are hydrogen or methyl;
or
the other radicals R(7) and R(9) in each case independently of one another are -OR(135) or - NR(135)R(136);
R(135) and R(136)
independently of one another are hydrogen or alkyl having 1, 2, 3, 4, 5 or 6 carbon atoms;
or
R(135) and R(136) together are 4 - 7 methylene groups, of which one CH₂ group can be replaced by oxygen, -S-, -NH-, -NCH₃ or -N-benzyl;
the other radical R(8) in each case
is hydrogen, -SR(125), -OR(125), -NR(125)R(126) or -CR(125)R(126)R(127);
R(125)
is hydrogen, alkyl having 1, 2, 3, 4, 5, 6, 7 or 8 carbon atoms or phenyl,
which is unsubstituted or substituted by 1 - 3 substituents selected from the group consisting of F, Cl, CF₃, CH₃, methoxy, hydroxyl, amino, methylamino and dimethylamino;
or
R(125)
is -(C₁-C₉)-heteroaryl,
which is unsubstituted or substituted by 1 - 3 substituents selected from the
group consisting of F, Cl, CF₃, CH₃, methoxy, hydroxyl, amino, methylamino and dimethylamino;
R(126) and R(127)
independently of one another are defined as R(125) or are hydrogen or alkyl having 1, 2, 3, 4, 5, 6, 7 or 8 carbon atoms;
A is absent or is -NR(11)-CO-, -NR(12)-CO-NR(13)-, -NR(17)-CO-NR(18)-SO₂-, -NR(19)-SO₂-, -SO₂-NR(19)-SO₂-, -SO₂-NR(19)-CO-, -O-CO-NR(19)-SO₂- or -CR(20)=CR(21)-;
R(11), R(12), R(13), R(17), R(18), R(19), R(20) and R(21)
independently of one another are hydrogen or alkyl having 1, 2, 3, 4, 5, 6, 7 or 8 carbon atoms
and their pharmaceutically tolerable salts;
at) substituted thiophenylalkenylcarboxylic acid guanidides of the formula I 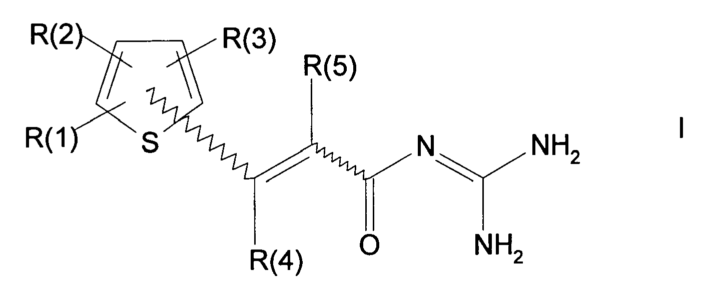 in which:
at least one of the substituents R(1), R(2) and R(3) is -Oₚ-(CH₂)ₛ-C_{q}F_{2q}+1, R(40)CO- or R(31)SOₖ-;
p is zero or 1;
s is zero, 1, 2, 3 or 4;
q is 1, 2, 3, 4, 5, 6, 7 or 8;
k is zero, 1 or 2;
R(40) is alkyl having 1, 2, 3, 4, 5, 6, 7 or 8 carbon atoms, perfluoroalkyl having 1, 2, 3, 4, 5, 6, 7 or 8 carbon atoms, cycloalkyl having 3, 4, 5, 6, 7 or 8 carbon atoms, or phenyl,
which is not substituted or is substituted by 1 - 3 substituents selected from the group consisting of F, Cl, CF₃, methyl and methoxy;
R(31) is alkyl having 1, 2, 3, 4, 5, 6, 7 or 8 carbon atoms, perfluoroalkyl having 1, 2, 3, 4, 5, 6, 7 or 8 carbon atoms, cycloalkyl having 3, 4, 5, 6, 7 or 8 carbon atoms, or phenyl,
which is not substituted or is substituted by 1 - 3 substituents selected from the group consisting of F, Cl, CF₃, methyl or methoxy;
or
R(31) is NR(41)R(42);
R(41) and R(42)
independently of one another are hydrogen, alkyl having 1, 2, 3 or 4 carbon atoms, perfluoroalkyl having 1, 2, 3 or 4 carbon atoms,
or
R(41) and R(42)
together are 4 or 5 methylene groups, of which one CH₂ group can be replaced by oxygen, S, NH, N-CH₃ or N-benzyl;
and the other substituents R(1), R(2) and R(3) in each case
independently of one another are H, F, Cl, Br, I, CN, -Oₙₐ-CₘₐH₂ₘₐ₊₁ or -O_{ga}CᵣₐH₂ᵣₐR(10);
na is zero or 1;
ma is zero, 1, 2, 3, 4, 5, 6, 7 or 8;
ga is zero or 1;
ra is zero, 1, 2, 3 or 4;
R(10) is cycloalkyl having 3, 4, 5, 6, 7 or 8 carbon atoms or phenyl,
where the phenyl is not substituted or is substituted by 1 - 3 substituents selected from the group consisting of F, Cl, CF₃, methyl and methoxy;
R(4) and R(5) independently of one another are hydrogen, F, Cl, Br, I, CN, alkyl having 1, 2, 3, 4, 5, 6, 7 or 8 carbon atoms, perfluoroalkyl having 1, 2, 3, 4, 5, 6, 7 or 8 carbon atoms, cycloalkyl having 3, 4, 5, 6, 7 or 8 carbon atoms or phenyl,
which is not substituted or is substituted by 1 - 3 substituents selected from the group consisting of F, Cl, CF₃, methyl, methoxy and NR(14)R(15);
R(14) and R(15)
independently of one another are H, alkyl having 1, 2, 3 or 4 carbon atoms or perfluoroalkyl having 1, 2, 3 or 4 carbon atoms;
and their pharmaceutically tolerable salts;
au) ortho-substituted benzoylguanidines of the formula I 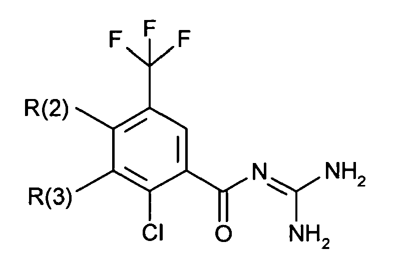 in which:
R(2) and R(3)
independently of one another are hydrogen, Cl, Br, I, (C₁-C₈)-alkyl, (C₃-C₈)-cycloalkyl or -OR(5); R(5) is (C₁-C₈)-alkyl or -C_{d}H_{2d}-(C₃-C₈)-cycloalkyl;
d is zero, 1 or 2;
where one of the two substituents R(2) and R(3) is always hydrogen but both substituents R(2) and R(3) are not simultaneously hydrogen,
and their pharmaceutically tolerable salts;
av) benzoylguanidines of the formula I 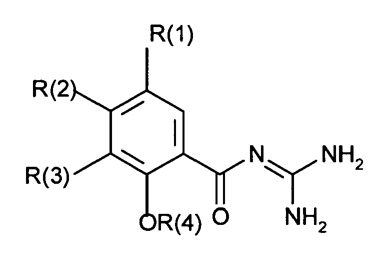 in which:
R(1) is H, F, Cl, Br, I, CN, NO₂, alkyl having 1, 2, 3, 4, 5, 6, 7 or 8 carbon atoms, alkoxy having 1, 2, 3, 4, 5, 6, 7 or 8 carbon atoms, cycloalkyl having 3, 4, 5, 6, 7 or 8 carbon atoms, cycloalkoxy having 3, 4, 5, 6, 7 or 8 carbon atoms or Xₐ-(CH₂) _{b}-(CF₂)_{c}-CF₃;
X is oxygen, S, NR(5),
a is zero or 1;
b is zero, 1 or 2;
c is zero, 1, 2 or 3;
R(5) is H, alkyl having 1, 2, 3 or 4 carbon atoms or -C_{d}H_{2d}R(6);
d is zero, 1, 2, 3 or 4;
R(6) is cycloalkyl having 3, 4, 5, 6, 7 or 8 carbon atoms, phenyl, biphenylyl or naphthyl,
where the aromatics phenyl, biphenylyl or naphthyl are not substituted or are substituted by 1 - 3 substituents selected from the group consisting of F, Cl, CF₃, methyl, methoxy and NR(7)R(8);
R(7) and R(8)
independently are H or alkyl having 1, 2, 3 or 4 carbon atoms;
or
R(1) is -SR(10), -OR(10) or -CR(10)R(11)R(12);
R(10) is -C_{f}H_{2f}-cycloalkyl having 3, 4, 5, 6, 7
or 8 carbon atoms in the cycloalkyl ring, or phenyl,
where phenyl is unsubstituted or substituted by 1 - 3 substituents selected from the group consisting of F, Cl, CF₃, CH₃, methoxy, hydroxyl, amino, methylamino and dimethylamino;
f is zero, 1 or 2;
R(11) and R(12)
independently of one another are defined as R(10) or are hydrogen or alkyl having 1, 2, 3 or 4 carbon atoms;
or
R(1) is phenyl, naphthyl, biphenylyl or heteroaryl having 1, 2, 3, 4, 5, 6, 7, 8 or 9 carbon atoms, with the latter being linked via a carbon atom or a nitrogen atom of the ring,
which are in each case unsubstituted or substituted by 1 - 3 substituents selected from the group consisting of F, Cl, CF₃, CH₃, methoxy, hydroxyl, amino, methylamino and dimethylamino,
or
R(1) is -SR(13), -OR(13), -NHR(13), -NR(13)R(14), - CHR(13)R(15), -C[R(15)R(16)OH], -C≡CR(18), - C[R(19)]=CHR(18), -C[R(20)R(21)]ₖ-(CO)-[CR(22)R(23)]₁-R(24),
k is zero, 1, 2, 3 or 4;
1 is zero, 1, 2, 3 or 4;
R(13) and R(14)
identically or differently are -(CH₂)g-(CHOH) h-(CH₂)ᵢ-(CHOH)ⱼ-R(17) or -(CH₂)_{g}-O-(CH₂-CH₂ O)ₕ-R(24);
R(17) is hydrogen or methyl,
g, h and i
identically or differently are zero, 1, 2, 3 or 4;
j is 1, 2, 3 or 4;
R(15) and R(16)
identically or differently are hydrogen, alkyl having 1, 2, 3, 4, 5 or 6 carbon atoms or, together with the carbon atom carrying them, are cycloalkyl having 3, 4, 5, 6, 7 or 8 carbon atoms;
R(18) is phenyl,
which is unsubstituted or substituted by 1 - 3 substituents selected from the group consisting of F, Cl, CF₃, methyl, methoxy and NR(25)R(26); R(25) and R(26)
are H or alkyl having 1, 2, 3 or 4 carbon atoms;
or
R(18) is heteroaryl having 1, 2, 3, 4, 5, 6, 7, 8 or 9 carbon atoms,
which is unsubstituted or substituted as phenyl;
or
R(18) is alkyl having 1, 2, 3, 4, 5 or 6 carbon atoms,
which is unsubstituted or substituted by 1 - 3 OH;
or
R(18)
is cycloalkyl having 3, 4, 5, 6, 7 or 8 carbon atoms;
R(19), R(20), R(21), R(22) and R(23) identically or differently are hydrogen or methyl;
R(24) is H, alkyl having 1, 2, 3, 4, 5 or 6 carbon atoms, cycloalkyl having 3, 4, 5, 6, 7 or 8 carbon atoms or -CₘH₂ₘ-R(18);
m is 1, 2, 3 or 4;
R(2) and R(3)
are defined as R(1);
R(4) is alkyl having 1, 2, 3 or 4 carbon atoms; and their pharmaceutically tolerable salts;
aw) ortho-substituted benzoylguanidines of the formula I 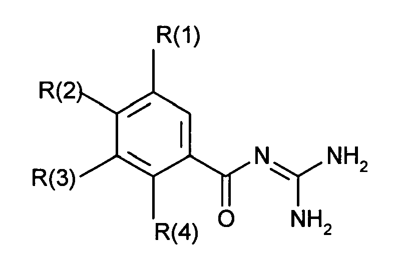 in which:
R(1) is H, F, Cl, Br, I, CN, NO₂, alkyl having 1, 2, 3, 4, 5, 6, 7 or 8 carbon atoms, alkoxy having 1, 2, 3, 4, 5, 6, 7 or 8 carbon atoms, cycloalkyl having 3, 4, 5, 6, 7 or 8 carbon atoms, cycloalkoxy having 3, 4, 5, 6, 7 or 8 carbon atoms or Xₐ-(CH₂) _{b}-(CF₂)_{c}-CF₃;
X is oxygen, S, NR(5),
a is zero or 1;
b is zero, 1 or 2;
c is zero, 1, 2 or 3;
R (5) is H, alkyl having 1, 2, 3 or 4 carbon atoms or -C_{d}H_{2d}R(6);
d is zero, 1, 2, 3 or 4;
R(6) is cycloalkyl having 3, 4, 5, 6, 7 or 8 carbon atoms, phenyl, biphenylyl or naphthyl,
where the aromatics phenyl, biphenylyl or naphthyl are unsubstituted or substituted by 1 - 3 substituents selected from the group consisting of F, Cl, CF₃, methyl, methoxy and NR(7)R(8);
R(7) and R(8)
independently are H or alkyl having 1, 2, 3 or 4 carbon atoms;
or
R(1) is -SR(10), -OR(10) or -CR(10)R(11)R(12); R(10) is -C_{f}H_{2f}-cycloalkyl having 3, 4, 5, 6, 7 or 8 carbon atoms in the cycloalkyl ring, or phenyl,
where phenyl is unsubstituted or substituted by 1 - 3 substituents selected from the group consisting of F, Cl, CF₃, CH₃, methoxy, hydroxyl, amino, methylamino and dimethylamino;
f is zero, 1 or 2;
R(11) and R(12)
independently of one another are defined as R(10), or are hydrogen or alkyl having 1, 2, 3 or 4 carbon atoms;
or
R(1) is phenyl, naphthyl, biphenylyl or heteroaryl having 1, 2, 3, 4, 5, 6, 7, 8 or 9 carbon atoms, with the latter being linked via a carbon atom or a nitrogen atom of the ring,
which are in each case unsubstituted or substituted by 1 - 3 substituents selected from the group consisting of F, Cl, CF₃, CH₃, methoxy, hydroxyl, amino, methylamino and dimethylamino,
or
R(1) is -SR(13), -OR(13), -NHR(13), -NR(13)R(14), - CHR(13)R(15), -C[R(15)R(16)OH], -C≡CR(18), - C[R(19)]=CHR(18), -C[R(20)R(21)]ₖ-(CO)-[CR(22)R(23)]₁-R(24),
k is zero, 1, 2, 3 or 4;
1 is zero, 1, 2, 3 or 4;
R(13) and R(14)
identically or differently are - (CH₂)g-(CHOH) ₕ-(CH₂)ᵢ-(CHOH)ⱼ-R(17) or -(CH₂)_{g}-O-(CH₂-CH₂ O)ₕ-R(24);
R(17) is hydrogen or methyl,
g, h and i
identically or differently are zero, 1, 2, 3 or 4;
j is 1, 2, 3 or 4;
R(15) and R(16)
identically or differently are hydrogen, alkyl having 1, 2, 3, 4, 5 or 6 carbon atoms or, together with the carbon atom carrying them, are cycloalkyl having 3, 4, 5, 6, 7 or 8 carbon atoms;
R(18)
is phenyl,
which is unsubstituted or substituted by 1 - 3 substituents selected from the group consisting of F, Cl, CF₃, methyl, methoxy and NR(25)R(26);
R(25) and R(26)
are H or alkyl having 1, 2, 3 or 4 carbon atoms;
or
R(18) is heteroaryl having 1, 2, 3, 4, 5, 6, 7, 8 or 9 carbon atoms,
which is unsubstituted or substituted as phenyl;
or
R(18) is alkyl having 1, 2, 3, 4, 5 or 6 carbon atoms,
which is unsubstituted or substituted by 1 - 3 OH;
or
R (18)
is cycloalkyl having 3, 4, 5, 6, 7 or 8 carbon atoms;
R(19), R(20), R(21), R(22) and R(23)
identically or differently are hydrogen or methyl;
R(24) is H, alkyl having 1, 2, 3, 4, 5 or 6 carbon atoms, cycloalkyl having 3, 4, 5, 6, 7 or 8 carbon atoms or -CₘH₂ₘ-R(18);
m is 1, 2, 3 or 4;
one of the two substituents R(2) and R(3) is hydroxyl;
and
the other of the substituents R(2) and R(3) in each case
is defined as R(1);
R(4) is alkyl having 1, 2, 3 or 4 carbon atoms; alkoxy having 1, 2, 3 or 4 carbon atoms, F, Cl, Br, I or - (CH₂)ₙ- (CF₂)ₒ-CF3;
n is zero or 1;
o is zero or 1;
and their pharmaceutically tolerable salts.
ax) bisortho-substituted benzoylguanidines of the formula I 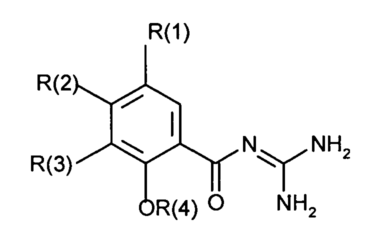 in which:
R(1), R(2) and R(3)
independently of one another are R(10)-SOₐ- or R(14)R(15)N-SO₂-;
a is zero, 1 or 2,
R(10), R(14) and R(15)
independently of one another are alkyl having 1, 2, 3, 4, 5, 6, 7 or 8 carbon atoms, perfluoroalkyl having 1, 2, 3, 4, 5, 6, 7 or 8 carbon atoms, alkenyl having 3, 4, 5 or 6 carbon atoms or -C_{ab}H_{2ab}-R(16) ;
ab is zero, 1, 2, 3 or 4;
R(16) is cycloalkyl having 3, 4, 5, 6 or 7 carbon atoms or phenyl,
which is unsubstituted or substituted by 1-3 substituents selected from the group consisting of F, Cl, CF₃, methyl, methoxy and NR(17)R(18);
R(17) and R(18)
independently of each other are hydrogen, CF₃ or alkyl having 1, 2, 3 or 4 carbon atoms; or
R(14) and R(15)
together are 4 or 5 methylene groups, of which one CH₂ group can be replaced by oxygen, sulfur, NH, N-CH₃ or N-benzyl;
or
R(14) and R(15)
are hydrogen;
or
R(1), R(2) and R(3) independently of each other are SR(21), -OR(22), -NR(23)R(24) or -CR(25)R(26)R(27); R(21), R(22), R(23) and R(25)
independently of one another are -C_{b}H_{2b}- (C₁-C₉) -heteroaryl,
which is unsubstituted or substituted by 1-3 substituents selected from the group consisting of F, Cl, CF₃, CH₃, methoxy, hydroxyl, amino, methylamino and dimethylamino;
b is zero, 1 or 2;
R(24), R(26) and R(27)
independently of each other are hydrogen, alkyl having 1, 2, 3 or 4 carbon atoms or perfluoroalkyl having 1, 2, 3 or 4 carbon atoms;
or
R(1), R(2) and R(3) independently of one another are hydrogen, F, Cl, Br, I, CN, -(Xa)_{dg}-C_{da}H_{2da+1}, -(X_{b})_{dh}-(CH₂)_{db}-C_{de}F_{2de+1}, alkenyl having 3, 4, 5, 6, 7 or 8 carbon atoms or -C_{df}H_{2df}R(30) ;
(Xa) is oxygen, sulfur or NR(33);
R(33) is hydrogen, alkyl having 1, 2, 3 or 4 carbon atoms or perfluoroalkyl having 1, 2, 3 or 4 carbon atoms;
dg is zero or 1;
(Xb) is oxygen, sulfur or NR(34);
R(34) is hydrogen, alkyl having 1, 2, 3, or 4 carbon atoms or perfluoroalkyl having 1, 2, 3 or 4 carbon atoms;
dh is zero or 1;
da is zero, 1, 2, 3, 4, 5, 6, 7 or 8;
db is zero, 1, 2, 3 or 4;
de is zero, 1, 2, 3, 4, 5, 6 or 7;
df is zero, 1, 2, 3 or 4;
R(30) is cycloalkyl having 3, 4, 5, 6, 7 or 8
carbon atoms, phenyl, biphenylyl or naphthyl, where the aromatics phenyl, biphenylyl or naphthyl are not substituted or substituted by 1-3 substituents selected from the group consisting of F, Cl, CF₃, methyl, methoxy and NR(31)R(32);
R(31) and R(32)
are hydrogen, alkyl having 1, 2, 3 or 4 carbon atoms or perfluoroalkyl having 1, 2, 3 or 4 carbon atoms;
or
R(1), R(2) and R(3) independently of one another are NR(40)R(41) or -(Xe)-(CH₂)_{eb}R(45);
R(40) and R(41)
independently of one another are hydrogen, alkyl having 1, 2, 3, 4, 5, 6, 7 or 8 carbon atoms, perfluoroalkyl having 1, 2, 3, 4, 5, 6, 7 or 8 carbon atoms or (CH₂)ₑ-R(42);
e is zero, 1, 2, 3 or 4;
R(42) is cycloalkyl having 3, 4, 5, 6 or 7 carbon atoms or phenyl,
which is not substituted or substituted by 1-3 substituents selected from the group consisting of F, Cl, CF₃, methyl, methoxy and NR(43)R(44);
R(43) and R(44) independently of one another are hydrogen, CF₃ or alkyl having 1, 2, 3 or 4 carbon atoms;
or
R(40) and R(41) together are 4 or 5 methylene groups, of which one CH₂ group can be replaced by oxygen, sulfur, NH, N-CH₃ or N-benzyl;
(Xe) is oxygen, sulfur or NR(47);
R(47) is hydrogen, alkyl having 1, 2, 3 or 4
carbon atoms or perfluoroalkyl having 1, 2, 3
or 4 carbon atoms;
eb is zero, 1, 2, 3 or 4;
R(45) is cycloalkyl having 3, 4, 5, 6 or 7 carbon atoms or phenyl,
which is not substituted or substituted by 1-3 substituents selected from the group consisting of F, Cl, CF₃, methyl, methoxy, NR(50)R(51) and -(Xfa)-(CH₂)_{ed}-(Xfb)R(46);
Xfa is CH₂, oxygen, sulfur or NR(48);
Xfb is oxygen, sulfur or NR(49);
R(48), R(49), R(50) and R(51) independently of one another are hydrogen, alkyl having 1, 2, 3 or 4 carbon atoms or perfluoroalkyl having 1, 2, 3 or 4 carbon atoms;
ed is 1, 2, 3 or 4;
R(46) is hydrogen, alkyl having 1, 2, 3 or 4 carbon atoms or perfluoroalkyl having 1, 2, 3 or 4 carbon atoms; or
R(1), R(2) and R(3) independently of one another are -CHR(52)R(53); R(52) is -(CH₂)_{g}-(CHOH)ₕ-(CH)ᵢ-(CHOH)ₖ-R(54) or - (CH₂)_{g}-O-(CH₂-CH₂O)h-R (54);
R(54) is hydrogen or methyl;
g, h, i
are identical or different and are zero, 1, 2, 3 or 4;
k is 1, 2, 3 or 4;
R(53) is hydrogen or alkyl having 1, 2, 3 or 4 carbon atoms;
or
R(1), R(2) and R(3)
independently of one another are -C(OH)R(55)R(56); R(55) and R(56)
are identical or different and are hydrogen or alkyl having 1, 2, 3 or 4 carbon atoms;
or
R(55) and R(56)
together are cycloalkyl having 3, 4, 5 or 6 carbon atoms;
or
R(55) is -CH₂OH;
and
R(4) and R(5) independently of one another are alkyl having 1, 2, 3 or 4 carbon atoms, alkoxy having 1, 2, 3 or 4 carbon atoms, OH, F, Cl, Br, I, CN, -On- (CH₂)ₒ- (CF₂)ₚ-CF₃;
n is zero or 1;
o is zero, 1 or 2;
p is zero, 1 or 2;
and their pharmaceutically tolerable salts.
ay) substituted 1-naphthoylguanidines of the formula I 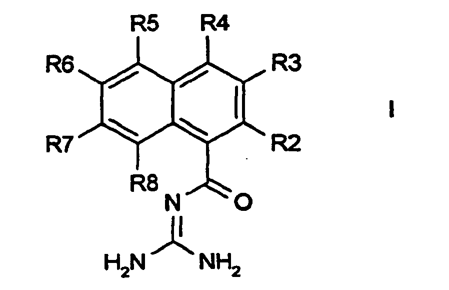 in which:
R2, R3, R4, R5, R6, R7 and R8 independently of one another are H, F, Cl, Br, I, CN, NO₂, CF₃, C₂F₅ or XₐY_{b}Z;
X is O, S, NR(10), CR(11)R(12), C=O, C(=O)NR(10), C(=O)O, SO, SO₂, SO₂NR(10), OC=O, NR(10)C=O or NR(10)SO₂, where the linkage with the naphthalene ring is in each case effected through the atom on the left;
R(10), R(11) and R(12)
independently of one another are H, alkyl having 1, 2, 3, 4, 5 or 6 carbon atoms, perfluoroalkyl having 1, 2, 3 or 4 carbon atoms or cycloalkyl having 3, 4, 5, 6 or 7 carbon atoms;
a is zero or 1;
Y is alkylene having 1, 2, 3, 4, 5, 6, 7 or 8 CH₂ groups;
it being possible for one of these CH₂ groups to be replaced by O, S, NR(13) or o-, p- or m-phenylene;
R(13) is H, alkyl having 1, 2, 3, 4, 5 or 6 carbon atoms, perfluoroalkyl having 1, 2, 3 or 4 carbon atoms or cycloalkyl having 3, 4, 5 or 6 carbon atoms;
b is zero or 1;
Z is H, alkyl having 1, 2, 3, 4, 5, 6 or 7 carbon atoms, cycloalkyl having 3, 4, 5, 6 or 7 carbon atoms, C(=O)R(15), SO₂R(15),
NR(16)R(17) or phenyl, which is not substituted or substituted by 1-3 substituents selected from the group consisting of F, Cl, Br, CF₃, methyl, methoxy, NR(21)R(22);
R(21) and R(22)
independently of one another are H or alkyl having 1, 2, 3 or 4 carbon atoms or perfluoroalkyl having 1, 2, 3 or 4 carbon atoms;
R(15) is N=C(NH₂)₂, NR(18)R(19), N(CH₂)_{c}NR(18)R(19) or OR(20);
c is 2 or 3;
R(18) and R(19)
independently of one another are H, alkyl having 1, 2, 3, 4, 5, 6, 7 or 8 carbon atoms or perfluoroalkyl having 1, 2, 3 or 4 carbon atoms;
or
R(18) and R(19)
together are 4 or 5 methylene groups, of which one CH₂ group can be replaced by oxygen, S, NH, N-CH₃, N-benzyl or N-(p-chlorophenyl);
R(20) is H, alkyl having 1, 2, 3, 4, 5 or 6 carbon atoms, perfluoroalkyl having 1, 2, 3 or 4 carbon atoms or cycloalkyl having 3, 4, 5, 6 or 7 carbon atoms;
R(16) and R(17)
independently of one another are H, alkyl having 1, 2, 3, 4, 5, 6, 7 or 8 carbon atoms or perfluoroalkyl having 1, 2, 3 or 4 carbon atoms;
or
R(16) and R(17)
together are 4 or 5 methylene groups, of which one CH₂ group can be replaced by oxygen, S, NH, N-CH₃, N-benzyl or N-(p-chlorophenyl);
or
Z is a N-containing heterocycle having 1, 2, 3, 4, 5, 6, 7, 8 or 9 carbon atoms,
where the N-containing heterocycle is linked via N or C and is not substituted or substituted by 1-3 substituents selected from the group consisting of F, Cl, Br, CF₃,
methyl, methoxy and NR(21)R(22);
but where in the case that R(4) is an alkoxy radical, at least one of the substituents R(2), R(3), R(5), R(6), R(7) and R(8) is not hydrogen;
and their pharmaceutically tolerable salts.
az) substituted 2-naphthoylguanidines of the formula I 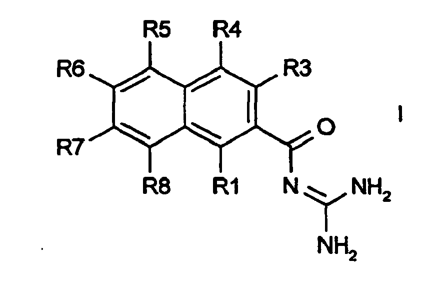 in which:
at least one of the substituents R1, R3, R4, R5, R6, R7 and R8
is XYₐWZ or X'YₐWZ' ;
X is O, S, NR(10) or CR(11)R(12);
R(10), R(11) and R(12)
independently of one another are H, alkyl having 1, 2, 3, 4, 5 or 6 carbon atoms, perfluoroalkyl having 1, 2, 3 or 4 carbon atoms or cycloalkyl having 3, 4, 5, 6 or 7 carbon atoms;
Y is alkylene having 1, 2, 3, 4, 5, 6, 7 or 8 CH₂ groups,
where one of these CH₂ groups can be replaced by O, S, NR(13) or o-, p- or m-phenylene;
R(13) is H, alkyl having 1, 2, 3, 4, 5 or 6 carbon atoms, perfluoroalkyl having 1, 2, 3 or 4 carbon atoms or cycloalkyl having 3, 4, 5 or 6 carbon atoms;
a is zero or 1;
W is CH₂, SO₂, S(=O)(=NH) or - if W does not immediately follow a hetero atom of the group XYₐ - also O or NR(14);
R(14) is H, alkyl having 1, 2, 3, 4, 5 or 6 carbon atoms, perfluoroalkyl having 1, 2, 3 or 4 carbon atoms or cycloalkyl having 3, 4, 5 or 6 carbon atoms;
Z is C(=O)R(15), SO₂R(15) or - if W is not O or NR(14) - also NR(16)R(17);
R(15)
is N=C(NH₂)₂, NR(18)R(19), N(CH₂)_{b}NR(18)R(19) or OR(20);
b is 2 or 3;
R(18) and R(19)
independently of one another are H, alkyl having 1, 2, 3, 4, 5, 6, 7 or 8 carbon atoms or perfluoroalkyl having 1, 2, 3 or 4 carbon atoms;
or
R(18) and R(19)
together are 4 or 5 methylene groups,
of which one CH₂ group can be replaced by oxygen, S, NH, N-CH₃, N-benzyl or N-(p-chlorophenyl);
R(20)
is H, alkyl having 1, 2, 3, 4, 5 or 6 carbon atoms, perfluoroalkyl having 1, 2, 3 or 4 carbon atoms or cycloalkyl having 3, 4, 5, 6 or 7 carbon atoms;
R(16) and R(17)
independently of one another are H, alkyl having 1, 2, 3, 4, 5, 6, 7 or 8 carbon atoms or perfluoroalkyl having 1, 2, 3 or 4 carbon atoms;
or
R(16) and R(17)
together are 4 or 5 methylene groups, of which one CH₂ group can be replaced by oxygen, S, NH, N-CH₃, N-benzyl or N-(p-chlorophenyl);
X' is C=O, C(=O)NR(30), C(=O)O, SO, SO₂, SO₂NR(30), OC=O, NR(30) C=O or NR(30)SO₂,
where the linkage with the naphthalene ring is in each case effected through the atom on the left;
R(30) is H, alkyl having 1, 2, 3, 4, 5 or 6 carbon atoms, perfluoroalkyl having 1, 2, 3 or 4 carbon atoms or cycloalkyl having 3, 4, 5, 6 or 7 carbon atoms;
Z' is C(=O)R(15), SO₂R(15), an N-containing heterocycle having 1, 2, 3, 4, 5, 6, 7, 8 or 9 carbon atoms,
where the N-containing heterocycle is linked via N or C and is not substituted or substituted by 1-3 substituents selected from the group consisting of F, Cl, Br, CF₃, methyl, methoxy and NR(21)R(22); R(21) and R(22)
independently of one another are H, alkyl having 1, 2, 3 or 4 carbon atoms or perfluoroalkyl having 1, 2, 3 or 4 carbon atoms;
R(15)
is N=C(NH₂)₂, NR(18)R(19), N(CH₂)_{b}NR(18)R(19) or OR(20);
R(18) and R(19)
independently of one another are H, alkyl having 1, 2, 3, 4, 5, 6, 7 or 8 carbon atoms or perfluoroalkyl having 1, 2, 3 or 4 carbon atoms;
or
R(18) and R(19)
together are 4 or 5 methylene groups, of which one CH₂ group can be replaced by oxygen, S, NH, N-CH₃, N-benzyl or N-(p-chlorophenyl);
b is 2 or 3;
R(20) is H, alkyl having 1, 2, 3, 4, 5 or 6 carbon atoms, perfluoroalkyl having 1, 2, 3 or 4 carbon atoms or cycloalkyl having 3, 4, 5, 6 or 7 carbon atoms;
or
Z' - if W is not O or NR(14) - is NR(16)R(17); R(16) and R(17)
independently of one another are H, alkyl having 1, 2, 3, 4, 5, 6, 7 or 8 carbon atoms or perfluoroalkyl having 1, 2, 3 or 4 carbon atoms;
or
R(16) and R(17)
together are 4 or 5 methylene groups, of which one CH₂ group can be replaced by oxygen S, NH, N-CH₃, N-benzyl or N-(p-chlorophenyl);
and in each case the remaining substituents R1, R3, R4, R5, R6, R7 and R8, to which none of the abovementioned definitions has been assigned, independently of one another are H, F, Cl, Br, I, CN, NO₂, CF₃, C₂F₅ or VₚQ_{q}U;
V is 0, S, SO, SO₂, NR(60), OC=O, C=O,
C(=O)NR(60), C(=O)O or CR(66)R(67);
R(60), R(66) and R(67)
independently of one another are H, alkyl having 1, 2, 3, 4, 5 or 6 carbon atoms, perfluoroalkyl having 1, 2, 3 or 4 carbon atoms or cycloalkyl having 3, 4, 5, 6 or 7 carbon atoms;
p is zero or 1;
Q is alkylene having 1, 2, 3, 4, 5, 6, 7 or 8 CH₂ groups,
where one of these CH₂ groups can be replaced by O, S, NR(68) or o-, p- or m-phenylene;
R(68)
is H, alkyl having 1, 2, 3, 4, 5 or 6 carbon atoms, perfluoroalkyl having 1, 2, 3 or 4 carbon atoms or cycloalkyl having 3, 4, 5 or 6 carbon atoms;
q is zero or 1;
U is H, alkyl having 1, 2, 3, 4, 5, 6 or 7 carbon atoms, cycloalkyl having 3, 4, 5, 6 or 7 carbon atoms, C(=O)R(65), SO₂R(65), NR(61)R(62) or phenyl, which is not substituted or substituted by 1-3 substituents selected from the group consisting of F, Cl, Br, CF₃, methyl, methoxy and NR(63)R(64);
R(63) and R(64)
independently of one another are H, alkyl having 1, 2, 3 or 4 carbon atoms or perfluoroalkyl having 1, 2, 3 or 4 carbon atoms;
R(65) is N=C(NH₂)₂, NR(61)R(62) or OR(60);
R(61) and R(62)
independently of one another are H, alkyl having 1, 2, 3, 4, 5, 6, 7 or 8 carbon atoms or perfluoroalkyl having 1, 2, 3 or 4 carbon atoms;
or
R(61) and R(62)
together are 4 or 5 methylene groups, of which one CH₂ group can be replaced by oxygen, S, NH, N-CH₃, N-benzyl or N-(p-chlorophenyl); or
U is an N-containing heterocycle having 1, 2, 3, 4, 5, 6, 7, 8 or 9 carbon atoms, where the N-containing heterocycle is linked via N or C and is not substituted or substituted by 1-3 substituents selected from the group consisting of F, Cl, Br, CF₃,
methyl, methoxy and NR(63)R(64);
where, however, at least one of the subtituents R5,R6, R7 and R8 is not hydrogen;
and their pharmaceutically tolerable salts.
ba) ortho-substituted benzoylguanidines of the formula I 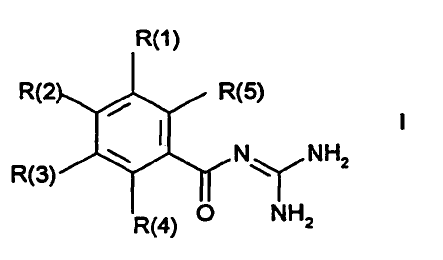 in which:
R(1) is H, F, Cl, Br, I, CN, NO₂, alkyl having 1, 2, 3, 4, 5, 6, 7 or 8 carbon atoms, alkoxy having 1, 2, 3, 4, 5, 6, 7 or 8 carbon atoms, cycloalkyl having 3, 4, 5, 6, 7 or 8 carbon atoms, cycloalkoxy having 3, 4, 5, 6, 7 or 8 carbon atoms or Xa- (CH₂)_{b}- (CF₂)_{c}-CF₃;
X is oxygen, sulfur or NR(9),
a is zero or 1;
b is zero, 1 or 2;
c is zero, 1, 2 or 3;
R(9) is H, alkyl having 1, 2, 3 or 4 carbon atoms or -C_{d}H_{2d}R(6);
d is zero, 1, 2, 3 or 4;
R(6) is cycloalkyl having 3, 4, 5, 6, 7 or 8 carbon atoms, phenyl, biphenylyl or naphthyl,
where the aromatics phenyl, biphenylyl or naphthyl are not substituted or substituted by 1-3 substituents selected from the group consisting of F, Cl, CF₃, methyl, methoxy and NR(7)R(8);
R(7) and R(8)
independently of one another are H or alkyl having 1, 2, 3 or 4 carbon atoms;
or
R(1) is -SR(10), -OR(10) or -CR(10)R(11)R(12); R(10) is -C_{f}H_{2f}-cycloalkyl having 3, 4, 5, 6, 7 or 8 carbon atoms in the cycloalkyl ring, heteroaryl having 1, 2, 3, 4, 5, 6, 7, 8 or 9 carbon atoms or phenyl, where heteroaryl and phenyl are unsubstituted or substituted by 1-3 substituents selected from the group consisting of F, Cl, CF₃, CH₃, methoxy, hydroxyl, amino, methylamino and dimethylamino;
f is zero, 1 or 2;
R(11) and R(12)
independently of one another are as defined for R(10) or are hydrogen or alkyl having 1,
2, 3 or 4 carbon atoms;
or
R(1) is phenyl, naphthyl, biphenylyl or heteroaryl having 1, 2, 3, 4, 5, 6, 7, 8 or 9 carbon atoms, the latter being linked via a carbon or a nitrogen ring atom,
each of the radicals being unsubstituted or substituted by 1-3 substituents selected from the group consisting of F, Cl, CF₃, CH₃, methoxy, hydroxyl, amino, methylamino and dimethylamino,
or
R(1) is -SR(13), -OR(13), -NHR(13), -NR(13)R(14), -CHR(13)R(15), -C[R(15)R(16)]OH, -C≡CR(18), -C[R(19)]=CHR(18), -C[R(20)R(21)]ₖ-(CO)-[CR(22)R(23)]₁-R(24),
k is zero, 1, 2, 3 or 4;
1 is zero, 1, 2, 3 or 4;
R(13) and R(14)
are identical or different and are - (CH₂)_{g}- (CHOH)ₕ-(CH₂)ᵢ-(CHOH)ₖₖ-R (17) or - (CH₂)_{g}-O-(CH₂-CH₂O)ₕ-R(24); R(17) is hydrogen or methyl, g, h and i
are identical or different and are zero, 1, 2, 3 or 4;
kk is 1, 2, 3 or 4;
R(15) and R(16)
are identical or different and are hydrogen, alkyl having 1, 2, 3, 4, 5 or 6 carbon atoms, or together with the carbon atom carrying them are cycloalkyl having 3, 4, 5, 6, 7 or 8 carbon atoms;
R(18) is phenyl,
which is unsubstituted or substituted by 1-3 substituents selected from the group consisting of F, Cl, CF₃, methyl, methoxy and NR(25)R(26);
R(25) and R(26)
are H or alkyl having 1, 2, 3 or 4 carbon atoms;
or
R(18) is heteroaryl having 1, 2, 3, 4, 5, 6, 7, 8 or 9 carbon atoms
which is unsubstituted or substituted as for phenyl;
or
R(18) is alkyl having 1, 2, 3, 4, 5 or 6 carbon atoms,
which is unsubstituted or substituted by 1-3 OH;
or
R(18)
is cycloalkyl having 3, 4, 5, 6, 7 or 8 carbon atoms;
R(19), R(20), R(21), R(22) and R(23)
are identical or different and are hydrogen or methyl;
R(24) is H, alkyl having 1, 2, 3, 4, 5 or 6 carbon atoms, cycloalkyl having 3, 4, 5, 6, 7 or 8 carbon atoms or -CₘH₂ₘ-R(18);
m is 1, 2, 3 or 4;
one of the two substituents R(2) and R(3) is -0-CO-R(27);
R(27) is alkyl having 1, 2, 3, 4, 5, 6, 7 or 8 carbon atoms, cycloalkyl having 3, 4, 5, 6, 7 or 8 carbon atoms, phenyl, biphenylyl, naphthyl, pyridyl or quinolyl,
where phenyl, biphenylyl, naphthyl, pyridyl or quinolyl are unsubstituted by 1-3 substituents selected from the group consisting of F, Cl, CF₃, methyl, methoxy and NR(7)R(8);
R(7) and R(8)
independently of one another are hydrogen or alkyl having 1, 2, 3 or 4 carbon atoms;
one of the substituents R(2) and R(3) always being defined as R(1);
R(4) and R(5) independently of one another are hydrogen, alkyl having 1, 2, 3 or 4 carbon atoms;
alkoxy having 1, 2, 3 or 4 carbon atoms, F, Cl, Br, I, CN or -(CH₂)ₙ-(CF₂)ₒ-CF₃;
n is zero or 1;
o is zero or 1;
and their pharmaceutically tolerable salts.
bb) benzoylguanidines of the formula I 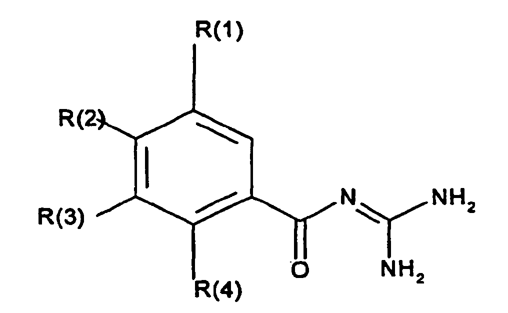 in which:
R(1) is R(13)-SOₘ or R(14)R(15)N-SO₂-;
m is 1 or 2;
R(13) is alkyl having 1, 2, 3, 4, 5, 6, 7 or 8 carbon atoms, perfluoroalkyl having 1, 2, 3, 4, 5, 6, 7 or 8 carbon atoms, alkenyl having 3, 4, 5, 6, 7 or 8 carbon atoms or
-CₙH₂ₙ-R(16),
n is zero, 1, 2, 3 or 4;
R(16) is cycloalkyl having 3, 4, 5, 6, 7 or 8 carbon atoms, phenyl, biphenylyl or naphthyl,
where phenyl, biphenylyl and naphthyl are not substituted or substituted by 1-3 substituents selected from the group consisting of F, Cl, CF₃, methyl, methoxy and NR(25)R(26);
R(25) and R(26)
independently of one another are hydrogen, alkyl having 1, 2, 3 or 4 carbon atoms or perfluoroalkyl having 1, 2, 3 or 4 carbon atoms;
R(14) is hydrogen, alkyl having 1, 2, 3, 4, 5, 6, 7 or 8 carbon atoms, perfluoroalkyl having 1, 2, 3, 4, 5, 6, 7 or 8 carbon atoms, alkenyl having 3, 4, 5, 6, 7 or 8 carbon atoms or -CₙH₂ₙ-R(27), n is zero, 1, 2, 3 or 4;
R(27) is cycloalkyl having 3, 4, 5, 6, 7 or 8 carbon atoms, phenyl, biphenylyl or naphthyl,
where phenyl, biphenylyl and naphthyl are not substituted or substituted by 1-3 substituents selected from the group consisting of F, Cl, CF₃, methyl, methoxy and NR(28)R(29);
R(28) and R(29)
independently of one another are hydrogen, alkyl having 1, 2, 3 or 4 carbon atoms or perfluoroalkyl having 1, 2, 3 or 4 carbon atoms;
R(15) is hydrogen, alkyl having 1, 2, 3 or 4 carbon atoms or perfluoroalkyl having 1, 2, 3 or 4 carbon atoms;
or
R(14) and R(15) together are 4 or 5 methylene groups, of which one CH₂ group can be replaced by oxygen, S, NH, N-CH₃
or N-benzyl;
one of the substituents R(2) and R(3) is hydrogen;
and the other substituent R(2) and R(3) in each case is -CHR(30)R(31);
R(30)
is -(CH₂)_{g}-(CHOH)ₕ-(CH₂)ᵢ-(CHOH)ₖ-R(32) or -(CH₂)_{g}-O-(CH₂-CH₂O)ₕ-R(24) ;
R(24) and R(32) independently of one another are hydrogen or methyl;
g, h, i are identical or different and are zero, 1, 2, 3 or 4;
k is 1, 2, 3 or 4;
or the other substituent R(2) and R(3) in each case
is-C(OH)R(33)R(34);
R(31), R(33) and R(34)
are identical or different and are hydrogen or alkyl having 1, 2, 3 or 4 carbon atoms or
R(33) and R(34)
together are cycloalkyl having 3, 4, 5 or 6 carbon atoms;
or
R(33) is -CH₂OH;
R(4) is alkyl having 1, 2, 3 or 4 carbon atoms, alkoxy having 1, 2, 3 or 4 carbon atoms, F, Cl, Br, I, CN, -(CH₂)ₙ-(CF₂)ₒ-CF₃;
n is zero or 1;
o is zero, 1 or 2;
and their pharmaceutically tolerable salts.
bc) indanylidineacetylguanidines of formula I 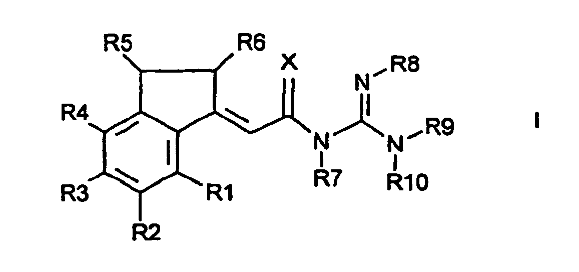 in which
R1, R2, R3, R4, R5 and R6
independently of one another are H, C₁-C₁₀-alkyl; haloalkyl having 1-6 carbon atoms, O-C₁-C₁₀-alkyl, haloalkoxy having 1-6 carbon atoms, F, Cl, Br, I, aryl, substituted aryl, heteroaryl, substituted heteroaryl, OH, O-lower alkyl, O-aryl, O-lower alkylaryl, O-substituted aryl, 0-lower alkyl-substituted aryl, O-C(=O)-C₁-C₄-alkylaryl, O-C(=O)-NH-C₁-C₄-alkyl, O-C(=O)-N(C₁-C₄-alkyl)₂, NO₂, CN, CF₃, NH₂, NH-C (=O) -C₁-C₄-alkyl, NH-C(=O)-NH₂, COOH, C(=O)-O-C₁-C₄-alkyl, C(=O)-NH₂, C(=O)-NH-C₁-C₄-alkyl, C(=O)-N(C₁-C₄-alkyl)₂, C₁-C₄-COOH, C₁-C₄-alkyl-C(=O)-O-C1-C4-alkyl, SO₃H, SO₂-alkyl, SO₂-alkylaryl, SO₂-N-(alkyl)₂, SO₂-N (alkyl) (alkylaryl), C(=O)-R¹¹, C₁-C₁₀-alkyl-C-(=O)-R11, C₂-C₁₀-alkenyl-C(=O)-R11, C₂-C₁₀-alkynyl-C(=O)-R11,
NH-C(=O)-C₁-C₁₀-alkyl-C(=O)-R11 or O-C₁-C₁₁-alkyl-C(=O)-R11;
R11 is C₁-C₄-alkyl, C₁-C₄-alkynyl, aryl, substituted aryl, NH₂, NH-C₁-C₄-alkyl, N-(C₁-C₄-alkyl)₂, SO₃H, SO₂-alkyl, SO₂-alkylaryl, SO₂-N-(alkyl)₂ or SO₂-N(alkyl)(alkylaryl);
X is O, S or NH;
R7, R8, R9 and R10
independently of one another are H, alkyl, cycloalkyl, aryl, alkylaryl,
or
R8 and R9
together are part of a 5-, 6- or 7-membered heterocyclic ring;
or their pharmaceutically tolerable salts.
bd) phenyl-substituted alkenylcarboxylic acid guanidines of the formula I 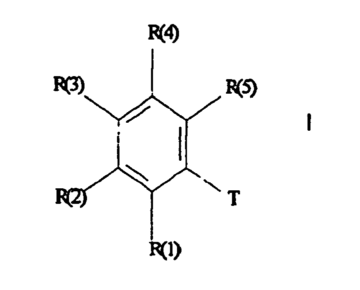 in which:
T is 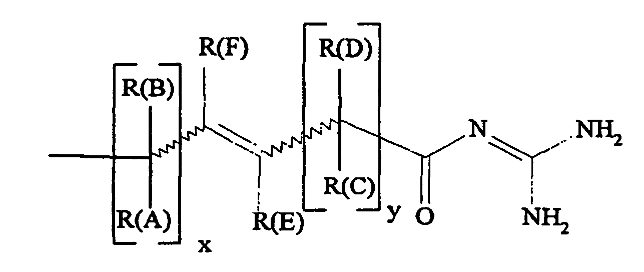 R(A) is hydrogen, F, Cl, Br, I, CN, OH, OR(6), (C₁-C₄)-alkyl, Oᵣ(CH₂)ₐC_{b}F_{2b+1}, (C₃-C₈) -cycloalkyl or NR(7)R(8)
r is zero or 1;
a is zero, 1, 2, 3 or 4;
b is 1, 2, 3 or 4;
R(6) is (C₁-C₄)-alkyl, (C₁-C₄)-perfluoroalkyl, (C₃-C₆)-alkenyl, (C₃-C₈) -cycloalkyl, phenyl or benzyl, where the phenyl ring is not substituted or substituted by 1-3 substituents selected from the group consisting of F, Cl, CF₃, methyl, methoxy and NR(9)R(10); R(9) and R(10)
are H, (C₁-C₄)-alkyl or (C₁-C₄)-perfluoroalkyl;
R(7) and R(8)
independently of one another are defined as R(6);
or
R(7) and R(8)
together are 4, or 5, methylene groups, of which one CH₂ group can be replaced by oxygen, sulfur, NH, N-CH₃ or N-benzyl;
R(B), R(C) and R(D)
independently are as defined for R(A);
x is zero, 1 or 2;
y is zero, 1 or 2;
R(F) is hydrogen, F, Cl, Br, I, CN, OR(12), (C₁-C₈)-alkyl, Oₚ(CH₂)_{f}C_{g}F_{2g+1}, (C₃-C₈) -cycloalkyl or (C₁-C₉)-heteroaryl;
p is zero or 1;
f is zero, 1, 2, 3 or 4;
g is 1, 2, 3, 4, 5, 6, 7 or 8;
R(12) is (C₁-C₈)-alkyl, (C₁-C₄)-perfluoroalkyl, (C₃-C₈) -alkenyl, (C₃-C₈) -cycloalkyl, phenyl or benzyl, where the phenyl ring is not substituted or substituted by 1-3 substituents selected from the group consisting of F, Cl, CF₃, methyl, methoxy and NR(13)R(14); R(13) and R(14)
are H, (C₁-C₄)-alkyl or (C₁-C₄)-perfluoroalkyl;
R(E) independently is as defined for R(F);
R(1) independently is as defined for T;
or
R(1) is hydrogen, -OₖCₘH₂ₘ₊₁, -Oₙ(CH₂)ₚC_{q}F_{2q+1}, F, Cl, Br, I, CN, -(C=O)-N=C(NH₂)₂, -SOᵣR(17), -SOᵣ₂NR (31) R(32) ; -Oᵤ(CH₂)ᵥC₆H₅, -Oᵤ₂-(C₁-C₉) -heteroaryl or -Sᵤ₂-(C₁-C₉) -heteroaryl;
k is zero or 1;
m is zero, 1, 2, 3, 4, 5, 6, 7 or 8;
n is zero or 1;
p is zero, 1, 2, 3 or 4;
q is 1, 2, 3, 4, 5, 6, 7 or 8;
r is zero, 1, 2;
r2 is zero, 1, 2;
R(31) and R(32)
independently of one another are hydrogen, (C₁-C₈)-alkyl or (C₁-C₈)-perfluoroalkyl;
or
R(31) and R(32)
together are 4 or 5 methylene groups, of which one CH₂ group can be replaced by oxygen, S, NH, N-CH₃ or N-benzyl;
R(17) is (C₁-C₈)-alkyl; u is zero or 1;
u2 is zero or 1;
v is zero, 1, 2, 3 or 4;
where the phenyl ring is not substituted or substituted by 1-3 substituents selected from the group consisting of F, Cl, CF₃, methyl, methoxy, -(CH₂)_{w}NR(21)R(22), NR(18)R(19) and (C₁-C₉)-heteroaryl;
R(18), R(19), R(21) and R(22)
independently of one another are (C₁-C₄)-alkyl or (C₁-C₄)-perfluoroalkyl;
w is 1, 2, 3 or 4;
where the heterocycle of the (C₁-C₉)-heteroaryl is unsubstituted or substituted by 1-3 substituents selected from the group consisting of F, Cl, CF₃, methyl or methoxy;
R(2), R(3), R(4) and R(5) independently of one another are as defined for R(1),
or
R(1) and R(2) or R(2) and R(3) in each case together are -CH-CH=CH-CH-,
which is unsubstituted or substituted by 1-3 substituents selected from the group consisting of F, Cl, CF₃, methyl, methoxy, -(CH₂)_{w2}NR(24)R(25) and NR(26)R(27); R(24), R(25), R(26) and R(27)
are H, (C₁-C₄)-alkyl or (C₁-C₄)-perfluoroalkyl;
w2 is 1, 2, 3 or 4;
the radical T being present in the molecule at least twice, but at most three times;
and their pharmaceutically tolerable salts.
be) benzoylguanidines of the formula I 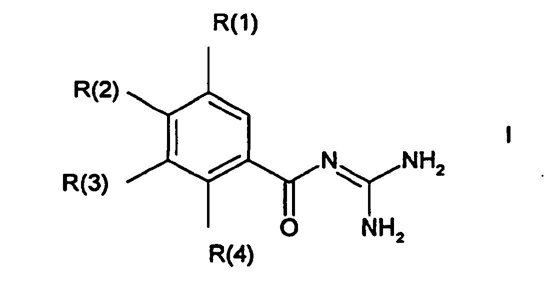 in which:
R(1) is CF₃;
one of the substituents R(2) and R(3) is hydrogen;
and the other substituent R(2) and R(3) in each case is -C(OH) (CH₃)-CH₂OH, -CH(CH₃)-CH₂OH or -C(OH) (CH₃)₂;
R(4) is methyl, methoxy, Cl or CF₃;
and their pharmaceutically tolerable salts.
or II. compounds of the formula 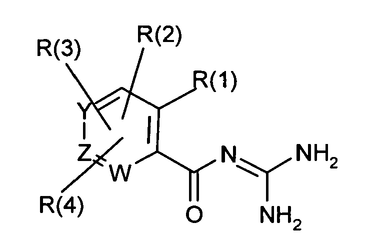 in which:
W, Y and Z
are a nitrogen atom or a carbon atom substituted by R(2) or R(3) or R(4);
R(1) is hydrogen, A, Hal, -CF₃, -CH₂F, -CHF₂, -CH₂CF₃, -C₂F₅, -CN, -NO₂, -ethynyl, or an X-R';
A is alkyl having 1 to 6 carbon atoms;
Hal is F, Cl, Br or I;
X is oxygen, S or NR" ;
R" is hydrogen, A or a cyclic methylene chain having 3 to 7 carbon atoms;
R' is H, A, HO-A-, HOOC-A-, (C₃-C₇)-cycloalkyl, (C₆-C₈)-cycloalkylalkyl, CF₃, CH₂F, CHF₂, CH₂-CF₃, Ph, -CH₂-Ph or Het;
Ph is phenyl, naphthyl or biphenylyl, which is unsubstituted or mono-, di- or trisubstituted by A, OA, NR'R", Hal, CF 3;
Het is a mono- or binuclear saturated, unsaturated or aromatic heterocycle having 1 to 4 nitrogen, oxygen and/or sulfur atoms, which is unsubstituted or mono-, di- or trisubstituted by Hal, CF₃, A, OH, OA, -X-R', -CN, -NO₂, and/or carbonyl oxygen,
where Het is bonded via N or an alkylene chain CₘH₂ₘ where m = zero to 6;
or
R' and R"
together are alkylene having 4 - 5 carbon atoms, in which one CH₂ group can also be replaced by oxygen, S, NH, N-A, N-Ph and N-CH₂-Ph;
R(2) and R(3) independently of one another are hydrogen, Hal, A, HO-A-, X-R', -C(=N-OH)-A, A-O-CO-(C₁-C₄)-alkyl-, CN, NO₂ COOH, halogen-substituted A, in particular CF₃, CH₂F, CHF₂, C₂F₅, CH₂CF₃, or S(O)ₙR"';
R'" is A, Ph or -Het;
n is zero, 1 or 2;
or
R(2) and R(3)
independently of one another are SO₂NR'R" , Ph or -O-Ph, -O-CH₂-Ph, -CO-A, -CHO, -COOA, -CSNR'R", CONR'R", -CH=CH-COOH, -CH=CH-COOA, indenyl, indanyl, decahydronaphthyl, cyclopentenyl, dihydrothienyl, dihydrofuryl, heterobicyclyl, alkylthienyl, halothienyl, haloalkylthienyl, acylthienyl, halofuryl, haloalkylfuryl or pyrrolyl;
or
R(2) and R(3)
independently of one another are R(5)-O-; R(5) is hydrogen, A, (C₁-C₆)-alkenyl or (C₃-C₇)-cycloalkyl;
R(4) is Ph, Het, -O-Het; CF₃, S(O)ₙR"', -SO₂NR'R", alk; 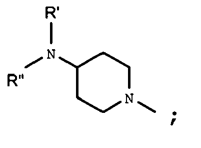 or
two of the substituents R(1) to R(4)
together are a group -O-CR(6)R(7)-CO-NR(8)-,
or 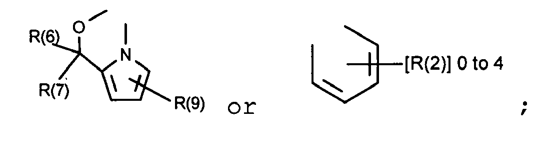 where R(2) has the meaning indicated;
R(6), R(7), R(8) and R(9)
independently of one another are H or A;
or
R(8) is (C₅-C₇)-cycloalkyl;
or
R(9) is cyano;
alk is straight-chain or branched (C₁-C₈)-alkyl or (C₃-C₈)-cycloalkyl,
which is unsubstituted or mono-, di- or trisubstituted by A;
or
alk is an ethenyl or ethynyl radical which is substituted by H, A, Ph or Het;
or
III. compounds of the formula 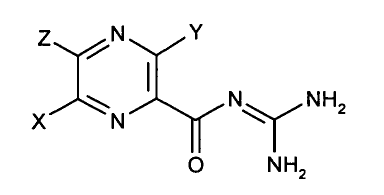 in which:
X is H, Hal, (Hal)₃C-, (C₁-C₆) -alkyl, (C₃-C₆) - cycloalkyl, substituted phenyl, (C₁-C₅)-alkyl-S- or (C₁-C₅)-alkyl-SO₂-;
Y is NH₂ or substituted amino;
or
X and Z
together are a -(CH₂)₄- or a 1,3-butadienylene chain;
or
Z is H, Hal, OH, HS, (C₁-C₅)-alkyl, (C₃-C₆) - cycloalkyl, substituted phenyl;
or
Z is an amino group -NR(1)R(2);
R(1) is H, straight- or branched-chain, optionally substituted (C₁-C₈)-alkyl, which can be interrupted by oxygen;
or
R(1) is (C₃-C₈)-alkenyl, (C₃-C₈)-alkynyl, (C₃-C₇)-cycloalkyl or OH-substituted phenyl or OH-substituted phenyl-(C₁-C₄)-alkyl or OH-substituted (C₃-C₇)-cycloalkyl;
R(2) is 1-morpholino, hydrogen or a straight or branched (C₁-C₈)-alkyl chain,
which can be interrupted by oxygen or an amino group,
which straight or branched (C₁-C₈)-alkyl chain is unsubstituted or substituted by
a substituted or unsubstituted mono- or polynuclear heterocycle which contains nitrogen, oxygen or sulfur atoms;
or which alkyl chain is substituted by phenyl, unsubstituted or mono- or polysubstituted by (C₁-C₄)-alkoxy, optionally substituted by OH, alkylamino, alkyl or phenyl;
or
by an aminocarbonyl group
or
by hydroxyl or (C₁-C₄)-alkoxy groups,
or
R(2) is phenyl, unsubstituted or substituted by alkyl, alkoxy, an amino group, which as substituents carries:
H, a mono- or polynuclear heterocycle which contains nitrogen, oxygen or sulfur atoms,
which is unsubstituted or substituted by H, Hal or (C₁-C₄)-alkyl;
a phenyl radical,
unsubstituted or substituted by a substituent selected from the group consisting of (C₁-C₄)-alkyl, (C₁-C₄)-alkoxy, Hal and OH;
or
R(2) is 1-piperidino,
unsubstituted or substituted in the 4-position by an acyl radical of an aliphatic, alicyclic, aromatic or heteroaromatic carboxylic acid, (C₁-C₈)-alkyl, which for its part can be substituted by OH or (C₁-C₄)-alkoxy or a (C₁-C₄)-alkoxy-substituted phenyl radical;
or
R(2) is amidino, which is unsubstituted or substituted by phenyl,
which is unsubstituted or substituted by Hal or alkyl;
or
R(2) is an acyl radical of an aliphatic, alicyclic, aromatic or heteroaromatic carboxylic acid,
or
R(2) is a (C₁-C₈)-alkyl chain, which can be substituted by a phenyl radical carrying OH, alkoxy or alkyl radicals,
or
R(1) and R(2)
together with the nitrogen atom to which they are bonded, are a piperazine ring,
which is unsubstituted or via a (C₁-C₆)-methylene chain carries a mono- or polynuclear heterocycle,
which contains nitrogen, oxygen or sulfur
Hal is F, Cl, Br or I;
or
IV. indoloylguanidine derivatives of the formula 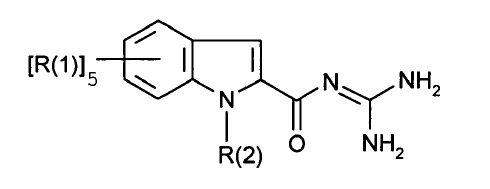 in which
R(2) is hydrogen, unsubstituted or substituted (C₁-C₈)-alkyl, (C₃-C₇)-cycloalkyl, OH, (C₁-C₆)-alkyl-O-, an aromatic radical or a group -CH₂-R(20); R(20) is (C₂-C₆)-alkenyl or (C₂-C₆)-alkynyl;
R(1) is 1 to 5 identical or different substituents, which are:
hydrogen, unsubstituted or substituted (C₁-C₈)-alkyl, (C₂-C₆)-alkenyl, (C₂-C₆)-alkynyl, (C₃-C₇)-cycloalkyl, halogen, -NO₂, (C₂-C₈)-alkanoyl, arylalkanoyl having up to 10 carbon atoms, aroyl having up to 11 carbon atoms, -COOH, (C₂-C₆)-alkoxycarbonyl, an aromatic group or one of the following mentioned groups: -OR(3), -NR(6)R(7) or -S(O)ₙR(40);
R(3) is hydrogen, (C₁-C₈)-alkyl, substituted (C₁-C₈)-alkyl, (C₃-C₇)-cycloalkyl, an aromatic radical or a group -CH₂-R(30)
R(30) is alkenyl or alkynyl;
R(6) and R(7) independently of one another are hydrogen, unsubstituted or substituted (C₁-C₈)-alkyl, (C₃-C₇) -cycloalkyl, (C₂-C₈)-alkanoyl, an arylalkanoyl group having up to 10 carbon atoms, an aroyl group having up to 11 carbon atoms, an aromatic group or -CH₂-R(60);
R(60) is (C₂-C₆)-alkenyl or (C₂-C₆) - alkynyl;
or
R(6) and R(7)
together with the nitrogen atom are a 5 - 7-membered cyclic amine, which can additionally contain further heteroatoms in the ring;
n is zero, 1 or 2;
R(40) is unsubstituted or substituted (C₁-C₈)-alkyl, or an aromatic group, or a group 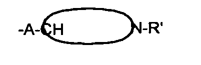 A is oxygen , -S(O)ₙ- or -N(R50)-; R(50) is hydrogen or (C₁-C₈)-alkyl;
R' is hydrogen, unsubstituted or substituted (C₁-C₈)-alkyl,
in which the ring represents a saturated 3 - 8-membered heterocycle having a nitrogen atom,
said substituted alkyl carries one or more groups selected from the group consisting of halogen, -
OH, (C₁-C₆)-alkoxy, -CN, -COOH, (C₂-C₆) - alkoxycarbonyl, (C₂-C₈)-alkanoyl, arylalkanoyl having up to 10 carbon atoms, aroyl having up to 11 carbon atoms, an aromatic group, -CONR(4)R(5),
R(4) and R(5)
identically or differently are hydrogen or (C₁-C₈)-alkyl;
or
R(4) and R(5)
are connected to one another and together form a 5 - 7-membered cyclic amine which can additionally contain further heteroatoms in the ring,
or said substituted alkyl carries a group 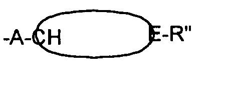 in which:
E is a nitrogen atom or a CH group;
R" is hydrogen, (C₁-C₈)-alkyl which is unsubstituted or substituted by OH, (C₁-C₆)-alkoxy, -CN, -COOH, (C₂-C₆)-alkoxycarbonyl, (C₂-C₈)-alkanoyl, aralkanoyl having up to 10 carbon atoms, aroyl having up to 11 carbon atoms, an aromatic group, -NR(6)R(7), -CONR(4)R(5);
R(4) and R(5)
independently of one another are hydrogen or (C₁-C₈)-alkyl;
where the cyclic system of the formula 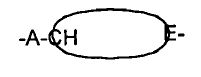 is a 3 - 8-membered saturated aliphatic or heterocyclic ring system having a nitrogen atom,
and where the aromatic groups mentioned are an aryl radical having up to 10 carbon atoms, a 5- or 6-membered heteroaryl radical having 1 - 4 nitrogen atoms, a 5- or 6-membered heteroaryl group containing 1 or 2 nitrogen atoms and a heteroatom which is oxygen or sulfur, or furyl,
and where the aryl radicals mentioned can be unsubstituted or substituted by unsubstituted (C₁-C₈)-alkyl or substituted (C₁-C₈)-alkyl, halogen, -NO₂, (C₂-C₆)-alkoxycarbonyl, COOH, -OR(3), NR(6)R(7), - CONR(4)R(5), -SO₂NR(6)R(7) or S(O)ₙR(40),
where R(1) and the guanidinocarbonyl radical can be in any desired position of the 5- or 6-membered ring of the indole system,
and the appropriate pharmaceutically tolerable salts.
or
V. heterocyclic guanidine derivatives of the formula 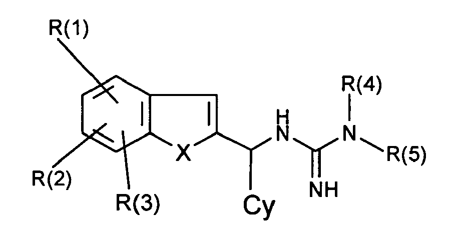 in which:
X is -O-, -S-, -NH-, -N[(C₁-C₄)-alkyl]-or-N(phenyl)-;
R(1), R(2) and R(3)
are hydrogen, halogen, (C₁-C₄)-alkyl, (C₁-C₄)-alkyl-0-, phenyl, benzyl;
or
two of the substituents R(1), R(2) and R(3)
together with one side of the benzo system are a 4 - 6-membered carbocyclic ring;
R(4) and R(5)
independently of one another are hydrogen, (C₁-C₁₂)-alkyl, benzhydryl, aralkyl,
which is unsubstituted or substituted by one or more substituents from the groups halogen, (C₁-C₄)-alkyl, (C₁-C₄)-alkyl-O- or -CF₃, -(CH₂ )ₘ-CH₂-T,
m is zero to 3;
T is -CO-O-T(1);
T(1) is hydrogen or (C₁-C₄)-alkyl;
Cy is a benzo-fused unsaturated or dihydro-5-membered ring heterocycle 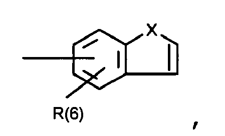 a pyrazole or imidazole ring of the formula 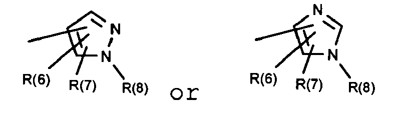 a naphthyl radical or a dihydro- or tetrahydronaphthyl radical 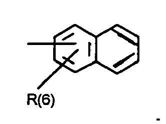 a 2-, 3- or 4-pyridyl radical 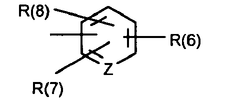 Z is N- or CH;
a thienyl radical 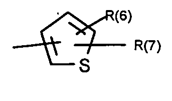 R(6) is hydrogen, halogen, hydroxyl, (C₁-C₁₀) - alkyl, (C₁-C₁₀)-alkyl-O-, phenoxy, (C₁-C₁₀) - alkyloxymethyloxy- or -(O)ₙS-R(9); R(9) is (C₁-C₁₀)-alkyl, thienyl, pyridyl,
thiazolyl, thiadiazolyl, imidazolyl, pyrazolyl or phenyl, each of which is unsubstituted or mono- or disubstituted by halogen, (C₁-C₄)-alkyl or (C₁-C₄)-alkyl-O-;
R(7) and R(8)
is hydrogen, halogen, hydroxyl, (C₁-C₁₀)-alkyl, (C₁-C₁₀)-alkyl-O-, phenyl, phenoxy or (C₁-C₁₀)-alkoxymethyloxy;
or
Cy is phenyl,
which is unsubstituted or mono- or disubstituted by halogen, (C₁-C₄)-alkyl or (C₁-C₄-alkyl-O-;
or
Cy is -Gr-Am;
Gr is -R(13)-R(12)-(CH₂)_{q}-C[W] [W(1)]-(CH₂)_{q'}-; R(13)R(14)- or -R(15)-; R(12) is a single bond, -O-, -(O)ₙS-,
-CO- or -CONH-;
R(13) is a single bond, phenyl, thienyl, pyridyl, thiazolyl, thiadiazolyl, imidazolyl or pyrazolyl;
R(14) is a single bond or SO₂-;
R(15) is (C₂-C₁₀)-alkenyl- or (C₂-C₁₀)-alkynyl;
W and W(1)
independently of one another are hydrogen, (C₁-C₄)-alkyl;
or
W and W(1)
cyclically connected to one another are a (C₃-C₈)-hydrocarbon ring;
q and q'
are zero to 9;
Am is -NR(10)R(11);
R(10) is hydrogen, (C₁-C₄)-alkyl or benzyl,
R(11) is (C₁-C₄)-alkyl, phenyl or benzyl;
or
R(10) and R(11)
together are a (C₃-C₁₀)-alkylene group, which is unsubstituted or substituted by -COOH, (C₁-C₅)-alkoxycarbonyl, (C₂-C₄)-hydroxyl-alkylene or benzyl;
or
Am is pyrrolyl, pyridyl, pyrazolyl, morpholinyl, dihydropyridyl, tetrahydropyridyl, quinuclidinyl, imidazolyl, 3-azabicyclo[3.2.1]octyl,
which is unsubstituted or substituted by (C₁-C₄)-alkyl,
or
Am is azabicyclo[3.2.2]nonyl;
or
Am is a piperazine group of the formula 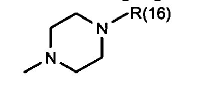 R(16) is hydrogen, (C₁-C₄)-alkyl, (C₃-C₆)-cycloalkyl, phenyl, tolyl, methoxyphenyl, halophenyl, diphenylmethylene, benzyl or pyridyl;
or
Am is an azido group - (O)t-(CH₂)q-C[W] [W(1)] - (CH₂)q'-N₃;
t is zero or 1;
where W and W(1) have the previously
indicated meaning;
and the optical enantiomers and the pharmacologically tolerable salts.
or
VI. Guanidine compounds of the fomula below 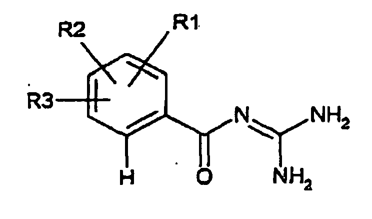 where R1 = R2 is H, halo, alkyl, CN, NO₂, perfluoroalkyl, SOₙCF₃; R3 = CH=CH₂, CH₂-CH=CH₂, CH₂-CH₂-CH=CH₂, cycloalkenyl, cycloalkenylalkyl; R4 = alkyl, (substituted) phenyl, and the substance having cardiovascular activity is a hypotensive medicament for the treatment of hypertension under cardioprotective conditions, a cardiac glycoside for the treatment of heart failure and congestive heart failure under cardioprotective conditions, an antiarrhythmic for the treatment of cardiac arrhythmias of various aetiology under cardioprotective conditions, a nitrate with cardiovascular activity, a K(ATP) channel opener or a K(ATP) blocker.

2. Pharmaceutical combination preparation according to Claim 1, comprising an NHE inhibitor and a beta-receptor blocker for the treatment of hypertension and the treatment of arrhythmia under cardioprotective conditions.

3. Pharmaceutical combination preparation according to Claim 1, comprising an NHE inhibitor and a calcium antagonist for the treatment of hypertension under cardioprotective conditions.

4. Pharmaceutical combination preparation according to Claim 1, comprising an NHE inhibitor and an angiotensin conversion enzyme inhibitor for the treatment of hypertension under cardioprotective conditions.

5. Pharmaceutical combination preparation according to Claim 1, comprising an NHE inhibitor and a diuretic or an aldosterone antagonist for the treatment of hypertension and cardiac insufficiency under cardioprotective conditions.

6. Pharmaceutical combination preparation according to Claim 1, comprising an NHE inhibitor and an antiarrhythmic of the classes I, II, III or IV for the treatment of cardiac arrhythmias of various genesis under cardioprotective conditions.

7. Pharmaceutical combination preparation according to Claim 1, comprising the NHE inhibitor cariporide (Hoe 642) in combination with an inhibitor of the noninactivating sodium channel.

## Revendications

1. Préparation pharmaceutique combinée, **caractérisée en ce qu'**elle contient un inhibiteur de l'échangeur Na⁺/H⁺ et une substance active d'un point de vue cardiovasculaire et **en ce que** l'inhibiteur de l'échangeur Na⁺/H⁺ est choisi parmi les composés suivants :
I.
a) benzoylguanidines de formule I 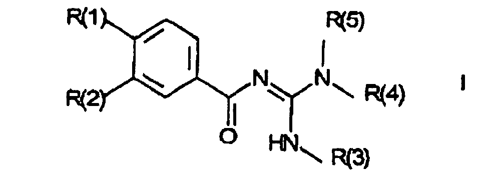 où :
R(1) ou R(2) signifie R(6)-S(O)ₙ- ou R(7)R(8)N-O₂Set à chaque fois l'autre substituant R(1) ou R(2)
signifie H, F, Cl, Br, (C₁-C₄) -alkyle, (C₁-C₄) - alcoxy ou phénoxy,
qui est non substitué ou substitué par 1-3 substituants choisis dans le groupe constitué par fluor, chlore, méthyle et méthoxy ;
ou à chaque fois l'autre substituant R(1) ou R(2) signifie R(6)-S(O)ₙ ou R(7)R(8)N- ;
n vaut zéro, 1 ou 2 ;
R(6) signifie (C₁-C₆)-alkyle, (C₅-C₇)-cycloalkyle, cyclopentylméthyle, cyclohexylméthyle ou phényle,
qui est non substitué ou substitué par 1-3 substituants choisis dans le groupe constitué par fluor, chlore, méthyle et méthoxy ;
R(7) et R(8)
identiques ou différents, signifient H ou (C₁-C₆)-alkyle ; ou
R(7) signifie phényl-(CH₂)ₘ ;
m vaut 1 - 4 ;
ou
R(7) signifie phényle,
qui est non substitué ou substitué par 1-2 substituants choisis dans le groupe constitué par fluor, chlore, méthyle et méthoxy ;
ou
R(7) et R(8)
signifient ensemble une chaîne en C₄-C₇ linéaire ou ramifiée, la chaîne pouvant en outre être interrompue par O, S ou NR(9) ;
R(9) signifie H ou méthyle,
ou
R(7) et R(8)
signifient ensemble avec l'atome d'azote auquel ils sont liés, un système dihydro-indole, tétrahydroquinoléine ou tétrahydro-isoquinoléine ; R(3), R(4) et R(5)
signifient indépendamment les uns des autres H ou (C₁-C₂)-alkyle ;
ou
R(3) et R(4)
signifient ensemble une chaîne (C₂-C₄)-alkylène ;
ou
R(4) et R(5)
signifient ensemble une chaîne (C₄-C₇)-alkylène ; ainsi que leurs sels pharmaceutiquement acceptables ;
b) benzoylguanidines de formule I 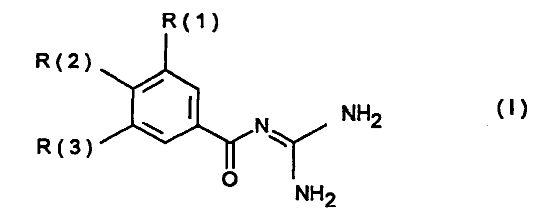 où :
R(1) signifie R(4)-SOₘ ou R(5)R(6)N-SO₂- ;
m vaut zéro, 1 ou 2 ;
R(4) et R(5)
signifient C₁-C₈-alkyle, C₃-C₆-alcényle ou -CₙH₂ₙ-R(7) ;
n vaut zéro, 1, 2, 3 ou 4 ;
R(7) signifie C₅-C₇-cycloalkyle ou phényle, qui est non substitué ou substitué par 1-3 substituants choisis dans le groupe constitué par F, Cl, CF₃, méthyle, méthoxy et NR(8)R(9) ;
R(8) et R(9) signifient H ou (C₁-C₄)-alkyle ;
ou
R(5) signifie H ;
R(6) signifie H ou (C₁-C₄)-alkyle ;
ou
R(5) et R(6) signifient ensemble 4 ou 5 groupements méthylène, dont un groupement CH₂ peut être remplacé par O, S, NH, N-CH₃ ou N-benzyle ;
R(2) signifie hydrogène, F, Cl, Br, (C₁-C₄)-alkyl-, O-(CH₂)ₘCₚF₂ₚ₊₁ ou -X-R(10) ;
m vaut zéro ou 1 ;
p vaut 1, 2 ou 3 ;
X signifie O, S ou NR(11) ;
R(10) signifie H, C₁-C₆-alkyle, C₅-C₇-cycloalkyle, cyclohexylméthyle, cyclopentylméthyle ou -CₙH₂ₙ-R(12) ;
n vaut zéro, 1, 2, 3 ou 4 ;
R(12) signifie phényle,
qui est non substitué ou substitué par 1-3 substituants choisis dans le groupe constitué par F, Cl, CF₃, méthyle, méthoxy et NR(8)R(9) ;
R(8) et R(9)
signifient H ou C₁-C₄-alkyle ;
R(11) signifie hydrogène ou C₁-C₃-alkyle ;
ou
R(10) et R(11)
signifient ensemble 4 ou 5 groupements méthylène, dont un groupement CH₂ peut être remplacé par O, S, NH, N-CH₃ ou N-benzyle ;
R(3) est défini comme R(1) ou signifie C₁-C₆-alkyle, nitro, cyano, trifluorométhyle, F, Cl, Br, I ou -X-R(10) ;
X signifie O, S ou NR(11) ;
R(10) signifie H, C₁-C₆-alkyle, C₅-C₇-cycloalkyle, cyclohexylméthyle, cyclopentylméthyle ou -CₙH₂ₙ-R(12) ;
n vaut zéro à 4 ;
R(12) signifie phényle,
qui est non substitué ou substitué par 1-3 substituants choisis dans le groupe constitué par F, Cl, CF₃, méthyle, méthoxy et NR(8)R(9) ;
R(8) et R(9)
signifient H ou (C₁-C₄)-alkyle ;
R(11) signifie (C₁-C₃)-alkyle,
ou
R(10) et R(11)
signifient ensemble 4 ou 5 groupements méthylène, dont un groupement CH₂ peut être remplacé par O, S, NH, N-CH₃ ou N-benzyle ;
ainsi que leurs sels pharmaceutiquement acceptables ;
c) benzoylguanidines substituées en position ortho de formule I 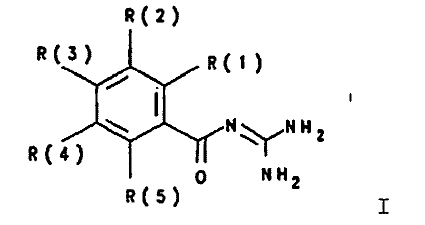 où :
R(1) signifie F, Cl, Br, I, C₁-C₆-alkyle ou -X-R(6) ;
X signifie O, S, NR(7) ou Y-ZO ;
Y signifie O ou NR(7) ;
Z signifie C ou SO ;
R(6) signifie H, C₁-C₆-alkyle, C₅-C₇-cycloalkyle, cyclohexylméthyle, cyclopentylméthyle, -(CH₂)ₘCₚF₂ₚ₊₁ ou -CₙH₂ₙ-R (8) ;
m vaut zéro ou 1 ;
p vaut 1 - 3 ;
n vaut zéro à 4 ;
R(8) signifie phényle,
qui est non substitué ou substitué par 1-3 substituants choisis dans le groupe constitué par les groupements F, Cl, CF₃, méthyle, méthoxy et NR(9)R(10) ;
R(9) et R(10)
signifient H ou (C₁-C₄)-alkyle ;
R(7) signifie H ou C₁-C₃-alkyle ;
ou
R(6) et R(7)
signifient ensemble 4 ou 5 groupements méthylène, dont un groupement CH₂ peut être remplacé par O, S, NH, N-CH₃ ou N-benzyle ;
R(3) signifie H ou -X-R(6) ;
X signifie O, S, NR(7) ou Y-ZO ;
R(7) signifie H ou C₁-C₃-alkyle ;
Y signifie O ou NR(7) ;
Y étant lié au radical phényle de formule I,
Z signifie C ou SO ;
R(6) signifie H, C₁-C₆-alkyle, C₅-C₇-cycloalkyle, cyclohexylméthyle, cyclopentylméthyle, -(CH₂)ₘCₚF₂ₚ₊₁ ou -CₙH₂ₙ-R (8) ;
m vaut zéro ou 1 ;
p vaut 1 - 3 ;
n vaut zéro à 4 ;
R(8) signifie phényle,
qui est non substitué ou substitué par 1-3 substituants choisis dans le groupe constitué par F, Cl, CF₃, méthyle, méthoxy et NR(9)R(10) ;
R(9) et R(10)
signifient H ou (C₁-C₄) -alkyle ;
ou
R(6) et R(7)
signifient ensemble 4 ou 5 groupements méthylène, dont un groupement CH₂ peut être remplacé par O, S, NH, N-CH₃ ou N-benzyle ;
R(2) et R(4)
identiques ou différents, signifient R(11)-SOq- ou R(12)R(13)N-SO₂- ;
q vaut zéro - 2 ;
R(11) signifie (C₁-C₄)-alkyle,
qui est non substitué ou qui porte phényle comme substituant, phényle étant non substitué ou substitué par 1-3 substituants choisis dans le groupe constitué par F, Cl, CF₃, méthyle, méthoxy et NR(9)R(10) ;
R(9) et R(10)
signifient H ou (C₁-C₄)-alkyle ;
R(12) et R(13)
sont définis comme R(6) et R(7) ;
ou un des deux radicaux R(2) ou R(4)
signifie hydrogène ou est défini comme R(1) ;
R(5) signifie H, méthyle, F, Cl ou méthoxy,
ainsi que leurs sels pharmaceutiquement acceptables.
d) benzoylguanidines de formule I 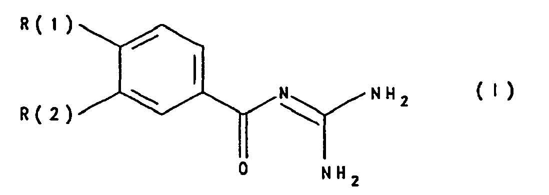 où :
R(1) ou R(2)
signifie un groupement amino -NR(3)R(4) ;
R(3) et R(4)
identiques ou différents, signifient H, C₁-C₆-alkyle ou C₃-C₇-cycloalkyle ;
ou
R (3) signifie phényl- (CH₂)ₚ ;
p vaut 0, 1, 2, 3 ou 4 ;
ou
R(3) signifie phényle,
phényle étant à chaque fois non substitué ou portant un à deux substituants choisis dans le groupe constitué par fluor, chlore, méthyle et méthoxy ;
ou
R(3) et R(4)
peuvent représenter ensemble une chaîne C₄-C₇-méthylène linéaire ou ramifiée, un élément -CH₂- de la chaîne méthylène pouvant être remplacé par oxygène, S ou NR(5) ;
R(5) signifie H ou alkyle inférieur ;
à chaque fois l'autre substituant R(1) ou R(2)
signifie H, F, Cl, C₁-C₄-alkyle, C₁-C₄-alcoxy, CF₃, CₘF₂ₘ₊₁-CH₂-, benzyle ou phénoxy,
chaque radical phényle étant à chaque fois non substitué ou portant un à deux substituants choisis dans le groupe constitué par méthyle, méthoxy, fluor et chlore ;
m vaut 1, 2 ou 3 ;
ainsi que leurs sels pharmaceutiquement acceptables ;
e) benzoylguanidines de formule I 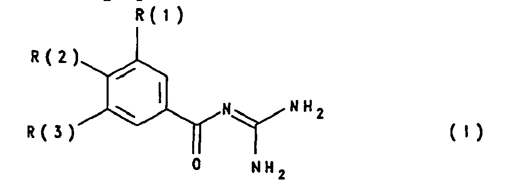 où :
R(1) signifie R(4)-SOₘ ou R(5)R(6)N-SO₂- ;
m vaut zéro, 1 ou 2 ;
R(4) et R(5)
signifient C₁-C₈-alkyle, C₃-C₆-alcényle ou -CₙH₂ₙ-R(7) ;
n vaut zéro, 1, 2, 3 ou 4 ;
R(7) signifie (C₅-C₇)-cycloalkyle ou phényle,
qui est non substitué ou substitué par 1-3 substituants choisis dans le groupe constitué par F, Cl, CF₃, méthyle, méthoxy et NR(8)R(9) ;
R(8) et R(9)
signifient H ou (C₁-C₄)-alkyle ;
ou
R(5) signifie H ;
R(6) signifie H ou (C₁-C₄)-alkyle ;
ou
R(5) et R(6)
signifient ensemble 4 ou 5 groupements méthylène, dont un groupement CH₂ peut être remplacé par 0, S, NH, N-CH₃ ou N-benzyle ;
R(2) signifie hydrogène, (C₅-C₈)-alkyle linéaire ou ramifié, -CR(13)=CHR(12) ou -C≡CR(12) ;
R(12) signifie phényle,
qui est non substitué ou substitué par 1-3 substituants choisis dans le groupe constitué par F, Cl, CF₃, méthyle, méthoxy et NR(14)R(15) ;
R(14) et R(15) signifient H ou (C₁-C₄)-alkyle ;
ou
R(12) signifie (C₁-C₉)-hétéroaryle,
qui est non substitué ou substitué comme phényle, ou
R(12) signifie (C₁-C₆)-alkyle, qui est non substitué ou substitué par 1-3 OH,
ou
R(12) signifie (C₃-C₈)-cycloalkyle ;
R(13) signifie hydrogène ou méthyle,
ou
R(12) signifie (C₃-C₈)-cycloalkyle, (C₃-C₈) - cycloalkyl-(C₁-C₄)-alkyle, phényle, C₆H₅-(C₁-C₄)-alkyle, naphtyle, biphénylyle, 1,1-diphényl-(C₁-C₄)-alkyle, cyclopentadiényle, pyridyle, pyrrolyle, furannyle, thiényle, thiazolyle, oxazolyle, indényle, quinoléyle, indolyle, benzofurannyle, benzothiényle, benzothiazolyle, benzoxazolyle, imidazolyle, pyrazolyle, triazolyle, tétrazolyle, isoxazolyle, isothiazolyle, pyrazinyle, pyrimidinyle, pyridazinyle, indazolyle, isoquinoléyle, phtalazinyle, quinoxalinyle, quinazolinyle ou cinnolinyle ;
R(3) est défini comme R(2) ;
et les substituants aromatiques R(2) ou, selon le cas, R(3) étant non substitués ou substitués par 1-3 substituants parmi les groupements F, Cl, CF₃, (C₁-C₄)-alkyle, (C₁-C₄)-alcoxy, ou NR(10)R(11), R(10) et R(11) signifiant H ou (C₁-C₄)-alkyle ;
ainsi que leurs sels pharmaceutiquement acceptables.
f) benzoylguanidines de formule I 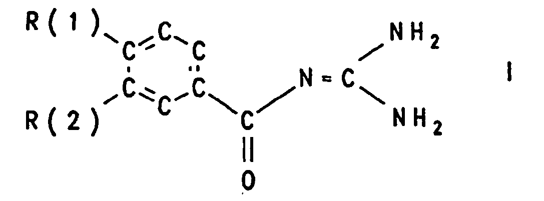 où :
R(1) ou R(2)
signifie R(3)-S(O)ₙ ou 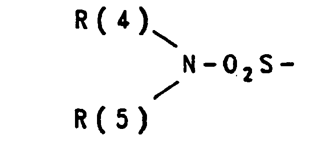 à chaque fois l'autre substituant R(1) ou R(2)
signifie H, OH, F, Cl, Br, I, C₁-C₄-alkyle, C₁-C₄-alcoxy, benzyloxy ou phénoxy,
qui est non substitué ou qui porte un à trois substituants choisis dans le groupe constitué par fluor, chlore, méthyle, méthoxy, hydroxy ou benzyloxy, R(3)-S(O)n, -NR(4)R(5) ou 3,4-déshydropipéridine
R(3) signifie C₁-C₆-alkyle, C₅-C₇-cycloalkyle, cyclopentylméthyle, cyclohexylméthyle ou phényle ;
qui est non substitué ou qui porte un à trois substituants choisis dans le groupe constitué par fluor, chlore, méthyle et méthoxy ;
R(4) et R(5)
identiques ou différents, signifient H ou C₁-C₆-alkyle ;
ou
R(4) signifie phényl- (CH₂)ₘ ;
m vaut 1, 2, 3 ou 4 ;
ou
R(4) signifie phényle,
qui est non substitué ou qui porte un à deux substituants choisis dans le groupe constitué par fluor, chlore, méthyle et méthoxy ;
ou
R(4) et R(5)
signifient ensemble une chaîne en C₄-C₇ linéaire ou ramifiée, la chaîne pouvant en outre être interrompue par O, S ou NR(6) ;
R(6) signifie H ou méthyle,
ou
R(4) et R(5)
signifient ensemble avec l'atome d'azote auquel ils sont liés, un système dihydro-indole, tétrahydroquinoléine ou tétrahydro-isoquinoléine ;
n vaut zéro, 1 ou 2 ;
ainsi que leurs sels pharmaceutiquement acceptables.
g) isoquinoléines de formule I 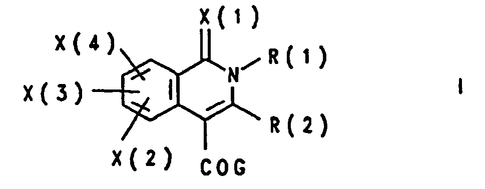 où :
R(1) signifie hydrogène, alkyle, cycloalkyle, arylalkyle, alcényle, aminoalkyle substitué ou un cycle aryle ou hétéroaryle ;
les cycles étant non substitués ou substitués par 1-3 groupements choisis dans le groupe constitué par halogène, nitro, amino, mono(alkyle inférieur)amino, di(alkyle inférieur)amino, alkyle inférieur, alcoxy inférieur, benzyloxy, phénoxy, hydroxy, trifluorométhyle,
R(2) signifie hydrogène, halogène, alkyle, ou aryle ;
qui est non substitué ou substitué par 1-3 groupements choisis dans le groupe constitué par halogène, nitro, amino, mono(alkyle inférieur)amino, di(alkyle inférieur)amino, alkyle inférieur, alcoxy inférieur, benzyloxy, phénoxy, hydroxy,
G signifie 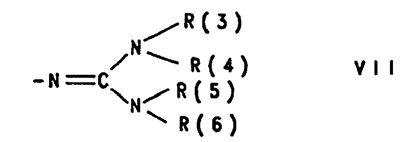
X(2), X(3) et X(4)
signifient, indépendamment les uns des autres, hydrogène, halogène, nitro, amino, alkyle, sulfonamide, mono(alkyle inférieur)amino, di(alkyle inférieur)amino, alkyle inférieur, benzyloxy, hydroxy ;
X(1) signifie hydrogène, oxygène, soufre ou NR(7) ;
R(7) signifie hydrogène, alkyle, cycloalkyle, arylalkyle, alcényle, aminoalkyle substitué ou un cycle aryle ou hétéroaryle ;
les cycles étant non substitués ou substitués par 1-3 groupements choisis dans le groupe constitué par halogène, nitro, amino, mono(alkyle inférieur)amino, di(alkyle inférieur)amino, alkyle inférieur, alcoxy inférieur, benzyloxy, phénoxy, hydroxy et trifluorométhyle,
chaque chaîne alkyle ou alcényle dans ces substituants pouvant être interrompue par oxygène, soufre ou NR(8) ;
R(8) signifie hydrogène, alkyle, cycloalkyle, arylalkyle, alcényle, aminoalkyle substitué ou un cycle aryle ou hétéroaryle ;
les cycles étant non substitués ou substitués par 1-3 groupements choisis dans le groupe constitué par halogène, nitro, amino, mono(alkyle inférieur)amino, di(alkyle inférieur)amino, alkyle inférieur, alcoxy inférieur, benzyloxy, phénoxy, hydroxy et trifluorométhyle,
et leurs sels pharmaceutiquement acceptables ;
h) composés de formule I 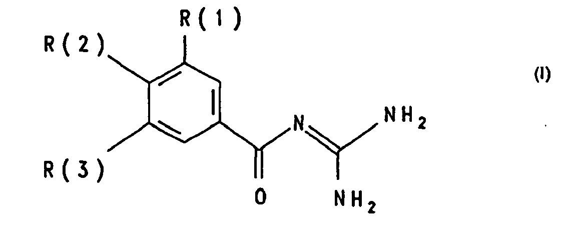 où :
R(1) signifie hydrogène, F, Cl, Br, I, -NO₂, -C=N, -CF₃, R(4)-SOₘ ou R(5)R(6)N-SO₂- ;
m vaut zéro, 1 ou 2 ;
R(4) et R(5)
signifient (C₁-C₈)-alkyle, (C₃-C₆)-alcényle, -CₙH₂ₙ-R(7) ou CF₃ ;
n vaut zéro, 1, 2, 3 ou 4 ;
R(7) signifie (C₃-C₇)-cycloalkyle ou phényle,
qui est non substitué ou substitué par 1-3 substituants choisis dans le groupe constitué par F, Cl, CF₃, méthyle, méthoxy et NR(8)R(9) ;
R(8) et R(9)
signifient H ou (C₁-C₄)-alkyle ;
ou
R(5) signifie H ;
R(6) signifie H ou (C₁-C₄)-alkyle ;
ou
R(5) et R(6)
signifient ensemble 4 ou 5 groupements méthylène, dont un groupement CH₂ peut être remplacé par oxygène, S, NH, N-CH₃ ou N-benzyle ;
R(2) signifie -SR(10), -OR(10), -NHR(10), -NR(10)R(11), -CHR(10)R(12), -[CR(12)R(13)OR(13')], -{C-[CH₂-OR(13')]R(12)(R(13)} ou -[CR(18)R(17)]ₚ-(CO)-[CR(19)R(20)]_{q}-R(14) ;
R(10), R(11) identiques ou différents, signifient
-[CHR(16)]ₛ(CH₂)ₚ-(CHOH)_{q}-(CH₂)ᵣ-(CHOH)ₜ-R(21) ou -(CH₂)ₚ-O-(CH₂-CH₂O)_{q}-R(21),
R(21) signifie hydrogène, méthyle,
p, q, r identiques ou différents,
valent zéro, 1, 2, 3 ou 4 ;
s vaut zéro ou 1 ;
t vaut 1, 2, 3 ou 4 ;
R(12) et R(13)
identiques ou différents, signifient hydrogène, (C₁-C₆)-alkyle ou, ensemble avec l'atome de carbone qui les porte, (C₃-C₈)-cycloalkyle,
R(13') signifie hydrogène ou (C₁-C₄)-alkyle ;
R(14) signifie H, (C₁-C₆)-alkyle, (C₃-C₈)-cycloalkyle ou -CₐH₂ₐ-R (15) ;
a vaut zéro, 1, 2, 3 ou 4 ;
R(15) signifie phényle,
qui est non substitué ou substitué par 1-3 substituants choisis dans le groupe constitué par F, Cl, CF₃, méthyle, méthoxy et NR(8)R(9) ;
R(8) et R(9)
signifient H ou (C₁-C₄)-alkyle ;
ou
R(15) signifie (C₁-C₉)-hétéroaryle,
qui est non substitué ou substitué comme phényle, ou
R(15) signifie (C₁-C₆)-alkyle,
qui est non substitué ou substitué par 1-3 OH, R(16), R(17), R(18), R(19) et R(20)
signifient hydrogène ou (C₁-C₃)-alkyle ;
R(3) est défini comme R(1) ;
ou
R(3) signifie (C₁-C₆)-alkyle ou -X-R(22) ;
X signifie oxygène, S ou NR(16) ;
R(16) signifie H ou (C₁-C₃)-alkyle ;
ou
R(22) et R(16)
signifient ensemble 4 ou 5 groupements méthylène, dont un groupement CH₂ peut être remplacé par oxygène, S, NH, N-CH₃ ou N-benzyle ;
R(22) est défini comme R(14) ;
ainsi que leurs sels pharmaceutiquement acceptables.
i) benzoylguanidines de formule I 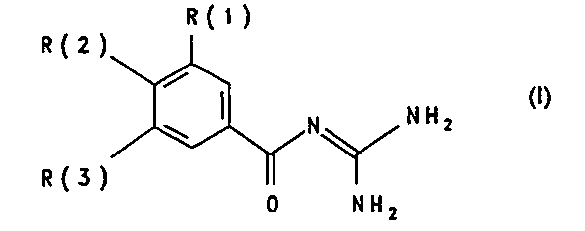 où :
R(1) signifie hydrogène, F, Cl, Br, I, -NO₂, -C=N, R(16)-CₚH₂ₚ-O_{q}, R(4)-SOₘ ou R(5)R(6)N-SO₂- ;
m vaut zéro, 1 ou 2 ;
p vaut zéro ou 1 ;
q vaut zéro, 1, 2 ou 3 ;
R(16) signifie CᵣF₂ᵣ₊₁ ;
r vaut 1, 2 ou 3 ;
R(4) et R(5)
signifient (C₁-C₈)-alkyle, (C₃-C₆)-alcényle, -CₙH₂ₙ-R(7) ou CF₃ ;
n vaut zéro, 1, 2, 3 ou 4 ;
R(7) signifie (C₃-C₇)-cycloalkyle ou phényle,
qui est non substitué ou substitué par 1-3 substituants choisis dans le groupe constitué par F, Cl, CF₃, méthyle, méthoxy et NR(8)R(9) ;
R(8) et R(9)
signifient H ou (C₁-C₄)-alkyle ;
ou
R(5) signifie H ;
R(6) signifie H ou (C₁-C₄)-alkyle ;
ou
R(5) et R(6)
signifient ensemble 4 ou 5 groupements méthylène, dont un groupement CH₂ peut être remplacé par oxygène, S, NH, N-CH₃ ou N-benzyle ;
R(2) signifie (C₁-C₉)-hétéroaryle,
qui est lié via C ou N et qui est non substitué ou substitué par 1-3 substituants choisis dans le groupe constitué par F, Cl, CF₃, CH₃, méthoxy, hydroxy, amino, méthylamino et diméthylamino ;
ou
R(2) signifie -SR(10), -OR(10), -NR(10)R(11), -CR(10)R(11)R(12) ;
R (10) signifie -CₐH₂ₐ-(C₁-C₉)-hétéroaryle,
qui est non substitué ou substitué par 1-3 substituants choisis dans le groupe constitué par F, Cl, CF₃, CH₃, méthoxy, hydroxy, amino, méthylamino et diméthylamino ;
a vaut zéro, 1 ou 2 ;
R(11) et R(12)
indépendamment l'un de l'autre, sont définis comme R(10) ou signifient hydrogène ou (C₁-C₄)-alkyle ;
R(3) est défini comme R(1) ou signifie (C₁-C₆)-alkyle ou -X-R(13) ;
X signifie oxygène, S, ou NR(14) ;
R(14) signifie H ou (C₁-C₃)-alkyle ;
R(13) signifie H, (C₁-C₆)-alkyle, (C₃-C₈)-cycloalkyle ou -C_{b}H_{2b}-R (15) ;
b vaut zéro, 1, 2, 3 ou 4 ;
ou
R(13) et R(14)
ensemble, signifient 4 ou 5 groupements méthylène, dont un groupement CH₂ peut être remplacé par oxygène, S, NH, N-CH₃ ou N-benzyle ;
R(15) signifie phényle,
qui est non substitué ou substitué par 1-3 substituants choisis dans le groupe constitué par F, Cl, CF₃, méthyle, méthoxy et NR(8)R(9) ;
R(8) et R(9)
signifient H ou (C₁-C₉)-alkyle ;
ainsi que leurs sels pharmaceutiquement acceptables ;
k) benzoylguanidines de formule I 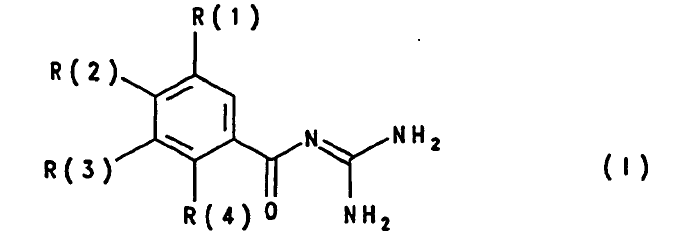 où :
un des substituants R(1), R(2), R(3) ou R(4) signifie : un groupe amino 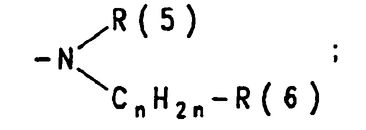
R(5) signifie hydrogène ou C₍₁₋₆₎-alkyle ;
n vaut zéro, 1, 2, 3 ou 4 ;
R(6) signifie H ou C₍₁₋₄)-alkyle ;
un groupement CH₂ pouvant être remplacé par un atome de S ou un groupement NR(7) ;
R(7) signifie hydrogène, méthyle ou éthyle ;
ou
R(6) signifie C₍₃₋₈₎-cycloalkyle ou phényle,
qui est non substitué ou substitué par 1, 2 ou 3 substituants choisis dans le groupe constitué par F, Cl, Br, méthyle, méthoxy et NR(8)R(9) ;
R(8) et R(9)
signifient H, méthyle ou éthyle,
ou
R(5) et R(6)
forment ensemble avec l'atome d'azote un cycle de 5, 6 ou 7 chaînons, dans lequel un atome de C peut être remplacé par oxygène, S ou NR(10) ;
R(10) signifie H, C₍₁₋₃₎-alkyle ou benzyle ;
et à chaque fois les autres substituants R(1), R(2), R(3), R(4) :
signifient hydrogène, F, Cl, Br, I, CN, CF₃, NO₂ CF₃-O-, CₘF₂ₘ₊₁-CH₂O- ou R(11)-C_{q}H_{2q}-Xₚ- ;
m vaut 1, 2 ou 3 ;
q vaut zéro, 1, 2, 3 ou 4 ;
p vaut zéro ou 1 ;
X signifie oxygène ou NR(12) ;
R(12) signifie H ou C(₁₋₃)-alkyle ;
R(11) signifie hydrogène, C(₁₋₆)-alkyle, C₍₃₋₈₎-cycloalkyle ou phényle,
qui est non substitué ou substitué par 1, 2 ou 3 substituants choisis dans le groupe constitué par F, Cl, CH₃, CH₃-O- et NR(13)R(14) ;
R(13), R(14)
signifient H, méthyle ou éthyle,
ainsi que leurs sels pharmaceutiquement acceptables.
1) benzoylguanidines de formule I 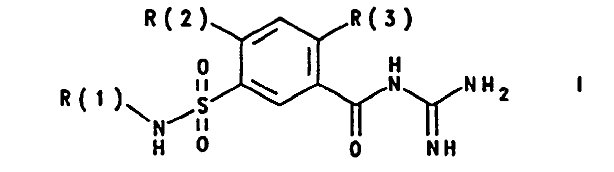 dans laquelle
R(1) signifie R(4)R(5)N-C(X)- ;
X signifie oxygène, S, ou N-R(6) ;
R(4) et R(5)
identiques ou différents, signifient H, (C₁-C₈)-alkyle, (C₃-C₆)-alcényle ou -CₙH₂ₙ-R (7) ;
n vaut zéro, 1, 2, 3 ou 4 ;
R(7) signifie (C₅-C₇)-cycloalkyle ou phényle,
qui est non substitué ou substitué par 1-3 substituants choisis dans le groupe constitué par F, Cl, CF₃, méthoxy et (C₁-C₉)-alkyle ;
ou
R(4) et R(5)
ensemble, signifient 4 ou 5 groupements méthylène, dont un groupement CH₂ peut être remplacé par oxygène, S, NH, N-CH₃ ou N-benzyle ;
R(6) est défini comme R(4) ou signifie amidine ;
R(2) signifie H, F, Cl, Br, I, (C₁-Cₑ) -alkyle, 1-alcényle ou 1-alcynyle, (C₃-C₈) -cycloalkyle, (C₃-C₈) - cycloalkyl-(C₁-C₄)-alkyle, phényle, C₆H₅-(C₁-C₄)-alkyle, naphtyle, biphénylyle, 1,1-diphényl-(C₁-C₄)-alkyle, cyclopentadiényle, pyridyle, thiopyridyle, pyrrolyle, furannyle, thiényle, thiazolyle, oxazolyle, indényle, quinoléyle, indolyle, benzofurannyle, benzothiényle, benzothiazolyle, benzoxazolyle ou -W-R(8) ;
W signifie oxygène, S ou NR(9) ;
R(8) signifie H, C₁-C₆-alkyle, C₅-C₇-cycloalkyle, cyclohexylméthyle, cyclopentylméthyle, -(CH₂)ₘCₚF₂ₚ₊₁ ou -C_{q}H_{2q}-R(10);
m vaut zéro ou 1 ;
p vaut 1, 2 ou 3 ;
q vaut zéro, 1, 2, 3 ou 4 ;
R(10) signifie phényle,
qui est non substitué ou substitué par 1-3 substituants choisis dans le groupe constitué par F, Cl, CF₃, méthyle, méthoxy et NR(11)R(12) ;
R(11) et R(12)
signifient H ou (C₁-C₄)-alkyle ;
R(9) signifie H ou (C₁-C₃) -alkyle ;
ou
R(8) et R(9)
ensemble, signifient 4 ou 5 groupements méthylène, dont un groupement CH₂ peut être remplacé par oxygène, S, NH, N-CH₃ ou N-benzyle ;
R(3) signifie H, F, Cl, Br, I, (C₁-C₆) -alkyle ou -W-R(8) comme défini pour R(2),
ainsi que leurs sels pharmaceutiquement acceptables.
m) benzoylguanidines de formule I 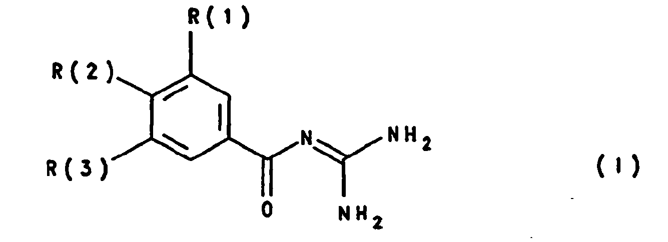 où :
R(1), R(2), R(3)
signifient hydrogène, F, Cl, Br, I ou (C₁-C₁₂) - alkyle ;
un des substituants R(1), R(2) ou R(3) signifie :
N₃, CN, OH ou (C₁-C₁₀)-alkyloxy, lorsqu'au moins un des substituants résiduels R(1), R(2) ou R(3) signifie un radical alkyle suffisamment lipophile comprenant 3 à 12 atomes de carbone ;
ou
un des substituants R(1), R(2) ou R(3) :
signifie R (4)-CₙH₂ₙ-Oₘ ;
m vaut zéro ou 1 ;
n vaut zéro, 1, 2 ou 3 ;
R(4) signifie CpF₂ₚ₊₁ ;
p vaut 1, 2 ou 3, pour autant que n vaille zéro ou 1 ;
ou
R(4) signifie (C₃-C₁₂)-cycloalkyle, phényle, pyridyle, quinoléyle ou isoquinoléyle, les systèmes cycliques aromatiques et hétéroaromatiques étant non substitués ou substitués par un substituant choisi dans le groupe constitué par F, Cl, CF₃, méthyle, méthoxy et NR(5)R(6) ;
R(5) et R(6)
signifient hydrogène ou (C₁-C₉) -alkyle ;
ou un des substituants R(1), R(2) ou R(3) :
signifie -C≡CR(5) ou -C[R(6)]=CR(5) ;
R(5) signifie phényle,
qui est non substitué ou substitué par 1-3 substituants choisis dans le groupe constitué par F, Cl, CF₃, méthyle, méthoxy, hydroxy, amino, méthylamino et diméthylamino, (C₁-C₉)-hétéroaryle,
qui est non substitué ou substitué comme phényle,
ou
R(5) signifie (C₁-C₆)-alkyle,
qui est non substitué ou substitué par 1-3 OH,
ou
R(5) signifie (C₃-C₈)-cycloalkyle,
R(6) signifie hydrogène ou méthyle ;
ainsi que leurs sels pharmacologiquement acceptables.
o) benzoylguanidines de formule I 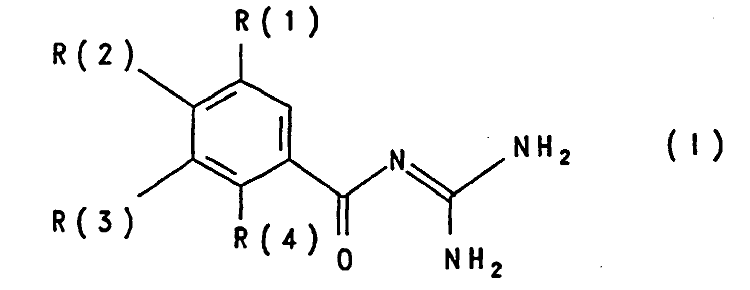 où :
R(1) signifie hydrogène, F, Cl, Br, I, -NO₂, -C=N, Xₒ-(CH₂)ₚ-(CF₂)_{q}-CF₃, R(5)-SOₘ, R(6)-CO- ou R(6)R(7)N-SO₂-, où
X signifie oxygène, S ou NR(14) ;
m vaut zéro, 1 ou 2 ;
o vaut zéro ou 1 ;
p vaut zéro, 1 ou 2 ;
q vaut zéro, 1, 2, 3, 4, 5 ou 6 ;
R(5) et R(6)
signifient (C₁-Cₐ) -alkyle, (C₃-C₈)-alcényle, -CₙH₂ₙ-R(8) ou CF₃ ;
n vaut zéro, 1, 2, 3 ou 4 ;
R(8) signifie (C₃-C₇)-cycloalkyle ou phényle,
qui est non substitué ou substitué par 1-3 substituants choisis dans le groupe constitué par F, Cl, CF₃, méthyle, méthoxy et NR(9)R(10) ;
R(9) et R(10)
signifient H ou (C₁-C₄)-alkyle ;
ou
R(6) signifie H ;
R(7) signifie H ou (C₁-C₄)-alkyle ;
ou
R(6) et R(7)
ensemble, signifient 4 ou 5 groupements méthylène, dont un groupement CH₂ peut être remplacé par oxygène, S, NH, N-CH₃ ou N-benzyle ;
R(2) signifie 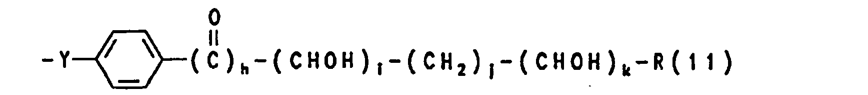 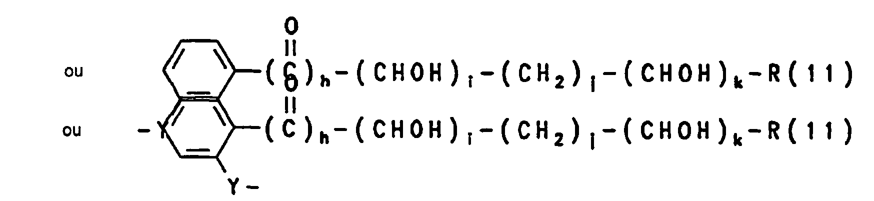
Y signifie oxygène, -S-, ou -NR(12)- ;
R(11) et R(12)
signifient hydrogène ou (C₁-C₃)-alkyle
h vaut zéro ou 1 ;
i, j et k
indépendamment, valent zéro, 1, 2, 3 ou 4 ;
h, i et k ne valant cependant pas simultanément zéro,
R(3) est défini comme R(1) ou signifie (C₁-C₆)-alkyle ou -X-R(13) ;
X signifie oxygène, S ou NR(14)
R(14) signifie H ou (C₁-C₃)-alkyle ;
R(13) signifie H, (C₁-C₆)-alkyle, (C₃-C₈)-cycloalkyle ou -C_{b}H_{2b}-R(15) ;
b vaut zéro, 1, 2, 3 ou 4 ;
ou
R(13) et R(14)
ensemble, signifient 4 ou 5 groupements méthylène, un groupement CH₂ pouvant être remplacé par oxygène, S, NH, N-CH₃ ou N-benzyle ;
R(15) signifie phényle,
qui est non substitué ou substitué par 1-3 substituants choisis dans le groupe constitué par F, Cl, CF₃, méthyle, méthoxy et NR(9)R(10) ;
R(9) et R(10)
signifient H ou (C₁-C₉)-alkyle ;
R(4) signifie hydrogène, -OR(16) ou -NR(16)R(17) ;
R(16) et R(17)
indépendamment, signifient hydrogène ou (C₁-C₃) - alkyle ;
ainsi que leurs sels pharmaceutiquement acceptables ;
p) benzoylguanidines de formule I 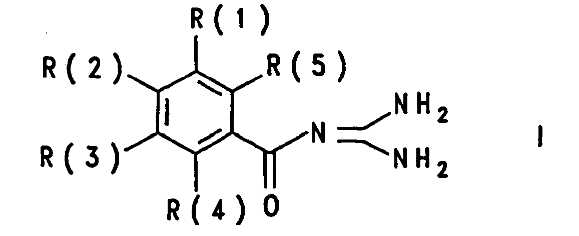 où :
R(1) signifie R(6)-CO ou R(7)R(8)N-CO ;
R(6) signifie (C₁-C₈)-alkyle, (C₁-C₈)-perfluoroalkyle, (C₃-C₈)-alcényle ou -CₙH₂ₙ-R (9) ;
n vaut zéro, 1, 2, 3 ou 4 ;
R(9) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(10)R(11) ;
R(10) et R(11)
signifient H, (C₁-C₄) -alkyle ou (C₁-C₄) - perfluoroalkyle ;
R(7) signifie H, (C₁-C₈)-alkyle, (C₁-C₈)-perfluoroalkyle, (C₃-C₈)-alcényle ou -CₙH₂ₙ-R(12) ;
n vaut zéro, 1, 2, 3 ou 4 ;
R(12) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(13)R(14) ;
R(13) et R(14)
signifient H, (C₁-C₄)-alkyle ou (C₁-C₄)-perfluoroalkyle ;
R(8) signifie H, (C₁-C₄)-alkyle ou (C₁-C₄)-perfluoroalkyle ;
ou
R(7) et R(8)
ensemble, signifient 4 ou 5 groupements méthylène, dont un groupement CH₂ peut être remplacé par oxygène, S, NH, N-CH₃ ou N-benzyle ;
R(2) est défini comme R(1) ou signifie H, F, Cl, Br, I, CN, NO₂, (C₁-C₈)-alkyle, (C₁-C₈)-perfluoroalkyle, (C₃-C₈)-alcényle ou-CₙH₂ₙR(₁₅) ;
n vaut zéro, 1, 2, 3 ou 4 ;
R(15) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(16)R(17) ;
R(16) et R(17)
signifient H, (C₁-C₄)-alkyle ou (C₁-C₄)-perfluoroalkyle ;
ou
R(2) signifie (C₁-C₉)-hétéroaryle,
qui est lié via C ou N et qui est non substitué ou substitué par 1-3 substituants choisis dans le groupe constitué par F, Cl, CF₃, CH₃, méthoxy, hydroxy, amino, méthylamino et diméthylamino ;
ou
R(2) signifie -SR(18), -OR(18), -NR(18)R(19), -CR(18)R(19)R(20) ;
R(18) signifie -CₐH₂ₐ-(C₁-C₉)-hétéroaryle, qui est non substitué ou substitué par 1-3 substituants choisis dans le groupe constitué par F, Cl, CF₃, CH₃, méthoxy, hydroxy, amino, méthylamino et diméthylamino ;
a vaut zéro, 1 ou 2 ;
R(19) et R(20) indépendamment l'un de l'autre, sont définis comme R(18) ou signifient hydrogène, (C₁-C₄)-alkyle ou (C₁-C₄)-perfluoroalkyle ; ou
R(2) signifie R(21)-SOₘ ou R(22)R(23)N-SO₂ ;
m vaut 1 ou 2 ;
R(21) signifie (C₁-C₈)-alkyle, (C₁-C₈)-perfluoroalkyle, (C₃-C₈)-alcényle, -CₙH₂ₙ-R(24),
n vaut zéro, 1, 2, 3 ou 4 ;
R(24) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(27)R(28) ;
R(27) et R(28)
signifient H, (C₁-C₄) -alkyle ou (C₁-C₄) - perfluoroalkyle ;
R(22) signifie H, (C₁-C₈)-alkyle, (C₁-C₈)-perfluoroalkyle, (C₃-C₈)-alcényle, -CₙH₂ₙ-R(29) ;
n vaut zéro, 1, 2, 3 ou 4 ;
R(29) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(30)R(31) ;
R(30) et R(31)
signifient H, (C₁-C₄) -alkyle ou (C₁-C₄) - perfluoroalkyle ;
R(23) signifie H, (C₁-C₄)-alkyle ou (C₁-C₄)-perfluoroalkyle ;
ou
R(22) et R(23)
ensemble, signifient 4 ou 5 groupements méthylène, dont un groupement CH₂ peut être remplacé par oxygène, S, NH, N-CH₃ ou N-benzyle ;
ou
R(2) signifie R(33)X- ;
X signifie oxygène, S, NR(34), (D=O)A-, NR(34)C=MN⁽*⁾R(35)- ;
M signifie oxygène ou S ;
A signifie oxygène ou NR(34) ;
D signifie C ou SO ;
R(33) signifie (C₁-C₈)-alkyle, (C₃-C₈)-alcényle, (CH₂)_{b}C_{d}F_{2d+1}, -CₙH₂ₙ-R (36),
b vaut zéro ou 1 ;
d vaut 1, 2, 3, 4, 5, 6 ou 7 ;
n vaut zéro, 1, 2, 3 ou 4 ;
R (36) signifie (C₃-C₈) -cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(37)R(38) ;
R(37) et R(38) signifient H, (C₁-C₄)-alkyle ou (C₁-C₄)-perfluoroalkyle ;
R(34) signifie H, (C₁-C₄)-alkyle ou (C₁-C₄)-perfluoroalkyle ;
R(35) est défini comme R(33) ;
ou
R(33) et R(34)
ensemble, signifient 4 ou 5 groupements méthylène, dont un groupement CH₂ peut être remplacé par oxygène, S, NH, N-CH₃ ou N-benzyle ;
A et N^{(*)} étant liés au noyau phénylique du corps de base de la benzoylguanidine ;
ou
R(2) signifie -SR(40), -OR(40), -NHR(40), -NR(40)R(41), -CHR(40)R(42), -C[R(42)R(43)OH], -C≡CR(45), -CR(46)=CHR(45), [CR(47)R(48)]ᵤ-(CO)-[CR49)R(50)]ᵥ-R(44) ;
R(40), R(41)
identiques ou différents, signifient -(CH₂)ₚ-(CHOH)_{q}-(CH₂)ᵣ-(CHOH)ₜ-R(51) ou -(CH₂)ₚ-O-(CH₂-CH₂O)_{q}-R(51) ;
R(51) signifie hydrogène ou méthyle ;
u vaut 1, 2, 3 ou 4 ;
v vaut zéro, 1, 2, 3 ou 4 ;
p, q, r
identiques ou différents, valent zéro, 1, 2, 3 ou 4 ;
t vaut 1, 2, 3 ou 4 ;
R(42) et R(43)
identiques ou différents, signifient hydrogène ou (C₁-C₆)-alkyle ;
ou
R(42) et R(43)
forment, ensemble avec l'atome de C qui les porte, (C₃-C₈)-cycloalkyle ;
R(44) signifie H, (C₁-C₆)-alkyle, (C₃-C₈)-cycloalkyle ou -CₑH₂ₑ-R(45) ;
e vaut zéro, 1, 2, 3 ou 4 ;
R(45) signifie phényle,
qui est non substitué ou substitué par 1-3 substituants du groupe constitué par F, Cl, CF₃, méthyle, méthoxy et NR(52)R(53),
R(52) et R(53) signifient H ou (C₁-C₄)-alkyle, ou R(45) signifie (C₁-C₉)-hétéroaryle,
qui est non substitué ou substitué comme phényle ; ou
R(45) signifie (C₁-C₆)-alkyle,
qui est non substitué ou substitué par 1-3 OH,
R(46), R(47), R(48), R(49) et R(50)
signifient hydrogène ou méthyle ;
ou
R(2) signifie R(55)-NH-SO₂- ;
R(55) signifie R(56)R(57)N-(C=Y)- ;
Y signifie oxygène, S, ou N-R(58) ;
R(56) et R(57) identiques ou différents, signifient H, (C₁-C₈)-alkyle, (C₃-C₆)-alcényle ou -CFH₂FR-R(59) ;
f vaut zéro, 1, 2, 3 ou 4 ;
R(59) signifie (C₅-C₇)-cycloalkyle ou phényle,
qui est non substitué ou substitué par 1-3 substituants choisis dans le groupe constitué par F, Cl, CF₃, méthoxy et (C₁-C₄)-alkyle ;
ou
R(56) et R(57)
ensemble, signifient 4 ou 5 groupements méthylène, dont un groupement CH₂ peut être remplacé par oxygène, S, NH, N-CH₃ ou N-benzyle ;
R(58) est défini comme R(56) ou signifie amidine ; R(3), R(4) et R(5)
indépendamment les uns des autres, sont définis comme R(1) ou R(2) ;
ainsi que leurs sels pharmaceutiquement acceptables ;
q) benzoylguanidines de formule I 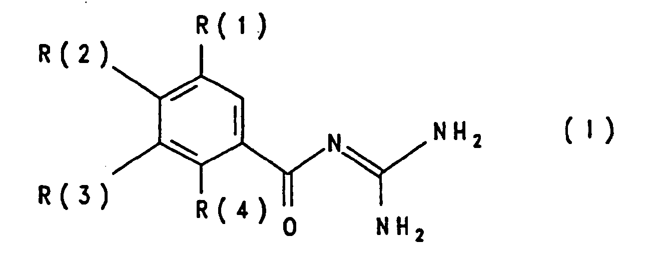 où :
R(1) signifie hydrogène, F, Cl, Br, I, -NO₂, -C=N, -Xₒ-(CH₂)ₚ-(CF₂)_{q}-CF₃, R(5)-SOₘ-, R(6)-CO-, R(6)R(7)N-CO- ou R(6)R(7)N-SO₂- ;
X signifie oxygène, S, ou NR(14) ;
m vaut zéro, 1 ou 2 ;
o vaut zéro ou 1 ;
p vaut zéro, 1 ou 2 ;
q vaut zéro, 1, 2, 3, 4, 5 ou 6 ;
R(5) et R(6)
signifient (C₁-C₈)-alkyle, (C₃-C₆)-alcényle, -CₙH₂ₙ-R(8) ou CF₃ ;
n vaut zéro, 1, 2, 3 ou 4 ;
R(8) signifie (C₃-C₇)-cycloalkyle ou phényle,
qui est non substitué ou substitué par 1 à 3 substituants choisis dans le groupe constitué par F, Cl, CF₃, méthyle, méthoxy et NR(9)R(10) :
R(9) et R(10) signifient H ou (C₁-C₄)-alkyle ;
ou
R(6) signifie hydrogène ;
R(7) signifie hydrogène ou (C₁-C₉)-alkyle ;
ou
R(6) et R(7) ensemble, signifient 4 ou 5 groupements méthylène, dont un groupement CH₂ peut être remplacé par oxygène, S, NH, N-CH₃ ou N-benzyle ;
R(2) signifie 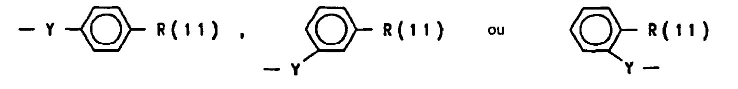
R(11) signifie (C₁-C₉)-hétéroaryle,
qui est lié via C ou N et qui est non substitué ou substitué par 1 à 3 substituants choisis dans le groupe constitué par F, Cl, CF₃, CH₃, méthoxy, hydroxy, amino, méthylamino, diméthylamino et benzyle ; Y signifie oxygène, S, ou NR(12) ;
R(12) signifie H ou (C₁-C₄)-alkyle ;
R(3) est défini comme R(1) ;
ou
R(3) signifie (C₁-C₆)-alkyle ou -X-R(13) ;
X signifie oxygène, S, ou NR(14) ;
R(14) signifie H ou (C₁-C₃)-alkyle ;
R (13) signifie H, (C₁-C₆)-alkyle, (C₃-C₈)-cycloalkyle ou -C_{b}H_{2b}-R(15) ;
b vaut zéro, 1, 2, 3 ou 4 ;
ou
R(13) et R(14)
ensemble, signifient 4 ou 5 groupements méthylène, dont un groupement CH₂ peut être remplacé par oxygène, S, NH, N-CH₃ ou N-benzyle ;
R(15) signifie phényle,
qui est non substitué ou substitué par 1-3 substituants choisis dans le groupe constitué par F, Cl, CF₃, méthyle, méthoxy et NR(9)R(10) ;
R(9) et R(10)
signifient H ou (C₁-C₄)-alkyle ;
R(4) signifie hydrogène, -OR(16), -NR(16)R(17) ou CᵣF₂ᵣ₊₁ ;
R(16) et R(17)
indépendamment, signifient hydrogène ou (C₁-C₃) - alkyle ;
r vaut 1, 2, 3 ou 4 ;
ainsi que leurs sels pharmaceutiquement acceptables ;
r) hétérocycles à 5 cycles benzocondensés de formule I 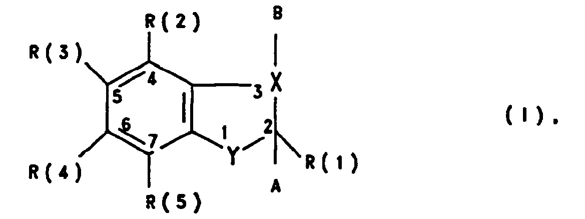 où :
X signifie N ou CR(6) ;
Y signifie oxygène, S ou NR(7) ;
A, B ensemble, signifient une liaison ; ou
A, B signifient tous deux hydrogène, pour autant que simultanément, X signifie CR(6) et Y signifie NR(7) ; un des substituants R(1) à R(6)
signifie un groupement -CO-N=C(NH₂)₂ :
à chaque fois les autres substituants R(1) à R(6) : signifient hydrogène, F, Cl, Br, I ou (C₁-C₆)-alkyle ;
jusqu'à deux des autres substituants R(1) à R(6) : signifient CN, NO₂, N₃, (C₁-C₉) -alkyloxy ou CF₃ ;
jusqu'à un des autres substituants : signifie R(8)-CₙH₂ₙ-Z- ;
n vaut zéro à 10 ;
la chaîne alkylène -CₙH₂ₙ- étant linéaire ou ramifiée et un atome de C pouvant être remplacé par un atome d'oxygène ou de S ou de N ;
R (8) signifie hydrogène, (C₂-C₆)-alcényle ou (C₃-C₁₀) - cycloalkyle,
qui est non substitué ou substitué par 1 à 4 groupements méthyle ou un groupement OH, ou qui peut contenir un groupement éthylène -CH=CH-, et un groupement méthylène pouvant être remplacé par un atome d'oxygène ou un atome de S ou un atome de N ;
ou
R(8) signifie phényle,
non substitué ou substitué par 1 à 3 substituants choisis dans le groupe constitué par F, Cl, Br, I, CF₃, CH₃-S(O)ₛ - ou R(9)-W_{y} ;
s vaut zéro, 1 ou 2 ;
R(9) signifie H, méthyle, éthyle,
W signifie oxygène ou NR(10) ;
R(10) signifie H ou méthyle,
y vaut zéro ou 1 ;
ou
R (8) signifie CₘF₂ₘ₊₁ ;
m vaut 1 à 3 ;
ou
R(8) signifie 1-naphtyle ou 2-naphtyle, pyridyle, quinoléyle ou isoquinoléyle ;
Z signifie -CO-, -CH₂- ou - [CR(11)(OH)]_{q}- ;
q vaut 1, 2 ou 3 ;
R(11) signifie H ou méthyle,
ou
Z signifie oxygène ou NR(12) ;
R(12) signifie H ou méthyle,
ou
Z signifie -S(O)ₛ- ;
s vaut zéro, 1 ou 2 ;
ou
Z signifie SO₂-R(13) ;
R(13) signifie H ou (C₁-C₄)-alkyle ;
R (7) signifie hydrogène, (C₁-C₁₀)-alkyle, (C₂-C₁₀) - alcényle ou R(8)-CₙH₂ₙ- ;
ainsi que leurs sels pharmaceutiquement acceptables ;
s) benzoylguanidines de formule I 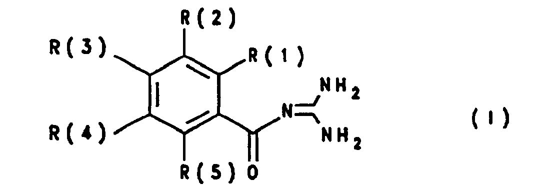 où :
R(1), R(3) ou R(4)
signifie -NR(6)C=XNR(7)R(8) ;
X signifie oxygène ou S ;
R(6) signifie hydrogène, (C₁-C₈)-alkyle, (C₁-C₈) - perfluoroalkyle, (C₃-C₈)-alcényle ou -CₙH₂ₙ-R (9) ;
n vaut zéro, 1, 2, 3 ou 4 ;
R(9) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(10)R(11) ;
R(10) et R(11)
signifient H, (C₁-C₄) -alkyle ou (C₁-C₄) - perfluoroalkyle ;
R(7) signifie hydrogène, (C₁-C₈)-alkyle, (C₁-C₈)-perfluoroalkyle, (C₃-C₈)-alcényle ou -CₒH₂ₒ-R(9) ;
o vaut zéro, 1, 2, 3 ou 4 ;
R(12) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(13)R(14) ;
R(13) et R(14)
signifient H, (C₁-C₄)-alkyle ou (C₁-C₄) - perfluoroalkyle ;
R(8) est défini comme R(7) ;
ou
R(7) et R(8) ensemble, signifient 4 ou 5 groupements méthylène, dont un groupement CH₂ peut être remplacé par oxygène, S, NH, N-CH₃ ou N-benzyle ;
les substituants à chaque fois restants R(2), R(3), R(4), R(5) ou R(1), R(2), R(4), R(5) ou R(1), R(2), R(3), R(5)
indépendamment les uns des autres, signifient hydrogène, F, Cl, Br, I, -Oₜₐ(C₁-C₈)-alkyle, -O_{tb}(C₃-C₈)-alcényle,
-O_{tc}C (CH₂) _{b}C_{d}F_{2d+1}, -O_{td}CₚH₂ₚR (18),
ou jusqu'à 2 groupements CN, NO₂, NR(16)R(17),
b vaut zéro ou 1 ;
d vaut 1, 2, 3, 4, 5, 6 ou 7 ;
ta vaut zéro ou 1 ;
tb vaut zéro ou 1 ;
tc vaut zéro ou 1 ;
td vaut zéro ou 1 ;
p vaut zéro, 1, 2, 3 ou 4 ;
R(18) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(19)R(20) ;
R(19) et R(20)
signifient hydrogène, (C₁-C₄)-alkyle ou (C₁-C₄) - perfluoroalkyle ;
R(16) signifie hydrogène, (C₁-C₈)-alkyle, (C₁-C₈) - perfluoroalkyle, (C₃-C₈)-alcényle, -CqH₂q-R (21) ;
q vaut zéro, 1, 2, 3 ou 4 ;
R(21) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy ou NR(22)R(23) ;
R(22) et R(23) signifient hydrogène, (C₁-C₄)-alkyle ou (C₁-C₄) - perfluoroalkyle ;
R(17) signifie hydrogène, (C₁-C₈)-alkyle, (C₁-C₈)-perfluoroalkyle, (C₃-C₈)-alcényle, -CᵣH₂ᵣ-R(24) ;
r vaut zéro, 1, 2, 3 ou 4 ;
R (24) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(25)R(26) ;
R(25) et R(26)
signifient hydrogène, (C₁-C₄)-alkyle ou (C₁-C₄)-perfluoroalkyle ;
ou
R(16) et R(17)
ensemble, signifient 4 ou 5 groupements méthylène, dont un groupement CH₂ peut être remplacé par oxygène, S, NH, N-CH₃ ou N-benzyle ;
ainsi que leurs sels pharmaceutiquement acceptables ;
t) guanidines disubstituées par acyle de formule I 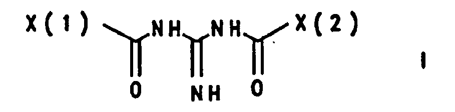 où :
X(1) et X(2) signifient 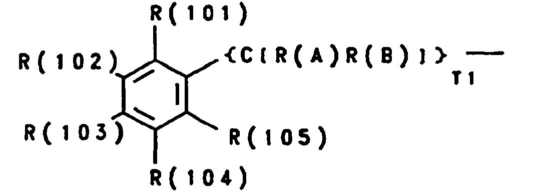
T1 vaut zéro, 1, 2, 3 ou 4 ;
R(A) et R(B)
indépendamment l'un de l'autre, signifient hydrogène, F, Cl, Br, I, CN, OR(106), (C₁-C₈)-alkyle, (C₃-C₈) -cycloalkyle, O_{zk}(CH₂)_{zl}C_{zm}F_{2zm+1}, NR (107) R (108), phényle ou benzyle,
les aromatiques étant non substitués ou substitués par 1-3 substituants choisis dans le groupe constitué par F, Cl, CF₃, méthyle, méthoxy et NR(109)R(110) ; R(109) et R(110)
signifient hydrogène, (C₁-C₄)-alkyle ou (C₁-C₄) - perfluoroalkyle ;
zl vaut zéro, 1, 2, 3 ou 4 ;
zk vaut zéro ou 1 ;
zm vaut 1, 2, 3, 4, 5, 6, 7 ou 8
R(106)
signifie hydrogène, (C₁-C₈)-alkyle, (C₁-C₈)-perfluoroalkyle, (C₃-C₈)-alcényle, (C₃-C₈)-cycloalkyle, phényle ou benzyle,
les aromatiques étant non substitués ou substitués par 1-3 substituants choisis dans le groupe constitué par F, Cl, CF₃, méthyle, méthoxy et NR(111)R(112) ; R(111) et R(112)
signifient hydrogène, (C₁-C₄)-alkyle ou (C₁-C₄)-perfluoroalkyle ;
R(107) et R(108)
indépendamment l'un de l'autre, sont définis comme R(106) ;
ou
R(107) et R(108)
ensemble, signifient 4 ou 5 groupements méthylène, dont un groupement CH₂ peut être remplacé par oxygène, S, NH, N-CH₃ ou N-benzyle ;
ou
X(1) et X(2) signifient 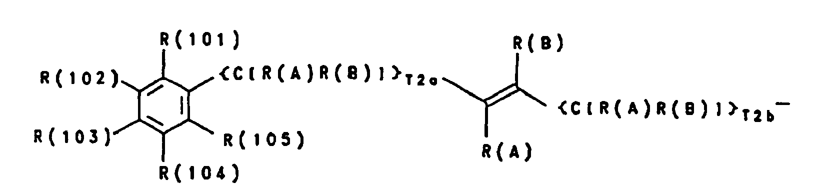
T2a et T2b
indépendamment l'un de l'autre, valent zéro, 1 ou 2 ;
la double liaison pouvant être en configuration (E) ou (Z) ; ;
ou
X(1) et X(2) signifient 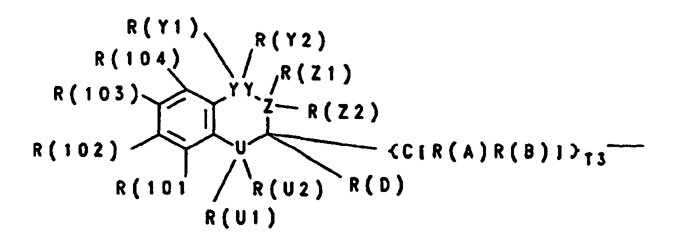
T3 vaut zéro, 1 ou 2 ;
U, YY et Z
indépendamment les uns des autres, signifient C ou N,
U, YY, Z pouvant porter le nombre suivant de substituants :
| U, YY ou Z | lié dans le cycle à une double liaison | nombre de substituants autorisés |
|---|---|---|
| C | oui | 1 |
| C | non | 2 |
| N | oui | 0 |
| N | non | 1 |
R(D) signifie hydrogène, (C₁-C₈)-alkyle ou (C₁-C₈) - perfluoroalkyle ;
R(U1), R(U2), R(Y1), R(Y2), R(Z1), R(Z2)
indépendamment les uns des autres, signifient hydrogène, F, Cl, Br, I, CN, OR(114), (C₁-C₈)-alkyle, (C₃-C₈) -cycloalkyle, O_{zka}(CH₂)_{zla}C_{zma}F_{2zma+1}, NR(115) R (116), phényle ou benzyle,
les aromatiques étant non substitués ou substitués par 1-3 substituants choisis dans le groupe constitué par F, Cl, CF₃, méthyle, méthoxy, NR(117)R(118) ;
R(117) et R(118)
signifient hydrogène, (C₁-C₄)-alkyle ou (C₁-C₄) - perfluoroalkyle ;
zka vaut zéro ou 1 ;
zla vaut zéro, 1, 2, 3 ou 4 ;
zma vaut 1, 2, 3, 4, 5, 6, 7 ou 8 ;
R(114)
signifie hydrogène, (C₁-C₈)-alkyle, (C₁-C₈) - perfluoroalkyle, (C₃-C₈)-alcényle, (C₃-C₈)-cycloalkyle, phényle ou benzyle,
les aromatiques étant non substitués ou substitués par 1-3 substituants choisis dans le groupe constitué par F, Cl, CF₃, méthyle, méthoxy et NR(119)R(120) ; R(119) et R(120)
signifient hydrogène, (C₁-C₉)-alkyle ou (C₁-C₄) - perfluoroalkyle ;
R(115) et R(116) indépendamment l'un de l'autre, sont définis comme R(114) ;
ou
R(115) et R(116)
ensemble, signifient 4 ou 5 groupements méthylène, dont un groupement CH₂ peut être remplacé par oxygène, S, NH, N-CH₃ ou N-benzyle ;
la constitution U représentant azote (N), YY représentant (N) et Z représentant carbone (C) étant cependant exclue,
R(101), R(102), R(103), R(104) et R(105)
indépendamment les uns des autres, signifient hydrogène, F, Cl, Br, I, -C≡N, X_{zoa}-(CH₂)_{zpa}-(C_{zqa}F_{2zqa}+1), R(110a)-SO_{zbm}, R(110b)R(110c)N-CO, R(111a)-CO- ou R(112a)R(113a)N-SO₂-,
le groupement perfluoroalkyle étant linéaire ou ramifié,
X signifie oxygène, S ou NR(114a) ;
R(114a)
signifie H ou (C₁-C₃)-alkyle ;
zoa vaut zéro ou 1 ;
zbm vaut zéro, 1 ou 2 ;
zpa vaut zéro, 1, 2, 3 ou 4 ;
zqa vaut 1, 2, 3, 4, 5, 6, 7 ou 8 ;
R(110a), R(110b), R(111a) et R(112a)
indépendamment, signifient (C₁-C₈)-alkyle, (C₃-C₈)-alcényle, -C_{zn}H_{2zn}-R(115a) ou (C₁-C₈)-perfluoroalkyle ;
zn vaut zéro, 1, 2, 3 ou 4 ;
R(115a) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(116a)R(117a) ; R(116a) et R(117a)
signifient hydrogène, (C₁-C₉)-perfluoroalkyle ou (C₁-C₄)-alkyle ;
ou
R(110b), R(111a) et R(112a)
signifient hydrogène ;
R(110c) et R(113a)
indépendamment, signifient hydrogène, (C₁-C₉)-perfluoroalkyle ou (C₁-C₄)-alkyle ;
ou
R(110b) et R(110c) ainsi que R(112a) et R(113a)
ensemble, signifient 4 ou 5 groupements méthylène, dont un groupement CH₂ peut être remplacé par oxygène, soufre, NH, N-CH₃ ou N-benzyle ;
ou
R(101), R(102), R(103), R(104), R(105)
indépendamment les uns des autres, signifient (C₁-C₈)-alkyle, -C_{za1}H_{2za1}R(118a) ou (C₃-C₈)-alcényle,
zal vaut zéro, 1, 2, 3 ou 4 ;
R(118a)
signifie (C₃-C₈)-cycloalkyle, phényle, biphénylyle ou naphtyle,
les aromatiques étant non substitués ou substitués par 1-3 substituants du groupe constitué par F, Cl, CF₃, méthyle, méthoxy ou NR(119a)R(119b) ;
R(119a) et R(119b)
signifient hydrogène, (C₁-C₄)-alkyle ou (C₁-C₄)-perfluoroalkyle ;
ou
R(101), R(102), R(103), R(104), R(105)
indépendamment les uns des autres, signifient (C₁-C₉)-hétéroaryle,
qui est lié via C ou N et qui est non substitué ou substitué par 1-3 substituants du groupe constitué par F, Cl, CF₃, CH₃, méthoxy, hydroxy, amino, méthylamino et diméthylamino ;
ou
R(101), R(102), R(103), R(104), R(105)
indépendamment les uns des autres, signifient -C≡C-R(193) ;
R(193) signifie phényle, qui est non substitué ou substitué par 1-3 substituants choisis dans le groupe constitué par F, Cl, CF₃, méthyle, méthoxy ou NR(194)R(195) ;
R(194) et R(195) signifient hydrogène ou CH₃ ;
ou
R(101), R(102), R(103), R(104), R(105)
indépendamment les uns des autres, signifient :
-Y-para-C₆H₄-(CO)_{zh}-(CHOH)_{zi}(CH₂)_{zj}-(CHOH)_{zk}-R(123), - Y-méta-C₆H₄-(CO)_{zad}-(CHOH)_{zae}-(CH₂)_{zaf}-(CHOH)_{zag}-R(124)
ou
-Y-ortho-C₆H₄-(CO)_{zah}-(CHOH)_{zao}-(CH₂)_{zap}-(CHOH)_{zak}-R(125) ;
Y signifie oxygène, -S- ou NR(122d) ;
zh, zad, zah
indépendamment, valent zéro ou 1 ;
zi, zj, zk, zae, zaf, zag, zao, zap et zak
indépendamment, valent zéro, 1, 2, 3 ou 4 ;
où, à chaque fois cependant
zh, zi et zk ne valent pas simultanément zéro,
zad, zae et zag ne valent pas simultanément zéro, et
zah, zao et zak ne valent pas simultanément zéro, R(123), R(124) R(125) et R(122d)
indépendamment, signifient hydrogène ou (C₁-C₃)-alkyle ;
ou
R(101), R(102), R(103), R(104) et R(105)
indépendamment les uns des autres, signifient SR(129), -OR(130), -NR(131)R(132) ou -CR(133)R(134)R(135) ;
R(129), R(130), R(131) et R(133)
indépendamment, signifient -C_{zab}H_{2zab}-(C₁-C₉)-hétéroaryle,
qui est non substitué ou substitué par 1-3 substituants du groupe constitué par F, Cl, CF₃, CH₃, méthoxy, hydroxy, amino, méthylamino et diméthylamino ; zab vaut zéro, 1 ou 2 ;
R(132), R(134) et R(135)
indépendamment les uns des autres, sont définis comme R(129) ou signifient hydrogène, (C₁-C₉)-alkyle ou (C₁-C₄)-perfluoroalkyle ;
ou
R(101), R(102), R(103), R(104) et R(105)
indépendamment les uns des autres, signifient -W-para-(C₆H₄)-R(196), -W-méta-(C₆H₄)-R(197) ou -W-ortho-(C₆H₄)-R(198) ;
R(196), R(197) et R(198)
indépendamment, signifient (C₁-C₉)-hétéroaryle,
qui est lié via C ou N et qui est non substitué ou substitué par 1 à 3 substituants du groupe constitué par F, Cl, CF₃, CH₃, méthoxy, hydroxy, amino, méthylamino, diméthylamino et benzyle ;
W signifie oxygène, S ou NR(136) ;
R(136) signifie hydrogène ou (C₁-C₄)-alkyle ;
ou
R(101), R(102), R(103), R(104) et R(105)
indépendamment les uns des autres, signifient R(146)X(1a)- ;
X(1a) signifie oxygène, S, NR(147), (D=O)A-, NR(148)C=MN(*)R⁽149)- ;
M signifie oxygène ou soufre ;
A signifie oxygène ou NR(150) ;
D signifie C ou SO ;
R(146)
signifie (C₁-C₈)-alkyle, (C₃-C₈)-alcényle, (CH₂)_{zbz}C_{zdz}F_{2zdz+1} OU -C_{zxa}H_{2zxa}-R(151) ;
zbz vaut zéro ou 1 ;
zdz vaut 1, 2, 3, 4, 5, 6 ou 7 ;
zxa vaut zéro, 1, 2, 3 ou 4 ;
R(151)
signifie (C₃-C₈)-cycloalkyle, phényle, biphénylyle ou naphtyle,
les aromatiques étant non substitués ou substitués par 1-3 substituants du groupe constitué par F, Cl, CF₃, méthyle, méthoxy et NR(152)R(153) ;
R(152) et R(153)
signifient hydrogène, (C₁-C₄)-alkyle ou (C₁-C₄)-perfluoroalkyle ;
R(147), R(148) et R(150)
indépendamment, signifient hydrogène, (C₁-C₄)-alkyle, (C₁-C₄)-perfluoroalkyle ;
R(149) est défini comme R(146) ;
ou
R(146) et R(147) ou, selon le cas, R(146) et R(148)
ensemble, signifient 4 ou 5 groupements méthylène, dont un groupement CH₂ peut être remplacé par oxygène, soufre, NH, N-CH₃ ou N-benzyle ;
A et N(*) étant liés au noyau phénylique du corps de base alcanoyole ;
ou
R(101), R(102), R(103), R(104) et R(105) indépendamment les uns des autres, signifient -SR(164), -OR(165), -NHR(166), -NR(167)R(168), -CHR(169)R(170), -CR(154)R(155)OH, -C≡CR(156), -CR(158)=CR(157) ou -[CR(159)R(160)]_{zu}-(C=O)-[CR(161)R(162)]_{zv}-R(163) ; R(164), R(165), R(166), R(167), R(169)
identiques ou différents, signifient -(CH₂)_{zy}-(CHOH)_{zz}-(CH₂)_{zaa}-(CHOH)_{zt}-R(171) ou -(CH₂)_{zab}-O-(CH₂-CH₂O)_{zac}-R(172) ; R(171) et R(172)
signifient hydrogène ou méthyle ;
zu vaut 1, 2, 3 ou 4 ;
zv vaut zéro, 1, 2, 3 ou 4 ;
zy, zz, zaa, zab, zac
identiques ou différents, valent zéro, 1, 2, 3 ou 4 ;
zt vaut 1, 2, 3 ou 4 ;
R(168), R(170), R(154), R(155)
identiques ou différents, signifient hydrogène ou (C₁-C₆)-alkyle ;
ou
R(169) et R(170) ou, selon le cas, R(154) et R(155)
forment, ensemble avec l'atome de C qui les porte, (C₃-C₈)-cycloalkyle ;
R(163)
signifie hydrogène, (C₁-C₆)-alkyle, (C₃-C₈)-cycloalkyle ou -C_{zeb}H_{2zeb}-R(173) ;
zeb vaut zéro, 1, 2, 3 ou 4 ;
R(156), R(157) et R(173)
indépendamment, signifient phényle qui est non substitué ou substitué par 1-3 substituants choisis dans le groupe constitué par F, Cl, CF₃, méthyle, méthoxy et NR(174)R(175) ;
R(174) et R(175)
signifient hydrogène ou (C₁-C₄)-alkyle ;
ou
R(156), R(157) et R(173)
indépendamment, signifient (C₁-C₉)-hétéroaryle,
qui est non substitué ou substitué comme phényle ;
R(158), R(159), R(160), R(161) et R(162)
signifient hydrogène ou méthyle,
ou
R(101), R(102), R(103), R(104), R(105)
indépendamment les uns des autres, signifient R(176)-NH-SO₂- ;
R(176)
signifie R(177)R(178)N-(C=Y')- ;
Y' signifie oxygène, S, ou N-R(179) ;
R(177) et R(178)
identiques ou différents, signifient hydrogène, (C1-C8)-alkyle, (C₃-C₆)-alcényle ou -C_{zfa}H_{2zfa}-R(180) ; zfa vaut zéro, 1, 2, 3 ou 4 ;
R(180)
signifie (C₅-C₇)-cycloalkyle ou phényle,
qui est non substitué ou substitué par 1-3 substituants choisis dans le groupe constitué par F, Cl, CF₃, méthyle, méthoxy ou (C₁-C₄)-alkyle ;
ou
R(177) et R(178)
ensemble, signifient 4 ou 5 groupements méthylène, dont un groupement CH₂- peut être remplacé par oxygène, soufre, NH, N-CH₃ ou N-benzyle ;
R(179)
est défini comme R(177) ou signifie amidine ;
ou
R(101), R(102), R(103), R(104), R(105)
indépendamment, signifient NR(184a)R(185), OR(184b), SR(184c) ou -C_{znx}H_{2znx}-R(184d) ;
znx vaut zéro, 1, 2, 3 ou 4 ;
R(184d)
signifie (C₃-C₇)-cycloalkyle ou phényle,
qui est non substitué ou substitué par 1-3 substituants du groupe constitué par F, Cl, CF₃, méthyle, méthoxy et NR(116k)R(117k) ;
R(116k) et R(117k)
signifient hydrogène ou C₁-C₄-alkyle ; R(184a), R(184b), R(184c), R(185)
indépendamment les uns des autres, signifient hydrogène, (C₁-C₈)-alkyle, (C₁-C₈)-perfluoroalkyle ou (CH₂)_{za}O-R(184g) ;
zao vaut zéro, 1, 2, 3 ou 4 ;
R(184g)
signifie (C₃-C₇)-cycloalkyle ou phényle,
qui est non substitué ou substitué par 1-3 substituants du groupe constitué par F, Cl, CF₃, méthyle, méthoxy et NR(184u)R(184v) ;
R(184u) et R(184v)
signifient hydrogène ou C₁-C₄-alkyle ;
ou
R(184a) et R(185)
ensemble, signifient 4 ou 5 groupements méthylène, dont un groupement CH₂ peut être remplacé par oxygène, soufre, NH, N-CH₃ ou N-benzyle ;
ainsi que leurs sels pharmaceutiquement acceptables ;
u) benzoylguanidines de formule I 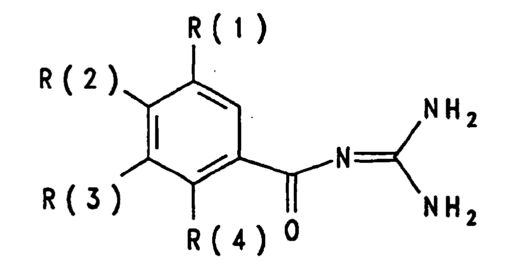 où :
R(1) signifie H, F, Cl, Br, I, CN, NO₂, (C₁-C₈)-alkyle, (C₃-C₈) -cycloalkyle ou Xₐ-(CH₂)_{b}-(CF₂)_{c}-CF₃ ;
X signifie oxygène, S ou NR(5) ;
a vaut zéro ou 1 ;
b vaut zéro, 1 ou 2 ;
c vaut zéro, 1, 2 ou 3 ;
R(5) signifie H, (C₁-C₄)-alkyle ou -C_{d}H_{2d}R(6) ;
d vaut zéro, 1, 2, 3 ou 4 ;
R(6) signifie (C₃-C₈)-cycloalkyle, phényle, biphénylyle ou naphtyle,
les aromatiques étant non substitués ou substitués par 1 à 3 substituants choisis dans le groupe constitué par F, Cl, CF₃, méthyle, méthoxy et NR(7)R(8) ;
R(7) et R(8)
indépendamment, signifient H ou (C₁-C₄) -alkyle ; ou
R(1) signifie -SR(10), -OR(10) ou -CR(10)R(11)R(12) ;
R(10) signifie -C_{f}H_{2f}-(C₃-C₈)-cycloalkyle, -(C₁-C₉)-hétéroaryle ou phényle,
les systèmes aromatiques étant non substitués ou substitués par 1 à 3 substituants choisis dans le groupe constitué par F, Cl, CF₃, CH₃, méthoxy, hydroxy, amino, méthylamino et diméthylamino ;
f vaut zéro, 1 ou 2 ;
R(11) et R(12)
indépendamment l'un de l'autre, sont définis comme R(10) ou signifient hydrogène ou (C₁-C₄)-alkyle ;
ou
R(1) signifie phényle, naphtyle, biphénylyle ou (C₁-C₉)-hétéroaryle,
ce dernier étant lié via C ou N,
et qui sont non substitués ou substitués par 1 à 3 substituants choisis dans le groupe constitué par F, Cl, CF₃, CH₃, méthoxy, hydroxy, amino, méthylamino et diméthylamino ;
ou
R(1) signifie -SR(13), -OR(13), -NHR(13), -NR(13)R(14), -CHR(13)R(15), -C[R(15)R(16)]OH, -C≡CR(18), -C[R(19)]=CR(18), -[CR(20)R(21)]ₖ-(CO)-[CR(22)R(23)R(24)]₁ R(13) et R(14)
identiques ou différents, signifient -(CH₂)_{g}-(CHOH)ₕ-(CH₂)ᵢ-(CHOH)ⱼ-R(17),
R(17) signifie hydrogène ou méthyle ;
- (CH₂)_{g}-O-(CH₂-CH₂O)ₕ-R(24),
g, h, i
identiques ou différents, valent zéro, 1, 2, 3 ou 4 ;
j vaut 1, 2, 3 ou 4 ;
R(15) et R(16)
identiques ou différents, signifient hydrogène, (C₁-C₆)-alkyle ou, ensemble avec l'atome de carbone qui les porte, (C₃-C₈)-cycloalkyle ;
R(18) signifie phényle,
qui est non substitué ou substitué par 1 à 3 substituants choisis dans le groupe constitué par F, Cl, CF₃, méthyle, méthoxy et NR(25)R(26) ;
R(25) et R(26)
signifient H ou (C₁-C₄)-alkyle ;
ou
R(18) signifie (C₁-C₉)-hétéroaryle,
qui est non substitué ou substitué comme phényle ;
ou
R(18) signifie (C₁-C₆) -alkyle,
qui est non substitué ou substitué par 1 à 3 OH ;
ou
R(18) signifie (C₃-C₈)-cycloalkyle ;
R(19), R(20), R(21), R(22) et R(23)
signifient hydrogène ou méthyle ;
k vaut zéro, 1, 2, 3 ou 4 ;
1 vaut zéro, 1, 2, 3 ou 4 ;
R(24) signifie H, (C₁-C₆)-alkyle, (C₃-C₈)-cycloalkyle ou -CₘHₘ-R(18) ;
m vaut 1, 2, 3 ou 4 ;
R(2) et R(3)
indépendamment l'un de l'autre, sont définis comme R(1) ;
R(4) signifie (C₁-C₃)-alkyle, F, Cl, Br, I, CN ou -(CH₂)ₙ-(CF₂)ₒ-CF₃ ;
n vaut zéro ou 1 ;
o vaut zéro, 1 ou 2 ;
ainsi que leurs sels pharmaceutiquement acceptables ;
v) acylguanidines de formule I 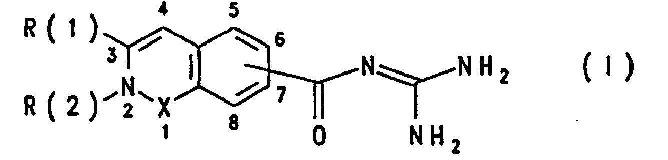 où :
X signifie carbonyle, sulfonyle,
R(1) signifie (C₁-C₈)-alkyle,
non substitué ou substitué par hydroxy,
signifie (C₃-C₈)-cycloalkyle, phényle,
qui est non substitué ou substitué par 1-3 substituants du groupe constitué par F, Cl, CF₃, CH₃, méthoxy, hydroxy, amino, méthylamino ou diméthylamino, R(2) signifie H, (C₁-C₄)-alkyle,
ainsi que leurs sels pharmaceutiquement acceptables ;
w) guanidides d'acides alkylcarboxyliques substitués par phényle portant des groupements perfluoroalkyle de formule I 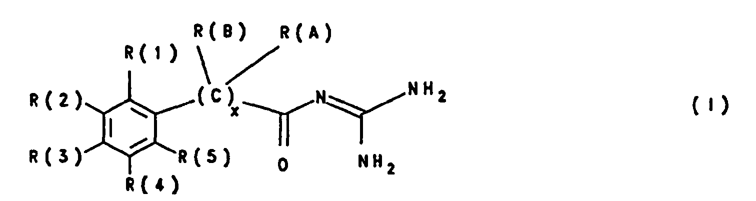 où :
R(A) signifie hydrogène, F, Cl, Br, I, CN, OR(6), (C₁-C₈)-alkyle, (C₃-C₈)-cycloalkyle, Oᵣ(CH₂)ₐC_{b}F_{2b+1} ou NR(7)R(8) ;
r vaut zéro ou 1 ;
a vaut zéro, 1, 2, 3 ou 4 ;
b vaut 1, 2, 3, 4, 5, 6, 7 ou 8 ;
R(6) signifie hydrogène, (C₁-C₈)-alkyle, (C₃-C₈)-alcényle, (C₃-C₈)-cycloalkyle, phényle ou benzyle,
les aromatiques étant non substitués ou substitués par 1-3 substituants choisis dans le groupe constitué par F, Cl, CF₃, méthyle, méthoxy et NR(9)R(10) ;
R(9) et R(10)
signifient H, (C₁-C₄) -alkyle ou (C₁-C₄)-perfluoroalkyle ;
R(7) et R(8)
indépendamment l'un de l'autre, sont définis comme R(6) ;
R(B) indépendamment, est défini comme R(A) ;
x vaut 1, 2 ou 3 ;
R(1) signifie hydrogène, (C₁-C₈)-alkyle, (C₃-C₈)-cycloalkyle, -Oₜ(CH₂)_{d}CₑF₂ₑ₊₁, F, Cl, Br, I ou CN ;
t vaut zéro ou 1 ;
d vaut zéro, 1, 2, 3 ou 4 ;
e vaut 1, 2, 3, 4, 5, 6, 7 ou 8 ;
R(2), R(3), R(4) et R(5)
indépendamment les uns des autres, sont définis comme R(1) ;
cependant à condition
qu'au moins un des substituants R(1), R(2), R(3), R(4), R(5), R(A) et R(B) représente un groupement -Oₜ(CH₂)_{d}CₑF₂ₑ₊₁ ou -Oᵣ(CH₂)ₐC_{b}F_{2b+1},
ainsi que leurs sels pharmaceutiquement acceptables ;
x) hétéroaroylguanidines de formule I 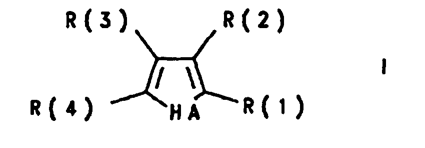 où :
HA signifie SOₘ, O ou NR(5) ;
m vaut zéro, 1 ou 2 ;
R(5) signifie hydrogène, (C₁-C₈)-alkyle ou -CₐₘH₂ₐₘR(81) ;
am vaut zéro, 1 ou 2 ;
R(81) signifie (C₃-C₈)-cycloalkyle ou phényle,
qui est non substitué ou substitué par 1-3 substituants choisis dans le groupe constitué par F, Cl, CF₃, méthyle, méthoxy et NR(82)R(83) ;
R(82) et R(83)
signifient H ou CH₃ ;
ou
R(81) signifie (C₁-C₉)-hétéroaryle,
qui est lié via C ou N et qui est non substitué ou substitué par 1-3 substituants choisis dans le groupe constitué par F, Cl, CF₃, CH₃, méthoxy, hydroxy, amino, méthylamino et diméthylamino ;
un des deux substituants R(1) et R(2) signifie -CO-N=C(NH₂)₂ ;
et à chaque fois l'autre substituant
signifie hydrogène, F, Cl, Br, I, (C₁-C₃)-alkyle, -OR(6), CᵣF₂ᵣ₊₁, -CO-N=C(NH₂)₂ ou -NR(6)R(7) ;
R(6) et R(7)
indépendamment, signifient hydrogène ou (C₁-C₃)-alkyle ;
r vaut 1, 2, 3 ou 4 ;
R(3) et R(4)
indépendamment l'un de l'autre, signifient hydrogène, F, Cl, Br, I, -C=N, X-(CH₂)ₚ-(C_{q}-F_{2q+1}), R(8)-SO_{bm}, R(9)R(10)N-CO, R(11)-CO- ou R(12)R(13)N-SO₂-,
le groupement perfluoroalkyle étant linéaire ou ramifié,
X signifie oxygène, S ou NR(14) ;
R(14) signifie H ou (C₁-C₃)-alkyle ;
bm vaut zéro, 1 ou 2 ;
p vaut zéro, 1 ou 2 ;
q vaut zéro, 1, 2, 3, 4, 5 ou 6 ;
R(8), R(9), R(11) et R(12)
indépendamment les uns des autres, signifient (C₁-C₈)-alkyle, (C₃-C₆)-alcényle, -CₙH₂ₙ-R(15) ou CF₃ ;
n vaut zéro, 1, 2, 3 ou 4 ;
R(15) signifie (C₃-C₇)-cycloalkyle ou phényle ;
qui est non substitué ou substitué par 1-3 substituants choisis dans le groupe constitué par F, Cl, CF₃, méthyle, méthoxy ou NR(16)R(17) ;
R(16) et R(17)
signifient H ou (C₁-C₄)-alkyle ;
ou
R(9), R(11) et R(12)
signifient H ;
R(10) et R(13)
indépendamment, signifient H ou (C₁-C₄)-alkyle ; ou
R(9) et R(10) ainsi que R(12) et R(13)
ensemble, signifient 4 ou 5 groupements méthylène, dont un groupement CH₂ peut être remplacé par oxygène, S, NH, N-CH₃ ou N-benzyle ;
ou
R(3) et R(4)
indépendamment l'un de l'autre, signifient (C₁-C₈)-alkyle ou -Cₐ₁H₂ₐ₁R(18) ; al vaut zéro, 1 ou 2 ;
R(18) signifie (C₃-C₈)-cycloalkyle ou phényle ;
qui est non substitué ou substitué par 1-3 substituants choisis dans le groupe constitué par F, Cl, CF₃, méthyle, méthoxy et NR(19)R(20) ;
R(19) et R(20)
signifient H ou CH₃ ;
ou
R(3) et R(4)
indépendamment l'un de l'autre, signifient (C₁-C₉)-hétéroaryle, qui est lié via C ou N et qui est non substitué ou substitué par 1-3 substituants choisis dans le groupe constitué par F, Cl, CF₃, CH₃, méthoxy, hydroxy, amino, méthylamino et diméthylamino ;
ou
R(3) et R(4)
indépendamment l'un de l'autre, signifient : 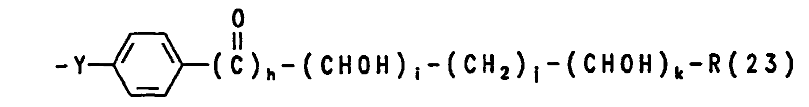 ou 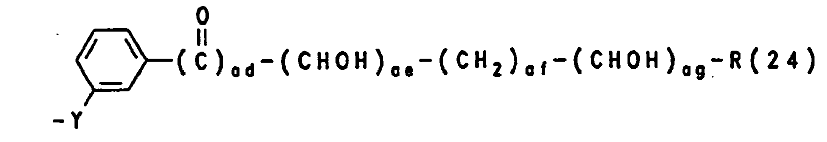 ou 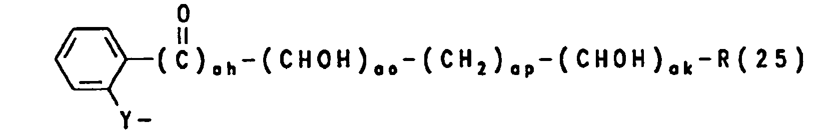
Y signifie oxygène, -S- ou NR(22) ;
h, ad, ah indépendamment, valent zéro ou 1 ;
j, k, ae, af, ag, ao, ap et ak indépendamment, valent zéro, 1, 2, 3, 4,
où, à chaque fois cependant
h, i et k ne valent pas simultanément zéro,
ad, ae et ag ne valent pas simultanément zéro et
ah, ao et ak ne valent pas simultanément zéro et
R(23), R(24) R(25) et R(22)
indépendamment, signifient hydrogène ou (C₁-C₃)-alkyle ;
ou
R(3) et R(4)
indépendamment l'un de l'autre, signifient hydrogène, F, Cl, Br, I, CN, (C₁-C₈)-alkyle, (C₁-C₈)-perfluoroalkyle, (C₃-C₈)-alcényle ou -C_{g}H_{2g}R(26) ;
g vaut zéro, 1, 2, 3 ou 4 ;
R(26) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(27)R(28) ;
R(27) et R(28)
signifient H, (C₁-C₄)-alkyle ou (C₁-C₄)-perfluoroalkyle ;
ou
R(3) et R(4)
indépendamment l'un de l'autre, signifient SR(29), -OR(30), -NR(31)R(32) ou -CR(33)R(34)R(35) ; R(29), R(30), R(31) et R(33) indépendamment, signifient -CₐH₂ₐ-(C₁-C₉)-hétéroaryle, qui est non substitué ou substitué par 1-3 substituants choisis dans le groupe constitué par F, Cl, CF₃, CH₃, méthoxy, hydroxy, amino, méthylamino et diméthylamino ;
a vaut zéro, 1 ou 2 ;
R(32), R(34) et R(35)
indépendamment l'un de l'autre, sont définis comme R(29) ou signifient hydrogène, (C₁-C₄)-alkyle ou (C₁-C₄)-perfluoroalkyle ;
ou
R(3) et R(4)
indépendamment l'un de l'autre, signifient : 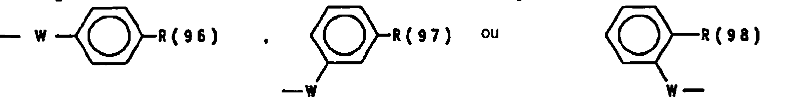
R(96), R(97) et R(98)
indépendamment, signifient (C₁-C₉)-hétéroaryle,
qui est lié via C ou N et qui est non substitué ou substitué par 1 à 3 substituants choisis dans le groupe constitué par F, Cl, CF₃, CH₃, méthoxy, hydroxy, amino, méthylamino, diméthylamino ou benzyle ;
W signifie oxygène, S ou NR(36) ;
R(36) signifie H ou (C₁-C₄) -alkyle ;
ou
R(3) et R(4)
indépendamment l'un de l'autre, signifient R(37)-SO_{cm}, ou R(38)R(39)N-SO₂- ;
cm vaut 1 ou 2 ;
R(37) signifie (C₁-C₈)-alkyle, (C₁-C₈)-perfluoroalkyle, (C₃-C₈)-alcényle ou -CₛH₂ₛR(40) ;
s vaut zéro, 1, 2, 3 ou 4 ;
R(40) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(41)R(42) ;
R(41) et R(42)
signifient H, (C₁-C₄)-alkyle ou (C₁-C₄)-perfluoroalkyle ;
R(38) signifie H, (C₁-C₈)-alkyle, (C₁-C₈)-perfluoroalkyle, (C₃-C₈)-alcényle ou -C_{w}H_{2w}-R(43) ;
w vaut zéro, 1, 2, 3 ou 4 ;
R(43) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(44)R(45) ;
R(44) et R(45)
signifient H, (C₁-C₄)-alkyle ou (C₁-C₄)-perfluoroalkyle ;
R(39) signifie H, (C₁-C₉)-alkyle ou (C₁-C₄)-perfluoroalkyle ;
ou
R(38) et R(39)
ensemble, signifient 4 ou 5 groupements méthylène, dont un groupement CH₂ peut être remplacé par oxygène, S, NH, N-CH₃ ou N-benzyle ;
ou
R(3) et R(4) indépendamment l'un de l'autre, signifient R(46)X(1)- ;
X(1) signifie oxygène, S, NR(47), (D=O)A-, NR(48)C=MN⁽*⁾R(49)-,
M signifie oxygène ou S ;
A signifie oxygène ou NR(50) ;
D signifie C ou SO ;
R(46) signifie (C₁-C₈)-alkyle, (C₃-C₈)-alcényle, (CH₂)_{b}C_{d}F_{2d+1} ou -CₓH₂ₓ-R(51) ;
b vaut zéro ou 1 ;
d vaut 1, 2, 3, 4, 5, 6 ou 7 ;
x vaut zéro, 1, 2, 3 ou 4 ;
R(51) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(52)R(53) ;
R(52) et R(53)
signifient H, (C₁-C₄)-alkyle ou (C₁-C₄)-perfluoroalkyle ;
R(47), R(48) et R(50)
indépendamment, signifient hydrogène, (C₁-C₄)-alkyle ou (C₁-C₄)-perfluoroalkyle ;
R(49) est défini comme R(46) ;
ou
R(46) et R(47) ou, selon le cas, R(46) et R(48)
ensemble, signifient 4 ou 5 groupements méthylène, dont un groupement CH₂ peut être remplacé par oxygène, S, NH, N-CH₃ ou N-benzyle ;
A et N(*) étant liés au noyau phénylique du corps de base de la benzoylguanidine ;
ou
R(3) et R(4)
indépendamment l'un de l'autre, signifient -SR(64), -OR(65), -NHR(66), -NR(67)R(68), -CHR(69)R(70), -C(OH)R(54)R(55), -C≡CR(56), -CR(58)=CHR(57), -[CR(59)R(60)]ᵤ-(CO)-[CR(61)R(62)]ᵥ-R(63) ;
R(64), R(65), R(66), R(67) et R(69)
identiques ou différents, signifient -(CH₂)_{y}-(CHOH)_{z}-(CH₂)ₐₐ-(CH₂OH)ₜ-R(71) ou - (CH₂)_{ab}-O-(CH₂-CH₂O)_{ac}-R(72),
R(71) et R(72)
signifient hydrogène ou méthyle ;
u vaut 1, 2, 3 ou 4 ;
v vaut zéro, 1, 2, 3 ou 4 ;
y, z, aa
identiques ou différents, valent zéro, 1, 2, 3 ou 4 ;
t vaut 1, 2, 3 ou 4 ;
R(68), R(70), R(54) et R(55)
identiques ou différents, signifient hydrogène, (C₁-C₆)-alkyle ;
ou
R(69) et R(70) ou, selon le cas, R(54) et R(55)
forment, ensemble avec l'atome de C qui les porte, (C₃-C₈)-cycloalkyle ;
R(63)
signifie H, (C₁-C₆)-alkyle, (C₃-C₈)-cycloalkyle ou -CₑH₂ₑ-R(73) ;
e vaut zéro, 1, 2, 3 ou 4 ;
R(56), R(57) et R(73)
indépendamment, signifient phényle
qui est non substitué ou substitué par 1-3 substituants choisis dans le groupe constitué par F, Cl, CF₃, méthyle, méthoxy et NR(74)R(75) ;
R(74) et R(75)
signifient H ou (C₁-C₄) -alkyle ;
ou
R(56), R(57) et R(73)
indépendamment, signifient (C₁-C₉)-hétéroaryle,
qui est non substitué ou substitué comme phényle ; R(58), R(59), R(60), R(61) et R(62)
signifient hydrogène ou méthyle,
ou
R(3) et R(4)
indépendamment l'un de l'autre, signifient R(76)-NH-SO₂- ;
R(76) signifie R(77)R(78)N-(C=Y')- ;
Y' signifie oxygène, S, ou N-R(79) ;
R(77) et R(78) identiques ou différents, signifient H, (C₁-C₈)-alkyle, (C₃-C₆)-alcényle, -C_{f}H_{2f}R(80) ;
f vaut zéro, 1, 2, 3 ou 4 ;
R(80) signifie (C₅-C₇)-cycloalkyle ou phényle,
qui est non substitué ou substitué par 1-3 substituants choisis dans le groupe constitué par F, Cl, CF₃, méthoxy et (C₁-C₄)-alkyle ;
ou
R(77) et R(78)
ensemble, signifient 4 ou 5 groupements méthylène, dont un groupement CH₂ peut être remplacé par oxygène, S, NH, N-CH₃ ou N-benzyle ;
R(79) est défini comme R(77) ou signifie amidine ;
ou
R(3) et R(4) indépendamment l'un de l'autre, signifient NR(84)R(85) ;
R(84) et R(85) indépendamment l'un de l'autre, signifient H, (C₁-C₄)-alkyle, ou, ensemble, signifient 4 ou 5 groupements méthylène,
dont un groupement CH₂ peut être remplacé par oxygène, S, NH, N-CH₃ ou N-benzyle ; ou dont un ou deux groupements CH₂ peuvent être remplacés par CH-C_{dm}H_{2dm+1}, ainsi que leurs sels pharmaceutiquement acceptables ;
y) hétéroaroylguanidines bicycliques de formule I 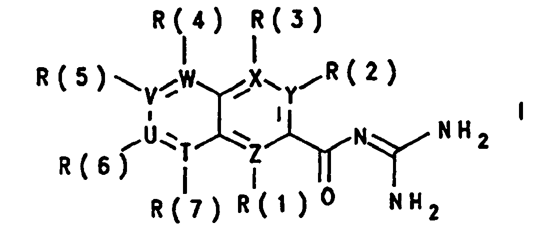 où :
T, U, V, W, X, Y et Z
indépendamment les uns des autres, signifient azote ou carbone ;
cependant, avec la limitation
que X et Z ne représentent pas simultanément azote,
et que T, U, V, W, X, Y et Z ne portent pas de substituants lorsqu'ils représentent azote,
et que pas plus de quatre d'entre eux représentent simultanément azote,
R(1) et R(2)
indépendamment l'un de l'autre, signifient hydrogène, F, Cl, Br, I, (C₁-C₃)-alkyle, (C₁-C₃)-perfluoroalkyle, OR(8), NR(8)R(9) ou C(=O)N=C(NH₂)₂ ; R(8) et R(9)
indépendamment l'un de l'autre, signifient hydrogène ou (C₁-C₃)-alkyle,
ou
R(8) et R(9)
ensemble, signifient 4 ou 5 groupements méthylène, dont un groupement CH₂ peut être remplacé par oxygène, S, NH, N-CH₃ ou N-benzyle ;
R(3), R(4), R(5), R(6) et R(7)
indépendamment les uns des autres, signifient hydrogène, F, Cl, Br, I, -C=N, Xₖ-(CH₂)ₚ-(C_{q}F_{2q+1}), R(10a)-SO_{bm}, R(10b)R(10c)N-CO, R(11)-CO- ou R(12)R(13)N-SO₂-,
le groupement perfluoroalkyle étant linéaire ou ramifié ;
X signifie oxygène, S ou NR(14) ;
R(14) signifie H ou (C₁-C₃)-alkyle ;
bm vaut zéro, 1 ou 2 ;
p vaut zéro, 1 ou 2 ;
k vaut zéro ou 1 ;
q vaut 1, 2, 3, 4, 5 ou 6 ;
R(10a), R(10b), R(11) et R(12)
indépendamment les uns des autres, signifient (C₁-C₈)-alkyle, (C₃-C₆)-alcényle, -CₙH₂ₙ-R(15) ou (C₁-C₈)-perfluoroalkyle ;
n vaut zéro, 1, 2, 3 ou 4 ;
R(15) signifie (C₃-C₇)-cycloalkyle ou phényle, qui est non substitué ou substitué par 1-3 substituants choisis dans le groupe constitué par F, Cl, CF₃, méthyle, méthoxy et NR(16)R(17) ;
R(16) et R(17)
signifient H ou C₁-C₄-alkyle ;
ou
R(10b), R(11) et R(12)
signifient hydrogène ;
R(10c) et R(13)
indépendamment, signifient hydrogène ou (C₁-C₄)-alkyle
ou
R(10b) et R(10c) ainsi que R(12) et R(13)
ensemble, signifient 4 ou 5 groupements méthylène, dont un groupement CH₂ peut être remplacé par oxygène, soufre, NH, N-CH₃ ou N-benzyle ;
ou
R(3), R(4), R(5), R(6) et R(7)
indépendamment les uns des autres, signifient (C₁-C₈)-alkyle, -Cₐ₁H₂ₐ₁R(18) ou (C₃-C₈)-alcényle ;
al vaut zéro, 1 ou 2 ;
R(18) (C₃-C₈)-cycloalkyle, phényle, biphényle ou naphtyle, les aromatiques étant non substitués ou substitués par 1-3 substituants choisis dans le groupe constitué par F, Cl, CF₃, méthyle, méthoxy et NR(19a)R(19b) ;
R(19a) et R(19b)
signifient hydrogène, (C₁-C₄)-alkyle ou (C₁-C₄)-perfluoroalkyle ;
ou
R(3), R(4), R(5), R(6) et R(7)
indépendamment les uns des autres, signifient (C₁-C₉)-hétéroaryle, qui est lié via C ou N et qui est non substitué ou substitué par 1-3 substituants du groupe constitué par F, Cl, CF₃, CH₃, méthoxy, hydroxy, amino, méthylamino et diméthylamino ;
ou
R(3), R(4), R(5), R(6) et R(7) indépendamment les uns des autres, signifient : 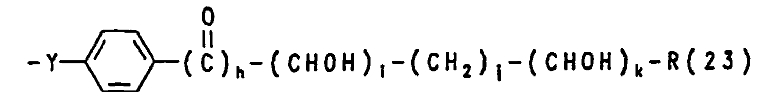 ou 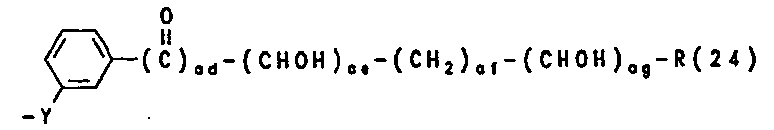 ou 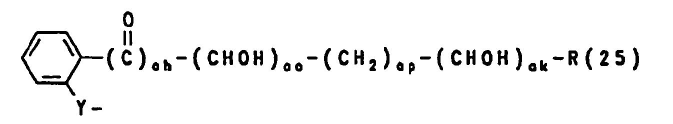
Y signifie oxygène, -S- ou NR(22) ;
h, ad, ah
indépendamment les uns des autres, valent zéro ou 1 ;
j, k, ae, af, ag, ao, ap et ak
indépendamment les uns des autres, valent zéro, 1, 2, 3 ou 4 ;
où, à chaque fois cependant
h, i et k ne valent pas simultanément zéro,
ad, ae et ag ne valent pas simultanément zéro et
ah, ao et ak ne valent pas simultanément zéro et R(23), R(24) R(25) et R(22)
indépendamment les uns des autres, signifient hydrogène ou (C₁-C₃)-alkyle ;
ou
R(3), R(4), R(5), R(6) et R(7)
indépendamment les uns des autres, signifient SR(29), -OR(30), -NR(31)R(32) ou -CR(33)R(34)R(35) ; R(29), R(30), R(31) et R(33)
indépendamment les uns des autres, signifient -CₐH₂ₐ-(C₁-C₉) -hétéroaryle, qui est non substitué ou substitué par 1-3 substituants choisis dans le groupe constitué par F, Cl, CF₃, CH₃, méthoxy, hydroxy, amino, méthylamino et diméthylamino ;
a vaut zéro, 1 ou 2 ;
R(32), R(34) et R(35)
indépendamment les uns des autres, sont définis comme R(29) ou signifient hydrogène, (C₁-C₄)-alkyle ou (C₁-C₉)-perfluoroalkyle ;
ou
R(3), R(4), R(5), R(6) et R(7)
indépendamment les uns des autres, signifient : 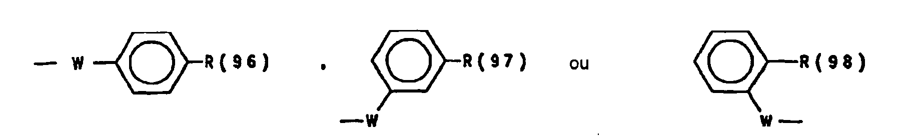
R(96), R(97) et R(98)
indépendamment les uns des autres, signifient (C₁-C₉)-hétéroaryle, qui est lié via C ou N et qui est non substitué ou substitué par 1 à 3 substituants du groupe constitué par F, Cl, CF₃, CH₃, méthoxy, hydroxy, amino, méthylamino, diméthylamino ou benzyle ;
W signifie oxygène, S ou NR(36) ;
R(36) signifie H ou (C₁-C₄)-alkyle ;
ou
R(3), R(4), R(5), R(6) et R(7)
indépendamment les uns des autres, signifient R(46)X(1)- ;
X(1) signifie oxygène, S, NR(47), (D=O)A-, NR(48)C=MN(*)R(49)- ;
M signifie oxygène ou soufre ;
A signifie oxygène ou NR(50) ;
D signifie C ou SO ;
R(46) signifie (C₁-C₈)-alkyle, (C₃-C₈)-alcényle, (CH₂)_{b}C_{d}F_{2d+1} ou -CₓH₂ₓ-R(51) ;
b vaut zéro ou 1 ;
d vaut 1, 2, 3, 4, 5, 6 ou 7 ;
x vaut zéro, 1, 2, 3 ou 4 ;
R(51) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(52)R(53) ;
R(52) et R(53)
signifient hydrogène, (C₁-C₄)-alkyle ou (C₁-C₄)-perfluoroalkyle ;
R(47), R(48) et R(50) indépendamment, signifient hydrogène, (C₁-C₄)-alkyle ou (C₁-C₄)-perfluoroalkyle : R(49) est défini comme R(46) ;
ou
R(46) et R(47) ou, selon le cas, R(46) et R(48)
ensemble, signifient 4 ou 5 groupements méthylène, dont un groupement CH₂ peut être remplacé par oxygène, soufre, NH, N-CH₃ ou N-benzyle ;
A et N(*) étant liés au noyau phénylique du corps de base hétéroaroylguanidine ;
ou
R(3), R(4), R(5), R(6) et R(7)
indépendamment les uns des autres, signifient -SR(64), -OR(65), -NHR(66), -NR(67)R(68), -CHR(69)R(70) ou -CR(54)R(55)OH, -C≡CR(56), -CR(58)=CR(57) ou -[CR(59)R(60)]ᵤ-CO-[CR(61)R(62)]ᵥ-R(63) ;
R(64), R(65), R(66), R(67) et R(69)
identiques ou différents,
signifient -(CH₂)_{y}-(CHOH)_{z}-(CH₂)ₐₐ-(CHOH)ₜ-R(71) ou -(CH₂)_{ab}-O-(CH₂-CH₂O)_{ac}-R(72) ;
R(71) et R(72)
indépendamment l'un de l'autre, signifient hydrogène ou méthyle ;
u vaut 1, 2, 3 ou 4 ;
v vaut zéro, 1, 2, 3 ou 4 ;
y, z, aa identiques ou différents,
valent zéro, 1, 2, 3 ou 4 ;
t vaut 1, 2, 3 ou 4 ;
R(68), R(70), R(54) et R(55)
identiques ou différents, signifient hydrogène ou (C₁-C₆)-alkyle ;
ou
R(69) et R(70) ou, selon le cas, R(54) et R(55)
forment, ensemble avec l'atome de C qui les porte, (C₃-C₈)-cycloalkyle ;
R(63)
signifie hydrogène, (C₁-C₆)-alkyle, (C₃-C₈)-cycloalkyle ou -CₑH₂ₑ-R(45) ;
e vaut zéro, 1, 2, 3 ou 4 ;
R(56), R(57) et R(73) indépendamment,
signifient phényle qui est non substitué ou substitué par 1-3 substituants choisis dans le groupe constitué par F, Cl, CF₃, méthyle, méthoxy et NR(74)R(75) ;
R(74) et R(75)
signifient hydrogène ou (C₁-C₄)-alkyle ;
ou
R(56), R(57) et R(73) indépendamment,
signifient (C₁-C₉)-hétéroaryle, qui est non substitué ou substitué comme phényle ;
R(58), R(59), R(60), R(61) et R(62)
signifient hydrogène ou méthyle ;
ou
R(3), R(4), R(5), R(6) et R(7)
indépendamment les uns des autres, signifient R(76)-NH-SO₂- ;
R(76) signifie R(77)R(78)N-(C=Y')- ;
Y' signifie oxygène, S, ou N-R(79) ;
R(77) et R(78)
identiques ou différents, signifient hydrogène, (C₁-C₈)-alkyle, (C₃-C₆)-alcényle ou -C_{f}H_{2f}R(80) ;
f vaut zéro, 1, 2, 3 ou 4 ;
R(80) signifie (C₅-C₇)-cycloalkyle ou phényle, qui est non substitué ou substitué par 1-3 substituants choisis dans le groupe constitué par F, Cl, CF₃, méthoxy et (C₁-C₄)-alkyle ;
ou
R(77) et R(78)
ensemble, signifient 4 ou 5 groupements méthylène, dont un groupement CH₂ peut être remplacé par oxygène, soufre, NH, N-CH₃ ou N-benzyle ;
R(79) est défini comme R(77) ou signifie amidine ; ou
R(3), R(4), R(5), R(6) et R(7)
indépendamment les uns des autres, signifient NR(84a)R(85), OR(84b), SR(84c) ou -CₙH₂ₙ-R(84d) ;
n vaut zéro, 1, 2, 3 ou 4 ;
R(84d) signifie (C₃-C₇)-cycloalkyle ou phényle, qui est non substitué ou substitué par 1-3 substituants du groupe constitué par F, Cl, CF₃, méthyle, méthoxy et NR(16)R(17) ;
R(16) et R(17)
signifient hydrogène ou C₁-C₄-alkyle ;
R(84a), R(84b), R(84c) et R(85)
indépendamment les uns des autres, signifient hydrogène, (C₁-C₈)-alkyle, (C₁-C₈)-perfluoroalkyle ou (CH₂)ₐₓ-R(84g) ;
ax vaut zéro, 1, 2, 3 ou 4 ;
R(84g) signifie (C₃-C₇)-cycloalkyle ou phényle, qui est non substitué ou substitué par 1-3 substituants choisis dans le groupe constitué par F, Cl, CF₃, méthyle, méthoxy et NR(84u)R(84v) ;
R(84u) et R(84v)
signifient hydrogène ou C₁-C₄-alkyle ;
ou
R(84a) et R(85)
ensemble, signifient 4 ou 5 groupements méthylène, dont un groupement CH₂ peut être remplacé par oxygène, soufre, NH, N-CH₃ ou N-benzyle,
ainsi que leurs sels pharmaceutiquement acceptables ;
z) benzoylguanidines de formule I 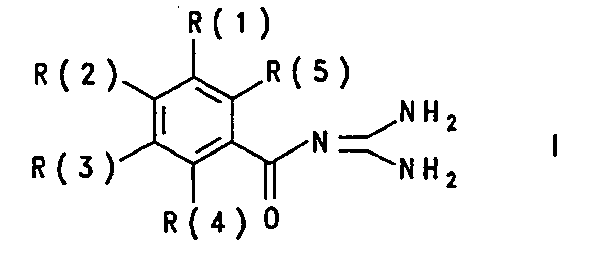 où :
R(1) signifie R(6)-SOₘ ;
m vaut zéro, 1 ou 2 ;
R(6) signifie perfluoroalkyle comprenant 1, 2, 3, 4, 5 ou 6 atomes de carbone, qui est linéaire ou ramifié ;
R(2) et R(3)
indépendamment l'un de l'autre, signifient hydrogène, F, Cl, Br, I, alkyle comprenant 1, 2, 3 ou 4 atomes de carbone, alcoxy comprenant 1, 2, 3 ou 4 atomes de carbone ou phénoxy,
qui est non substitué ou substitué par 1-3 substituants choisis dans le groupe constitué par F, Cl, méthyle, et méthoxy ;
ou
R(2) et R(3)
indépendamment l'un de l'autre, signifient pyrrol-1-yle, pyrrol-2-yle ou pyrrol-3-yle,
qui est non substitué ou substitué par 1 à 4 substituants, choisis dans le groupe constitué par F, Cl, Br, I, CN, alcanoyle comprenant 2, 3, 4, 5, 6, 7 ou 8 atomes de carbone, alcoxycarbonyle comprenant 2, 3, 4, 5, 6, 7 ou 8 atomes de carbone, formyle, carboxy, CF₃, méthyle et méthoxy ;
R(4) et R(5)
indépendamment l'un de l'autre, signifient hydrogène, alkyle comprenant 1, 2 ou 3 atomes de carbone, F, Cl, Br, I, CN, OR(7), NR(8)R(9) ou -(CH₂)ₙ-(CF₂)o-CF₃ ;
R(7), R(8) et R(9)
indépendamment les uns des autres, signifient hydrogène ou alkyle comprenant 1, 2, 3 ou 4 atomes de carbone ;
n vaut zéro ou 1 ;
o vaut zéro, 1 ou 2 ;
ainsi que leurs sels pharmacologiquement acceptables.
aa) guanidides d'acides alcénylcarboxyliques substitués par phényle portant des groupements perfluoroalkyle de formule I 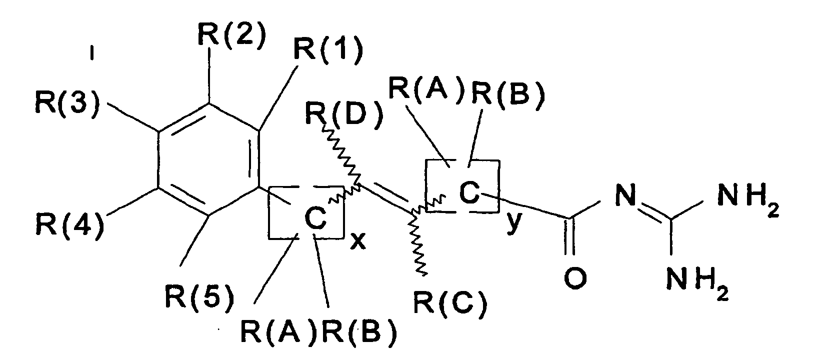 où :
R(A) signifie hydrogène, F, Cl, Br, I, CN, OH, OR(6), (C₁-C₈)-alkyle, Oᵣ(CH₂)ₐC_{b}F_{2b+1}, (C₃-C₈)-cycloalkyle ou NR(7)R(8) ; ;
r vaut zéro ou 1 ;
a vaut zéro, 1, 2, 3 ou 4 ;
b vaut 1, 2, 3, 4, 5, 6, 7 ou 8 ;
R(6) signifie (C₁-C₈)-alkyle, (C₁-C₄)-perfluoroalkyle, (C₃-C₈)-alcényle, (C₃-C₈)-cycloalkyle, phényle ou benzyle ;
les aromatiques étant non substitués ou substitués par 1-3 substituants du groupe constitué par F, Cl, CF₃, méthyle, méthoxy et NR(9)R(10) ;
R(9) et R(10)
signifient H, (C₁-C₄)-alkyle ou (C₁-C₄)-perfluoroalkyle ;
R(7) et R(8)
indépendamment l'un de l'autre, sont définis comme R(6) ;
ou
R(7) et R(8)
signifient ensemble 4 ou 5 groupements méthylène, dont un groupement CH₂ peut être remplacé par oxygène, soufre, NH, N-CH₃ ou N-benzyle ;
R(B) indépendamment, est défini comme R(A) ;
X vaut zéro, 1 ou 2 ;
Y vaut zéro, 1 ou 2 ;
R(C) signifie hydrogène, F, Cl, Br, I, CN, OR(12), (C₁-C₈)-alkyle, Oₚ(CH₂)_{f}C_{g}F_{2g+1} ou (C₃-C₈-cycloalkyle ;
p vaut zéro ou 1 ;
f vaut zéro, 1, 2, 3 ou 4 ;
g vaut 1, 2, 3, 4, 5, 6, 7 ou 8 ;
R(12)
signifie (C₁-C₈)-alkyle, (C₁-C₄)-perfluoroalkyle, (C₃-C₈)-alcényle, (C₃-C₈)-cycloalkyle, phényle ou benzyle ;
les aromatiques phényle ou benzyle étant non substitués ou substitués par 1-3 substituants du groupe constitué par F, Cl, CF₃, méthyle, méthoxy et NR(13)R(14) ;
R(13) et R(14)
indépendamment l'un de l'autre, signifient H, (C₁-C₉)-alkyle ou (C₁-C₉)-perfluoroalkyle ;
R(D) indépendamment, est défini comme R(C) ;
R(1) signifie hydrogène, (C₁-C₈)-alkyle, -Oₜ(CH₂)_{d}CeF₂ₑ₊₁, (C₃-C₈)-cycloalkyle, F, Cl, Br, I ou CN ;
t vaut zéro ou 1 ;
d vaut zéro, 1, 2, 3 ou 4 ;
e vaut 1, 2, 3, 4, 5, 6, 7 ou 8 ;
R(2), R(3), R(4) et R(5)
indépendamment les uns des autres, sont définis comme R(1) ;
cependant à condition
qu'au moins un des substituants R(A), R(B), R(C), R(D), R(1), R(2), R(4) ou R(5) représente un groupement Oᵣ(CH₂)ₐC_{b}F_{2b+1}, Oₚ(CH₂)_{f}CgF_{2g+1} ou Oₜ(CH₂)_{d}CₑF₂ₑ₊₁ et que R(3) ne représente pas un groupement Oₜ(CH₂)_{d}CₑF₂ₑ₊₁ ;
ainsi que leurs sels pharmaceutiquement acceptables ;
ab) benzoylguanidines substituées en position ortho par amino de formule I 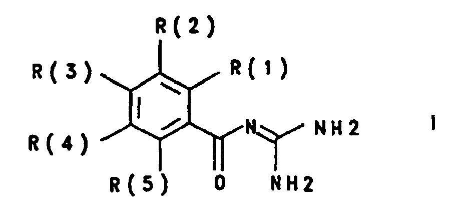 où :
R(1) signifie NR(50)R(6),
R(50) et R(6)
indépendamment l'un de l'autre, signifient hydrogène, (C₁-C₈-alkyle ou (C₁-C₈)-perfluoroalkyle ; R(2), R(3), R(4) et R(5)
indépendamment les uns des autres, signifient R(10)-SOₐ-_{,} R(11)R(12)N-CO-, R(13)-CO- ou R(14)R(15)N-SO₂- ;
a vaut zéro, 1 ou 2,
R(10), R(11), R(12), R(13), R(14) et R(15)
indépendamment les uns des autres, signifient (C₁-C₈)-alkyle, (C₁-C₈)-perfluoroalkyle, (C₃-C₆)-alcényle ou -C_{ab}H_{2ab}-R(16) ; ab vaut zéro, 1, 2, 3 ou 4 ;
R(16) signifie (C₃-C₇)-cycloalkyle ou phényle,
qui est non substitué ou substitué par 1-3 substituants choisis dans le groupe constitué par F, Cl, CF₃, méthyle, méthoxy ou NR(17)R(18) ;
R(17) et R(18)
indépendamment l'un de l'autre, signifient H, CF₃ ou (C₁-C₄)-alkyle ;
ou
R(11) et R(12) ainsi que R(14) et R(15)
signifient ensemble 4 ou 5 groupements méthylène, dont un groupement CH₂ peut être remplacé par oxygène, S, NH, N-CH₃ ou N-benzyle ;
ou
R(11), R(12), R(14) et R(15)
indépendamment les uns des autres, signifient hydrogène ;
ou
R(2), R(3), R(4) et R(5)
indépendamment les uns des autres, signifient SR(21), -OR(22), -NR(23)R(24) ou -CR(25)R(26)R(27) ; R(21), R(22), R(23) et R(25) indépendamment les uns des autres, signifient -C_{b}H_{b}-(C₁-C₉)-hétéroaryle, qui est non substitué ou substitué par 1-3 substituants choisis dans le groupe constitué par F, Cl, CF₃, CH₃, méthoxy, hydroxy, amino, méthylamino et diméthylamino ;
b vaut zéro, 1 ou 2 ;
R(24), R(26) et R(27)
indépendamment les uns des autres, signifient hydrogène, (C₁-C₉)-alkyle ou (C₁-C₄)-perfluoroalkyle ; ou
R(2), R(3), R(4) et R(5)
indépendamment les uns des autres, signifient hydrogène, F, Cl, Br, I, CN, -(Xa)_{dg}C_{da}H_{2da+1}, (Xb)_{dh}-(CH₂)_{db}-C_{de}F_{2de+1}, (C₃-C₈)-alcényle ou -C_{df}H_{2df}R(30) ; (Xa) signifie O, S ou NR(33) ;
R(33) signifie H, (C₁-C₄)-alkyle ou (C₁-C₄)-perfluoroalkyle ;
dg vaut zéro ou 1 ;
(Xb) signifie O, S ou NR(34) ;
R(34)
signifie H, (C₁-C₄)-alkyle ou (C₁-C₄)-perfluoroalkyle ;
dh vaut zéro ou 1 ;
da vaut zéro, 1, 2, 3, 4, 5, 6, 7, 8 ;
db vaut zéro, 1, 2, 3 ou 4 ;
de vaut zéro, 1, 2, 3, 4, 5, 6, 7 ;
df vaut zéro, 1, 2, 3 ou 4 ;
R(30) signifie (C₃-C₈)-cycloalkyle, phényle, biphénylyle ou naphtyle, les aromatiques phényle, biphénylyle ou naphtyle étant non substitués ou substitués par 1-3 substituants choisis dans le groupe constitué par F, Cl, CF₃, méthyle, méthoxy et NR(31)R(32) ;
R(31) et R(32) signifient H, (C₁-C₄)-alkyle ou (C₁-C₄)-perfluoroalkyle ;
ou
R(2), R(3), R(4) et R(5)
indépendamment les uns des autres, signifient NR(40)R(41) ou -(Xe)-(CH₂)_{eb}R(45) ;
R(40) et R(41)
indépendamment l'un de l'autre, signifient hydrogène, (C₁-C₈)-alkyle, (C₁-C₈)-perfluoroalkyle ou (CH₂)ₑ-R(42) ;
e vaut zéro, 1, 2, 3 ou 4 ;
R(42)
signifie (C₃-C₇)-cycloalkyle, phényle, qui est non substitué ou substitué par 1-3 substituants choisis dans le groupe constitué par F, Cl, CF₃, méthyle, méthoxy et NR(43)R(44) ;
R(43) et R(44)
indépendamment l'un de l'autre, signifient H, CF₃ ou (C₁-C₄)-alkyle ;
ou
R(40) et R(41)
signifient ensemble 4 ou 5 groupements méthylène, dont un groupement CH₂ peut être remplacé par oxygène, soufre, NH, N-CH₃ ou N-benzyle ;
(Xe) signifie O, S ou NR(47) ;
R(47) signifie H, (C₁-C₄)-alkyle ou (C₁-C₄)-perfluoroalkyle ;
eb vaut zéro, 1, 2, 3 ou 4 ;
R(45) signifie (C₃-C₇)-cycloalkyle, phényle, qui est non substitué ou substitué par 1-3 substituants choisis dans le groupe constitué par F, Cl, CF₃, méthyle, méthoxy et NR(50)R(51) et -(Xfa)-(CH₂)_{ed}-(Xfb)R(46) ;
Xfa signifie CH₂, O, S ou NR(48) ;
Xfb signifie O, S ou NR(49) ;
ed vaut 1, 2, 3 ou 4 ;
R(46) signifie H, (C₁-C₄)-alkyle ou (C₁-C₄)-perfluoroalkyle ;
R(48), R(49), R(50) et R(51) indépendamment les uns des autres, signifient H ou (C₁-C₄)-alkyle ou (C₁-C₄)-perfluoroalkyle ;
R(3) et R(4) ne pouvant cependant pas représenter hydrogène,
ainsi que leurs sels pharmaceutiquement acceptables ;
ac) benzoylguanidines de formule I 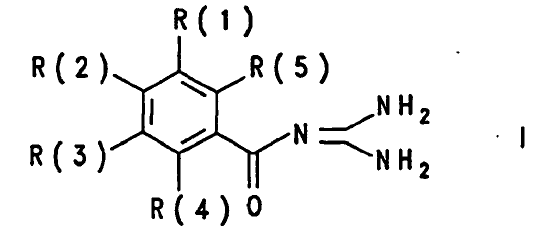 où :
un des trois substituants R(1), R(2) et R(3) :
signifie (C₁-C₉)-hétéroaryl-N-oxyde,
qui est lié via C ou N et qui est non substitué ou substitué par 1-3 substituants choisis dans le groupe constitué par F, Cl, CF₃, CH₃, méthoxy, hydroxy, amino, méthylamino et diméthylamino ;
ou
un des trois substituants R(1), R(2) et R(3) :
signifie -SR(10), -OR(10), -NR(10)R(11) ou -CR(10)R(11)R(12) ;
R(10)
signifie -CₐH₂ₐ-(C₁-C₉)-hétéroaryl-N-oxyde,
qui est non substitué ou substitué par 1-3 substituants choisis dans le groupe constitué par F, Cl, CF₃, CH₃, méthoxy, hydroxy, amino, méthylamino et diméthylamino ;
a vaut zéro, 1 ou 2 ;
R(11) et R(12)
indépendamment l'un de l'autre, sont définis comme R(10) ou signifient hydrogène ou (C₁-C₄)-alkyle ;
et à chaque fois les autres substituants R(1), R(2) et R(3) :
indépendamment les uns des autres, signifient (C₁-C₈)-alkyle, (C₂-C₈)-alcényle ou -CₘH₂ₘR(14) ;
m vaut zéro, 1 ou 2 ;
R(14) signifie (C₃-C₈)-cycloalkyle ou phényle,
qui est non substitué ou substitué par 1-3 substituants choisis dans le groupe constitué par F, Cl, CF₃, méthyle, méthoxy et NR(15)R(16),
R(15) et R(16)
signifient hydrogène ou CH₃ ;
ou
à chaque fois les autres substituants R(1), R(2) et R(3) :
indépendamment les uns des autres, signifient hydrogène, F, Cl, Br, I, -C=N, X-(CH₂)ₚ-(CqF_{2q+1}), R(22)-SOᵤ, R(23)R(24)N-CO, R(25)-CO- ou R(26)R(27)N-SO₂-,
le groupement perfluoroalkyle étant linéaire ou ramifié ;
X signifie une liaison, oxygène S ou NR(28) ;
u vaut zéro, 1 ou 2,
p vaut zéro, 1 ou 2,
q vaut zéro, 1, 2, 3, 4, 5 ou 6 ;
R(22), R(23), R(25) et R(26)
indépendamment les uns des autres, signifient (C₁-C₈)-alkyle, (C₂-C₆)-alcényle, -CₙH₂ₙ-R(29) ou CF₃ ;
n vaut zéro, 1, 2, 3 ou 4 ;
R(28) signifie hydrogène ou (C₁-C₃)-alkyle ;
R(29) signifie (C₃-C₇)-cycloalkyle ou phényle ;
qui est non substitué ou substitué par 1-3 substituants choisis dans le groupe constitué par F, Cl, CF₃, méthyle, méthoxy et NR(30)R(31) ;
R(30) et R(31)
signifient hydrogène ou C₁-C₄-alkyle ;
ou
R(23), R(25) et R(26)
signifient également hydrogène ;
R(24) et R(27)
indépendamment l'un de l'autre, signifient hydrogène ou (C₁-C₄)-alkyle ;
ou
R(23) et R(24) ainsi que R(26) et R(27) signifient ensemble 4 ou 5 groupements méthylène, dont un groupement CH₂ peut être remplacé par oxygène, S, NH, N-CH₃ ou N-benzyle ;
ou
à chaque fois les autres substituants R(1), R(2) et R(3) :
indépendamment les uns des autres, signifient OR(35) ou NR(35)R(36) ;
R(35) et R(36)
indépendamment l'un de l'autre, signifient, hydrogène ou (C₁-C₆)-alkyle ;
ou
R(35) et R(36)
signifient ensemble 4-7 groupements méthylène, dont un groupement CH₂ peut être remplacé par oxygène, S, NH, N-CH₃ ou N-benzyle ;
R(4) et R(5)
indépendamment l'un de l'autre, signifient, hydrogène, (C₁-C₄) -alkyle, F, Cl, -OR(32), -NR(33)R(34) ou CᵣF2ᵣ₊₁ ;
R(32), R(33) et R(34)
indépendamment les uns des autres, signifient hydrogène ou (C₁-C₃)-alkyle ;
r vaut 1, 2, 3 ou 4 ;
ainsi que leurs sels pharmaceutiquement acceptables ;
ad) benzoylguanidines de formule I 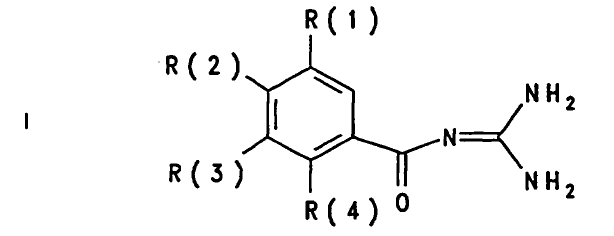 où :
R(1) signifie hydrogène, F, Cl, Br, I, CN, NO₂, OH, (C₁-C₈)-alkyle, (C₃-C₈)-cycloalkyle, Oₐ-(CH₂)_{b}-(CF₂)_{c}-CF3 ;
a vaut zéro ou 1 ;
b vaut zéro, 1 ou 2,
c vaut zéro, 1, 2 ou 3 ;
ou
R(1) signifie R(5)-SOₘ ou R(6)R(7)N-SO₂- ;
m vaut zéro, 1 ou 2,
R(5) et R(6) indépendamment l'un de l'autre, signifient :
(C₁-C₈)-alkyle, (C₃-C₆)-alcényle, CF₃ ou -CₙH₂ₙ-R(8) ;
n vaut zéro, 1, 2, 3 ou 4 ;
R(7) signifie hydrogène ou (C₁-C₄)-alkyle ;
R(8) signifie (C₃-C₇)-cycloalkyle ou phényle,
qui est non substitué ou substitué par 1-3 substituants choisis dans le groupe constitué par F, Cl, CF₃, méthyle, méthoxy et NR(9)R(10) ;
R(9) et R(10) indépendamment l'un de l'autre, signifient
hydrogène ou (C₁-C₄)-alkyle ;
ou
R(6) signifie H ;
ou R(6) et R(7)
ensemble, signifient 4 ou 5 groupements méthylène, dont un groupement CH₂ peut être remplacé par oxygène, S, NH, N-CH₃ ou N-benzyle ;
ou
R(1) signifie -SR(11), -OR(11) ou -CR(11)R(12)R(13) ; R(11) signifie -CₚH₂ₚ-(C₃-C₈)-cycloalkyle, -(C₁-C₉)-hétéroaryle ou phényle,
les systèmes aromatiques étant non substitués ou substitués par 1-3 substituants choisis dans le groupe constitué par F, Cl, CF₃, CH₃, méthoxy, hydroxy, amino, méthylamino et diméthylamino ;
R(12), R(13) indépendamment l'un de l'autre,
sont définis comme R(11) ou signifient hydrogène ou (C₁-C₄) -alkyle ;
p vaut zéro, 1 ou 2 ;
ou
R(1) signifie phényle, naphtyle, biphénylyle ou (C₁-C₉)-hétéroaryle, ce dernier étant lié via C ou N
qui sont non substitués ou substitués par 1-3 substituants choisis dans le groupe constitué par F, Cl, CF₃, CH₃, méthoxy, hydroxy, amino, méthylamino et diméthylamino ;
R(2) signifie -CF₂R(14), -CF[R(15)[R(16)], -CF[(CF₂)_{q}-CF₃)][R(15)], -C[(CF₂)ᵣ-CF₃]=CR(15)R(16) ;
R(14) signifie (C₁-C₄)-alkyle ou (C₃-C₆)-cycloalkyle ;
R(15) et R(16) indépendamment l'un de l'autre,
signifient hydrogène ou (C₁-C₄)-alkyle ;
q vaut zéro, 1 ou 2 ;
r vaut zéro, 1 ou 2 ;
R(3) est défini comme R(1) ;
R(4) signifie hydrogène, (C₁-C₃)-alkyle, F, Cl, Br, I, CN, - (CH₂)ₛ- (CF₂)ₜ-CF₃ ;
s vaut zéro ou 1 ;
t vaut zéro, 1 ou 2 ;
ainsi que leurs sels pharmaceutiquement acceptables ;
ae) benzoylguanidines de formule I 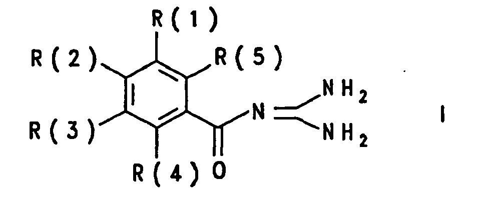 où :
un des trois substituants R(1), R(2) et R(3) :
signifie Y-4-[(CH₂)ₖ-CHR(7)-(C=O)R(8)]-phényle, -Y-3-[(CH₂)ₖ-CHR(7)-(C=O)R(8)]-phényle ou -Y-2-[(CH₂)ₖ-CHR(7)-(C=O)R(8)]-phényle,
phényle étant à chaque fois non substitué ou substitué par 1-2 substituants du groupe constitué par F, Cl, -CF₃, méthyle, hydroxy, méthoxy ou -NR(37)R(38) ;
R(37) et R(38)
indépendamment l'un de l'autre, signifient hydrogène ou -CH₃ ;
Y signifie une liaison, oxygène, S ou NR(9) ;
R(9) signifie hydrogène ou -(C₁-C₄)-alkyle ;
R(7) signifie -OR(10) ou -NR(10)R(11) ;
R(10) et R(11)
indépendamment l'un de l'autre, signifient hydrogène, -(C₁-C₈)-alkyle, -(C₁-C₈)-alcanoyle, -(C₁-C₈)-alcoxycarbonyle, benzyle, benzyloxycarbonyle ;
ou
R(10) signifie trityle ;
R(8) signifie -OR(12) ou -NR(12)R(13) ;
R(12) et R(13)
indépendamment l'un de l'autre, signifient, hydrogène, (C₁-C₈)-alkyle ou benzyle ;
k vaut zéro, 1, 2, 3 ou 4 ;
et à chaque fois les autres radicaux R(1), R(2) et R(3) :
indépendamment les uns des autres, signifient -(C₁-C₈)-alkyle, -(C₂-C₈)-alcényle ou -(CH₂)ₘR(14);
m vaut zéro, 1 ou 2,
R(14) signifie -(C₃-C₈)-cycloalkyle ou phényle,
qui est non substitué ou substitué par 1-3 substituants choisis dans le groupe constitué par F, Cl, -CF₃, méthyle, méthoxy et -NR(15)R(16),
R(15) et R(16)
signifient hydrogène ou -CH₃ ;
ou
à chaque fois les autres radicaux R(1), R(2) et R(3) :
indépendamment les uns des autres, signifient R(18)R(19)N-(C=Y')-NH-SO₂- ;
Y' signifie oxygène, -S- ou NR(20) ;
R(18) et R(19) indépendamment l'un de l'autre, signifient hydrogène, -(C₁-C₈)-alkyle, -(C₃-C₆)-alcényle ou -(CH₂)ₜ-R(21) ;
t vaut zéro, 1, 2, 3 ou 4 ;
R(21) signifie -(C₅-C₇)-cycloalkyle ou phényle, qui est non substitué ou substitué par 1-3 substituants choisis dans le groupe constitué par F, Cl, CF₃, méthoxy et (C₁-C₄)-alkyle ;
ou
R(18) et R(19)
ensemble, signifient 4 ou 5 groupements méthylène, dont un groupement CH₂ peut être remplacé par oxygène, -S-, -NH-, -N-CH₃ ou -N-benzyle ;
R(20) est défini comme R(18) ou signifie amidine ;
ou
à chaque fois les autres radicaux R(1), R(2) et R(3) :
indépendamment les uns des autres, signifient hydrogène, F, Cl, Br, I, -C=N, X-(CH₂)ₚ-(C_{q}F_{2q+1}), R(22)-SOᵤ-, R(23)R(24)N-CO-, R(25)-CO- ou R(26)R(27)N-SO₂-, le groupement perfluoroalkyle étant linéaire ou ramifié ;
X signifie une liaison, oxygène, -S- ou -NR(28) ;
u vaut zéro, 1 ou 2,
p vaut zéro, 1 ou 2,
q vaut 1, 2, 3, 4, 5 ou 6 ;
R(22), R(23), R(25) et R(26)
indépendamment les uns des autres, signifient - (C₁-C₈)-alkyle, - (C₃-C₆) -alcényle, - (CH₂)ₙ-R(29) ou -CF₃ ;
n vaut zéro, 1, 2, 3 ou 4 ;
R(28) signifie hydrogène ou -(C₁-C₃)-alkyle ;
R(29) signifie -(C₃-C₂)-cycloalkyle ou phényle,
qui est non substitué ou substitué par 1-3 substituants choisis dans le groupe constitué par F, Cl, -CF₃, méthyle, méthoxy et -NR(30)R(31),
R(30) et R(31)
signifient hydrogène ou -(C₁-C₄)-alkyle ;
ou
R(23), R(25) et R(26)
signifient hydrogène ;
R(24) et R(27)
indépendamment l'un de l'autre, signifient hydrogène ou (C₁-C₄)-alkyle ;
ou
R(23) et R(24) ainsi que R(26) et R(27)
ensemble, signifient 4 ou 5 groupements méthylène, dont un groupement CH₂ peut être remplacé par oxygène, -S-, -NH-, -N-CH₃ ou -N-benzyle ;
ou
à chaque fois les autres radicaux R(1), R(2) et R(3) :
indépendamment les uns des autres, signifient -OR(35) ou -NR(35)R(36) ;
R(35) et R(36)
indépendamment l'un de l'autre, signifient hydrogène ou -(C₁-C₆)-alkyle ;
ou
R(35) et R(36)
ensemble, signifient 4-7 groupements méthylène, dont un groupement CH₂ peut être remplacé par oxygène, -S-, -NH-, -N-CH₃ ou -N-benzyle :
R(4) et R(5).
indépendamment l'un de l'autre, signifient, hydrogène, - (C₁-C₄)-alkyle, F, Cl, -OR(32), -NR(33)R(34) ou CᵣF₂ᵣ₊₁ ;
R(32), R(33) et R(34)
indépendamment les uns des autres, signifient hydrogène ou - (C₁-C₃) -alkyle ;
r vaut 1, 2, 3 ou 4 ;
ainsi que leurs sels pharmaceutiquement acceptables ;
af) benzoylguanidines de formule I 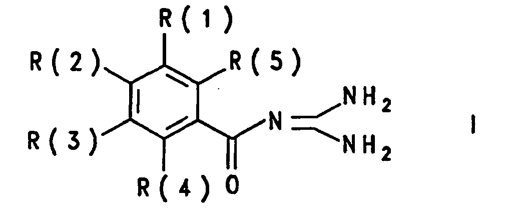 où :
R(1) signifie R(6)-CO ou R(7)R(8)N-CO ;
R(6) signifie (C₁-C₈) -alkyle, (C₁-C₈)-perfluoroalkyle, (C₃-C₈) -alcényle ou CₙH₂ₙ-R(9) ;
n vaut zéro, 1, 2, 3 ou 4 ;
R(9) signifie (C₃-C₈)-cycloalkyle, phényle, biphényle ou naphtyle, les aromatiques étant non substitués ou substitués par 1-3 substituants choisis dans le groupe constitué par F, Cl, CF₃, méthyle, méthoxy et NR(10)R(11) ;
R(10) et R(11)
signifient H, (C₁-C₄)-alkyle ou (C₁-C₄)-perfluoroalkyle ;
R(7) signifie H, (C₁-C₈)-alkyle, (C₁-C₈) - perfluoroalkyle, (C₃-C₈)-alcényle ou CₙH₂ₙ-R(12) ;
n vaut zéro, 1, 2, 3 ou 4 ;
R(12) signifie (C₃-C₈)-cycloalkyle, phényle, biphényle ou naphtyle, les aromatiques étant non substitués ou substitués par 1-3 substituants choisis dans le groupe constitué par F, Cl, CF₃, méthyle, méthoxy et NR(13)R(14) ;
R(13) et R(14) signifient H, (C₁-C₄)-alkyle ou (C₁-C₄) - perfluoroalkyle ;
R(8) signifie H, (C₁-C₄)-alkyle ou (C₁-C₄)-perfluoroalkyle ;
ou
R(7) et R(8)
ensemble, signifient 4 ou 5 groupements méthylène, dont un groupement CH₂ peut être remplacé par oxygène, S, NH, N-CH₃ ou N-benzyle ;
R(2) est défini comme R(1) ou signifie H, OH, F, Cl, Br, I, CN, NO₂, (C₁-C₈)-alkyle, (C₁-C₈)-perfluoroalkyle, (C₃-C₈)-alcényle ou CₙH₂ₙR(15) ;
n vaut zéro, 1, 2, 3 ou 4 ;
R(15) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(16)R(17) ;
R(16) et R(17)
signifient H, (C₁-C₉)-alkyle ou (C₁-C₄)-perfluoroalkyle ;
ou
R(2) signifie (C₁-C₉)-hétéroaryle,
qui est lié via C ou N et qui est non substitué ou substitué par 1-3 substituants choisis dans le groupe constitué par F, Cl, CF₃, CH₃, méthoxy, hydroxy, amino, méthylamino et diméthylamino ;
ou
R(2) signifie SR(18), -OR(18), -NR(18)R(19) ou -CR(18)R(19)R(20) ;
R(18) signifie -CₐH₂ₐ-(C₁-C₉)-hétéroaryle, qui est non substitué ou substitué par 1-3 substituants choisis dans le groupe constitué par F, Cl, CF₃, CH₃, méthoxy, hydroxy, amino, méthylamino, diméthylamino ;
a vaut zéro, 1 ou 2,
R(19) et R(20)
indépendamment l'un de l'autre, sont définis comme R(18) ou signifient hydrogène, (C₁-C₄) -alkyle ou (C₁-C₄)-perfluoroalkyle ;
ou
R(2) signifie R(21)-SOₘ ou R(22)R(23)N-SO₂-;
m vaut 1 ou 2 ;
R(21) signifie (C₁-C₈) -alkyle, (C₁-C₈) - perfluoroalkyle, (C₃-C₈)-alcényle ou CₙH₂ₙ-R(24) ;
n vaut zéro, 1, 2, 3 ou 4 ;
R(24) signifie (C₃-C₅)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(27)R(28) ;
R(27) et R(28)
signifient H, (C₁-C₄) -alkyle ou (C₁-C₄) - perfluoroalkyle ;
R(22) signifie H, (C₁-C₈)-alkyle, (C₁-C₈)-perfluoroalkyle, (C₃-C₈)-alcényle ou CₙH₂ₙ-R(29) ;
n vaut zéro, 1, 2, 3 ou 4 ;
R(29) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(30)R(31) ;
R(30) et R(31)
signifient H, (C₁-C₄-alkyle ou (C₁-C₄) - perfluoroalkyle ;
R(23) signifie hydrogène, (C₁-C₉)-alkyle ou (C₁-C₄)-perfluoroalkyle ;
ou
R(22) et R(23)
ensemble, signifient 4 ou 5 groupements méthylène, dont un groupement CH₂ peut être remplacé par oxygène, S, NH, N-CH₃ ou N-benzyle ;
ou
R(2) signifie R(33)X- ;
X signifie oxygène, S, NR(34), (D=O)A- ou NR(34)C=MN^{(*)}R(35)- ;
M signifie oxygène ou S :
A signifie oxygène ou NR(34) ;
D signifie C ou SO ;
R(33) signifie (C₁-C₈)-alkyle, (C₃-C₈)-alcényle, (CH₂)_{b}C_{d}F_{2d+1} ou CₙH₂ₙ-R(36) ;
b vaut zéro ou 1 ;
d vaut 1, 2, 3, 4, 5, 6 ou 7 ;
n vaut zéro, 1, 2, 3 ou 4 ;
R(36) signifie (C₃-C₈)-cycloalkyle, phényle, biphénylyle ou naphtyle,
les aromatiques étant non substitués ou substitués par 1-3 substituants choisis dans le groupe constitué par F, Cl, CF₃, méthyle, méthoxy et NR(37)R(38) ;
R(37) et R(38)
signifient H, (C₁-C₄)-alkyle ou (C₁-C₄)-perfluoroalkyle ;
R(34) signifie hydrogène, (C₁-C₄)-alkyle ou (C₁-C₄)-perfluoroalkyle ;
R(35) est défini comme R(33) ;
ou
R(33) et R(34)
ensemble, signifient 4 ou 5 groupements méthylène, dont un groupement CH₂ peut être remplacé par oxygène, S, NH, N-CH₃ ou N-benzyle ;
A et N^{(*)} étant liés au noyau phénylique du corps de base de la benzoylguanidine ; ou
R(2) signifie -SR(40), -OR(40), -NHR(40), -NR(40)R(41), -CHR(40)R(42), -CR(42)R(43)OH, -C≡CR(45), -CR(46)=CR(45) ou -[CR(47)R(48)]ᵤ-CO-[C(R49)R(50)]ᵥ-R(44) ;
R(40) et R(41)
indépendamment l'un de l'autre, signifient -(CH₂)ₚ-(CHOH)_{q}-(CH₂)ᵣ-(CHOH)ₜ-R(51) ou - (CH₂)ₚ-O- (CH₂-CH₂O)_{q}-R(51) ;
R(51) signifie hydrogène ou méthyle ;
u vaut 1, 2, 3 ou 4 ;
v vaut zéro, 1, 2, 3 ou 4 ;
p, q et r
indépendamment les uns des autres, valent zéro, 1, 2, 3 ou 4 ;
t vaut 1, 2, 3 ou 4 ;
R(42) et R(43)
indépendamment l'un de l'autre, signifient, hydrogène ou (C₁-C₆)-alkyle ;
ou
R(42) et R(43)
forment, ensemble avec l'atome de C qui les porte, (C₃-C₈)-cycloalkyle ;
R(44) signifie hydrogène, (C₁-C₆)-alkyle, (C₃-C₈)-cycloalkyle, -CₑH₂ₑ-R(45) ;
e vaut zéro, 1, 2, 3 ou 4 ;
R(45) signifie phényle,
qui est non substitué ou substitué par 1-3 substituants choisis dans le groupe constitué par F, Cl, CF₃, méthyle, méthoxy et NR(52)R(53) ;
R(52) et R(53)
signifient H ou (C₁-C₄)-alkyle ;
ou
R(45) signifie (C₁-C₉)-hétéroaryle,
qui est non substitué ou substitué comme phényle ;
ou
R(45) signifie (C₁-C₆)-alkyle,
qui est non substitué ou substitué par 1-3 OH,
R(46), R(47), R(48), R(49) et R(50)
indépendamment l'un de l'autre, signifient hydrogène ou méthyle ;
ou
R(2) signifie R(55)-NH-SO₂;
R(55) signifie R(56)R(57)N-(C=Y)- ;
Y signifie oxygène, S, ou N-R(58) ;
R(56) et R(57)
indépendamment l'un de l'autre, signifient hydrogène, (C₁-C₈)-alkyle, (C₃-C₆) -alcényle ou -C_{f}H_{2f}-R(59) ;
f vaut zéro, 1, 2, 3 ou 4 ;
R(59) signifie (C₅-C₇)-cycloalkyle, phényle,
qui est non substitué ou substitué par 1-3 substituants choisis dans le groupe constitué par F, Cl, CF₃, méthoxy et (C₁-C₄)-alkyle ;
ou
R(56) et R(57)
ensemble, signifient 4 ou 5 groupements méthylène, dont un groupement CH₂ peut être remplacé par oxygène, S, NH, N-CH₃ ou N-benzyle ;
R(58)
est défini comme R(56) ou signifie amidine ;
R(3), R(4) et R(5) indépendamment les uns des autres sont définis comme R(1) ou R(2), mais cependant, au moins un des substituants R(2), R(3), R(4) et R(5) doit représenter OH ;
ainsi que leurs sels pharmaceutiquement acceptables ;
ag) benzoylguanidines de formule I 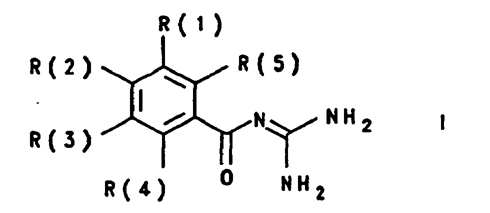 où :
un des trois substituants R(1), R(2) et R(3) :
signifie R(6)-A-B-D- ;
R(6) signifie un radical basique protonable, c'est-à-dire un groupement amino -NR(7)R(8), un groupement amidino R(7)R(8)N-C[=N-R(9)]- ou un groupement guanidino 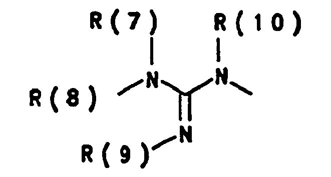
R(7), R(8), R(9) et R(10)
indépendamment les uns des autres, signifient hydrogène ou alkyle comprenant 1, 2, 3 ou 4 atomes de carbone ;
ou
R(7) et R(8)
ensemble, signifient CₐH₂ₐ ;
a vaut 4, 5, 6 ou 7 ;
dans le cas de a = 5, 6 ou 7, un groupement méthylène du groupement CₐH₂ₐ pouvant être remplacé par un groupement hétéroaromatique O, SOₘ ou NR(11),
ou
R(8) et R(9) ou R(9) et R(10) ou R(7) et R(10)
signifient un groupement CₐH₂ₐ ;
a vaut 2, 3, 4 ou 5 ;
dans le cas de a = 3, 4 ou 5, un groupement méthylène du groupement CₐH₂ₐ pouvant être remplacé par un groupement hétéroaromatique O, SOₘ ou NR(11),
m vaut zéro, 1 ou 2,
R(11) signifie hydrogène ou méthyle ;
ou
R(6) signifie un système cyclique basique hétéroaromatique comprenant 1-9 atomes de carbone ;
A signifie C_{b}H_{2b} ;
b vaut 1, 2, 3, 4, 5, 6, 7, 8, 9 ou 10 ;
dans le groupement C_{b}H_{2b}, un ou deux groupements méthylène pouvant être remplacés par un des groupements choisis dans le groupe constitué par -O-, -CO-, -CH[OR(20)]-, -SOₘ-, -NR(20)-, -NR(20)-CO-, -NR(20)-CO-NH-, -NR(20)-CO-NH-SO₂- 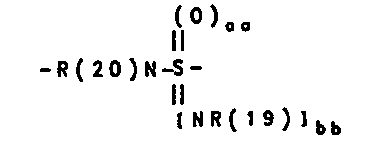 et -SOₐₐ[NR(19)]_{bb}- ; %
et dans le groupement C_{b}H_{2b}, un groupement méthylène pouvant être remplacé par -CH-R(99), R(99) formant, ensemble avec R(7) un cycle pyrrolidine ou pipéridine ;
aa vaut 1 ou 2 ;
bb vaut 0 ou 1 ;
aa + bb = 2 ;
R(19) signifie hydrogène ou alkyle comprenant 1, 2, 3 ou 4 atomes de carbone ;
R(20) signifie hydrogène ou méthyle ;
B signifie un radical phénylène ou naphtylène, 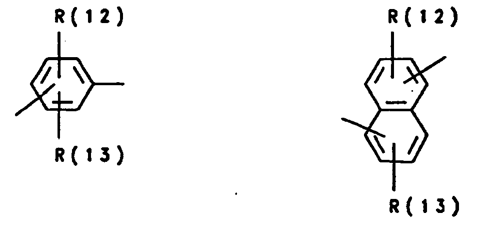
R(12) et R(13)
indépendamment l'un de l'autre, signifient hydrogène, méthyle, F, Cl, Br, I, -CF₃ ou -SO_{w}-R(14); R(14) signifie méthyle ou NR(15)R(16) ;
R(15) et R(16)
indépendamment l'un de l'autre, signifient hydrogène ou alkyle comprenant 1, 2, 3 ou 4 atomes de carbone ;
w vaut zéro, 1 ou 2,
D signifie -C_{d}H_{2d}-X_{f}- ;
d vaut zéro, 1, 2, 3 ou 4 ;
X signifie -O-, -CO-, -CH[OR(21)]-, -SOₘ- ou -NR(21)-;
f vaut zéro ou 1 ;
R(21) signifie hydrogène ou méthyle ;
m vaut zéro, 1 ou 2,
et à chaque fois les autres substituants R(1), R(2) et R(3):
indépendamment les uns des autres, signifient hydrogène, F, Cl, Br, I, -CN, -(C₁-C₈)-alkyle, -(C₂-C₈)-alcényle, -NR(35)R(36) ou R(17)-C_{g}H_{2g}-Zₕ- ;
g vaut zéro, 1, 2, 3 ou 4 ;
h vaut zéro ou 1 ;
R(35) et R(36)
indépendamment l'un de l'autre, signifient hydrogène ou alkyle comprenant 1, 2, 3, 4, 5 ou 6 atomes de carbone ;
ou
R(35) et R(36)
ensemble, signifient 4-7 groupements méthylène, dont un groupement CH₂ peut être remplacé par oxygène, -S-, -NH-, -NCH₃ ou -N-benzyle ; Z signifie -O-, -CO-, -SOᵥ-, -NR(18)-, -NR(18)-CO-, -NR(18)-CO-NH- ou -NR(18)-SO₂- ;
R(18) signifie hydrogène ou méthyle ;
v vaut zéro, 1 ou 2,
R(17) signifie hydrogène, cycloalkyle comprenant 3, 5 ou 6 atomes de carbone ou CₖF₂ₖ₊₁- ;
k vaut 1, 2 ou 3,
ou
R(17) signifie pyrrol-1-yle, pyrrol-2-yle ou pyrrol-3-yle,
qui est non substitué ou substitué par 1-4 substituants choisis dans le groupe constitué par F, Cl, Br, I, -CN, (C₂-C₈)-alcanoyle, (C₂-C₈)-alcoxycarbonyle, formyle, carboxy, -CF₃, méthyle et méthoxy ;
ou
R(17) signifie -(C₃-C₈)-cycloalkyle ou phényle,
qui est non substitué ou substitué par 1-3 substituants choisis dans le groupe constitué par F et Cl, -CF₃, méthyle, hydroxy, méthoxy, -NR(37)R(38), CH₃SO₂- et H₂NO₂S- ;
R(37) et R(38)
signifient hydrogène ou -CH₃ ;
R(4) et R(5)
indépendamment l'un de l'autre, signifient hydrogène, alkyle comprenant 1, 2, 3 ou 4 atomes de carbone, F, Cl, -OR(32), -NR(33)R(34) ou -CᵣF₂ᵣ₊₁ ; R(32), R(33) et R(34) indépendamment les uns des autres, signifient hydrogène ou alkyle comprenant 1, 2 ou 3 atomes de carbone ;
r vaut 1, 2, 3 ou 4 ;
ainsi que leurs sels pharmacologiquement acceptables ;
ah) Indenoylguanidines de formule I 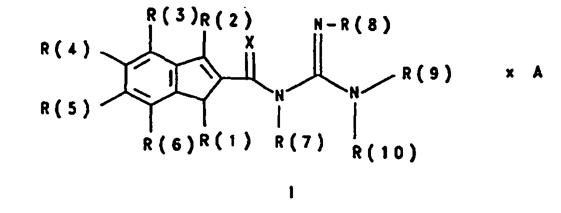 où :
R(1) et R(2)
indépendamment l'un de l'autre, signifient hydrogène, alkyle comprenant 1, 2, 3, 4, 5, 6, 7, 8, 9 ou 10 atomes de carbone, cycloalkyle comprenant 3, 4, 5 ou 6 atomes de carbone, O-alkyle comprenant 1, 2, 3 ou 4 atomes de carbone, O-C(=O)-alkyle comprenant 1, 2, 3 ou 4 atomes de carbone ou CₘH₂ₘ-NR(12)R(13) ;
R(12) et R(13)
indépendamment l'un de l'autre, signifient hydrogène ou alkyle comprenant 1, 2, 3 ou 4 atomes de carbone ;
m vaut zéro, 2, 3 ou 4 ;
NH-C((=O)-NH₂, C(=O)-O-alkyle comprenant 1, 2, 3 ou 4 atomes de carbone, C(=O)-NH2, C(=O)-NH-alkyle comprenant 1, 2, 3 ou 4 atomes de carbone, C(=O)-N(alkyle)₂ comprenant 1, 2, 3 ou 4 atomes de carbone dans chaque groupement alkyle, alcényle comprenant 2, 3, 4, 5, 6, 7, 8, 9 ou 10 atomes de carbone, alcynyle comprenant 2, 3, 4, 5, 6, 7, 8, 9 ou 10 atomes de carbone, alkylaryle comprenant 1, 2, 3 ou 4 atomes de carbone dans le groupement alkyle, alcénylaryle comprenant 2, 3, 4, 5, 6, 7, 8, 9 ou 10 atomes de carbone dans le groupement alcényle, alcynylaryle comprenant 2, 3, 4, 5, 6, 7, 8, 9 ou 10 atomes de carbone dans le groupement alcynyle, aryle substitué par C₁-C₄-alkyle, C₁-C₄-alkylhétéroaryle, C₁-C₄-alcénylhétéroaryle, aminoalkylaryle comprenant 1, 2, 3 ou 4 atomes de carbone dans le groupe alkyle, aryle substitué, hétéroaryle et hétéroaryle substitué ;
R(3), R(4), R(5) et R(6)
indépendamment les uns des autres, signifient hydrogène, alkyle comprenant 1, 2, 3, 4, 5, 6, 7, 8, 9 ou 10 atomes de carbone, O-alkyle comprenant 1, 2, 3, 4, 5, 6, 7, 8, 9 ou 10 atomes de carbone, halogène, (tel que F, Cl, Br, I), OH, aryle, aryle substitué, hétéroaryle, hétéroaryle substitué, 0-alkyle inférieur, O-aryle, O-(alkyle inférieur)aryle, aryle O-substitué, aryle O-substitué par alkyle inférieur, O-C(=O)-C₁-C₄-alkylaryle, O-C(=O)-NH-C₁-C₄-alkyle, O-C(=O)-N(C₁-C₄-alkyle)₂, NO₂, CN, CF₃, NH₂, NH-C(=O)-C₁-C₄-alkyle, NH-C(=O)-NH₂, COOH, C(=O)-O-C₁-C₄-alkyle, C(=O)-NH₂, C(=O)-NH-C₁-C₄-alkyle, C(=O)-N(C₁-C₄-alkyle)₂, C₁-C₄-COOH, C₁-C₄-alkyl-C (=O) -O-C₁-C₄-alkyle, SO₃H, SO₂-alkyle, SO₂-alkylaryle, SO₂-N-(alkyle)₂, SO₂-N(alkyl)(alkylaryle), C(=O)-R(11), C₁-C₁₀-alkyl-C (=O) -R(11), C₂-C₁₀-alcényl-C(=O)-R(11), C₂-C₁₀-alcynyl-C(=O)-R(11), NH-C(=O)-C₁-C₁₀-alkyl-C(=O)-R(11), O-C₁-C₁₁-alkyl-C(=O)-R(11) ;
R(11) signifie C₁-C₄-alkyle, C₁-C₄-alcynyle, aryle, aryle substitué, NH₂, NH-C₁-C₄-alkyle, N-(C₁-C₄-alkyle)₂, SO₃H, SO₂-alkyle, SO₂-alkylaryle, SO₂-N-(alkyle)₂, SO₂-N(alkyl)(alkylaryle) ;
X signifie O, S ou NH ;
R(7), R(8), R(9) et R(10)
indépendamment les uns des autres, signifient hydrogène, alkyle, cycloalkyle, aryle, alkylaryle ;
ou
R(8) et R(9)
ensemble, forment une partie d'un cycle hétérocyclique de 5, 6 ou 7 chaînons ;
A est absent ou représente un acide organique ou minéral non toxique,
ai) benzoyloxycarbonylguanidines de formule I 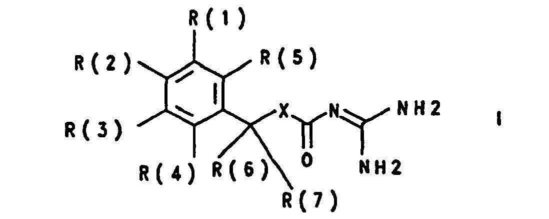 où :
R(1), R(2) et R(3)
indépendamment les uns des autres, signifient -Y-[4-R(8)-phényle], -Y-[3-R(8)-phényle] ou Y-[2-R(8)-phényle],
phényle étant à chaque fois non substitué ou substitué par 1-2 substituants du groupe constitué par F, Cl, -CF₃, méthyle, hydroxy, méthoxy et -NR(96)R(97) ;
R(96) et R(97)
indépendamment l'un de l'autre, signifient hydrogène ou -CH₃ ;
Y signifie une liaison, CH₂, oxygène, -S- ou -NR(9) ;
R(9) signifie hydrogène ou alkyle comprenant 1, 2, 3 ou 4 atomes de carbone ;
R(8) signifie SOₐ[NR(98)]_{b}NR(99)R(10) ;
a vaut 1 ou 2 ;
b vaut 0 ou 1 ;
a + b = 2 ;
R(98), R(99) et R(10)
indépendamment les uns des autres, signifient hydrogène, - (C₁-C₈)-alkyle, benzyle, -(C₂-C₈)-alkylène-NR(11)R(12), (C₂-C₈)-alkylène-NR(13)-(C₂-C₈)-alkylène-NR(37)R(38) ou (C₀-C₈)-alkylène-CR(39) R(40) -CR(41)R(42)(C₀-C₈)-alkylène-NR (43) R (44) ;
R(11), R(12), R(13), R(37), R(38), R(43) et R(44)
indépendamment les uns des autres, signifient, hydrogène, -(C₁-C₈)-alkyle ou benzyle ; R(39), R(40), R(41) et R(42)
indépendamment les uns des autres, signifient hydrogène, - (C₁-C₈) -alkyle ou - (C₀-C₃)-alkylène-phényle,
phényle étant non substitué ou substitué par 1-3 substituants choisis dans le groupe constitué par F, Cl, -CF₃, méthyle et méthoxy ;
ou
R(99) et R(10)
ensemble, signifient 4-6 groupements méthylène, dont un groupement CH₂ peut être remplacé par oxygène, -S-, -NH-, -N-CH₃ ou -N-benzyle ; ou
R(8) signifie SOₐ[NR(98)]_{b}NR(95)-C[=N-R(94)]-NR(93)R(92) ;
R(92), R(93), R(94) et R(95)
indépendamment les uns des autres, signifient hydrogène ou alkyle comprenant 1, 2, 3 ou 4 atomes de carbone ;
ou
R(1), R(2) et R(3)
indépendamment les uns des autres, signifient pyrrol-1-yle, pyrrol-2-yle ou pyrrol-3-yle,
qui est non substitué ou substitué par 1-4 substituants choisis dans le groupe constitué par F, Cl, Br, I, -CN, (C₂-C₈)-alcanoyle, (C₂-C₈) - alcoxycarbonyle, formyle, carboxy, -CF₃, méthyle, méthoxy ;
ou
R(1), R(2) et R(3) indépendamment les uns des autres, signifient hydrogène, - (C₁-C₈) -alkyle, (C₂-C₈) -alcényle ou -(CH₂)ₘR(14) ;
m vaut zéro, 1 ou 2,
R(14) signifie -(C₃-C₈)-cycloalkyle ou phényle,
qui est non substitué ou substitué par 1-3 substituants choisis dans le groupe constitué par F et Cl, -CF₃, méthyle, méthoxy et -NR(15)R(16),
R(15) et R(16)
signifient hydrogène ou -CH₃ ;
ou
R(1), R(2) et R(3)
indépendamment les uns des autres, signifient -Q-4-[(CH2)ₖ-CHR(17)-(C=O)R(20)]-phényle, -Q-3-(CH₂)ₖ-CHR(17)-(C=O)R(20)]-phényle ou -Q-2-[(CH₂)ₖ-CHR(17)-(C=O)R(20)]-phényle,
phényle étant à chaque fois non substitué ou substitué par 1-2 substituants du groupe constitué par F, Cl, -CF₃, méthyle, hydroxy, méthoxy et -NR(35)R(36) ;
R(35) et R(36)
indépendamment l'un de l'autre, signifient hydrogène ou -CH₃ ; S
Q signifie une liaison, oxygène, -S- ou -NR(18) ; R(18) signifie hydrogène ou - (C₁-C₄) -alkyle ;
R(17) signifie -OR(21) ou -NR(21)R(22) ;
R(21) et R(22)
indépendamment l'un de l'autre, signifient hydrogène, - (C₁-C₈) -alkyle, - (C₁-C₈) -alcanoyle, - (C₁-C₈) - alcoxycarbonyle, benzyle, benzyloxycarbonyle ;
ou
R(21) signifie trityle ;
R(20) signifie -OR(23) ou -NR(23)R(24) ;
R(23), R(24) indépendamment l'un de l'autre,
signifient, hydrogène, (C₁-C₈)-alkyle ou benzyle ;
k vaut zéro, 1, 2, 3 ou 4 ;
ou
R(1), R(2) et R(3)
indépendamment les uns des autres, signifient (C₁-C₉)-hétéroaryle,
qui est lié via C ou N et qui est non substitué ou substitué par 1-3 substituants du groupe constitué par F, Cl, CF₃, CH₃, méthoxy, hydroxy, amino, méthylamino et diméthylamino ;
ou
R(1), R(2) et R(3)
signifient -SR(25), -OR(25), -NR(25)R(26), -CR(25)R(26)R(27) ;
R(25) signifie -C_{f}H_{2f}-(C₁-C₉)-hétéroaryle,
qui est non substitué ou substitué par 1-3 substituants du groupe constitué par F, Cl, CF₃, CH₃, méthoxy, hydroxy, amino, méthylamino et diméthylamino,
f vaut zéro, 1 ou 2,
R(26) et R(27)
indépendamment l'un de l'autre, sont définis comme R(25) ou signifient hydrogène ou (C₁-C₄)-alkyle ;
ou
R(1), R(2) et R(3)
indépendamment les uns des autres, signifient (C₁-C₉)-hétéroaryl-N-oxyde,
qui est lié via C ou N et qui est non substitué ou substitué par 1-3 substituants choisis dans le groupe constitué par F, Cl, CF₃, CH₃, méthoxy, hydroxy, amino, méthylamino et diméthylamino ;
ou
R(1), R(2) et R(3)
indépendamment les uns des autres, signifient -SR(28), -OR(28), -NR(28)R(29) ou -CR(28)R(29)R(30) R(28) signifie -C_{g}H_{2g}-(C₁-C₉)-hétéroaryl-N-oxyde qui est non substitué ou substitué par 1-3 substituants choisis dans le groupe constitué par F, Cl, CF₃, CH₃, méthoxy, hydroxy, amino, méthylamino et diméthylamino ;
g vaut zéro, 1 ou 2,
R(29) et R(30)
indépendamment l'un de l'autre, sont définis comme R(28) ou signifient hydrogène ou (C₁-C₄)-alkyle ;
ou
R(1), R(2) et R(3)
indépendamment les uns des autres, signifient hydrogène, F, Cl, Br, I, -C=N, T-(CH₂)ₕ-(CᵢF₂ᵢ₊₁), R(31)-SO₁-, R(32)R(33)N-CO-, R(34)-CO- ou R (45) R(46) N-SO₂-, le groupement perfluoroalkyle étant linéaire ou ramifié ;
T signifie une liaison, oxygène, -S- ou -NR(47) ;
1 vaut zéro, 1 ou 2,
h vaut zéro, 1 ou 2,
i vaut 1, 2, 3, 4, 5 ou 6 ;
R(31), R(32), R(34) et R(45)
indépendamment les uns des autres, signifient - (C₁-C₈) -alkyle, - (C₃-C₆) alcényle, (CH₂)ₙR (48) ou -CF₃ ;
n vaut zéro, 1, 2, 3 ou 4 ;
R(47) signifie hydrogène ou alkyle comprenant 1, 2 ou 3 atomes de carbone ;
R(48) signifie -(C₃-C₇)-cycloalkyle ou phényle,
qui est non substitué ou substitué par 1-3 substituants choisis dans le groupe constitué par F, Cl, -CF₃, méthyle, méthoxy et -NR(49)R(50),
R(49) et R(50)
signifient hydrogène ou alkyle comprenant 1, 2, 3 ou 4 atomes de carbone ;
ou
R(32), R(34) et R(45)
signifient hydrogène ;
R(33) et R(46)
indépendamment l'un de l'autre, signifient hydrogène ou alkyle comprenant 1, 2, 3 ou 4 atomes de carbone ;
ou
R(32) et R(33) ainsi que R(45) et R(46)
ensemble, signifient 5 ou 6 groupements méthylène, dont un groupement CH₂ peut être remplacé par oxygène, -S-, -NH-, -NCH₃ ou -N-benzyle ; ou
R(1), R(2) et R(3)
indépendamment les uns des autres, signifient R(51)-A-G-D- ;
R(51) signifie un radical basique protonable, c'est-à-dire un groupement amino -NR(52)R(53), un groupement amidino R(52)R(53)N-C[=N-R(54)]- ou un groupement guanidino R(52)R(53)N-C[=N-R(54)]-NR(55)- ;
R(52), R(53), R(54) et R(55)
indépendamment les uns des autres, signifient hydrogène ou alkyle comprenant 1, 2, 3 ou 4 atomes de carbone ;
ou
R(52) et R(53)
signifient un groupement C_{α}H_{2α} ;
α vaut 4, 5, 6 ou 7 ;
dans le cas de α = 5, 6 ou 7, un atome de carbone du groupement C_{α}H_{2α} pouvant être remplacé par un groupement hétéroatomique, O, SO_{d} ou NR(56),
ou
R(53) et R(54) ou R(54) et R(55) ou R(52) et R(55) signifient un groupement C_{y}H_{2y} ;
y vaut 2, 3, 4 ou 5 ;
dans le cas de y = 3, 4 ou 5, un atome de carbone du groupement C_{y}H_{2y} pouvant être remplacé par un groupement hétéroatomique, 0, SO_{d} ou NR(56),
d vaut zéro, 1 ou 2,
R(56) signifie hydrogène ou méthyle ;
ou
R(51) signifie un système cyclique basique hétéroaromatique comprenant 1-9 atomes de carbone ;
A signifie un groupement CₑH₂ₑ ;
e vaut zéro, 1, 2, 3, 4, 5, 6, 7, 8, 9 ou 10 ; dans le groupement CₑH₂ₑ, un atome de carbone pouvant être remplacé par un des groupements -O-, -CO-, -CH[OR(57)]-, -SOᵣ-, -NR(57)-, -NR(57)-CO-, -NR(57)-CO-NH-, -NR(57)-CO-NH-SO₂- ou -NR(57)-SO₂- ;
r vaut zéro, 1 ou 2,
G signifie un radical phénylène 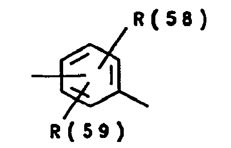
R(58) et R(59)
indépendamment l'un de l'autre, signifient hydrogène, méthyle, méthoxy, F, Cl, Br, I, CF₃ ou -SOₛ-R(60) ;
R(60) signifie méthyle ou NR(61)R(62);
R(61) et R(62)
indépendamment l'un de l'autre, signifient hydrogène ou alkyle comprenant 1, 2, 3 ou 4 atomes de carbone ;
D signifie -CᵥH₂ᵥ-E_{w}- ;
v vaut zéro, 1, 2, 3 ou 4 ;
E signifie -O-, -CO-, -CH[OR(63)]-, -SOₐₐ- ou -NR(63)- ;
w vaut zéro ou 1 ;
aa vaut zéro, 1 ou 2,
R(63) signifie hydrogène ou méthyle,
ou
R(1), R(2) et R(3) indépendamment les uns des autres, signifient -CF₂R(64), -CF[R(65)] [R(66)], -CF[(CF₂)_{q}-CF₃)][R(65)], -CR(CF₂)ₚ-CF₃]=CR(65)R(66) ;
R(64) signifie alkyle comprenant 1, 2, 3 ou 4 atomes de carbone ou cycloalkyle comprenant 3, 4, 5, 6 ou 7 atomes de carbone ;
R(65) et R(66) indépendamment l'un de l'autre, signifient hydrogène ou alkyle comprenant 1, 2, 3 ou 4 atomes de carbone ;
q vaut zéro, 1 ou 2,
p vaut zéro, 1 ou 2,
ou
R(1), R(2) et R(3)
indépendamment les uns des autres, signifient -OR(67) ou -NR(67)R(68) ;
R(67) et R(68)
indépendamment l'un de l'autre, signifient hydrogène ou alkyle comprenant 1, 2, 3, 4, 5 ou 6 atomes de carbone ;
ou
R(67) et R(68)
ensemble, signifient 4, 5, 6 ou 7 groupements méthylène, dont un groupement CH₂ peut être remplacé par oxygène, -S-, SO₂, -NH-, -NCH₃ ou -N-benzyle ;
R(4) et R(5)
indépendamment l'un de l'autre, signifient hydrogène, alkyle comprenant 1, 2, 3 ou 4 atomes de carbone, F, Cl, -OR(69), -NR(70)R(71) ou -C_{z}F_{2z+1} ; R(69), R(70) et R(71)
indépendamment les uns des autres, signifient hydrogène ou alkyle comprenant 1, 2 ou 3 atomes de carbone ;
z vaut 1, 2, 3 ou 4 ;
R(6) et R(7)
indépendamment l'un de l'autre, signifient hydrogène ou alkyle comprenant 1, 2, 3 ou 4 atomes de carbone ;
X signifie oxygène ou NR(72) ;
R(72) signifie hydrogène ou alkyle comprenant 1, 2, 3 ou 4 atomes de carbone ;
ainsi que leurs sels pharmaceutiquement acceptables ;
ak) guanidides d'acides alcénylcarboxyliques portant des groupements fluorophényle de formule I 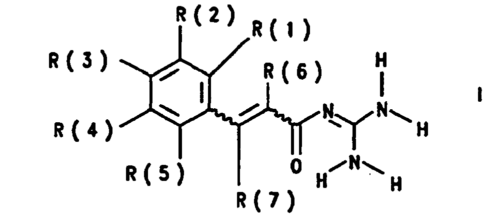 où :
R(6) signifie hydrogène, (C₁-C₈)-alkyle, (C₃-C₈)-cycloalkyle ou phényle ;
le groupement phényle étant non substitué ou substitué par 1-3 substituants choisis dans le groupe constitué par F, Cl, CF₃, méthyle, méthoxy et NR(9)R(10)
R(9) et R(10)
signifient hydrogène, (C₁-C₄)-alkyle ou (C₁-C₄)-perfluoroalkyle ;
R(7) indépendamment, est défini comme R(6) ;
R(1), R(2), R(3), R(4) et R(5)
indépendamment les uns des autres, signifient hydrogène ou F ;
au moins un des radicaux R(1), R(2), R(3), R(4) et R(5) devant cependant signifier fluor ;
ainsi que leurs sels pharmaceutiquement acceptables ;
al) benzoylguanidines de formule I 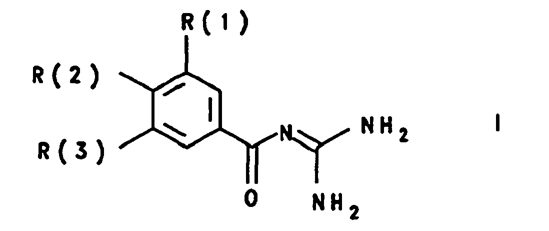 où :
R(1) signifie R(4)-SOₘ ou R(5)R(6)N-SO₂- ;
m vaut 1 ou 2 ;
R(4) et R(5)
indépendamment l'un de l'autre, signifient alkyle comprenant 1, 2, 3, 4, 5, 6, 7 ou 8 atomes de carbone, alcényle comprenant 3, 4, 5 ou 6 atomes de carbone, CF₃ ou -CₙH₂ₙ-R(7) ;
n vaut zéro, 1, 2, 3 ou 4 ;
R(6) signifie H ou alkyle comprenant 1, 2, 3 ou 4 atomes de carbone ;
R(7) signifie cycloalkyle comprenant 3, 4, 5, 6, 7 ou 8 atomes de carbone ou phényle
qui est non substitué ou substitué par 1-3 substituants choisis dans le groupe constitué par F, Cl, CF₃, méthyle, méthoxy et NR(8)R(9) ;
R(8) et R(9)
signifient H ou alkyle comprenant 1, 2, 3 ou 4 atomes de carbone ;
ou
R(5) signifie également hydrogène ;
ou
R(5) et R(6)
ensemble, signifient 4 ou 5 groupements méthylène, dont un groupement CH₂ peut être remplacé par oxygène, S, NH, N-CH₃ ou N-benzyle ;
ou
R(1) signifie -Oₚ-(CH₂)_{q}-(CF₂)ᵣ-CF3 ;
p vaut zéro ou 1 ;
q vaut zéro, 1 ou 2,
r vaut zéro, 1, 2 ou 3 ;
ou
R(1) signifie -SR(10), -OR(10) ou -CR(10)R(11)R(12) ; R(10), R(11) et R(12)
indépendamment les uns des autres, signifient hydrogène, alkyle comprenant 1, 2, 3, 4, 5, 6, 7 ou 8 atomes de carbone, -CₛH₂ₛ-(C₃-C₈)-cycloalkyle ou un système aromatique choisi dans le groupe constitué par pyridyle, pyrrolyle, quinoléyle, isoquinoléyle, imidazolyle ou phényle ;
s vaut zéro, 1 ou 2,
les systèmes aromatiques pyridyle, pyrrolyle, quinoléyle, isoquinoléyle, imidazolyle et phényle étant non substitués ou substitués par 1-3 substituants choisis dans le groupe constitué par F, Cl, CF₃, CH₃, méthoxy, hydroxy, amino, méthylamino et diméthylamino ; R(2) signifie -(CH₂)ᵤ-(CF₂)ₜ-CF₃ ;
t vaut zéro, 1, 2 ou 3 ;
u vaut zéro ou 1 ;
R(3) signifie hydrogène ou, indépendamment, est défini comme R(1) ;
ainsi que leurs sels pharmaceutiquement acceptables ;
am) guanidides substitués de l'acide cinnamique de formule I 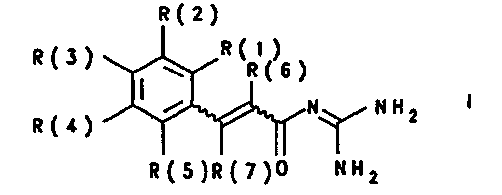 où :
au moins un des substituants R(1), R(2), R(3) R(4) et R(5) signifie :
-Xₐ-Y_{b}-Lₙ-U ;
X signifie CR(16)R(17), O, S ou NR(18) ;
R(16), R(17) et R(18)
indépendamment les uns des autres, signifient H, alkyle comprenant 1, 2, 3 ou 4 atomes de carbone ou perfluoroalkyle comprenant 1, 2, 3 ou 4 atomes de carbone ;
a vaut zéro ou 1 ;
Y alkylène comprenant 1, 2, 3, 4, 5, 6, 7 ou 8 atomes de carbone, alkylène-T comprenant 1, 2, 3, 4, 5, 6, 7 ou 8 atomes de carbone dans le groupement alkylène, T, T-alkylène comprenant 1, 2, 3, 4, 5, 6, 7 ou 8 atomes de carbone dans le groupement alkylène ;
T signifie NR(20), O, S ou phénylène,
le groupement phénylène étant non substitué ou substitué par 1-3 substituants choisis dans le groupe constitué par F, Cl, CF₃, méthyle, méthoxy et NR(21)R(22) ;
R(20), R(21) et R(22)
indépendamment les uns des autres, signifient H, alkyle comprenant 1, 2, 3 ou 4 atomes de carbone ou perfluoroalkyle comprenant 1, 2, 3 ou 4 atomes de carbone ;
b vaut zéro ou 1 ;
L signifie O, S, NR(23) ou CₖH₂ₖ ;
k vaut 1, 2, 3, 4, 5, 6, 7, 8 ;
n vaut zéro ou 1 ;
U signifie NR(24)R(25) ou un hétérocycle contenant N, comprenant 1, 2, 3, 4, 5, 6, 7, 8 ou 9 atomes de carbone ;
R(24) et R(25)
indépendamment l'un de l'autre, signifient H, alkyle comprenant 1, 2, 3, 4, 5, 6, 7 ou 8 atomes de carbone, perfluoroalkyle comprenant 1, 2, 3, 4, 5, 6, 7 ou 8 atomes de carbone ;
ou
R(24) et R(25) ensemble, signifient 4 ou 5 groupements méthylène, dont un groupement CH₂ peut être remplacé par oxygène, S, NH, N-CH₃ ou N-benzyle ;
les hétérocycles contenant N étant pontés par N ou C et non substitués ou substitués par 1-3 substituants choisis dans le groupe constitué par F, Cl, CF₃, méthyle, méthoxy et NR(27)R(28) ;
R(23), R(27) et R(28)
indépendamment les uns des autres, signifient H, alkyle comprenant 1, 2, 3 ou 4 atomes de carbone ou perfluoroalkyle comprenant 1, 2, 3 ou 4 atomes de carbone ;
et à chaque fois les autres substituants R(1), R(2), R(3), R(4) et R(5)
indépendamment les uns des autres, signifient H, F, Cl, Br, I, CN, -Oₙ-CₘH₂ₘ₊₁, -Oₚ-(CH₂)ₛ-C_{q}F_{2q+1} ou -CᵣH₂ᵣR(10) ;
n vaut zéro ou 1 ;
m vaut zéro, 1, 2, 3, 4, 5, 6, 7 ou 8 ;
p vaut zéro ou 1 ;
q vaut 1, 2, 3, 4, 5, 6, 7 ou 8 ;
s vaut zéro, 1, 2, 3 ou 4 ;
r vaut zéro, 1, 2, 3 ou 4 ;
R(10)
signifie cycloalkyle comprenant 3, 4, 5, 6, 7 ou 8 atomes de carbone ou phényle
le groupement phényle étant non substitué ou substitué par 1-3 substituants choisis dans le groupe constitué par F, Cl, CF₃, méthyle, méthoxy et NR(11)R(12) ;
R(11) et R(12)
indépendamment l'un de l'autre, signifient H, alkyle comprenant 1, 2, 3 ou 4 atomes de carbone ou perfluoroalkyle comprenant 1, 2, 3 ou 4 atomes de carbone ;
R(6) et R(7)
indépendamment l'un de l'autre, signifient hydrogène, F, Cl, Br, I, CN, alkyle comprenant 1, 2, 3, 4, 5, 6, 7 ou 8 atomes de carbone, perfluoroalkyle comprenant 1, 2, 3, 4, 5, 6, 7 ou 8 atomes de carbone, cycloalkyle comprenant 3, 4, 5, 6, 7 ou 8 atomes de carbone, ou phényle,
qui est non substitué ou substitué par 1-3 substituants choisis dans le groupe constitué par F, Cl, CF₃, méthyle, méthoxy et NR(14)R(15) ;
R(14) et R(15)
indépendamment l'un de l'autre, signifient H, alkyle comprenant 1, 2, 3 ou 4 atomes de carbone ou perfluoroalkyle comprenant 1, 2, 3 ou 4 atomes de carbone ;
ainsi que leurs sels pharmaceutiquement acceptables ;
an) benzoylguanidines de formule I 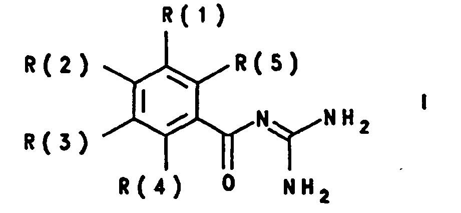 où :
au moins un des substituants R(1), R(2) et R(3) signifie R(6)-C(OH)₂- ;
R(6) signifie perfluoroalkyle comprenant 1, 2 ou 3 atomes de carbone, qui est linéaire ou ramifié ;
et les autres substituants R(1), R(2) et R(3)
indépendamment les uns des autres, signifient hydrogène, OH, F, Cl, Br, I, alkyle comprenant 1, 2, 3, 4, 5 ou 6 atomes de carbone, cycloalkyle comprenant 3, 4, 5 ou 6 atomes de carbone, alcoxy comprenant 1, 2, 3 ou 4 atomes de carbone ou phénoxy,
qui est non substitué ou substitué par 1-3 substituants choisis dans le groupe constitué par F, Cl, méthyle, et méthoxy ;
ou
les autres substituants R(1), R(2) et R(3)
indépendamment les uns des autres, signifient alkyl-SOₓ, -CR(7)=CR(8)R(9) ou -C≡CR(9) ;
x vaut zéro, 1 ou 2,
R(7) signifie hydrogène ou méthyle;
R(8) et R(9)
indépendamment l'un de l'autre, signifient hydrogène, alkyle comprenant 1, 2, 3 ou 4 atomes de carbone, cycloalkyle comprenant 3, 4, 5, 6, 7 ou 8 atomes de carbone ou phényle,
qui est non substitué ou substitué par 1-3 substituants choisis dans le groupe constitué par F, Cl, CF₃, méthyle, et méthoxy ;
ou
les autres substituants R(1), R(2) et R(3)
indépendamment les uns des autres, signifient phényle, C₆H₅- (C₁-C₄) -alkyle, naphtyle, biphénylyle, quinoléinyle, isoquinoléinyle ou imidazolyle,
quinoléinyle, isoquinoléinyle ou imidazolyle étant liés via C ou N, et phényle, C₆H₅- (C₁-C₄) -alkyle, naphtyle, biphénylyle, quinoléinyle, isoquinoléinyle et imidazolyle étant non substitués ou substitués par 1-3 substituants choisis dans le groupe constitué par F, Cl, CF₃, CH₃, méthoxy, hydroxy, amino, méthylamino et diméthylamino ;
ou
les autres substituants R(1), R(2) et R(3)
indépendamment les uns des autres, signifient -SR(10), -OR(10), -CR(10)R(11)R(12) ;
R(10)
signifie CFH₂F- (C₃-C₈) -cycloalkyle, quinoléinyle, isoquinoléinyle, pyridinyle, imidazolyle ou phényle,
les systèmes aromatiques quinoléyle, isoquinoléyle, pyridinyle, imidazolyle et phényle étant non substitués ou substitués par 1-3 substituants choisis dans le groupe constitué par F, Cl, CF₃, CH₃, méthoxy, hydroxy, amino, méthylamino et diméthylamino ; f vaut zéro, 1 ou 2,
R(11) et R(12)
indépendamment l'un de l'autre, sont définis comme R(10) ou signifient hydrogène ou alkyle comprenant 1, 2, 3 ou 4 atomes de carbone ;
R(4) et R(5)
indépendamment l'un de l'autre, signifient hydrogène, alkyle comprenant 1, 2 ou 3 atomes de carbone, F, Cl, Br, I, CN, OR(13), NR(14)R(15) ou -(CH₂)ₙ-(CF₂)ₒ-CF₃ ;
R(13), R(14) et R(15)
indépendamment l'un de l'autre, signifient hydrogène ou alkyle comprenant 1, 2, 3 ou 4 atomes de carbone ;
n vaut zéro ou 1 ;
o vaut zéro, 1 ou 2,
ainsi que leurs sels pharmacologiquement acceptables.
ao) sulfonimidamides de formule I 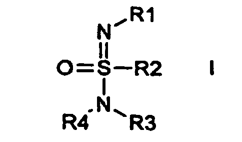 où :
au moins un des trois substituants R(1), R(2) et R(3) signifie une benzoylguanidine, 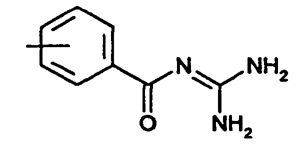 qui est non substituée ou substituée dans la partie phényle par 1-4 radicaux choisis dans le groupe constitué par alkyle comprenant 1, 2, 3, 4, 5, 6, 7 ou 8 atomes de carbone, alcényle comprenant 2, 3, 4, 5, 6, 7 ou 8 atomes de carbone, -(CH₂)ₘ-R(14), F, Cl, Br, I, -C≡N, CF₃, R(22)SO₂-, R(23)R(24)N-CO-, R(25)-CO-, R(26)R(27)N-SO_{z}, -OR(35), -SR(35) ou -NR(35)R(36) ; m vaut zéro, 1 ou 2,
R(14)
signifie -(C₃-C₈)-cycloalkyle ou phényle,
qui est non substitué ou substitué par 1-3 substituants choisis dans le groupe constitué par F et Cl, -CF₃, méthyle, méthoxy et -NR(15)R(16),
R(15) et R(16)
indépendamment l'un de l'autre, signifient hydrogène ou -CH₃ ;
R(22), R(23), R(25) et R(26)
indépendamment les uns des autres, signifient alkyle comprenant 1, 2, 3, 4, 5, 6, 7 ou 8 atomes de carbone, alcényle comprenant 2, 3, 4, 5, 6, 7 ou 8 atomes de carbone, (CH₂)ₙR(29) ou -CF₃ ;
n vaut zéro, 1, 2, 3 ou 4 ;
R(29) signifie -(C₃-C₇)-cycloalkyle ou phényle,
qui est non substitué ou substitué par 1-3 substituants choisis dans le groupe constitué par F, Cl, -CF₃, méthyle, méthoxy et -NR(30)R(31),
R(30) et R(31)
signifient hydrogène ou alkyle comprenant 1, 2, 3 ou 4 atomes de carbone ;
ou
R(23), R(25) et R(26)
signifient hydrogène ;
R(24) et R(27)
indépendamment l'un de l'autre, signifient hydrogène ou alkyle comprenant 1, 2, 3 ou 4 atomes de carbone ;
ou
R(23) et R(24) ainsi que R(26) et R(27)
ensemble, signifient 5 ou 6 groupements méthylène, dont un groupement CH₂ peut être remplacé par oxygène, -S-, -NH-, -NCH₃ ou -N-benzyle ;
R(35) et R(36)
indépendamment l'un de l'autre, signifient hydrogène ou alkyle comprenant 1, 2, 3, 4, 5 ou 6 atomes de carbone ;
ou
R(35) et R(36)
ensemble, signifient 4-7 groupements méthylène, dont un groupement CH₂ peut être remplacé par oxygène, -S-, -NH-, -NCH₃ ou -N-benzyle ; ou
R(35)
signifie phényle,
qui est non substitué ou substitué par 1-3 substituants choisis dans le groupe constitué par F, Cl, -CF₃, méthyle, méthoxy, SO₂R(5), SO₂NR(6)R(7) et -NR(32)R(33) ;
R(5) signifie alkyle comprenant 1, 2, 3, 4, 5 ou 6 atomes de carbone ;
R(6) et R(7)
indépendamment l'un de l'autre, signifient hydrogène ou alkyle comprenant 1, 2, 3 ou 4 atomes de carbone ;
R(32) et R(33) indépendamment l'un de l'autre, signifient hydrogène ou alkyle comprenant 1, 2, 3 ou 4 atomes de carbone ;
ou
R(35) signifie C₁-C₉-hétéroaryle,
qui est non substitué ou substitué par 1-3 substituants choisis dans le groupe constitué par F, Cl, CF₃, CH₃, méthoxy, hydroxy, amino, méthylamino et diméthylamino ;
et à chaque fois les autres substituants R(1), R(2) et R(3) :
indépendamment les uns des autres, signifient alkyle comprenant 1, 2, 3, 4, 5, 6, 7 ou 8 atomes de carbone, (CH₂)ₚR(10)
p vaut zéro, 1, 2, 3 ou 4 ;
R(10) signifie phényle,
qui est non substitué ou substitué par 1-3 substituants choisis dans le groupe constitué par F, Cl, -CF₃, méthyle, méthoxy, -SO₂NR(17)R(8) et -SO₂R(9) ; R(17) et R(8)
indépendamment l'un de l'autre, signifient hydrogène ou alkyle comprenant 1, 2, 3 ou 4 atomes de carbone ;
R(9) signifie alkyle comprenant 1, 2, 3 ou 4 atomes de carbone ;
ou à chaque fois l'autre substituant R(1) et R(3) signifie hydrogène,
R(4) signifie hydrogène ou alkyle comprenant 1, 2, 3 ou 4 atomes de carbone ;
ainsi que leurs sels pharmaceutiquement acceptables ;
ap) benzoylguanidines de formule I 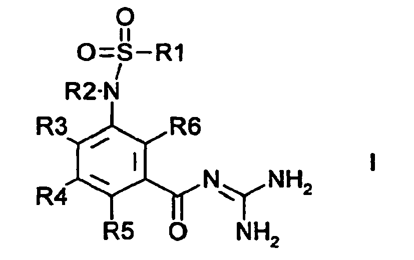 où :
R(1) signifie alkyle comprenant 1, 2, 3, 4, 5, 6, 7 ou 8 atomes de carbone ou NR(7)R(8) ;
R(7) et R(8)
indépendamment l'un de l'autre, signifient hydrogène ou alkyle comprenant 1, 2, 3, 4, 5, 6, 7 ou 8 atomes de carbone ;
R(2) signifie hydrogène, alkyle comprenant 1, 2, 3, 4, 5, 6, 7 ou 8 atomes de carbone ou -SO₂R(9) ;
R(9) indépendamment, est défini comme R(1) ;
R(3) signifie hydrogène, -SR(25), -OR(25), -NR(25)R(26) ou -CR(25)R(26)R(27) ;
R(25) signifie hydrogène, alkyle comprenant 1, 2, 3, 4, 5, 6, 7 ou 8 atomes de carbone ou phényle, qui est non substitué ou substitué par 1-3 substituants choisis dans le groupe constitué par F, Cl, CF₃, CH₃, méthoxy, hydroxy, amino, méthylamino et diméthylamino ; ou
R(25)
signifie -(C₁-C₉)-hétéroaryle,
qui est non substitué ou substitué par 1-3 substituants choisis dans le groupe constitué par les groupements F, Cl, CF₃, CH₃, méthoxy, hydroxy, amino, méthylamino et diméthylamino ;
R(26) et R(27)
indépendamment l'un de l'autre, sont définis comme R(25) ou signifient hydrogène ou alkyle comprenant 1, 2, 3, 4, 5, 6, 7 ou 8 atomes de carbone ;
R(4) signifie hydrogène, F, Cl, Br, I, OH, -C≡N, CF₃, alkyle comprenant 1, 2, 3, 4, 5, 6, 7 ou 8 atomes de carbone, alcényle comprenant 2, 3, 4, 5, 6, 7 ou 8 atomes de carbone ou -(CH₂)ₘR(14) ;
m vaut zéro, 1 ou 2,
R(14) signifie -(C₃-C₈)-cycloalkyle ou phényle,
qui est non substitué ou substitué par 1-3 substituants choisis dans le groupe constitué par F et Cl, -CF₃, méthyle, méthoxy et -NR(15)R(16),
R(15) et R(16)
indépendamment l'un de l'autre, signifient hydrogène ou -CH₃ ;
R(5) et R(6)
indépendamment l'un de l'autre, signifient hydrogène, alkyle comprenant 1, 2, 3 ou 4 atomes de carbone, F, Cl, -OR(32), -NR(33)R(34) ou -CF₃ ;
R(32), R(33) et R(34)
indépendamment les uns des autres, signifient hydrogène ou alkyle comprenant 1, 2, 3 ou 4 atomes de carbone ;
ainsi que leurs sels pharmaceutiquement acceptables ;
aq) diguanidides d'acides benzènedicarboxyliques de formule I 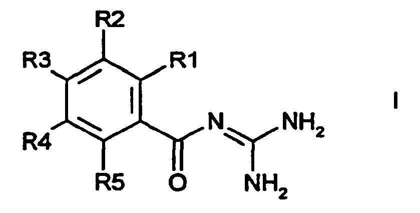 où :
un des radicaux R(1), R(2), R(3) et R(4)
signifie -CO-N=C(NH₂)₂ ;
et à chaque fois les autres radicaux R(1), R(2), R(3) et R(4) .
R(1) signifie hydrogène, alkyle comprenant 1, 2, 3 ou 4 atomes de carbone, F, Cl, Br, I, -OR(32), -NR(33)R(34) ou CF₃ ;
R(32), R(33) et R(34)
indépendamment les uns des autres, signifient hydrogène ou alkyle comprenant 1, 2, 3 ou 4 atomes de carbone ;
R(2) et R(4)
indépendamment l'un de l'autre, signifient hydrogène, F, Cl, Br, I, OH, -CN, CF₃, -CO-N=C(NH₂)₂, alkyle comprenant 1, 2, 3, 4, 5, 6, 7 ou 8 atomes de carbone, alcényle comprenant 2, 3, 4, 5, 6, 7 ou 8 atomes de carbone ou -(CH₂)ₘR(14) ;
m vaut zéro, 1 ou 2,
R(14) signifie -(C₃-C₈)-cycloalkyle ou phényle,
qui est non substitué ou substitué par 1-3 substituants choisis dans le groupe constitué par F et Cl, -CF₃, méthyle, méthoxy et NR(15)R(16),
R(15) et R(16)
signifient hydrogène ou -CH₃ ;
ou
R(2) et R(4)
indépendamment l'un de l'autre, signifient pyrrol-1-yle, pyrrol-2-yle ou pyrrol-3-yle,
qui est non substitué ou substitué par 1-4 substituants choisis dans le groupe constitué par F, Cl, Br, I, -CN, (C₂-C₈)-alcanoyle, (C₂-C₈)-alcoxycarbonyle, formyle, carboxy, -CF₃, méthyle, méthoxy ;
ou
R(2) et R(4)
indépendamment l'un de l'autre, signifient R(22)-SO₂-, R(23)R(24)N-CO-, R(28)-CO- ou R(29)R(30)N-SO₂ ;
R(22) et R(28)
indépendamment l'un de l'autre, signifient méthyle ou -CF₃ ;
R(23), R(24), R(29) et R(30)
indépendamment les uns des autres, signifient hydrogène ou méthyle ;
ou
R(2) et R(4)
indépendamment l'un de l'autre, signifient -OR(35) ou -NR(35)R(36) ;
R(35) et R(36)
indépendamment l'un de l'autre, signifient hydrogène ou alkyle comprenant 1, 2, 3, 4, 5 ou 6 atomes de carbone ;
ou
R(35) et R(36)
signifient ensemble 4-7 groupements méthylène, dont un groupement CH₂ peut être remplacé par oxygène, -S-, -NH-, -NCH₃ ou -N-benzyle ;
R(3) signifie hydrogène, -SR(25), -OR(25), -NR(25)R(26), -CR(25)R(26)R(27) ;
R(25) signifie hydrogène, alkyle comprenant 1, 2, 3, 4, 5, 6, 7 ou 8 atomes de carbone ou phényle,
qui est non substitué ou substitué par 1-3 substituants choisis dans le groupe constitué par F, Cl, CF₃, CH₃, méthoxy, hydroxy, amino, méthylamino et diméthylamino ;
ou
R(25) signifie -(C₁-C₉)-hétéroaryle,
qui est non substitué ou substitué par 1-3 substituants choisis dans le groupe constitué par F, Cl, CF₃, CH₃, méthoxy, hydroxy, amino, méthylamino et diméthylamino ;
R(26) et R(27)
indépendamment l'un de l'autre, sont définis comme R(25) ou signifient hydrogène ou alkyle comprenant 1, 2, 3, 4, 5, 6, 7 ou 8 atomes de carbone ;
R(5) signifie alkyle comprenant 1, 2, 3 ou 4 atomes de carbone, F, Cl, Br, I, X- (CH₂)_{y}-CF₃ ou phényle,
qui est non substitué ou substitué par 1-3 substituants choisis dans le groupe constitué par F et Cl, -CF₃, méthyle, méthoxy et -NR(6)R(7),
R(6) et R(7)
indépendamment l'un de l'autre, signifient hydrogène ou -CH₃ ;
X signifie une liaison ou oxygène
y vaut zéro, 1 ou 2,
ainsi que leurs sels pharmaceutiquement acceptables ;
ar) diguanidides d'acides benzènedicarboxyliques de formule I 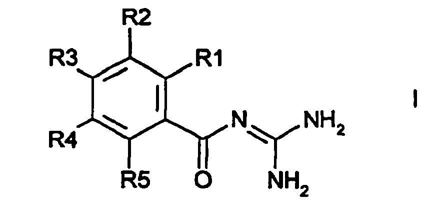 où :
un des radicaux R(1), R(2), R(3) et R(5)
signifie -CO-N=C (NH₂)₂ ;
et à chaque fois les autres radicaux R(1), R(2), R(3) et R(5) :
R(1) et R(5)
indépendamment l'un de l'autre, signifient hydrogène, alkyle comprenant 1, 2, 3 ou 4 atomes de carbone, F, Cl, -OR(32), -NR(33)R(34) ou -CF₃ ;
R(32), R(33) et R(34)
indépendamment les uns des autres, signifient hydrogène ou alkyle comprenant 1, 2, 3 ou 4 atomes de carbone ;
R(2) signifie hydrogène, F, Cl, Br, I, OH, -C≡N, CF₃, -CO-N=C(NH₂)₂, alkyle comprenant 1, 2, 3, 4, 5, 6, 7 ou 8 atomes de carbone, alcényle comprenant 2, 3, 4, 5, 6, 7 ou 8 atomes de carbone ou -(CH₂)ₘR(14) ;
m vaut zéro, 1 ou 2,
R(14) signifie -(C₃-C₈)-cycloalkyle ou phényle,
qui est non substitué ou substitué par 1-3 substituants choisis dans le groupe constitué par F et Cl, -CF₃, méthyle, méthoxy et -NR(15)R(16),
R(15) et R(16)
indépendamment l'un de l'autre, signifient hydrogène ou -CH₃ ;
ou
R(2) signifie R(22)-SO₂-, R(23)R(24)N-CO-, R(28)-CO- ou R(29)R(30)N-SO₂ ;
R(22) et R(28)
indépendamment l'un de l'autre, signifient méthyle ou -CF₃ ;
R(23), R(24), R(29) et R(30)
indépendamment les uns des autres, signifient hydrogène ou méthyle ;
ou
R(2) signifie -OR(35) ou -NR(35)R(36) ;
R(35) et R(36)
indépendamment l'un de l'autre, signifient hydrogène ou alkyle comprenant 1, 2, 3, 4, 5 ou 6 atomes de carbone ;
ou
R(35) et R(36)
ensemble, signifient 4-7 groupements méthylène, dont un groupement CH₂ peut être remplacé par oxygène, -S-, -NH-, -NCH₃ ou -N-benzyle ;
R(3) signifie hydrogène, -SR(25), -OR(25), -NR(25)R(26), -CR(25)R(26)R(27) ;
R(25) signifie hydrogène, alkyle comprenant 1, 2, 3, 4, 5, 6, 7 ou 8 atomes de carbone ou phényle,
qui est non substitué ou substitué par 1-3 substituants choisis dans le groupe constitué par F, Cl, CF₃, CH₃, méthoxy, hydroxy, amino, méthylamino et diméthylamino ;
ou
R(25) signifie - (C₁-C₉) -hétéroaryle,
qui est non substitué ou substitué par 1-3 substituants choisis dans le groupe constitué par F, Cl, CF₃, CH₃, méthoxy, hydroxy, amino, méthylamino et diméthylamino ;
R(26) et R(27)
indépendamment l'un de l'autre, sont définis comme R(25) ou signifient hydrogène ou alkyle comprenant 1, 2, 3, 4, 5, 6, 7 ou 8 atomes de carbone ;
R(4) signifie CF₃, alkyle comprenant 1, 2, 3, 4, 5, 6, 7 ou 8 atomes de carbone, alcényle comprenant 2, 3, 4, 5, 6, 7 ou 8 atomes de carbone, -(C₃-C₈)-cycloalkyle ou -(CH₂)ₘR(14) ;
m vaut 1 ou 2 ;
R(14) signifie -(C₃-C₈)-cycloalkyle ou phényle, qui est non substitué ou substitué par 1-3 substituants choisis dans le groupe constitué par F et Cl, -CF₃, méthyle, méthoxy et -NR(15)R(16),
R(15) et R(16) indépendamment l'un de l'autre, signifient hydrogène ou -CH₃ ;
ou
R(4) signifie phényle,
qui est non substitué ou substitué par 2, 3, 4 ou cinq substituants choisis dans le groupe constitué par F, Cl, CF₃, méthyle, méthoxy et -NR(15)R(16) ;
R(15) et R(16)
indépendamment l'un de l'autre, signifient hydrogène ou CH₃ ;
ainsi que leurs sels pharmaceutiquement acceptables ;
as) diguanidides d'acides diaryldicarboxyliques de formule I 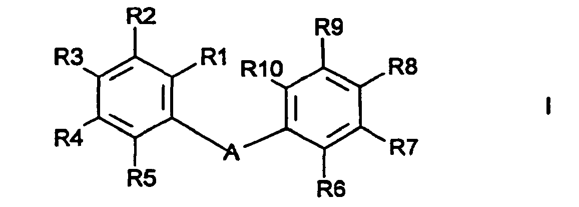 où :
un des radicaux R(1), R(2), R(3), R(4) et R(5) signifie -CO-N=C(NH₂)₂ ;
à chaque fois les autres radicaux R(1) et R(5) : indépendamment l'un de l'autre, signifient hydrogène, alkyle comprenant 1, 2, 3 ou 4 atomes de carbone, F, Cl, -OR(32), -NR(33)R(34) ou -CF₃ ;
R(32), R(33) et R(34)
indépendamment les uns des autres, signifient hydrogène ou alkyle comprenant 1, 2, 3 ou 4 atomes de carbone ;
à chaque fois les autres radicaux R(2) et R(4) :
indépendamment l'un de l'autre, signifient hydrogène, F, Cl, Br, I, OH, -CN, CF₃, -CO-N=C(NH₂)₂, alkyle comprenant 1, 2, 3, 4, 5, 6, 7 ou 8 atomes de carbone, alcényle comprenant 2, 3, 4, 5, 6, 7 ou 8 atomes de carbone ou -(CH₂)ₘR(14) ;
m vaut zéro, 1 ou 2,
R(14) signifie -(C₃-C₈)-cycloalkyle ou phényle,
qui est non substitué ou substitué par 1-3 substituants choisis dans le groupe constitué par F et Cl, -CF₃, méthyle, méthoxy et -NR(15)R(16),
R(15) et R(16)
signifient hydrogène ou -CH₃ ; ou
à chaque fois les autres radicaux R(2) et R(4) :
indépendamment l'un de l'autre, signifient pyrrol-1-yle, pyrrol-2-yle ou pyrrol-3-yle,
qui est non substitué ou substitué par 1-4 substituants choisis dans le groupe constitué par F, Cl, Br, I, -CN, (C₂-C₈)-alcanoyle, (C₂-C₈) - alcoxycarbonyle, formyle, carboxy, -CF₃, méthyle, méthoxy ;
ou
à chaque fois les autres radicaux R(2) et R(4) :
signifient R(22)-SO₂-, R(23)R(24)N-CO-, R(28)-CO- ou R(29)R(30)N-SO₂ ;
R(22) et R(28)
indépendamment l'un de l'autre, signifient méthyle ou -CF₃ ;
R(23), R(24), R(29) et R(30)
indépendamment les uns des autres, signifient hydrogène ou méthyle ;
ou
à chaque fois les autres radicaux R(2) et R(4) :
indépendamment l'un de l'autre, signifient -OR(35) ou -NR(35)R(36) ;
R(35) et R(36)
indépendamment l'un de l'autre, signifient hydrogène ou alkyle comprenant 1, 2, 3, 4, 5 ou 6 atomes de carbone ;
ou
R(35) et R(36)
ensemble, signifient 4-7 groupements méthylène, dont un groupement CH₂ peut être remplacé par oxygène, -S-, -NH-, -NCH₃ ou -N-benzyle ; à chaque fois l'autre substituant R(3)
signifie hydrogène, -SR(25), -OR(25), -NR(25)R(26), -CR(25)R(26)R(27) ;
R(25) signifie hydrogène, alkyle comprenant 1, 2, 3, 4, 5, 6, 7 ou 8 atomes de carbone ou phényle,
qui est non substitué ou substitué par 1-3 substituants choisis dans le groupe constitué par F, Cl, CF₃, CH₃, méthoxy, hydroxy, amino, méthylamino et diméthylamino ;
ou
R(25) signifie -(C₁-C₉)-hétéroaryle,
qui est non substitué ou substitué par 1-3 substituants choisis dans le groupe constitué par F, Cl, CF₃, CH₃, méthoxy, hydroxy, amino, méthylamino et diméthylamino ;
R(26) et R(27)
indépendamment l'un de l'autre, sont définis comme R(25) ou signifient hydrogène ou alkyle comprenant 1, 2, 3, 4, 5, 6, 7 ou 8 atomes de carbone ;
un des radicaux R(6), R(7), R(8), R(9) et R(10) signifie -CO-N=C(NH₂)₂ ;
à chaque fois les autres radicaux R(6) et R(10) :
indépendamment l'un de l'autre, signifient hydrogène, alkyle comprenant 1, 2, 3 ou 4 atomes de carbone, F, Cl, -OR(132), -NR(133)R(134) ou -CF₃ ; R(132), R(133) et R(134)
indépendamment les uns des autres, signifient hydrogène ou alkyle comprenant 1, 2, 3 ou 4 atomes de carbone ;
à chaque fois les autres radicaux R(7) et R(9) :
indépendamment l'un de l'autre, signifient hydrogène, F, Cl, Br, I, OH, -CN, CF₃, -CO-N=C(NH₂)₂, alkyle comprenant 1, 2, 3, 4, 5, 6, 7 ou 8 atomes de carbone, alcényle comprenant 2, 3, 4, 5, 6, 7 ou 8 atomes de carbone ou -(CH₂)ₘₘR(114); mm vaut zéro, 1 ou 2,
R(114)
signifie -(C₃-C₈)-cycloalkyle ou phényle,
qui est non substitué ou substitué par 1-3 substituants choisis dans le groupe constitué par F et Cl, -CF₃, méthyle, méthoxy et -NR(115)R(116),
R(115) et R(116)
signifient hydrogène ou -CH₃ ;
ou
à chaque fois les autres radicaux R(7) et R(9) :
indépendamment l'un de l'autre, signifient pyrrol-1-yle, pyrrol-2-yle ou pyrrol-3-yle,
qui est non substitué ou substitué par 1-4 substituants choisis dans le groupe constitué par F, Cl, Br, I, -CN, (C₂-C₈) -alcanoyle, (C₂-C₈)-alcoxycarbonyle, formyle, carboxy, -CF₃, méthyle et méthoxy ;
ou
à chaque fois les autres radicaux R(7) et R(9) :
signifient R(122)-SO₂-, R(123)R(124)N-CO-, R(128)-CO- ou R(129)R(130)N-SO₂ ;
R(122) et R(128)
indépendamment l'un de l'autre, signifient méthyle ou -CF₃ ;
R(123), R(124), R(129) et R(130)
indépendamment les uns des autres, signifient hydrogène ou méthyle ;
ou
à chaque fois les autres radicaux R(7) et R(9) :
indépendamment l'un de l'autre, signifient -OR(135) ou -NR(135)R(136) ;
R(135) et R(136)
indépendamment l'un de l'autre, signifient hydrogène ou alkyle comprenant 1, 2, 3, 4, 5 ou 6 atomes de carbone ;
ou
R(135) et R(136)
ensemble, signifient 4-7 groupements méthylène, dont un groupement CH₂ peut être remplacé par oxygène, -S-, -NH-, -NCH₃ ou -N-benzyle ;
à chaque fois l'autre substituant R(8)
signifie hydrogène, -SR(125), -OR(125), -NR(125)R(126) ou -CR(125)R(126)R(127) ;
R(125)
signifie hydrogène, alkyle comprenant 1, 2, 3, 4, 5, 6, 7 ou 8 atomes de carbone ou phényle,
qui est non substitué ou substitué par 1-3 substituants choisis dans le groupe constitué par F, Cl, CF₃, CH₃, méthoxy, hydroxy, amino, méthylamino et diméthylamino ;
ou
R(125)
signifie -(C₁-C₉)-hétéroaryle,
qui est non substitué ou substitué par 1-3 substituants choisis dans le groupe constitué par les groupements F, Cl, CF₃, CH₃, méthoxy, hydroxy, amino, méthylamino et diméthylamino ;
R(126) et R(127)
indépendamment l'un de l'autre, sont définis comme R(125) ou signifient hydrogène ou alkyle comprenant 1, 2, 3, 4, 5, 6, 7 ou 8 atomes de carbone ;
A est absent, signifie -NR(11)-CO-, -NR(12)-CO- -NR(13)-, -NR(17)-CO-NR(18)-SO₂-, -NR(19)-SO₂-, -SO₂-NR(19)-SO₂-, -SO₂-NR(19)-CO-, -O-CO-NR(19)--SO₂- ou -CR(20)=CR(21)- ;
R(11), R(12), R(13), R(17), R(18), R(19), R(20) et R(21)
indépendamment les uns des autres, signifient hydrogène ou alkyle comprenant 1, 2, 3, 4, 5, 6, 7 ou 8 atomes de carbone ;
ainsi que leurs sels pharmaceutiquement acceptables ;
at) guanidides substitués des acides thiophénylalcénylcarboxyliques de formule I 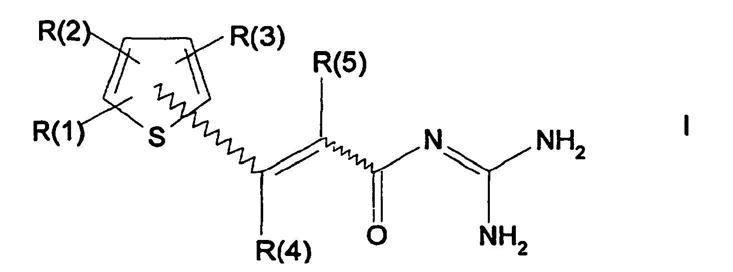 où :
au moins un des substituants R(1), R(2) et R(3) signifie -Oₚ-(CH₂)ₛ-C_{q}F_{2q+1}, R(40)CO- ou R(31)SOₖ- ;
P vaut zéro ou 1 ;
s vaut zéro, 1, 2, 3 ou 4 ;
q vaut 1, 2, 3, 4, 5, 6, 7 ou 8 ;
k vaut zéro, 1 ou 2 ;
R(40) signifie alkyle comprenant 1, 2, 3, 4, 5, 6, 7 ou 8 atomes de carbone, perfluoroalkyle comprenant 1, 2, 3, 4, 5, 6, 7 ou 8 atomes de carbone, cycloalkyle comprenant 3, 4, 5, 6, 7 ou 8 atomes de carbone, ou phényle,
qui est non substitué ou substitué par 1-3 substituants choisis dans le groupe constitué par F, Cl, CF₃, méthyle et méthoxy ;
R(31) signifie alkyle comprenant 1, 2, 3, 4, 5, 6, 7 ou 8 atomes de carbone, perfluoroalkyle comprenant 1, 2, 3, 4, 5, 6, 7 ou 8 atomes de carbone, cycloalkyle comprenant 3, 4, 5, 6, 7 ou 8 atomes de carbone, ou phényle,
qui est non substitué ou substitué par 1-3 substituants choisis dans le groupe constitué par F, Cl, CF₃, méthyle ou méthoxy ;
ou
R(31) signifie NR(41)R(42) ;
R(41) et R(42)
indépendamment l'un de l'autre, signifient hydrogène, alkyle comprenant 1, 2, 3 ou 4 atomes de carbone, perfluoroalkyle comprenant 1, 2, 3 ou 4 atomes de carbone,
ou
R(41) et R(42)
ensemble, signifient 4 ou 5 groupements méthylène, dont un groupement CH₂ peut être remplacé par oxygène, S, NH, N-CH₃ ou N-benzyle ;
et à chaque fois les autres substituants R(1), R(2) et R(3) :
indépendamment les uns des autres, signifient H, F, Cl, Br, I, CN, -Oₙₐ-CₘₐH₂ₘₐ₊₁ ou -O_{ga}CᵣₐH₂ᵣₐR(10);
na vaut zéro ou 1 ;
ma vaut zéro, 1, 2, 3, 4, 5, 6, 7 ou 8 ;
ga vaut zéro ou 1 ;
ra vaut zéro, 1, 2, 3 ou 4 ;
R(10) signifie cycloalkyle comprenant 3, 4, 5, 6, 7 ou 8 atomes de carbone ou phényle
phényle étant non substitué ou substitué par 1-3 substituants choisis dans le groupe constitué par F, Cl, CF₃, méthyle et méthoxy ;
R(4) et R(5)
indépendamment les uns des autres, signifient hydrogène, F, Cl, Br, I, CN, alkyle comprenant 1, 2, 3, 4, 5, 6, 7 ou 8 atomes de carbone, perfluoroalkyle comprenant 1, 2, 3, 4, 5, 6, 7 ou 8 atomes de carbone, cycloalkyle comprenant 3, 4, 5, 6, 7 ou 8 atomes de carbone, ou phényle,
qui est non substitué ou substitué par 1-3 substituants choisis dans le groupe constitué par F, Cl, CF₃, méthyle, méthoxy et NR(14)R(15) ;
R(14) et R(15)
indépendamment l'un de l'autre, signifient H, alkyle comprenant 1, 2, 3 ou 4 atomes de carbone ou perfluoroalkyle comprenant 1, 2, 3 ou 4 atomes de carbone ;
ainsi que leurs sels pharmaceutiquement acceptables ;
au) benzoylguanidines substituées en position ortho de formule I 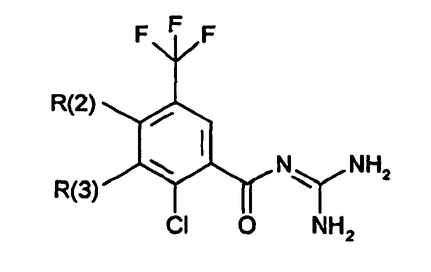 où :
R(2) et R(3) indépendamment l'un de l'autre, signifient hydrogène, Cl, Br, I, (C₁-C₈)-alkyle, (C₃-C₈)-cycloalkyle ou -OR(5) ;
R(5) signifie (C₁-C₈)-alkyle ou -C_{d}H_{2d}-(C₃-C₈) - cycloalkyle ;
d vaut zéro, 1 ou 2 ;
un des deux substituants R(2) et R(3) signifie toujours hydrogène, mais les deux substituants R(2) et R(3) ne signifiant pas simultanément hydrogène,
ainsi que leurs sels pharmaceutiquement acceptables ;
av) benzoylguanidines de formule I 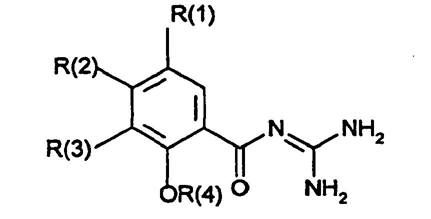 où :
R(1) signifie H, F, Cl, Br, I, CN, NO₂, alkyle comprenant 1, 2, 3, 4, 5, 6, 7 ou 8 atomes de carbone, alcoxy comprenant 1, 2, 3, 4, 5, 6, 7 ou 8 atomes de carbone, cycloalkyle comprenant 3, 4, 5, 6, 7 ou 8 atomes de carbone, cycloalcoxy comprenant 3, 4, 5, 6, 7 ou 8 atomes de carbone ou Xₐ-(CH₂)_{b}(CF₂)_{c}-CF3 ;
X signifie oxygène, S, ou NR(5) ;
a vaut zéro ou 1 ;
b vaut zéro, 1 ou 2 ;
c vaut zéro, 1, 2 ou 3 ;
R(5) signifie H, alkyle comprenant 1, 2, 3 ou 4 atomes de carbone ou -C_{d}H_{2d}R(6) ;
d vaut zéro, 1, 2, 3 ou 4 ;
R(6) signifie cycloalkyle comprenant 3, 4, 5, 6, 7 ou 8 atomes de carbone, phényle, biphénylyle ou naphtyle,
les aromatiques phényle, biphényle ou naphtyle étant non substitués ou substitués par 1-3 substituants choisis dans le groupe constitué par F, Cl, CF₃, méthyle, méthoxy et NR(7)R(8) ;
R(7) et R(8)
indépendamment l'un de l'autre, signifient H ou alkyle comprenant 1, 2, 3 ou 4 atomes de carbone ;
ou
R(1) signifie -SR(10), -OR(10) ou -CR(10)R(11)R(12) ; R(10) signifie -C_{f}H_{2f}-cycloalkyle comprenant 3, 4, 5, 6, 7 ou 8 atomes de carbone dans le cycle cycloalkyle, ou phényle,
phényle étant non substitué ou substitué par 1-3 substituants choisis dans le groupe constitué par F, Cl, CF₃, CH₃, méthoxy, hydroxy, amino, méthylamino et diméthylamino ;
f vaut zéro, 1 ou 2,
R(11) et R(12)
indépendamment l'un de l'autre, sont définis comme R(10) ou signifient hydrogène ou alkyle comprenant 1, 2, 3 ou 4 atomes de carbone ;
ou
R(1) signifie phényle, naphtyle, biphénylyle ou hétéroaryle comprenant 1, 2, 3, 4, 5, 6, 7, 8 ou 9 atomes de carbone, ce dernier étant lié via un atome C ou N du cycle,
qui sont à chaque fois non substitués ou substitués par 1-3 substituants choisis dans le groupe constitué par F, Cl, CF₃, CH₃, méthoxy, hydroxy, amino, méthylamino et diméthylamino ;
ou
R(1) signifie -SR(13), -OR(13), -NHR(13), -NR(13)R(14), -CHR(13)R(15), -C[R(15)R(16)0H], -C≡CR(18), -C[R(19)]=CHR(18), -C[R(20)R(21)]ₖ(-(CO)- [CR(22)R(23]₁-R(24),
k vaut zéro, 1, 2, 3 ou 4 ;
l vaut zéro, 1, 2, 3 ou 4 ;
R(13) et R(14)
identiques ou différents, signifient -(CH₂)_{g}-(CHOH)ₕ-(CH₂)ᵢ-(CHOH)ⱼ-R(17) ou - (CH₂)_{g}-O- (CH₂-CH₂O)ₕ-R(24) ;
R(17) signifie hydrogène ou méthyle,
g, h et i
identiques ou différents, valent zéro, 1, 2, 3 ou 4 ;
j vaut 1, 2, 3 ou 4 ;
R(15) et R(16)
identiques ou différents, signifient hydrogène, alkyle comprenant 1, 2, 3, 4, 5 ou 6 atomes de carbone, ou, ensemble avec l'atome de carbone qui les porte, forment cycloalkyle comprenant 3, 4, 5, 6, 7 ou 8 atomes de carbone ;
R(18)
signifie phényle,
qui est non substitué ou substitué par 1-3 substituants choisis dans le groupe constitué par F, Cl, CF₃, méthyle, méthoxy et NR(25)R(26) ;
R(25) et R(26) signifient H ou alkyle comprenant 1, 2, 3 ou 4 atomes de carbone ;
ou
R(18) signifie hétéroaryle comprenant 1, 2, 3, 4, 5, 6, 7, 8 ou 9 atomes de carbone,
qui est non substitué ou substitué comme phényle ; ou
R(18) signifie alkyle comprenant 1, 2, 3, 4, 5 ou 6 atomes de carbone ;
qui est non substitué ou substitué par 1-3 OH,
ou
R (18)
signifie cycloalkyle comprenant 3, 4, 5, 6, 7 ou 8 atomes de carbone ;
R(19), R(20), R(21), R(22) et R(23)
identiques ou différents, signifient hydrogène ou méthyle ;
R(24) signifie H, alkyle comprenant 1, 2, 3, 4, 5 ou 6 atomes de carbone, cycloalkyle comprenant 3, 4, 5, 6, 7 ou 8 atomes de carbone ou -CₘH₂ₘ-R(18) ;
m vaut 1, 2, 3 ou 4 ;
R(2) et R(3)
sont définis comme R(1) ;
R(4) signifie alkyle comprenant 1, 2, 3 ou 4 atomes de carbone ;
ainsi que leurs sels pharmaceutiquement acceptables ;
aw) benzoylguanidines substituées en position ortho de formule I 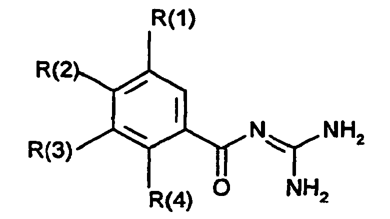 où
R(1) signifie H, F, Cl, Br, I, CN, NO₂, alkyle comprenant 1, 2, 3, 4, 5, 6, 7 ou 8 atomes de carbone, alcoxy comprenant 1, 2, 3, 4, 5, 6, 7 ou 8 atomes de carbone, cycloalkyle comprenant 3, 4, 5, 6, 7 ou 8 atomes de carbone, cycloalcoxy comprenant 3, 4, 5, 6, 7 ou 8 atomes de carbone ou Xₐ-(CH₂)_{b}-(CF₂)_{c}-CF₃;
X signifie oxygène, S, ou NR(5) ;
a vaut zéro ou 1 ;
b vaut zéro, 1 ou 2 ;
c vaut zéro, 1, 2 ou 3 ;
R(5) signifie H, alkyle comprenant 1, 2, 3 ou 4 atomes de carbone ou -C_{d}H_{2d}R(6) ;
d vaut zéro, 1, 2, 3 ou 4 ;
R(6) signifie cycloalkyle comprenant 3, 4, 5, 6, 7 ou 8 atomes de carbone, phényle, biphénylyle ou naphtyle,
les aromatiques phényle, biphényle ou naphtyle étant non substitués ou substitués par 1-3 substituants choisis dans le groupe constitué par F, Cl, CF₃, méthyle, méthoxy et NR(7)R(8) ;
R(7) et R(8)
indépendamment l'un de l'autre, signifient H ou alkyle comprenant 1, 2, 3 ou 4 atomes de carbone ;
ou
R(1) signifie -SR(10), -OR(10) ou -CR(10)R(11)R(12) ; R(10) signifie -C_{f}H_{2f}-cycloalkyle comprenant 3, 4, 5, 6, 7 ou 8 atomes de carbone dans le cycle cycloalkyle, ou phényle,
phényle étant non substitué ou substitué par 1-3 substituants choisis dans le groupe constitué par F, Cl, CF₃, CH₃, méthoxy, hydroxy, amino, méthylamino et diméthylamino ;
f vaut zéro, 1 ou 2 ;
R(11) et R(12)
indépendamment l'un de l'autre, sont définis comme R(10) ou signifient hydrogène ou alkyle comprenant 1, 2, 3 ou 4 atomes de carbone ;
ou
R(1) signifie phényle, naphtyle, biphénylyle ou hétéroaryle comprenant 1, 2, 3, 4, 5, 6, 7, 8 ou 9 atomes de carbone, ce dernier étant lié via un atome C ou N du cycle,
qui sont à chaque fois non substitués ou substitués par 1-3 substituants choisis dans le groupe constitué par F, Cl, CF₃, CH₃, méthoxy, hydroxy, amino, méthylamino et diméthylamino ;
ou
R(1) signifie -SR(13), -OR(13), -NH(13), -NR(13)R(14), -CHR(13)R(15), -C[R(15)R(16)OH], -C=CR(18), -C[R(19)]=CHR(18), -C[R(20)R(21)]ₖ-(CO)- [CR(22)R(23)]ₗ-R(24),
k vaut zéro, 1, 2, 3 ou 4 ;
l vaut zéro, 1, 2, 3 ou 4 ;
R(13) et R(14)
identiques ou différents, signifient -(CH₂)_{g}-(CHOH)ₕ-(CH₂)ᵢ-(CHOH)ⱼ-R(17) ou -(CH₂)_{g}-O- (CH₂-CH₂O)ₕ-R(24) ;
R(17) signifie hydrogène ou méthyle,
g, h et i identiques ou différents, valent zéro, 1, 2, 3 ou 4 ;
j vaut 1, 2, 3 ou 4 ;
R(15) et R(16) identiques ou différents, signifient hydrogène, alkyle comprenant 1, 2, 3, 4, 5 ou 6 atomes de carbone, ou, ensemble avec l'atome de carbone qui les porte, forment cycloalkyle comprenant 3, 4, 5, 6, 7 ou 8 atomes de carbone ;
R(18) signifie phényle,
qui est non substitué ou substitué par 1-3 substituants choisis dans le groupe constitué par F, Cl, CF₃, méthyle, méthoxy et NR(25)R(26) ;
R(25) et R(26) signifient H ou alkyle comprenant 1, 2, 3 ou 4 atomes de carbone ; ou
R(18) signifie hétéroaryle comprenant 1, 2, 3, 4, 5, 6, 7, 8 ou 9 atomes de carbone,
qui est non substitué ou substitué comme phényle ;
ou
R(18) signifie alkyle comprenant 1, 2, 3, 4, 5 ou 6 atomes de carbone ;
qui est non substitué ou substitué par 1-3 OH,
ou
R(18) signifie cycloalkyle comprenant 3, 4, 5, 6, 7 ou 8 atomes de carbone ;
R(19), R(20), R(21), R(22) et R(23) identiques ou différents, signifient hydrogène ou méthyle ;
R(24) signifie H, alkyle comprenant 1, 2, 3, 4, 5 ou 6 atomes de carbone, cycloalkyle comprenant 3, 4, 5, 6, 7 ou 8 atomes de carbone ou -CₘH₂ₘ-R(18) ;
m vaut 1, 2, 3 ou 4 ;
un des deux substituants R(2) et R(3) signifie hydroxyle ;
et
et à chaque fois l'autre des substituants R(2) ou R(3) est défini comme R(1) ;
R(4) signifie alkyle comprenant 1, 2, 3 ou 4 atomes de carbone ; alcoxy comprenant 1, 2, 3 ou 4 atomes de carbone, F, Cl, Br, I ou -(CH2)ₙ-(CF₂)ₒ-CF₃;
n vaut zéro ou 1 ;
o vaut zéro ou 1 ;
ainsi que leurs sels pharmaceutiquement acceptables.
ax) benzoylguanidines disubstituées en position ortho de formule I 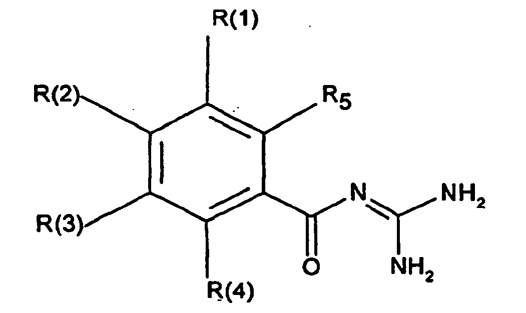 où
R(1), R(2) et R(3) indépendamment les uns des autres, signifient R(10)-SOₐ- ou R(14)R(15)N-SO₂- ;
a vaut zéro, 1 ou 2,
R(10), R(14) et R(15)
indépendamment les uns des autres, signifient alkyle comprenant 1, 2, 3, 4, 5, 6, 7 ou 8 atomes de carbone, perfluoroalkyle comprenant 1, 2, 3, 4, 5, 6, 7 ou 8 atomes de carbone, alcényle comprenant 3, 4, 5 ou 6 atomes de carbone ou -C_{ab}H_{2ab}-R(16);
ab vaut zéro, 1, 2, 3 ou 4 ;
R(16) signifie cycloalkyle comprenant 3, 4, 5, 6 ou 7 atomes de carbone ou phényle
qui est non substitué ou substitué par 1-3 substituants choisis dans le groupe constitué par F, Cl, CF₃, méthyle, méthoxy et NR(17)R(18) ;
R(17) et R(18)
indépendamment l'un de l'autre, signifient hydrogène, CF₃ ou alkyle comprenant 1, 2, 3 ou 4 atomes de carbone ;
ou
R(14) et R(15)
signifient ensemble 4 ou 5 groupements méthylène, dont un groupement CH₂ peut être remplacé par oxygène, soufre, NH, N-CH₃ ou N-benzyle ;
ou
R(14) et R(15)
signifient hydrogène ;
ou
R(1), R(2) et R(3)
indépendamment les uns des autres, signifient SR(21), -OR(22), -NR(23)R(24) ou -CR(25)R(26)R(27) ; R(21), R(22), R(23) et R(25)
indépendamment les uns des autres, signifient -C_{b}H_{2b}- (C₁-C₉) -hétéroaryle,
qui est non substitué ou substitué par 1-3 substituants choisis dans le groupe constitué par F, Cl, CF₃, CH₃, méthoxy, hydroxy, amino, méthylamino et diméthylamino ;
b vaut zéro, 1 ou 2 ;
R(24), R(26) et R(27)
indépendamment les uns des autres, signifient hydrogène, alkyle comprenant 1, 2, 3 ou 4 atomes de carbone ou perfluoroalkyle comprenant 1, 2, 3 ou 4 atomes de carbone ;
ou
R(1), R(2) et R(3)
indépendamment les uns des autres, signifient hydrogène, F, Cl, Br, I, CN, -(Xa)_{dg}-C_{da}H₂dₐ₊₁, -(Xb)_{dh}-(CH₂)_{db}-C_{de}F_{2de+1}. alcényle comprenant 3, 4, 5, 6, 7 ou 8 atomes de carbone ou -C_{df}H_{2df}R(30) ;
(Xa) signifie oxygène, soufre ou NR(33) ;
R(33) signifie hydrogène, alkyle comprenant 1, 2, 3 ou 4 atomes de carbone ou perfluoroalkyle comprenant 1, 2, 3 ou 4 atomes de carbone ;
dg vaut zéro ou 1 ;
(Xb) signifie oxygène, soufre ou NR(34) ;
R(34) signifie hydrogène, alkyle comprenant 1, 2, 3 ou 4 atomes de carbone ou perfluoroalkyle comprenant 1, 2, 3 ou 4 atomes de carbone ;
dh vaut zéro ou 1 ;
da vaut zéro, 1, 2, 3, 4, 5, 6, 7 ou 8 ;
db vaut zéro, 1, 2, 3 ou 4 ;
de vaut zéro, 1, 2, 3, 4, 5, 6 ou 7 ;
df vaut zéro, 1, 2, 3 ou 4 ;
R(30) signifie cycloalkyle comprenant 3, 4, 5, 6, 7 ou 8 atomes de carbone, phényle, biphénylyle ou naphtyle,
les aromatiques phényle, biphényle ou naphtyle étant non substitués ou substitués par 1-3 substituants choisis dans le groupe constitué par F, Cl, CF₃, méthyle, méthoxy et NR(31)R(32) ;
R(31) et R(32)
signifient hydrogène, alkyle comprenant 1, 2, 3 ou 4 atomes de carbone ou perfluoroalkyle comprenant 1, 2, 3 ou 4 atomes de carbone ;
ou
R(1), R (2) et R(3)
indépendamment les uns des autres, signifient NR(40)R(41) ou -(Xe)-(CH₂)_{eb}R(45) ;
R(40) et R(41)
indépendamment l'un de l'autre, signifient hydrogène, alkyle comprenant 1, 2, 3, 4, 5, 6, 7 ou 8 atomes de carbone, perfluoroalkyle comprenant 1, 2, 3, 4, 5, 6, 7 ou 8 atomes de carbone ou (CH₂)ₑ-R(42) ;
e vaut zéro, 1, 2, 3 ou 4 ;
R(42) signifie cycloalkyle comprenant 3, 4, 5, 6 ou 7 atomes de carbone ou phényle
qui est non substitué ou substitué par 1-3 substituants choisis dans le groupe constitué par F, Cl, CF₃, méthyle, méthoxy et NR(43)R(44) ;
R(43) et R(44)
indépendamment l'un de l'autre, signifient hydrogène, CF₃ ou alkyle comprenant 1, 2, 3 ou 4 atomes de carbone ;
ou
R(40) et R(41)
signifient ensemble 4 ou 5 groupements méthylène, dont un groupement CH₂ peut être remplacé par oxygène, soufre, NH, N-CH₃ ou N-benzyle ;
(Xe) signifie oxygène, soufre ou NR(47) ;
R(47) signifie hydrogène, alkyle comprenant 1, 2, 3 ou 4 atomes de carbone ou perfluoroalkyle comprenant 1, 2, 3 ou 4 atomes de carbone ;
eb vaut zéro, 1, 2, 3 ou 4 ;
R(45) signifie cycloalkyle comprenant 3, 4, 5, 6 ou 7 atomes de carbone ou phényle
qui est non substitué ou substitué par 1-3 substituants choisis dans le groupe constitué par F, Cl, CF₃, méthyle, méthoxy et NR(50)R(51) et -(Xfa)-(CH₂)_{ed}-(Xfb)R(46) ;
Xfa signifie CH₂, oxygène, soufre ou NR(48) ;
Xfb signifie oxygène, soufre ou NR(49) ;
R(48), R(49), R(50) et R(51)
indépendamment les uns des autres, signifient hydrogène, alkyle comprenant 1, 2, 3 ou 4 atomes de carbone ou perfluoroalkyle comprenant 1, 2, 3 ou 4 atomes de carbone ;
ed vaut 1, 2, 3 ou 4 ;
R(46) signifie hydrogène, alkyle comprenant 1, 2, 3 ou 4 atomes de carbone ou perfluoroalkyle comprenant 1, 2, 3 ou 4 atomes de carbone ;
ou
R(1), R(2) et R(3)
indépendamment les uns des autres, signifient -CHR(52)R(53) ;
R(52) signifie -(CH₂)_{g}-(CHOH)ₕ-(CH)ᵢ-(CHOH)ₖ-R(54) ou -(CH₂)_{g}-O-(CH₂-CH₂O)ₕ-R(54) ;
R(54) signifie hydrogène ou méthyle ;
g, h, i
identiques ou différents, valent zéro, 1, 2, 3 ou 4 ;
k vaut 1, 2, 3 ou 4 ;
R(53) signifie hydrogène ou alkyle comprenant 1, 2, 3 ou 4 atomes de carbone ;
ou
R(1), R(2) et R(3)
indépendamment les uns des autres, signifient -C(OH)R(55)R(56) ;
R(55) et R(56)
identiques ou différents, signifient hydrogène ou alkyle comprenant 1, 2, 3 ou 4 atomes de carbone ;
ou
R(55) et R(56)
ensemble, signifient cycloalkyle comprenant 3, 4, 5 ou 6 atomes de carbone ;
ou
R(55) signifie -CH₂OH ;
et
R(4) et R(5)
indépendamment l'un de l'autre, signifient alkyle comprenant 1, 2, 3 ou 4 atomes de carbone, alcoxy comprenant 1, 2, 3 ou 4 atomes de carbone, OH, F, Cl, Br, I, CN, -Oₙ-(CH₂)ₒ-(CF₂)ₚ-CF₃;
n vaut zéro ou 1 ;
o vaut zéro, 1 ou 2 ;
p vaut zéro, 1 ou 2 ;
ainsi que leurs sels pharmaceutiquement acceptables.
ay) 1-naphtoylguanidines substituées de formule I 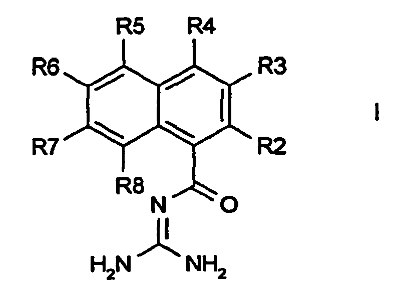 où
R2, R3, R4, R5, R6, R7 et R8
indépendamment les uns des autres, signifient H, F, Cl, Br, I, CN, NO₂, CF₃, C₂F₅ ou XₐY_{b}Z ;
X signifie 0, S, NR(10), CR(11)R(12), C=O, C(=O)NR(10), C(=O)O, SO, SO₂, SO₂NR(10), OC=O, NR(10)C=O ou NR(10)SO₂,
la liaison au cycle naphtalène se faisant à chaque fois via l'atome de gauche ;
R(10), R(11) et R(12)
indépendamment les uns des autres, signifient H, alkyle comprenant 1, 2, 3, 4, 5 ou 6 atomes de carbone, perfluoroalkyle comprenant 1, 2, 3 ou 4 atomes de carbone ou cycloalkyle comprenant 3, 4, 5, 6 ou 7 atomes de carbone ;
a vaut zéro ou 1 ;
Y signifie alkylène comprenant 1, 2, 3, 4, 5, 6, 7 ou 8 groupements CH₂,
un de ces groupements CH₂ pouvant être remplacé par O, S, NR(13) ou o-phénylène, p-phénylène ou m-phénylène ;
R(13) signifie H, alkyle comprenant 1, 2, 3, 4, 5 ou 6 atomes de carbone, perfluoroalkyle comprenant 1, 2, 3 ou 4 atomes de carbone ou cycloalkyle comprenant 3, 4, 5 ou 6 atomes de carbone ;
b vaut zéro ou 1 ;
Z signifie H, alkyle comprenant 1, 2, 3, 4, 5, 6 ou 7 atomes de carbone, cycloalkyle comprenant 3, 4, 5, 6 ou 7 atomes de carbone, C(=O)R(15), SO₂R(15), NR(16)R(17) ou phényle,
qui est non substitué ou substitué par 1-3 substituants choisis dans le groupe constitué par F, Cl, Br, CF₃, méthyle, méthoxy ou NR(21)R(22) ;
R(21) et R(22)
indépendamment l'un de l'autre, signifient H ou alkyle comprenant 1, 2, 3 ou 4 atomes de carbone ou perfluoroalkyle comprenant 1, 2, 3 ou 4 atomes de carbone ;
R(15) signifie N=C(NH₂)₂, NR(18)R(19), N(CH₂)_{c}NR(18)R(19) ou OR(20) ;
c vaut 2 ou 3 ;
R(18) et R(19)
indépendamment l'un de l'autre, signifient H, alkyle comprenant 1, 2, 3, 4, 5, 6, 7 ou 8 atomes de carbone ou perfluoroalkyle comprenant 1, 2, 3 ou 4 atomes de carbone ;
ou
R(18) et R(19)
signifient ensemble 4 ou 5 groupements méthylène, dont un groupement CH₂ peut être remplacé par oxygène, S, NH, N-CH₃, N-benzyle ou N-(p-chlorophényle) ;
R(20) signifie H, alkyle comprenant 1, 2, 3, 4, 5 ou 6 atomes de carbone, perfluoroalkyle comprenant 1, 2, 3 ou 4 atomes de carbone ou cycloalkyle comprenant 3, 4, 5, 6 ou 7 atomes de carbone ;
R(16) et R(17)
indépendamment l'un de l'autre, signifient H, alkyle comprenant 1, 2, 3, 4, 5, 6, 7 ou 8 atomes de carbone ou perfluoroalkyle comprenant 1, 2, 3 ou 4 atomes de carbone ;
ou
R(16) et R(17)
signifient ensemble 4 ou 5 groupements méthylène, dont un groupement CH₂ peut être remplacé par oxygène, S, NH, N-CH₃, N-benzyle ou N-(p-chlorophényle) ;
ou
Z signifie un hétérocycle contenant N, comprenant 1, 2, 3, 4, 5, 6, 7, 8 ou 9 atomes de carbone ;
l'hétérocycle contenant N étant lié via N ou C et étant non substitué ou substitué par 1-3 substituants choisis dans le groupe constitué par F, Cl, Br, CF₃, méthyle, méthoxy et NR(21)R(22) ;
cependant, pour le cas où R(4) signifie un radical alcoxy, au moins un des substituants R(2), R(3), R(5), R(6), R(7) et R(8) étant différent d'hydrogène ;
ainsi que leurs sels pharmaceutiquement acceptables.
az) 2-naphtoylguanidines substituées de formule I 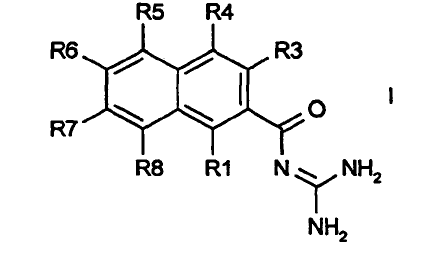 où
au moins un des substituants R1, R3, R4, R5, R6, R7 et R8
signifie XYₐWZ ou X'YₐWZ' ;
X signifie O, S, NR(10) ou CR(11)R(12) ;
R(10), R(11) et R(12)
indépendamment les uns des autres, signifient H, alkyle comprenant 1, 2, 3, 4, 5 ou 6 atomes de carbone, perfluoroalkyle comprenant 1, 2, 3 ou 4 atomes de carbone ou cycloalkyle comprenant 3, 4, 5, 6 ou 7 atomes de carbone ;
Y signifie alkylène comprenant 1, 2, 3, 4, 5, 6, 7 ou 8 groupements CH₂,
un de ces groupements CH₂ pouvant être remplacé par O, S, NR(13) ou o-phénylène, p-phénylène ou m-phénylène ;
R(13) signifie H, alkyle comprenant 1, 2, 3, 4, 5 ou 6 atomes de carbone, perfluoroalkyle comprenant 1, 2, 3 ou 4 atomes de carbone ou cycloalkyle comprenant 3, 4, 5 ou 6 atomes de carbone ;
a vaut zéro ou 1 ;
W signifie CH₂, SO₂, S(=O)(=NH) ou - si W ne suit pas directement un hétéroatome du groupe XYₐ - également O ou NR(14) ;
R(14) signifie H, alkyle comprenant 1, 2, 3, 4, 5 ou 6 atomes de carbone, perfluoroalkyle comprenant 1, 2, 3 ou 4 atomes de carbone ou cycloalkyle comprenant 3, 4, 5 ou 6 atomes de carbone ;
Z signifie C(=O)R(15) SO₂R(15) ou - si W ne signifie ni O ni NR(14) - également NR(16)R(17) ;
R(15)
signifie N=C(NH₂)₂, NR(18)R(19), N(CH₂)_{b}NR(18)R(19) ou OR(20) ;
b vaut 2 ou 3 ;
R(18) et R(19)
indépendamment l'un de l'autre, signifient H, alkyle comprenant 1, 2, 3, 4, 5, 6, 7 ou 8 atomes de carbone ou perfluoroalkyle comprenant 1, 2, 3 ou 4 atomes de carbone ;
ou
R(18) et R(19)
signifient ensemble 4 ou 5 groupements méthylène, dont un groupement CH₂ peut être remplacé par oxygène, S, NH, N-CH₃, N-benzyle ou N-(p-chlorophényle) ;
R(20)
signifie H, alkyle comprenant 1, 2, 3, 4, 5 ou 6 atomes de carbone, perfluoroalkyle comprenant 1, 2, 3 ou 4 atomes de carbone ou cycloalkyle comprenant 3, 4, 5, 6 ou 7 atomes de carbone ;
R(16) et R(17)
indépendamment l'un de l'autre, signifient H, alkyle comprenant 1, 2, 3, 4, 5, 6, 7 ou 8 atomes de carbone ou perfluoroalkyle comprenant 1, 2, 3 ou 4 atomes de carbone ;
ou
R(16) et R(17)
signifient ensemble 4 ou 5 groupements méthylène, dont un groupement CH₂ peut être remplacé par oxygène, S, NH, N-CH₃, N-benzyle ou N-(p-chlorophényle) ;
X' signifie C=O, C(=O)NR(30), C(=O)O, SO, SO₂ SO₂NR(30), OC=O, NR(30)C=O ou NR(30)SO₂,
la liaison au cycle naphtalène se faisant à chaque fois via l'atome de gauche ;
R(30) signifie H, alkyle comprenant 1, 2, 3, 4, 5 ou 6 atomes de carbone, perfluoroalkyle comprenant 1, 2, 3 ou 4 atomes de carbone ou cycloalkyle comprenant 3, 4, 5, 6 ou 7 atomes de carbone ;
Z' signifie C(=O)R(15), SO₂R(15), un hétérocycle contenant N comprenant 1, 2, 3, 4, 5, 6, 7, 8 ou 9 atomes de carbone,
l'hétérocycle contenant N étant lié via N ou C et étant non substitué ou substitué par 1-3 substituants choisis dans le groupe constitué par F, Cl, Br, CF₃, méthyle, méthoxy et NR(21)R(22) ;
R(21) et R(22)
indépendamment l'un de l'autre, signifient H, alkyle comprenant 1, 2, 3 ou 4 atomes de carbone ou perfluoroalkyle comprenant 1, 2, 3 ou 4 atomes de carbone ;
R(15)
signifie N=C(NH₂)₂, NR(18)R(19), N(CH₂)_{b}NR(18)R(19) ou OR(20) ;
R(18) et R(19)
indépendamment l'un de l'autre, signifient H, alkyle comprenant 1, 2, 3, 4, 5, 6, 7 ou 8 atomes de carbone ou perfluoroalkyle comprenant 1, 2, 3 ou 4 atomes de carbone ;
ou
R(18) et R(19)
signifient ensemble 4 ou 5 groupements méthylène, dont un groupement CH₂ peut être remplacé par oxygène, S, NH, N-CH₃, N-benzyle ou N-(p-chlorophényle) ;
b vaut 2 ou 3 ;
R(20) signifie H, alkyle comprenant 1, 2, 3, 4, 5 ou 6 atomes de carbone, perfluoroalkyle comprenant 1, 2, 3 ou 4 atomes de carbone ou cycloalkyle comprenant 3, 4, 5, 6 ou 7 atomes de carbone ;
ou
Z' - si W ne signifie ni O ni NR(14) - signifie NR(16)R(17) ;
R(16) et R(17)
indépendamment l'un de l'autre, signifient H, alkyle comprenant 1, 2, 3, 4, 5, 6, 7 ou 8 atomes de carbone ou perfluoroalkyle comprenant 1, 2, 3 ou 4 atomes de carbone ;
ou
R(16) et R(17)
signifient ensemble 4 ou 5 groupements méthylène, dont un groupement CH₂ peut être remplacé par oxygène, S, NH, N-CH₃, N-benzyle ou N-(p-chlorophényle) ;
et à chaque fois les autres substituants R1, R3, R4, R5, R6, R7 et R8, qui n'ont pas encore été abordés par les définitions ci-dessus,
indépendamment les uns des autres, signifient H, F, Cl, Br, I, CN, NO₂, CF₃, C₂F₅ ou VₚQ_{q}U ;
V signifie O, S, SO, SO₂, NR(60), OC=O, C=O, C(=O)NR(60), C(=O)O ou CR(66)R(67) ;
R(60), R(66) et R(67)
indépendamment les uns des autres, signifient H, alkyle comprenant 1, 2, 3, 4, 5 ou 6 atomes de carbone, perfluoroalkyle comprenant 1, 2, 3 ou 4 atomes de carbone ou cycloalkyle comprenant 3, 4, 5, 6 ou 7 atomes de carbone ;
p vaut zéro ou 1 ;
Q signifie alkylène comprenant 1, 2, 3, 4, 5, 6, 7 ou 8 groupements CH₂,
un de ces groupements CH₂ pouvant être remplacé par O, S, NR(68) ou o-phénylène, p-phénylène ou m-phénylène ;
R(68)
signifie H, alkyle comprenant 1, 2, 3, 4, 5 ou 6 atomes de carbone, perfluoroalkyle comprenant 1, 2, 3 ou 4 atomes de carbone ou cycloalkyle comprenant 3, 4, 5 ou 6 atomes de carbone ;
q vaut zéro ou 1 ;
U signifie H, alkyle comprenant 1, 2, 3, 4, 5, 6 ou 7 atomes de carbone, cycloalkyle comprenant 3, 4, 5, 6 ou 7 atomes de carbone, C(=O)R(65), SO₂R(65), NR(61)R(62) ou phényle,
qui est non substitué ou substitué par 1-3 substituants choisis dans le groupe constitué par F, Cl, Br, CF₃, méthyle, méthoxy et NR(63)R(64) ;
R(63) et R(64)
indépendamment l'un de l'autre, signifient H, alkyle comprenant 1, 2, 3 ou 4 atomes de carbone ou perfluoroalkyle comprenant 1, 2, 3 ou 4 atomes de carbone ;
R(65) signifie N=C(NH₂)₂, NR(61)R(62) ou OR(60) ; R(61) et R(62)
indépendamment l'un de l'autre, signifient H, alkyle comprenant 1, 2, 3, 4, 5, 6, 7 ou 8 atomes de carbone ou perfluoroalkyle comprenant 1, 2, 3 ou 4 atomes de carbone ;
ou
R(61) et R(62)
signifient ensemble 4 ou 5 groupements méthylène, dont un groupement CH₂ peut être remplacé par oxygène, S, NH, N-CH₃, N-benzyle ou N-(p-chlorophényle) ;
ou
U signifie un hétérocycle contenant N, comprenant 1, 2, 3, 4, 5, 6, 7, 8 ou 9 atomes de carbone ;
l'hétérocycle contenant N étant lié via N ou C et étant non substitué ou substitué par 1-3 substituants choisis dans le groupe constitué par F, Cl, Br, CF₃, méthyle, méthoxy et NR(63)R(64) ;
où cependant au moins un des substituants R5, R6, R7 et R8 est différent d'hydrogène ;
ainsi que leurs sels pharmaceutiquement acceptables ;
ba) benzoylguanidines substituées en position ortho de formule I 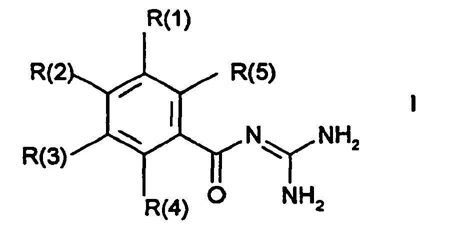 où
R(1) signifie H, F, Cl, Br, I, CN, NO₂, alkyle comprenant 1, 2, 3, 4, 5, 6, 7 ou 8 atomes de carbone, alcoxy comprenant 1, 2, 3, 4, 5, 6, 7 ou 8 atomes de carbone, cycloalkyle comprenant 3, 4, 5, 6, 7 ou 8 atomes de carbone, cycloalcoxy comprenant 3, 4, 5, 6, 7 ou 8 atomes de carbone ou Xₐ-(CH₂)_{b}-(CF₂)_{c}-CF₃;
X signifie oxygène, soufre ou NR(9) ;
a vaut zéro ou 1 ;
b vaut zéro, 1 ou 2 ;
c vaut zéro, 1, 2 ou 3 ;
R(9) signifie H, alkyle comprenant 1, 2, 3 ou 4 atomes de carbone ou -C_{d}H_{2d}R(6) ;
d vaut zéro, 1, 2, 3 ou 4 ;
R(6) signifie cycloalkyle comprenant 3, 4, 5, 6, 7 ou 8 atomes de carbone, phényle, biphénylyle ou naphtyle, les aromatiques phényle, biphényle ou naphtyle étant non substitués ou substitués par 1-3 substituants choisis dans le groupe constitué par F, Cl, CF₃, méthyle, méthoxy et NR(7)R(8) ;
R(7) et R(8)
indépendamment l'un de l'autre, signifient H ou alkyle comprenant 1, 2, 3 ou 4 atomes de carbone ;
ou
R(1) signifie -SR(10), -OR(10) ou -CR(10)R(11)R(12) ; R(10) signifie -C_{f}H_{2f}-cycloalkyle comprenant 3, 4, 5, 6, 7 ou 8 atomes de carbone dans le cycle cycloalkyle,
hétéroaryle comprenant 1, 2, 3, 4, 5, 6, 7, 8 ou 9 atomes de carbone ou phényle, hétéroaryle et phényle étant non substitués ou substitués par 1-3 substituants choisis dans le groupe constitué par F, Cl, CF₃, CH₃, méthoxy, hydroxy, amino, méthylamino et diméthylamino ;
f vaut zéro, 1 ou 2 ;
R(11) et R(12)
indépendamment l'un de l'autre, sont définis comme R(10) ou signifient hydrogène ou alkyle comprenant 1, 2, 3 ou 4 atomes de carbone ;
ou
R(1) signifie phényle, naphtyle, biphénylyle ou hétéroaryle comprenant 1, 2, 3, 4, 5, 6, 7, 8 ou 9 atomes de carbone, ce dernier étant lié via un atome C ou N du cycle,
qui sont à chaque fois non substitués ou substitués par 1-3 substituants choisis dans le groupe constitué par F, Cl, CF₃, CH₃, méthoxy, hydroxy, amino, méthylamino et diméthylamino ;
ou
R(1) signifie -SR(13), -OR(13), -NHR(13), -NR(13)R(14), -CHR(13)R(15), -C[R(15)R(16)]OH, -C=CR(18), -C[R(19)]=CHR(18), -C[R(20)R(21)]ₖ-(CO)-[CR(22)R(23)]ₗ-R(24),
k vaut zéro, 1, 2, 3 ou 4 ;
l vaut zéro, 1, 2, 3 ou 4 ;
R(13) et R(14)
identiques ou différents, signifient -(CH₂)g-(CHOH)ₕ-(CH₂)ᵢ- (CHOH)ₖₖ-R(17) ou - (CH₂)_{g}-O-(CH₂-CH₂O)ₕ-R(24) ;
R(17 signifie hydrogène ou méthyle,
g, h et i
identiques ou différents, valent zéro, 1, 2, 3 ou 4 ;
kk vaut 1, 2, 3 ou 4 ;
R(15) et R(16)
identiques ou différents, signifient hydrogène, alkyle comprenant 1, 2, 3, 4, 5 ou 6 atomes de carbone, ou, ensemble avec l'atome de carbone qui les porte, forment cycloalkyle comprenant 3, 4, 5, 6, 7 ou 8 atomes de carbone ;
R(18) signifie phényle,
qui est non substitué ou substitué par 1-3 substituants choisis dans le groupe constitué par F, Cl, CF₃, méthyle, méthoxy et NR(25)R(26) ;
R(25) et R(26)
signifient H ou alkyle comprenant 1, 2, 3 ou 4 atomes de carbone ;
ou
R(18) signifie hétéroaryle comprenant 1, 2, 3, 4, 5, 6, 7, 8 ou 9 atomes de carbone,
qui est non substitué ou substitué comme phényle ;
ou
R(18) signifie alkyle comprenant 1, 2, 3, 4, 5 ou 6 atomes de carbone ;
qui est non substitué ou substitué par 1-3 OH,
ou
R(18) signifie cycloalkyle comprenant 3, 4, 5, 6, 7 ou 8 atomes de carbone ;
R(19), R(20), R(21), R(22) et R(23)
identiques ou différents, signifient hydrogène ou méthyle ;
R(24) signifie H, alkyle comprenant 1, 2, 3, 4, 5 ou 6 atomes de carbone, cycloalkyle comprenant 3, 4, 5, 6, 7 ou 8 atomes de carbone ou -CₘH₂ₘ-R(18) ;
m vaut 1, 2, 3 ou 4 ;
un des deux substituants R(2) et R(3)
signifie -O-CO-R(27) ;
R(27) signifie alkyle comprenant 1, 2, 3, 4, 5, 6, 7 ou 8 atomes de carbone, cycloalkyle comprenant 3, 4, 5, 6, 7 ou 8 atomes de carbone, phényle, biphénylyle, naphtyle, pyridyle ou quinoléinyle,
phényle, biphénylyle, naphtyle, pyridyle ou quinoléinyle étant non substitués ou substitués par 1-3 substituants choisis dans le groupe constitué par F, Cl, CF₃, méthyle, méthoxy et NR(7)R(8) ;
R(7) et R(8)
indépendamment l'un de l'autre, signifient hydrogène ou alkyle comprenant 1, 2, 3 ou 4 atomes de carbone ;
un des substituants R(2) et R(3)
étant toujours défini comme R(1) ;
R(4) et R(5)
indépendamment l'un de l'autre, signifient hydrogène, alkyle comprenant 1, 2, 3 ou 4 atomes de carbone ; alcoxy comprenant 1, 2, 3 ou 4 atomes de carbone, F, Cl, Br, I, CN ou -(CH₂)ₙ-(CF₂)ₒ- CF₃ ;
n vaut zéro ou 1 ;
o vaut zéro ou 1 ;
ainsi que leurs sels pharmaceutiquement acceptables ;
bb) benzoylguanidines de formule I 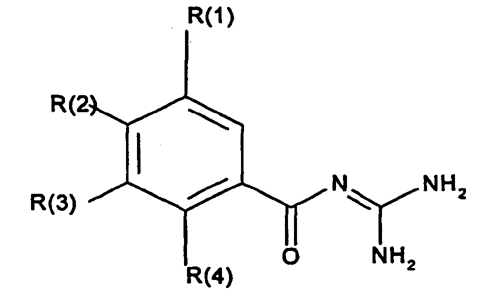 où
R(1) signifie R(13)-SOₘ ou R(14)R(15)N-SO₂- ;
m vaut 1 ou 2 ;
R(13) signifie alkyle comprenant 1, 2, 3, 4, 5, 6, 7 ou 8 atomes de carbone, perfluoroalkyle comprenant 1, 2, 3, 4, 5, 6, 7 ou 8 atomes de carbone, alcényle comprenant 3, 4, 5, 6, 7 ou 8 atomes de carbone ou -CₙH₂ₙ-R(16),
n vaut zéro, 1, 2, 3 ou 4 ;
R(16) signifie cycloalkyle comprenant 3, 4, 5, 6, 7 ou 8 atomes de carbone, phényle, biphénylyle ou naphtyle,
phényle, biphénylyle et naphtyle étant non substitués ou substitués par 1-3 substituants choisis dans le groupe constitué par F, Cl, CF₃, méthyle, méthoxy et NR(25)R(26) ;
R(25) et R(26)
indépendamment l'un de l'autre, signifient hydrogène, alkyle comprenant 1, 2, 3 ou 4 atomes de carbone ou perfluoroalkyle comprenant 1, 2, 3 ou 4 atomes de carbone ;
R(14) signifie hydrogène, alkyle comprenant 1, 2, 3, 4, 5, 6, 7 ou 8 atomes de carbone, perfluoroalkyle comprenant 1, 2, 3, 4, 5, 6, 7 ou 8 atomes de carbone, alcényle comprenant 3, 4, 5, 6, 7 ou 8 atomes de carbone ou -CₙH₂ₙ-R(27),
n vaut zéro, 1, 2, 3 ou 4 ;
R(27) signifie cycloalkyle comprenant 3, 4, 5, 6, 7 ou 8 atomes de carbone, phényle, biphénylyle ou naphtyle,
phényle, biphénylyle et naphtyle étant non substitués ou substitués par 1-3 substituants choisis dans le groupe constitué par F, Cl, CF₃, méthyle, méthoxy et NR(28)R(29) ;
R(28) et R(29)
indépendamment l'un de l'autre, signifient hydrogène, alkyle comprenant 1, 2, 3 ou 4 atomes de carbone ou perfluoroalkyle comprenant 1, 2, 3 ou 4 atomes de carbone ;
R(15) signifie hydrogène, alkyle comprenant 1, 2, 3 ou 4 atomes de carbone ou perfluoroalkyle comprenant 1, 2, 3 ou 4 atomes de carbone ;
ou
R(14) et R(15)
signifient ensemble 4 ou 5 groupements méthylène, dont un groupement CH₂ peut être remplacé par oxygène, S, NH, N-CH₃ ou N-benzyle ;
un des deux substituants R(2) et R(3)
signifie hydrogène ;
et à chaque fois l'autre substituant R(2) ou R(3) signifie -CHR(30)R(31) ;
R(30) signifie -(CH₂)_{g}-(CHOH)ₕ-(CH₂)ᵢ-(CHOH)ₖ-R(32) ou -(CH₂)_{g}-O-(CH₂-CH_{2O})ₕ-R(24) ;
R(24) et R(32) indépendamment l'un de l'autre, signifient hydrogène ou méthyle ;
g, h, i
identiques ou différents, valent zéro, 1, 2, 3 ou 4 ;
k vaut 1, 2, 3 ou 4 ;
ou à chaque fois l'autre substituant R(2) ou R(3) signifie -C(OH)R(33)R(34) ;
R(31), R(33) et R(34)
identiques ou différents, signifient hydrogène ou alkyle comprenant 1, 2, 3 ou 4 atomes de carbone ;
ou
R(33) et R(34)
ensemble, signifient cycloalkyle comprenant 3, 4, 5 ou 6 atomes de carbone ;
ou
R(33) signifie -CH₂OH ;
R(4) signifie alkyle comprenant 1, 2, 3 ou 4 atomes de carbone ; alcoxy comprenant 1, 2, 3 ou 4 atomes de carbone, F, Cl, Br, I, CN, - (CH₂)ₙ-(CF₂)ₒ-CF₃;
n vaut zéro ou 1 ;
o vaut zéro, 1 ou 2 ;
ainsi que leurs sels pharmaceutiquement acceptables ;
bc) indanylidinacétylguanidines de formule I 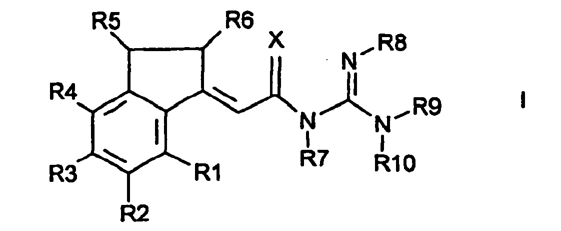 où
R1, R2, R3, R4, R5 et R6
indépendamment les uns des autres, signifient H, C₁-C₁₀-alkyle; halogénoalkyle comprenant 1-6 atomes de carbone, O-C₁-C₁₀-alkyle, halogénoalcoxy comprenant 1-6 atomes de carbone, F, Cl, Br, I, aryle, aryle substitué, hétéroaryle, hétéroaryle substitué, OH, 0-alkyle inférieur, O-(alkyle inférieur)aryle, 0-aryle substitué, O-aryle substitué par alkyle inférieur, OC(=O)-C₁-C₄-alkylaryle, O-C(=O)-NH-C₁-C₄-alkyle, OC(=O)-N(C₁-C₄-alkyle)₂, NO₂, CN, CF₃, NH₂, NH-C(=O)-C₁-C₄-alkyle, NH-C(=O)-NH₂, COOH, C(=O)-O-C₁-C₄-alkyle, C(=O)-NH₂, C(=O)-NH-C₁-C₄-alkyle, C(=O)-N(C₁-C₄-alkyle)₂, C₁-C₄-COOH, C₁-C₄-alkyl-C(=O)-O-C₁-C₄-alkyle, SO₃H, SO₂-alkyle ; SO₂-alkylaryle, SO₂-N-(alkyle)₂, SO₂-N(alkyl)(alkylaryle), C(=O)-R11, C₁-C₁₀-alkyl-C(=O)-R11, C₂-C₁₀-alcényl-C (=O) -R11, C₂-C₁₀-alcynyl-C (=O) -R11, NH-C(=O)-C₁-C₁₀-alkyl-C(=O)-R11 ou O-C₁-C₁₁-alkyl-C(=O)-R11 ;
R11 signifie C₁-C₄-alkyle, C₁-C₄-alcynyle, aryle, aryle substitué, NH₂, NH-C₁-C₄-alkyle, N-(C₁-C₄-alkyle)₂, SO₃H, SO₂-alkyle, SO₂-alkylaryle, SO₂-N-(alkyle)₂ ou SO₂-N(alkyl)(alkylaryle) ;
X signifie 0, S ou NH ;
R7, R8, R9 et R10
indépendamment les uns des autres, signifient H, alkyle, cycloalkyle, aryle, alkylaryle ;
ou
R8 et R9 ensemble, forment une partie d'un cycle hétérocyclique de 5, 6 ou 7 chaînons ;
ou leurs sels pharmaceutiquement acceptables.
bd) guanidides d'acides alcénylcarboxyliques substitués par phényle de formule I 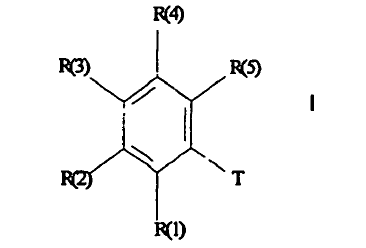 où
T signifie 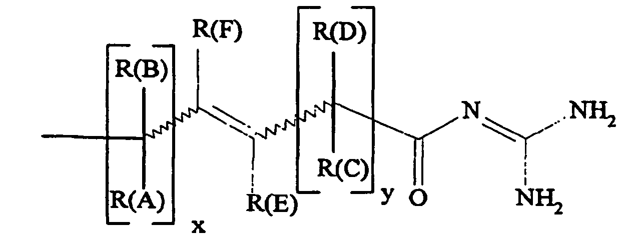
R(A) signifie hydrogène, F, Cl, Br, I, CN, OH, OR(6), (C₁-C₄)-alkyle, Oᵣ(CH₂)ₐC_{b}F_{2b+1}, (C₃-C₈)-cycloalkyle ou NR(7)R(8)
r vaut zéro ou 1 ;
a vaut zéro, 1, 2, 3 ou 4 ;
b vaut 1, 2, 3 ou 4 ;
R (6) signifie (C₁-C₄)-alkyle, (C₁-C₄)-perfluoroalkyle, (C₃-C₆)-alcényle, (C₃-C₃)-cycloalkyle, phényle ou benzyle ;
le noyau phényle étant non substitué ou substitué par 1-3 substituants choisis dans le groupe constitué par F, Cl, CF₃, méthyle, méthoxy et NR(9)R(10) ;
R(9) et R(10)
signifie H, (C₁-C₄)-alkyle ou (C₁-C₄)-perfluoroalkyle ;
R(7) et R(8)
indépendamment l'un de l'autre, sont définis comme R(6) ;
ou
R(7) et R(8)
signifient ensemble 4 ou 5 groupements méthylène, dont un groupement CH₂ peut être remplacé par oxygène, soufre, NH, N-CH₃ ou N-benzyle ;
R(B), R(C) et R(D)
indépendamment, sont définis comme R(A) ;
x vaut zéro, 1 ou 2 ;
y vaut zéro, 1 ou 2 ;
R(F) signifie hydrogène, F, Cl, Br, I, CN, OR(12), (C₁-C₈)-alkyle, Oₚ(CH₂)_{f}C_{g}F_{2g+1} ou (C₃-C₈)-cycloalkyle ou (C₁-C₉)-hétéroaryle ;
p vaut zéro ou 1 ;
f vaut zéro, 1, 2, 3 ou 4 ;
g vaut 1, 2, 3, 4, 5, 6, 7 ou 8 ;
R(12) signifie (C₁-C₈)-alkyle, (C₁-C₄)-perfluoroalkyle, (C₃-C₈)-alcényle, (C₃-C₈)-cycloalkyle, phényle ou benzyle ;
le noyau phényle étant non substitué ou substitué par 1-3 substituants choisis dans le groupe constitué par F, Cl, CF₃, méthyle, méthoxy et NR(13)R(14) ;
R(13) et R(14)
signifient hydrogène, (C₁-C₄) -alkyle ou (C₁-C₄)-perfluoroalkyle ;
R(E) indépendamment, est défini comme R(F) ;
R(1) indépendamment, est défini comme T ;
ou
R(1) signifie hydrogène, -OₖCₘH₂ₘ₊₁, -Oₙ(CH₂)ₚC_{q}F_{2q+1}, F, Cl, Br, I, CN, -(C=O)-N=C(NH₂)₂, -SOᵣR(17), -SOᵣ₂NR(31)R(32); -Oᵤ(CH₂)ᵥC₆H₅, -Oᵤ₂- (C₁-C₉)-hétéroaryle ou -Sᵤ₂- (C₁-C₉)-hétéroaryle ;
k vaut zéro ou 1 ;
m vaut zéro, 1, 2, 3, 4, 5, 6, 7 ou 8 ;
n vaut zéro ou 1 ;
p vaut zéro, 1, 2, 3 ou 4 ;
q vaut 1, 2, 3, 4, 5, 6, 7 ou 8 ;
r vaut zéro, 1, 2 ;
r2 vaut zéro, 1, 2 ;
R(31) et R(32)
indépendamment l'un de l'autre, signifient hydrogène, (C₁-C₈)-alkyle ou (C₁-C₈)-perfluoroalkyle ; ou
R(31) et R(32)
signifient ensemble 4 ou 5 groupements méthylène, dont un groupement CH₂ peut être remplacé par oxygène, S, NH, N-CH₃ ou N-benzyle ;
R(17) signifie (C₁-C₈)-alkyle ;
u vaut zéro ou 1 ;
u2 vaut zéro ou 1 ;
v vaut zéro, 1, 2, 3 ou 4 ;
le noyau phényle étant non substitué ou substitué par 1-3 substituants choisis dans le groupe constitué par F, Cl, CF₃, méthyle, méthoxy, -(CH₂)_{w}NR(21)R(22), NR(18)R(19) et (C₁-C₉)-hétéroaryle ;
R(18), R(19), R(21) et R(22)
indépendamment les uns des autres, signifient (C₁-C₄)-alkyle ou (C₁-C₄)-perfluoroalkyle ;
w vaut 1, 2, 3 ou 4 ;
l'hétérocycle du groupement (C₁-C₉)-hétéroaryle étant non substitué ou substitué par 1-3 substituants choisis dans le groupe constitué par F, Cl, CF₃, méthyle ou méthoxy ;
R(2), R(3), R(4) et R(5)
indépendamment les uns des autres, sont définis comme R(1) ;
ou
R(1) et R(2) ou R(2) et R(3)
ensemble, signifient à chaque fois -CH-CH=CH-CH-,
qui est non substitué ou substitué par 1-3 substituants choisis dans le groupe constitué par F, Cl, CF₃, méthyle, méthoxy, -(CH₂)_{w2}NR(24)R(25) et NR(26)R(27) ;
R(24), R(25), R(26) et R(27)
signifient hydrogène, (C₁-C₄)-alkyle ou (C₁-C₄) - perfluoroalkyle ;
w2 vaut 1, 2, 3 ou 4 ;
le radical T étant présent au moins deux fois, mais au plus seulement trois fois dans la molécule ;
ainsi que leurs sels pharmaceutiquement acceptables ;
be) benzoylguanidines de formule I 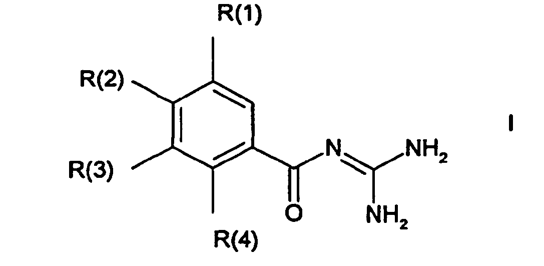 où
R(1) signifie CF₃ ;
un des deux substituants R(2) et R(3) signifie hydrogène ;
et à chaque fois l'autre substituant R(2) ou R(3) signifie -C (OH) (CH₃) -CH₂OH, -CH (CH₃)-CH₂OH ou -C(OH)(CH₃)₂;
R(4) signifie méthyle, méthoxy, Cl ou CF₃ ;
ainsi que leurs sels pharmaceutiquement acceptables ; ou
II. des composés de formule 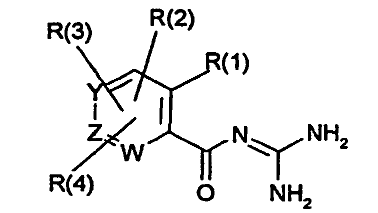 où
W, Y et Z
signifient un atome d'azote ou un atome de carbone substitué par R(2) ou R(3) ou R(4) ;
R(1) signifie hydrogène, A, Hal, -CF₃, -CH₂F, -CHF₂, -CH₂CF₃, -C₂F₅, -CN, -NO₂, -éthynyle, ou X-R' ;
A signifie alkyle comprenant 1 à 6 atomes de carbone ;
Hal signifie F, Cl, Br ou I,
X signifie oxygène, S ou NR" ;
R" signifie hydrogène, A ou une chaîne méthylène cyclique comprenant 3 à 7 atomes de carbone ;
R' signifie H, A, HO-A-, HOOC-A-, (C₃-C₇)-cycloalkyle, (C₆-C₈)-cycloalkylalkyle, CF₃, CH₂F, CHF₂, CH₂-CF₃, Ph, -CH₂-Ph ou Het ;
Ph signifie phényle, naphtyle ou biphénylyle non substitué ou monosubstitué, disubstitué ou trisubstitué par A, OA, NR'R", Hal, CF₃ ;
Het signifie un hétérocycle à un ou deux noyaux, saturé, insaturé ou aromatique, comprenant 1 à 4 atomes de N, O et/ou S,
qui est non substitué ou monosubstitué, disubstitué ou trisubstitué par Hal, CF₃, A, OH, OA, -X-R', -CN, -NO₂, et/ou oxygène à fonction carbonyle,
Het étant lié via N ou une chaîne alkylène CₘH₂ₘ, m = zéro à 6 ;
ou
R' et R"
ensemble, signifient alkylène comprenant 4-5 atomes de carbone, un groupement CH₂ pouvant également être remplacé par oxygène, S, NH, N-A, N-Ph et N-CH₂-Ph ;
R(2) et R(3)
indépendamment l'un de l'autre, signifient hydrogène, Hal, A, HO-A-, X-R', -C(=N-OH)-A, A-O-CO-(C₁-C₄)-alkyl-, CN, NO₂ COOH, A halogénosubstitué, en particulier CF₃, CH₂F, CHF₂, C₂F₅, CH₂CF₃, ou S(O)ₙR"';
R"' signifie A, Ph ou -Het ;
n vaut zéro, 1 ou 2 ;
ou
R(2) et R(3) indépendamment l'un de l'autre, signifient SO₂NR'R", Ph ou -O-Ph, -O-CH₂-Ph, -CO-A, -CHO, -COOA, -CSNR'R", CONR'R", -CH=CH-COOH, -CH=CH-COOA, indényle, indanyle, décahydronaphtyle, cyclopentényle, dihydrothiényle, dihydrofuryle, hétérobicyclyle, alkylthiényle, halogénothiényle, halogénoalkylthiényle, acylthiényle, halogénofuryle, halogénoalkylfuryle ou pyrrolyle ;
ou
R(2) et R(3) indépendamment l'un de l'autre, signifient R(5)-O- ;
R(5) signifie hydrogène, A, (C₁-C₆)-alcényle ou (C₃-C₇)-cycloalkyle ;
R(4) signifie Ph, Het, -O-Het ; CF₃, S(O)ₙR'", -SO₂NR'R", alk ; 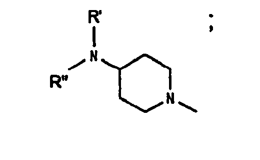 ou
deux des substituants R(1) à R(4) :
ensemble, signifient un groupement -O-CR(6)R(7)-CO-NR(8)-,
ou 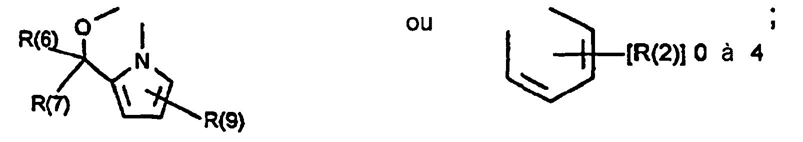
R(2) ayant la signification indiquée ;
R(6), R(7), R(8) et R(9)
indépendamment les uns des autres, signifient H ou A ;
ou
R(8) signifie (C₅-C₇)-cycloalkyle ;
ou
R(9) signifie cyano ;
Alk signifie (C₁-C₈)-alkyle linéaire ou ramifié ou (C₃-C₈)-cycloalkyle,
qui est non substitué ou monosubstitué, disubstitué ou trisubstitué par A,
ou
Alk signifie un radical éthényle ou éthynyle substitué par H, A, Ph ou Het ;
ou
III. des composés de formule 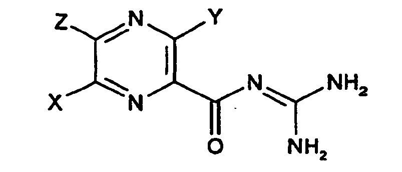 où
X signifie H, Hal, (Hal)₃C-, (C₁-C₆) -alkyle, (C₃-C₆) - cycloalkyle, phényle substitué, (C₁-C₅) -alkyl-S- ou (C₁-C₅)-alkyl-SO₂- ;
Y NH₂ ou amino substitué ;
ou
X et Z
ensemble, signifient une chaîne -(CH₂)₄- ou 1,3-butadiénylène ;
ou
Z signifie H, Hal, OH, HS, (C₁-C₅)-alkyle, (C₃-C₆) - cycloalkyle, phényle substitué ;
ou
Z signifie un groupement amino -NR(1)R(2) ;
R(1) signifie H, (C₁-C₈)-alkyle à chaîne linéaire ou ramifiée, le cas échéant substitué
qui peut être interrompu par oxygène ;
ou
R(1) signifient (C₃-C₈)-alcényle, (C₃-C₈)-alcynyle, (C₃-C₇)-cycloalkyle ou phényle substitué par OH ou phényl-(C₁-C₄)-alkyle substitué par OH ou (C₃-C₇)-cycloalkyle substitué par OH ;
R(2) signifie 1-morpholino, hydrogène ou une chaîne (C₁-C₈)-alkyle linéaire ou ramifiée,
qui peut être interrompue par oxygène, un groupement amino ;
la chaîne (C₁-C₈)-alkyle linéaire ou ramifiée étant non substituée ou substituée par
un hétérocycle à un ou plusieurs noyaux, substitué ou non substitué, qui contient des atomes d'azote, d'oxygène ou de soufre ;
ou
la chaîne alkyle étant substituée par phényle,
non substitué ou monosubstitué ou polysubstitué par (C₁-C₄)-alcoxy, le cas substitué par OH, alkylamino, alkyle ou phényle ;
ou
par un groupement aminocarbonyle
ou
par des groupements hydroxy, (C₁-C₄)-alcoxy,
ou
R(2) signifie phényle,
non substitué ou substitué par alkyle, alcoxy, un groupement amino, qui porte comme substituants :
H, un hétérocycle à un ou plusieurs noyaux, qui contient des atomes d'azote, d'oxygène ou de soufre ;
qui est non substitué ou substitué par H, Hal ou (C₁-C₄)-alkyle ;
un radical phényle,
non substitué ou substitué par un substituant choisi dans le groupe constitué par (C₁-C₄) -alkyle, (C₁-C₄)-alcoxy, Hal et OH ;
ou
R(2) signifie 1-pipéridino,
non substitué ou substitué en 4ème position par un radical acyle d'un acide carboxylique aliphatique, alicyclique, aromatique ou hétéroaromatique, (C₁-C₈)-alkyle, qui peut à son tour être substitué par OH ou par (C₁-C₄)-alcoxy ou un radical phényle substitué par (C₁-C₄)-alcoxy ;
ou
R(2) signifie amidino,
qui est non substitué ou substitué par phényle ;
qui est non substitué ou substitué par Hal ou alkyle ;
ou
R(2) signifie un radical acyle d'un acide carboxylique aliphatique, alicyclique, aromatique ou hétéroaromatique,
ou
R(2) signifie une chaîne (C₁-C₈)-alkyle qui peut être substituée par OH, alcoxy ou un radical phényle portant des radicaux alkyle.
ou
R(1) et R(2)
ensemble avec l'atome de N auquel ils sont liés, signifient un cycle pipérazine,
qui est non substitué ou qui porte via un chaîne (C₁-C₆)-méthylène un hétérocycle à un ou plusieurs noyaux,
qui contient des atomes d'azote, d'oxygène ou de soufre.
Hal signifie F, Cl, Br ou I,
ou
IV. des dérivés d'indoloylguanidine de formule 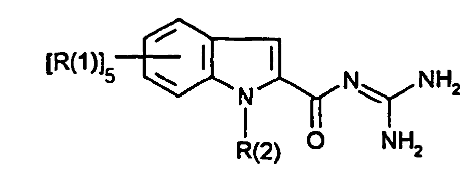 où
R(2) signifie hydrogène, (C₁-C₈)-alkyle non substitué ou substitué, (C₃-C₇)-cycloalkyle, OH, (C₁-C₆)-alkyl-O-, un radical aromatique ou un groupement -CH₂-R(20) ;
R(20) signifie (C₂-C₆)-alcényle ou (C₂-C₆)-alcynyle ; R(1) signifie 1 à 5 substituants identiques ou différents, signifiant :
hydrogène, (C₁-C₈)-alkyle non substitué ou substitué, (C₂-C₆)-alcényle, (C₂-C₆)-alcynyle, (C₃-C₇)-cycloalkyle, halogène, -NO₂, (C₂-C₈)-alcanoyle, arylalcanoyle comprenant jusqu'à 10 atomes de carbone, aroyle comprenant jusqu'à 11 atomes de carbone, -COOH, (C₂-C₆)-alcoxycarbonyle, un groupement aromatique, un des groupements indiqués dans la suite : -OR(3), -NR(6)R(7) ou -S(O)ₙR(40) ;
R(3) signifie hydrogène, (C₁-C₈)-alkyle, (C₁-C₈)-alkyle substitué, (C₃-C₇)-cycloalkyle, un radical aromatique ou un groupement -CH₂-R(30) ;
R(30) signifie alcényle ou alcynyle ;
R(6) et R(7)
indépendamment l'un de l'autre, signifient hydrogène, (C₁-C₈)-alkyle non substitué ou substitué, (C₃-C₇) -cycloalkyle, (C₂-C₈) -alcanoyle, un groupement arylalcanoyle, comprenant jusqu'à 10 atomes de carbone, un groupement aroyle comprenant jusqu'à 11 atomes de carbone, un groupement aromatique ou -CH₂-R(60) ;
R(60) signifie (C₂-C₆)-alcényle ou (C₂-C₆)-alcynyle ; ou
R(6) et R(7)
ensemble avec l'atome de N, signifient une amine cyclique de 5-7 chaînons, qui peut encore contenir d'autres hétéroatomes dans le cycle ;
n vaut zéro, 1 ou 2 ;
R(40) signifie (C₁-C₈)-alkyle non substitué ou substitué ou un groupement aromatique, ou un groupement ; 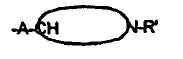
A signifie oxygène, -S(O)ₙ-, ou -NR(R50)- ;
R(50) signifie hydrogène ou (C₁-C₈)-alkyle ;
R' signifie hydrogène, (C₁-C₈)-alkyle non substitué ou substitué,
le cycle représentant un hétérocycle saturé de 3-8 chaînons, comprenant un atome de N,
ledit alkyle substitué portant un ou plusieurs groupements choisis dans le groupe constitué par halogène, -OH, (C₁-C₆)-alcoxy, -CN, -COOH, (C₂-C₆) - alcoxycarbonyle, (C₂-C₈)-alcanoyle, arylalcanoyle comprenant jusqu'à 10 atomes de carbone, aroyle comprenant jusqu'à 11 atomes de carbone, un groupement aromatique, -CONR(4)(R5),
R(4) et R(5)
identiques ou différents, signifient hydrogène ou (C₁-C₈)-alkyle ;
ou
R(4) et R(5)
sont liés l'un à l'autre et forment ensemble une amine cyclique de 5-7 chaînons, qui peut encore contenir d'autres hétéroatomes dans le cycle,
ou ledit alkyle substitué portant un groupement 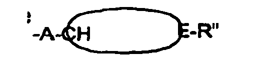 où
E signifie un atome N ou un groupement CH ;
R" signifie hydrogène, (C₁-C₈)-alkyle non substitué ou substitué par OH ou (C₁-C₈)-alkyle substitué, (C₁-C₆) - alcoxy, -CN, -COOH, (C₂-C₆) -alcoxycarbonyle, (C₂-C₈)-alcanoyle, aralcanoyle comprenant jusqu'à 10 atomes de carbone, aroyle comprenant jusqu'à 11 atomes de carbone, un groupement aromatique, -NR(6)R(7), -CONR(4)R(5) ;
R(4) et R(5)
signifient, indépendamment l'un de l'autre, hydrogène ou (C₁-C₈)-alkyle ;
le système cyclique de formule 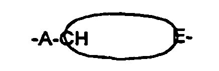 signifiant un système cyclique de 3-8 chaînons, saturé, aliphatique ou hétérocyclique comprenant un atome de N,
et les groupements aromatiques mentionnés signifiant un radical aryle comprenant jusqu'à 10 atomes de carbone, un radical hétéroaryle de 5 ou 6 chaînons comprenant 1-4 atomes de N, un groupement hétéroaryle de 5 ou 6 chaînons contenant 1 ou 2 atomes de N et un hétéroatome signifiant oxygène ou soufre, ou furyle,
et les radicaux aryle mentionnés pouvant être non substitués ou substitués par (C₁-C₈)-alkyle non substitué ou (C₁-C₈)-alkyle substitué, halogène, -NO₂, (C₂-C₈)-alcoxycarbonyle, COOH, -OR(3), NR(6)R(7), -CONR(4)R(5), -SO₂NR(6)R(7) ou S(O)ₙR(40),
R(1) et le radical guanidinocarbonyle pouvant se trouver en un endroit quelconque du cycle de 5 ou 6 chaînons du système indole,
ainsi que leurs sels pharmaceutiquement acceptables associés.
ou
V. des dérivés de guanidine hétérocycliques de formule 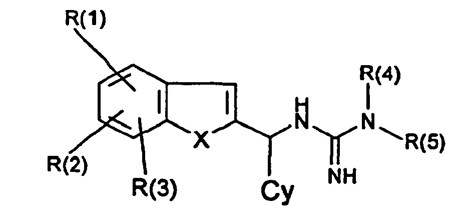 où
X signifie -O-, -S-, -NH-, -N[(C₁-C₄)-alkyl]- ou -N (phényl) - ;
R(1), R(2) et R(3)
signifie hydrogène, halogène, (C₁-C₄)-alkyle, (C₁-C₄)-alkyl-O-, phényle, benzyle ;
ou
deux des substituants R(1), R(2) et R(3) :
ensemble avec un côté du système benzo, signifient un cycle carbocyclique de 4-6 chaînons ;
R(4) et R(5)
indépendamment l'un de l'autre, signifient hydrogène, (C₁-C₁₂)-alkyle, benzydryle, aralkyle,
qui est non substitué ou substitué par un ou plusieurs substituants du groupe formé par halogène, (C₁-C₄-alkyle, (C₁-C₄)-alkyl-O- ou -CF₃, -(CH₂)ₘ-CH₂-T,
m vaut zéro à 3 ;
T signifie -CO-O-T(1) ;
T(1) signifie hydrogène ou (C₁-C₄)-alkyle ;
Cy signifie un hétérocycle à 5 chaînons, benzocondensé, insaturé ou dihydro 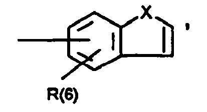
un cycle pyrazole ou imidazole de formule 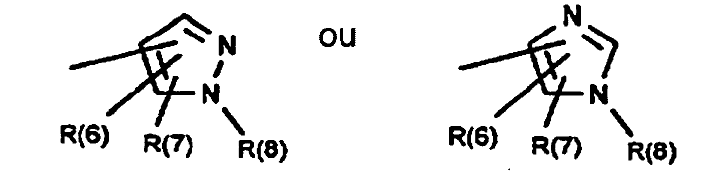
un radical naphtyle ou un radical dihydronaphtyle ou tétrahydronaphtyle 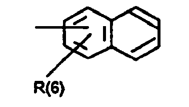
un radical 2-pyridyle, 3-pyridyle ou 4-pyridyle 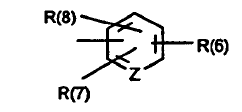
Z signifie N- ou CH ;
un radical thiényle, 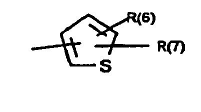
R(6) signifie hydrogène, halogène, hydroxy, (C₁-C₁₀)-alkyle, (C₁-C₁₀)-alkyl-O-, phénoxy, (C₁-C₁₀)-alkyloxyméthyloxy- ou -(O)ₙS-R(9) ;
R(9) signifie (C₁-C₁₀)-alkyle, thiényle, pyridyle, thiazolyle, thiadiazolyle, imidazolyle, pyrazolyle ou phényle,
qui est non substitué ou monosubstitué ou disubstitué par halogène, (C₁-C₄)-alkyle ou (C₁-C₄) - alkyl-O- ;
R(7) et R(8)
signifient hydrogène, halogène, hydroxy, (C₁-C₁₀)-alkyle, (C₁-C₁₀)-alkyl-O-, phényle, phénoxy ou (C₁-C₁₀)-alcoxyméthyloxy ;
ou
Cy signifie phényle,
qui est non substitué ou monosubstitué ou disubstitué par halogène, (C₁-C₄)-alkyle ou (C₁-C₄) - alkyl-O- ;
ou
Cy signifie -Gr-Am ;
Gr signifie -R(13)-R(12)-(CH₂)_{q}-C[W][W(1)]-(CH₂)_{q'}- ; R(13)R(14)- ou -R(15)- ;
R(12) signifie une simple liaison, -O-, -(O)ₙS-, -CO- ou -CONH- ; R(13) signifie une simple liaison, phényle, thiényle, pyridyle, thiazolyle, thiadiazolyle, imidazolyle ou pyrazolyle ;
R(14) signifie une simple liaison, SO₂- ;
R(15) signifie (C₂-C₁₀)-alcényle ou (C₂-C₁₀)-alcynyle ;
W et W(1)
indépendamment l'un de l'autre, signifient hydrogène ou (C₁-C₄)-alkyle,
ou
W et W(1)
liés cycliquement ensemble, signifient un cycle hydrocarboné en C₃-C₈;
q et q'
valent zéro à 9 ;
Am signifie -NR(10)R(11) ;
R(10) signifie hydrogène, (C₁-C₄)-alkyle ou benzyle ;
R(11) signifie (C₁-C₄)-alkyle, phényle ou benzyle ;
ou
R(10) et R(11)
ensemble, signifient un groupement (C₃-C₁₀) - alkylène ;
qui est non substitué ou substitué par -COOH, (C₁-C₅)-alcoxycarbonyle, (C₂-C₄)-hydroxyalkylène ou benzyle ;
ou
Am signifie pyrrolyle, pyridyle, pyrazolyle, morpholinyle, dihydropyridyle, tétrahydropyridyle, quinuclidinyle, imidazolyle, 3-azabicyclo[3,2,1]octyle,
qui est non substitué ou substitué par (C₁-C₄)-alkyle,
ou
Am signifie azabicyclo[3.2.2]nonyle ;
ou
Am signifie un groupement pipérazine de formule 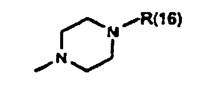
R(16) signifie hydrogène, (C₁-C₄)-alkyle, (C₃-C₆)-cycloalkyle, phényle, toluyle, méthoxyphényle, halogénophényle, diphénylméthylène, benzyle ou pyridyle ;
ou
Am signifie un groupement azido -(O)ₜ-(CH₂)_{q}-C[W] [W(1)]-(CH₂)_{q},-N₃ ;
t vaut zéro ou 1 ;
W et W(1) présentent la signification indiquée ci-dessus ;
ainsi que les énantiomères optiques et les sels pharmacologiquement acceptables ou
VI. des composés de guanidine de la formule suivante 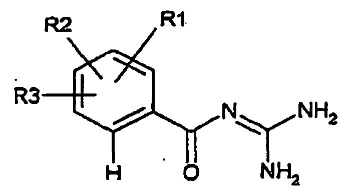 R1 = R2 signifient H, halogéno, alkyle, CN, NO₂, perfluoroalkyle, SOₙCF₃ ; R3 = CH=CH₂, CH₂-CH=CH₂, CH₂-CH₂-CH=CH₂, cycloalcényle, cycloalcénylalkyle ; R4 = alkyle, phényle (substitué) et la substance active d'un point de vue cardiovasculaire
est un médicament hypotenseur pour le traitement de l'hypertension dans des conditions de cardioprotection, un glycoside cardiaque pour le traitement de l'insuffisance cardiaque et de l'insuffisance cardiaque congestive dans des conditions de cardioprotection, un antiarythmique pour le traitement d'arythmies cardiaques de diverses origines dans des conditions de cardioprotection, un nitrate actif d'un point de vue cardiovasculaire, un agent d'ouverture du canal K(ATP) ou un bloquant de K(ATP).

2. Préparation combinée pharmaceutique selon la revendication 1, **caractérisée en ce qu'**elle contient un inhibiteur de NHE (échangeur sodium-hydrogène) et un bloquant des récepteurs bêta pour le traitement de l'hypertension et de l'arythmie dans des conditions de cardioprotection.

3. Préparation combinée pharmaceutique selon la revendication 1, **caractérisée en ce qu'**elle contient un inhibiteur de NHE et un antagoniste du calcium pour le traitement de l'hypertension dans des conditions de cardioprotection.

4. Préparation combinée pharmaceutique selon la revendication 1, **caractérisée en ce qu'**elle contient un inhibiteur de NHE et un inhibiteur de l'enzyme de conversion de l'angiotensine pour le traitement de l'hypertension dans des conditions de cardioprotection.

5. Préparation combinée pharmaceutique selon la revendication 1, **caractérisée en ce qu'**elle contient un inhibiteur de NHE et un diurétique ou un antagoniste de l'aldostérone pour le traitement de l'hypertension et de l'insuffisance cardiaque dans des conditions de cardioprotection.

6. Préparation combinée pharmaceutique selon la revendication 1, **caractérisée en ce qu'**elle contient un inhibiteur de NHE et un antiarythmique des classes I, II, III ou IV pour le traitement d'arythmies cardiaques d'origines diverses dans des conditions de cardioprotection.

7. Préparation combinée pharmaceutique selon la revendication 1, **caractérisée en ce qu'**elle contient l'inhibiteur de NHE Cariporide (Hoe 642) en combinaison avec un inhibiteur du canal calcique non inactivant.
